(19) Europäisches Patentamt
European Patent Office
Office européen des brevets

(11) EP 4 617 278 A1

(12) **EUROPEAN PATENT APPLICATION**
published in accordance with Art. 153(4) EPC

(43) Date of publication:
**17.09.2025 Bulletin 2025/38**

(21) Application number: **23900037.5**

(22) Date of filing: **07.12.2023**

(51) International Patent Classification (IPC):
***C07F 9/6561*** *(2006.01)* ***A61K 31/53*** *(2006.01)*
***A61P 31/12*** *(2006.01)* ***A61P 31/14*** *(2006.01)*
***A61P 31/16*** *(2006.01)*

(52) Cooperative Patent Classification (CPC):
**C07F 9/6561; A61P 31/12; A61P 31/14; A61P 31/16;** C07B 2200/07

(86) International application number:
**PCT/CN2023/136943**

(87) International publication number:
**WO 2024/120464 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.12.2022 CN 202211581049**

(71) Applicant: **Shenzhen TargetRx, Inc.**
**Shenzhen, Guangdong 518057 (CN)**

(72) Inventors:
- **WANG, Yihan**
  **Shenzhen, Guangdong 518057 (CN)**
- **ZHAO, Jiuyang**
  **Shenzhen, Guangdong 518057 (CN)**

(74) Representative: **Novitas Patent AB**
**P.O. Box 55557**
**102 04 Stockholm (SE)**

# (54) NUCLEOTIDE ANALOG, AND COMPOSITION AND USE THEREOF

(57) The present invention relates to a compound of formula (I) or a tautomer, a stereoisomer, a prodrug, a crystalline form, a pharmaceutically acceptable salt, a hydrate or a solvate thereof, a pharmaceutical composition thereof, and use of these substances in the treatment and/or prevention of viral infections.

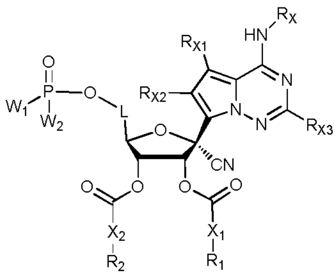

(I)

EP 4 617 278 A1

## Description

## TECHNICAL FIELD

The present invention belongs to the field of medicament, and in particular relates to a nucleotide analogue for the treatment and/or prevention of viral infections, a pharmaceutical composition comprising same, as well as a preparation method and use thereof.

## BACKGROUND OF THE INVENTION

Nucleotide analogues are a class of compounds that have been proven to exhibit antiviral activity both in vitro and in vivo, making them an extensively studied subject for treating viral infections. Nucleotide analogues are typically therapeutically inactive compounds that are converted by host or viral enzymes into their corresponding active antimetabolites, which can then inhibit polymerases involved in viral or cellular proliferation. Activation occurs through various mechanisms, such as the addition of one or more phosphate groups and/or combination with other metabolic processes.

Currently, there is still a lack of effective treatment methods for *Coronaviridae*, *Paramyxoviridae, Pneumoviridae, Picornaviridae, Flaviviridae, Filoviridae, Arenaviridae, Orthomyxovirus*, and monkeypox, which cause numerous diseases in humans and animals.

Therefore, it is necessary to further develop new nucleotide analogues for the treatment of viral infections.

## SUMMARY OF THE INVENTION

The present invention provides a novel nucleotide analogue, a composition comprising the compound, and uses thereof. The compound has potent inhibitory activity against viral RNA polymerases (especially RNA-dependent RNA polymerases), and is effective in treating infections caused by *Coronaviridae*, *Paramyxoviridae*, *Pneumoviridae*, *Picornaviridae*, *Flaviviridae*, *Filoviridae*, *Arenaviridae*, and *Orthomyxoviridae.* The compound exhibits excellent pharmacokinetic properties and is suitable for oral administration.

To this end, the present invention adopts the following technical solutions:

In one aspect, the present invention relates to a compound of formula (I), or a tautomer, stereoisomer, prodrug, crystalline form, pharmaceutically acceptable salt, hydrate or solvate thereof:

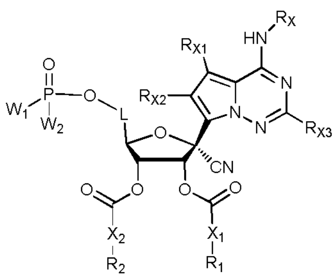

formula (I)

wherein,

$R_X$ is H or OH;

$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$W_1$ is formula (A):

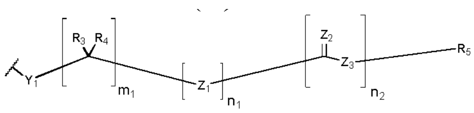

formula (A)

wherein,

$Y_1$ is O or S;
$Z_1$ is O, NH or S;
$Z_2$ is O, NH or S;
$Z_3$ is a bond, O, NH or S;
$m_1$ is 1, 2, 3, 4, 5 or 6;
$n_1$ is 0 or 1;
$n_2$ is 0 or 1;
each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
$W_2$ is formula (B):

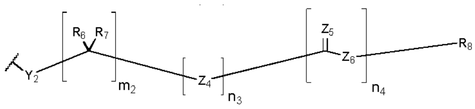

formula (B)

wherein,

$Y_2$ is O, NH or S;
$Z_4$ is O, NH or S;
$Z_5$ is O, NH or S;
$Z_6$ is a bond, O, NH or S;
$m_2$ is 0, 1, 2, 3, 4, 5 or 6;
$n_3$ is 0 or 1;
$n_4$ is 0 or 1;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
or, any one of $W_1$ or $W_2$ together with the 3'-position C atom of glycosyl form $-Y_3-$, wherein $Y_3$ is O, NH or S;
or , $W_1$ and $W_2$ together with the P atom to which they are attached, form $-Y_4(C(R_3R_4)_p)Y_4-$, wherein $Y_4$ is O, NH or S, and p is 2, 3, 4 or 5;
each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R'', up to full deuteration;
each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R' on the same atom or two adjacent atoms, together with the atoms to which they are attached, form a $C_{3-6}$ cycloalkyl, 3-7

membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration.

In another aspect, the present invention relates to a pharmaceutical composition comprising the compound of the present invention, or a tautomer, stereoisomer, prodrug, crystalline form, pharmaceutically acceptable salt, hydrate or solvate thereof, and a pharmaceutically acceptable excipient, as well as optionally, other therapeutic agents.

In another aspect, the present invention relates to a compound of the present invention, or a tautomer, stereoisomer, prodrug, crystalline form, pharmaceutically acceptable salt, hydrate or solvate thereof, or the pharmaceutical composition of the present invention in the preparation of a medicament for use in treatment and/or prevention of viral infections.

In another aspect, the present invention relates to a method for treating and/or preventing viral infections in a subject, the method comprising administering the compound of the present invention, or a pharmaceutically acceptable salt, stereoisomer, solvate, hydrate, polymorph, prodrug or isotopic variant thereof, or the pharmaceutical composition of the present invention to the subject.

In another aspect, the present invention relates to a use of the compound of the present invention, or a tautomer, stereoisomer, prodrug, crystalline form, pharmaceutically acceptable salt, hydrate or solvate thereof, or the pharmaceutical composition of the present invention for the treatment and/or prevention of viral infections.

In a more specific aspect, the viral infections described in the present invention are *Coronaviridae, Paramyxoviridae, Pneumoviridae, Picornavirridae, Flaviviridae, Filoviridae, Arenaviridae*, *Orthomyxoviridae*, or monkeypox.

In a more specific aspect, the coronavirus described in the present invention is SARS-CoV (Severe Acute Respiratory Syndrome Coronavirs) and variants thereof, MERS-CoV (Middle East Respiratory Syndrome Coronavirs) and variants thereof and SARS-CoV-2 (Severe Acute Respiratory Syndrome Coronavirs 2') and variants thereof, other human coronaviruses (229E, NL63, OC43, HKU1 or WIV1), zoonotic coronavirus (PEDV or HKU CoV isolate, such as HKU3, HKU5 or HKU9), feline coronavirus (Feline Enteric Coronavirus (FECV) or Feline infectious peritonitis virus (FIPV)) or porcine epidemic diarrhea virus (PEDV). In a more specific aspect, the coronavirus described in the present invention is SARS-CoV virus and variants thereof, MERS-CoV virus and variants thereof and SARS-CoV-2 virus and variants thereof. In a more specific aspect, the coronavirus described in the present invention is SARS-CoV-2 virus and variants thereof. In a more specific aspect, the variants of SARS-CoV-2 virus described in the present invention are Alpha variants, Beta variants, Delta variants, Gamma variants, or Omicron variants.

In a more specific aspect, the *Paramyxoviridae* described in the present invention is Parainfluenza virus, Measles or Mumps virus.

In a more specific aspect, the *Pneumoviridae* described in the present invention is RSV (respiratory syncytial virus) or human metapneumovirus. In a more specific aspect, the *Pneumoviridae* described in the present invention is RSV.

In a more specific aspect, the *Picornavirridae* described in the present invention is Enterovirus or Erbovirus.

In a more specific aspect, the *Flaviviridae* described in the present invention is dengue virus, Yellow Fever virus, West Nile virus, Zika virus, Japanese encephalitis virus and Hepatitis C virus.

In a more specific aspect, the *Filoviridae* described in the present invention is Ebola virus, Zaire Ebola virus, Bundibugio Ebola virus, Sudan Ebola virus, Tai forest Ebola virus, Reston Ebola virus or Marburg virus. In a more specific aspect, the *Filoviridae* described in the present invention is Ebola virus or Marburg virus.

In a more specific aspect, the *Arenaviridae* described in the present invention is LASA virus or JUNV virus.

In another aspect, the present invention provides a method for inhibiting RNA polymerases, which comprises

contacting the virus-infected cells with the compound of the present invention, or a tautomer, stereoisomer, prodrug, crystalline form, pharmaceutically acceptable salt, hydrate or solvate thereof. In a more specific aspect, the RNA polymerase is an RNA-dependent RNA polymerase.

The other objectives and advantages of the present invention will be apparent to those skilled in the art from the subsequent detailed description, embodiments, and claims.

Definitions

Chemical definitions

Definitions of specific functional groups and chemical terms will be described below in more details.

When a numerical range is recited, it is to be understood that both each individual value and every sub-range within the stated range are expressly included. For example, "$C_{1-6}$ alkyl" includes $C_1$, $C_2$, $C_3$, $C_4$, $C_5$, $C_6$, $C_{1-6}$, $C_{1-5}$, $C_{1-4}$, $C_{1-3}$, $C_{1-2}$, $C_{2-6}$, $C_{2-5}$, $C_{2-4}$, $C_{2-3}$, $C_{3-6}$, $C_{3-5}$, $C_{3-4}$, $C_{4-6}$, $C_{4-5}$ and $C_{5-6}$ alkyl.

"$C_{1-28}$ alkyl" refers to a linear or branched saturated hydrocarbon group having 1 to 28 carbon atoms, and "$C_{1-6}$ alkyl" is designated as "lower alkyl". In some embodiments, $C_{1-4}$ alkyl is particularly preferred. Examples of the alkyl include, but are not limited to: methyl ($C_1$), ethyl ($C_2$), n-propyl ($C_3$), isopropyl ($C_3$), n-butyl ($C_4$), tert-butyl ($C_4$), sec-butyl ($C_4$), iso-butyl ($C_4$), n-pentyl ($C_5$), 3-pentyl ($C_5$), pentyl ($C_5$), neopentyl ($C_5$), 3-methyl-2-butyl ($C_5$), tert-pentyl ($C_5$) and n-hexyl ($C_6$). Whether or not preceded by the term "substituted", each alkyl is independently optionally substituted, for example, with 1 to 5 substituents, 1 to 3 substituents, or 1 substituent, wherein suitable substituents are as defined below.

"$C_{2-28}$ alkenyl" refers to a linear or branched hydrocarbon group having 2 to 28 carbon atoms and one or more carbon-carbon double bonds (e.g., 1, 2, or 3 carbon-carbon double bonds). One or more carbon-carbon double bonds can be located at internal (e.g., in 2-butenyl) or at the ends (e.g., in 1-butenyl). In some embodiments, $C_{2-4}$ alkenyl is particularly preferred. Examples of the alkenyl include, but are not limited to: ethenyl ($C_2$), 1-propenyl ($C_3$), 2-propenyl ($C_3$), 1-butenyl ($C_4$), 2-butenyl ($C_4$), butadienyl ($C_4$), pentenyl ($C_5$), pentadienyl ($C_5$), and hexenyl ($C_6$), etc. Whether or not preceded by the term "substituted", each alkenyl is independently optionally substituted, for example, with 1 to 5 substituents, 1 to 3 substituents, or 1 substituent, wherein suitable substituents are as defined below.

"$C_{2-28}$ alkynyl" refers to a linear or branched hydrocarbon group having 2 to 28 carbon atoms, one or more carbon-carbon triple bonds (e.g., 1, 2, or 3 carbon-carbon triple bonds) and optionally one or more carbon-carbon double bonds (e.g., 1, 2, or 3 carbon-carbon double bonds).

In some embodiments, $C_{2-4}$ alkynyl is particularly preferred. In some embodiments, the alkynyl contains no double bonds. The one or more carbon-carbon triple bonds can be located at internal (e.g., in 2-butynyl) or at the ends (e.g., in 1-butynyl). Examples of the alkynyl include, but are not limited to: ethynyl ($C_2$), 1-propynyl ($C_3$), 2-propynyl ($C_3$), 1-butynyl ($C_4$), 2-butynyl ($C_4$), pentynyl ($C_5$), and hexynyl ($C_6$), etc. Whether or not preceded by the term "substituted", each alkynyl is independently optionally substituted, for example, with 1 to 5 substituents, 1 to 3 substituents, or 1 substituent, wherein suitable substituents are as defined below.

"$C_{1-6}$ alkoxy" refers to the group -OR, wherein R is a substituted or unsubstituted $C_{1-6}$ alkyl. In some embodiments, $C_{1-4}$ alkoxy is particularly preferred. Examples of the alkoxy include, but are not limited to: methoxy, ethoxy, n-propoxy, isopropoxy, n-butoxy, tert-butoxy, sec-butoxy, n-pentyloxy, n-hexyloxy, and 1,2-dimethylbutoxy.

"Halo" or "halogen" refers to fluorine (F), chlorine (Cl), bromine (Br) and iodine (I). In some embodiments, the halogen group is F, Cl or Br. In some embodiments, the halogen group is F or Cl. In some embodiments, the halogen group is F.

Accordingly, "$C_{1-6}$ haloalkyl" and "$C_{1-6}$ haloalkoxy" refer to the above "$C_{1-6}$ alkyl" and "$C_{1-6}$ alkoxy", respectively, substituted with one or more halogen groups. In some embodiments, $C_{1-4}$ haloalkyl is particularly preferred, more preferably $C_{1-2}$. In some embodiments, $C_{1-4}$ haloalkoxy is particularly preferred, more preferably $C_{1-2}$ haloalkoxy. Exemplary haloalkyl includes, but is not limited to: $-CF_3$, $-CH_2F$, $-CHF_2$, $-CHFCH_2F$, $-CH_2CHF_2$, $-CF_2CF_3$, $-CCl_3$, $-CH_2Cl$, $-CHCl_2$, 2,2,2-trifluoro-1,1-dimethyl-ethyl, etc. Exemplary haloalkoxy includes, but is not limited to: - $OCH_2F$, $-OCHF_2$, $-OCF_3$, etc.

"$C_{3-10}$ cycloalkyl" refers to a nonaromatic cyclic hydrocarbon group having 3 to 10 ring carbon atoms and zero heteroatoms. In some embodiments, $C_{3-7}$ cycloalkyl is preferred, $C_{3-6}$ cycloalkyl is particularly preferred, and more preferably $C_{5-6}$ cycloalkyl. Cycloalkyl also includes ring systems in which the aforementioned cycloalkyl ring is fused with one or more aryl or heteroaryl groups, wherein the connection point is on the cycloalkyl ring, and in such cases, the number of carbons continues to represent the number of carbon atoms in the cycloalkyl ring system. Exemplary cycloalkyl includes, but is not limited to: cyclopropyl ($C_3$), cyclopropenyl ($C_3$), cyclobutyl ($C_4$), cyclobutenyl ($C_4$), cyclopentyl ($C_5$), cyclopentenyl ($C_5$), cyclohexyl ($C_6$), cyclohexenyl ($C_6$), cyclohexadienyl ($C_6$), cycloheptyl ($C_7$), cycloheptenyl ($C_7$), cycloheptadienyl ($C_7$), cycloheptatrienyl ($C_7$), cyclooctyl ($C_8$), cyclooctenyl ($C_8$), bicyclo[2.2.1]heptyl ($C_7$), bicyclo [2.2.2]octyl ($C_8$), cyclononyl ($C_9$), cyclononenyl ($C_9$), cyclodecyl ($C_{10}$), cyclodecenyl ($C_{10}$), octahydro-1H-indenyl ($C_9$), decahydronaphthyl ($C_{10}$), spiro[4.5]decyl ($C_{10}$), etc. Whether or not preceded by the term "substituted", each cycloalkyl is

independently optionally substituted, for example, with 1 to 5 substituents, 1 to 3 substituents, or 1 substituent, wherein suitable substituents are as defined below.

"3-10 membered heterocyclyl" refers to a group having a 3-10 membered nonaromatic ring system with ring carbon atoms and 1 to 4 ring heteroatoms, wherein each heteroatom is independently selected from nitrogen, oxygen, sulfur, boron, phosphorus, and silicon. In heterocyclyl containing one or more nitrogen atoms, the connection point may be a carbon or nitrogen atom, as valency permits. In some embodiments, 3-7 membered heterocyclyl is preferred, which is a 3-7 membered nonaromatic ring system having ring carbon atoms and 1-3 ring heteroatoms; in some embodiments, 3-6 membered heterocyclyl is particularly preferred, which is a 3-6 membered nonaromatic ring system having ring carbon atoms and 1-3 ring heteroatoms; more preferably 5-6 membered heterocyclyl, which is a 5-6 membered nonaromatic ring system having ring carbon atoms and 1-3 ring heteroatoms. Heterocyclyl also includes ring systems in which the aforementioned heterocyclyl ring is fused with one or more cycloalkyl, aryl or heteroaryl groups, wherein the connection point is on the heterocyclyl ring; and in such cases, the number of ring members continues to represent the number of ring members in the heterocyclyl ring system. Whether or not preceded by the term "substituted", each heterocyclyl is independently optionally substituted, for example, with 1 to 5 substituents, 1 to 3 substituents, or 1 substituent, wherein suitable substituents are as defined below.

Exemplary 3-membered heterocyclyl containing one heteroatom includes, but is not limited to: aziridinyl, oxiranyl, and thiorenyl. Exemplary 4-membered heterocyclyl containing one heteroatom includes, but is not limited to: azetidinyl, oxetanyl, and thietanyl. Exemplary 5-membered heterocyclyl containing one heteroatom includes, but is not limited to: tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl containing two heteroatoms includes, but is not limited to: dioxolanyl, oxasulfuranyl, disulfuranyl and oxazolidin-2-one. Exemplary 5-membered heterocyclyl containing three heteroatoms includes, but is not limited to: triazolinyl, oxadiazolinyl and thiadiazolinyl. Exemplary 6-membered heterocyclyl containing one heteroatom includes, but is not limited to: piperidyl, tetrahydropyranyl, dihydropyridyl and thianyl. Exemplary 6-membered heterocyclyl containing two heteroatoms includes, but is not limited to: piperazinyl, morpholinyl, dithianyl, dioxanyl. Exemplary 6-membered heterocyclyl containing three heteroatoms includes, but is not limited to: triazinanyl. Exemplary 7-membered heterocyclyl containing one heteroatom includes, but is not limited to: azepanyl, oxepanyl and thiepanyl. Exemplary 8-membered heterocyclyl containing one heteroatom includes, but is not limited to: azocanyl, oxocanyl and thiocanyl. Exemplary 5-membered heterocyclyl fused with $C_6$ aryl rings (also referred to as 5,6-dicyclic heterocyclyl groups herein) includes, but is not limited to: indolinyl, isoindoliny, dihydrobenzofuranyl, dihydrobenzothiophenyl, benzoxazolinonyl, etc. Exemplary 6-membered heterocyclyl fused with $C_6$ aryl rings (also referred to as 6,6-dicyclic heterocyclyl groups herein) includes, but is not limited to: tetrahydroquinolinyl, tetrahydroisoquinolinyl, etc.

"$C_{6-14}$ aryl" refers to a group having a monocyclic or polycyclic (e.g., bicyclic or tricyclic) 4n+2 aromatic ring system (e.g., having 6, 10, or 14 $\pi$ electrons arranged in a cyclic manner) having 6-14 ring carbon atoms and zero heteroatoms. In some embodiments, the aryl has six ring carbon atoms ("$C_6$ aryl"; for example, phenyl). In some embodiments, the aryl has ten ring carbon atoms ("$C_{10}$ aryl"; for example, naphthyl, such as, 1-naphthyl and 2-naphthyl). In some embodiments, the aryl has fourteen ring carbon atoms ("$C_{14}$ aryl"; for example, anthracyl). In some embodiments, $C_{6-10}$ aryl is particularly preferred, more preferably $C_6$ aryl. Aryl also includes ring systems in which the aforementioned aryl ring is fused with one or more cycloalkyl or heterocyclyl groups, and the connection point is on the aryl ring, and in such cases, the number of carbon atoms continues to represent the number of carbon atoms in the aryl ring system. Whether or not preceded by the term "substituted", each aryl is independently optionally substituted, for example, with 1 to 5 substituents, 1 to 3 substituents, or 1 substituent, wherein suitable substituents are as defined below.

"5-10 membered heterocyclyl" refers to a 5-10 membered monocyclic or bicyclic 4n+2 aromatic ring system (e.g., having 6 or 10 $\pi$ electrons arranged in a cyclic manner) containing ring carbon atoms and 1-4 ring heteroatoms, wherein each heteroatom is independently selected from nitrogen, oxygen, and sulfur. In heterocyclyl containing one or more nitrogen atoms, the connection point may be a carbon or nitrogen atom, as valency permits. The heteroaryl bicyclic system can include one or more heteroatoms in one or two rings. Heteroaryl also includes ring systems in which the aforementioned heteroaryl ring is fused with one or more cycloalkyl or heterocyclyl groups, and the connection point is on the heteroaryl ring, and in such cases, the number of carbon atoms continues to represent the number of carbon atoms in the heteroaryl ring system. In some embodiments, 5-6 membered heteroaryl is particularly preferred, which is a 5-6 membered monocyclic or bicyclic 4n+2 aromatic ring system containing ring carbon atoms and 1 to 4 ring heteroatoms. Whether or not preceded by the term "substituted", each heteroaryl is independently optionally substituted, for example, with 1 to 5 substituents, 1 to 3 substituents, or 1 substituent, wherein suitable substituents are as defined below.

Exemplary 5-membered heteroaryl containing one heteroatom includes, but is not limited to: pyrrolyl, furanyl and thienyl. Exemplary 5-membered heteroaryl containing two heteroatoms includes, but is not limited to: imidazolyl, pyrazolyl, oxazolyl, isoxazolyl, thiazolyl and isothiazolyl. Exemplary 5-membered heteroaryl containing three heteroatoms includes, but is not limited to: triazolyl, oxadiazolyl and thiadiazolyl. Exemplary 5-membered heteroaryl containing four heteroatoms includes, but is not limited to: tetrazolyl. Exemplary 6-membered heteroaryl containing one heteroatom includes, but is not limited to: pyridyl. Exemplary 6-membered heteroaryl containing two heteroatoms includes, but is not

limited to: pyridazinyl, pyrimidinyl, and pyrazinyl. Exemplary 6-membered heteroaryl containing three or four heteroatoms includes, but is not limited to: triazinyl and tetrazinyl. Exemplary 7-membered heteroaryl containing one heteroatom includes, but is not limited to: azepinyl, oxepinyl and thiepinyl. Exemplary 5,6-bicyclic heteroaryl includes, but is not limited to: indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl, and purinyl. Exemplary 6,6-bicyclic heteroaryl includes, but is not limited to: naphthyridinyl, pteridinyl, quinolyl, isoquinolyl, cinolinyl, quinoxalinyl, phthalazinyl and quinazolinyl.

Exemplary substituents on the carbon atom include, but are not limited to: halogen, -CN, $-NO_2$, $-N_3$, $-SO_2H$, $-SO_3H$, -OH, $-OR^{aa}$, $-ON(R^{bb})_2$, $-N(R^{bb})_2$, $-N(R^{bb})_3^+X^-$, $-N(OR^{cc})R^{bb}$, -SH, $-SR^{aa}$, $-SSR^{cc}$, $-C(=O)R^{aa}$, $-CO_2H$, -CHO, $-C(OR^{cc})_2$, $-CO_2R^{aa}$, $-OC(=O)R^{aa}$, $-OCO_2R^{aa}$, $-C(=O)N(R^{bb})_2$, $-OC(=O)N(R^{bb})_2$, $-NR^{bb}C(=O)R^{aa}$, $-NR^{bb}CO_2R^{aa}$, $-NR^{bb}C(=O)N(R^{bb})_2$, $-C(=NR^{bb})R^{aa}$, $-C(=NR^{bb})OR^{aa}$, $-OC(=NR^{bb})R^{aa}$, $-OC(=NR^{bb})OR^{aa}$, $-C(=NRb^{b})N(R^{bb})_2$, $-OC(=NR^{bb})N(R^{bb})_2$, $-NR^{bb}C(=NR^{bb})N(R^{bb})_2$, $-C(=O)NR^{bb}SO_2R^{aa}$, $-NR^{bb}SO_2R^{aa}$, $-SO_2N(R^{bb})_2$, $-SO_2R^{aa}$, $-SO_2OR^{aa}$, $-OSO_2R^{aa}$, $-S(=O)R^{aa}$, $-OS(=O)R^{aa}$, $-Si(R^{aa})_3$, $-OSi(R^{aa})_3$, $-C(=S)N(R^{bb})_2$, $-C(=O)SR^{aa}$, $-C(=S)SR^{aa}$, $-SC(=S)SR^{aa}$, $-SC(=O)SR^{aa}$, $-OC(=O)SR^{aa}$, $-SC(=O)OR^{aa}$, $-SC(=O)R^{aa}$, $-P(=O)_2R^{aa}$, $-OP(=O)_2R^{aa}$, $-P(=O)(R^{aa})_2$, $-OP(=O)(R^{aa})_2$, $-OP(=O)(OR^{cc})_2$, $-P(=O)_2N(R^{bb})_2$, $-OP(=O)_2N(R^{bb})_2$, $-P(=O)(NR^{bb})_2$, $-OP(=O)(NR^{bb})_2$, $-NR^{bb}P(=O)(OR^{cc})_2$, $-NR^{bb}P(=O)(NR^{bb})_2$, $-P(R^{cc})_2$, $-P(R^{cc})_3$, $-OP(R^{cc})_2$, $-OP(R^{cc})_3$, $-B(R^{aa})_2$, $-B(OR^{cc})_2$, $-BR^{aa}(OR^{cc})$, alkyl, haloalkyl, alkenyl, alkynyl, carbocyclyl, heterocyclyl, aryl and heteroaryl, wherein, each alkyl, alkenyl, alkynyl, carbocyclyl, heterocyclyl, aryl and heteroaryl is independently substituted with 0, 1, 2, 3, 4 or 5 $R^{dd}$ groups;

or two geminal hydrogens on a carbon atom are substituted with =O, =S, $=NN(R^{bb})_2$, $=NNR^{bb}C(=O)R^{aa}$, $=NNR^{bb}C(=O)OR^{aa}$, $=NNR^{bb}S(=O)_2R^{aa}$, $=Nrbb^{bb}$ or $=NOR^{cc}$;

each $R^{aa}$ is independently selected from alkyl, haloalkyl, alkenyl, alkynyl, carbocyclyl, heterocyclyl, aryl and heteroaryl, or two $R^{aa}$ groups join to form a heterocyclyl or heteroaryl ring, wherein, each alkyl, alkenyl, alkynyl, carbocyclyl, heterocyclyl, aryl and heteroaryl is independently substituted with 0, 1, 2, 3, 4 or 5 $R^{dd}$ groups;

each $R^{bb}$ is independently selected from: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{cc})OR^{aa}$, $-C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, $-C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{bb}$ groups join to form a heterocyclyl or heteroaryl ring, wherein, each alkyl, alkenyl, alkynyl, carbocyclyl, heterocyclyl, aryl and heteroaryl is independently substituted with 0, 1, 2, 3, 4 or 5 个 $R^{dd}$ groups;

each $R^{cc}$ is independently selected from hydrogen, alkyl, haloalkyl, alkenyl, alkynyl, carbocyclyl, heterocyclyl, aryl and heteroaryl, or two $R^{cc}$ groups join to form a heterocyclyl or heteroaryl ring, wherein, each alkyl, alkenyl, alkynyl, carbocyclyl, heterocyclyl, aryl and heteroaryl is independently substituted with 0, 1, 2, 3, 4 or 5 $R^{dd}$ groups;

each $R^{dd}$ is independently selected from: halogen, -CN, $-NO_2$, $-N_3$, $-SO_2H$, $-SO_3H$, -OH, $-OR^{ee}$, $-ON(R^{ff})_2$, $-N(R^{ff})_2$, , $-N(R^{ff})_3^+X^-$, $-N(OR^{ee})R^{ff}$, -SH, $-SR^{ee}$, $-SSR^{ee}$, $-C(=O)R^{ee}$, $-CO_2H$, $-CO_2R^{ee}$, $-OC(=O)R^{ee}$, $-OCO_2R^{ee}$, $-C(=O)N(R^{ff})_2$, $-OC(=O)N(R^{ff})_2$, $-NR^{ff}C(=O)R^{ee}$, $-NR^{ff}CO_2R^{ee}$, $-NR^{ff}C(=O)N(R^{ff})_2$, $-C(=NR^{ff})OR^{ee}$, $-OC(=NR^{ff})R^{ee}$, $-OC(=NR^{ff})OR^{ee}$, $-C(=NR^{ff})N(R^{ff})_2$, $-OC(=NR^{ff})N(R^{ff})_2$, $-NR^{ff}C(=NR^{ff})N(R^{ff})_2$, $-NR^{ff}SO_2R^{ee}$, $-SO_2N(R^{ff})_2$, $-SO_2R^{ee}$, $-SO_2OR^{ee}$, $-OSO_2R^{ee}$, $-S(=O)R^{ee}$, $-Si(R^{ee})_3$, $-OSi(R^{ee})_3$, $-C(=S)N(R^{ff})_2$, $-C(=O)SR^{ee}$, $-C(=S)SR^{ee}$, $-SC(=S)SR^{ee}$, $-P(=O)_2R^{ee}$, $-P(=O)(R^{ee})_2$, $-OP(=O)(R^{ee})_2$, $-OP(=O)(OR^{ee})_2$, alkyl, haloalkyl, alkenyl, alkynyl, carbocyclyl, heterocyclyl, aryl and heteroaryl, wherein, each alkyl, haloalkyl, alkenyl, alkynyl, carbocyclyl, heterocyclyl, aryl and heteroaryl is independently substituted with 0, 1, 2, 3, 4 or 5 $R^{gg}$ groups, or two geminal $R^{dd}$ substituents can be bound to form =O or =S;

each $R^{ee}$ is independently selected from alkyl, haloalkyl, alkenyl, alkynyl, carbocyclyl, aryl, heterocyclyl and heteroaryl, wherein, each alkyl, alkenyl, alkynyl, carbocyclyl, heterocyclyl, aryl and heteroaryl is independently substituted with 0, 1, 2, 3, 4 or 5 $R^{gg}$ groups;

each $R^{ff}$ is independently selected from hydrogen, alkyl, haloalkyl, alkenyl, alkynyl, carbocyclyl, heterocyclyl, aryl and heteroaryl, or two $R^{ff}$ groups join to form a heterocyclyl or heteroaryl ring, wherein, each alkyl, alkenyl, alkynyl, carbocyclyl, heterocyclyl, aryl and heteroaryl is independently substituted with 0, 1, 2, 3, 4 or 5 $R^{gg}$ groups;

each $R^{gg}$ is independently selected from: halogen, -CN, $-NO_2$, $-N_3$, $-SO_2H$, $-SO_3H$, -OH, $-OC_{1-6}$ alkyl, $-ON(C_{1-6}$ alkyl$)_2$, $-N(C_{1-6}$ alkyl$)_2$, $-N(C_{1-6}$ alkyl$)_3^+X^-$, $-NH(C_{1-6}$ alkyl$)_2^+X^-$, $-NH_2(C_{1-6}$ alkyl$)^+X^-$, $-NH_3^+X^-$, $-N(OC_{1-6}$ alkyl$)(C_{1-6}$ alkyl), $-N(OH)(C_{1-6}$ alkyl), -NH(OH), -SH, $-SC_{1-6}$ alkyl, $-SS(C_{1-6}$ alkyl), $-C(=O)(C_{1-6}$ alkyl), $-CO_2H$, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl$)C(=O)(C_{1-6}$ alkyl), $-NHCO_2(C_{1-6}$ alkyl), $-NHC(=O)N(C_{1-6}$ alkyl$)_2$, $-NHC(=O)NH(C_{1-6}$ alkyl), $-NHC(=O)NH_2$, $-C(=NH)O(C_{1-6}$ alkyl), $-OC(=NH)(C_{1-6}$ alkyl), $-OC(=NH)OC_{1-6}$ alkyl, $-C(=NH)N(C_{1-6}$ alkyl$)_2$, $-C(=NH)NH(C_{1-6}$ alkyl), $-C(=NH)NH_2$, $-OC(=NH)N(C_{1-6}$ alkyl$)_2$, $-OC(NH)NH(C_{1-6}$ alkyl), $-OC(NH)NH_2$, $-NHC(NH)N(C_{1-6}$ alkyl$)_2$, $-NHC(=NH)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, $-SO_2OC_{1-6}$ alkyl, $-OSO_2C_{1-6}$ alkyl, $-SOC_{1-6}$ alkyl, $-Si(C_{1-6}$ alkyl$)_3$, $-OSi(C_{1-6}$ alkyl$)_3$, $-C(=S)N(C_{1-6}$ alkyl$)_2$, $C(=S)NH(C_{1-6}$ alkyl), $C(=S)NH_2$, $-C(=O)S(C_{1-6}$ alkyl), $-C(=S)SC_{1-6}$ alkyl, $-SC(=S)SC_{1-6}$ alkyl, $-P(=O)_2(C_{1-6}$ alkyl), $-P(=O)(C_{1-6}$ alkyl$)_2$,

$-OP(=O)(C_{1-6}$ alkyl$)_2$, - $OP(=O)(OC_{1-6}$ alkyl$)_2$, $C_{1-6}$ alkyl, $C_{1-6}$ haloalkyl, $C_2$-$C_6$ alkenyl, $C_2$-$C_6$ alkynyl, $C_3$-$C_7$ carbocyclyl, $C_6$-$C_{10}$ aryl, $C_3$-$C_7$ heterocyclyl, $C_5$-$C_{10}$ heteroaryl; or two geminal $R^{gg}$ substituents can be bound to form =O or =S; wherein, $X^-$ is a counterion.

Exemplary substituents on nitrogen atoms include, but are not limited to: hydrogen, -OH, $-OR^{aa}$, $-N(R^{cc})_2$, -CN, $-C(=O)R^{aa}$, $-C(=O)N(R^{cc})_2$, $-CO_2R^{aa}$, $-SO_2R^{aa}$, $-C(=NR^{bb})R^{aa}$, $-C(=NR^{cc})OR^{aa}$, - $C(=NR^{cc})N(R^{cc})_2$, $-SO_2N(R^{cc})_2$, $-SO_2R^{cc}$, $-SO_2OR^{cc}$, $-SOR^{aa}$, $-C(=S)N(R^{cc})_2$, $-C(=O)SR^{cc}$, - $C(=S)SR^{cc}$, $-P(=O)_2R^{aa}$, $-P(=O)(R^{aa})_2$, $-P(=O)_2N(R^{cc})_2$, $-P(=O)(NR^{cc})_2$, alkyl, haloalkyl, alkenyl, alkynyl, carbocyclyl, heterocyclyl, aryl and heteroaryl, or two $R^{cc}$ groups connected to a nitrogen atom can be bound to form a heterocyclyl or heteroaryl ring, wherein each alkyl, alkenyl, alkynyl, carbocyclyl, heterocyclyl, aryl and heteroaryl is independently substituted with 0, 1, 2, 3, 4 or 5 $R^{dd}$ groups, and wherein $R^{aa}$, $R^{bb}$, $R^{cc}$ and $R^{dd}$ are as defined above.

"Deuteration" or "D" refers to the substitution of one or more hydrogen atoms in a compound or group with deuterium. Deuterium substitution may be mono-, di-, poly- or full-substitution. The terms "substituted with one or more deuterium atoms" and "deuterated one or more times" can be used interchangeably.

A "non-deuterated compound" refers to a compound containing deuterium atoms in a proportion not higher than the natural deuterium isotopic abundance (0.015%).

The deuterium content at deuterated positions should be at least greater than the natural deuterium isotopic abundance of 0.015%, preferably greater than 30%, more preferably greater than 50%, still more preferably greater than 75%, even more preferably greater than 95%, and most preferably greater than 99%.

The term "pharmaceutically acceptable salts" refers to those salts that, within reliable medical judgment range, are suitable for contact with the tissues of humans and lower animals without excessive toxicity, irritation, and allergic reactions, etc., and are proportionate to reasonable benefit/risk ratio. Pharmaceutically acceptable salts are well known in the art. For example, the pharmaceutically acceptable salts as described in detail in Berge, et al "J. Pharmaceutical Sciences (1977) 66:1-19". The pharmaceutically acceptable salts of the compounds of the present invention include salts derived from suitable inorganic and organic acids and inorganic and organic bases. Examples of pharmaceutically acceptable, non-toxic acid addition salts are those formed with inorganic acids such as hydrochloric acid, hydrobromic acid, phosphoric acid, sulfuric acid, and perchloric acid, or those formed with organic acids such as acetic acid, oxalic acid, maleic acid, tartaric acid, citric acid, succinic acid, or malonic acid. Also included are salts formed by conventional methods in the art, for example, by ion exchange. Other pharmaceutically acceptable salts include: adipates, alginates, ascorbates, aspartates, benzenesulfonates, benzoates, bisulfates, borates, butyrates, camphorates, camphorsulfonates, citrates, cypionates, digluconates, dodecyl sulfates, esilates, formates, fumarates, glucoheptonates, glycerophosphates, gluconates, hemisulfates, heptanoates, hexanoates, hydroiodates, 2-hydroxy-esilates, lactobionates, lactates, laurates, lauryl sulfates, malates, maleates, malonates, mesylates, 2-naphthalenesulfonates, nicotinates, nitrates. oleates, oxalates, palmitates, pamoates, pectates, persulfates, 3-phenpropionates, phosphates, picrates, tervalerates, propionates, stearates, succinates, sulfates, tartrates, thiocyanates, tosilates, undecanoates, valerates, etc. Pharmaceutically acceptable salts derived from suitable bases include alkali metals, alkaline earth metals, ammonium, and $N^+(C_{1-4}alkyl)_4$ salts. Representative alkali or alkaline earth metal salts include sodium, lithium, potassium, calcium, and magnesium salts, etc. Other pharmaceutically acceptable salts, when appropriate, include non-toxic ammonium salts, quaternary ammonium salts, and amine cations formed with counterions such as halide, hydroxide, carboxylate, sulfate, phosphate, nitrate, lower alkyl sulfonate and aryl sulfonate. The "subject" to be treated includes, but is not limited to: humans (i.e., males or females of any age group, for example, pediatric subjects (e.g., infants, children, adolescents) or adult subjects (e.g., young adults, middle-aged adults or elderly adults)) and/or non-human animals, for example, mammals, for example, primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "person", "patient", and "subject" are used interchangeably herein.

The terms "disease", "disorder", and "condition" are used interchangeably herein.

Unless otherwise specified, the term "treatment" as used herein includes the effects that occur when the subject suffers from a specific disease, disorder, or condition, which reduces the severity of the disease, disorder, or condition, or delays or slows down the development of the disease, disorder, or condition ("therapeutic treatment"), as well as the effects that occur before the subject begins to suffer from the specific disease, disorder, or condition ("prophylactic treatment").

"Combination" and related terms refer to the simultaneous or sequential administration of a therapeutic agent of the present invention. For example, the compound of the present invention can be administered simultaneously or sequentially in separate unit dosage forms with another therapeutic agent, or simultaneously in a single unit dosage form with another therapeutic agent.

## DETAILED DESCRIPTION OF THE INVENTION

Compounds

In the present invention, "compound of the present invention" refers to a compound of formula (I) (including subsets of each formula), or a pharmaceutically acceptable salt, a hydrate, or a solvate thereof.

In an embodiment, the present invention relates to a compound of formula (I), or a tautomer, stereoisomer, prodrug, crystalline form, pharmaceutically acceptable salt, hydrate or solvate thereof:

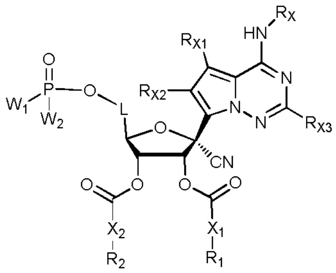

formula (I)

wherein,

$R_X$ is H or OH;

$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$W_1$ is formula (A):

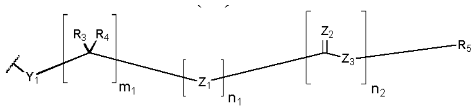

formula (A)

wherein,

$Y_1$ is O or S;

$Z_1$ is O, NH or S;

$Z_2$ is O, NH or S;

$Z_3$ is a bond, O, NH or S;

$m_1$ is 1, 2, 3, 4, 5 or 6;

$n_1$ is 0 or 1;

$n_2$ is 0 or 1;

each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

$W_2$ is formula (B):

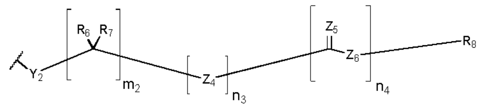

formula (B)

wherein,
$Y_2$ is O, NH or S;
$Z_4$ is O, NH or S;
$Z_5$ is O, NH or S;
$Z_6$ is a bond, O, NH or S;
$m_2$ is 0, 1, 2, 3, 4, 5 or 6;
$n_3$ is 0 or 1;
$n_4$ is 0 or 1;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
or, any one of $W_1$ or $W_2$ together with the 3'-position C atom of glycosyl form $-Y_3-$, wherein $Y_3$ is O, NH or S;
or , $W_1$ and $W_2$ together with the P atom to which they are attached, form $-Y_4(C(R_3R_4)_p)Y_4-$, wherein $Y_4$ is O, NH or S, and p is 2, 3, 4 or 5;
each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;
each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";
each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;
wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration.

$R_X$

In an embodiment, $R_X$ is H; and in another embodiment, $R_X$ is OH.

$R_{X1}$, $R_{X2}$ and $R_{X3}$

In an embodiment, $R_{X1}/R_{X2}/R_{X3}$ is H; in another embodiment, $R_{X1}/R_{X2}/R_{X3}$ is D; in another embodiment, $R_{X1}/R_{X2}/R_{X3}$ is halogen; in another embodiment, $R_{X1}/R_{X2}/R_{X3}$ is $-R_a$; in another embodiment, $R_{X1}/R_{X2}/R_{X3}$ is $-C(O)R_a$; in another embodiment, $R_{X1}/R_{X2}/R_{X3}$ is $-C(O)OR_a$; in another embodiment, $R_{X1}/R_{X2}/R_{X3}$ is $-C(O)NR_bR_c$; in another embodiment, $R_{X1}/R_{X2}/R_{X3}$ is $-NR_bR_c$; in another embodiment, $R_{X1}/R_{X2}/R_{X3}$ is $-NR_aC(O)R_b$; in another embodiment, $R_{X1}/R_{X2}/R_{X3}$ is $-NR_aC(O)OR_b$; in another embodiment, $R_{X1}/R_{X2}/R_{X3}$ is $-NR_aC(O)NR_bR_c$; in another embodiment, $R_{X1}/R_{X2}/R_{X3}$ is $-OR_a$; in another embodiment, $R_{X1}/R_{X2}/R_{X3}$ is $-OC(O)R_a$; in another embodiment, $R_{X1}/R_{X2}/R_{X3}$ is $-OC(O)NR_bR_c$; in another embodiment, $R_{X1}/R_{X2}/R_{X3}$ is $-NR_aS(O)_2R_b$; in another embodiment, $R_{X1}/R_{X2}/R_{X3}$ is $-S(O)_2NR_a$; in another embodiment, $R_{X1}/R_{X2}/R_{X3}$ is $-S(O)R_a$; and in another embodiment, $R_{X1}/R_{X2}/R_{X3}$ is $-S(O)_2R_a$.

In a specific embodiment, $R_{X1}$, $R_{X2}$ and $R_{X3}$ are independently H, D, halogen or $C_{1-6}$ alkyl, wherein the $C_{1-6}$ alkyl is optionally substituted with one or more D, up to full deuteration; in another specific embodiment, $R_{X1}$, $R_{X2}$ and $R_{X3}$ are independently H, D or halogen; in another specific embodiment, $R_{X1}$, $R_{X2}$ and $R_{X3}$ are independently H, D, I, Br, Cl, F, $CH_3$ or $CD_3$; in another specific embodiment, $R_{X1}$, $R_{X2}$ and $R_{X3}$ are independently H, D, I or Br; in another specific embodiment, $R_{X1}$, $R_{X2}$ and $R_{X3}$ are independently H or D; and in another specific embodiment, $R_{X1}$, $R_{X2}$ and $R_{X3}$ are independently H or D.

$R_a$, $R_b$ and $R_c$

In an embodiment, $R_a/R_b/R_c$ is H; in another embodiment, $R_a/R_b/R_c$ is D; in another embodiment, $R_a/R_b/R_c$ is $C_{1-6}$ alkyl; in another embodiment, $R_a/R_b/R_c$ is $C_{1-6}$ haloalkyl; in another embodiment, $R_a/R_b/R_c$ is $C_{3-6}$ cycloalkyl; in another embodiment, $R_a/R_b/R_c$ is 3-7 membered heterocyclyl; in another embodiment, $R_a/R_b/R_c$ is $C_{2-6}$ alkenyl; in another embodiment, $R_a/R_b/R_c$ is $C_{2-6}$ alkynyl; in another embodiment, $R_a/R_b/R_c$ is $C_{6-10}$ aryl; in another embodiment, $R_a/R_b/R_c$ is 5-10 membered heteroaryl; in another embodiment, $R_b$ and $R_c$ together with the N atom to which they are attached, form a 3-7 membered heterocyclyl; and in another embodiment, $R_b$ and $R_c$ together with the N atom to which they are attached, form a 3-7 membered heterocyclyl; in another embodiment, each group as defined in $R_a/R_b/R_c$ is optionally substituted with one or more R.

In a specific embodiment, $R_a$, $R_b$ and $R_c$ are independently $C_{1-6}$ alkyl, $C_{6-10}$ aryl or 5-10 membered heteroaryl; and in another specific embodiment, $R_a$, $R_b$ and $R_c$ are independently $C_{1-6}$ alkyl.

$X_1$ and $X_2$

In an embodiment, $X_1$ is a bond; in another embodiment, $X_1$ is O; and in another embodiment, $X_1$ is S; in another embodiment, $X_1$ is NH.

In an embodiment, $X_2$ is a bond; in another embodiment, $X_2$ is O; in another embodiment, $X_2$ is S; and in another embodiment, $X_2$ is NH.

In a specific embodiment, $X_1$ and $X_2$ are independently a bond, O, S or NH; in another specific embodiment, $X_1$ and $X_2$ are independently a bond or O; in another specific embodiment, $X_1$ and $X_2$ are a bond; and in another specific embodiment, $X_1$ and $X_2$ are O.

$R_1$ and $R_2$

In an embodiment, $R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

In a specific embodiment, $R_1$ and $R_2$ are independently $C_{1-28}$ alkyl, $C_{3-6}$ cycloalkyl, $C_{2-28}$ alkenyl, $C_{2-28}$ alkynyl or $C_{6-10}$ aryl, wherein the $C_{1-28}$ alkyl, $C_{3-6}$ cycloalkyl, $C_{2-28}$ alkenyl, $C_{2-28}$ alkynyl or $C_{6-10}$ aryl is optionally substituted with one or more R; in another specific embodiment, $R_1$ and $R_2$ are independently $C_{1-6}$ alkyl, $C_{3-6}$ cycloalkyl or $C_{6-10}$ aryl, wherein the $C_{1-6}$ alkyl, $C_{3-6}$ cycloalkyl or $C_{6-10}$ aryl is optionally substituted with one or more R; in another specific embodiment, $R_1$ and $R_2$ are independently $C_{1-6}$ alkyl, wherein the $C_{1-6}$ alkyl is optionally substituted with one or more R; in another specific embodiment, $R_1$ and $R_2$ are independently $C_{1-6}$ alkyl; in another specific embodiment, $R_1$ and $R_2$ are independently $C_{1-3}$ alkyl; in another specific embodiment, $R_1$ and $R_2$ are independently methyl, ethyl, isopropyl or tert-butyl; and in another specific embodiment, $R_1$ and $R_2$ are independently methyl, ethyl or isopropyl.

L

In an embodiment, L is $CH_2$; in another embodiment, L is $CD_2$; and in another embodiment, L is CHD.

In a specific embodiment, L is $CH_2$, $CD_2$ or CHD; in another specific embodiment, L is $CH_2$ or $CD_2$; and in another specific embodiment, L is $CH_2$.

$W_1$ and $W_2$

In an embodiment, $W_1$ is independently formula (A):

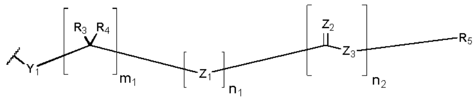

formula (A)

wherein,

- $Y_1$ is O or S;
- $Z_1$ is O, NH or S;
- $Z_2$ is O, NH or S;
- $Z_3$ is a bond, O, NH or S;
- $m_1$ is 1, 2, 3, 4, 5 or 6;
- $n_1$ is 0 or 1;
- $n_2$ is 0 or 1;
- each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
- $R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R.

In an embodiment, $Y_1$ is O; and in another embodiment, $Y_1$ is S.

In a specific embodiment, $Y_1$ is O or S; and in another specific embodiment, $Y_1$ is O.

In an embodiment, $Z_1$ is O; in another embodiment, $Z_1$ is NH; and in another embodiment, $Z_1$ is S.

In a specific embodiment, $Z_1$ is O, NH or S; and in another specific embodiment, $Z_1$ is O or S.

In an embodiment, $Z_2$ is O; in another embodiment, $Z_2$ is NH; and in another embodiment, $Z_2$ is S.

In a specific embodiment, $Z_2$ is O, NH or S; and in another specific embodiment, $Z_2$ is O or S.

In an embodiment, $Z_3$ is a bond; in another embodiment, $Z_3$ is O; in another embodiment, $Z_3$ is NH; and in another embodiment, $Z_3$ is S.

In a specific embodiment, $Z_3$ is a bond, O, NH or S; and in another specific embodiment, $Z_3$ is a bond or O.

In an embodiment, $m_1$ is 1; in another embodiment, $m_1$ is 2; in another embodiment, $m_1$ is 3; in another embodiment, $m_1$ is 4; in another embodiment, $m_1$ is 5; and in another embodiment, $m_1$ is 6.

In a specific embodiment, $m_1$ is 1, 2, 3, 4, 5 or 6; in another specific embodiment, $m_1$ is 1, 2, 3 or 4; in another specific embodiment, $m_1$ is 1, 2 or 3; and in another specific embodiment, $m_1$ is 1 or 3.

In an embodiment, $n_1$ is 0; and in another embodiment, $n_1$ is 1.

In an embodiment, $n_2$ is 0; and in another embodiment, $n_2$ is 1.

In an embodiment, each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R'; in another specific embodiment, each of $R_3$ and $R_4$ is independently H, D, $C_{1-6}$ alkyl, $C_{1-6}$ alkoxy, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R'; in another specific embodiment, each of $R_3$ and $R_4$ is independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{3-6}$ cycloalkyl and 3-7 membered heterocyclyl are optionally substituted with one or more R'; and in another specific embodiment, each of $R_3$ and $R_4$ is independently H, D, methyl, benzyl,

$-CH_2C(O)OC_5H_{11}$,

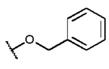 or 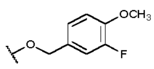 ;

or $R_3$ and $R_4$ together with the C atom to which they are attached, form cyclobutane.

In an embodiment, each $R_5$ is independently H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; in another specific embodiment, each $R_5$ is independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl is optionally substituted with one or more R; in another specific embodiment, each $R_5$ is independently $C_{1-6}$ alkyl, $C_{1-6}$ haloalkyl, $C_{3-6}$ cycloalkyl, 3-7 membered heterocyclyl, $C_{2-6}$ alkenyl or $C_{2-6}$ alkynyl, wherein the $C_{1-6}$ alkyl, $C_{1-6}$ haloalkyl, $C_{3-6}$ cycloalkyl, 3-7 membered heterocyclyl, $C_{2-6}$ alkenyl and $C_{2-6}$ alkynyl are optionally substituted with one or more R; in another specific embodiment, each $R_5$ is independently $C_{1-6}$ alkyl, $C_{1-6}$ haloalkyl, $C_{3-6}$ cycloalkyl or 3-7 membered heterocyclyl, wherein the $C_{1-6}$ alkyl, $C_{1-6}$ haloalkyl, $C_{3-6}$ cycloalkyl and 3-7 membered heterocyclyl are optionally substituted with one or more R; in another specific embodiment, each $R_5$ is independently $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl or $C_{8-28}$ alkynyl is optionally substituted with one or more R; in another specific embodiment, each $R_5$ is independently $C_{8-28}$ alkyl or $C_{8-28}$ alkenyl, wherein the $C_{8-28}$ alkyl and $C_{8-28}$ alkenyl are optionally substituted with one or more R; in another specific embodiment, each $R_5$ is independently $C_{8-28}$ alkyl, wherein the $C_{8-28}$ alkyl is optionally substituted with one or more R; in another specific embodiment, each $R_5$ is independently $C_{8-28}$ alkenyl, wherein the $C_{8-28}$ alkenyl is optionally substituted with one or more R; in another specific embodiment, each $R_5$ is independently $C_{6-10}$ aryl or 5-10 membered heteroaryl, wherein the $C_{6-10}$ aryl or 5-10 membered heteroaryl is optionally substituted with one or more R; in another specific embodiment, each $R_5$ is independently $C_{6-10}$ aryl, wherein the $C_{6-10}$ aryl is optionally substituted with one or more R; in another specific embodiment, each $R_5$ is independently methyl, ethyl, isopropyl, tert-butyl,

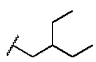

, n-pentyl,

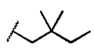 , 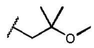 ,

cyclobutane, $-CH_2(CH_2)_{10}CH_3$, $-CH_2(CH_2)_{12}CH_3$, $-CH_2(CH_2)_{14}CH_3$, $-CH_2(CH_2)_{16}CH_3$, - $CH_2(CH_2)_7$ or $-CH_2(CH_2)_7CH=CHCH_2CH=CH(CH_2)_4CH_3$; in another specific embodiment, each $R_5$ is independently methyl, ethyl, isopropyl, tert-butyl,

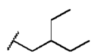

, n-pentyl,

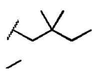

; in another specific embodiment, each $R_5$ is independently isopropyl, tert-butyl or

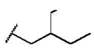;

and in another specific embodiment, each $R_5$ is independently isopropyl or

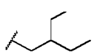.

In an embodiment, $W_1$ is independently:

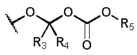, 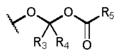, 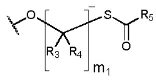, 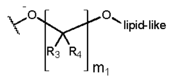, 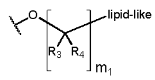 or 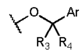;

wherein,

$m_1$ is 1, 2, 3, 4, 5 or 6;

each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R.

In a specific embodiment, $W_1$ is

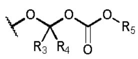,

wherein $R_3$ and $R_4$ are independently H, D, $C_{1-6}$ alkyl, $C_{1-6}$ alkoxy, $C_{3-6}$ cycloalkyl or 3-7 membered heterocyclyl, or $R_3$and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

$R_5$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_1$ is

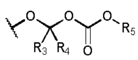,

wherein $R_3$ and $R_4$ is H;

$R_5$ is independently $C_{1-28}$ alkyl, $C_{3-6}$ cycloalkyl, 3-7 membered heterocyclyl or $C_{2-28}$ alkenyl.

In another specific embodiment, $W_1$ is

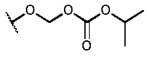.

In a specific embodiment, $W_1$ is

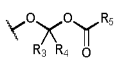,

wherein, $R_3$ and $R_4$ are independently H, D, $C_{1-6}$ alkyl, $C_{1-6}$ alkoxy, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

$R_5$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_1$ is

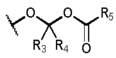,

wherein, $R_3$ and $R_4$ are H;
$R_5$ is independently $C_{1-28}$ alkyl, $C_{3-6}$ cycloalkyl, 3-7 membered heterocyclyl or $C_{2-28}$ alkenyl.

In another specific embodiment, $W_1$ is

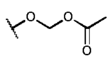, 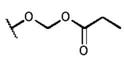 or 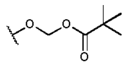;

preferably, $W_2$ is

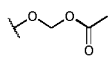, 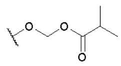 or 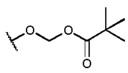.

In a specific embodiment, $W_1$ is

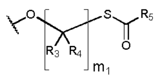,

wherein, $m_1$ is 1, 2, 3 or 4;
each of $R_3$ and $R_4$ is independently H, D, $C_{1-6}$ alkyl, $C_{1-6}$ alkoxy, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';
$R_5$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_1$ is

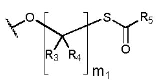,

wherein, $m_1$ is 1 or 2;
$R_3$ and $R_4$ are H;
$R_5$ is independently $C_{1-28}$ alkyl, $C_{3-6}$ cycloalkyl, 3-7 membered heterocyclyl or $C_{2-28}$ alkenyl.

In another specific embodiment, $W_1$ is

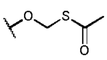, 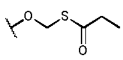, 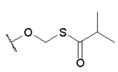, 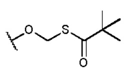, 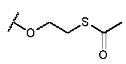,

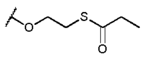, 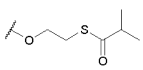 or 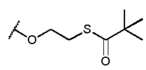.

In a specific embodiment, $W_1$

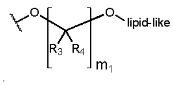,

wherein, $m_1$ is 1, 2, 3 or 4;

each of $R_3$ and $R_4$ is independently H, D, $C_{1-6}$ alkyl, $C_{1-6}$ alkoxy, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R.

In a specific embodiment, $W_1$ is

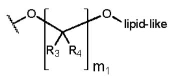,

wherein, $m_1$ is 2, 3 or 4;

$R_3$ and $R_4$ are independently H or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;

lipid-like is $C_{8-28}$ alkyl or $C_{8-28}$ alkenyl.

In another specific embodiment, $W_1$ is

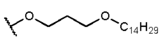, 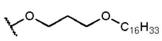, 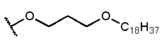, 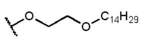,

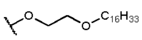, 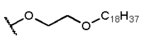, 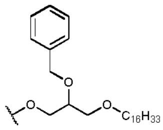, 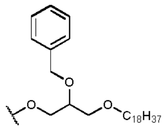, 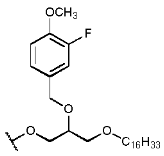 or 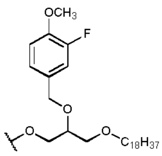;

In another specific embodiment, $W_1$ is

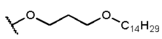, 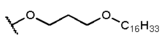, 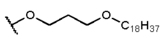, 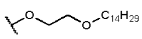,

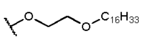, 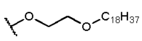, 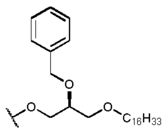, 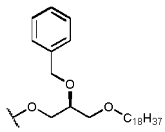, 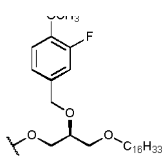 or 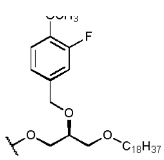.

In a specific embodiment, $W_1$ is

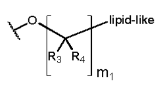,

wherein, $m_1$ is 1, 2, 3 or 4;

each of $R_3$ and $R_4$ is independently H, D, $C_{1-6}$ alkyl, $C_{1-6}$ alkoxy, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl,

wherein the $C_{1-6}$ alkyl, $C_{1-6}$ alkoxy, $C_{3-6}$ cycloalkyl and 3-7 membered heterocyclyl are optionally substituted with one or more R';
lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R.

In another specific embodiment, $W_1$ is

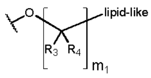,

wherein, $m_1$ is 2, 3 or 4;
$R_3$ and $R_4$ are H;
lipid-like is $C_{8-28}$ alkenyl.

In another specific embodiment, $W_1$ is

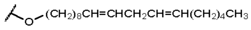.

In a specific embodiment, $W_1$ is

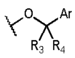,

wherein, $R_3$ and $R_4$ are independently H, D, $C_{1-6}$ alkyl, $C_{1-6}$ alkoxy, $C_{3-6}$ cycloalkyl or 3-7 membered heterocyclyl, or $R_3$and $R_4$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';
Ar is $C_{6-10}$ aryl or 5-10 membered heteroaryl, wherein the $C_{6-10}$ aryl and 5-10 membered heteroaryl are optionally substituted with one or more R.

In another specific embodiment, $W_1$ is

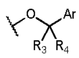,

wherein, $R_3$ and $R_4$ are H;
Ar is $C_{6-10}$ aryl or 5-10 membered heteroaryl, wherein the $C_{6-10}$ aryl or 5-10 membered heteroaryl is optionally substituted with one or more groups selected from $C_{1-6}$ alkyl or $-C(O)OC_{1-6}$ alkyl.

In another specific embodiment, $W_1$ is

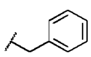, 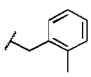, 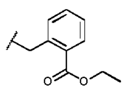 or 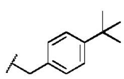.

In a specific embodiment, $W_1$ is

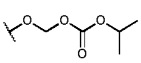, 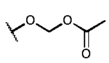, 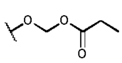, 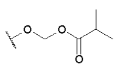, 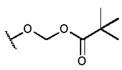,

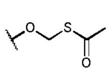, 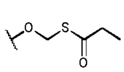, 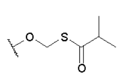, 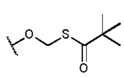, 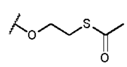, 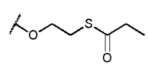, 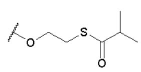, 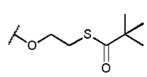,

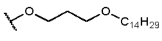, 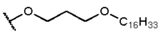, 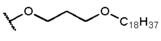, 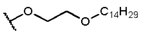, 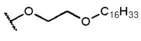, 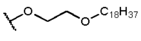, 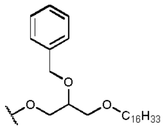,

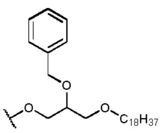, 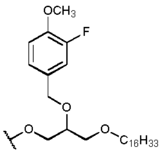, 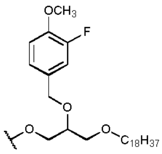, 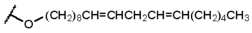, 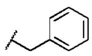, 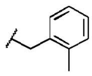, 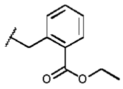

or

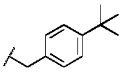.

In another specific embodiment, $W_1$ is

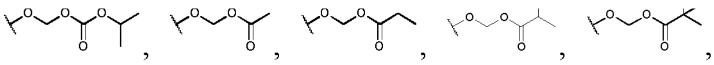

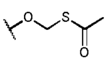, 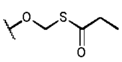, 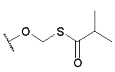, 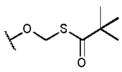, 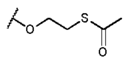, 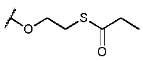, 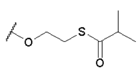, 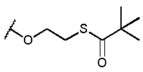,

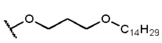, 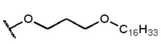, 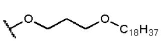, 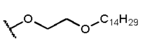, 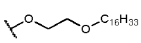, 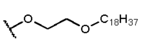, 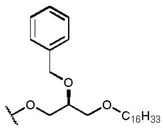,

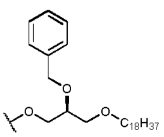, 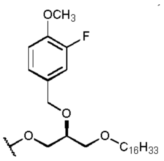, 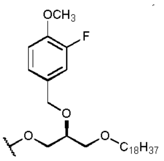, 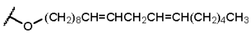, 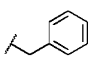, 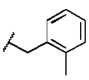, 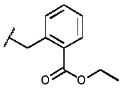

or

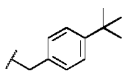.

In an embodiment, $W_2$ is independently formula (B):

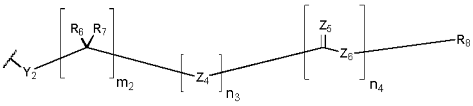 formula (B)

wherein,

$Y_2$ is O, NH or S;
$Z_4$ is O, NH or S;
$Z_2$ is O, NH or S;
$Z_6$ is a bond, O, NH or S;

$m_2$ is 0, 1, 2, 3, 4, 5 or 6;
$n_3$ is 0 or 1;
$n_4$ is 0 or 1;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R.

In an embodiment, $Y_2$ is O; in another embodiment, $Y_2$ is NH; and in another embodiment, $Y_2$ is S.

In a specific embodiment, $Y_2$ is O, NH or S; and in another specific embodiment, $Y_2$ is O or NH.

In an embodiment, $Z_4$ is O; in another embodiment, $Z_4$ is NH; and in another embodiment, $Z_4$ is S.

In a specific embodiment, $Z_4$ is O, NH or S; and in another specific embodiment, $Z_4$ is O or S.

In an embodiment, $Z_5$ is O; in another embodiment, $Z_5$ is NH; and in another embodiment, $Z_5$ is S.

In a specific embodiment, $Z_5$ is O, NH or S; and in another specific embodiment, $Z_5$ is O or S.

In an embodiment, $Z_6$ is a bond; in another embodiment, $Z_6$ is O; in another embodiment, $Z_6$ is NH; and in another embodiment, $Z_6$ is S.

In a specific embodiment, $Z_6$ is a bond, O, NH or S; and in another specific embodiment, $Z_6$ is a bond or O.

In an embodiment, $m_2$ is 0; in another embodiment, $m_2$ is 1; in another embodiment, $m_2$ is 2; in another embodiment, $m_2$ is 3; in another embodiment, $m_2$ is 4; in another embodiment, $m_2$ is 5; and in another embodiment, $m_2$ is 6.

In a specific embodiment, $m_2$ is 0, 1, 2, 3, 4, 5 or 6; in another specific embodiment, $m_2$ is 0, 1, 2, 3 or 4; in another specific embodiment, $m_2$ is 0, 1, 2 or 3; and in another specific embodiment, $m_2$ is 0, 1 or 3.

In an embodiment, $n_3$ is 0; and in another embodiment, $n_3$ is 1.

In an embodiment, $n_4$ is 0; and in another embodiment, $n_4$ is 1.

In an embodiment, each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R'; in another specific embodiment, each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R'; in another specific embodiment, each of $R_6$ and $R_7$ is independently H, D, $C_{1-6}$ alkyl, $C_{3-6}$ cycloalkyl or 3-7 membered heterocyclyl, or $R_6$and $R_7$ together with the C atom to which they are attached, form a $C_{3-6}$ cycloalkyl or 3-7 membered heterocyclyl, wherein the $C_{1-6}$ alkyl, $C_{3-6}$ cycloalkyl and 3-7 membered heterocyclyl are optionally substituted with one or more R'; in another specific embodiment, each of $R_6$ and $R_7$ is independently H, D, methyl, benzyl, $-CH_2C(O)OC_5H_{11}$,

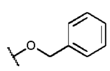 or 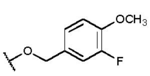 ;

or $R_6$ and $R_7$ together with the C atom to which they are attached, form cyclobutane.

In an embodiment, each $R_8$ is independently H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; in another specific embodiment, each $R_8$ is independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; in another specific embodiment, each $R_8$ independently is $C_{1-6}$ alkyl, $C_{1-6}$ haloalkyl, $C_{3-6}$ cycloalkyl, 3-7 membered heterocyclyl, $C_{2-6}$ alkenyl or $C_{2-6}$ alkynyl, wherein the $C_{1-6}$ alkyl, $C_{1-6}$ haloalkyl, $C_{3-6}$ cycloalkyl, 3-7 membered heterocyclyl, $C_{2-6}$ alkenyl and $C_{2-6}$ alkynyl are optionally substituted with one or more R; in another specific embodiment, each $R_8$ is independently $C_{1-6}$ alkyl, $C_{1-6}$ haloalkyl, $C_{3-6}$ cycloalkyl or 3-7 membered heterocyclyl, wherein the $C_{1-6}$ alkyl, $C_{1-6}$ haloalkyl, $C_{3-6}$ cycloalkyl and 3-7

membered heterocyclyl are optionally substituted with one or more R; in another specific embodiment, each $R_8$ is independently $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl or $C_{8-28}$ alkynyl is optionally substituted with one or more R; in another specific embodiment, each $R_8$ is independently $C_{8-28}$ alkyl or $C_{8-28}$ alkenyl, wherein the $C_{8-28}$ alkyl and $C_{8-28}$ alkenyl are optionally substituted with one or more R; in another specific embodiment, each $R_8$ is independently $C_{8-28}$ alkyl, wherein the $C_{8-28}$ alkyl is optionally substituted with one or more R; in another specific embodiment, each $R_8$ is independently $C_{8-28}$ alkenyl, wherein the $C_{8-28}$ alkenyl is optionally substituted with one or more R; in another specific embodiment, each $R_8$ is independently $C_{6-10}$ aryl or 5-10 membered heteroaryl, wherein the $C_{6-10}$ aryl or 5-10 membered heteroaryl is optionally substituted with one or more R; in another specific embodiment, each $R_8$ is independently $C_{6-10}$ aryl, wherein the $C_{6-10}$ aryl is optionally substituted with one or more R; in another specific embodiment, each $R_8$ is independently methyl, ethyl, isopropyl , tert-butyl,

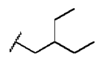,

n-pentyl,

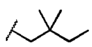, 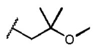,

cyclobutane, $-CH_2(CH_2)_{10}CH_3$, $-CH_2(CH_2)_{12}CH_3$, $-CH_2(CH_2)_{14}CH_3$, $-CH_2(CH_2)_{16}CH_3$, $-CH_2(CH_2)_7$ or $-CH_2(CH_2)_7CH{=}CHCH_2CH{=}CH(CH_2)_4CH_3$; in another specific embodiment, each $R_8$ is independently methyl, ethyl, isopropyl, tert-butyl,

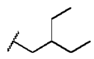,

n-pentyl,

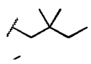;

in another specific embodiment, each $R_8$ is independently isopropyl, tert-butyl or

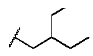;

and in another specific embodiment, each $R_8$ is independently isopropyl or

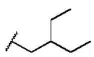.

In an embodiment, $W_2$ is independently:

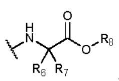, 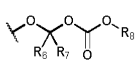, 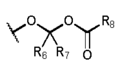, 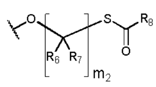, 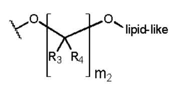, 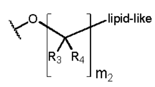, 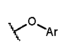 or 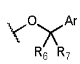;

wherein,

$m_2$ is 1, 2, 3, 4, 5 or 6;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;
Ar is $C_{6-10}$ aryl or 5-10 membered heteroaryl, wherein the $C_{6-10}$ aryl and 5-10 membered heteroaryl are optionally substituted with one or more R.

In a specific embodiment, $W_2$ is

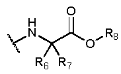,

wherein, $R_6$ and $R_7$ are independently H, D, $C_{1-6}$ alkyl, $C_{1-6}$ alkoxy, $C_{3-6}$ cycloalkyl or 3-7 membered heterocyclyl, or $R_6$and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';
$R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_2$ is

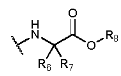,

wherein, $R_6$ is $C_{1-6}$ alkyl, and $R_7$ is H, or $R_6$ and $R_7$ together with the C atom to which they are attached, form a $C_{3-6}$ cycloalkyl, wherein the $C_{1-6}$ alkyl and $C_{3-6}$ cycloalkyl are optionally substituted with one or more groups selected from phenyl or -C(O)O$C_{1-6}$ alkyl;
$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl are optionally substituted with one or more $C_{1-6}$ alkoxy.

In another specific embodiment, $W_2$ is

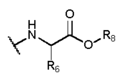,

wherein, $R_6$ is $C_{1-6}$ alkyl, wherein the $C_{1-6}$ alkyl is optionally substituted with one or more groups selected from phenyl or -C(O)O$C_{1-6}$ alkyl;
$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl are optionally substituted with one or more $C_{1-6}$ alkoxy.

In another specific embodiment, $W_2$ is

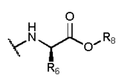,

wherein, $R_6$ is $C_{1-6}$ alkyl, wherein the $C_{1-6}$ alkyl is optionally substituted with one or more groups selected from phenyl or -C(O)O$C_{1-6}$ alkyl;
$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl are optionally substituted with one or more $C_{1-6}$ alkoxy.

In another specific embodiment, $W_2$ is

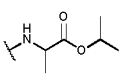, 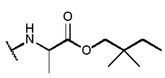, 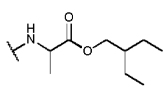, 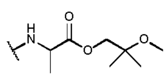,

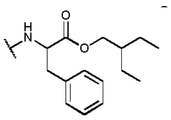, 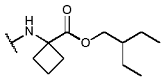, 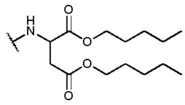, 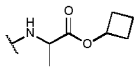, 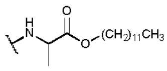

or

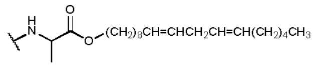;

In another specific embodiment, $W_2$ is

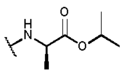, 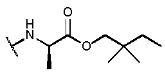, 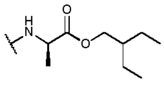, 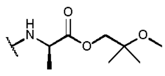,

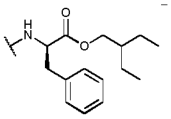, 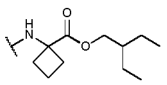, 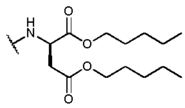, 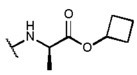, 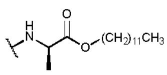

or

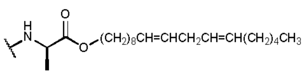.

In another specific embodiment, $W_2$ is

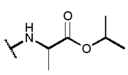, 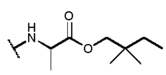, 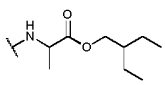, 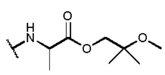,

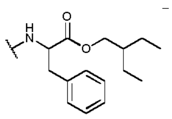, 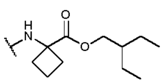, 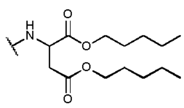, 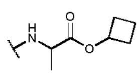, 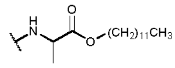,

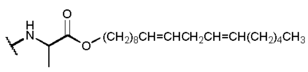, 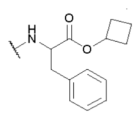, 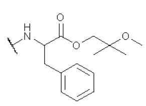 or 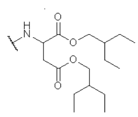.

In another specific embodiment, $W_2$ is

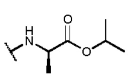, 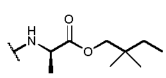, 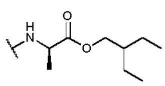, 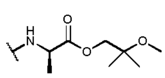,

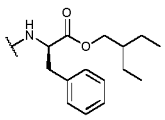, 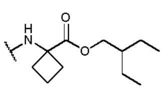, 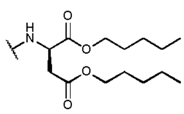, 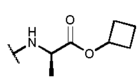, 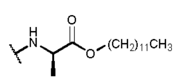,

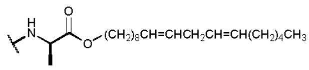, 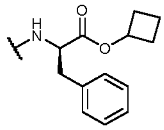, 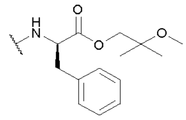 or 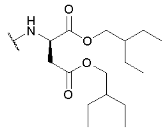.

In a specific embodiment, $W_2$ is

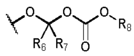,

wherein $R_6$ and $R_7$ are independently H, D, $C_{1-6}$ alkyl, $C_{1-6}$ alkoxy, $C_{3-6}$ cycloalkyl or 3-7 membered heterocyclyl, or $R_6$and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

$R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_2$ is

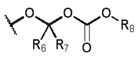,

wherein $R_6$ and $R_7$ is H;

$R_8$ is independently $C_{1-28}$ alkyl, $C_{3-6}$ cycloalkyl, 3-7 membered heterocyclyl or $C_{2-28}$ alkenyl.

In another specific embodiment, $W_2$ is

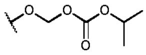.

In a specific embodiment, $W_2$ is

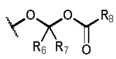,

wherein, $R_6$ and $R_7$ are independently H, D, $C_{1-6}$ alkyl, $C_{1-6}$ alkoxy, $C_{3-6}$ cycloalkyl or 3-7 membered heterocyclyl, or $R_6$and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

$R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_2$ is

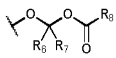,

wherein, $R_6$ and $R_7$ are H;

$R_8$ is independently $C_{1-28}$ alkyl, $C_{3-6}$ cycloalkyl, 3-7 membered heterocyclyl or $C_{2-28}$ alkenyl.

In another specific embodiment, $W_2$ is

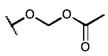, 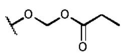, 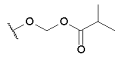 or 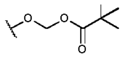.

In a specific embodiment, $W_2$ is

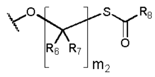,

wherein, $m_2$ is 1, 2, 3 or 4;
each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';
$R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R.

In another specific embodiment, $W_2$ is

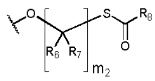,

wherein, $R_6$ and $R_7$ are H;
$m_2$ is 1 or 2;
$R_8$ is independently $C_{1-28}$ alkyl, $C_{3-6}$ cycloalkyl, 3-7 membered heterocyclyl or $C_{2-28}$ alkenyl.

In another specific embodiment, $W_2$ is

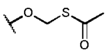, 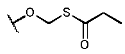, 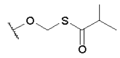, 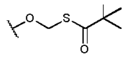, 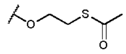,

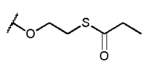, 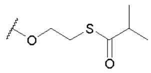 or 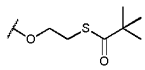.

In a specific embodiment, $W_2$

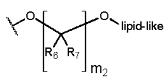,

wherein, $m_2$ is 1, 2, 3 or 4;
each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';
lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R.

In a specific embodiment, $W_2$ is

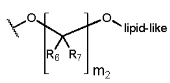,

wherein, $m_2$ is 2, 3 or 4;

$R_6$ and $R_7$ are independently H or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;

lipid-like is $C_{8-28}$ alkyl or $C_{8-28}$ alkenyl.

In another specific embodiment, $W_2$ is

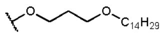, 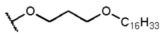, 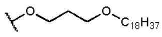, 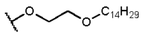,

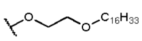, 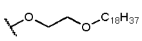, 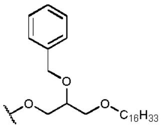, 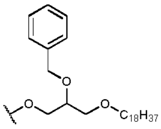, 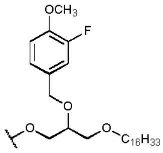 or 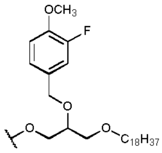;

In another specific embodiment, $W_2$ is

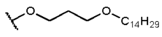, 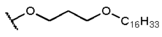, 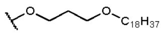, 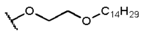,

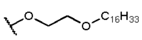, 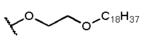, 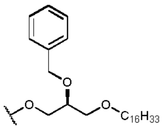, 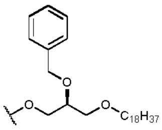, 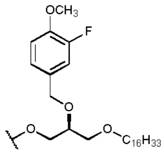 or 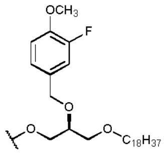.

In a specific embodiment, $W_2$ is

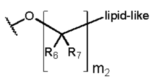,

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R.

In another specific embodiment, $W_2$ is

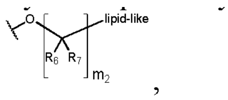,

wherein, $R_6$ and $R_7$ are H;

$m_2$ is 2, 3 or 4;

lipid-like is $C_{8-28}$ alkenyl.

In another specific embodiment, $W_2$ is

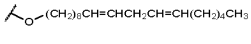.

In a specific embodiment, $W_2$ is

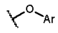,

wherein, Ar is $C_{6-10}$ aryl or 5-10 membered heteroaryl, wherein the $C_{6-10}$ aryl and 5-10 membered heteroaryl are optionally substituted with one or more R.

In another specific embodiment, $W_2$ is

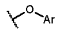,

wherein, Ar is $C_{6-10}$ aryl or 5-10 membered heteroaryl, wherein the $C_{6-10}$ aryl or 5-10 membered heteroaryl is optionally substituted with one or more $C_{1-6}$ alkyl.

In another specific embodiment, $W_2$ is

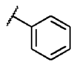, 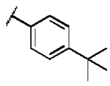 or 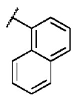.

In a specific embodiment, $W_2$ is

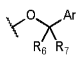,

wherein, $R_6$ and $R_7$ are independently H, D, $C_{1-6}$ alkyl, $C_{1-6}$ alkoxy, $C_{3-6}$ cycloalkyl or 3-7 membered heterocyclyl, or $R_6$and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

Ar is $C_{6-10}$ aryl or 5-10 membered heteroaryl, wherein the $C_{6-10}$ aryl and 5-10 membered heteroaryl are optionally substituted with one or more R.

In another specific embodiment, $W_2$ is

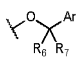,

wherein, $R_6$ and $R_7$ are H;

Ar is $C_{6-10}$ aryl or 5-10 membered heteroaryl, wherein the $C_{6-10}$ aryl or 5-10 membered heteroaryl is optionally substituted with one or more groups selected from $C_{1-6}$ alkyl or $-C(O)OC_{1-6}$ alkyl.

In another specific embodiment, $W_2$ is

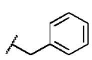, 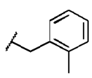, 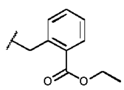 or 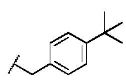.

In a specific embodiment, $W_2$ is

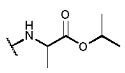, 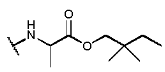, 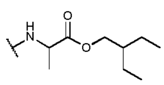, 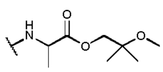,

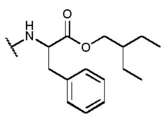, 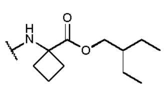, 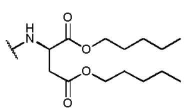, 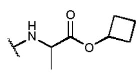, 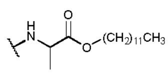,

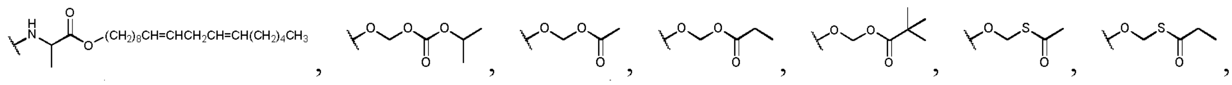
, , , , , , ,

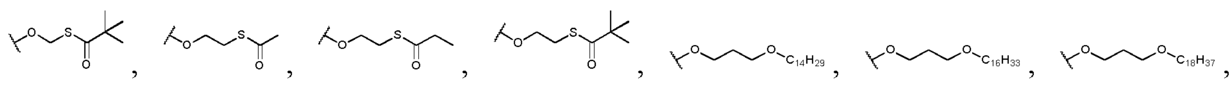
, , , , , , ,

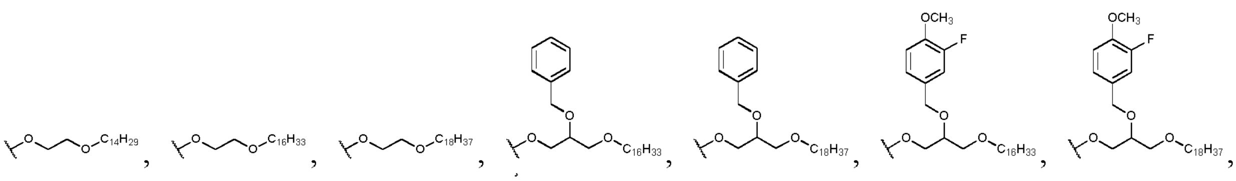
, , , , , , ,

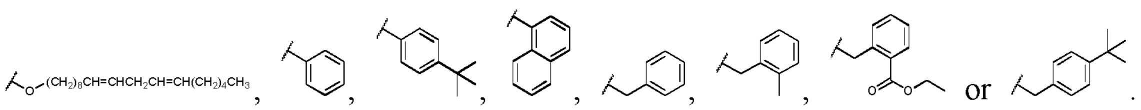
, , , , , , or .

In another specific embodiment, $W_2$ is

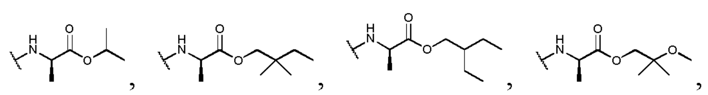
, , , ,

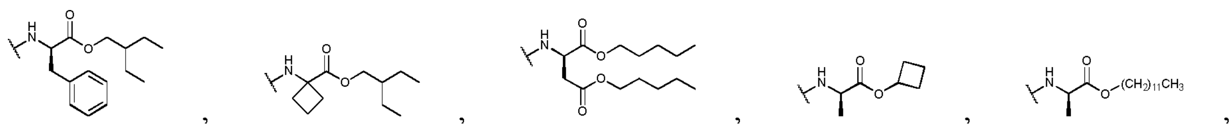
, , , , ,

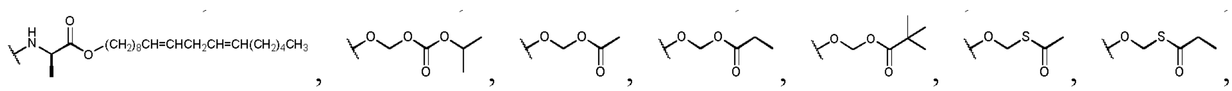
, , , , , , ,

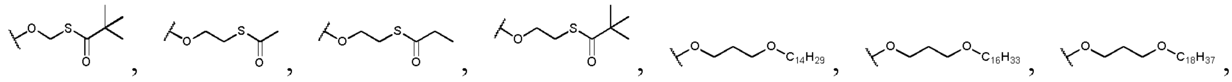
, , , , , , ,

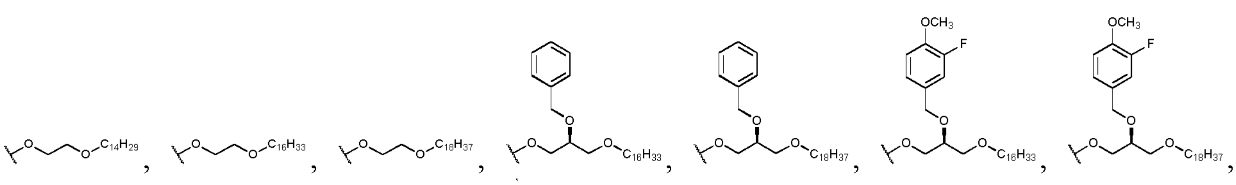
, , , , , , ,

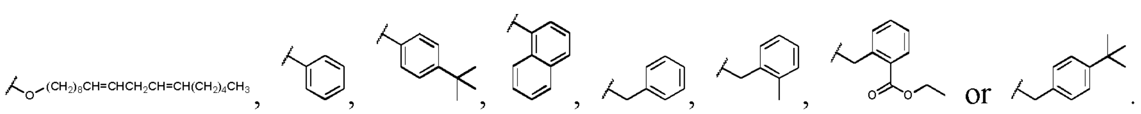
, , , , , , or .

In another specific embodiment, $W_2$ is

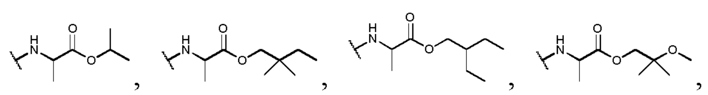
, , , ,

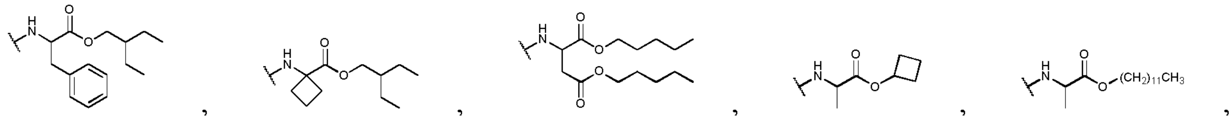

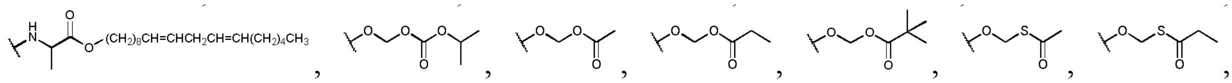

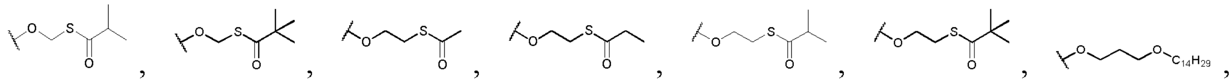

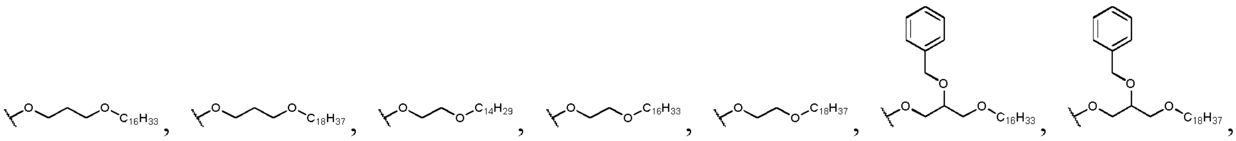

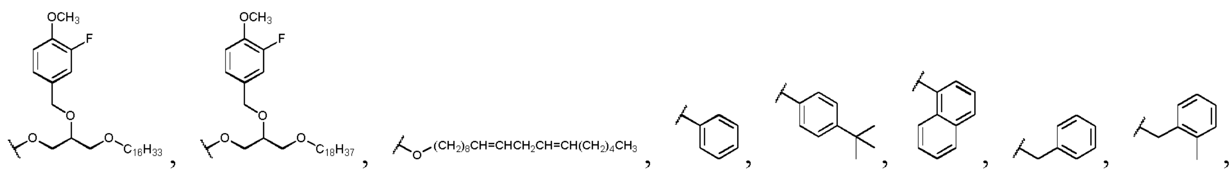

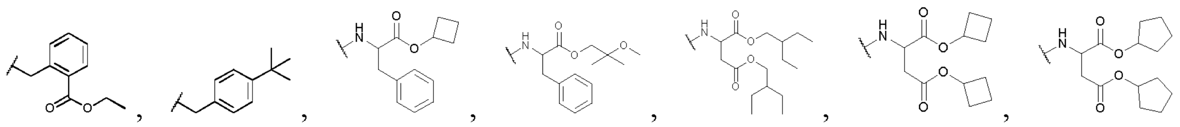

or

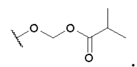

In another specific embodiment, $W_2$ is

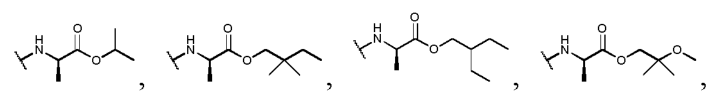

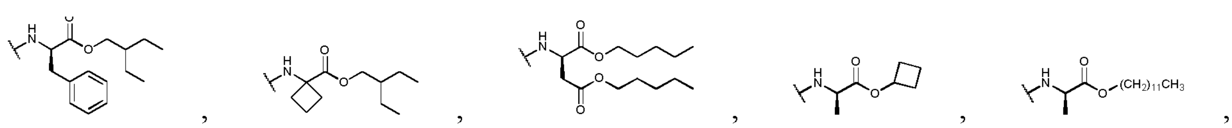

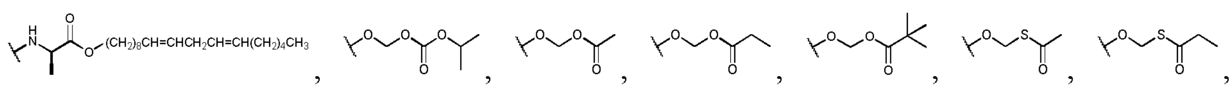

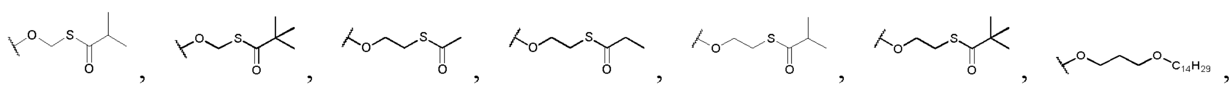

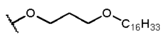, 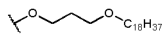, 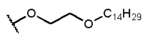, 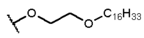, 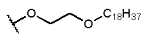, 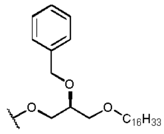, 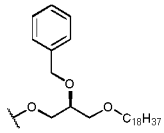,

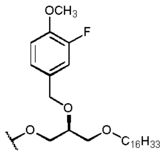, 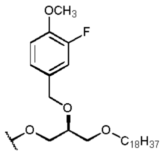, 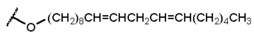, 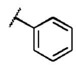, 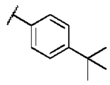, 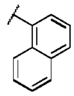, 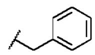, 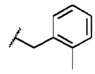,

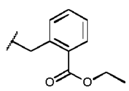, 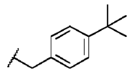, 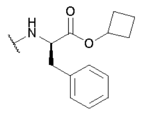, 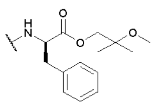, 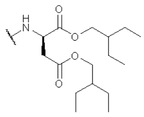, 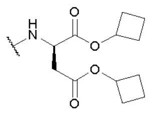, 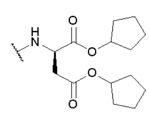

or

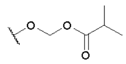.

In an embodiment, any one of $W_1$ and $W_2$ together with the 3'-position C atom of glycosyl form $-Y_3-$, wherein $Y_3$ is O, NH or S; in a specific embodiment, any one of $W_1$ and $W_2$ together with the 3'-position C atom of glycosyl form -O-; in another specific embodiment, any one of $W_1$ and $W_2$ together with the 3'-position C atom of glycosyl form -NH-; and in another specific embodiment, any one of $W_1$ and $W_2$ together with the 3'-position C atom of glycosyl form -S-. In another embodiment, $W_1$ and $W_2$ together with the P atom to which they are attached, form $-Y_4(C(R_3R_4)_p)Y_4-$, wherein $Y_4$ is O, NH or S, p is 2, 3, 4 or 5; in a specific embodiment, $W_1$ and $W_2$ together with the P atom to which they are attached, form $-O(C(R_3R_4)_p)O-$, wherein p is 2, 3, 4 or 5; and in a specific embodiment, $W_1$ and $W_2$ together with the P atom to which they are attached, form $-O(C(R_3R_4)_3)O-$.

R

In an embodiment, R is D; in another embodiment, R is halogen; in another embodiment, R is $-R_d$; in another embodiment, R is $-C(O)R_d$; in another embodiment, R is $-C(O)OR_d$; in another embodiment, R is $-C(O)NR_eR_f$; in another embodiment, R is $-NR_eR_f$; in another embodiment, R is $-NR_dC(O)R_e$; in another embodiment, R is $-NR_dC(O)OR_e$; in another embodiment, R is $-NR_dC(O)NR_eR_f$; in another embodiment, R is $-OR_d$; in another embodiment, R is $-OC(O)R_d$; in another embodiment, R is $-OC(O)NR_eR_f$; in another embodiment, R is $-NR_dS(O)_2R_e$; in another embodiment, R is $-S(O)_2NR_d$; in another embodiment, R is $-S(O)R_d$; in another embodiment, R is $-S(O)_2R_d$; in another embodiment, or two R on the same atom or two adjacent atoms, together with the atoms to which they are attached, form a $C_{3-6}$ cycloalkyl; in another embodiment, two R on the same atom or two adjacent atoms, together with the atoms to which they are attached, form a 3-7 membered heterocyclyl; in another embodiment, two R on the same atom or two adjacent atoms, together with the atoms to which they are attached, form a $C_{6-10}$ aryl; in another embodiment, two R on the same atom or two adjacent atoms, together with the atoms to which they are attached, form 5-10 membered heteroaryl; in another embodiment, the above $C_{3-6}$ cycloalkyl, 3-7 membered heterocyclyl, $C_{6-10}$ aryl and 5-10 membered heteroaryl are optionally substituted with one or more R".

R'

In an embodiment, R' is D; in another embodiment, R' is halogen; in another embodiment, R' is $-R_d$; in another embodiment, R' is $-C(O)R_d$; in another embodiment, R' is $-C(O)OR_d$; in another embodiment, R' is $-C(O)NR_eR_f$; in another embodiment, R' is $-NR_eR_f$; in another embodiment, R' is $-NR_dC(O)R_e$; in another embodiment, R' is $-NR_dC(O)OR_e$; in another embodiment, R' is $-NR_dC(O)NR_eR_f$; in another embodiment, R' is $-OR_d$; in another embodiment, R' is $-OC(O)R_d$; in another embodiment, R' is $-OC(O)NR_eR_f$; in another embodiment, R' is $-NR_dS(O)_2R_e$; in another embodiment, R' is $-S(O)_2NR_d$; in another embodiment, R' is $-S(O)R_d$; in another embodiment, R' is $-S(O)_2R_d$; in another embodiment, two R on the same atom or two adjacent atoms, together with the atoms to which they are attached, form a $C_{3-6}$ cycloalkyl; in another embodiment, two R' on the same atom or two adjacent atoms, together with the atoms to which they are attached,

form a 3-7 membered heterocyclyl; in another embodiment, two R' on the same atom or two adjacent atoms, together with the atoms to which they are attached, form a $C_{6-10}$ aryl; in another embodiment, two R' on the same atom or two adjacent atoms, together with the atoms to which they are attached, form 5-10 membered heteroaryl; in another embodiment, the above $C_{3-6}$ cycloalkyl, 3-7 membered heterocyclyl, $C_{6-10}$ aryl and 5-10 membered heteroaryl are optionally substituted with one or more R".

$R_d$, $R_e$ and $R_f$

In an embodiment, $R_d/R_e/R_f$ is H; in another embodiment, $R_d/R_e/R_f$ is D; in another embodiment, $R_d/R_e/R_f$ is $C_{1-6}$ alkyl; in another embodiment, $R_d/R_e/R_f$ is $C_{1-6}$ haloalkyl; in another embodiment, $R_d/R_e/R_f$ is $C_{3-6}$ cycloalkyl; in another embodiment, $R_d/R_e/R_f$ is 3-7 membered heterocyclyl; in another embodiment, $R_d/R_e/R_f$ is $C_{2-6}$ alkenyl; in another embodiment, $R_d/R_e/R_f$ is $C_{2-6}$ alkynyl; in another embodiment, $R_d/R_e/R_f$ is $C_{6-10}$ aryl; in another embodiment, $R_d/R_e/R_f$ is 5-10 membered heteroaryl; in another embodiment, $R_e$ and $R_f$ together with the N atom to which they are attached, form a 3-7 membered heterocyclyl; in another embodiment, $R_e$ and $R_f$ together with the N atom to which they are attached, form 5-10 membered heteroaryl; in another embodiment, the above $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R".

R"

In an embodiment, R" is D; in another embodiment, R" is halogen; in another embodiment, R" is $-R_g$; in another embodiment, R" is $-C(O)R_g$; in another embodiment, R" is $-C(O)OR_g$; in another embodiment, R" is $-C(O)NR_hR_j$; in another embodiment, R" is $-NR_hR_j$; in another embodiment, R" is $-NR_gC(O)R_h$; in another embodiment, R" is $-NR_gC(O)OR_h$; in another embodiment, R" is $-NR_gC(O)NR_hR_j$; in another embodiment, R" is $-OR_g$; in another embodiment, R" is $-OC(O)R_g$; in another embodiment, R" is $-OC(O)NR_hR_j$; in another embodiment, R" is $-NR_gS(O)_2R_h$; in another embodiment, R" is $-S(O)_2NR_g$; in another embodiment, R" is $-S(O)R_g$; in another embodiment, R" is $-S(O)_2R_g$; in another embodiment, two R" on the same atom or two adjacent atoms, together with the atoms to which they are attached, form a $C_{3-6}$ cycloalkyl; in another embodiment, two R" on the same atom or two adjacent atoms, together with the atoms to which they are attached, form a 3-7 membered heterocyclyl; in another embodiment, two R" on the same atom or two adjacent atoms, together with the atoms to which they are attached, form a $C_{6-10}$ aryl; in another embodiment, two R" on the same atom or two adjacent atoms, together with the atoms to which they are attached, form 5-10 membered heteroaryl; in another embodiment, the above $C_{3-6}$ cycloalkyl, 3-7 membered heterocyclyl, $C_{6-10}$ aryl and 5-10 membered heteroaryl are optionally substituted with one or more D, up to full deuteration.

$R_g$, $R_h$ and $R_j$

In an embodiment, $R_g/R_h/R_j$ is H; in another embodiment, $R_g/R_h/R_j$ is D; in another embodiment, $R_g/R_h/R_j$ is $C_{1-6}$ alkyl; in another embodiment, $R_g/R_h/R_j$ is $C_{1-6}$ haloalkyl; in another embodiment, $R_g/R_h/R_j$ is $C_{3-6}$ cycloalkyl; in another embodiment, $R_g/R_h/R_j$ is 3-7 membered heterocyclyl; in another embodiment, $R_g/R_h/R_j$ is $C_{2-6}$ alkenyl; in another embodiment, $R_g/R_h/R_j$ is $C_{2-6}$ alkynyl; in another embodiment, $R_g/R_h/R_j$ is $C_{6-10}$ aryl; in another embodiment, $R_g/R_h/R_j$ is 5-10 membered heteroaryl; in another embodiment, $R_h$ and $R_j$ together with the N atom to which they are attached, form a 3-7 membered heterocyclyl; in another embodiment, $R_h$ and $R_j$ together with the N atom to which they are attached, form 5-10 membered heteroaryl; in another embodiment, the above $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D, up to full deuteration.

Any technical solution in any of the above specific embodiments or any combination thereof may be combined with any technical solution in other specific embodiments or any combination thereof. For example, any technical solution of $R_X$, $R_{X1}$, $R_{X2}$, $R_{X3}$, $X_1$, $X_2$, $R_1$, $R_2$, L, $W_1$, $W_2$, R, R' and R" or any combination thereof may be combined. The present invention is intended to encompass combinations of all these technical solutions, which are not exhaustively enumerated herein due to space limitations.

In a more specific embodiment, the present invention relates to the aforementioned compound of formula (I):

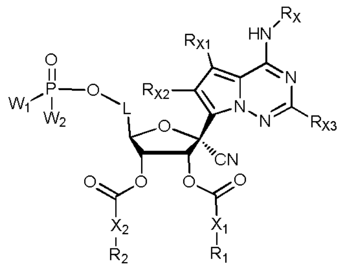

(I)

wherein,

$R_X$ is H or OH;

$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_d$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$W_1$ is formula (A):

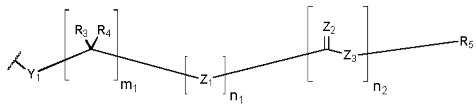

formula (A)

wherein,

$Y_1$ is O or S;

$Z_1$ is O, NH or S;

$Z_2$ is O, NH or S;

$Z_3$ is a bond, O, NH or S;

$m_1$ is 1, 2, 3, 4, 5 or 6;

$n_1$ is 0 or 1;

$n_2$ is 0 or 1;

each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

$W_2$ is formula (B):

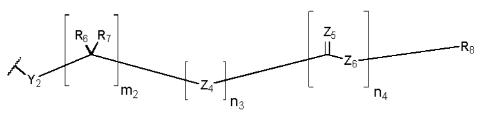

formula (B)

wherein,

$Y_2$ is O, NH or S;

$Z_4$ is O, NH or S;
$Z_5$ is O, NH or S;
$Z_6$ is a bond, O, NH or S;
$m_2$ is 0, 1, 2, 3, 4, 5 or 6;
$n_3$ is 0 or 1;
$n_4$ is 0 or 1;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
or, any one of $W_1$ or $W_2$ together with the 3'-position C atom of glycosyl form $-Y_3-$, wherein $Y_3$ is O, NH or S;
or, $W_1$ and $W_2$ together with the P atom to which they are attached, form $-Y_4(C(R_3R_4)_p)Y_4-$, wherein $Y_4$ is O, NH or S, and p is 2, 3, 4 or 5;
each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;
each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";
each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;
wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;
or a tautomer, stereoisomer, prodrug, crystalline form, pharmaceutically acceptable salt, hydrate or solvate thereof.

In a more specific embodiment, the present invention relates to the aforementioned compound of formula (Ia) or formula (Ib):

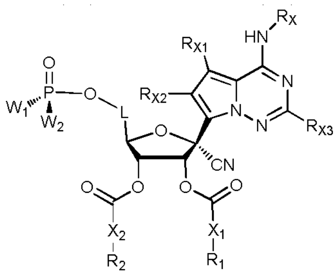 formula (Ia), or 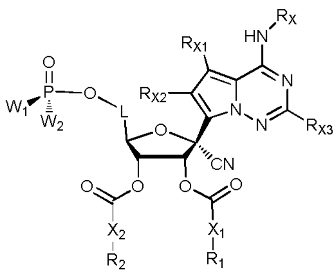 formula (Ib)

or a tautomer, stereoisomer, prodrug, crystalline form, pharmaceutically acceptable salt, hydrate or solvate thereof.

In a more specific embodiment, the present invention relates to the aforementioned compound, or a tautomer, stereoisomer, prodrug, crystalline form, pharmaceutically acceptable salt, hydrate or solvate thereof, wherein $W_1$ is:

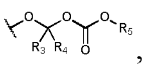, 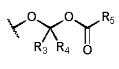, 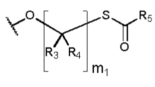, 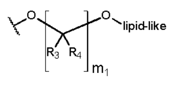, 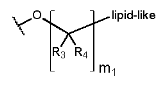 or 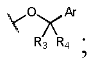;

wherein, lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R;

$R_3$, $R_4$, $R_5$, $m_1$ and R are as described in claim 1.

In a more specific embodiment, the present invention relates to the compound of formulas (II), (III) or (IV):

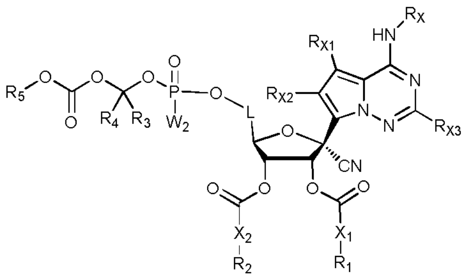 formula (II), 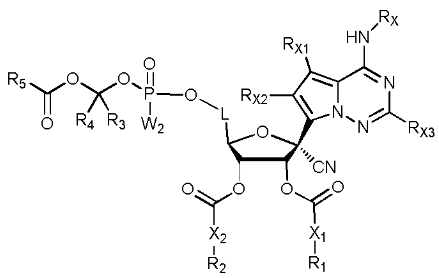 formula (III), or 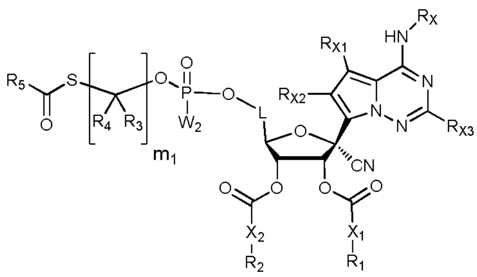 formula (IV)

wherein,

$R_X$ is H or OH;

$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_d$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_d$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$m_1$ is 1, 2, 3, 4, 5 or 6;

$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

$W_2$ is formula (B):

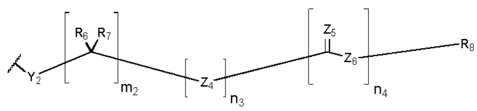
formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, $W_2$ together with the 3'-position C atom in the glycosyl form a $-Y_3-$, wherien $Y_3$ is O, NH or S;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_g$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7

membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

or a tautomer, stereoisomer, prodrug, crystalline form, pharmaceutically acceptable salt, hydrate or solvate thereof.

In a more specific embodiment, the present invention relates to the compound of formula (IIa), formula (IIb), formula (IIIa), formula (IIIb), formula (IVa) or formula (IVb):

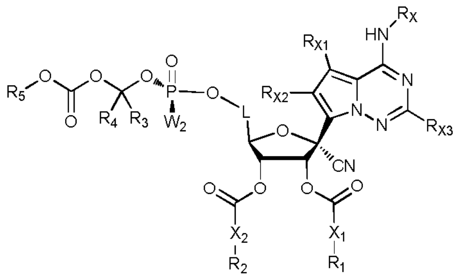

formula (IIa),

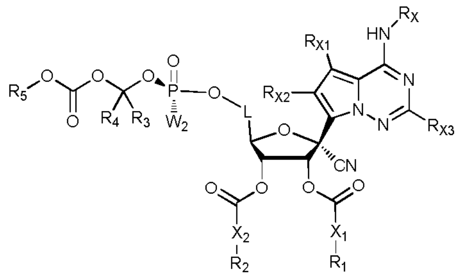

formula (IIb),

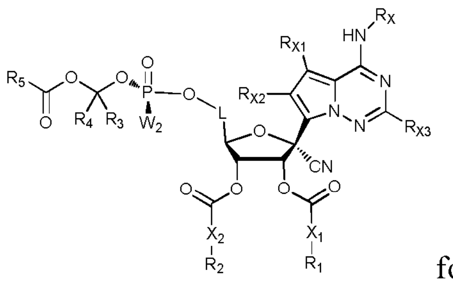

formula (IIIa),

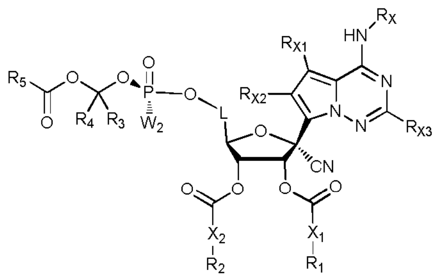

formula (IIIb)

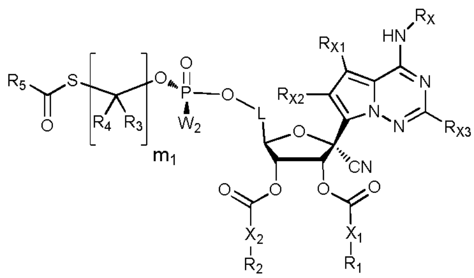

formula (IVa),

or

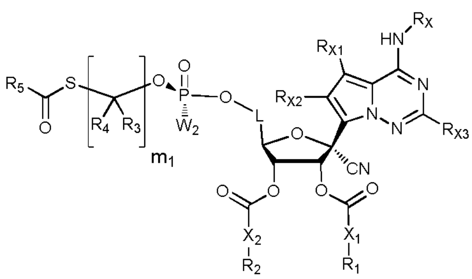

formula (IVb)

or a tautomer, stereoisomer, prodrug, crystalline form, pharmaceutically acceptable salt, hydrate or solvate thereof.

In a more specific embodiment, the present invention relates to the aforementioned compound, or a tautomer,

stereoisomer, prodrug, crystalline form, pharmaceutically acceptable salt, hydrate or solvate thereof, wherein:

$R_X$ is H or OH;

$R_{X1}$, $R_{X2}$ and $R_{X3}$ are independently H, D, halogen or $C_{1-6}$ alkyl, wherein the $C_{1-6}$ alkyl is optionally substituted with one or more deuterium, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$m_1$ is 1, 2 or 3;

$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{3-6}$ cycloalkyl and 3-7 membered heterocyclyl are optionally substituted with one or more R';

$R_5$ is $C_{1-28}$ alkyl, $C_{1-28}$ haloalkyl, $C_{3-6}$ cycloalkyl, 3-7 membered heterocyclyl or $C_{2-28}$ alkenyl, wherein the $C_{1-28}$ alkyl, $C_{1-28}$ haloalkyl, $C_{3-6}$ cycloalkyl, 3-7 membered heterocyclyl and $C_{2-28}$ alkenyl are optionally substituted with one or more R;

$W_2$ is:

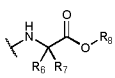 , 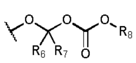 , 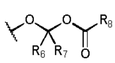 , 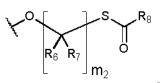 , 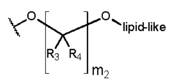 , 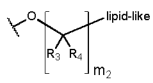 , 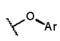 or 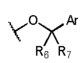 ;

wherein,

$m_2$ is 1, 2, 3, 4, 5 or 6;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to

full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration.

In a more specific embodiment, the present invention relates to the aforementioned compound, or a tautomer, stereoisomer, prodrug, crystalline form, pharmaceutically acceptable salt, hydrate or solvate thereof, wherein:

$W_2$ is

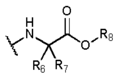, 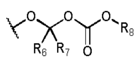, 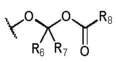, 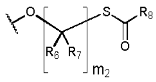, 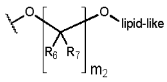, 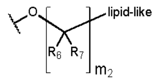, 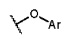

or

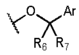;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

each of $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

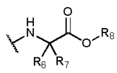,

wherein, $R_6$ is $C_{1-6}$ alkyl, and $R_7$ is H, or $R_6$ and $R_7$ together with the C atom to which they are attached, form a $C_{3-6}$ cycloalkyl, wherein the $C_{1-6}$ alkyl and $C_{3-6}$ cycloalkyl are optionally substituted with one or more groups selected from phenyl or -C(O)O$C_{1-6}$ alkyl;

$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl is optionally substituted with one or more $C_{1-6}$ alkoxy;

preferably, $W_2$ is

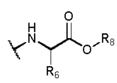;

preferably, $W_2$ is

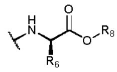;

preferably, $W_2$ is

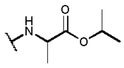, 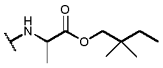, 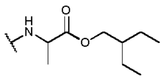, 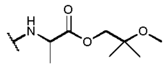, 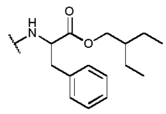,

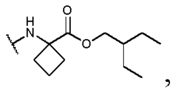, 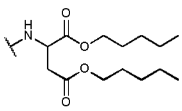, 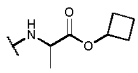, 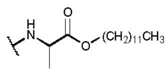 or 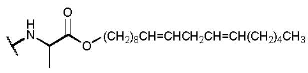;

preferably, $W_2$ is

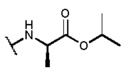, 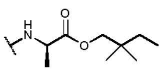, 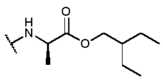, 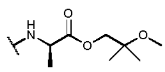, 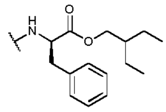,

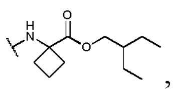, 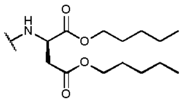, 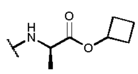, 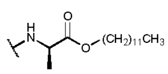 or 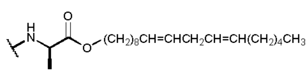;

preferably, $W_2$ is

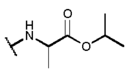, 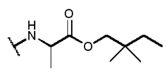, 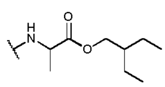, 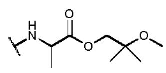, 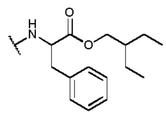,

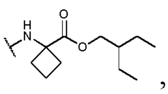, 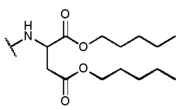, 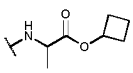, 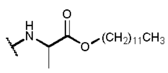, 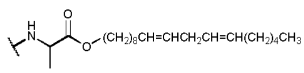, 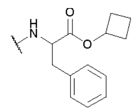,

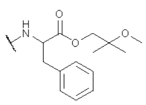 or 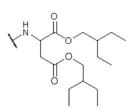;

preferably, $W_2$ is

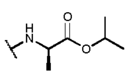, 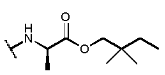, 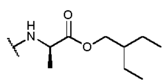, 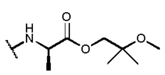, 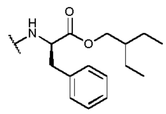,

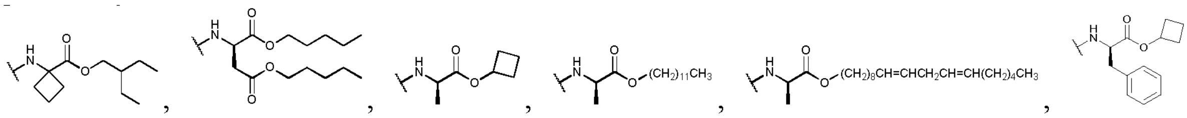,

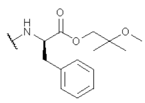 or 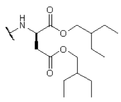.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

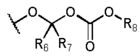,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

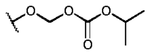.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

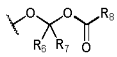,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$

为 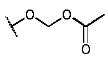, 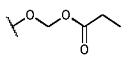 or 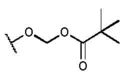;

preferably, $W_2$

为 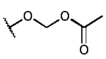, 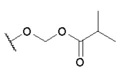 or 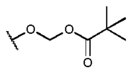.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

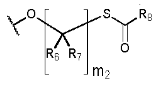,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

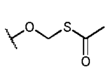, 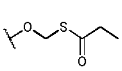, 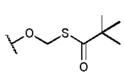, 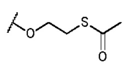, 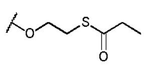 or 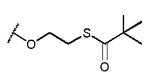.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

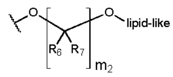,

wherein, $m_2$ is 2, 3 or 4;
$R_6$ and $R_7$ are independently H or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;
lipid-like is $C_{8-28}$ alkyl or $C_{8-28}$ alkenyl;
preferably, $W_2$ is

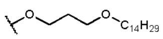, 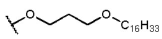, 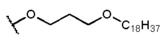, 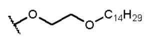, 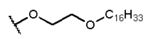,

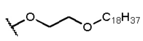, 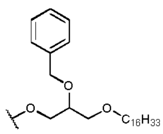, 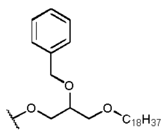, 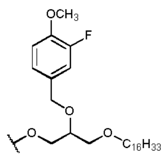 or 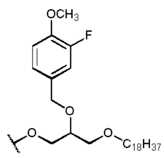;

preferably, $W_2$ is

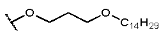, 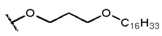, 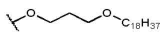, 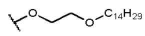, 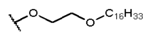,

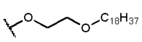, 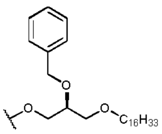, 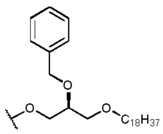, 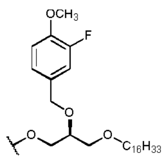 or 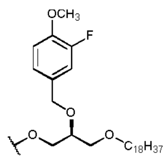.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

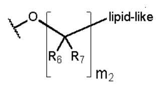,

wherein, $m_2$ is 2, 3 or 4;
$R_6$ and $R_7$ are H;
lipid-like is $C_{8-28}$ alkenyl;
preferably, $W_2$ is

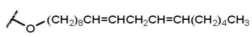.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

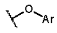,

wherein, Ar is $C_{6-10}$ aryl or 5-10 membered heteroaryl, wherein the $C_{6-10}$ aryl or 5-10 membered heteroaryl is optionally substituted with one or more $C_{1-6}$ alkyl;

preferably, $W_2$ is

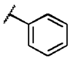, 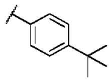 or 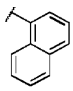.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

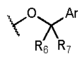,

wherein, $R_6$ and $R_7$ are H;
Ar is $C_{6-10}$ aryl or 5-10 membered heteroaryl, wherein the $C_{6-10}$ aryl or 5-10 membered heteroaryl is optionally substituted with one or more groups selected from $C_{1-6}$ alkyl or $-C(O)OC_{1-6}$ alkyl;
preferably, $W_2$ is

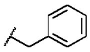, 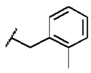, 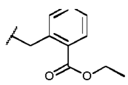 or 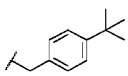.

In a more specific embodiment, the present invention relates to the aforementioned compound, or a tautomer, stereoisomer, prodrug, crystalline form, pharmaceutically acceptable salt, hydrate or solvate thereof, wherein, $W_2$ is

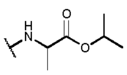, 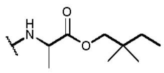, 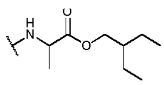, 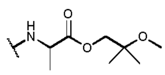,

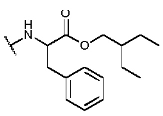, 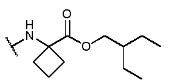, 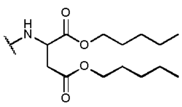, 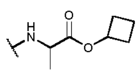, 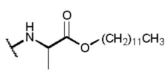,

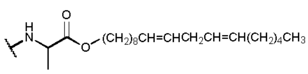, 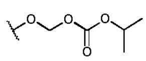, 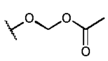, 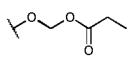, 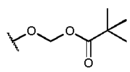, 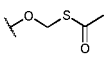, 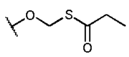,

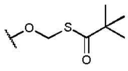, 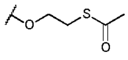, 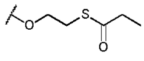, 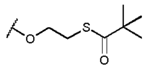, 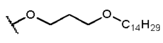, 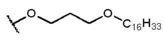, 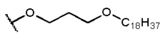,

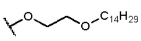, 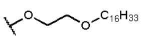, 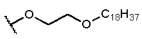, 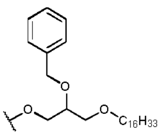, 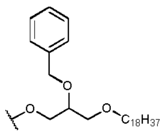, 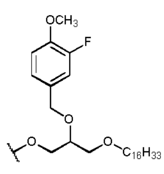, 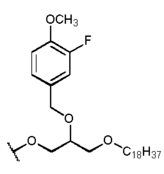,

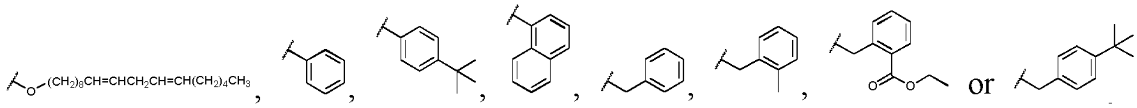

, , , , , , or ;

preferably, $W_2$ is

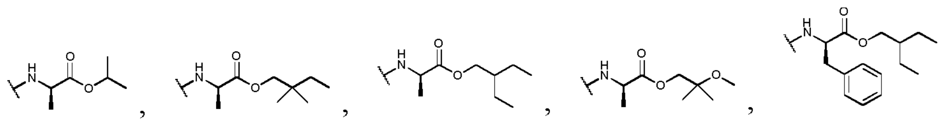

, , , , ,

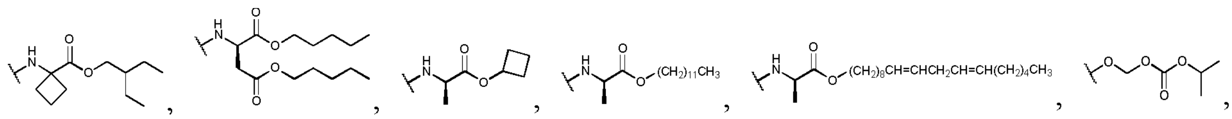

, , , , , ,

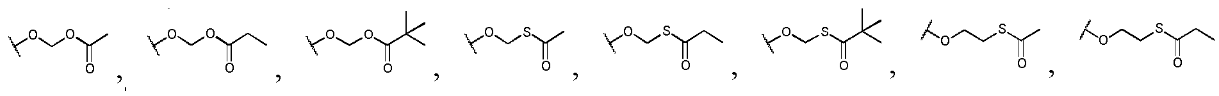

, , , , , , , ,

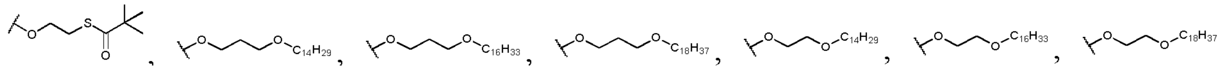

, , , , , , ,

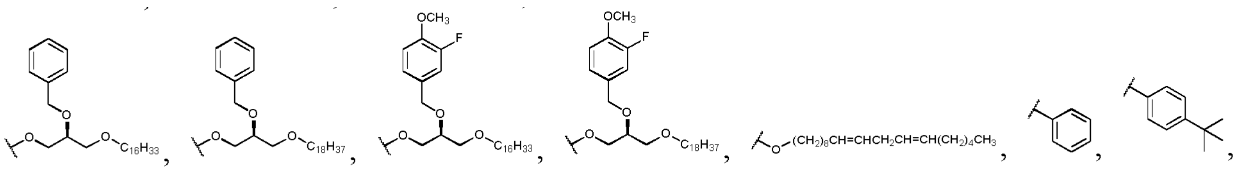

, , , , , , ,

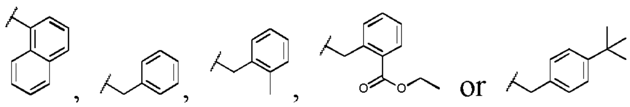

, , , or ;

preferably, $W_2$ is

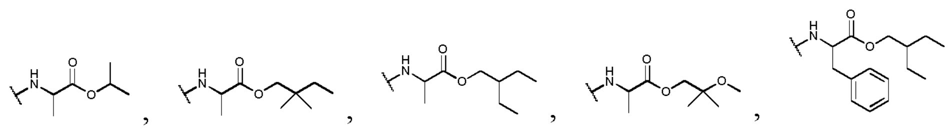

, , , , ,

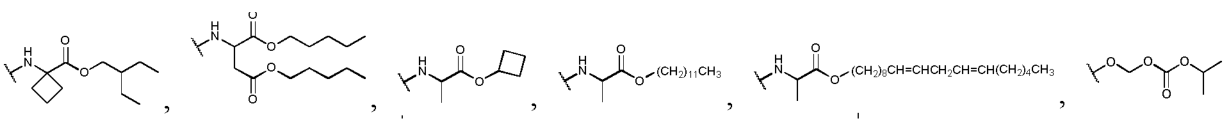

, , , , , ,

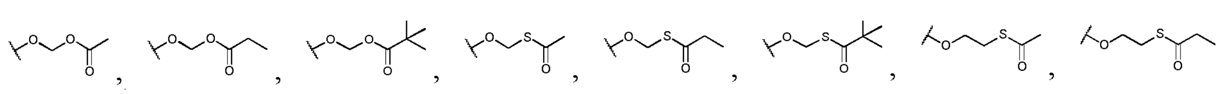

, , , , , , , ,

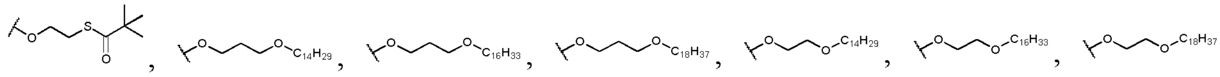

, , , , , , ,

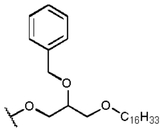, 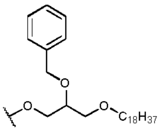, 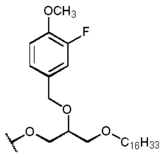, 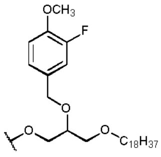, 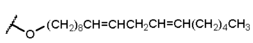, 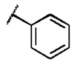, 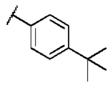,

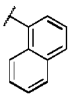, 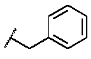, 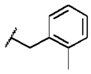, 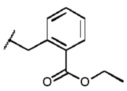, 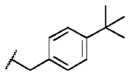, 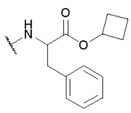, 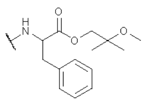, 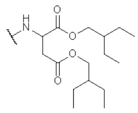

or

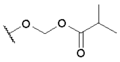;

preferably, $W_2$ is

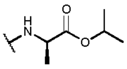, 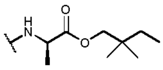, 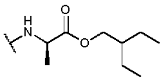, 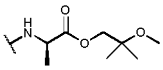, 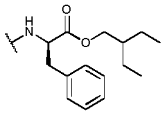,

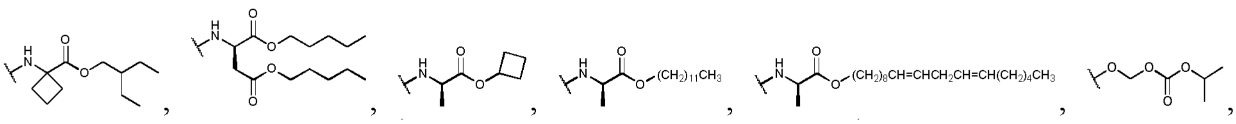

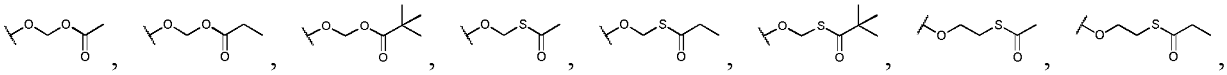

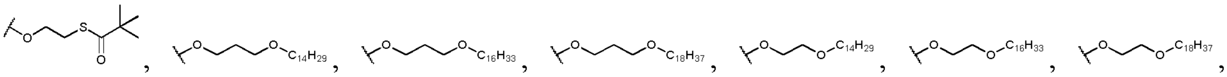

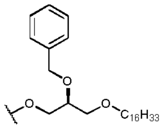, 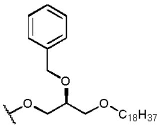, 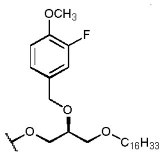, 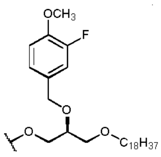, 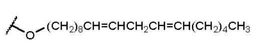, 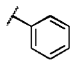, 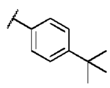,

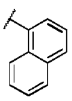, 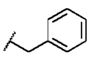, 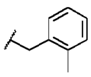, 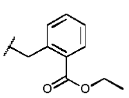, 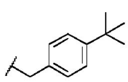, 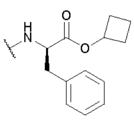, 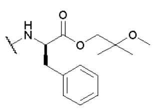, 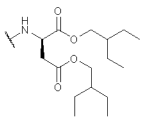

or

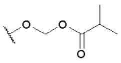.

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;

$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$m_1$ is 1, 2 or 3;
$R_3$ and $R_4$ are independently H, D or $C_{1-6}$ alkyl;
$R_5$ is $C_{1-28}$ alkyl, $C_{3-6}$ cycloalkyl, 3-7 membered heterocyclyl or $C_{2-28}$ alkenyl.

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$m_1$ is 1, 2 or 3;
$R_3$ and $R_4$ are independently H or D;
$R_5$ is $C_{1-6}$ alkyl.

In a more specific embodiment, the present invention relates to the aforementioned compound, or a tautomer, stereoisomer, prodrug, crystalline form, pharmaceutically acceptable salt, hydrate or solvate thereof, which is a compound of formula (IIa) or formula (IIb):

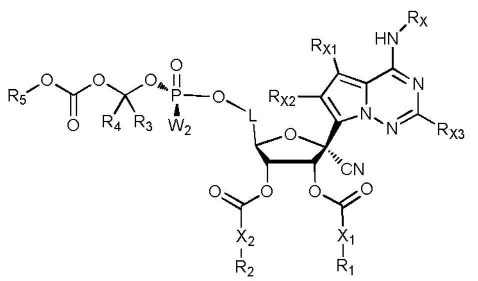 formula (IIa), 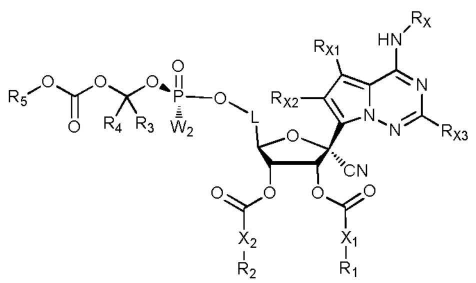

formula (IIb),

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond, O, S or NH, preferably a bond or O, and more preferably a bond;
$R_1$ and $R_2$ are independently $C_{1-6}$ alkyl (preferably $C_{1-3}$ alkyl), which is optionally substituted with one or more deuterium, up to full deuteration;
L is $CH_2$, $CD_2$ or CHD;
$R_3$ and $R_4$ are independently H, D, halogen, $C_{1-6}$ alkyl or $C_{1-6}$ haloalkyl, preferably H or D;
$R_5$ is $C_{1-6}$ alkyl (preferably isopropyl), which is optionally substituted with one or more deuterium, up to full deuteration;
$W_2$ is

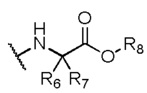, 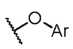, 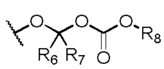 or 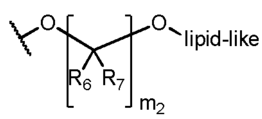 ;

each of $R_6$ and $R_7$ is independently H, D, $C_{1-6}$ alkyl (preferably $C_{1-3}$ alkyl) or $-OR_d$, or $R_6$ and $R_7$ together with the C atom to which they are attached, form a $C_{3-6}$ cycloalkyl or 3-7 membered heterocyclyl, which is optionally substituted with one or more R', and optionally substituted with one or more deuterium atoms, up to full deuteration;
each R' is independently $-C(O)OC_{1-6}$ alkyl, $C_{6-10}$ aryl or 5-10 membered heteroaryl, preferably - $C(O)OC_{1-6}$ alkyl or

phenyl;

$R_8$ is $C_{1-6}$ alkyl, $C_{3-6}$ cycloalkyl or 3-7 membered heterocyclyl, preferably $C_{1-6}$ alkyl or $C_{3-6}$ cycloalkyl, which is optionally substituted with one or more R, which is optionally substituted with one or more deuterium atoms, up to full deuteration;

Ar is phenyl, which is optionally substituted with one or more R;

R is independently D, $C_{1-6}$ alkyl or -O$C_{1-6}$ alkyl, which is optionally substituted with one or more deuterium atoms, up to full deuteration;

$m_2$ is 1, 2, 3, 4, 5 or 6, preferably 2, 3 or 4;

lipid-like is $C_{8-28}$ alkyl or $C_{8-28}$ alkenyl, preferably $C_{12-20}$ alkyl, which is optionally substituted with one or more deuterium atoms, up to full deuteration;

$R_d$ is independently $(CH_2)_{1-3}$-$C_{6-10}$ aryl or $(CH_2)_{1-3}$-5-10 membered heteroaryl, preferably $CH_2$-phenyl, which is optionally substituted with one or more R";

R" is independently D, halogen or -O$C_{1-6}$ alkyl, which is optionally substituted with one or more deuterium atoms, up to full deuteration;

or a tautomer, stereoisomer, prodrug, crystalline form, pharmaceutically acceptable salt, hydrate or solvate thereof.

In a more specific embodiment, the present invention relates to the aforementioned compound of formula (IIa) or formula (IIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more deuterium, up to full deuteration;

L is $CH_2$, $CD_2$ or CHD;

$R_3$ and $R_4$ are independently H, D, halogen, $C_{1-6}$ alkyl or $C_{1-6}$ haloalkyl, preferably H or D;

$R_5$ is $C_{1-6}$ alkyl (preferably isopropyl), which is optionally substituted with one or more deuterium, up to full deuteration;

$W_2$ is

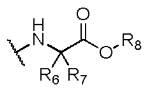 or 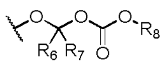 ;

each of $R_6$ and $R_7$ is independently H, D or $C_{1-3}$ alkyl, or $R_6$ and $R_7$ together with the C atom to which they are attached, form a $C_{3-6}$ cycloalkyl or 3-7 membered heterocyclyl, which is optionally substituted with one or more R', and optionally substituted with one or more deuterium atoms, up to full deuteration;

each of R' is independently -C(O)O$C_{1-6}$ alkyl or $C_{6-10}$ aryl, preferably -C(O)O$C_{1-6}$ alkyl or phenyl; $R_8$ is $C_{1-6}$ alkyl or $C_{3-6}$ cycloalkyl, which is optionally substituted with one or more R, which is optionally substituted with one or more deuterium atoms, up to full deuteration;

R is independently -O$C_{1-6}$ alkyl;

or a tautomer, stereoisomer, prodrug, crystalline form, pharmaceutically acceptable salt, hydrate or solvate thereof.

In a more specific embodiment, the present invention relates to the aforementioned compound of formula (IIa) or formula (IIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more deuterium, up to full deuteration;

L is $CH_2$, $CD_2$ or CHD;

$R_3$ and $R_4$ are independently H, D, halogen, $C_{1-6}$ alkyl or $C_{1-6}$ haloalkyl, preferably H or D;

$R_5$ is $C_{1-6}$ alkyl (preferably isopropyl), which is optionally substituted with one or more deuterium, up to full deuteration;

$W_2$ is

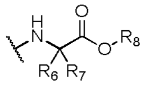 or 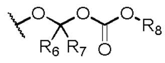 ;

each of $R_6$ and $R_7$ is independently H, D or $C_{1-3}$ alkyl, which is optionally substituted with one or more deuterium atoms, up to full deuteration;

$R_8$ is $C_{1-6}$ alkyl or $C_{3-6}$ cycloalkyl, which is optionally substituted with one or more deuterium atoms, up to full deuteration;

or a tautomer, stereoisomer, prodrug, crystalline form, pharmaceutically acceptable salt, hydrate or solvate thereof.

In a more specific embodiment, the present invention relates to the aforementioned compound of formula (IIa) or formula (IIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more deuterium, up to full deuteration;

L is $CH_2$, $CD_2$ or CHD;

$R_3$ and $R_4$ are independently H, D, halogen, $C_{1-6}$ alkyl or $C_{1-6}$ haloalkyl, preferably H or D;

$R_5$ is $C_{1-6}$ alkyl (preferably isopropyl), which is optionally substituted with one or more deuterium, up to full deuteration;

$W_2$ is

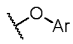 or 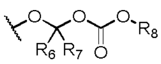 ;

Ar is phenyl, which is optionally substituted with one or more R;

R is independently $C_{1-6}$ alkyl, which is optionally substituted with one or more deuterium atoms, up to full deuteration;

each of $R_6$ and $R_7$ is independently H, D or $C_{1-3}$ alkyl, which is optionally substituted with one or more deuterium atoms, up to full deuteration;

$R_8$ is $C_{1-6}$ alkyl or $C_{3-6}$ cycloalkyl, which is optionally substituted with one or more deuterium atoms, up to full deuteration;

or a tautomer, stereoisomer, prodrug, crystalline form, pharmaceutically acceptable salt, hydrate or solvate thereof.

In a more specific embodiment, the present invention relates to the aforementioned compound of formula (IIa) or formula (IIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more deuterium, up to full deuteration;

L is $CH_2$, $CD_2$ or CHD;

$R_3$ and $R_4$ are independently H or D;

$R_5$ is isopropyl, which is optionally substituted with one or more deuterium atoms, up to full deuteration;

$W_2$ is

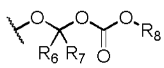 ;

each of $R_6$ and $R_7$ is independently H or D;

$R_8$ is isopropyl, which is optionally substituted with one or more deuterium atoms, up to full deuteration;

or a tautomer, stereoisomer, prodrug, crystalline form, pharmaceutically acceptable salt, hydrate or solvate thereof.

In a more specific embodiment, the present invention relates to the aforementioned compound, or a tautomer,

stereoisomer, prodrug, crystalline form, pharmaceutically acceptable salt, hydrate or solvate thereof, which is a compound of formula (IIIa) or formula (IIIb):

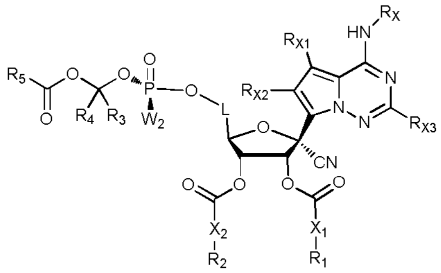 formula (IIIa), 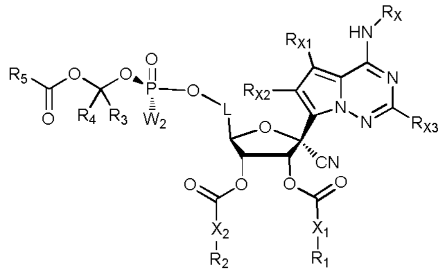 formula (IIIb)

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$R_3$ and $R_4$ are independently H or D;
$R_5$ is $C_{1-6}$ alkyl, which is optionally substituted with one or more D, up to full deuteration;
$W_2$ is

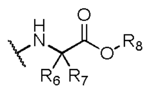, 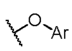 or 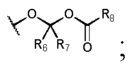;

each of $R_6$ and $R_7$ is independently H, D or $C_{1-3}$ alkyl, which is optionally substituted with one or more D, up to full deuteration;
$R_8$ is $C_{1-6}$ alkyl, which is optionally substituted with one or more D, up to full deuteration;
Ar is phenyl, which is optionally substituted with one or more R;
R is independently $C_{1-6}$ alkyl, which is optionally substituted with one or more D, up to full deuteration;
or a tautomer, stereoisomer, prodrug, crystalline form, pharmaceutically acceptable salt, hydrate or solvate thereof.

In a more specific embodiment, the present invention relates to the aforementioned compound of formula (IIIa) or formula (IIIb), or a tautomer, stereoisomer, prodrug, crystalline form, pharmaceutically acceptable salt, hydrate or solvate thereof, wherein,
wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$R_3$ and $R_4$ are independently H or D;
$R_5$ is $C_{1-6}$ alkyl (preferably tert-butyl), which is optionally substituted with one or more D, up to full deuteration;
$W_2$ is

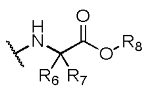;

each of $R_6$ and $R_7$ is independently H, D or $C_{1-3}$ alkyl, which is optionally substituted with one or more D, up to full

deuteration;

$R_8$ is $C_{1-6}$ alkyl, which is optionally substituted with one or more D, up to full deuteration;

or a tautomer, stereoisomer, prodrug, crystalline form, pharmaceutically acceptable salt, hydrate or solvate thereof.

In a more specific embodiment, the present invention relates to the aforementioned compound of formula (IIIa) or formula (IIIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more D, up to full deuteration;

L is $CH_2$, $CD_2$ or CHD;

$R_3$ and $R_4$ are independently H or D;

$R_5$ is $C_{1-6}$ alkyl (preferably $C_{1-3}$ alkyl, preferably isopropyl), which is optionally substituted with one or more D, up to full deuteration;

$W_2$ is

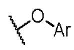

;

Ar is phenyl, which is optionally substituted with one or more R;

R is independently $C_{1-6}$ alkyl, which is optionally substituted with one or more D, up to full deuteration;

or a tautomer, stereoisomer, prodrug, crystalline form, pharmaceutically acceptable salt, hydrate or solvate thereof.

In a more specific embodiment, the present invention relates to the aforementioned compound of formula (IIIa) or formula (IIIb), or a tautomer, stereoisomer, prodrug, crystalline form, pharmaceutically acceptable salt, hydrate or solvate thereof, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more D, up to full deuteration;

L is $CH_2$, $CD_2$ or CHD;

$R_3$ and $R_4$ are independently H or D;

$R_5$ is $C_{1-6}$ alkyl (preferably $C_{1-3}$ alkyl, preferably tert-butyl, more preferably isopropyl), which is optionally substituted with one or more D, up to full deuteration;

$W_2$ is

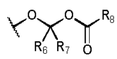

;

each of $R_6$ and $R_7$ is independently H or D;

$R_8$ is $C_{1-6}$ alkyl (preferably $C_{1-3}$ alkyl, preferably tert-butyl, more preferably isopropyl), which is optionally substituted with one or more D, up to full deuteration;

or a tautomer, stereoisomer, prodrug, crystalline form, pharmaceutically acceptable salt, hydrate or solvate thereof.

In a more specific embodiment, the present invention relates to the aforementioned compound, or a tautomer, stereoisomer, prodrug, crystalline form, pharmaceutically acceptable salt, hydrate or solvate thereof, which is a compound of formula (IVa) or formula (IVb),

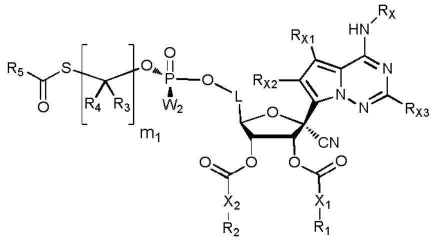 formula (IVa), or 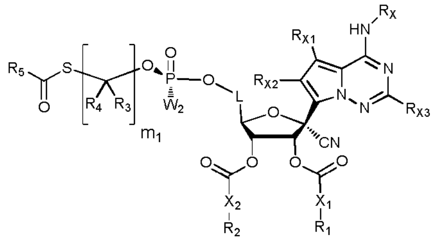 formula (IVb)

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$m_1$ is 1, 2 or 3;
$R_3$ and $R_4$ are independently H or D;
$R_5$ is $C_{1-6}$ alkyl (preferably tert-butyl), which is optionally substituted with one or more D, up to full deuteration;
$W_2$ is

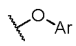 or 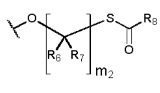 ;

$m_2$ is 1, 2 or 3;
each of $R_6$ and $R_7$ is independently H or D, which is optionally substituted with one or more D, up to full deuteration;
$R_8$ is $C_{1-6}$ alkyl (preferably tert-butyl), which is optionally substituted with one or more D, up to full deuteration;
Ar is phenyl, which is optionally substituted with one or more R;
R is independently $C_{1-6}$ alkyl, which is optionally substituted with one or more D, up to full deuteration;
or a tautomer, stereoisomer, prodrug, crystalline form, pharmaceutically acceptable salt, hydrate or solvate thereof.

In a more specific embodiment, the present invention relates to the compound of formulas (V) or (VI):

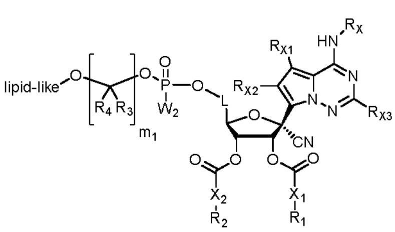 formula (V), or 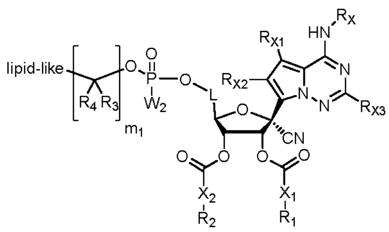 formula (VI)

wherein,

$R_X$ is H or OH;
$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl,

$C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$m_1$ is 1, 2, 3, 4, 5 or 6;

$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl or $C_{8-28}$ alkynyl is optionally substituted with one or more R;

$W_2$ is formula (B):

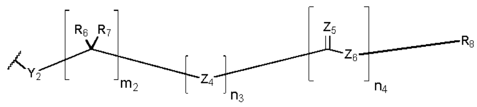

formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, $W_2$ together with the 3'-position C atom in the glycosyl form a -$Y_3$-, wherien $Y_3$ is O, NH or S;

each R is independently D, halogen, -$R_d$, -C(O)$R_d$, -C(O)O$R_d$, -C(O)N$R_eR_f$, -N$R_eR_f$, - N$R_d$C(O)$R_e$, -N$R_d$C(O)O$R_e$, -N$R_d$C(O)N$R_eR_f$, -O$R_d$, -OC(O)$R_d$, -OC(O)N$R_eR_f$, -N$R_d$S(O)$_2R_e$, - S(O)$_2$N$R_d$, -S(O)$R_d$ or -S(O)$_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, -$R_d$, -C(O)$R_d$, -C(O)O$R_d$, -C(O)N$R_eR_f$, -N$R_eR_f$, - N$R_d$C(O)$R_e$, -N$R_d$C(O)O$R_e$, -N$R_d$C(O)N$R_eR_f$, -O$R_d$, -OC(O)$R_d$, -OC(O)N$R_eR_f$, -N$R_d$S(O)$_2R_e$, - S(O)$_2$N$R_d$, -S(O)$R_d$ or -S(O)$_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, -$R_g$, -C(O)$R_g$, -C(O)O$R_g$, -C(O)N$R_hR_j$, -N$R_hR_j$, - N$R_g$C(O)$R_h$, -N$R_g$C(O)O$R_h$, -N$R_g$C(O)N$R_hR_j$, -O$R_g$, -OC(O)$R_g$, -OC(O)N$R_hR_j$, -N$R_g$S(O)$_2R_h$, - S(O)$_2$N$R_g$, -S(O)$R_g$ or -S(O)$_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;
or a tautomer, stereoisomer, prodrug, crystalline form, pharmaceutically acceptable salt, hydrate or solvate thereof.

In a more specific embodiment, the present invention relates to the compound of formula (Va), formula (Vb), formula (VIa) or formula (VIb):

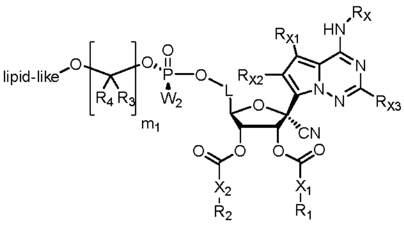

formula (Va),

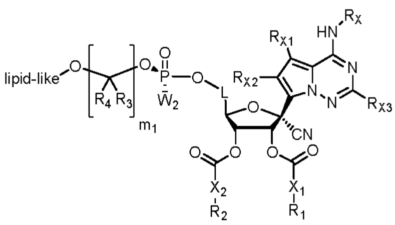

formula (Vb),

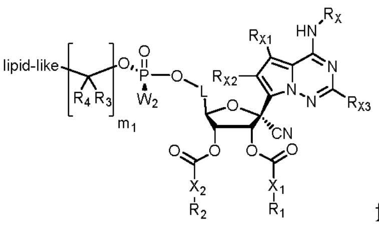

formula (VIa), or

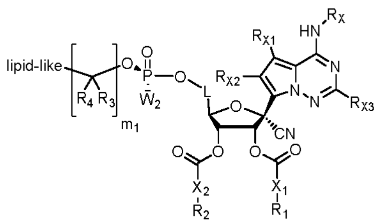

formula (VIb)

or a tautomer, stereoisomer, prodrug, crystalline form, pharmaceutically acceptable salt, hydrate or solvate thereof.

In a more specific embodiment, the present invention relates to the aforementioned compound, or a tautomer, stereoisomer, prodrug, crystalline form, pharmaceutically acceptable salt, hydrate or solvate thereof, wherein:

$R_X$ is H or OH;
$R_{X1}$, $R_{X2}$ and $R_{X3}$ are independently H, D, halogen or $C_{1-6}$ alkyl, wherein the $C_{1-6}$ alkyl is optionally substituted with one or more deuterium, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R'; L is $CH_2$, $CD_2$ or CHD;
$m_1$ is 2, 3 or 4;
$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{3-6}$ cycloalkyl and 3-7 membered heterocyclyl are optionally substituted with one or more R';
lipid-like is $C_{1-28}$ alkyl, $C_{1-28}$ haloalkyl, $C_{2-28}$ alkenyl or $C_{2-28}$ alkynyl, wherein the $C_{1-28}$ alkyl, $C_{1-28}$ haloalkyl, $C_{2-28}$ alkenyl and $C_{2-28}$ alkynyl are optionally substituted with one or more R;
$W_2$ is:

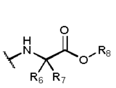 , 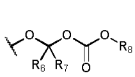 , 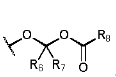 , 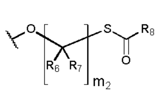 , 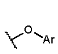 or 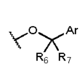 ;

wherein,
$m_2$ is 1, 2, 3, 4, 5 or 6;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration.

In a more specific embodiment, the present invention relates to the aforementioned compound, or a tautomer, stereoisomer, prodrug, crystalline form, pharmaceutically acceptable salt, hydrate or solvate thereof, wherein:

$W_2$ is

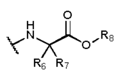 , 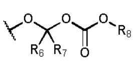 , 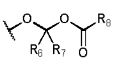 , 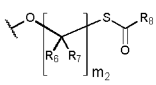 , 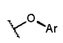 or 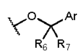 ;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

each of $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

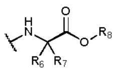,

wherein, $R_6$ is $C_{1-6}$ alkyl, and $R_7$ is H, or $R_6$ and $R_7$ together with the C atom to which they are attached, form a $C_{3-6}$ cycloalkyl, wherein the $C_{1-6}$ alkyl and $C_{3-6}$ cycloalkyl are optionally substituted with one or more groups selected from phenyl or -C(O)O$C_{1-6}$ alkyl;

$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl is optionally substituted with one or more $C_{1-6}$ alkoxy;

preferably, $W_2$ is

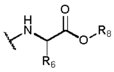;

preferably, $W_2$ is

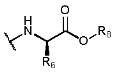;

preferably, $W_2$ is

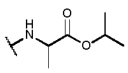, 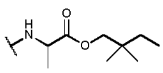, 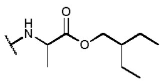, 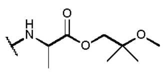, 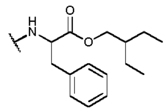,

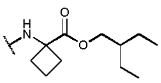, 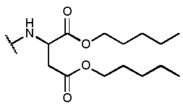, 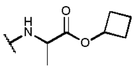, 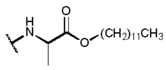 or 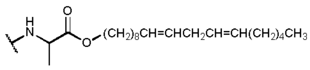;

preferably, $W_2$ is

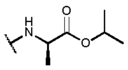, 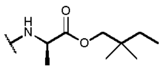, 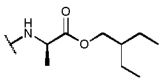, 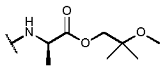, 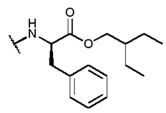,

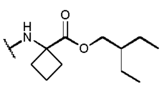, 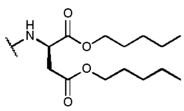, 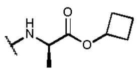, 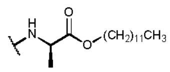 or 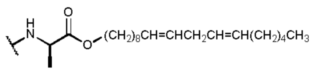.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

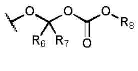,

wherein, $R_6$ and $R_7$ are H;

$R_8$ is independently $C_{1-28}$ alkyl, $C_{3-6}$ cycloalkyl, 3-7 membered heterocyclyl or $C_{2-28}$ alkenyl;

preferably, $W_2$ is

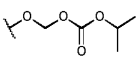.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

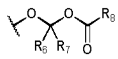,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

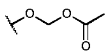, 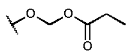 or 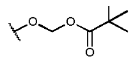.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

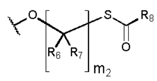,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

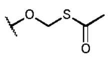, 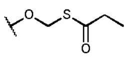, 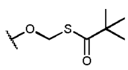, 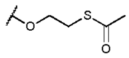, 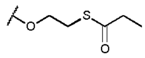 or 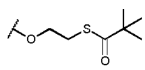.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

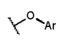,

wherein, Ar is $C_{6-10}$ aryl or 5-10 membered heteroaryl, wherein the $C_{6-10}$ aryl or 5-10 membered heteroaryl is optionally substituted with one or more $C_{1-6}$ alkyl;

preferably, $W_2$ is

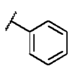, 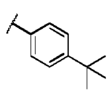 or 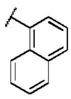.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

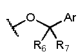,

wherein, $R_6$ and $R_7$ are H;

Ar is $C_{6-10}$ aryl or 5-10 membered heteroaryl, wherein the $C_{6-10}$ aryl or 5-10 membered heteroaryl is optionally substituted with one or more groups selected from $C_{1-6}$ alkyl or $-C(O)OC_{1-6}$ alkyl;

preferably, $W_2$ is

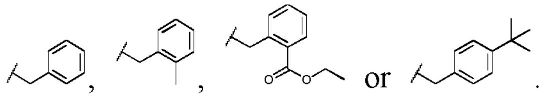
, , or .

In a more specific embodiment, the present invention relates to the aforementioned compound, or a tautomer, stereoisomer, prodrug, crystalline form, pharmaceutically acceptable salt, hydrate or solvate thereof, wherein, $W_2$ is

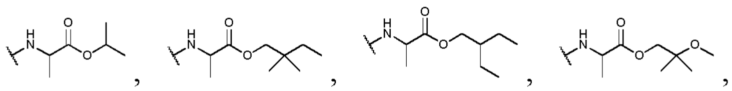
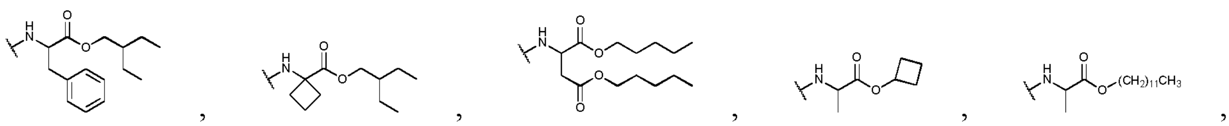
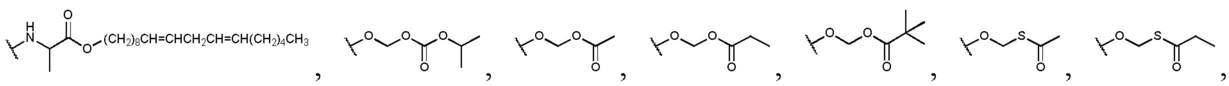
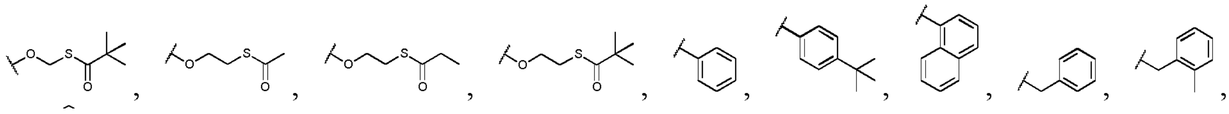
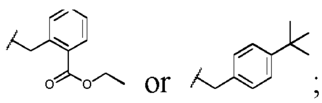
or ;

preferably, $W_2$ is

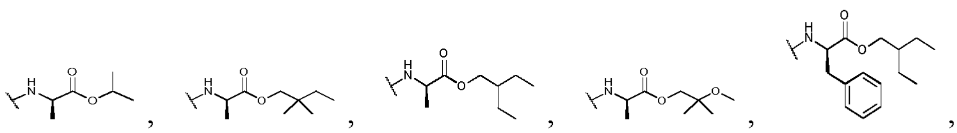
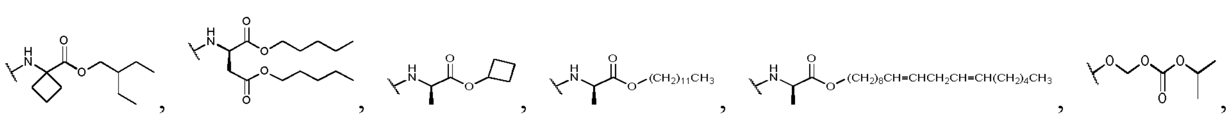
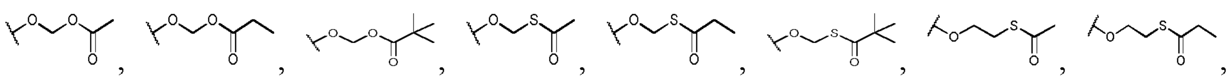
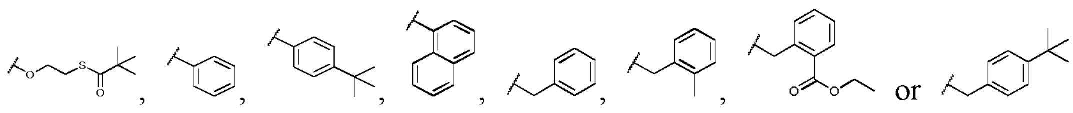
or .

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$m_1$ is 2, 3 or 4;
$R_3$ and $R_4$ are independently H, D or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;
lipid-like is $C_{1-28}$ alkyl or $C_{2-28}$ alkenyl.

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$m_1$ is 2, 3 or 4;
$R_3$ and $R_4$ are independently H, D or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;
lipid-like is $C_{1-28}$ alkyl or $C_{2-28}$ alkenyl.

In a more specific embodiment, the present invention relates to the compound of formula (VII):

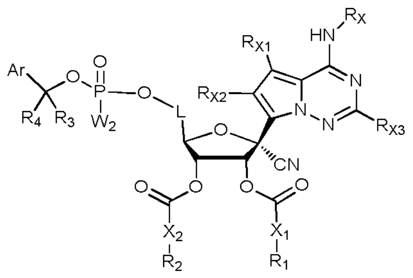

formula (VII)

wherein,

$R_X$ is H or OH;
$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$; wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
L is $CH_2$, $CD_2$ or CHD;
$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

Ar is $C_{6-10}$ aryl or 5-10 membered heteroaryl, wherein the $C_{6-10}$ aryl and 5-10 membered heteroaryl are optionally substituted with one or more R;

$W_2$ is formula (B):

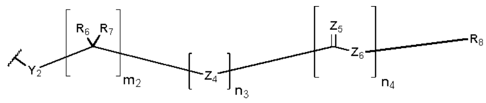

formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, $W_2$ together with the 3'-position C atom in the glycosyl form a $-Y_3-$, wherien $Y_3$ is O, NH or S;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration;

or a tautomer, stereoisomer, prodrug, crystalline form, pharmaceutically acceptable salt, hydrate or solvate thereof.

In a more specific embodiment, the present invention relates to the compound of formula (VIIa) or formula (VIIb):

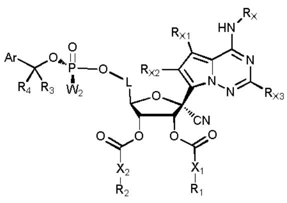 formula (VIIa), or 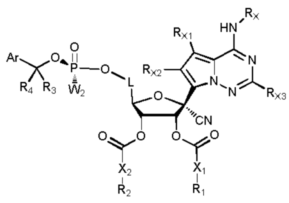 formula (VIIb)

or a tautomer, stereoisomer, prodrug, crystalline form, pharmaceutically acceptable salt, hydrate or solvate thereof.

In a more specific embodiment, the present invention relates to the aforementioned compound, or a tautomer, stereoisomer, prodrug, crystalline form, pharmaceutically acceptable salt, hydrate or solvate thereof, wherein:

$R_X$ is H or OH;
$R_{X1}$, $R_{X2}$ and $R_{X3}$ are independently H, D, halogen or $C_{1-6}$ alkyl, wherein the $C_{1-6}$ alkyl is optionally substituted with one or more deuterium, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R'; L is $CH_2$, $CD_2$ or CHD;
$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein the $C_{1-6}$ alkyl, $C_{3-6}$ cycloalkyl and 3-7 membered heterocyclyl are optionally substituted with one or more R';
Ar is $C_{6-10}$ aryl or 5-10 membered heteroaryl, wherein the $C_{6-10}$ aryl and 5-10 membered heteroaryl are optionally substituted with one or more R;
$W_2$ is:

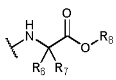 , 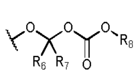 , 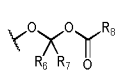 , 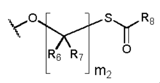 , 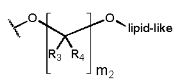 or 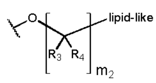 ;

wherein,
$m_2$ is 1, 2, 3, 4, 5 or 6;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein the $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;
Ar is $C_{6-10}$ aryl or 5-10 membered heteroaryl, wherein the $C_{6-10}$ aryl and 5-10 membered heteroaryl are optionally substituted with one or more R;
each R is independently D, halogen, $-R_d$, $-C(O)R_d$, $-C(O)OR_d$, $-C(O)NR_eR_f$, $-NR_eR_f$, - NR4C(O)Re, $-NR_dC(O)ORe$, $-NR_dC(O)NR_eR_f$, $-OR_d$, $-OC(O)R_d$, $-OC(O)NR_eR_f$, $-NR_dS(O)_2R_e$, - $S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R", up to full deuteration;
each R' is independently D, halogen, $-R_d$, $-C(O)R_d$, $-C(O)OR_d$, $-C(O)NR_eR_f$, $-NR_eR_f$, - $NR4C(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, - $S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom

to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, - $NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, - $S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein the $C_{3-6}$ cycloalkyl, 3-7 membered heterocyclyl, $C_{6-10}$ aryl and 5-10 membered heteroaryl are optionally substituted with one or more D atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein the $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more D atoms, up to full deuteration.

In a more specific embodiment, the present invention relates to the aforementioned compound, or a tautomer, stereoisomer, prodrug, crystalline form, pharmaceutically acceptable salt, hydrate or solvate thereof, wherein:

$W_2$ is

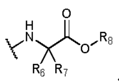, 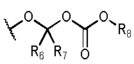, 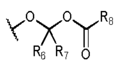, 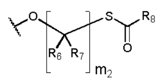, 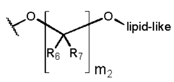 or 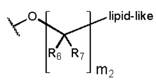;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein the $C_{1-6}$ alkyl, $C_{1-6}$ alkoxy, $C_{3-6}$ cycloalkyl and 3-7 membered heterocyclyl are optionally substituted with one or more R';

each of $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein the $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

lipid-like is $C_{8-28}$ alkyl, $C_{8-28}$ haloalkyl, $C_{8-28}$ alkenyl or $C_{8-28}$ alkynyl, wherein the $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein the $C_{6-10}$ aryl and 5-10 membered heteroaryl are optionally substituted with one or more R.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

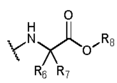,

wherein, $R_6$ is $C_{1-6}$ alkyl, and $R_7$ is H, or $R_6$ and $R_7$ together with the C atom to which they are attached, form a $C_{3-6}$ cycloalkyl, wherein the $C_{1-6}$ alkyl and $C_{3-6}$ cycloalkyl are optionally substituted with one or more groups selected from phenyl or $-C(O)OC_{1-6}$ alkyl;

$R_8$ is independently $C_{1-28}$ alkyl, $C_{3-6}$ cycloalkyl, 3-7 membered heterocyclyl or $C_{2-28}$ alkenyl, wherein the $C_{1-28}$ alkyl, $C_{3-6}$ cycloalkyl, 3-7 membered heterocyclyl or $C_{2-28}$ alkenyl is optionally substituted with one or more $C_{1-6}$ alkoxy;

preferably, $W_2$ is

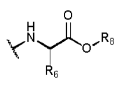;

preferably, $W_2$ is

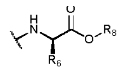;

preferably, $W_2$ is

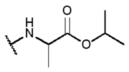, 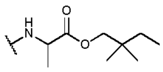, 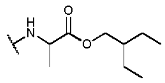, 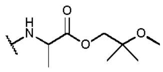, 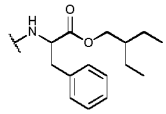,

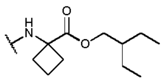, 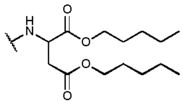, 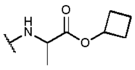, 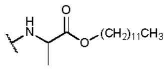 or 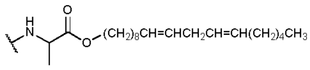;

preferably, $W_2$ is

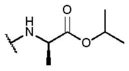, 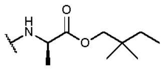, 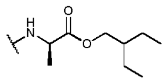, 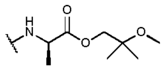, 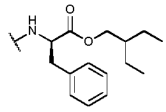,

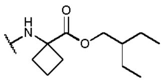, 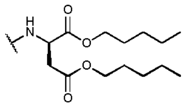, 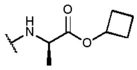, 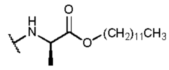 or 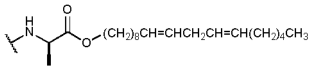.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

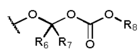,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

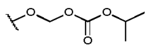.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

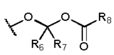,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

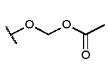, 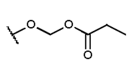 or 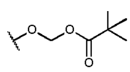.

In a more specific embodiment, the present invention relates to the aforementioned compound, or a tautomer,

stereoisomer, prodrug, crystalline form, pharmaceutically acceptable salt, hydrate or solvate thereof, wherein,

$W_2$ is

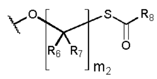,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

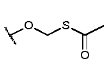, 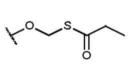, 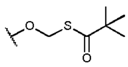, 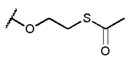, 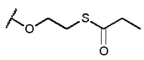 or 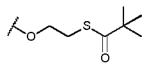.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

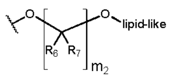,

wherein, $m_2$ is 2, 3 or 4;
$R_6$ and $R_7$ are independently H or $C_{1-6}$ alkoxy, wherein the $C_{1-6}$ alkoxy is optionally substituted with one or more groups selected from $C_{6-10}$ aryl or 5-10 membered heteroaryl, wherein the $C_{6-10}$ aryl or 5-10 membered heteroaryl is optionally substituted with one or more groups selected from halogen or $C_{1-6}$ alkoxy;
lipid-like is $C_{8-28}$ alkyl or $C_{8-28}$ alkenyl;
preferably, $W_2$ is

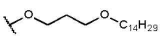, 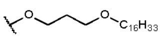, 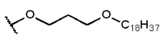, 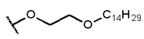, 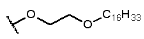,

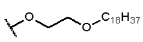, 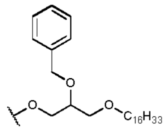, 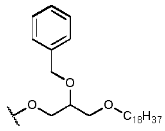, 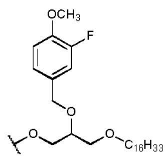 or 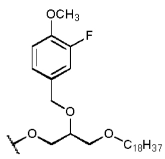;

preferably, $W_2$ is

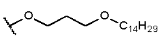, 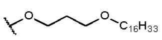, 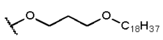, 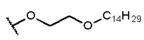, 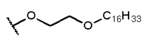,

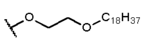, 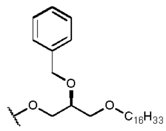, 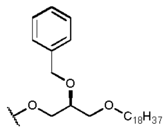, 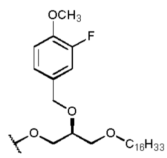 or 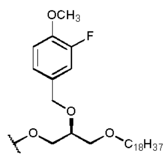.

In a more specific embodiment, the present invention relates to the aforementioned compound, or a tautomer, stereoisomer, prodrug, crystalline form, pharmaceutically acceptable salt, hydrate or solvate thereof, wherein,

$W_2$ is

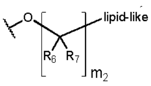,

wherein, $m_2$ is 2, 3 or 4;
$R_6$ and $R_7$ are H;
lipid-like is $C_{8-28}$ alkenyl;
preferably, $W_2$ is

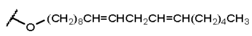.

In a more specific embodiment, the present invention relates to the aforementioned compound, or a tautomer, stereoisomer, prodrug, crystalline form, pharmaceutically acceptable salt, hydrate or solvate thereof, wherein, $W_2$ is

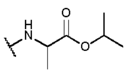, 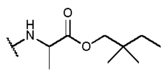, 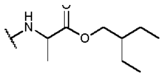, 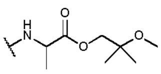,

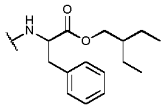, 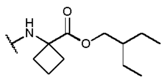, 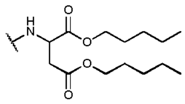, 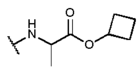, 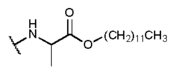,

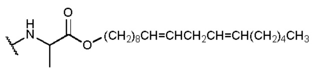, 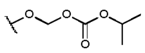, 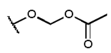, 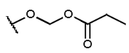, 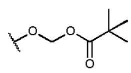, 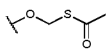, 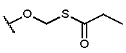,

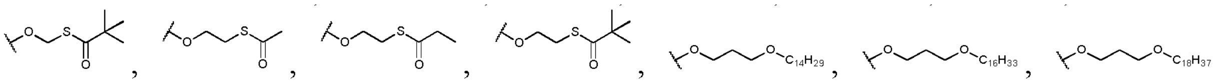
, , , , , , ,

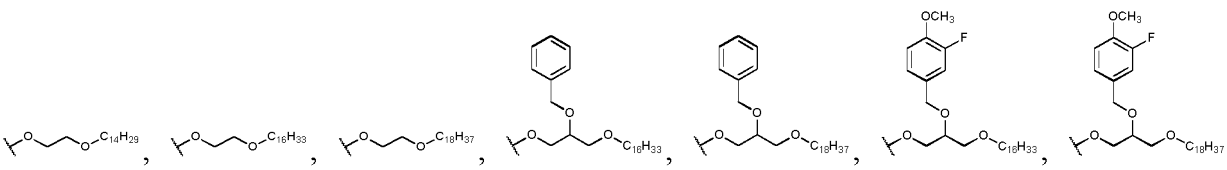
, , , , , , or

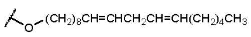.

preferably, $W_2$ is

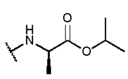, 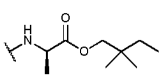, 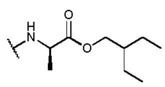, 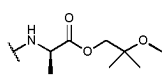, 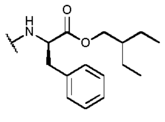,

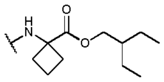, 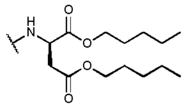, 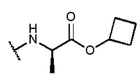, 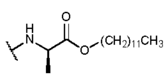, 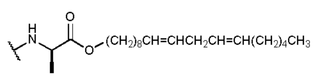, 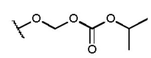,

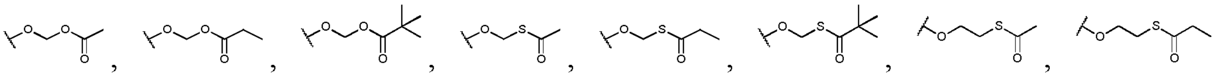
, , , , , , , ,

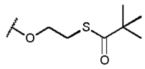, 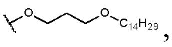, 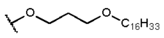, 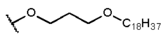, 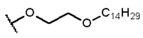, 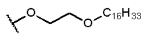, 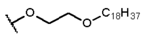,

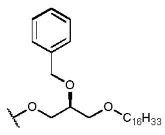, 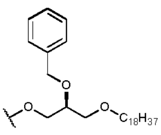, 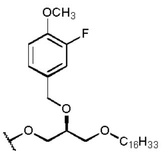, 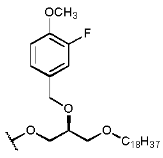 or 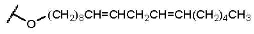.

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$R_3$ and $R_4$ are independently H, D or $C_{1-6}$ alkyl;
Ar is $C_{6-10}$ aryl or 5-10 membered heteroaryl.

In a more specific embodiment, the present invention relates to the aforementioned compound, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$R_3$ and $R_4$ are independently H or D;
Ar is $C_{6-10}$ aryl.

In a more specific embodiment, the present invention relates to compounds of formula (VIIa), formula (VIIa) or formula (VIIb):

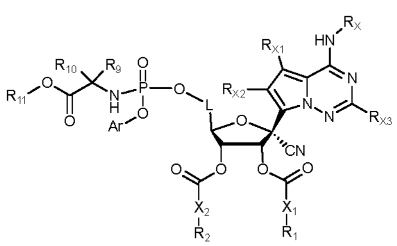 formula (VII), 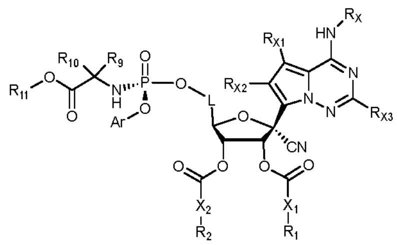 formula (VIIa)

or

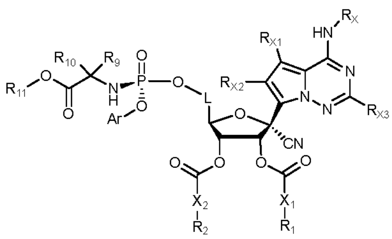 formula (VIIb)

wherein,

$R_X$ is H or OH, preferably H;

$R_{X1}$, $R_{X2}$ and $R_{X3}$ are independently H, D or halogen, preferably H or D;
$X_1$ and $X_2$ are independently a bond, O, S or NH, preferably a bond or O, and more preferably a bond;
$R_1$ and $R_2$ are independently $C_{1-6}$ alkyl (preferably $C_{1-3}$ alkyl), which is optionally substituted with one or more deuterium, up to full deuteration;
L is $CH_2$, $CD_2$ or CHD;
$R_9$ is $CH_2$-$C_{6-10}$ aryl, $CH_2$-5-10 membered heteroaryl, $CH_2C(O)O$-$C_{1-6}$ alkyl, $CH_2C(O)O$-$C_{3-6}$ cycloalkyl or $CH_2C(O)O$-3-7 membered heteroaryl, preferably $CH_2$-phenyl, $CH_2C(O)O$-$C_{1-6}$ alkyl or $CH_2C(O)O$-$C_{3-6}$ cycloalkyl, which is optionally substituted with one or more deuterium, up to full deuteration; and $R_{10}$ is H or D;
or, $R_9$ and $R_{10}$ together with the C atom to which they are attached, form a $C_{3-6}$ cycloalkyl or 3-7 membered heterocyclyl, which is optionally substituted with one or more deuterium, up to full deuteration;
Ar is phenyl, which is optionally substituted with one or more R;
R is independently D, $C_{1-6}$ alkyl or -O$C_{1-6}$ alkyl, which is optionally substituted with one or more deuterium atoms, up to full deuteration;
or a tautomer, stereoisomer, prodrug, crystalline form, pharmaceutically acceptable salt, hydrate or solvate thereof.

In a more specific embodiment, the present invention relates to the compound of formula (VIIa), formula (VIIa) or formula (VIIb), or a tautomer, stereoisomer, prodrug, crystalline form, pharmaceutically acceptable salt, hydrate or solvate thereof, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl, more preferably isopropyl), which is optionally substituted with one or more deuterium, up to full deuteration;
L is $CH_2$, $CD_2$ or CHD;
$R_9$ is $CH_2$-phenyl, $CH_2C(O)O$-$C_{1-6}$ alkyl or $CH_2C(O)O$-$C_{3-6}$ cycloalkyl, which is optionally substituted with one or more deuterium, up to full deuteration; and $R_{10}$ is H or D;
Ar is phenyl, which is optionally substituted with one or more R;
R is independently $C_{1-6}$ alkyl (preferably tert-butyl), which is optionally substituted with one or more deuterium, up to full deuteration;
or a tautomer, stereoisomer, prodrug, crystalline form, pharmaceutically acceptable salt, hydrate or solvate thereof.

In a more specific embodiment, the present invention relates to the aforementioned compound, or a tautomer, stereoisomer, prodrug, crystalline form, pharmaceutically acceptable salt, hydrate or solvate thereof, wherein, the compound is selected from:

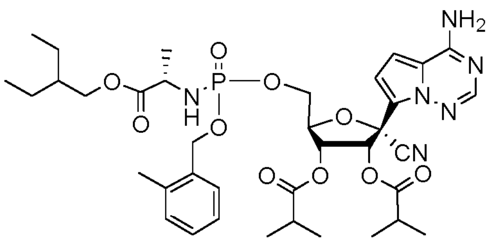 , 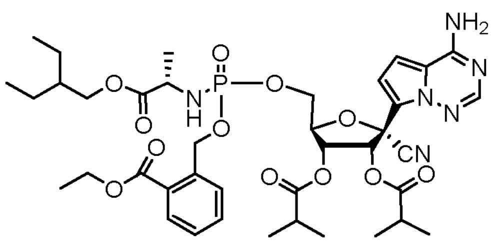 ,

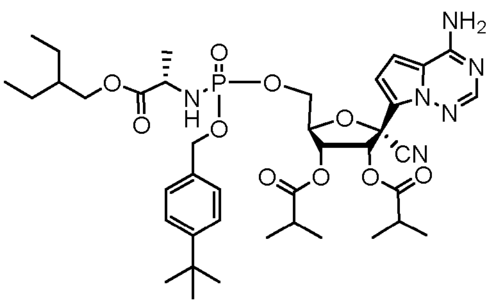 , 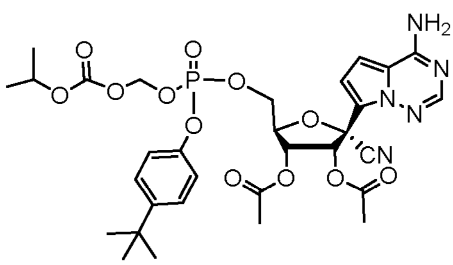 ,

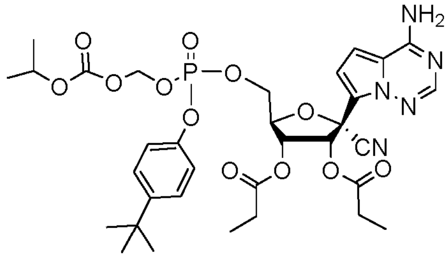,
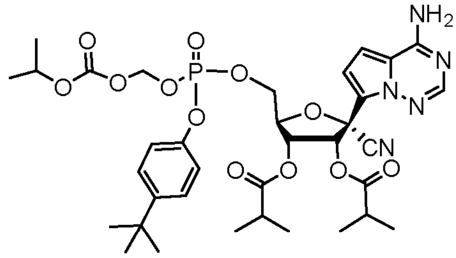,

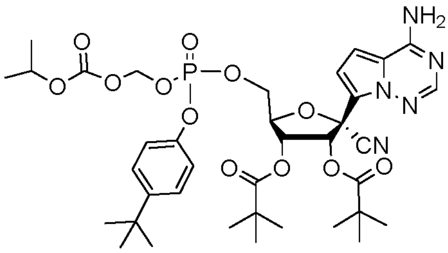,
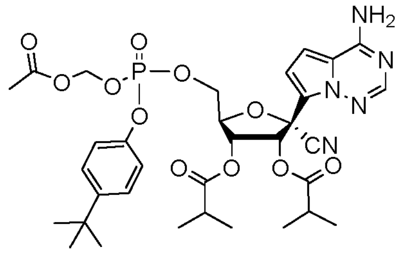,

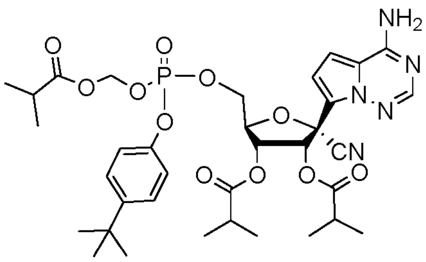,
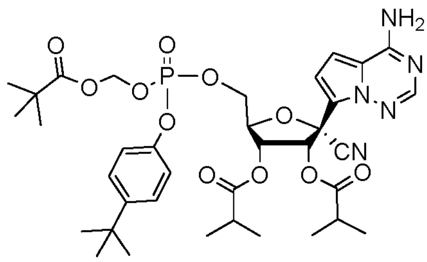,

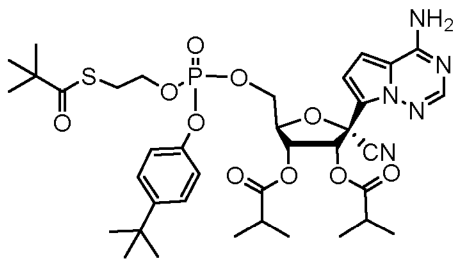,
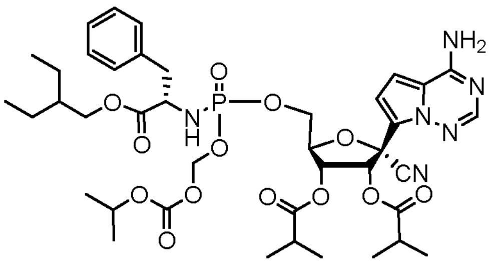,

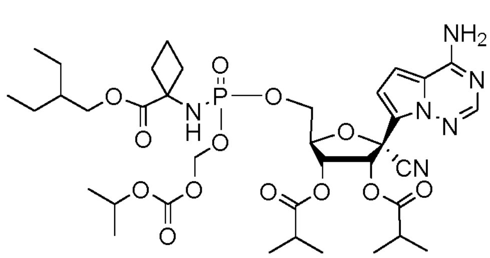,
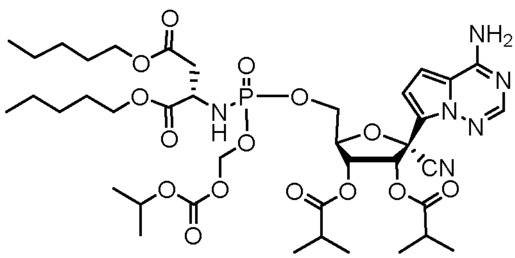,

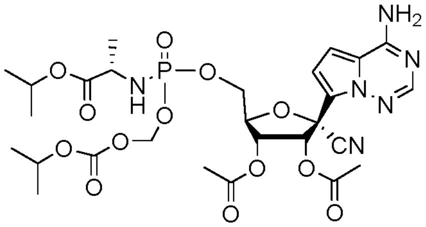,
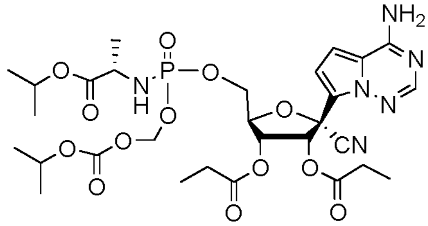,

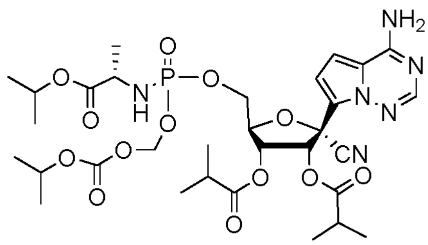,
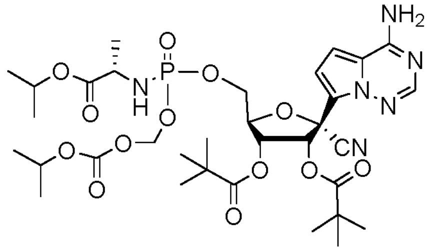,
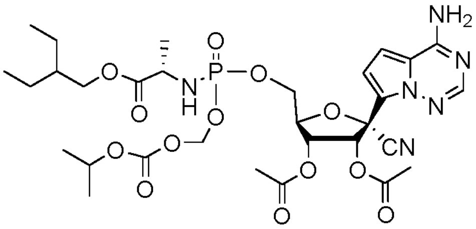,
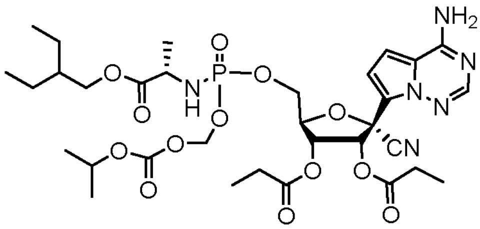,
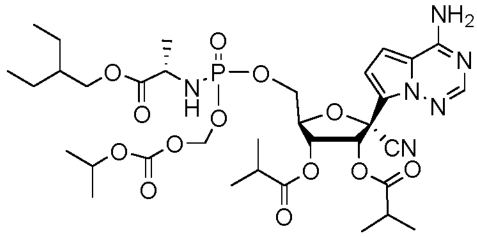,
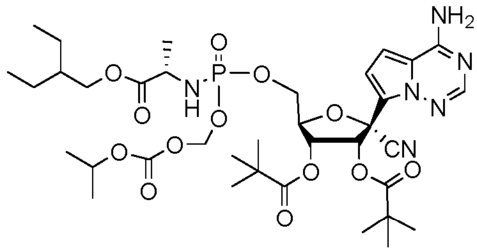,
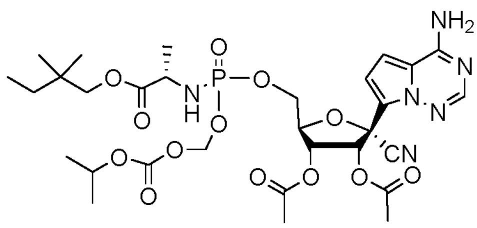,
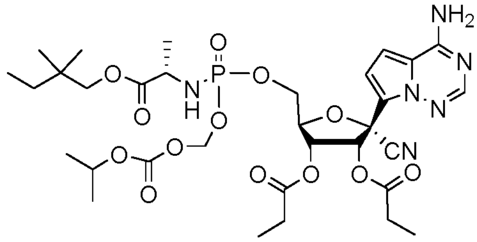,
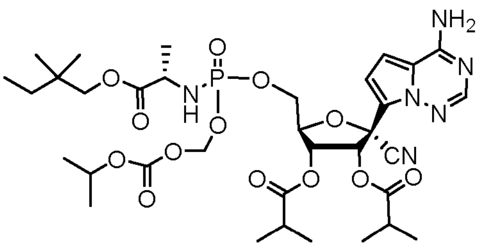,
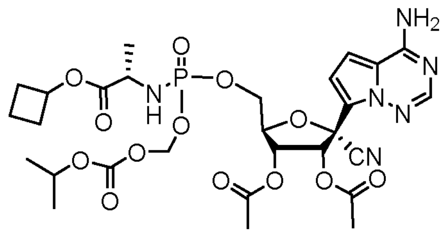,
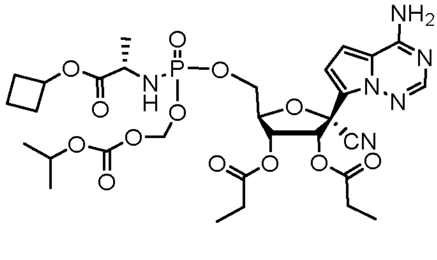,
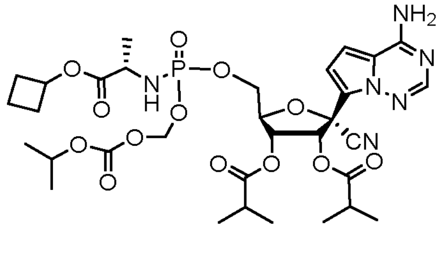,

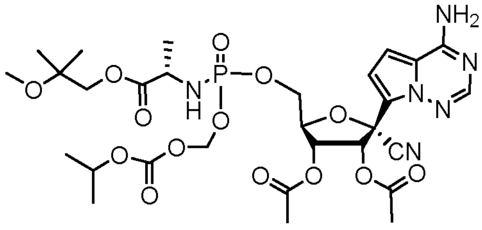,

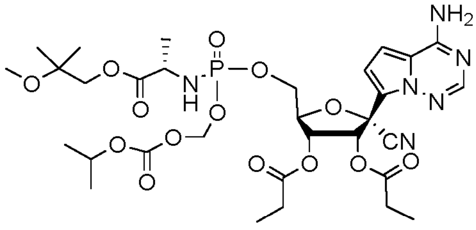,

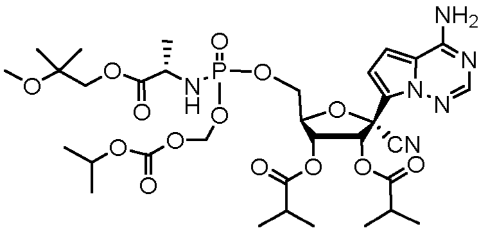,

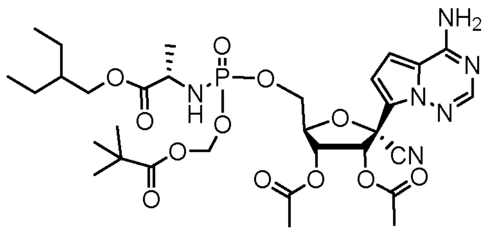,

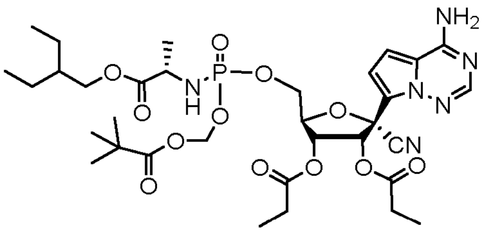,

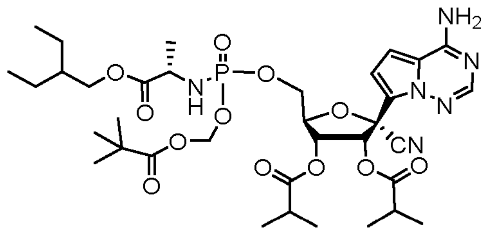,

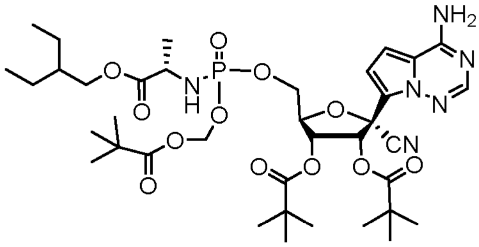,

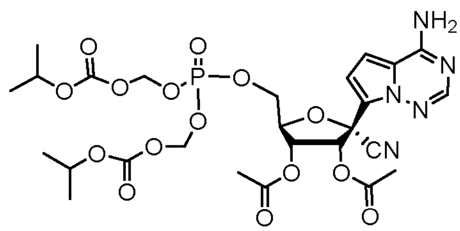,

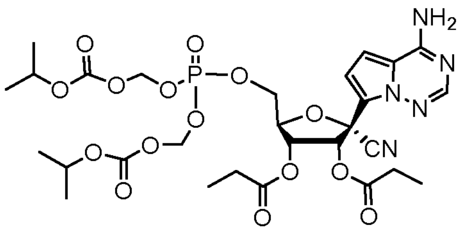,

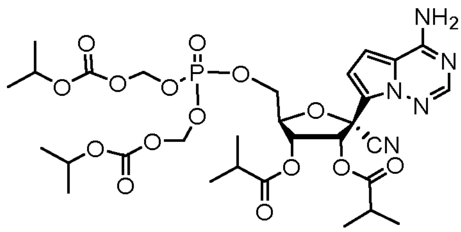,

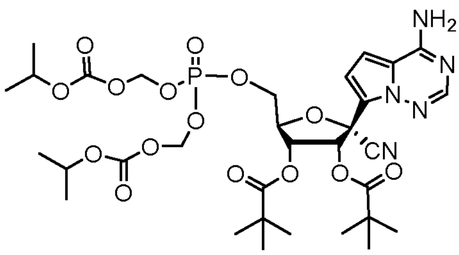,

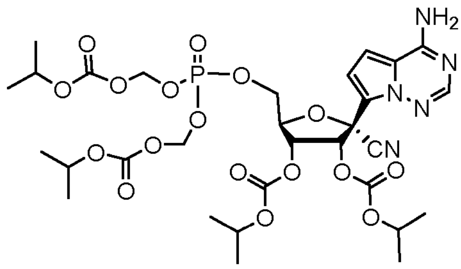,

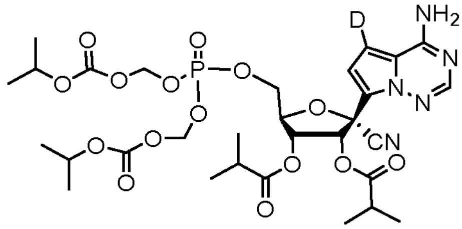
,
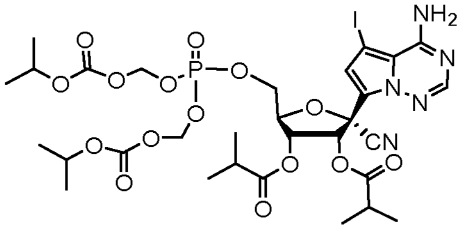
,
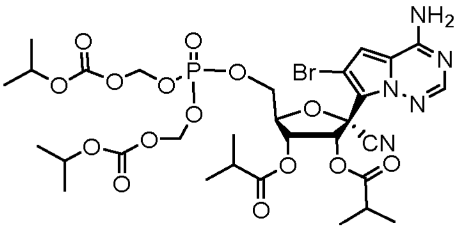
,
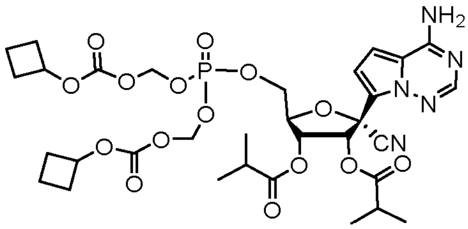
,
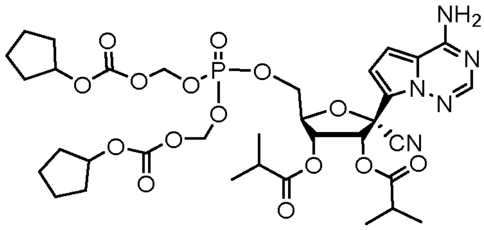
,
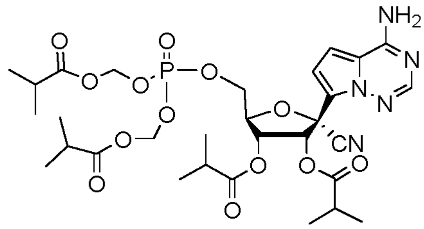
,
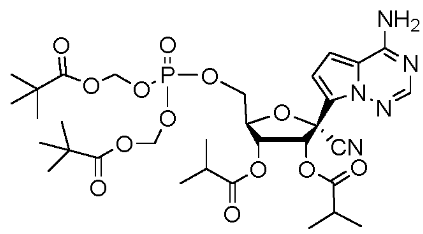
,
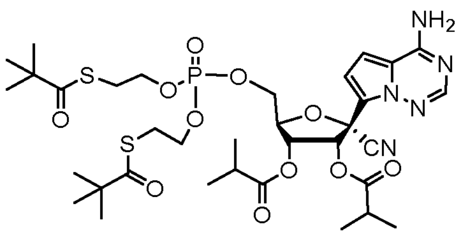
,
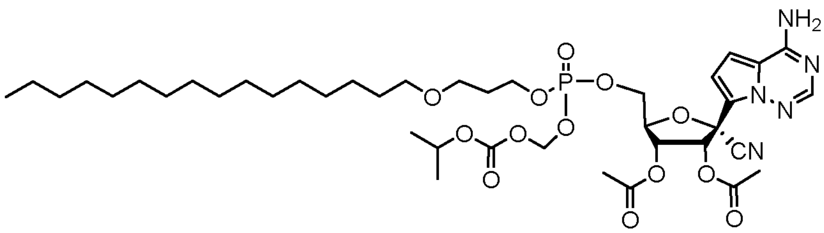
,
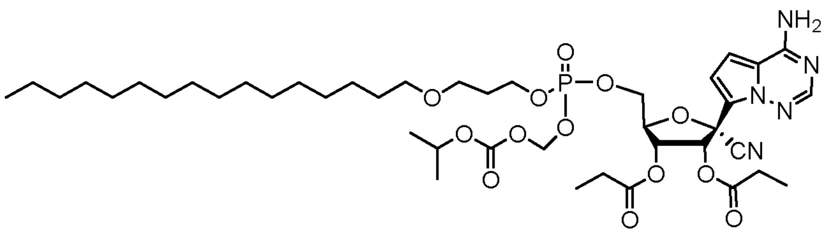
,

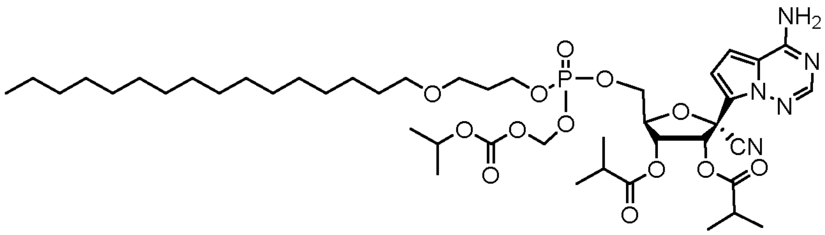

,

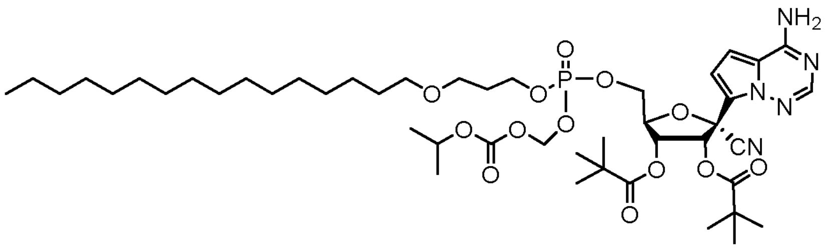

,

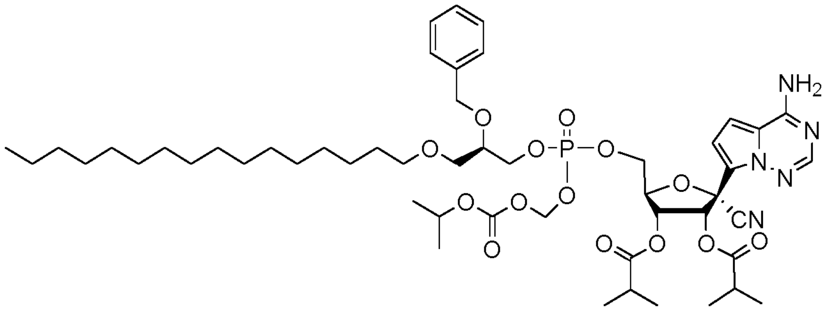

,

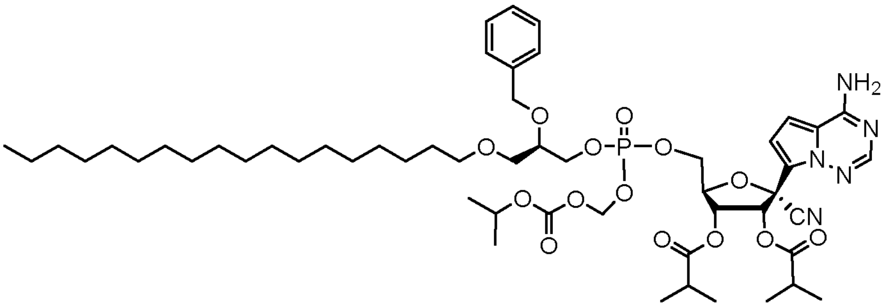

,

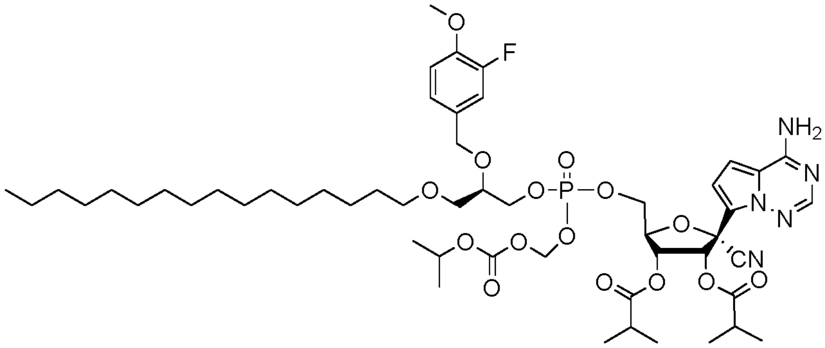

,

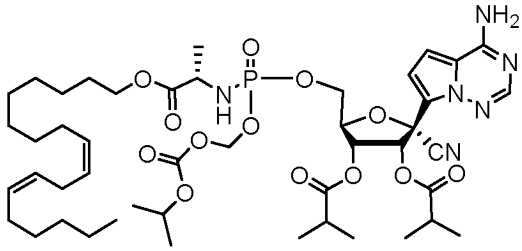,

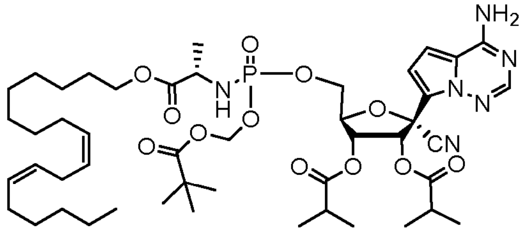,

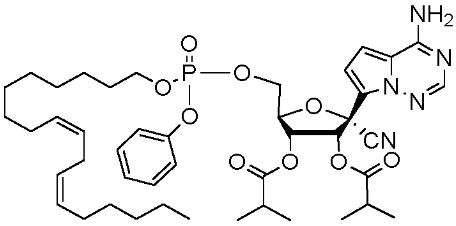,

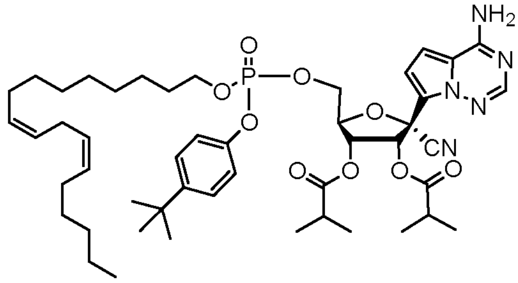,

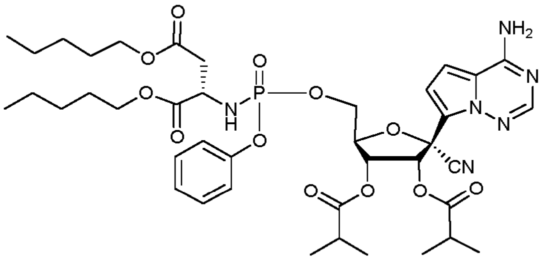,

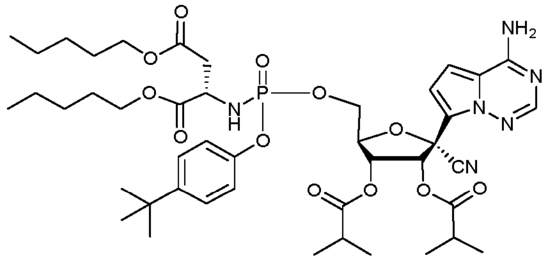,

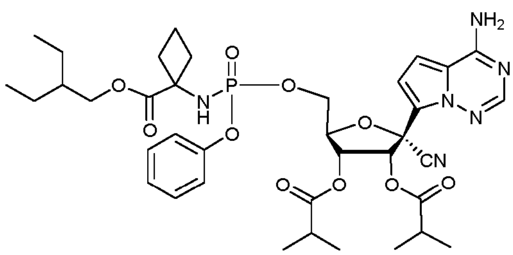,

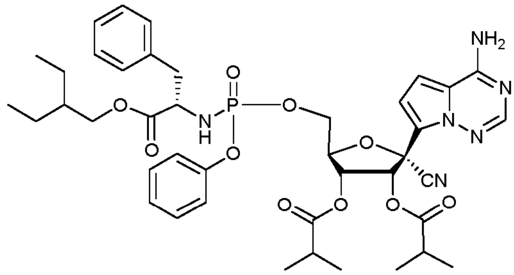,

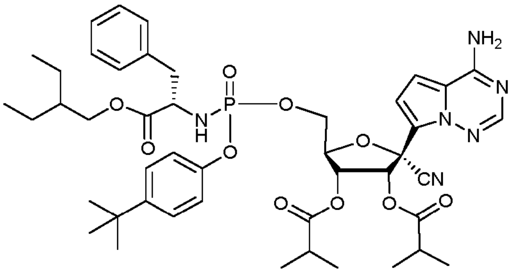,

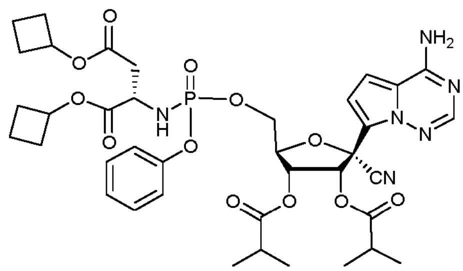,

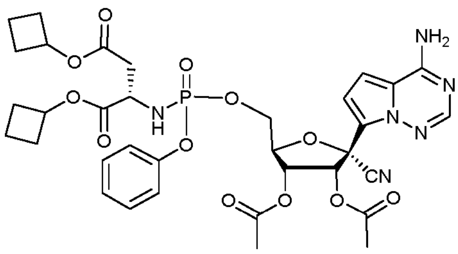,
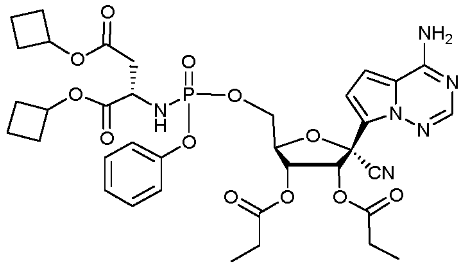,
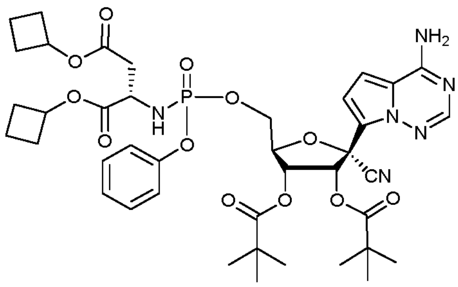,
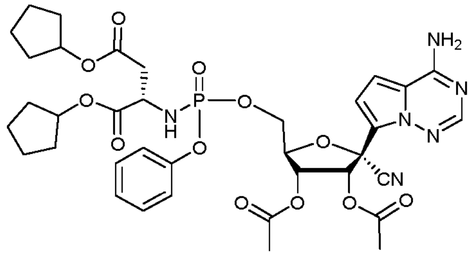,
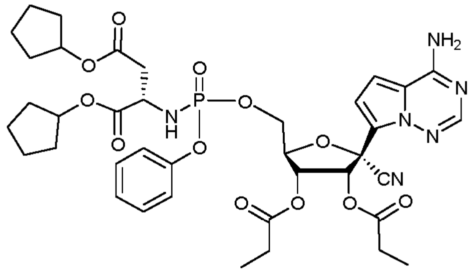,
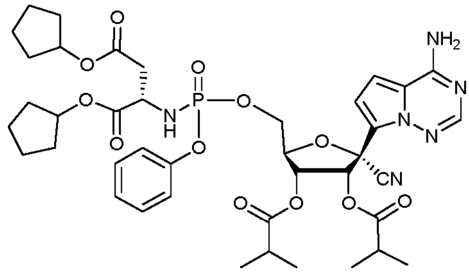,
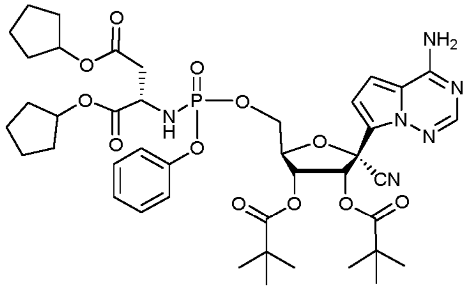,
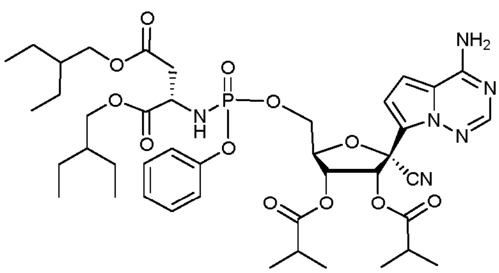,
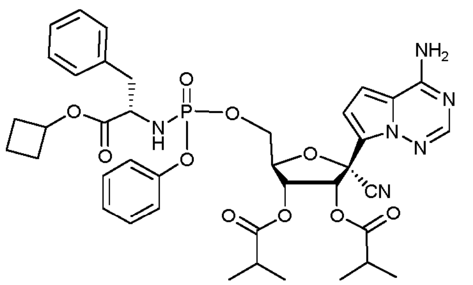 or
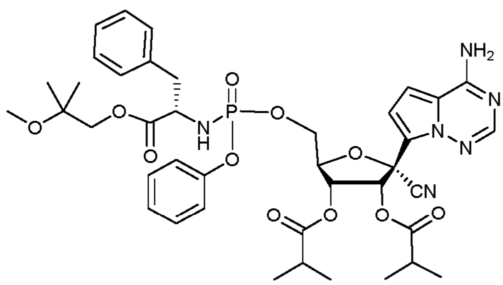;

preferably, the compound is selected from:

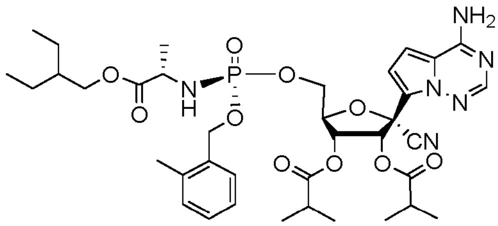
,
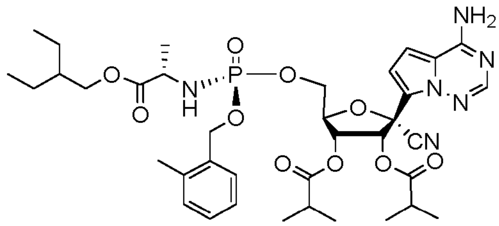
,

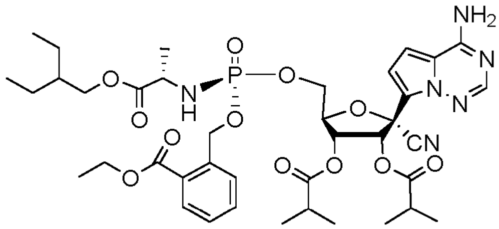
,
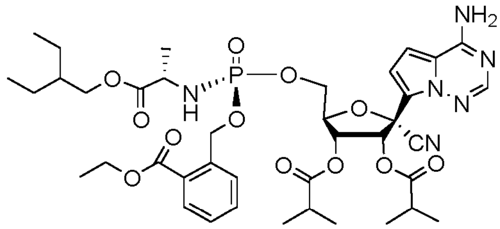
,

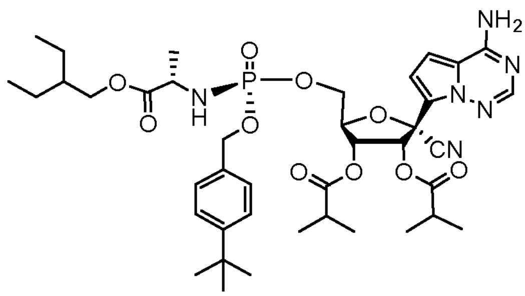
,
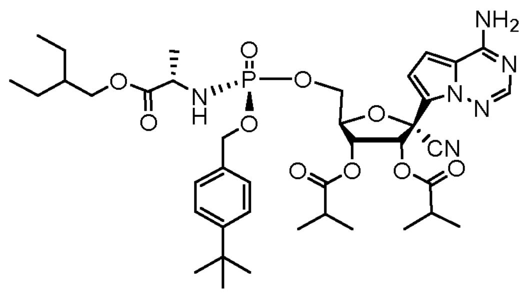
,

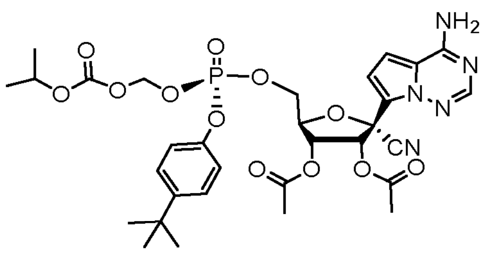
,
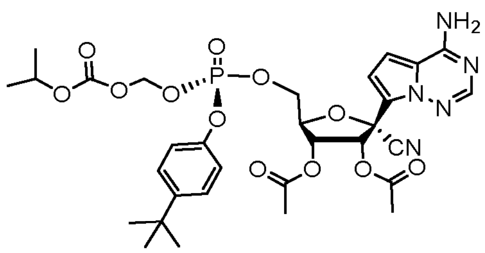
,

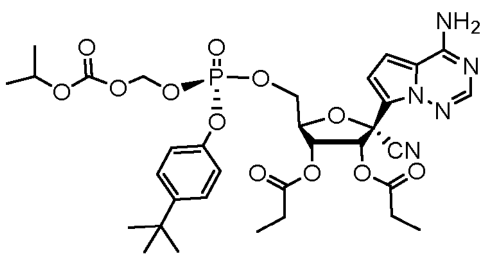
,
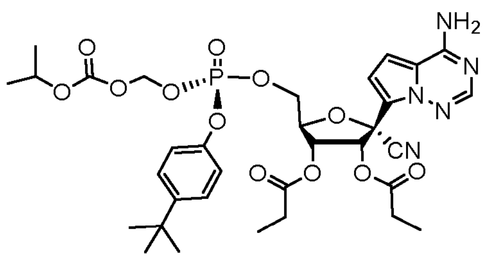
,

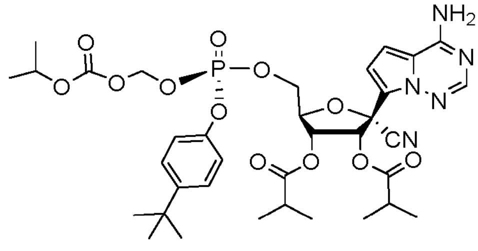
,
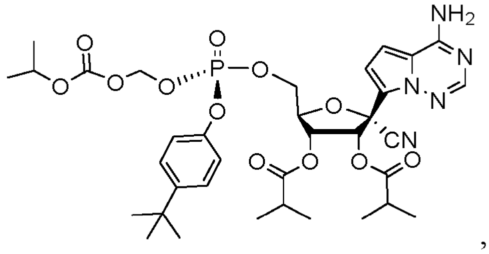
,

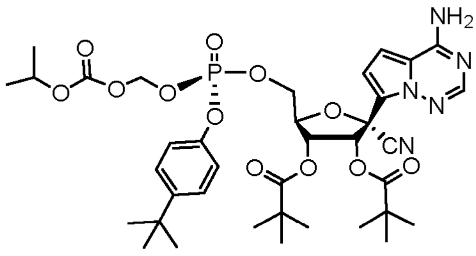
,
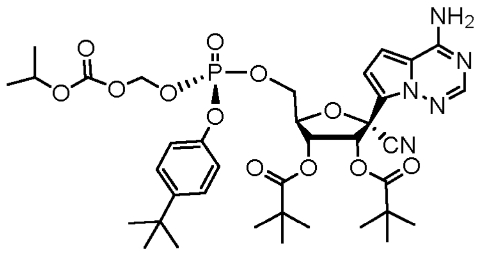
,

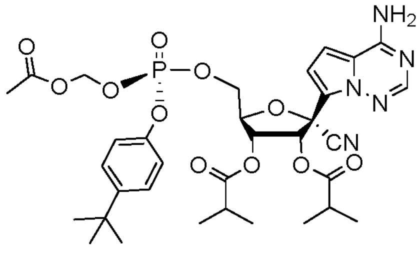
,
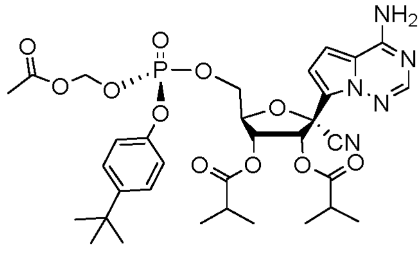
,

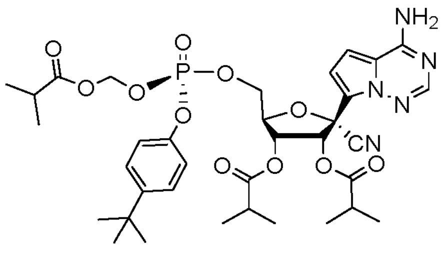
,
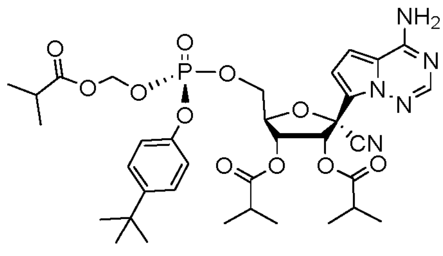
,

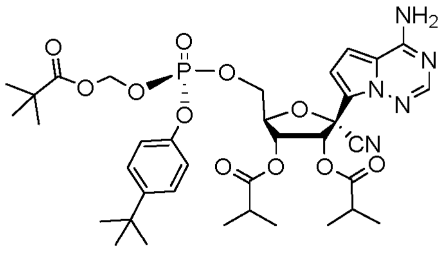
,
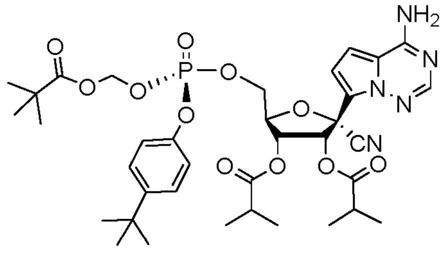
,

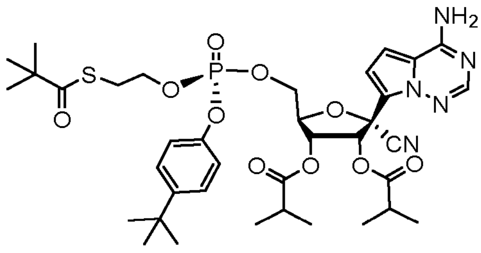
,
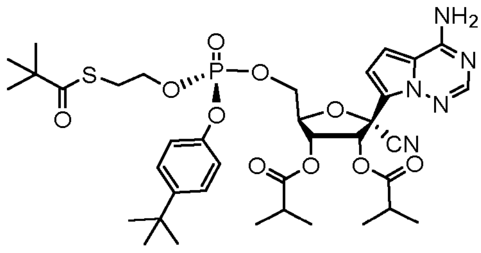
,

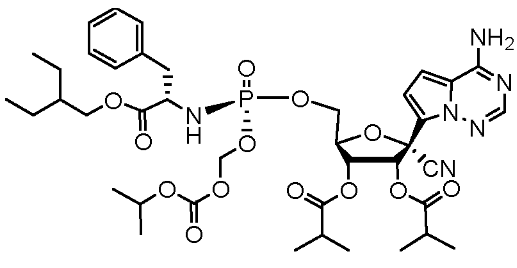
,
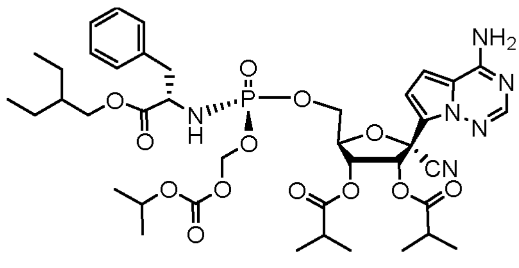
,

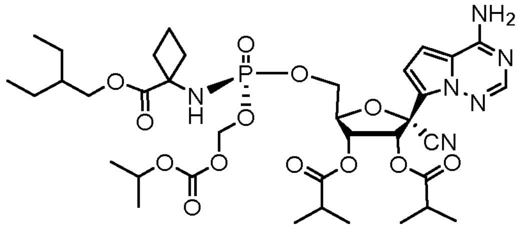,
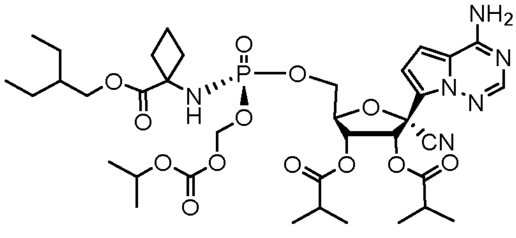,

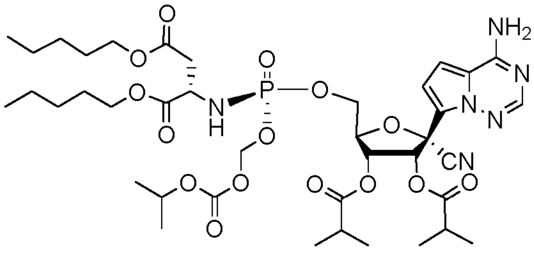,
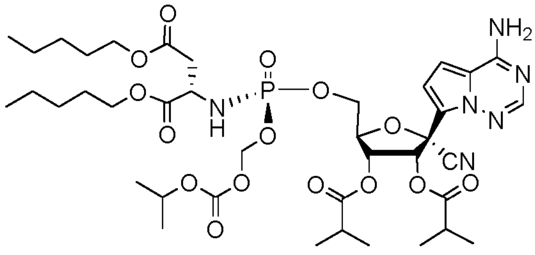,

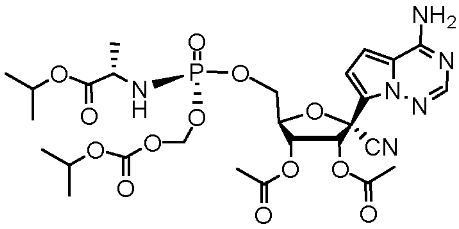,
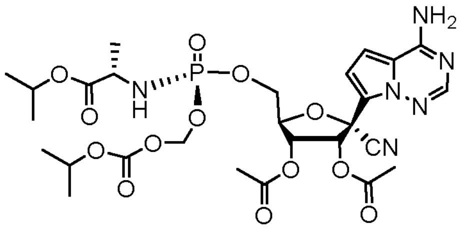,

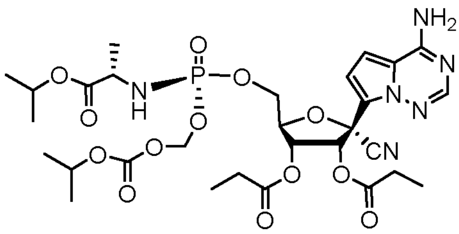,
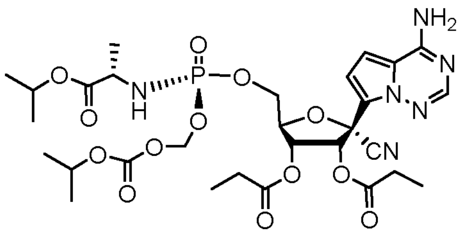,

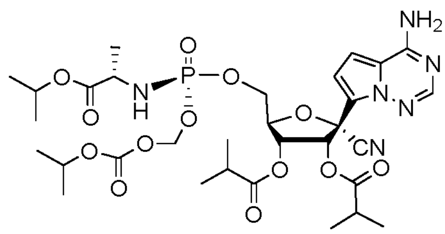,
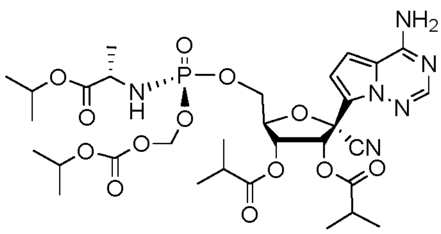,

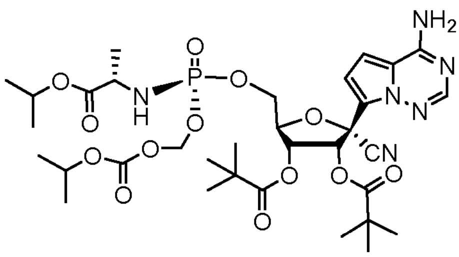,
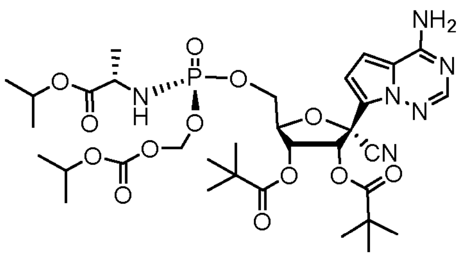,

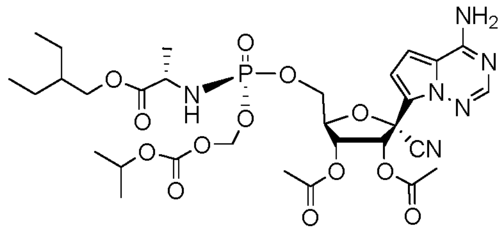,
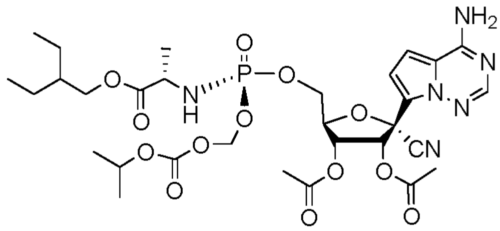,

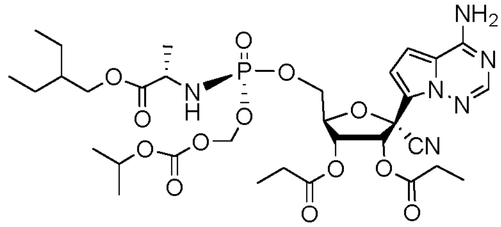,
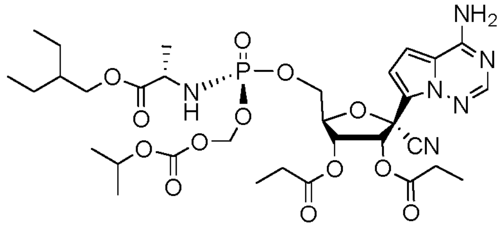,

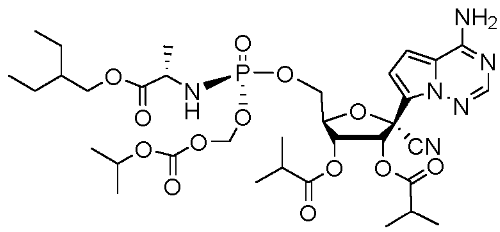,
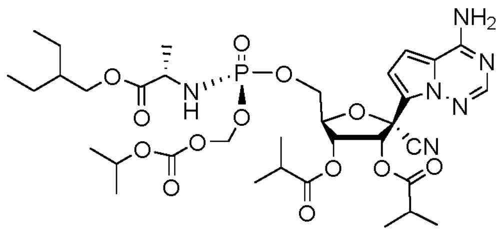,

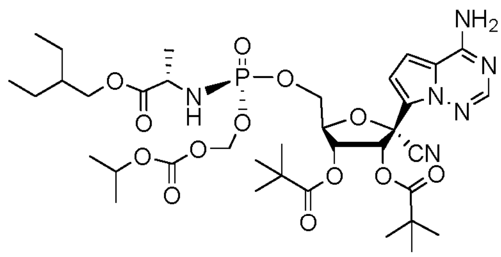,
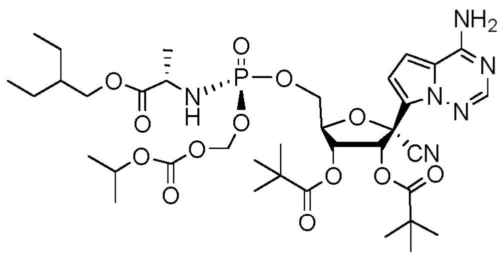,

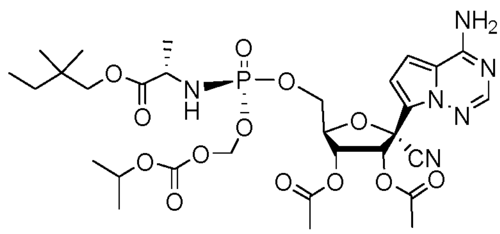,
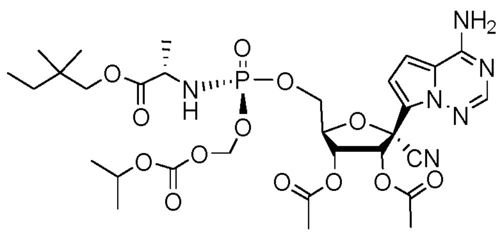,

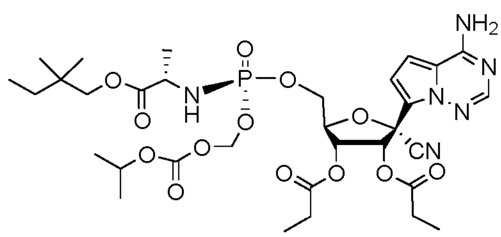,
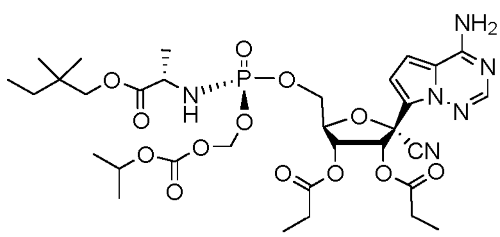,

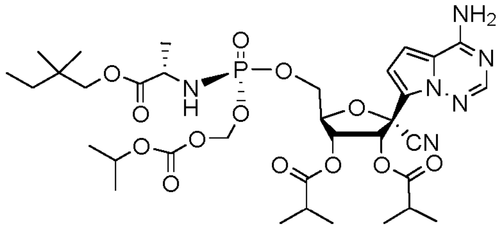
,
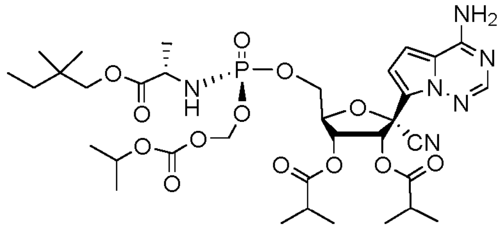
,

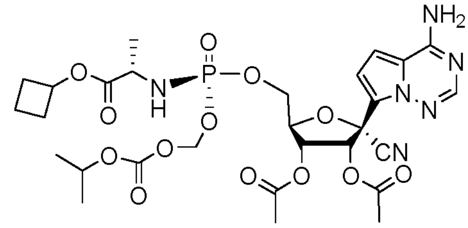
,
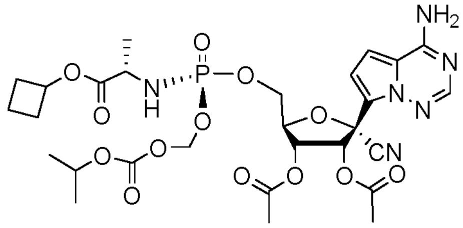
,

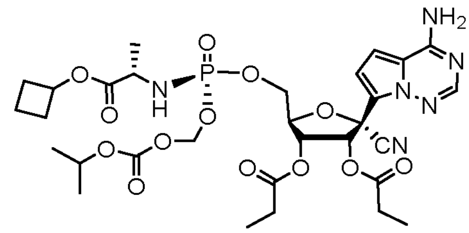
,
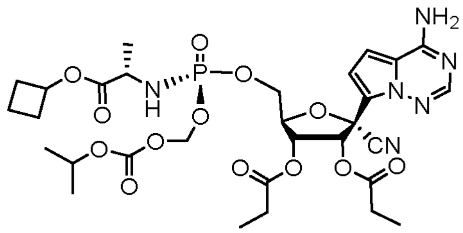
,

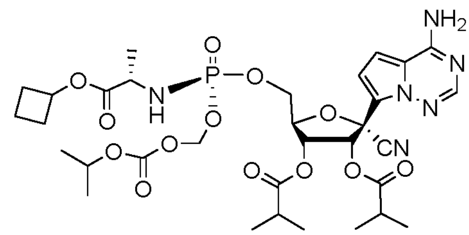
,
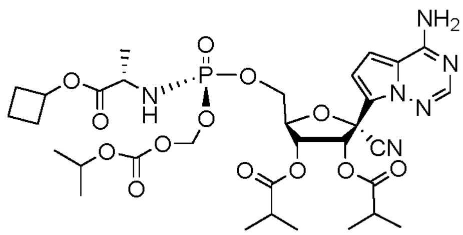
,

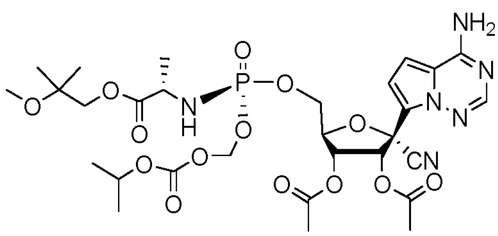
,
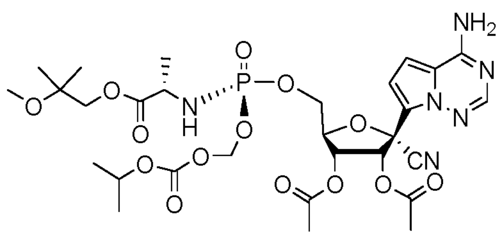
,

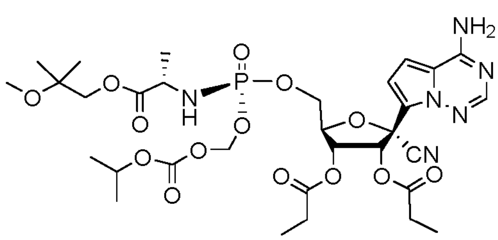
,
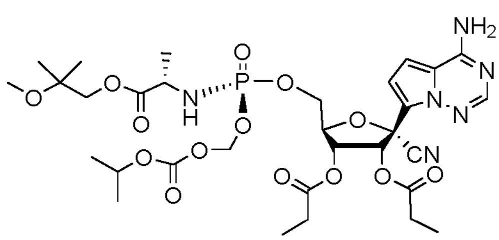
,

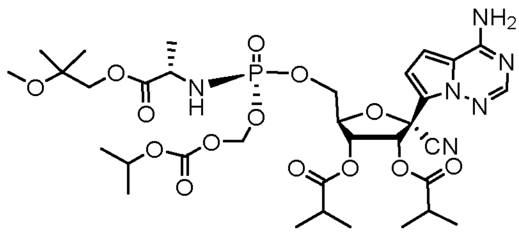,
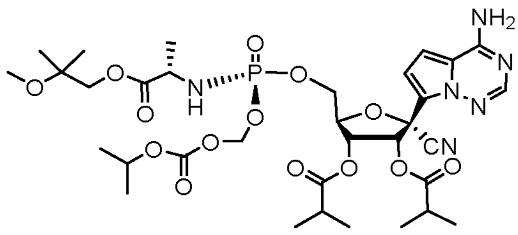,

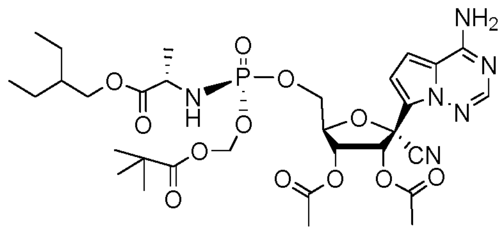,
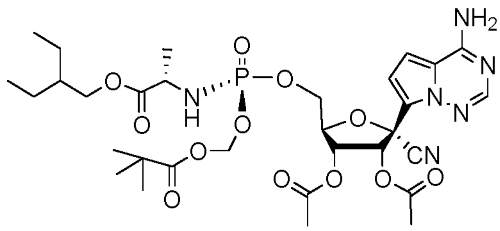,

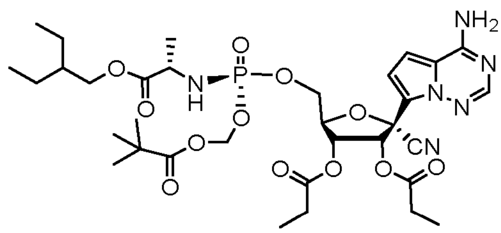,
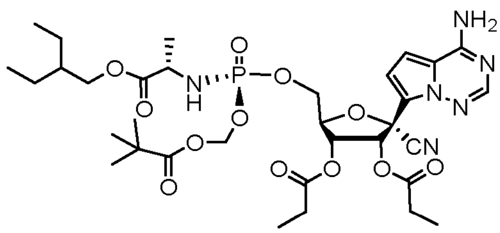,

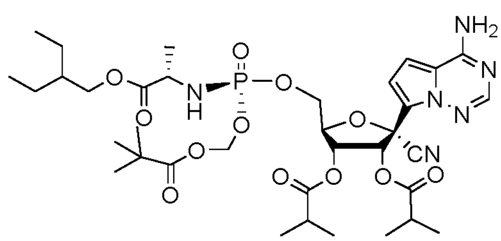,
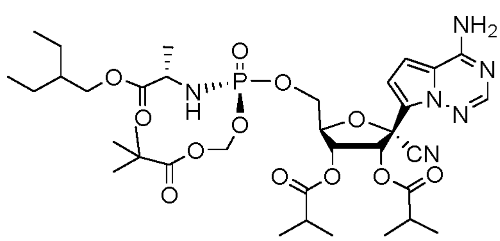,

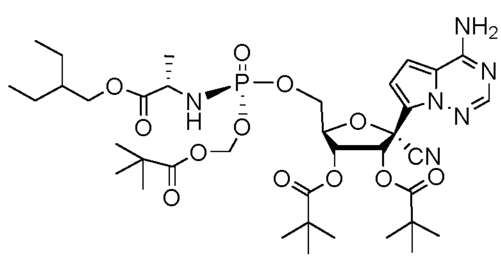,
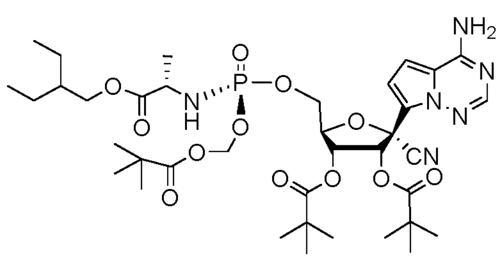,

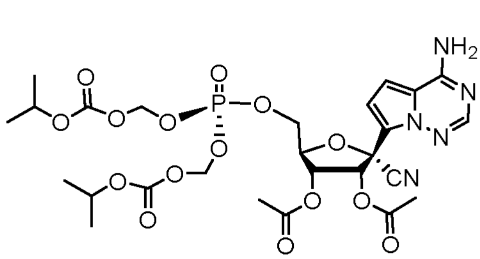,
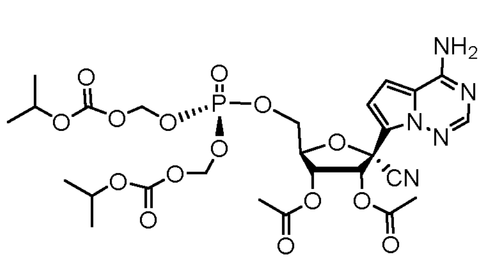,

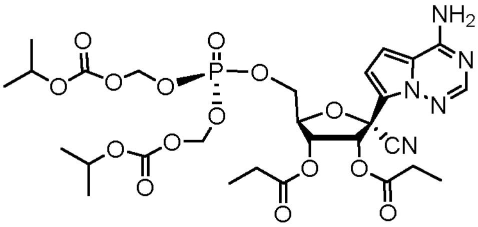,
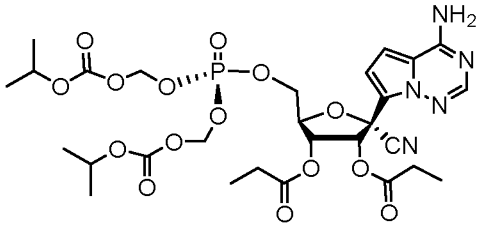,
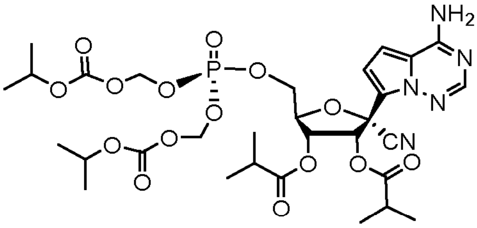,
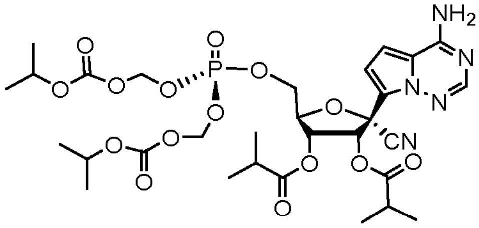,
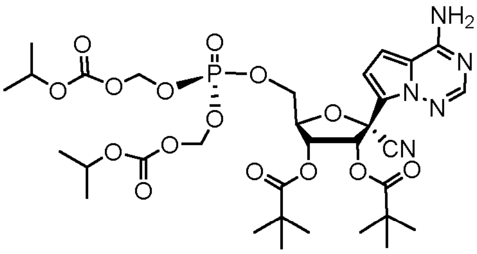,
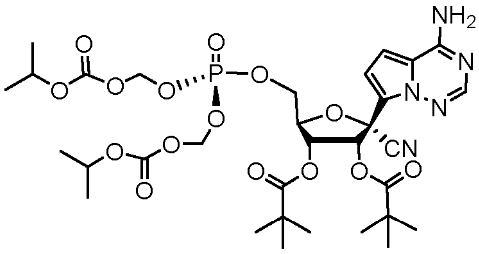,
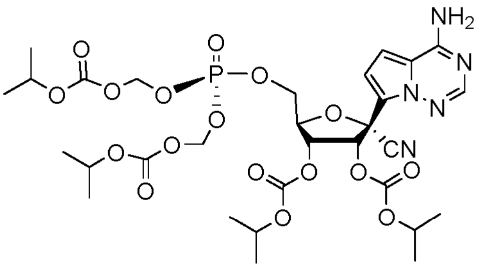,
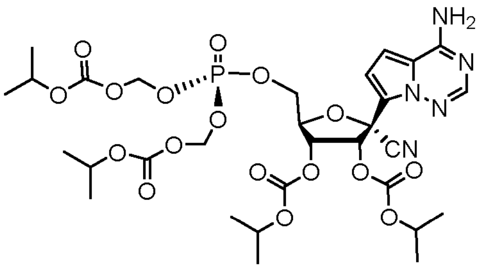,
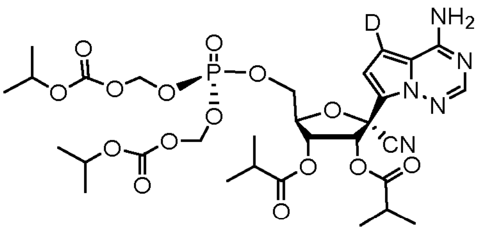,
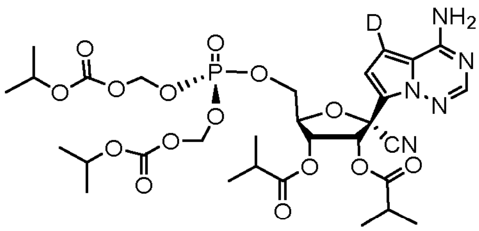,
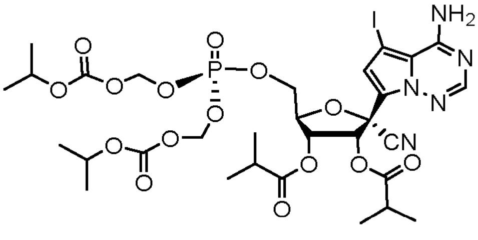,
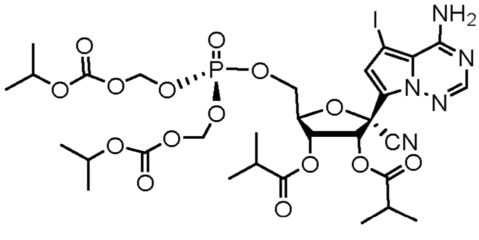,

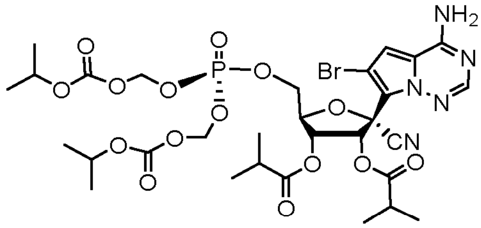,

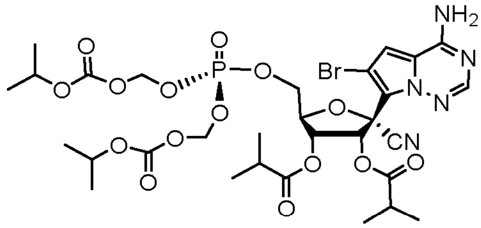,

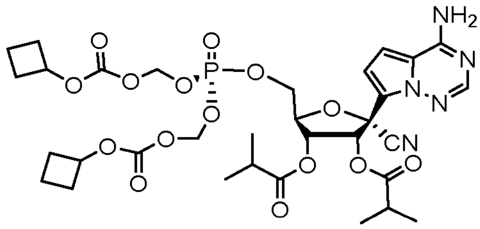,

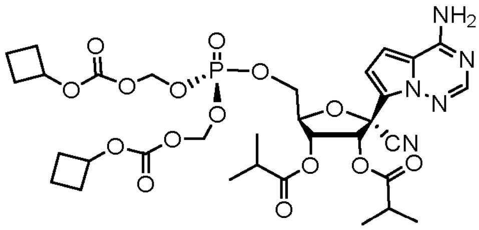,

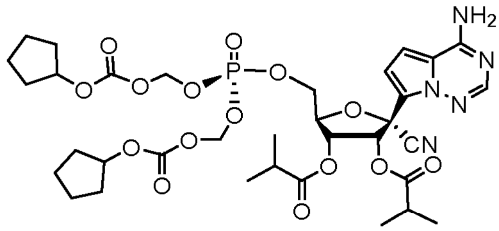,

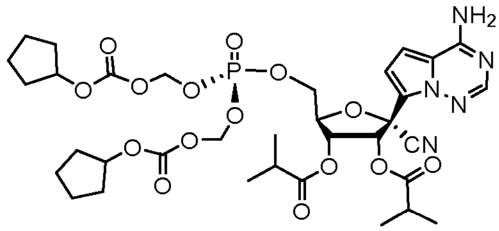,

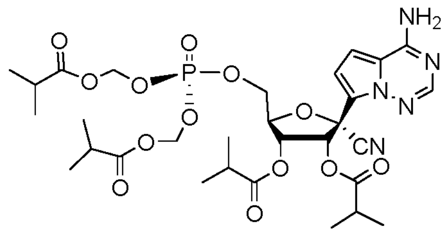,

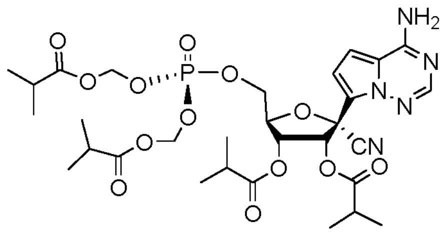,

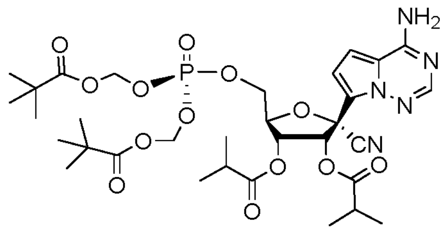,

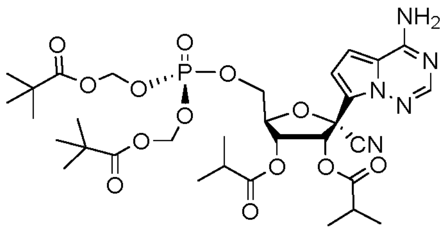,

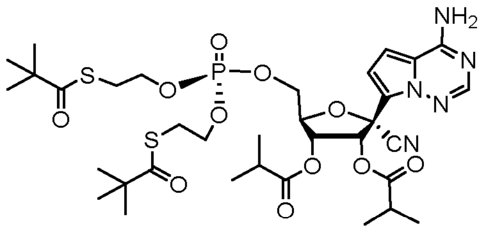,

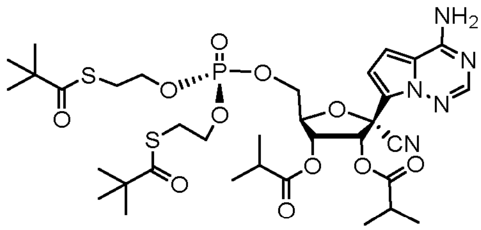,

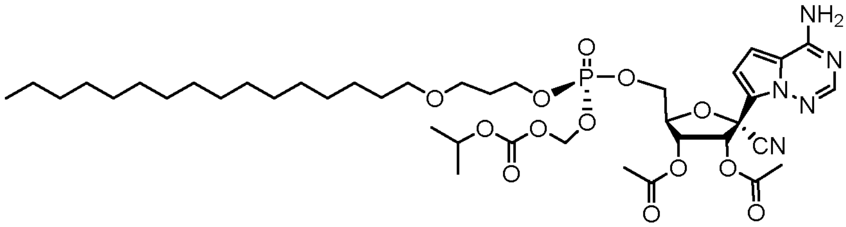

,

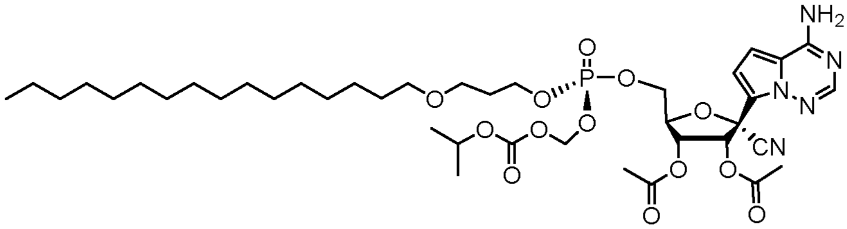

,

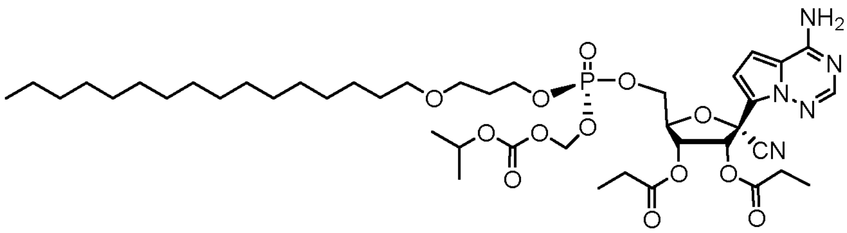

,

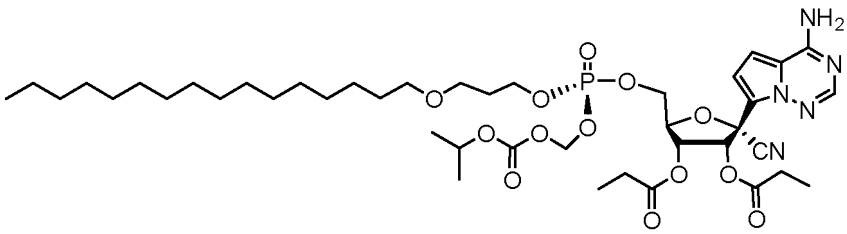

,

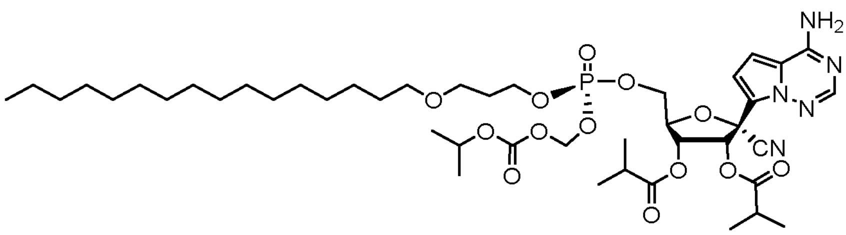

,

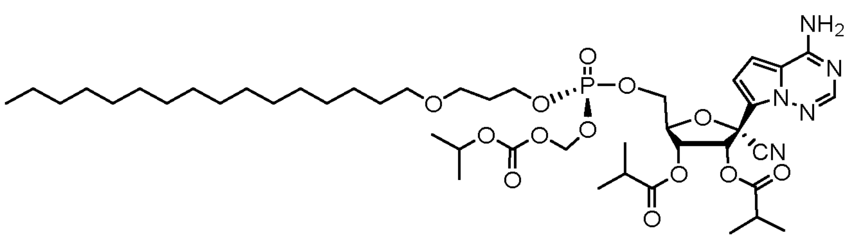

,

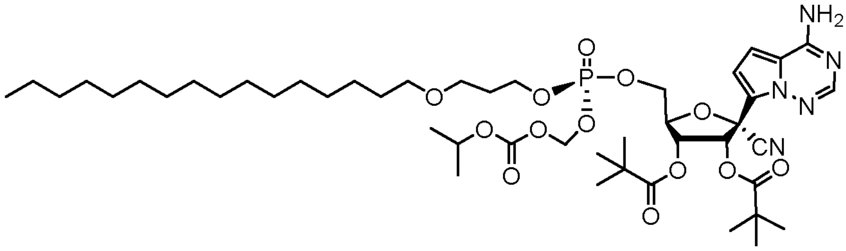

,

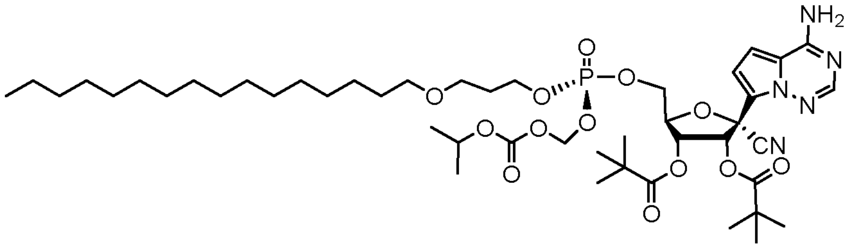

,

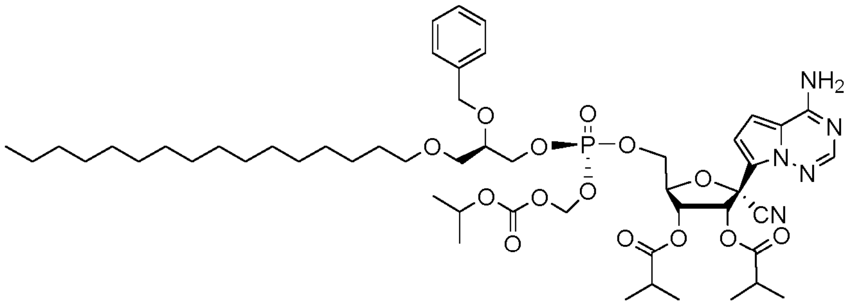

,

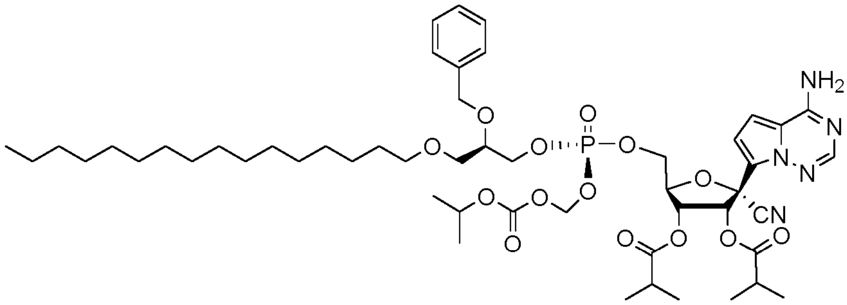

,

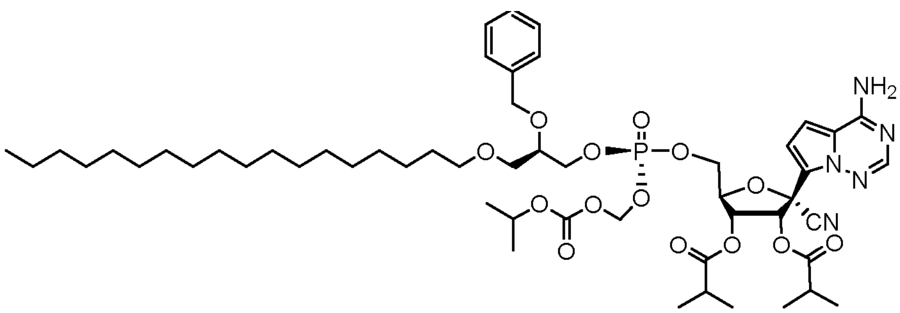

,

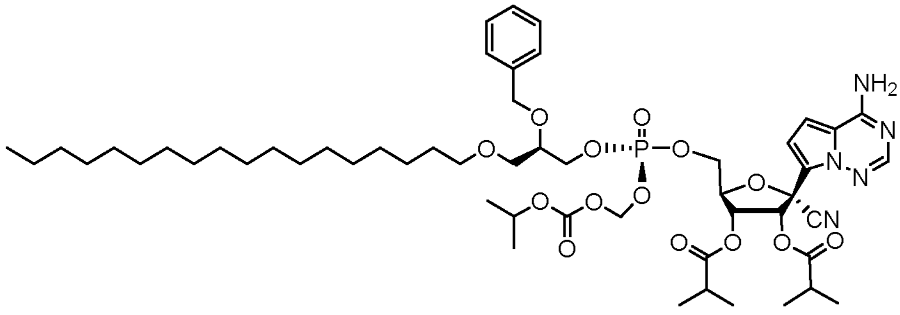

,

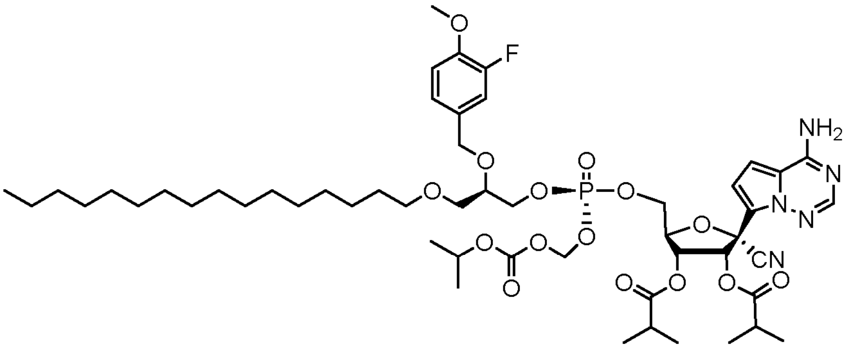

,

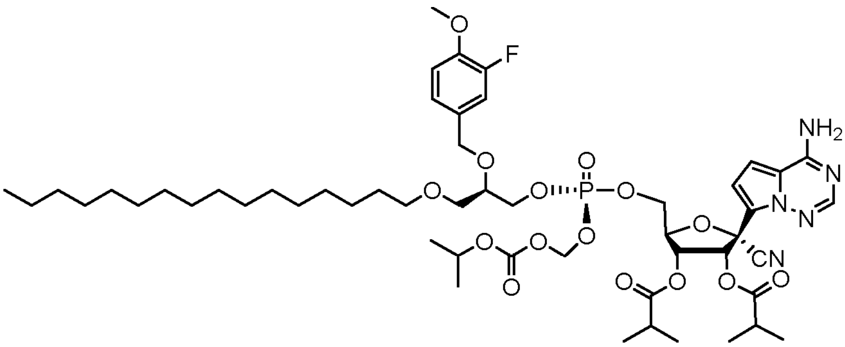

,

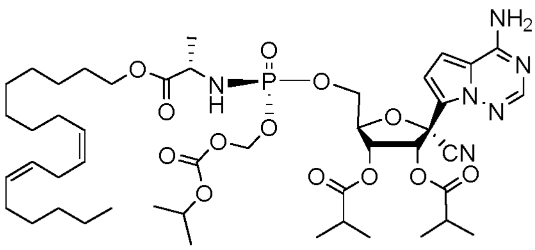

,

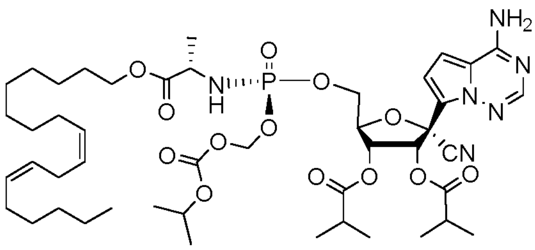

,

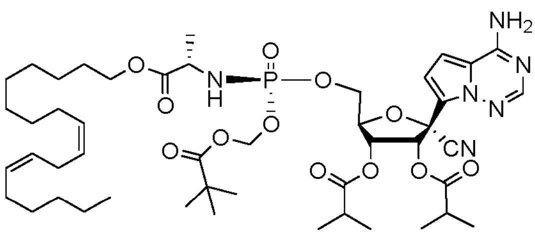

,

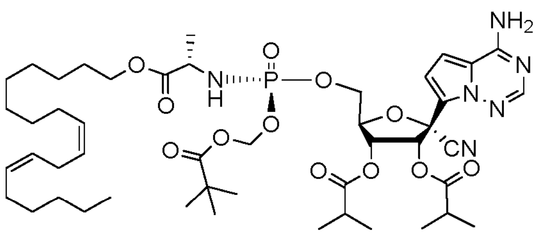

,

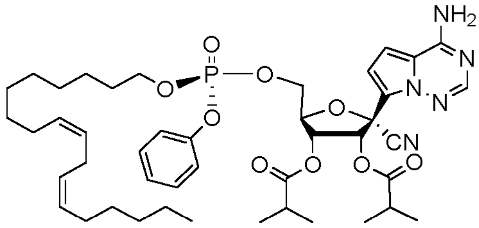,

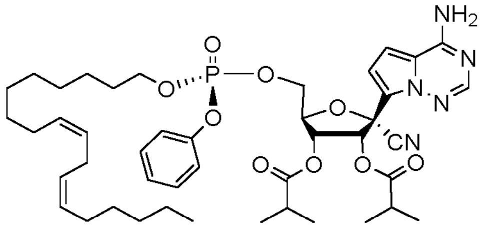,

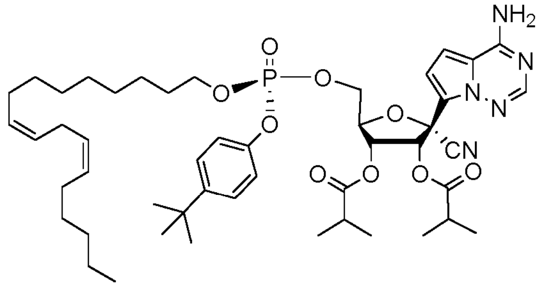,

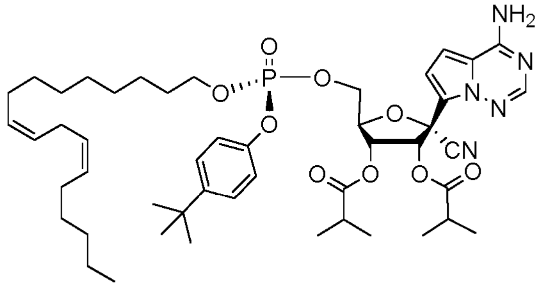,

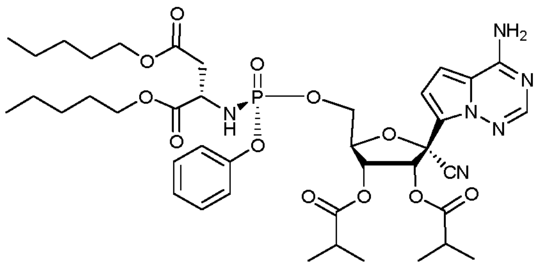,

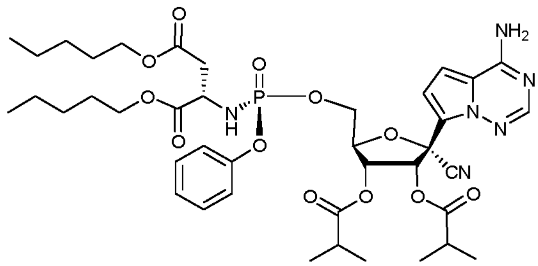,

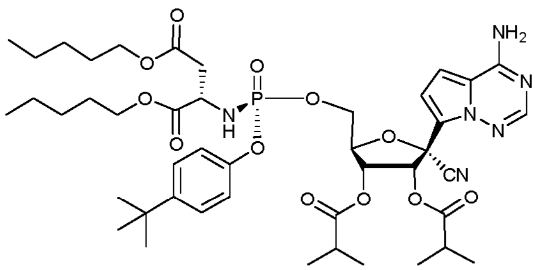,

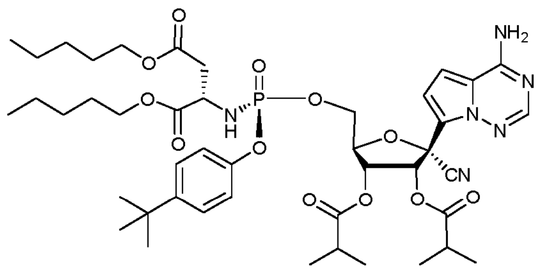,

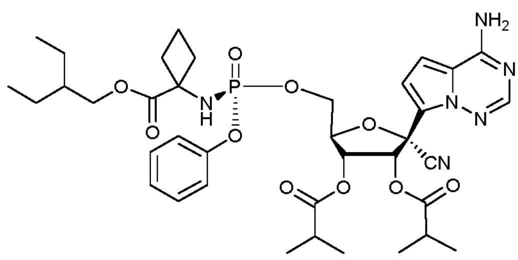,

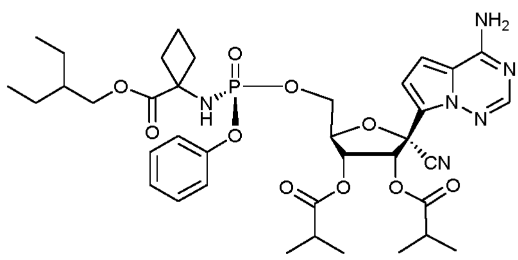,

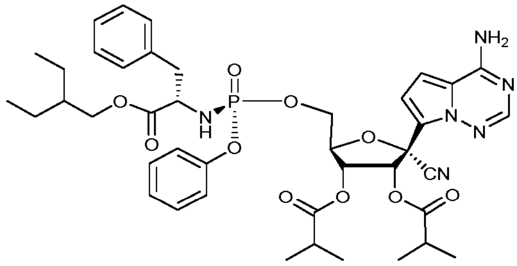,

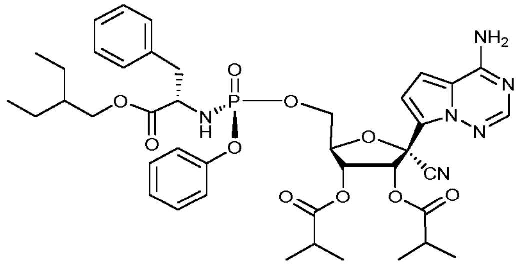,

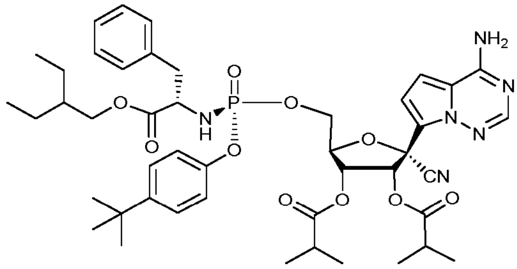,

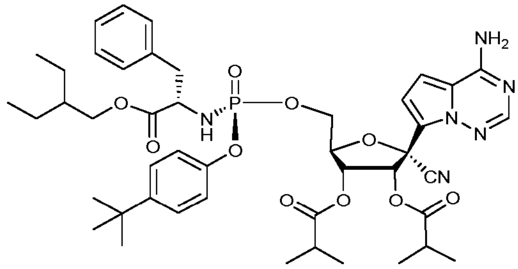,

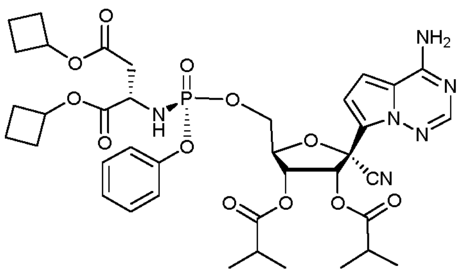,

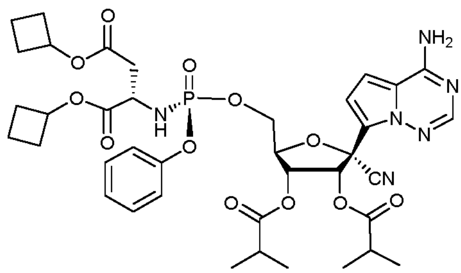,

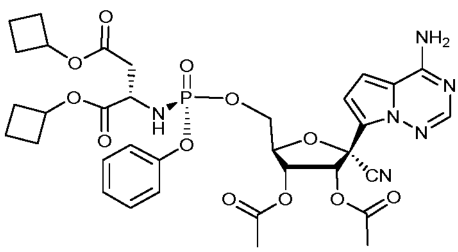,

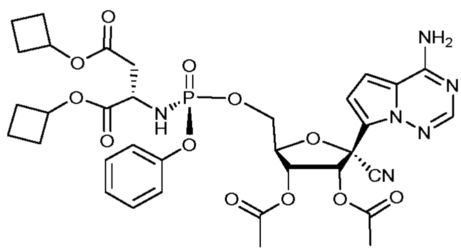,

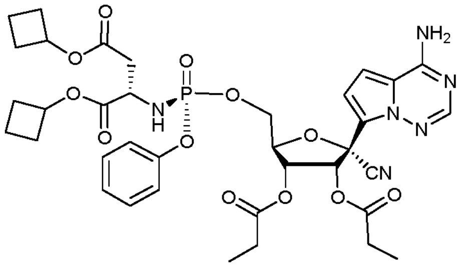,

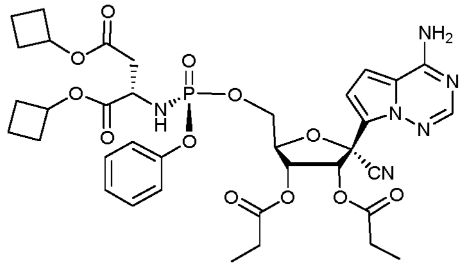,

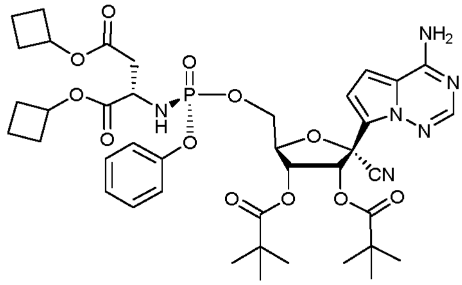,

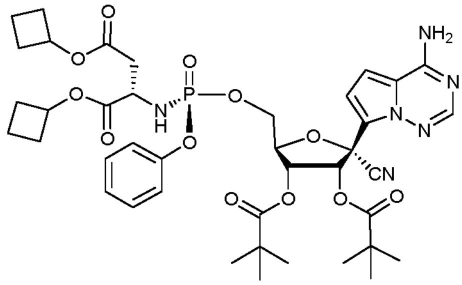,

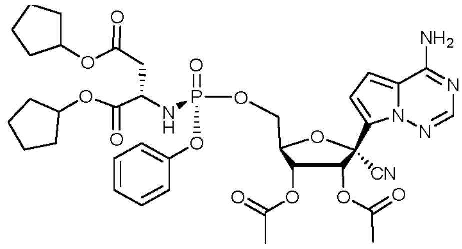,

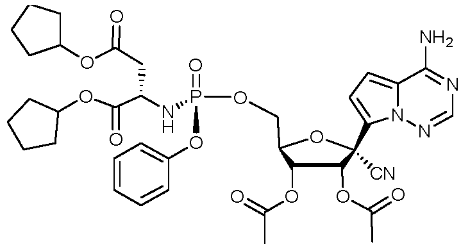,

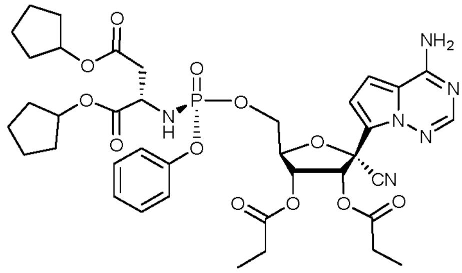,

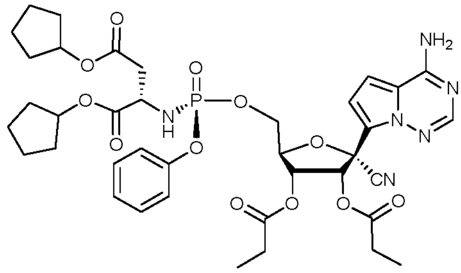,

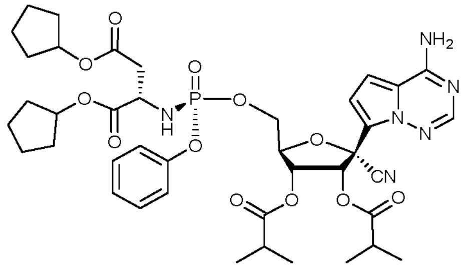,

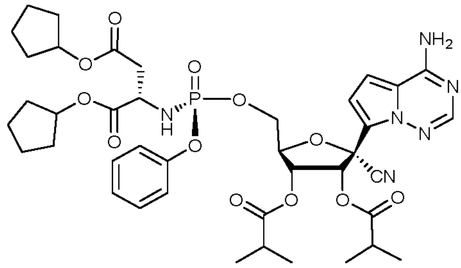,

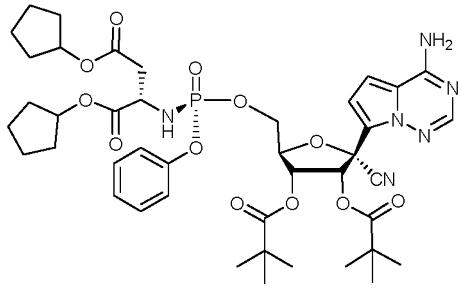,

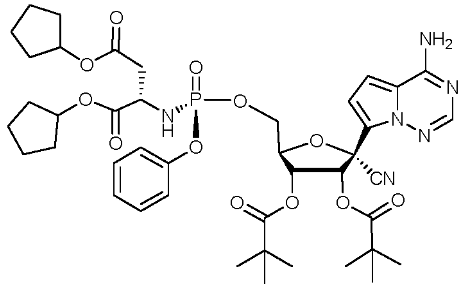,

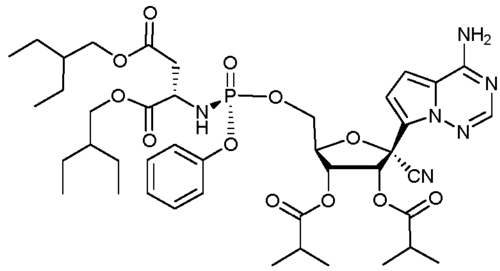, 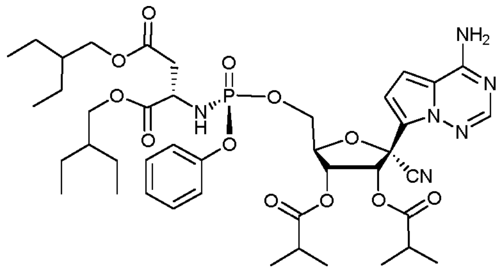,

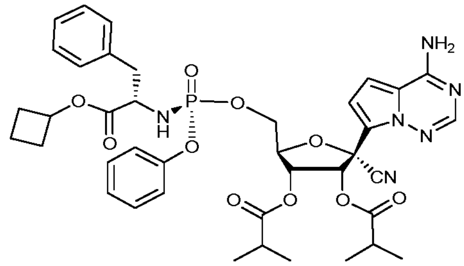, 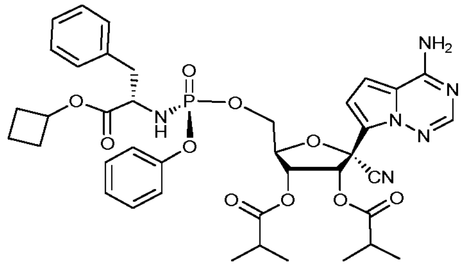,

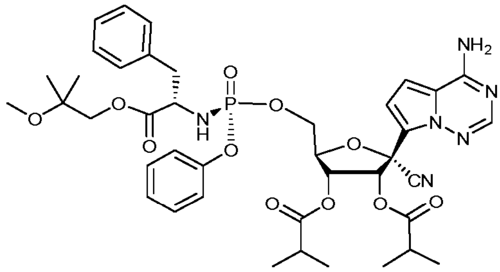 or 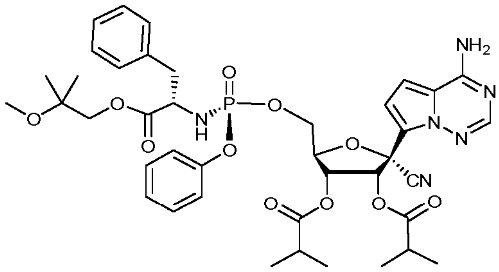.

An intermediate compound, or a tautomer, stereoisomer, prodrug, crystalline form, pharmaceutically acceptable salt, hydrate or solvate thereof, wherein, the intermediate compound is selected from the group consisting of:

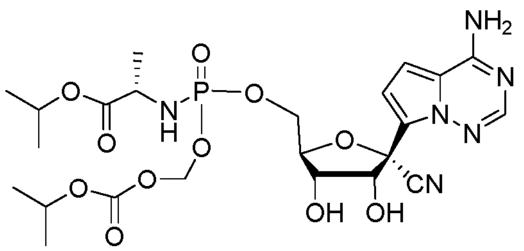, 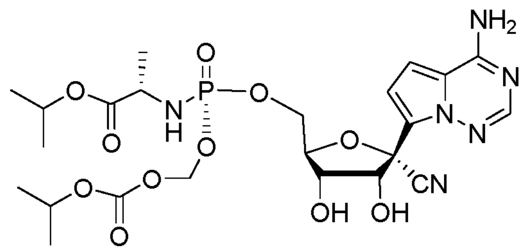,

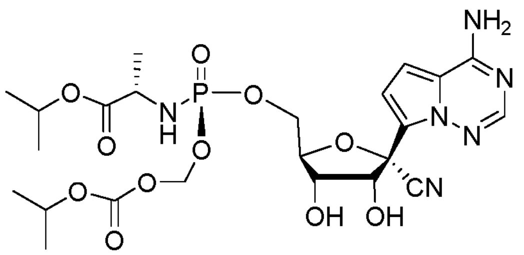, 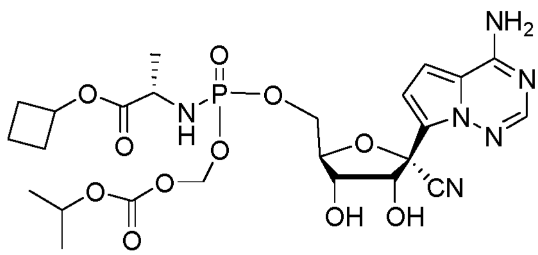,

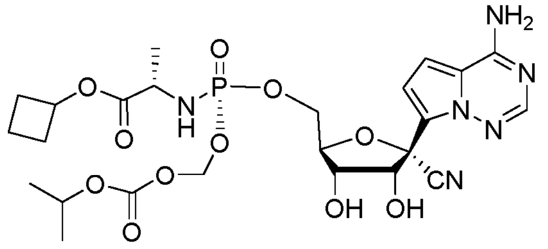
,

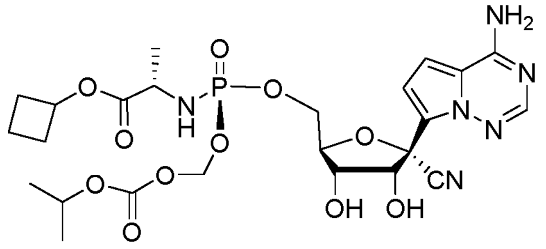
,

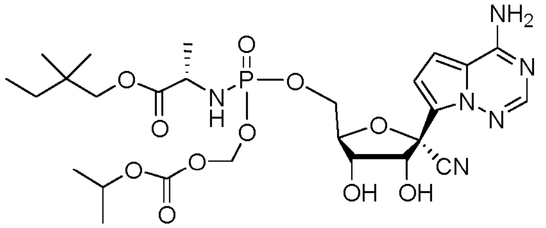
,

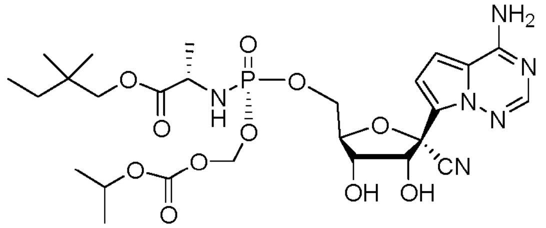
,

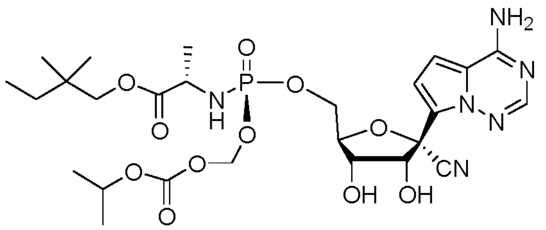
,

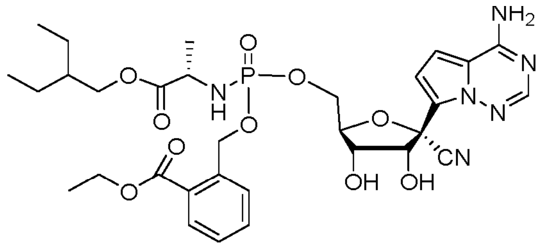
,

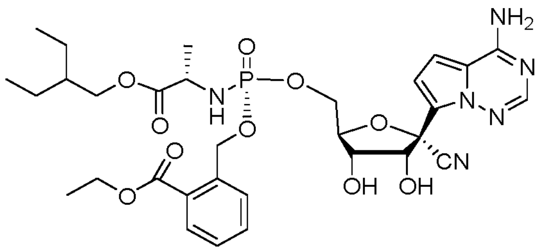
,

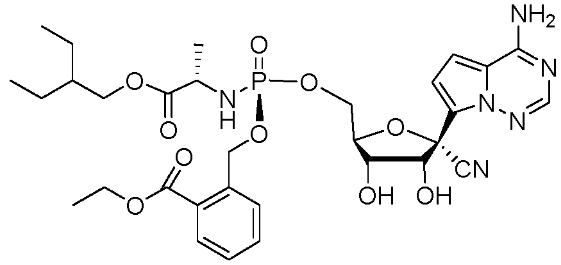
,

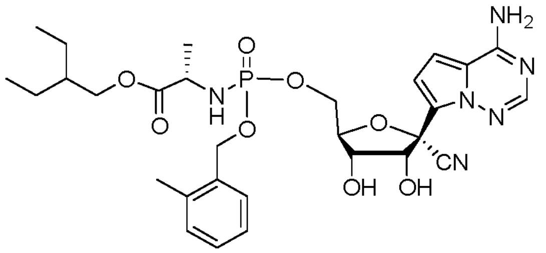
,

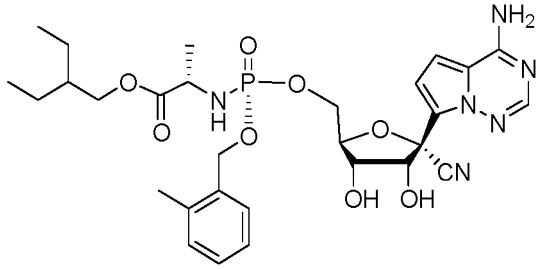
,

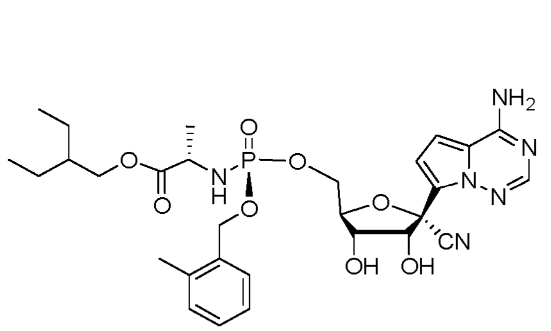

,

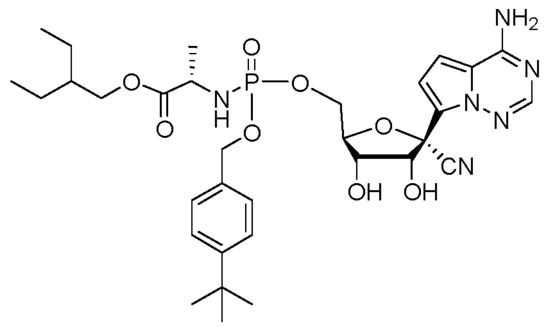

,

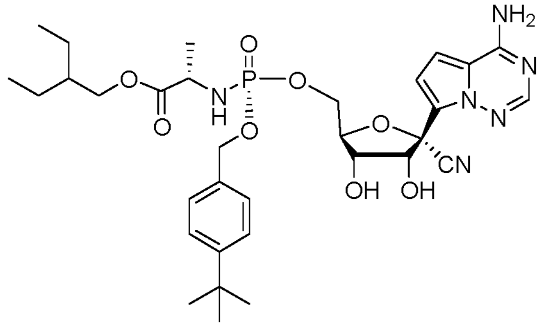

,

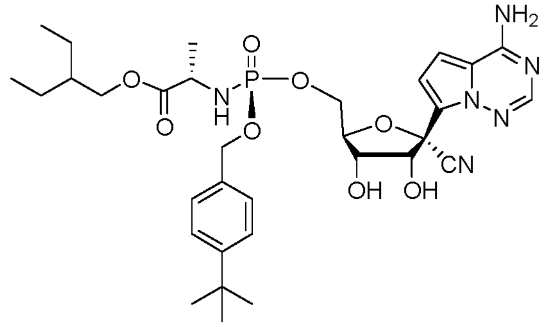

,

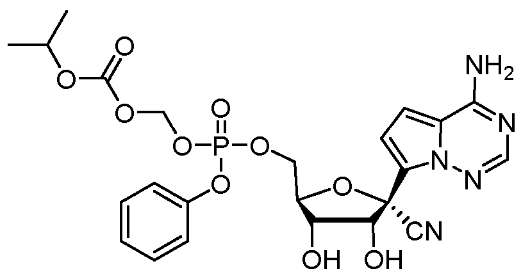

,

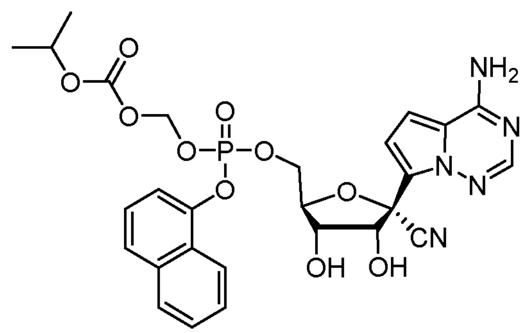

,

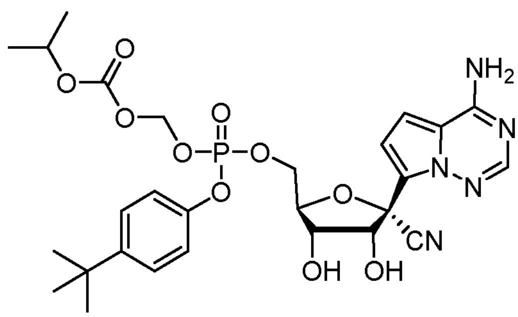

,

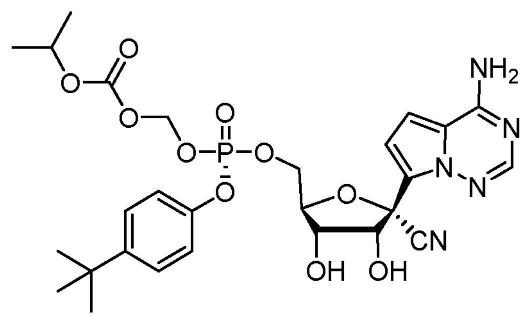

,

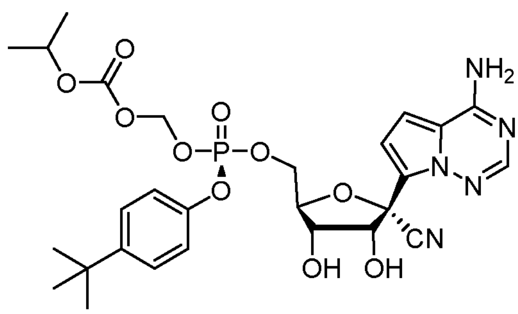

,

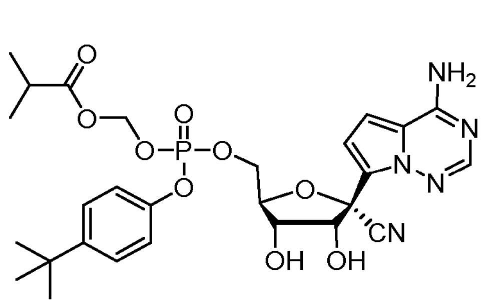

,

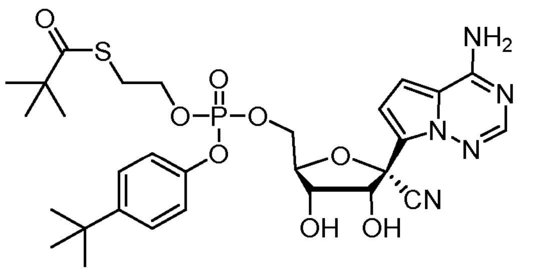

,

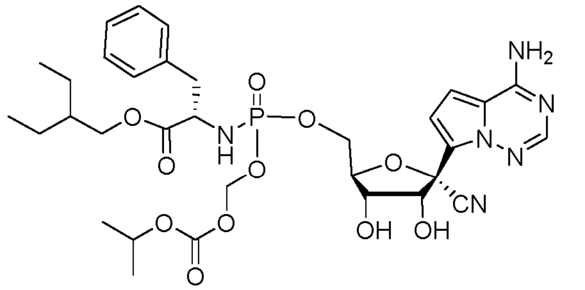

,

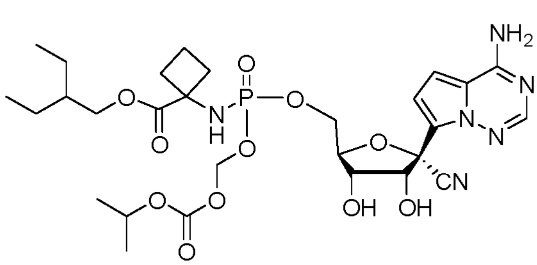

,

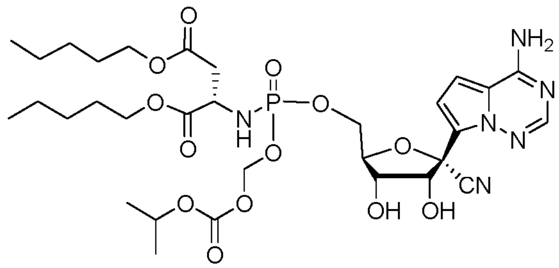

,

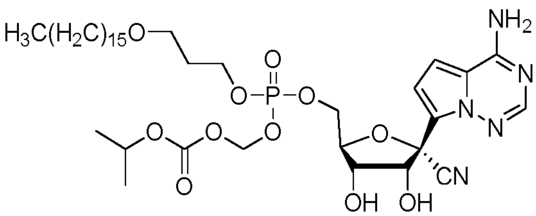

,

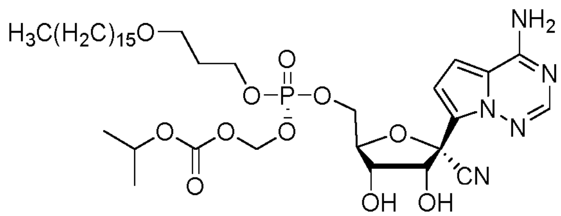

,

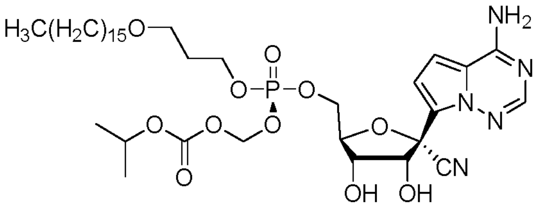

,

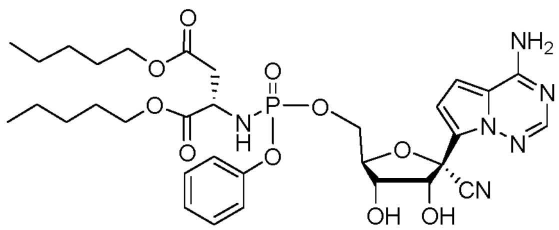

,

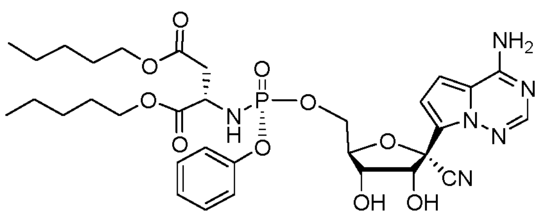

,

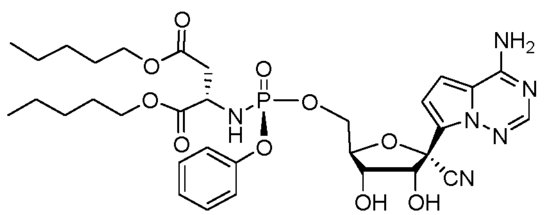

,

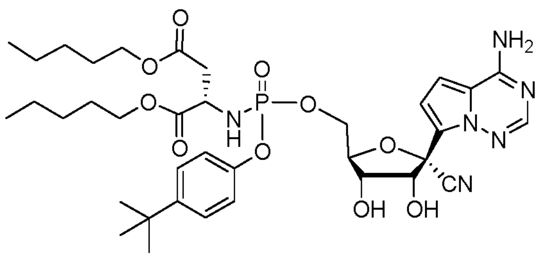

,

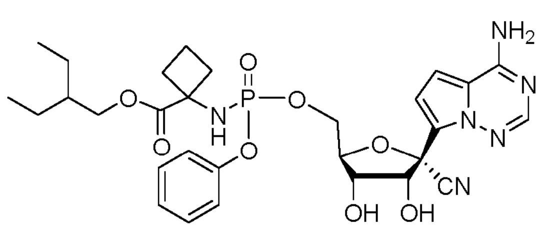

,

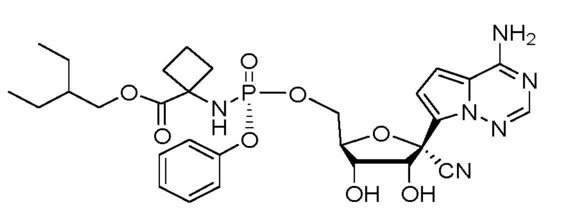
,
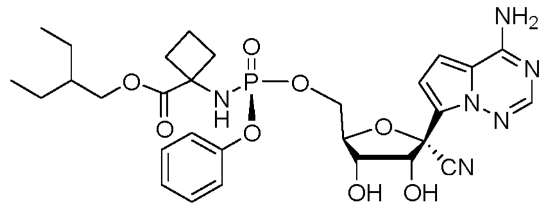
,
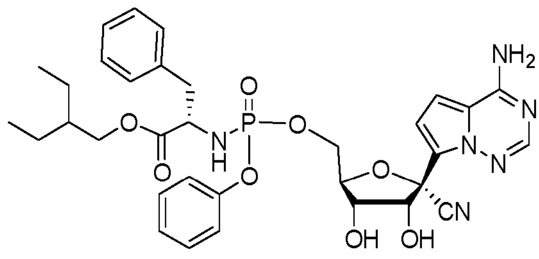
,
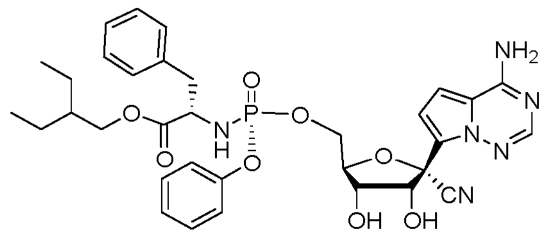
,
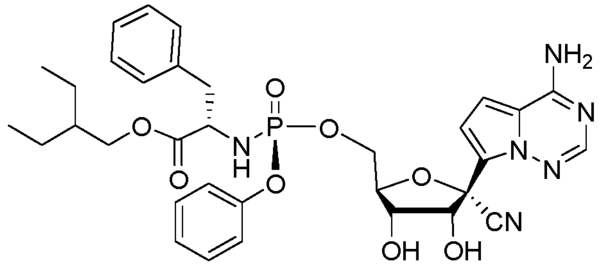
,
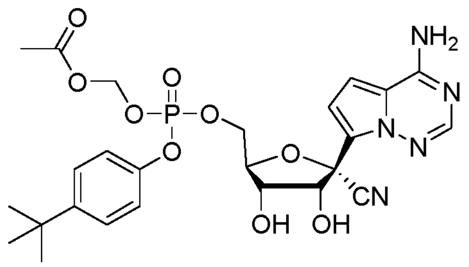
,
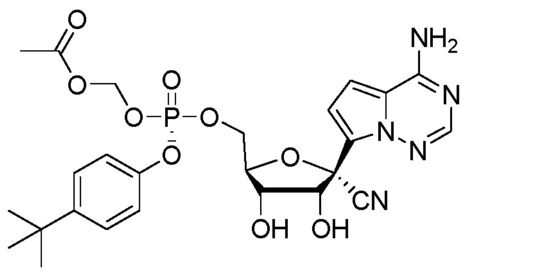
,
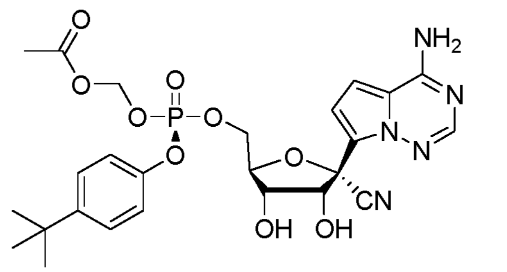
,
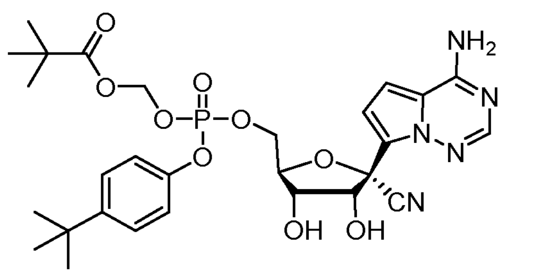
,
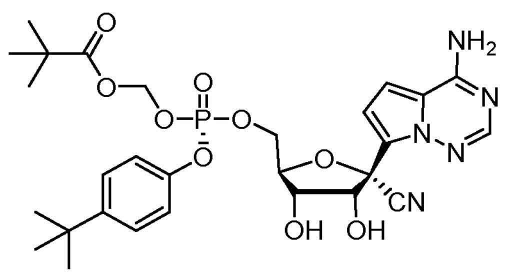
,
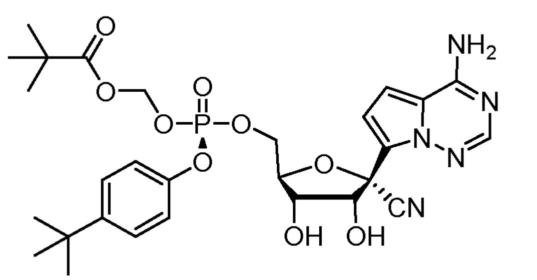
,
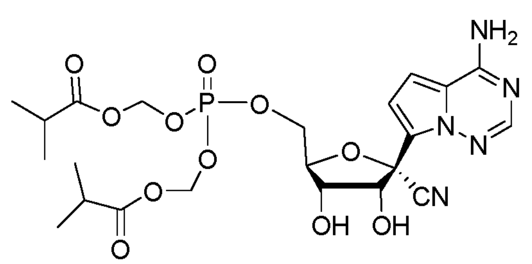
,

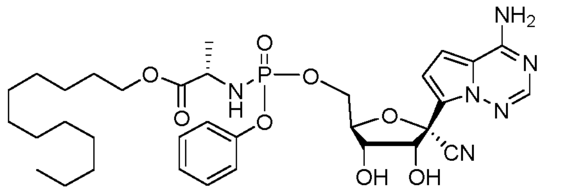
,
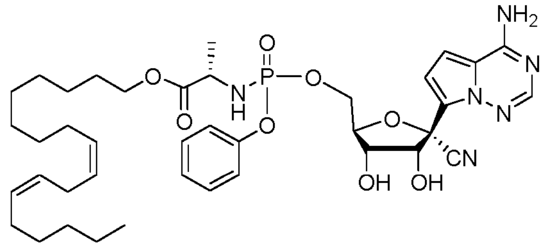
,
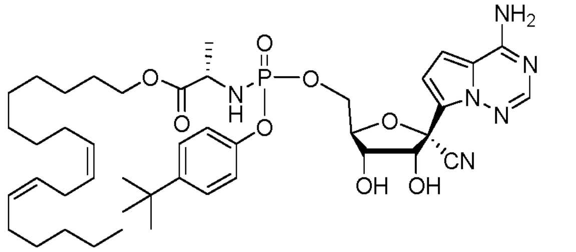
,
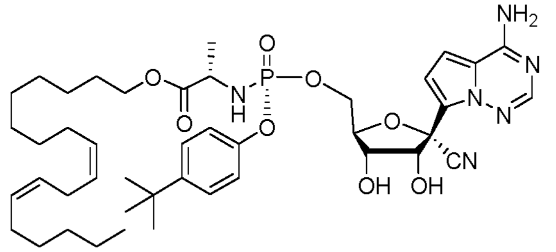
,
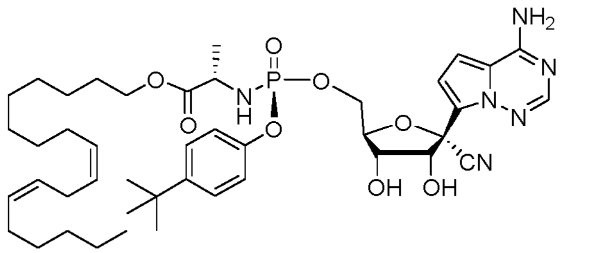
,
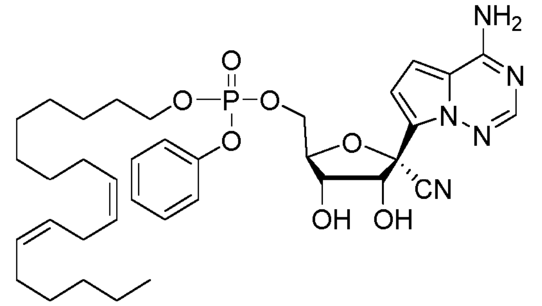
,
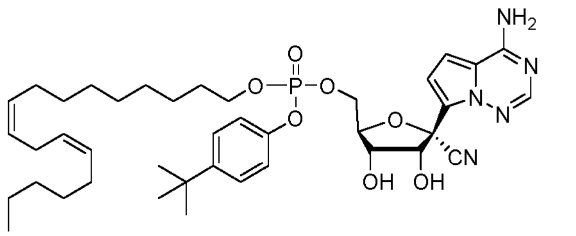
,
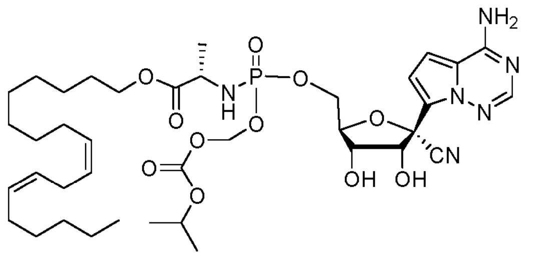
,
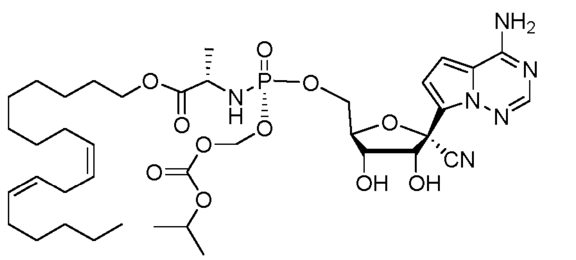
,
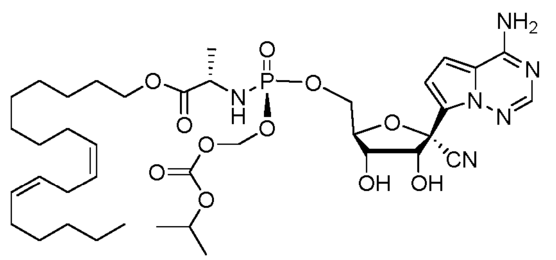
,

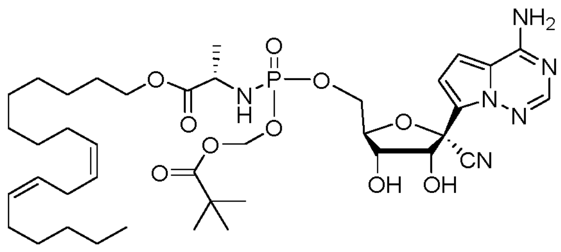

,

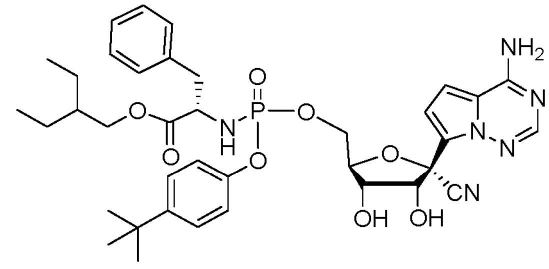

,

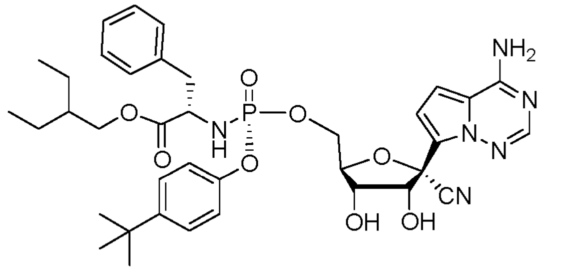

,

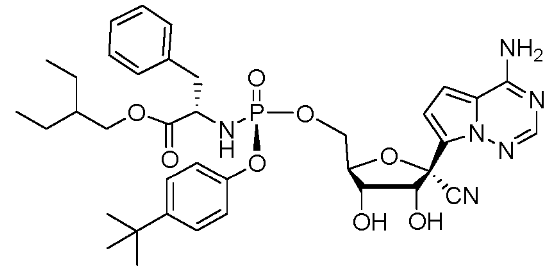

,

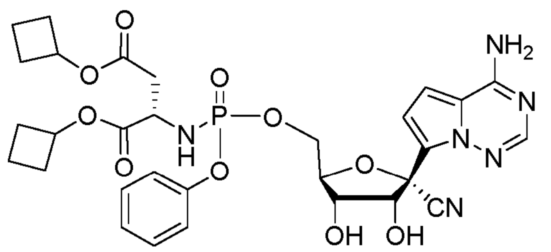

,

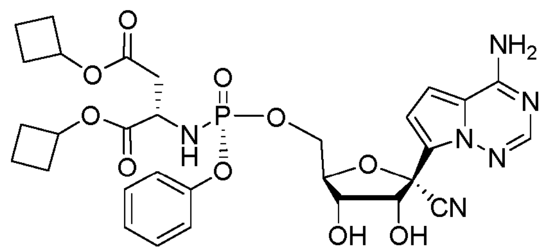

,

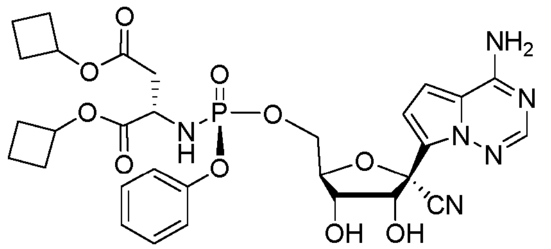

,

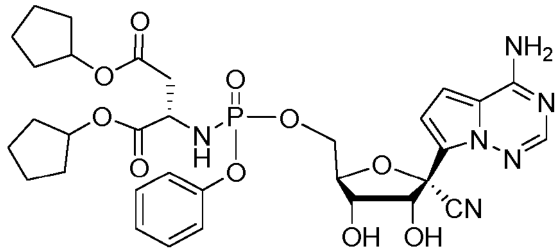

,

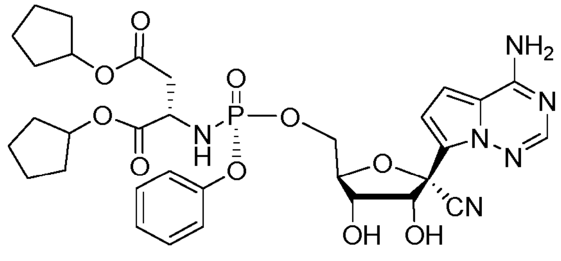

,

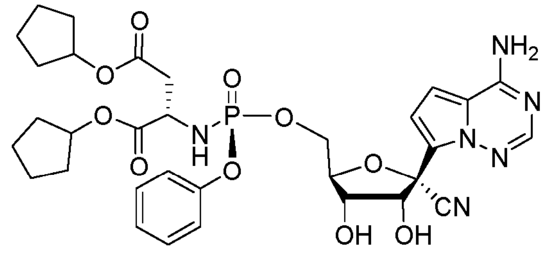

,

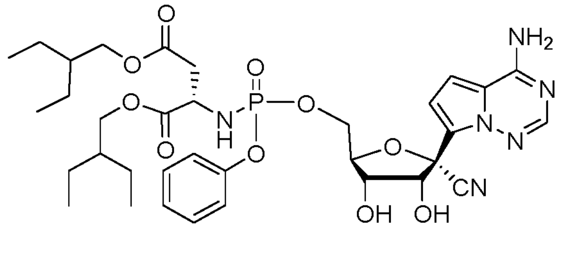

,

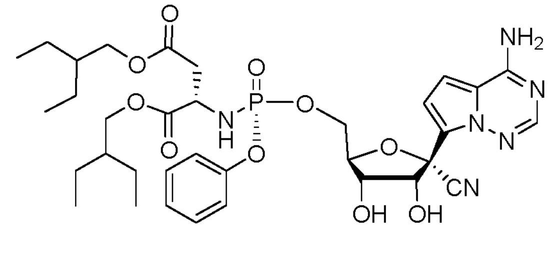

,

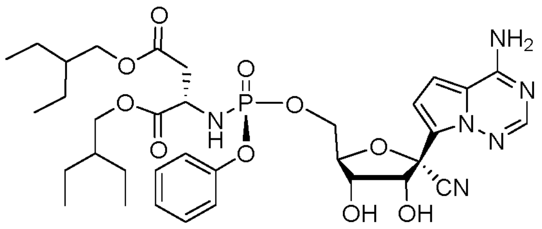,
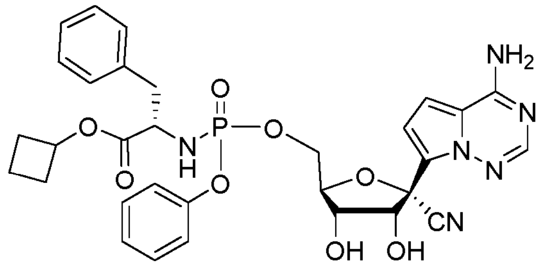,

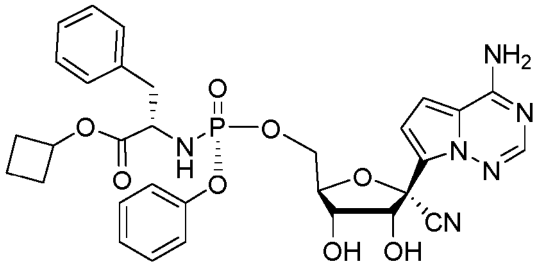,
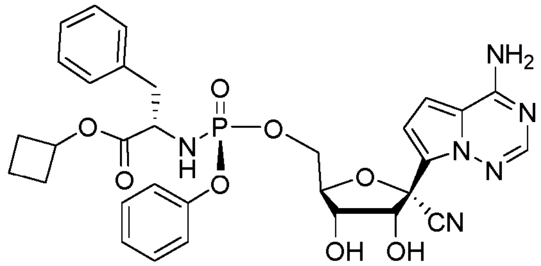,

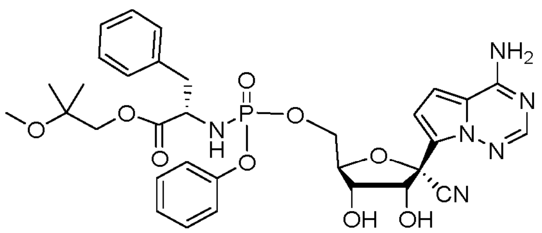,
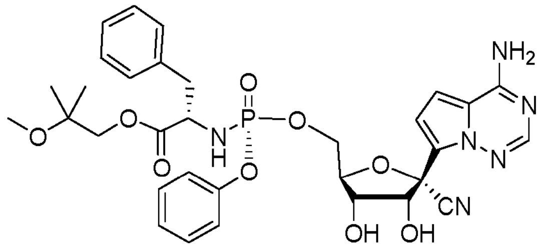,

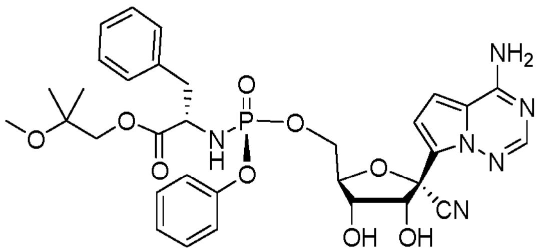,
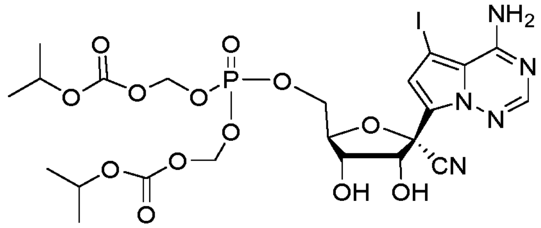,

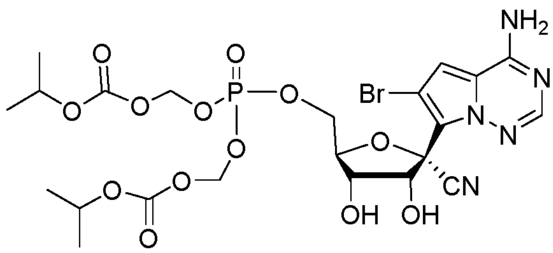.

The compounds of the present invention may include one or more asymmetric centers, and therefore may exist in various stereoisomeric forms, such as enantiomeric and/or diastereomeric forms. For example, the compounds of the present invention may be in the form of individual enantiomers, diastereomers, or geometric isomers (such as cis and trans isomers), or may be in the form of mixtures of stereoisomers, including racemic mixtures and mixtures enriched in one or more stereoisomers. Isomers can be separated from mixtures using methods known to those skilled in the art, including chiral high-pressure liquid chromatography (HPLC) and the formation and crystallization of chiral salts; alternatively, preferred isomers can be prepared through asymmetric synthesis.

"Tautomers" refers to certain compounds in which one functional group changes its structure to become another functional group isomer, and can rapidly interconvert, resulting in the two isomers existing in dynamic equilibrium, with

these two isomers being referred to as tautomers. Those skilled in the art will appreciate that organic compounds may form complexes with solvents in which they react or from which they precipitate or crystallize. These complexes are called "solvates". When the solvent is water, the complex is called a "hydrate". The present invention encompasses all solvates of the compounds of the present invention.

The term "solvate" refers to a compound or its salt that is typically formed by solvolysis reactions and combines with a solvent. Such a physical association may include hydrogen bonding. Conventional solvents include water, methanol, ethanol, acetic acid, DMSO, THF, diethyl ether, etc. The compounds described herein can be prepared, for example, into crystalline forms and can be solvated. Suitable solvates include pharmaceutically acceptable solvates and further include stoichiometric solvates and nonstoichiometric solvates. In some cases, the solvate will be able to be separated, for example, when one or more solvent molecules are incorporated into the lattice of a crystalline solid. "Solvates" include solvates in a solution state and separable solvates. Representative solvates include hydrates, ethanolates, and methylates.

The term "hydrate" refers to a compound combined with water. Generally, the number of water molecules contained in a hydrate of a compound is determined by the ratio to the number of molecules of the compound in the hydrate. Therefore, a hydrate of a compound can be represented by, for example, the general formula $R \cdot x\ H_2O$, wherein R is the compound, and x is a number greater than 0. A given compound can form more than one type of hydrate, including, for example, monohydrate (x is 1), lower hydrate (x is a number greater than 0 and less than 1, such as hemihydrate ($R \cdot 0.5\ H_2O$)) and polyhydrate (x is a number greater than 1, such as dihydrate ($R \cdot 2\ H_2O$) and hexahydrate ($R \cdot 6\ H_2O$)).

The compounds of the present invention may exist in amorphous or crystalline forms (including polymorphs). In addition, the compounds of the present invention may exist in one or more crystalline forms. Therefore, all amorphous or crystalline forms of the compounds of the present invention are included within the scope of the present invention. The term "polymorph" refers to a crystalline form of a compound (or its salt, hydrate or solvate) with a specific crystal packing arrangement. All polymorphs have the same elemental composition. Different crystalline forms typically have different X-ray diffraction patterns, infrared spectra, melting points, densities, hardness, crystal shapes, optoelectronic properties, stabilities, and solubilities. Recrystallization solvents, crystallization rate, storage temperature, and other factors may cause one crystalline form to predominate. Various polymorphs of the compound can be prepared by crystallization under different conditions.

The present invention also includes isotopically labeled compounds which are identical to those described by formula (I) except that one or more atoms are replaced by atoms having an atomic mass or mass number different from the atomic mass or mass number usually found in nature. Examples of isotopes that can be introduced into the compounds of the present invention include isotopes of hydrogen, carbon, nitrogen, oxygen, phosphorus, sulfur, fluorine, and chlorine, such as $^{2}H$, $^{3}H$, $^{13}C$, $^{11}C$, $^{14}C$, $^{15}N$, $^{18}O$, $^{17}O$, $^{31}P$, $^{32}P$, $^{35}S$, $^{18}F$ and $^{31}Cl$, respectively. The compounds of the present invention containing the aforementioned isotopes and/or other isotopes of other atoms, their prodrugs , or pharmaceutically acceptable salts of these compounds or prodrugs are all within the scope of the present invention. Certain isotopically labeled compounds of the present invention, such as those incorporating radioactive isotopes (e.g., $^{3}H$ and $^{14}C$), can be used in drug and/or substrate tissue distribution assays. Tritium ($^{3}H$) and carbon-14 ($^{14}C$) isotopes are particularly preferred because they are easy to prepare and detect. Furthermore, substitution with heavier isotopes, such as deuterium ($^{2}H$), may provide therapeutic advantages due to greater metabolic stability, such as increased in vivo half-life or reduced dosage requirements, and thus may be preferable in some cases. Isotopically labeled compounds of formula (I) of the present invention and their prodrugs can generally be prepared by carrying out the procedures disclosed in the following schemes and/or examples and preparations, using readily available isotopically labeled reagents in place of non-isotopically labeled reagents.

In addition, prodrugs are also included in the context of the present invention. The term "prodrug" as used herein refers to a compound that is converted in vivo, for example by hydrolysis in the blood, into its active form having medical effects. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is introduced herein by reference. A prodrug is any covalently bonded compound of the present invention that releases the parent compound in vivo when administered to a patient. Prodrugs are usually prepared by modifying functional groups in such a way that the modifications can be cleaved by routine operations or in vivo to produce the parent compound. Prodrugs include, for example, compounds of the invention wherein a hydroxy, amino, or mercapto group is bonded to any group that can be cleaved to form a free hydroxy, amino, or mercapto group when administered to a patient. Therefore, representative examples of prodrugs include, but are not limited to acetate/amide, formate/amide, and benzoate/amide derivatives of the hydroxyl, thiol, and amino functional groups in compounds of formula (I). In addition, in the case of carboxylic acids (-COOH), esters such as methyl ester, and ethyl ester, etc. can be used. The esters themselves may be active and/or may be hydrolyzable under in vivo conditions in humans. Suitable pharmaceutically acceptable ester groups that are hydrolysable in vivo include those groups that readily decompose in humans to release

the parent acid or its salt.

Pharmaceutical compositions, formulations, and kits

In another aspect, the present invention provides a pharmaceutical composition comprising the compound of the present invention (also referred to as an "active ingredient") and a pharmaceutically acceptable excipient. In some embodiments, the pharmaceutical composition comprises an effective amount of the active ingredient. In some embodiments, the pharmaceutical composition comprises a therapeutically effective amount of the active ingredient. In some embodiments, the pharmaceutical composition comprises a prophylactically effective amount of the active ingredient.
Pharmaceutically acceptable excipients used in the present invention refer to non-toxic carriers, adjuvants, or vehicles that will not destroy the pharmacological activity of the compounds with which they are formulated. Pharmaceutically acceptable carriers, adjuvants, or vehicles that can be used in the compositions of the present invention include, but are not limited to, ion exchangers, alumina, aluminum stearate, lecithins, serum proteins (such as human serum albumin), buffer substances (such as phosphates), glycine, sorbic acid, potassium sorbate, mixtures of partial glycerides of saturated plant fatty acids, water, salts, or electrolytes (such as protamine sulfate), disodium hydrogen phosphate, potassium hydrogen phosphate, sodium chloride, zinc salts, silica gel, magnesium trisilicate, polyvinylpyrrolidone, cellulose-based materials, polyethylene glycol, sodium carboxymethylcellulose, polyacrylates, waxes, polyethylene-polyoxypropylene-block polymers, polyethylene glycol, and lanolin.
The present invention also includes a kit (e.g., pharmaceutical packages). The provided kit may comprise a compound of the present invention, another therapeutic agent, and first and second containers (e.g., vials, ampules, bottles, syringes, and/or dispersible packages or other suitable containers) containing the compound of the present invention and the other therapeutic agent. **In** some embodiments, the provided kit may also optionally include a third container containing a pharmaceutical excipient for diluting or suspending the compound of the present invention and/or the other therapeutic agent. **In** some embodiments, the compound of the present invention provided in the first and second containers together with other therapeutic agent form a unit dosage form.
The pharmaceutical composition provided by the present invention can be administered through various routes, including but not limited to oral administration, parenteral administration, inhalation administration, local administration, rectal administration, nasal administration, buccal administration, vaginal administration, administration via implant, or other administration methods. For example, the parenteral administration as used herein includes subcutaneous administration, intradermal administration, intravenous administration, intramuscular administration, intra-articular administration, intraarterial administration, intrasynovial administration, intrasternal administration, intrathecal administration, intralesional administration, and intracranial injection or infusion techniques.
Usually, an effective amount of the compound provided herein is administered. The actual amount of the compound to be administered can be determined by a physician in light of the relevant circumstances, including the condition to be treated, the selected administration route, the actual compound administered, the age, weight and response of the individual patient, the severity of the patient's symptoms, etc.
When used for the prevention of the conditions described in the present invention, the compound provided herein is administered to a subject at risk of developing the conditions, typically based on a physician's recommendation and under a physician's supervision, at the dosage level as described above. Subjects at risk of developing specific conditions typically include those with a family history of the conditions, or those identified as particularly susceptible to developing the condition through genetic testing or screening.
The pharmaceutical composition provided herein may also be administered long-term ("long term administration"). Long term administration refers to administration of the compound or its pharmaceutical composition over an extended period of time, such as 3 months, 6 months, 1 year, 2 years, 3 years, 5 years, etc, or can be continuously administered indefinitely, for example, for the rest of the subject's life. In some embodiments, long term administration is intended to provide a constant level of the compound in the blood over a long period of time, such as within the therapeutic window.
Various administration methods can be used to further deliver the pharmaceutical composition of the present invention. For example, in some embodiments, the pharmaceutical composition can be administered via bolus, for example, in order to rapidly increase the concentration of the compound in the blood to an effective level. The bolus dose depends on the target systemic level of the active ingredient, for example, an intramuscular or subcutaneous bolus dose allows slow release of the active ingredient, while a bolus delivered directly into a vein (e.g., via IV drip) enables more rapid delivery, allowing the concentration of the active ingredient in the blood to rapidly rise to an effective level. In other embodiments, the pharmaceutical composition can be administered as a continuous infusion, for example, via IV drip, to provide a steady-state concentration of the active ingredient in the subject's body. In addition, in other embodiments, a bolus dose of the pharmaceutical composition may be administered first, followed by continuous infusion.
Oral compositions can be in the form of bulk liquid solutions, suspensions, or bulk powders. However, more typically, in order to facilitate precise dosing, the composition is provided in unit dosage form. The term "unit dosage form"

refers to physically discrete units suitable as dosages for human patients and other mammals, each unit contains a predetermined quantity of active substance calculated to produce the desired therapeutic effect and appropriate pharmaceutical excipients. Typical unit dosage forms include pre-filled, pre-measured ampoules or syringes of liquid compositions, or pills, tablets, and capsules, etc. in the case of solid compositions. In such compositions, the compound typically is typically a minor component (about 0.1 to about 50 wt%, or preferably about 1 to about 40 wt%), with the remaining portion being various carriers or excipients and processing aids useful for forming the desired administration form.

For oral dosage, a representative regimen is one to five oral doses per day, especially two to four oral doses, typically three oral doses. Using these dosing regimens, each dose provides about 0.01 to about 20 mg/kg of the compound of the present invention, with the preferred doses each providing about 0.1 to about 10 mg/kg, particularly about 1 to about 5 mg/kg.

To provide blood levels similar to or lower than those achieved with injectable doses, transdermal doses are typically selected in amounts of about 0.01 to about 20 wt%, preferably from about 0.1 to about 20 wt%, preferably from about 0.1 to about 10 wt%, and more perfectly from about 0.5 to about 15 wt%.

Injectable dose levels range from about 0.1 mg/kg/hour to at least 10 mg/kg/hour over about 1 to about 120 hours, especially 24 to 96 hours. To obtain sufficient steady-state levels, a preloaded bolus of about 0.1 mg/kg to about 10 mg/kg or more can also be administered. For human patients weighing 40 to 80 kg, the maximum total dose should not exceed about 2 g/day.

Liquid forms suitable for oral administration may include suitable aqueous or non-aqueous carriers as well as buffers, suspending and dispersing agents, coloring agents, flavoring agents, etc. Solid forms may include, for example, any of the following components, or compounds with similar properties: binders, such as microcrystalline cellulose, tragacanth gum, or gelatin; excipients, such as starch or lactose, disintegrants, such as alginate, Primogel or corn starch; lubricants, such as magnesium stearate; glidants, such as colloidal silica; sweeteners, such as sucrose or saccharin; or flavouring agents, such as peppermint, methyl salicylate, or orange flavor.

Injectable compositions are typically based on sterile saline or phosphate buffered saline for injection, or other injectable excipients known in the art. As mentioned earlier, in such compositions, the active compound is typically a minor component, often about 0.05 to 10 wt%, with the remaining portion being injectable excipients, etc.

Transdermal compositions are typically formulated as topical ointments or creams containing the active ingredient. When formulated as an ointment, the active ingredient is typically combined with paraffin or a water-miscible ointment matrix. Or, the active ingredient can be formulated as a cream with, for example, an oil-in-water cream base. This transdermal formulation is well-known in the art and typically includes other components for enhancing the stable skin penetration of the active ingredient or formulation. All such known transdermal formulations and components are included within the scope provided by the present invention.

The compounds of the present invention can also be administered through transdermal devices. Therefore, transdermal administration can be achieved by using reservoirs or porous membrane types, or various solid matrix patches.

The above components for oral, injectable or topical administration compositions are only representative. Other materials and processing techniques, etc., are described in Part 8 of Remington's Pharmaceutical Sciences, 17th edition, 1985, Mack Publishing Company, Easton, Pennsylvania, which is introduced herein by reference.

The compounds of the present invention can also be administered in sustained-release forms, or administered from sustained release drug delivery systems. A description of the representative sustained release materials can be found in Remington's Pharmaceutical Sciences.

The present invention also relates to pharmaceutically acceptable formulations of the compounds of the present invention. In an embodiment, the formulation contains water. In another embodiment, the formulation contains a cyclodextrin derivatives. The most common cyclodextrins are α-, β-, and γ- cyclodextrins composed of 6, 7, and 8 α-1,4-linked glucose units, respectively, which optionally include one or more substituents on the linked sugar moiety, including but not limited to methylated, hydroxyalkylated, acylated, and sulfoalkyl ether substituents. In some embodiments, the cyclodextrin is a sulfoalkyl ether β-cyclodextrin, such as sulfobutyl ether β-cyclodextrin, also known as Captisol. See, for example, U.S.5,376,645. In some embodiments, the formulation includes hexapropyl-β-cyclodextrin (e.g., 10-50% in water).

## Indications

In another aspect, the present invention relates to a method for the treatment and/or prevention of viral infections in a subject, comprising administering the compound of the present invention, or a pharmaceutically acceptable salt, stereoisomer, solvate, hydrate, polymorph, prodrug or isotopic variant thereof, or the pharmaceutical composition of the present invention to the subject. In another aspect, the compound of the present invention, or a pharmaceutically acceptable salt, stereoisomer, solvate, hydrate, polymorph, prodrug or isotopic variant thereof, or the pharmaceutical

composition of the present invention can be used for the treatment and/or prevention of coronavirus infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of coronaviruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of Coronaviridae viruses. In a specific embodiment, the coronavirus is SARS-CoV virus and its variants, MERS-CoV virus and its variants, SARS-CoV-2 virus and its variants, other human coronavirus (e.g., 229E, NL63, OC43, HKU1 or WIV1), zoonotic coronavirus (e.g., PEDV or HKU CoV isolate, such as HKU3, HKU5 or HKU9), feline coronavirus (e.g., Feline Enteric Coronavirus (FECV) or Feline infectious peritonitis virus (FIPV)) or porcine epidemic diarrhea virus (PEDV). In a specific embodiment, the coronavirus is SARS-CoV virus and its variants, MERS-CoV virus and its variants or SARS-CoV-2 virus and its variants. In a specific embodiment, the coronavirus is SARS-CoV-2 virus and its variants. In a specific embodiment, the variants of SARS-CoV-2 virus are Alpha variants, Beta variants, Delta variants, Gamma variants, or Omicron variants. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses possessing polymerases homologous to SARS-CoV-2 polymerase. For example, it can be used for the treatment and/or prevention of viral infections caused by viruses possessing polymerases with at least 60% sequence homology to SARS-CoV-2 polymerase. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses possessing polymerases with at least 70%, at least 75%, at least 80%, at least 85%, at least 90%, at least 95%, at least 96%, at least 97%, at least 98%, or at least 99% sequence homology to SARS-CoV-2 polymerase. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses possessing polymerases homologous to SARS-CoV-2 polymerase. For example, it can be used for the treatment and/or prevention of viral infections caused by viruses possessing polymerases with at least 60% sequence homology to SARS-CoV-2 polymerase. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses possessing polymerases with at least 70%, at least 75%, at least 80%, at least 85%, at least 90%, at least 95%, at least 96%, at least 97%, at least 98%, or at least 99% sequence homology to SARS-CoV-2 polymerase. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses exhibiting at least 60% sequence homology to the complete genomic sequence of SARS-CoV-2. In a specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of viral infections caused by viruses exhibiting at least 70%, at least 75%, at least 80%, at least 85%, at least 90%, at least 95%, at least 96%, at least 97%, at least 98% or at least 99% sequence homology to whole genome sequence of SARS-CoV-2.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Paramyxoviridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Paramyxoviridae* viruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of *Paramyxoviridae* viruses. In a specific embodiment, the *Paramyxoviridae* virus is parainfluenza virus, measles or mumps virus.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Pneumoviridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Pneumoviridae* viruses. In a more specific embodiment, the compound of the present invention, or a pharmaceutically acceptable salt, stereoisomer, solvate, hydrate, polymorph, prodrug or isotopic variant thereof, or the

pharmaceutical composition of the present invention can be used for the treatment and/or prevention of RNA-dependent RNA polymerase of *Pneumoviridae* viruses. In a specific embodiment, the *Pneumoviridae* virus is RSV or human metapneumovirus. In a specific embodiment, the *Pneumoviridae* virus is RSV.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Picornavirridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Picornavirridae* viruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of *Picornavirridae* viruses. In a specific embodiment, the *Picornavirridae* virus is enterovirus or rhinovirus.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Flaviviridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Flaviviridae* viruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of Flaviviridae viruses. In a specific embodiment, the *Flaviviridae* virus is dengue virus, Yellow Fever virus, West Nile virus, Zika virus, Japanese encephalitis virus and Hepatitis C virus.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Filoviridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Filoviridae* viruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of *Filoviridae* viruses. In a more specific embodiment, the *Filoviridae* is Ebola virus, Zaire Ebola virus, Bundibugio Ebola virus, Sudan Ebola virus, Tai forest Ebola virus, Reston Ebola virus or Marburg virus. In a more specific aspect, the *Filoviridae* virus described in the present invention is Ebola virus or Marburg virus.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of *Arenaviridae* viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of *Arenaviridae* viruses. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of *Arenaviridae* viruses. In a specific embodiment, the *Arenaviridae* is LASA virus or JUNV virus.

In another embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of monkeypox viral infections. In a more specific embodiment, the compound of the present invention, or a pharmaceutically acceptable salt, stereoisomer, solvate, hydrate, polymorph, prodrug or isotopic variant thereof, or the pharmaceutical composition of the present invention can inhibit the replication of monkeypox virus. In a more specific embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment and/or prevention of RNA-dependent RNA polymerase of monkeypox virus.

Methods of administration

The compound of the present invention (referred to as "an active ingredient" herein), or a pharmaceutically acceptable salt, stereoisomer, solvate, hydrate, polymorph, prodrug or isotopic variant thereof, or the pharmaceutical composition of the present invention can be administrated through any routes suitable for the conditions to be treated. The

preferred routes may vary depending on, for example, the conditions of subjects. The advantage of the compounds of the present invention is that they are orally bioavailable and can be administered orally.

In an embodiment, the compound of the present invention, or a pharmaceutically acceptable salt, stereoisomer, solvate, hydrate, polymorph, prodrug or isotopic variant thereof, or the pharmaceutical composition of the present invention can be used for the treatment of viral infections. The compound of the present invention, or a pharmaceutically acceptable salt, stereoisomer, solvate, hydrate, polymorph, prodrug or isotopic variant thereof, or the pharmaceutical composition of the present invention can be administered to individuals who may come into contact with those having viral infections or have already been infected with viruses (e.g., SARS-CoV-2) at any time. In another embodiment, the compound of the present invention, or a pharmaceutically acceptable salt, stereoisomer, solvate, hydrate, polymorph, prodrug or isotopic variant thereof, or the pharmaceutical composition of the present invention can be prophylactically administered to individuals who come into contact with those having viral infections (e.g., SARS-CoV-2) or are at risk of coming into contact with individuals having viral infections (e.g., SARS-CoV-2), such as healthcare providers. In another embodiment, the administration of the compound of the present invention, or a pharmaceutically acceptable salt, stereoisomer, solvate, hydrate, polymorph, prodrug or isotopic variant thereof, or the pharmaceutical composition of the present invention can be performed on individuals who are positive for viral infections (e.g., SARS-CoV-2) testing but have not yet shown symptoms of viral infections (e.g., SARS-CoV-2). In another embodiment, the administration of the compound of the present invention, or a pharmaceutically acceptable salt, stereoisomer, solvate, hydrate, polymorph, prodrug or isotopic variant thereof, or the pharmaceutical composition of the present invention can be performed on individuals after the onset of symptoms of viral infections (e.g., SARS-CoV-2).

In an embodiment, the method for the treatment and/or prevention of viral infections disclosed herein includes event-driven administration to a subject of the compound of the present invention, or a pharmaceutically acceptable salt, stereoisomer, solvate, hydrate, polymorph, prodrug or isotopic variant thereof, or the pharmaceutical composition of the present invention.

As described herein, the term "event-driven" or "event-driven administration" refers to administration of the compound of the present invention, or a pharmaceutically acceptable salt, stereoisomer, solvate, hydrate, polymorph, prodrug or isotopic variant thereof, or the pharmaceutical composition of the present invention under the following circumstances: (1) prior to an event that exposes an individual to a virus (e.g., SARS-CoV-2) or otherwise increases the individual's risk of viral infection (e.g., SARS-CoV-2) (e.g., 2 hours, 1 day, 2 days, 5 days, or 7 days prior to the event); and/or (2) during an event (or more than one repeated event) that exposes an individual to a virus (e.g., SARS-CoV-2) or otherwise increases the individual's risk of viral infection (e.g., SARS-CoV-2); and/or (3) after an event (or after the final event in a series of repeated events) that exposes an individual to a virus (e.g., SARS-CoV-2) or otherwise increases the individual's risk of viral infection (e.g., SARS-CoV-2). In an embodiment, the event-driven administration is performed before exposing the subject to a virus (e.g., SARS-CoV-2). In another embodiment, the event-driven administration is performed after the subject has been exposed to a virus (e.g., SARS-CoV-2). In another embodiment, the event-driven administration is performed before exposing the subject to a virus (e.g., SARS-CoV-2) and after exposing the subject to a virus (e.g., SARS-CoV-2).

In an embodiment, the methods for the treatment and/or prevention of viral infections disclosed herein involve administration before and/or after an event of exposing an individual to a virus (e.g., SARS-CoV-2) or otherwise increasing the individual's risk of viral infection (e.g., SARS-CoV-2), such as serving as pre-exposure prophylaxis (PrEP) and/or post-exposure prophylaxis (PEP). In a specific embodiment, the methods disclosed herein include pre-exposure prophylaxis. In another specific embodiment, the methods disclosed herein include post-exposure prophylaxis. In an embodiment, the compound of the present invention, or a pharmaceutically acceptable salt, stereoisomer, solvate, hydrate, polymorph, prodrug or isotopic variant thereof, or the pharmaceutical composition of the present invention is administrated prior to exposure of the subject to a virus (e.g., SARS-CoV-2).

In another embodiment, the compound of the present invention, or a pharmaceutically acceptable salt, stereoisomer, solvate, hydrate, polymorph, prodrug or isotopic variant thereof, or the pharmaceutical composition of the present invention is administrated both prior to and after exposure of the subject to a virus (e.g., SARS-CoV-2).

In another embodiment, the compound of the present invention, or a pharmaceutically acceptable salt, stereoisomer, solvate, hydrate, polymorph, prodrug or isotopic variant thereof, or the pharmaceutical composition of the present invention is administrated after exposure of the subject to a virus (e.g., SARS-CoV-2).

Examples of event-driven administration regimens include administration of the compound of the present invention, or a pharmaceutically acceptable salt, stereoisomer, solvate, hydrate, polymorph, prodrug or isotopic variant thereof, or the pharmaceutical composition of the present invention within 24 to 2 hours prior to exposure to a virus (e.g., SARS-CoV-2), followed by further administration of the compound of the present invention, or a pharmaceutically acceptable salt, stereoisomer, solvate, hydrate, polymorph, prodrug or isotopic variant thereof, or the pharmaceutical composition of the present invention after the last exposure, and the last administration of the compound of the present invention, or a pharmaceutically acceptable salt, stereoisomer, solvate, hydrate, polymorph, prodrug or isotopic variant thereof, or the pharmaceutical composition of the present invention after 24 hours.

Another example of event-driven administration regimens includes the administration of the compound of the present invention, or a pharmaceutically acceptable salt, stereoisomer, solvate, hydrate, polymorph, prodrug or isotopic variant thereof, or the pharmaceutical composition of the present invention within 24 hours prior to exposure to a virus (e.g., SARS-CoV-2), followed by the last administration about 24 hours after the last exposure (which can be an increased dose, such as a double dose).

The effective dosage of the active ingredient depends on at least the nature and toxicity of the condition to be treated, whether the compound is administered prophylactically or used to combat active viral infections, delivery methods, and drug formulations, and will be determined by clinicians using routine dose-escalation studies. The dose may be expected to be about 0.0001 to about 100 mg/kg body weight per day; typically, it is about 0.01 to 10 mg/kg body weight per day; more typically, it is about 0.01 to about 5 mg/kg body weight per day; and most typically, it is about 0.05 to about 0.5 mg/kg body weight per day. For example, the daily candidate dose range for an adult weighing approximately 70 kg would be 1 mg to 1000 mg, preferably 5 mg to 500 mg, which could be in the form of a single dose or multiple doses.

The effective dose of the active ingredient of the compound of the present invention for treating viral infections (e.g., SARS-CoV-2) may depend on whether the dose is used prophylactically or for treating individuals already suffering from viral infection (e.g., SARS-CoV-2). In addition, the dose may depend on whether an individual infected with a virus (e.g., SARS-CoV-2) has not yet shown symptoms or has already shown symptoms of viral infection (e.g., SARS-CoV-2). Compared to individuals receiving prophylactic treatment, treatment for those who are positive for viral infection (e.g., SARS-CoV-2) testing and exhibit symptoms of viral infection (e.g., SARS-CoV-2) may require higher doses.

Any suitable time period for administering the compound of the present invention, or a pharmaceutically acceptable salt, stereoisomer, solvate, hydrate, polymorph, prodrug or isotopic variant thereof, or the pharmaceutical composition of the present invention is also contemplated. For example, the administration can last from 1 day to 100 days, including 2, 3, 4, 5, 6, 7, 8, 9, 10, 15, 20, 25, 30, 40, 50, 60, 70, 80 or 90 days. The administration can also last from 1 week to 15 weeks, including 2, 3, 4, 5, 6, 7, 8, 9, 10, 11, 12, 13 or 14 weeks. A longer period of administration time is also contemplated. The administration period may depend on whether the compound is administered prophylactically or for treating a person suffering from viral infection (e.g., SARS-CoV-2) for a period of time during normal contact and for a suitable period of time after last contact with an individual suffering from viral infection (e.g., SARS-CoV-2). For individuals who have already been infected with a virus (e.g., SARS-CoV-2), the administration period of time can be any length of time required to treat the patients, as well as an appropriate period of time after testing negative for viral infection (e.g., SARS-CoV-2) to ensure that the viral infection (e.g., SARS-CoV-2) does not recur.

In another aspect, the compound of the present invention, or a pharmaceutically acceptable salt, stereoisomer, solvate, hydrate, polymorph, prodrug or isotopic variant thereof, or the pharmaceutical composition of the present invention can be administered once daily. In other embodiments, the compound of the present invention, or a pharmaceutically acceptable salt, stereoisomer, solvate, hydrate, polymorph, prodrug or isotopic variant thereof, or the pharmaceutical composition of the present invention can be administered once every other day. In another embodiment, the compound of the present invention is administered once every week. In another embodiment, the compound of the present invention, or a pharmaceutically acceptable salt, stereoisomer, solvate, hydrate, polymorph, prodrug or isotopic variant thereof, or the pharmaceutical composition of the present invention is administered twice a week.

In an embodiment, the method for the treatment and/or prevention of viral (e.g., SARS-CoV-2) infections of the present invention comprises administering a loading dose of the compound of the present invention, or a pharmaceutically acceptable salt, stereoisomer, solvate, hydrate, polymorph, prodrug or isotopic variant thereof, or the pharmaceutical composition of the present invention on the first day, then administering the maintenance dose of the compound of the present invention, or a pharmaceutically acceptable salt, stereoisomer, solvate, hydrate, polymorph, prodrug or isotopic variant thereof, or the pharmaceutical composition of the present invention once a day for the following days. The once-daily maintenance dose of the compound of the present invention, or a pharmaceutically acceptable salt, stereoisomer, solvate, hydrate, polymorph, prodrug or isotopic variant thereof, or the pharmaceutical composition of the present invention can be administered as needed, for example, up to 5 days, up to 7 days, up to 10 days, up to 15 days, up to 20 days, up to 25 days, up to one month or longer. In another embodiment, the once-daily maintenance dose is administered for about 6-12 days, such as about 8-10 days. In another embodiment, the once-daily maintenance dose is administered for about 4 days. In another embodiment, the once-daily maintenance dose is administered for about 5 days. In another embodiment, the once-daily maintenance dose is administered for about 9 days. In another embodiment, the once-daily maintenance dose is administered for about 10 days. The loading dose can be equal to, less than, or greater than the maintenance dose. In another embodiment, the loading dose is greater than the maintenance dose.

In an embodiment, the method for the treatment and/or prevention of viral infection (e.g., SARS-CoV-2) of the present invention comprises administering a loading dose of 150-250 mg (e.g., about 200 mg) on the first day, followed by administering an once-daily maintenance dose of about 50-150 mg (e.g., about 100 mg) on the following days. In another embodiment, the once-daily maintenance dose is administered for about 6-12 days, such as about 8-10 days. In another embodiment, the once-daily maintenance dose is administered for about 9 days. In another embodiment, the once-daily maintenance dose is administered for about 2-6 days, such as about 3-5 days. In another embodiment, the once-daily

maintenance dose is administered for about 4 days.

In an embodiment, the method for the treatment and/or prevention of viral infection (e.g., SARS-CoV-2) of the present invention comprises administering a loading dose of 50-250 mg (e.g., about 100mg) on the first day, followed by administering an once-daily maintenance dose of about 10-100mg (e.g., about 50mg) on the following days. In another embodiment, the once-daily maintenance dose is administered for about 6-12 days, such as about 8-10 days. In another embodiment, the once-daily maintenance dose is administered for about 9 days. In another embodiment, the once-daily maintenance dose is administered for about 2-6 days, such as about 3-5 days. In another embodiment, the once-daily maintenance dose is administered for about 4 days.

In an embodiment, the loading dose is equal to the maintenance dose. The once-daily dose can be administered as needed, for example, for up to 5 days, up to 7 days, up to 10 days, up to 15 days, up to 20 days, up to 25 days, up to one month or longer. In another embodiment, the once-daily dose is administered for up to 20 days, up to 15 days, up to 14 days, up to 13 days, up to 12 days, up to 10 days, up to 8 days, up to 6 days, up to 4 days, up to 3 days, up to 2 days or 1 day.

In another embodiment, the compound of the present invention, or a pharmaceutically acceptable salt, stereoisomer, solvate, hydrate, polymorph, prodrug or isotopic variant thereof, or the pharmaceutical composition of the present invention is administered once daily, lasting for about 6-12 days, such as about 8-10 days. In another embodiment, the compound of the present invention, or a pharmaceutically acceptable salt, stereoisomer, solvate, hydrate, polymorph, prodrug or isotopic variant thereof, or the pharmaceutical composition of the present invention is administered once daily, lasting for about 9 days. In another embodiment, the compound of the present invention, or a pharmaceutically acceptable salt, stereoisomer, solvate, hydrate, polymorph, prodrug or isotopic variant thereof, or the pharmaceutical composition of the present invention is administered once daily, lasting for about 10 days. In another embodiment, about 50-150 mg of the compound of the present invention is administered once daily, lasting for about 6-12 days, such as about 10 days. In another embodiment, about 100 mg of the compound of the present invention is administered once daily, lasting for about 6-12 days, such as about 10 days.

## Combination therapy

In another aspect, the compound of the present invention, or a pharmaceutically acceptable salt, stereoisomer, solvate, hydrate, polymorph, prodrug or isotopic variant thereof, or the pharmaceutical composition of the present invention can be used in combination with one or more additional agents for the treatment and/or prevention of *Coronaviridae, Paramyxoviridae, Pneumoviridae, Picornavirridae, Flaviviridae, Filoviridae, Arenaviridae,* and *Orthomyxoviridae* infections.

In a specific embodiment, the compound of the present invention, or a pharmaceutically acceptable salt, stereoisomer, solvate, hydrate, polymorph, prodrug or isotopic variant thereof, or the pharmaceutical composition of the present invention can be administered together with one or more additional agents in a single pharmaceutical composition. In another specific embodiment, the compound of the present invention, or a pharmaceutically acceptable salt, stereoisomer, solvate, hydrate, polymorph, prodrug or isotopic variant thereof, or the pharmaceutical composition of the present invention can be administered together with one or more additional agents in two or more separate pharmaceutical compositions. For example, the compound of the present invention, or a pharmaceutically acceptable salt, stereoisomer, solvate, hydrate, polymorph, prodrug or isotopic variant thereof, or the pharmaceutical composition of the present invention can administered in a single pharmaceutical composition, and at least one of the additional agents can be administered in a second pharmaceutical composition. If there are at least two additional agents, one or more of the additional agents can be in the first pharmaceutical composition comprising the compound of the present invention, or a pharmaceutically acceptable salt, stereoisomer, solvate, hydrate, polymorph, prodrug or isotopic variant thereof, and at least one of the other additional agents can be in the second pharmaceutical composition.

When the compound of the present invention, or a pharmaceutically acceptable salt, stereoisomer, solvate, hydrate, polymorph, prodrug or isotopic variant thereof, or a pharmaceutical composition comprising the compound of the present invention, or a pharmaceutically acceptable salt, stereoisomer, solvate, hydrate, polymorph, prodrug or isotopic variant thereof and one or more additional agents is used, the dosage and administration regimens are within the knowledge of those skilled in the art. For example, when the recognized dosage and administration regimens in the art are used as the standard of care treatments, in addition to the treatment described, an effective amount or administration regimen as described herein can also be used for administrating the compound of the present invention, or a pharmaceutically acceptable salt, stereoisomer, solvate, hydrate, polymorph, prodrug or isotopic variant thereof, or a pharmaceutical composition comprising the compound of the present invention, or a pharmaceutically acceptable salt, stereoisomer, solvate, hydrate, polymorph, prodrug or isotopic variant thereof and one or more additional agents, or replacing an agent in the combination therapy.

The order of administration of the compound of the present invention, or a pharmaceutically acceptable salt, stereoisomer, solvate, hydrate, polymorph, prodrug or isotopic variant thereof, and one or more additional agents can be varied. In a specific embodiment, the compound of the present invention, or a pharmaceutically acceptable salt,

stereoisomer, solvate, hydrate, polymorph, prodrug or isotopic variant thereof can be administrated before all additional agents. In another specific embodiment, the compound of the present invention, or a pharmaceutically acceptable salt, stereoisomer, solvate, hydrate, polymorph, prodrug or isotopic variant thereof can be administered before at least one agent. In another specific embodiment, the compound of the present invention, or a pharmaceutically acceptable salt, stereoisomer, solvate, hydrate, polymorph, prodrug or isotopic variant thereof can be administered simultaneously with one or more additional agents. In another specific embodiment, the compound of the present invention, or a pharmaceutically acceptable salt, stereoisomer, solvate, hydrate, polymorph, prodrug or isotopic variant thereof can be administered after administration of at least one additional agent. In a specific embodiment, the combination of the compound of the present invention, or a pharmaceutically acceptable salt, stereoisomer, solvate, hydrate, polymorph, prodrug or isotopic variant thereof with one or more additional agents can result in an additive effect. In another specific embodiment, the combination of the compound of the present invention, or a pharmaceutically acceptable salt, stereoisomer, solvate, hydrate, polymorph, prodrug or isotopic variant thereof with one or more additional agents can result in a synergistic enhancement effect. In another specific embodiment, the combination of the compound of the present invention, or a pharmaceutically acceptable salt, stereoisomer, solvate, hydrate, polymorph, prodrug or isotopic variant thereof with one or more additional agents can result in a synergistic enhancement effect. In some embodiments, the combination of the compound of the present invention, or a pharmaceutically acceptable salt, stereoisomer, solvate, hydrate, polymorph, prodrug or isotopic variant thereof with one or more additional agents can result in a strong synergistic enhancement effect. The combination of the compound of the present invention, or a pharmaceutically acceptable salt, stereoisomer, solvate, hydrate, polymorph, prodrug or isotopic variant thereof with one or more additional agents is not antagonistic.

As used herein, the term "antagonistic" refers to a combination of compounds having lower activity than the sum of the activities of each compound when measured individually (i.e., as single compounds).

The term "synergistic effect" refers to the activity of the combination of compounds being greater than the sum of the individual activities of the compounds when each compound's activity is measured separately and combined.

The term "additive effect" refers to the activity of the combination of compounds being approximately equal to the sum of the individual activities of the compounds when each compound's activity is measured separately and combined.

Potential advantages of using the combination of the compound of the present invention, or a pharmaceutically acceptable salt, stereoisomer, solvate, hydrate, polymorph, prodrug or isotopic variant thereof with one or more additional agents may include that the amount of one or more additional agents required for effective treatment of viral infection is reduced compared to the amount needed to achieve the same therapeutic effect when administering one or more additional agents without the compound of the present invention, or a pharmaceutically acceptable salt, a stereoisomer, a solvate, a hydrate, a polymorph, a prodrug or an isotopically variant thereof. Another potential advantage of utilizing the combination of the compound of the present invention, or a pharmaceutically acceptable salt, stereoisomer, solvate, hydrate, polymorph, prodrug or isotopic variant thereof with one or more additional agents may be that using two or more compounds with different mechanisms of action can create a higher barrier against the development of resistant viral strains compared to the barrier created when administering a single compound as monotherapy.

Additional advantages of utilizing the combination of the compound of the present invention, or a pharmaceutically acceptable salt, stereoisomer, solvate, hydrate, polymorph, prodrug or isotopic variant thereof with one or more additional agents may include: little to no cross resistance between the compound of the present invention, or a pharmaceutically acceptable salt, stereoisomer, solvate, hydrate, polymorph, prodrug or isotopic variant thereof, and one or more additional agents; different routes of elimination for the compound of the present invention, or a pharmaceutically acceptable salt, stereoisomer, solvate, hydrate, polymorph, prodrug or isotopic variant thereof, and one or more additional agents; little to no overlapping toxicity between the compound of the present invention, or a pharmaceutically acceptable salt, stereoisomer, solvate, hydrate, polymorph, prodrug or isotopic variant thereof, and one or more additional agents; and little to no significant impact on cytochrome P450; little to no pharmacokinetic interaction between the compound of the present invention, or a pharmaceutically acceptable salt, stereoisomer, solvate, hydrate, polymorph, prodrug or isotopic variant thereof, and one or more additional agents; a greater percentage of subjects achieving sustained virologic response compared to when the compound is administered as monotherapy, and/or a reduction in treatment time for the sustained virologic response compared to when the compound is administered as monotherapy.

## Examples

The present invention will be further elaborated below in conjunction with specific examples. It should be understood that these examples are only used to illustrate the present invention and are not intended to limit the scope of the present invention. The experimental methods without specifying the specific conditions in the following examples are usually conducted under conventional conditions, or according to the conditions recommended by the manufacturers. Unless otherwise specified, the parts and percentage are parts by weight and percentage by weight.

Usually, in the preparation process, the reactions are carried out in an inert solvent at room temperature to reflux

temperature (such as 0°C to 100°C, preferably 0°C to 80°C). The reaction time is usually 0.1-60 hours, preferably 0.5-24 hours.

The abbreviations as used herein have the following meanings:

$H_2SO_4$: Sulfuric acid
NIS: N-iodosuccinimide
TFA: Trifluoroacetic acid
DMF: N,N-dimethylformamide
$Pd(dppf)Cl_2$: (1,1'-bis(diphenylphosphino)ferrocene)dichloropalladium
TMEDA: Tetramethylethylenediamine
$NaBD_4$: Sodium borodeuteride
$T_3P$: 1-propylphosphonic acid cyclic anhydride
NMM: N-methylmorpholine
DMAP: N,N-dimethyl-4-pyridylamine
DCM: Dichloromethane

**Preparation of Intermediate A-1: (3aR,4R,6R,6aR)-4-(4-aminopyrrolo[2,1-f]]1,2,4]triazin-7-yl)-6-(hydroxymethyl)-2,2-dimethyltetrahydrofuro[3,4-d][1,3]dioxole-4-carbonitrile**

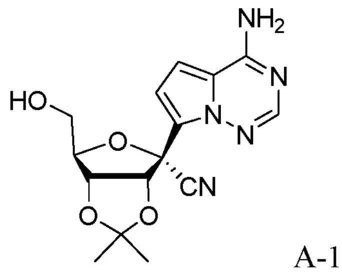

A-1

The following synthetic routes were adopted:

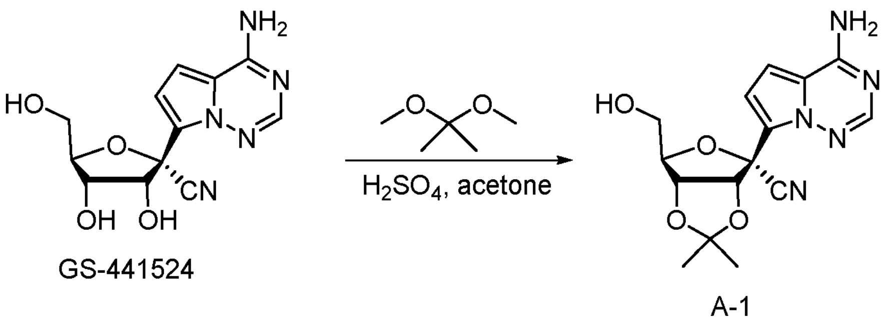


GS-441524 (2.0 g, 6.9 mmol) was added to a reaction flask, followed by the addition of 50 mL of acetone to dissolve. 2,2-dimethoxypropane (1.09 g, 10.4 mmol) and concentrated sulfuric acid (0.55 mL, 10.4 mmol) were added at room temperature. The mixture was stirred at room temperature under nitrogen atmosphere for 4-6 hours. After completion of the reaction as monitored by TLC, 15 mL of triethylamine was added to quench the reaction. The solvent was removed by concentration. The residue was purified by column chromatography and dried under vacuum to obtain 1.7 g of white solid (yield: 74%). LC-MS (APCI): m/z=332.3 (M+1)+.

**Preparation of Intermediate A-2: (3aR,4R,6R,6aR)-4-(4-amino-5-iodopyrrolo[2,1-f][1,2,41triazin-7-yl)-6-(hydroxymethyl)-2,2-dimethyltetrahydrofuro[3,4-d][1,3]dioxole-4-carbonitrile**

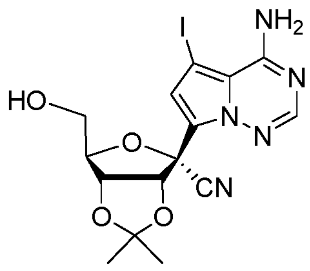

A-2

The following synthetic routes were adopted:

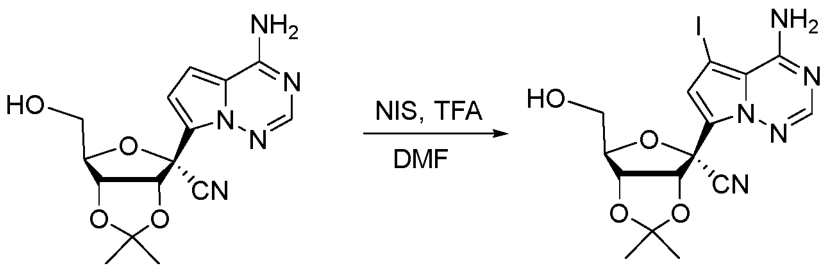


A-1 A-2

The intermediate A-1 (11.8 g, 35.6 mmol) was added to a reaction flask, followed by the addition of 70 mL of anhydrous DMF to dissolve. NIS (8.82 g, 39.2 mmol) and trifluoroacetic acid (0.81 g, 7.1 mmol) were added sequentially. The mixture was heated to 50°C under nitrogen atmosphere and stirred for 1-2 hours. After completion of the reaction as monitored by TLC, the mixture was cooled to room temperature and quenched with saturated aqueous sodium sulfite solution. The resulting mixture was extracted with ethyl acetate 3-4 times. The organic layers were combined, washed with saturated saline solution, concentrated, and purified by silica gel column chromatography to obtain 14.3 g of the product (yield: 88%). LC-MS (APCI): m/z=458.4 $(M+1)^+$.

**Preparation of Intermediate A-3: (3aR,4R,6R,6aR)-4-(4-amino-5-deuteriopyrrolo[2,1-f][1,2,4]triazin-7-yl)-6-(hydroxymethyl)-2,2-dimethyltetrahydrofuro[3,4-d]|1,3]dioxole-4-carbonitrile**

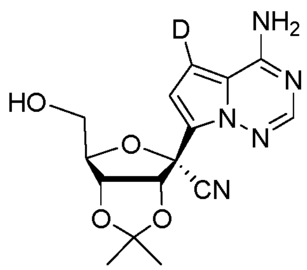

A-3

The following synthetic routes were adopted:

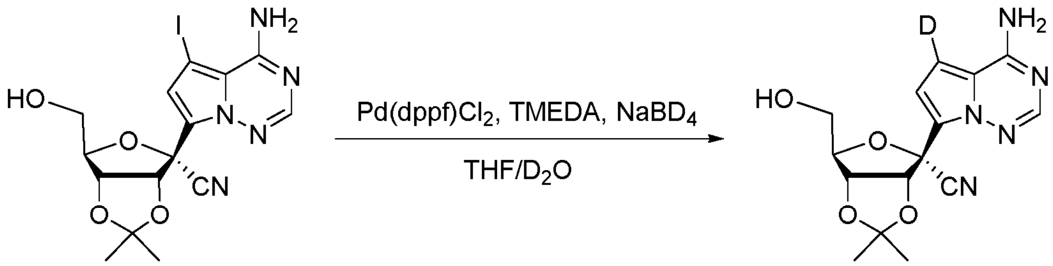


A-2 A-3

The intermediate A-2 (1.2 g, 2.57 mmol) was added to a reaction flask, followed by the addtion of 20 mL of anhydrous tetrahydrofuran and 2 mL of deuterium oxide. (Pd(dppf)$Cl_2$ (0.1 g, 0.13 mmol) and TMEDA (0.6 g, 5.14 mmol) were added sequentially. After the addition, the mixture was stirred at room temperature for 10 minutes, followed by the addition of $NaBD_4$ (0.22 g, 5.14 mmol) in portions over 30 minutes. The reaction was continued at room temperature for 2-3 hours. After completion of the reaction as monitored by TLC, the reaction was quenched with saturated aqueous ammonium chloride solution. The mixture was extracted with ethyl acetate 3-4 times. The organic layers were combined, washed with saturated saline solution, concentrated, and purified by silica gel column chromatography to obtain 0.47 g of the product (yield: 55%). LC-MS (APCI): m/z=333.6 $(M+1)^+$.

**Preparation of Intermediate A-4: Cyclobutyl L-alaninate hydrochloride**

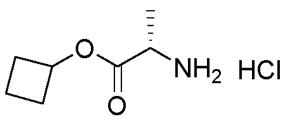

A-4

The following synthetic routes were adopted:

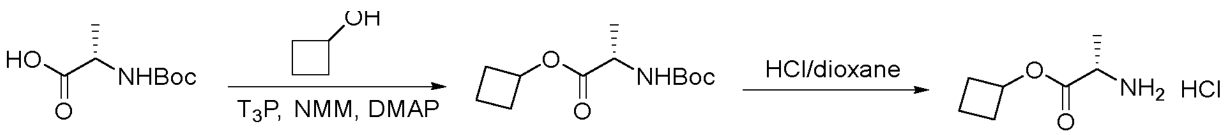


A-4

Step 1 Synthesis of cyclobutyl (tert-butoxycarbonyl)-L-alaninate

(tert-Butoxycarbonyl)-L-alanine (4.91 g, 26 mmol) and cyclobutanol (1.68 g, 23.4 mmol) were added to a reaction flask, followed by the addition of 50 mL of anhydrous dichloromethane. The mixture was cooled to 0°C under nitrogen atmosphere. NMM (7.08 g, 70.2 mmol), 1-propylphosphonic acid cyclic anhydride (16.8 mL, 28 mmol), and DMAP (57.6 mg, 0.47 mmol) were added sequentially. After the addition, the mixture was warmed to room temperature and stirred for 2-4 hours. After completion of the reaction as monitored by TLC, the reaction was quenched with water (40 mL). The organic layers were separated, washed with saturated saline solution, concentrated, and purified by silica gel column chromatography to obtain 2.4 g of white solid (yield: 42%). $^1H$ NMR (400 MHz, DMSO) δ 7.95 (s, 1H), 6.95 (s, 1H), 6.86 (s, 1H), 5.92 - 5.76 (m, 1H), 5.50 (d, $J$ = 12.9 Hz, 2H), 4.92 - 4.76 (m, 2H), 4.65 (s, 1H), 4.17 (d, $J$ = 24.2 Hz, 3H), 3.96 (d, $J$ = 36.1 Hz, 3H), 3.67 (s, 2H), 2.22 (s, 2H), 2.06 - 1.87 (m, 2H), 1.70 (d, $J$ = 9.6 Hz, 1H), 1.55 (d, $J$ = 9.3 Hz, 1H), 1.23 (m, 9H).

Step 2 Synthesis of Intermediate A-4

The compound (tert-butoxycarbonyl)-L-alaninate (2.4 g, 9.9 mmol) obtained from the previous step was added to a reaction flask, followed by addition of 4 N hydrogen chloride solution in dioxane (25 mL, 100 mmol). The mixture was stirred at room temperature for 1-2 hours. After completion of the reaction as monitored by TLC, the solvent was removed by concentration. The crude product was used directly in the next step without further purification. LC-MS (APCI): m/z=144.4 $(M+1)^+$.

**Preparation of Intermediate A-5: 2,2-dimethylbutyl L-alaninate hydrochloride**

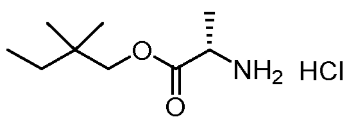

A-5

The following synthetic routes were adopted:

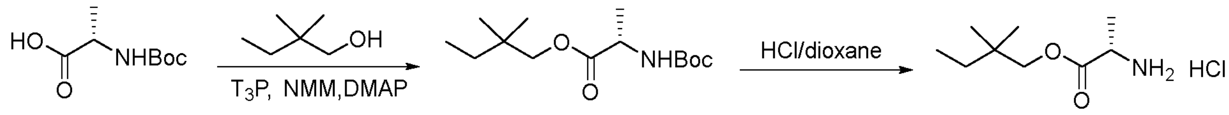


A-5

Step 1 Synthesis of 2,2-dimethylbutyl (tert-butoxycarbonyl)-L-alaninate

(tert-Butoxycarbonyl)-L-alanine (4.91 g, 26 mmol) and 2,2-dimethyl-1-butanol (2.4 g, 23.4 mmol) were added to a reaction flask, followed by the addition of 50 mL of anhydrous dichloromethane. The mixture was cooled to 0°C under nitrogen atmosphere. NMM (7.08 g, 70.2 mmol), 1-propylphosphonic acid cyclic anhydride (16.8 mL, 28 mmol), and DMAP (57.6 mg, 0.47 mmol) were added sequentially. After the addition, the mixture was warmed to room temperature and stirred for 2-4 hours. After completion of the reaction as monitored by TLC, the reaction was quenched with water (40 mL). The organic layers were separated, washed with saturated saline solution, concentrated, and purified by silica gel column chromatography to obtain 1.8 g of white solid (yield: 28%). LC-MS (APCI): m/z=274.6 $(M+1)^+$.

Step 2 Synthesis of Intermediate A-5

The compound 2,2-dimethylbutyl (tert-butoxycarbonyl)-L-alaninate (1.8 g, 6.6 mmol) obtained from the previous step was added to a reaction flask, followed by addition of 4 N hydrogen chloride solution in dioxane (16.5 mL, 66 mmol). The mixture was stirred at room temperature for 1-2 hours. After completion of the reaction as monitored by TLC, the solvent was removed by concentration. The crude product was used directly in the next step without further purification. LC-MS (APCI): m/z=174.5 $(M+1)^+$.

**Preparation of Intermediate A-6: S-2-hydroxyethyl 2,2-dimethyl thiopropionate**

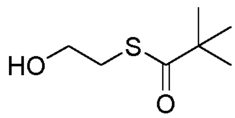

A-6

The following synthetic routes were adopted:

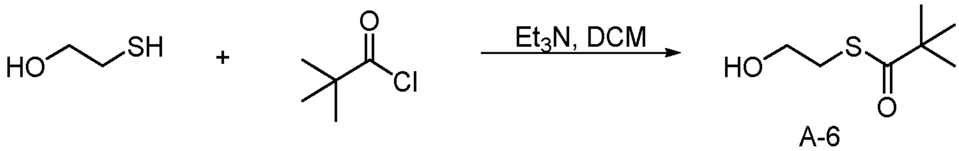


A-6

2-Mercaptoethanol (3.9 g, 50 mmol) was dissolved in anhydrous dichloromethane (40 mL) and cooled to -78 °C under a nitrogen atmosphere. Triethylamine (7.6 g, 75 mmol) was added, followed by dropwise addition of a solution of pivaloyl chloride (6.6 g, 55 mmol) in dichloromethane (10 mL). After the addition, the mixture was stirred at low temperature for 2-3 hours. After completion of the reaction as monitored by TLC, the reaction was quenched with water (20 mL). The organic layers were separated, washed with saturated saline solution, concentrated, and purified by column chromatography to obtain 6.9 g of colorless oily liquid, i.e., compound 11 (yield: 86%). LC-MS(APCI): m/z=163.6$(M+1)^+$.

**Preparation of Intermediate A-7: 3-(hexadecyloxy)propane-1-ol**

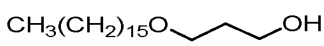

The following synthetic routes were adopted:

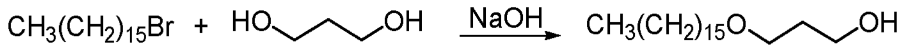


A-7

Bromohexadecane (1.52 g, 5 mmol) and 1,3-propanediol (1.14 g, 15 mmol) were added into a reaction flask, followed by the addition of dimethyl sulfoxide (5 mL) and tetrahydrofuran (5 mL).

Sodium hydroxide (800 mg, 20 mmol) was added, and the mixture was stirred at room temperature for 24 hours. The mixture was diluted with water (10 mL), and the pH was adjusted to neutral using 2 M aqueous hydrochloric acid. The resulting mixture was extracted with ethyl acetate 3 times. The organic layers were combined, washed with saturated saline solution, concentrated, and purified by silica gel column chromatography to obtain 1.0 g of compound (yield: 67%). LC-MS(APCI): m/z=301.3$(M+1)^+$.

**Preparation of Intermediate A-8: Dodecyl L-alaninate hydrochloride**

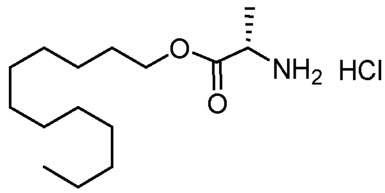

A-8

According to the preparation method of intermediate A-4, n-dodecanol (0.93 g, 5 mmol) was used instead of cyclobutanol (1.68 g, 23.4 mmol) to prepare intermediate A-8. LC-MS (APCI): m/z=258.6 $(M+1)^+$.

**Preparation of Intermediate A-9: Octadeca-9,12-dien-1-yl L-alaninate (9Z,12Z) hydrochloride**

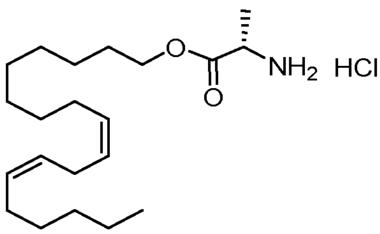

A-9

According to the preparation method of intermediate A-4, (9Z,12Z)-octadeca-9,12-dien-1-ol (1.33 g, 5 mmol) was used instead of cyclobutanol (1.68 g, 23.4 mmol) to prepare intermediate A-9. LC-MS (APCI): m/z=338.6 $(M+1)^+$.

**Preparation of Intermediate A-10: 1.4-bicyclobutyl L-aspartate hydrochloride**

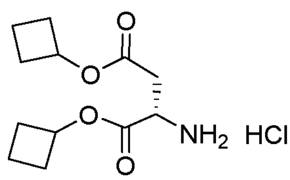

A-10

The following synthetic routes were adopted:

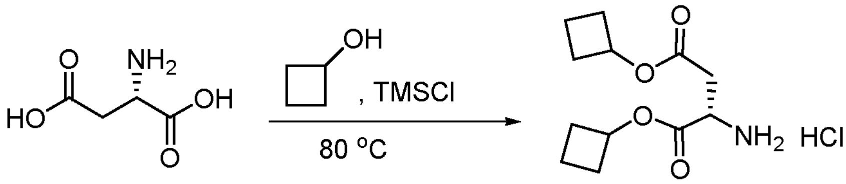


A-10

L-Aspartic acid (1.33 g, 10 mmol), cyclobutanol (10 mL), and trimethylchlorosilane (3.3 g, 30 mmol) were added into a reaction flask. The mixture was heated to 80 °C under a nitrogen atmosphere and stirred overnight. After completion of the reaction as monitored by TLC, the solvent was removed by concentration. The crude product was used directly in the next step without purification. LC-MS (APCI): m/z=242.5 $(M+1)^+$.

**Preparation of Intermediate A-11: 1,4-dicyclopentyl L-aspartate hydrochloride**

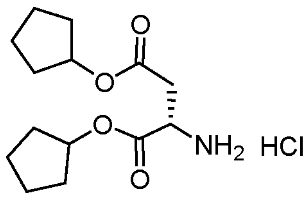

A-11

The following synthetic routes were adopted:

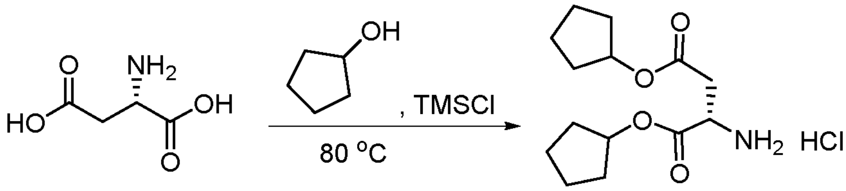


A-11

L-Aspartic acid (1.33 g, 10 mmol), cyclopentanol (10 mL), and trimethylchlorosilane (3.3 g, 30 mmol) were added into a reaction flask. The mixture was heated to 80 °C under a nitrogen atmosphere and stirred overnight. After completion of the reaction as monitored by TLC, the solvent was removed by concentration. The crude product was used directly in the next step without purification. LC-MS (APCI): m/z=270.1 $(M+1)^+$.

**Preparation of Intermediate A-12: 1,4-bis(2-ethyl butyl) L-aspartate hydrochloride**

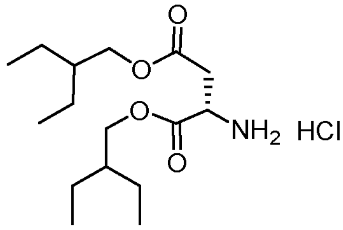

A-12

The following synthetic routes were adopted:

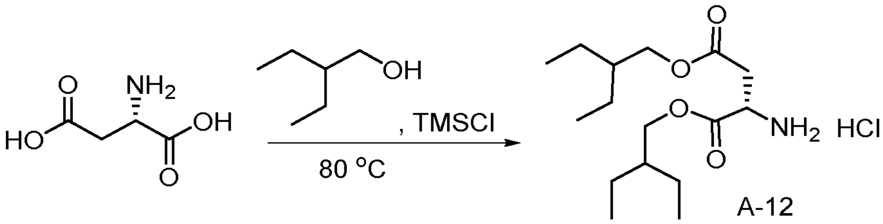


A-12

L-Aspartic acid (1.33 g, 10 mmol), 2-ethyl-1-butanol (10 mL), and trimethylchlorosilane (3.3 g, 30 mmol) were added into a reaction flask. The mixture was heated to 80 °C under a nitrogen atmosphere and stirred overnight. After completion of the reaction as monitored by TLC, the solvent was removed by concentration. The crude product was used directly in the next step without purification. LC-MS (APCI): m/z=302.3 $(M+1)^+$.

**Preparation of Intermediate A-13: cyclobutyl L-phenylalaninate hydrochloride**

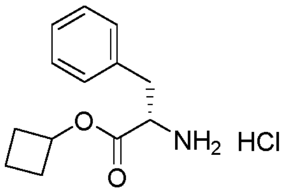

A-13

According to the preparation method of intermediate A-4, (tert-butoxycarbonyl)-L-phenylalanine (1.32 g, 5 mmol) was used instead of (tert-butoxycarbonyl)-L-alanine (4.91 g, 26 mmol) to obtain intermediate A-13. LC-MS (APCI): m/z=220.6 $(M+1)^+$.

**Preparation of Intermediate A-14: 2-methoxy-2-methyl propyl L-phenylalanine hydrochloride**

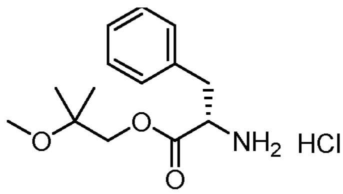

A-14

According to the preparation method of intermediate A-4, (tert-butoxycarbonyl)-L-phenylalanine (1.32 g, 5 mmol) and 2-methoxy-2-methyl-1-propanol (0.52 g, 5 mmol) were used instead of (tert-butoxycarbonyl)-L-alanine (4.91 g, 26 mmol) and cyclobutanol (1.68 g, 23.4 mmol), respectively, to prepare intermediate A-14. LC-MS (APCI): m/z=252.4 $(M+1)^+$.

**Preparation of Intermediate B-1: isopropyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino) propionate,**

**B-1a: isopropyl (S)-2-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate and**

**B-1b: isopropyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate**

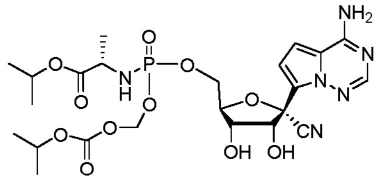

B-1

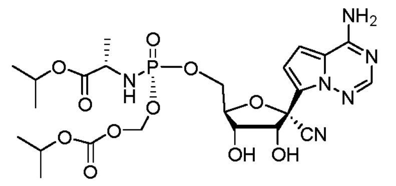

B-1a

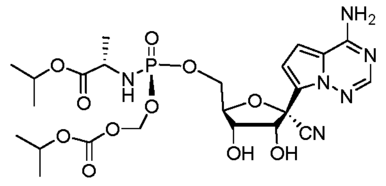

B-1b

The following synthetic routes were adopted:

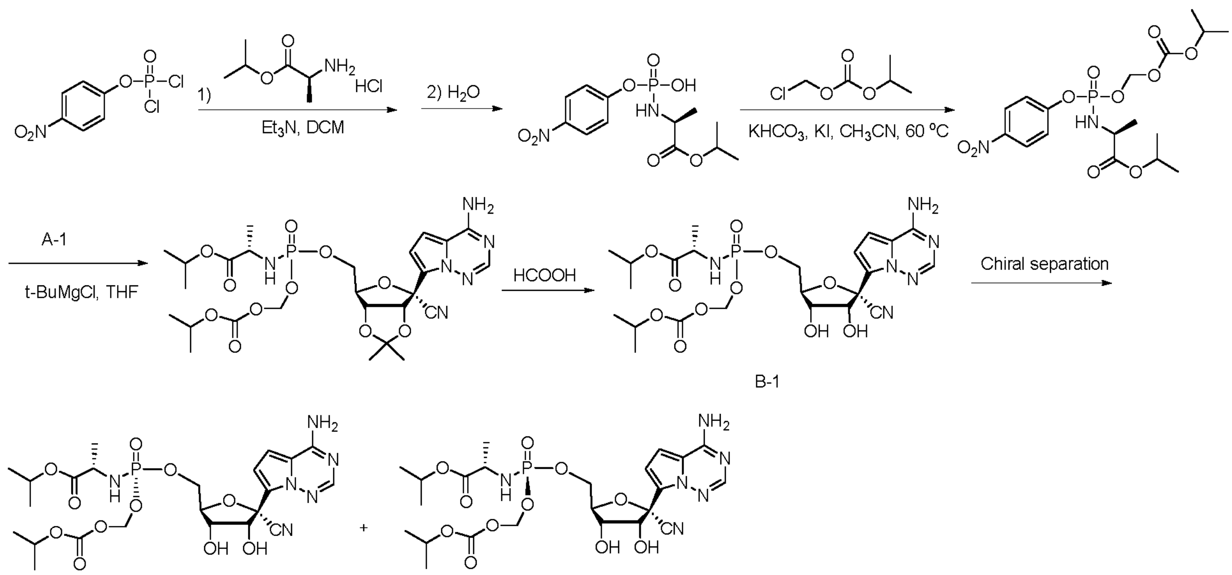


B-1

B-1a

B-1b

Step 1 Synthesis of isopropyl (hydroxy(4-nitrophenoxy)phosphoryl)-L-alaninate

4-Nitrophenyl phosphorodichloridate (5.0 g, 19.6 mmol) was added to a reaction flask, followed by the addition of 30 mL of anhydrous dichloromethane to dissolve. The mixture was cooled to -78°C under nitrogen atmosphere, followed by dropwise addition of isopropyl L-alaninate hydrochloride (3.3 g, 19.6 mmol) in 10 mL dichloromethane and triethylamine (5.95 g, 58.8 mmol) sequentially. After the addition, the reaction was continued at low temperature for 1-2 hours with stirring. After completion of the reaction as monitored by TLC, 10 mL of water was added and the mixture was slowly warmed to room temperature over 0.5 hour. Reaction completion was confirmed by LC-MS. The solvent was removed by concentration. The crude product was directly used in the next step.

Step 2 Synthesis of isopropyl ((((isopropoxycarbonyl)oxy)methoxy)(4-nitrophenoxy)phosphoryl)-L-alaninate

The intermediate isopropyl (hydroxy(4-nitrophenoxy)phosphoryl)-L-alaninate from the previous step was dissolved in acetonitrile (80 mL). Isopropyl chloromethyl carbonate (5.91 g, 38.7 mmol), potassium bicarbonate (3.88 g, 38.7 mmol), and potassium iodide (6.43 g, 38.7 mmol) were added sequentially. The mixture was heated to 60 °C under nitrogen atmosphere and stirred overnight. After completion of the reaction as monitored by TLC, the solvent was removed by concentration. The residue was purified by silica gel column chromatography (petroleum ether/ethyl acetate 2: 1) to obtain 3.7 g of colorless oily liquid (yield: 42%). LC-MS (APCI): m/z=449.7 $(M+1)^+$.

Step 3 Synthesis of isopropyl (S)-2-(((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxole-4-yl)methoxy)(((isopropoxycarbonyl)oxy)methoxy)phosphoryl)amino)propionate

The intermediate A-1 (0.83 g, 2.5 mmol) and isopropyl ((((isopropoxycarbonyl)oxy)methoxy)(4-nitrophenoxy) phosphoryl)-L-alaninate (1.66 g, 3.7 mmol) were added to a reaction flask, followed by the addition of 30 mL of anhydrous tetrahydrofuran. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly

warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 1: 1) to obtain 0.94 g of colorless oily liquid (yield: 59%). LC-MS (APCI): m/z=641.7 $(M+1)^+$.

Step 4 Synthesis of B-1

(S)-Isopropyl 2-(((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxol-4-yl)methoxy)(((isopropoxycarbonyl)oxy)methoxy)phosphoryl)amino)propanoate (1.15 g, 1.8 mmol) was added to a reaction flask, followed by the additon of formic acid (20 mL), and stirred at room temperature overnight. After completion of the reaction as monitored by TLC, the solvent was removed by concentration. The residue was purified by silica gel column chromatography to obtain 637 mg of white solid (yield: 59%). LC-MS (APCI): m/z=601.5 $(M+1)^+$. $^1H$ NMR (400 MHz, DMSO-$d_6$) δ 7.93 (s, 1H), 6.95 (d, *J*= 3.6 Hz, 1H), 6.80 (d, *J*= *4.5* Hz, 1H), 6.02 (d, *J* = 5.8 Hz, 1H), 5.84 (dd, *J* = 22.5, 12.4 Hz, 1H), 5.55 - 5.32 (m, 3H), 4.90 - 4.72 (m, 2H), 4.58 (dd, *J* = 15.2, 3.8 Hz, 1H), 4.26 - 4.04 (m, 2H), 3.59 (ddd, *J* = 26.9, 16.9, 9.7 Hz, 1H), 1.23 (d, *J* = 6.2 Hz, 6H), 1.20 - 1.09 (m, 9H).

Step 5 Synthesis of B-1a and B-1b

The racemate compound B-1 was separated with reverse phase preparative chromatography to obtain the target product B-1a (retention time: 8.34 min, relative content: 53.3%) and B-1b (retention time: 9.21 min, relative content: 46.7%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-2: 2-ethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate,**

**B-2a: 2-ethyl butyl (S)-2-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate and**

**B-2b: 2-ethyl butyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate**

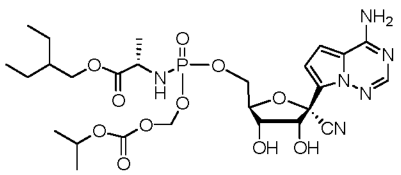

B-2

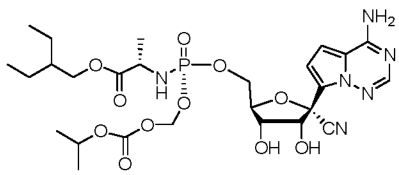

B-2a

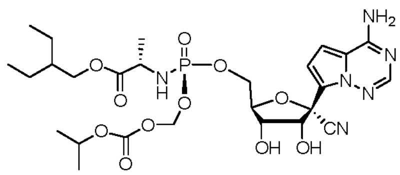

B-2b

Following the synthetic procedure for intermediate B-1, 2-ethyl butyl L-alaninate hydrochloride (2.09 g, 10 mmol) was used instead of isopropyl L-alaninate hydrochloride (3.3 g, 19.6 mmol) to obtain compound B-2 (1.41 g, overall yield: 22%) as a white solid. LC-MS (APCI): m/z=643.1 $(M+1)^+$. $^1H$ NMR (400 MHz, DMSO-$d_6$) δ 7.94 (s, 1H), 6.92 (d, *J*= *4.5* Hz, 1H), 6.80 (d, *J*= *4.5* Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 2H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 1.46 (dt, *J* = 12.3, 6.1 Hz, 1H), 1.33 - 1.00 (m, 13H), 0.82 (t, *J* = 7.4 Hz, 6H).

The racemate compound B-2 was separated with reverse phase preparative chromatography to obtain the target product B-2a (retention time: 10.6 min, relative content: 52.9%) and B-2b (retention time: 11.4 min, relative content:

47.1%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-3: 2-ethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)((pivaloyloxy)methoxy)phosphoryl)amino)propionate,**

**B-3a: 2-ethyl butyl (S)-2-(((R)-(((2R,3S,4R,5R)-5-(4-aminopyrrolo[2,1-f][1,2,41triazin-7-yl)-5-cyano-3,4-dihydroxytetrahydrofuran-2-yl)methoxy)((pivaloyloxy)methoxy)phosphoryl)amino)propionate and**

**B-3b: 2-ethyl butyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)((pivaloyloxy)methoxy)phosphoryl)amino)propionate**

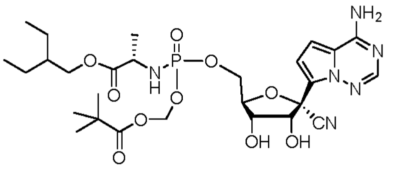

B-3

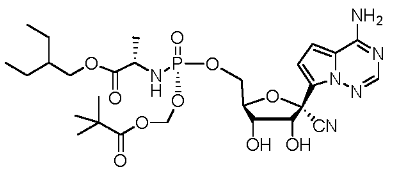

B-3a

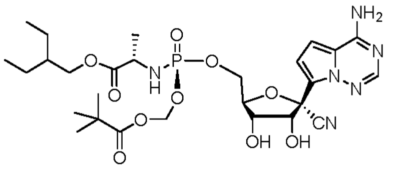

B-3b

Following the synthetic procedure for intermediate B-1, 2-ethylbutyl L-alaninate hydrochloride (1.87 g, 5 mmol) and chloromethyl pivalate (1.5 g, 10 mmol) were used instead of isopropyl L-alaninate hydrochloride (3.3 g, 19.6 mmol) and isopropyl chloromethyl carbonate (5.91 g, 38.7 mmol), respectively, to obtain compound B-3 (0.74 g, overall yield: 23%) as a white solid. LC-MS (APCI): m/z 641.2 $[M+1]^+$. $^1H$ NMR (400 MHz, DMSO-$d_6$) δ 7.94 (s, 1H), 6.92 (d, *J= 4.5* Hz, 1H), 6.80 (d, *J*=4.5 Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 1H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 1.46 (dt, *J* = 12.3, 6.1 Hz, 1H), 1.33 - 0.85 (m, 22H).

Step 2 Synthesis of B-3a and B-3b

The racemate compound B-3 was separated with reverse phase preparative chromatography to obtain the target product B-3a (retention time: 8.14 min, relative content: 53.8%) and B-3b (retention time: 8.82 min, relative content: 46.2%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-4: cyclobutyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate,**

**B-4a: cyclobutyl (S)-2-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate and**

**B-4b: cyclobutyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate**

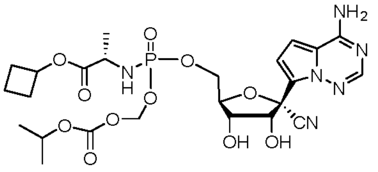

B-4

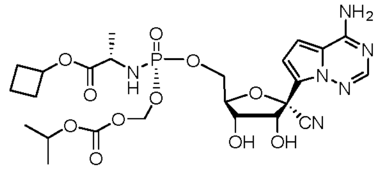

B-4a

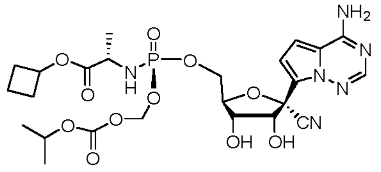

B-4b

Following the synthetic procedure for intermediate B-1, intermediate A-4 (1.43 g, 10 mmol) was used instead of isopropyl L-alaninate hydrochloride (3.3 g, 19.6 mmol) to obtain compound B-4 (2.26 g, overall yield: 37%) as a white solid. LC-MS (APCI): m/z 613.5 $[M+1]^+$. $^1H$ NMR (400 MHz, DMSO-$d_6$).

Step 4 Synthesis of B-4a and B-4b

The racemate compound B-4 was separated with reverse phase preparative chromatography to obtain the target product B-4a (retention time: 7.82 min, relative content: 54.6%) and B-4b (retention time: 8.35 min, relative content: 45.4%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-5: 2,2-dimethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate,**

**B-5a: 2,2-dimethyl butyl (S)-2-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate and**

**B-5b: 2,2-dimethyl butyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)propionate**

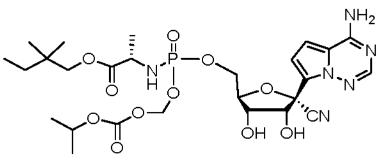

B-5

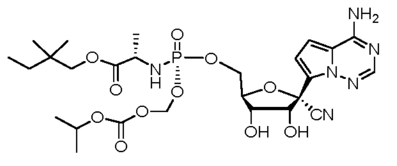

B-5a

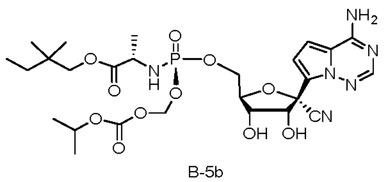

B-5b

Following the synthetic procedure for intermediate B-1, intermediate A-5 (2.09 g, 10 mmol) was used instead of isopropyl L-alaninate hydrochloride (3.3 g, 19.6 mmol) to obtain compound B-5 (1.41 g, overall yield: 22%) as a white solid. LC-MS (APCI): m/z 643.1 $[M+1]^+$. $^1H$ NMR (400 MHz, DMSO-$d_6$) δ 7.94 (s, 1H), 6.92 (d, *J* = 4.5 Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 2H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 1.46 (dt, *J* = 12.3, 6.1 Hz, 1H), 1.33 - 0.88 (m, 19H).

The racemate compound B-5 was separated with reverse phase preparative chromatography to obtain the target product B-5a (retention time: 8.54 min, relative content: 51.2%) and B-5b (retention time: 9.62 min, relative content: 48.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-6: ethyl 2-((((((2R,3S,4R,5R)-5-(4-aminopyrrolo[2,1-f][1,2,4]triazin-7-yl)-5-cyano-3,4-dihydroxytetrahydrofuran-2-yl)methoxy)(((S)-1-(2-ethylbutoxy)-1-oxopropan-2-yl)amino)phosphoryl)oxy)methyl)benzoate,**

**B-6a: Ethyl 2-((((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((S)-1-(2-ethylbutoxy)-1-oxopropan-2-yl)amino)phosphoryl)oxy)methyl)benzoate and**

**B-6b: Ethyl 2-((((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((S)-1-(2-ethylbutoxy)-1-oxopropan-2-yl)amino)phosphoryl)oxy)methyl)benzoate**

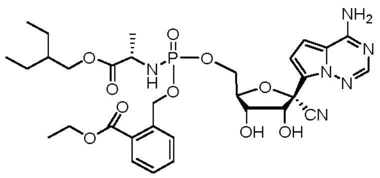
B-6

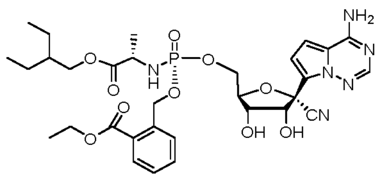
B-6a

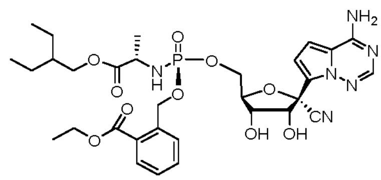
B-6b

The following synthetic routes were adopted:

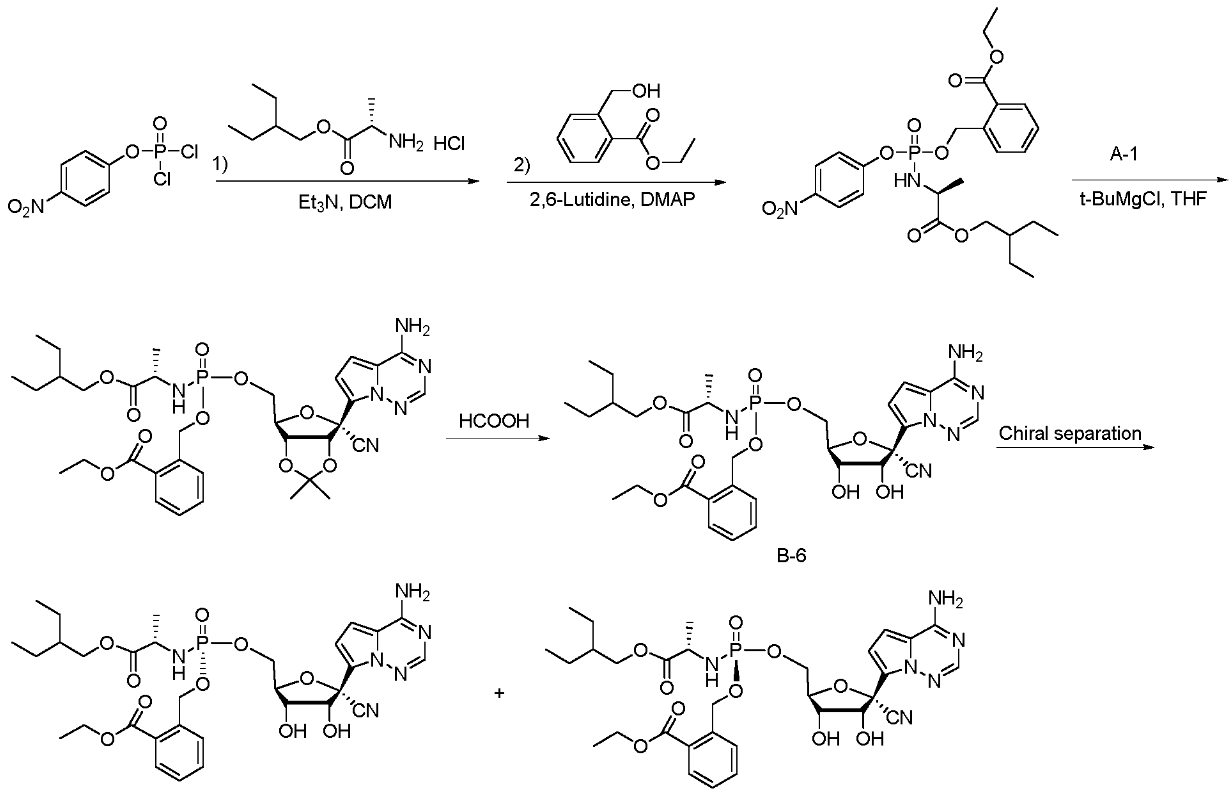

B-6
B-6a
B-6b

Step 1 Synthesis of ethyl 2-((((((S)-1-(2-ethylbutoxy)-1-oxopropan-2-yl)amino)(4-nitrophenoxy)phosphoryl)oxy)methyl) benzoate

4-nitrophenylphosphorus dichloride (1.27 g, 5.0 mmol) was dissolved in anhydrous dichloromethane (20 mL), the solution was cooled to -78°C under nitrogen atmosphere, and a mixed solution of triethylamine (759 mg, 7.5 mmol) and 2-ethyl butyl L-alaninate hydrochloride (1.05 g, 5.0 mmol) in 10 mL dichloromethane was slowly added dropwise, stirred for reaction at low temperature for 1-2 hours. After completion of the reaction as monitored by TLC, ethyl 2-hydroxymethylbenzoate (1.08 g, 6.0 mmol), 2,6-dimethylpyridine (1.1 g, 10 mmol) and DMAP (305 mg, 2.5 mmol) were added, raised to room temperature and stirred for reaction overnight. After completion of the reaction as monitored by TLC, excessive water was added for dilution. The mixture was extracted with dichloromethane for 3-4 times. The organic layers were combined, washed with saturated saline solution, concentrated and purified by silica gel column chromatography to obtain 1.02 g of light yellow oily liquid (yield: 38%). LC-MS(APCI): m/z=537.5$(M+1)^+$.

Step 2 Synthesis of ethyl 2-((((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxole-4-yl)methoxy)(((S)-1-(2-ethylbutoxy)-1-oxopropan-2-yl)amino)phosphoryl)oxy)methyl) benzoate

The intermediate A-1 (0.83 g, 2.5 mmol) and ethyl 2-((((((S)-1-(2-ethylbutoxy)-1-oxopropan-2-yl)amino)(4-nitrophenoxy)phosphoryl)oxy)methyl)benzoate (1.98 g, 3.7 mmol) were added into a reaction flask, followed by the addtion of 30 mL of anhydrous tetrahydrofuran to dissolve. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 1:1) to obtain 1.35 g of light yellow oily liquid (yield: 74%). LC-MS (APCI): m/z=729.3 $(M+1)^+$.

Step 3 Synthesis of Intermediate B-6

Ethyl 2-((((((3aR,4R,6R,6aR)-6-(4-aminopyrrolo[2,1-f][1,2,4]triazin-7-yl)-6-cyano-2,2-dimethyltetrahydrofuro [3,4-d][1,3]dioxol-4-yl)methoxy)(((S)-1-(2-ethylbutoxy)-1-oxopropan-2-yl)amino)phosphoryl)oxy)methyl)benzoate (320 mg, 0.44 mmol) was added into a reaction flask, followed by the addtion of formic acid (10 mL). The mixture was stirred at room temperature overnight. After completion of the reaction as monitored by TLC, the solvent was removed by concentration. The residue was purified by silica gel column chromatography to obtain 154 mg of light yellow solid (yield: 51%). LC-MS (APCI): m/z=689.7 $(M+1)^+$. $^1$H NMR (400 MHz, DMSO-$d_6$) $\delta$ 7.93 (s, 1H), 7.44 (d, $J$ = 3.5 Hz,1H), 7.41 (t, $J$ = 3.5 Hz, 1H), 7.22 (m, 2H), 6.90 (d, $J$ = 4.5 Hz, 1H), 6.80 (d, $J$ = 4.5 Hz, 1H), 6.06 (d, $J$ = 5.8 Hz, 1H), 5.86 (dd, $J$ = 12.7, 10.1 Hz, 1H), 5.51 - 5.39 (m, 2H), 4.80 (dt, $J$ = 12.5, 6.2 Hz, 2H), 4.61 (d, $J$ = 3.5 Hz, 2H), 4.25 - 4.10 (m, 6H), 3.99 (dd, $J$ = 10.8, 5.9 Hz, 1H), 2.1 (dd, $J$ = 16.8, 9.6 Hz, 2H), 1.32 - 0.85 (m, 16H).

Step 4 Synthesis of B-6a and B-6b

The racemate compound B-6 was separated with reverse phase preparative chromatography to obtain the target product B-6a (retention time: 8.14 min, relative content: 52.2%) and B-6b (retention time: 9.02 min, relative content: 47.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-7: 2-ethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)((2-methyl benzyl)oxy)phosphoryl)amino)propionate,**

**B-7a: 2-ethyl butyl (S)-2-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)((2-methyl benzyl)oxy)phosphoryl)amino)propionate and**

**B-7b: 2-ethyl butyl (S)-2-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)((2-methyl benzyl)oxy)phosphoryl)amino)propionate**

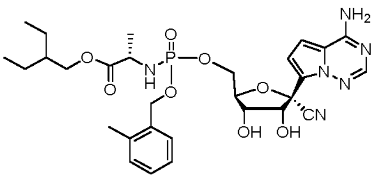
B-7

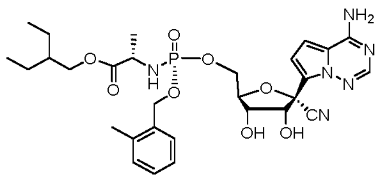
B-7a

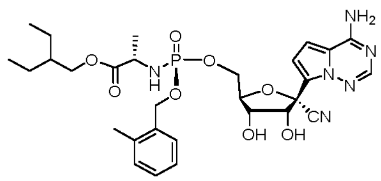
B-7b

Following the synthetic procedure for intermediate B-6, 2-methylbenzyl alcohol (1.46 g, 12 mmol) was used instead of ethyl 2-(hydroxymethyl)benzoate (1.08 g, 6.0 mmol) to obtain compound B-7 (2.08 g, overall yield: 33%) as a white solid. LC-MS (APCI): m/z 631.1 $[M+1]^+$. $^1$H NMR (400 MHz, DMSO-$d_6$) δ 7.93 (s, 1H), 7.44 (*d, J* = 3.5 Hz,1H), 7.41 (t, *J* = 3.5 Hz, 1H), 7.22 (m, 2H), 6.90 (d, *J* = 4.5 Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 2H), 4.80 (dt, *J* = 12.5, 6.2 Hz, 2H), 4.61 (d, *J= 3.5* Hz, 2H), 4.25 - 4.10 (m, 4H), 3.99 (dd, *J* = 10.8, 5.9 Hz, 1H), 2.2 (s, 3H), 2.1 (dd, *J* = 16.8, 9.6 Hz, 2H), 1.32 - 0.85 (m, 13H).

The racemate compound B-7 was separated with reverse phase preparative chromatography to obtain the target product B-7a (retention time: 8.24 min, relative content: 53.3%) and B-7b (retention time: 9.07 min, relative content: 46.7%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-8: 2-ethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)((4-(tert-butyl)benzyl)oxy)phosphoryl)amino)propionate,**

**B-8a: 2-ethyl butyl (S)-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)((4-(tert-butyl)benzyl)oxy)phosphoryl)amino)propionate and**

**B-8b: 2-ethyl butyl (S)-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)((4-(tert-butyl)benzyl)oxy)phosphoryl)amino)propionate**

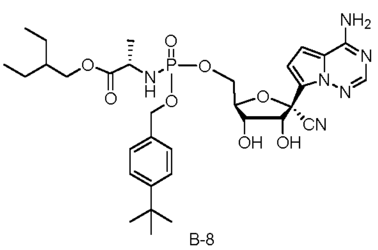
B-8

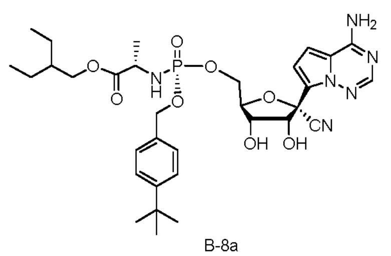
B-8a

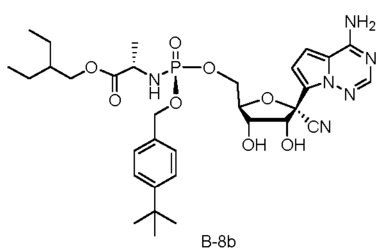
B-8b

Following the synthetic procedure for intermediate B-6, 4-tert-butylbenzyl alcohol (1.97 g, 12 mmol) was used instead of ethyl 2-(hydroxymethyl)benzoate (1.08 g, 6.0 mmol) to obtain compound B-8 (1.61 g, overall yield: 24%) as a white solid. LC-MS (APCI): m/z 673.4 $[M+1]^+$. $^1$H NMR (400 MHz, DMSO-$d_6$) δ 7.93 (s, 1H), 7.44 (*d, J* = 3.5 Hz,1H), 7.41 (t, *J= 3.5* Hz, 1H), 7.22 (m, 2H), 6.90 (d, *J* = 4.5 Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 2H), 4.80 (dt, *J* = 12.5, 6.2 Hz, 2H), 4.61 (d, *J= 3.5* Hz, 2H), 4.25 - 4.10 (m, 4H), 3.99 (dd, *J* = 10.8, 5.9 Hz, 1H), 2.1 (dd, *J* = 16.8, 9.6 Hz, 2H), 1.35 - 0.88 (m, 22H).

The racemate compound B-8 was separated with reverse phase preparative chromatography to obtain the target

product B-8a (retention time: 7.37 min, relative content: 55.0%) and B-8b (retention time: 8.23 min, relative content: 45.0%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate** B-9: **(((((2R,3S,4R,5R)-5-(4-aminopyrrolo[2,1-f][1,2,4]triazin-7-yl)-5-cyano-3,4-dihydroxytetrahydrofuran-2-yl)methoxy)phosphoryl)bis(oxy))bis(methylene)diisopropyl bis(carbonate)**

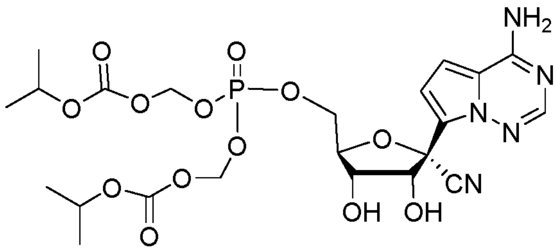

B-9

The following synthetic routes were adopted:

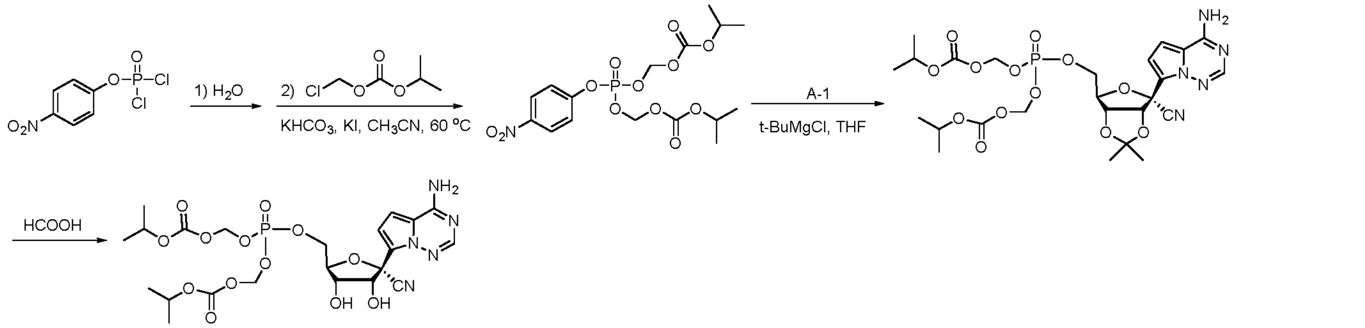


B-9

Step 1 Synthesis of (((4-nitrophenoxy)phosphoryl)bis(oxy))bis(methylene)diisopropyl bis(carbonate)

4-Nitrophenyl phosphorodichloridate (2.2 g, 8.6 mmol) was added to a reaction flask, followed by the addtion of 25 mL of tetrahydrofuran to dissolve. Purified water (5 mL) was added dropwise, and the mixture was stirred at room temperature for 2 h. The solvent was removed by concentration. The crude product was used directly in the next step.

The intermediate from the previous step was dissolved in 40 mL of acetonitrile. Isopropyl chloromethyl carbonate (3.28 g, 21.5 mmol), potassium bicarbonate (2.15 g, 21.5 mmol), and potassium iodide (2.85 g, 17.2 mmol) were added sequentially. After the addition, the mixture was heated to 60°C under nitrogen atmosphere and stirred overnight. After completion of the reaction as monitored by TLC, the solvent was removed by concentration. The residue was purified by silica gel column chromatography (petroleum ether/ethyl acetate 3:1) to obtain 1.74 g of colorless oily liquid (yield: 45%). LC-MS (APCI): m/z=452.7 $(M+1)^+$.

Step 2 Synthesis of (((((2R,3S,4R,5R)-6-(4-aminopyrrolo[2,1-f][1,2,4]triazin-7-yl)-6-cyano-2,2-dimethyltetrahydrofuro [3,4-d][1,3]dioxole-4-yl)methoxy)phosphoryl)bis(oxy))bis(methylene)diisopropyl bis(carbonate)

The intermediate A-1 (0.82 g, 2.5 mmol) and ((((4-nitrophenoxy)phosphoryl)bis(oxy))bis(methylene)) diisopropyl bis(carbonate) (1.68 g, 3.7 mmol) were added into a reaction flask, followed by the addtion of 30 mL of anhydrous tetrahydrofuran. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly

warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 1:1) to obtain 1.04 g of colorless oily liquid (yield: 65%). LC-MS (APCI): m/z=644.2 $(M+1)^+$.

Step 3 Synthesis of Intermediate B-9

(((((2R,3 S,4R,5R)-6-(4-Aminopyrrolo[2,1-f][1,2,4]triazin-7-yl)-6-cyano-2,2-dimethyltetrahydrofuro[3,4-d][1,3]dioxol-4-yl)methoxy)phosphoryl)bis(oxy))bis(methylene) diisopropyl dicarbonate (1.04 g, 1.6 mmol) was added into a reaction flask, followed by the addtion of 20 mL of formic acid. The mixture was stirred at room temperature overnight. After completion of the reaction as monitored by TLC, the solvent was removed by concentration. The residue was purified by silica gel column chromatography to obtain 608 mg of white solid (yield: 63%). LC-MS (APCI): m/z=604.3 $(M+1)^+$. $^1H$ NMR (400 MHz, DMSO-$d_6$) $\delta$ 8.04 (s, 1H), 7.07 (d, *J* = 4.5 Hz, 1H), 6.87 (d, *J* = 4.5 Hz, 1H), 5.57 (d, *J* = 13.6 Hz, 4H), 4.82 (td, *J* = 12.2, 6.1 Hz, 2H), 4.65 (d, *J* = 4.7 Hz, 1H), 4.30 (m, 1H), 4.11 (m, 1H), 3.96 - 3.88 (m, 2H), 1.24 (d, *J* = 6.4 Hz, 12H).

**Preparation of Intermediate B-10: (((((2R,3S,4R,5R)-5-(4-amino-5-deuteriopyrrolo[2,1-f][1,2,4]triazin-7-yl)-5-cyano-3,4-dihydroxytetrahydrofuran-2-**yl)methoxy)phosphoryl)bis(oxy))bis(methylene)diisopropyl bis(carbonate)

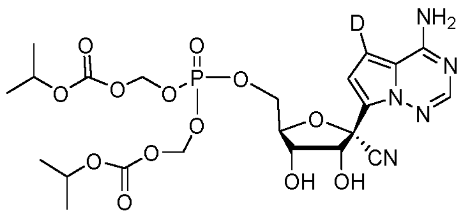

B-10

Following the synthetic procedure for intermediate B-9, intermediate A-3 (0.82 g, 2.5 mmol) was used instead of intermediate A-1 (0.82 g, 2.5 mmol) to obtain 521 mg of compound B-10 (yield: 54%) as a white solid. LC-MS (APCI): m/z=605.5 $(M+1)^+$. $^1H$ NMR (400 MHz, DMSO-$d_6$) $\delta$ 8.08 (s, 1H), 6.88 (s, 1H), 5.52 (d, *J* = 13.6 Hz, 4H), 4.81 (td, *J* = 12.0, 6.0 Hz, 2H), 4.63 (d, *J*= 4.7 Hz, 1H), 4.30 (m, 1H), 4.11 (m, 1H), 3.96 - 3.85 (m, 2H), 1.22 (d, *J* = 6.4 Hz, 12H).

Preparation of **Intermediate** B-11: (((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)oxy)methyl isopropyl carbonate

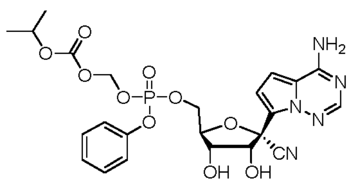

B-11

The following synthetic routes were adopted:

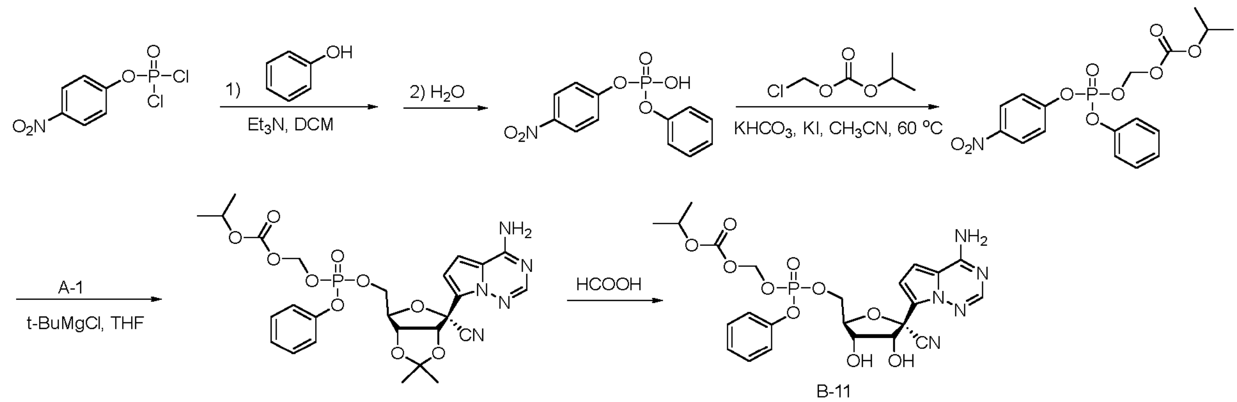


B-11

Step 1 Synthesis of (4-nitrophenyl)(phenyl)hydrophosphate

4-Nitrophenyl phosphorodichloridate (5.0 g, 19.6 mmol) was added to a reaction flask, followed by the addition of 30 mL of anhydrous dichloromethane to dissolve. The mixture was cooled to -78°C under nitrogen atmosphere, followed by dropwise addition of phenol (1.84 g, 19.6 mmol) in 5 mL dichloromethane and triethylamine (5.95 g, 58.8 mmol) sequentially. After the addition, the reaction was continued at low temperature for 1-2 hours with stirring. After completion of the reaction as monitored by TLC, 10 mL of water was added and the mixture was slowly warmed to room temperature over 0.5 hour. Reaction completion was confirmed by LC-MS. The solvent was removed by concentration. The crude product was directly used in the next step. LC-MS (APCI): m/z=296.4 $(M+1)^+$.

Step 2 Synthesis of isopropyl ((((4-nitrophenoxy)(phenoxy)phosphoryl)oxy)methyl)carbonate

The intermediate (4-nitrophenyl)(phenyl)phosphinic acid from the previous step was dissolved in acetonitrile (80 mL). Isopropyl chloromethyl carbonate (5.91 g, 38.7 mmol), potassium bicarbonate (3.88 g, 38.7 mmol), and potassium iodide (6.43 g, 38.7 mmol) were added sequentially. The mixture was heated to 60°C under nitrogen atmosphere and stirred overnight. After completion of the reaction as monitored by TLC, the solvent was removed by concentration. The residue was purified by silica gel column chromatography (petroleum ether/ethyl acetate 2:1) to obtain 3.4 g of colorless oily liquid (yield: 37%). LC-MS (APCI): m/z=468.7 $(M+1)^+$.

Step 3 Synthesis of isopropyl (((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxole-4-yl)methoxy)(phenoxy)phosphoryl)oxy)methyl carbonate

The intermediate A-1 (0.83 g, 2.5 mmol) and isopropyl ((((4-nitrophenoxy)(phenoxy)phosphoryl)oxy)methyl) carbonate (1.52 g, 3.7 mmol) were added to a reaction flask, followed by the addtion of 30 mL of anhydrous tetrahydrofuran. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 1:1) to obtain 1.07 g of colorless oily liquid (yield: 71%). LC-MS (APCI): m/z=604.8 $(M+1)^+$.

Step 4 Synthesis of Intermediate B-11

Isopropyl (((((3aR,4R,6R,6aR)-6-(4-aminopyrrolo[2,1-f][1,2,4]triazin-7-yl)-6-cyano-2,2-dimethyltetrahydrofuro [3,4-d][1,3]dioxol-4-yl)methoxy)(phenoxy)phosphoryl)oxy)methyl carbonate (1.07 g, 1.8 mmol) was added into a reaction flask, followed by the addtion of 20 mL of formic acid. The mixture was stirred at room temperature overnight. After completion of the reaction as monitored by TLC, the solvent was removed by concentration. The residue was purified by silica gel column chromatography to obtain 476 mg of white solid (yield: 47%). LC-MS (APCI): m/z=564.5 $(M+1)^+$. $^1H$ NMR (400 MHz, DMSO-$d_6$) δ 7.93 (d, $J$ = 1.5 Hz, 1H), 7.35 (dd, $J$ = 17.6, 8.1 Hz, 2H), 7.22 (dd, $J$ = 13.7, 7.1 Hz, 1H), 7.16 (d, $J$ = 7.7 Hz, 2H), 6.91 (t, $J$ = 4.7 Hz, 1H), 6.80 (d, $J$ = 4.4 Hz, 1H), 6.38 (s, 1H), 5.65 (dd, $J$ = 14.0, 2.6 Hz, 2H), 5.47 (s, 1H), 4.79 (td, $J$ = 12.3, 6.2 Hz, 1H), 4.69 (s, 1H), 4.46 - 4.37 (m, 1H), 4.30 (dd, $J$ = 15.1, 5.9 Hz, 2H), 3.97 (d, $J$ = 3.9 Hz, 1H), 1.24 - 1.16 (m, 6H).

**Preparation of Intermediate B-12: (((((2R,3S,4R,5R)-5-(4-aminopyrrolo[2,1-f][1,2,4]triazin-7-yl)-5-cyano-3,4-dihydroxytetrahydrofuran-2-yl)methoxy)(naphthalen-1-yloxy)phosphoryl)oxy)methyl isopropyl carbonate**

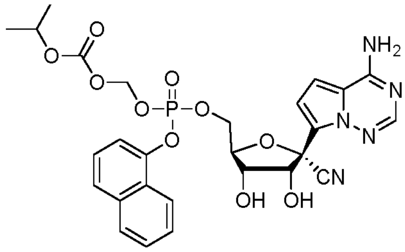

B-12

Following the synthetic procedure for intermediate B-11, 1-naphthol (0.94 g, 6.5 mmol) was used instead of phenol (1.84 g, 19.6 mmol) to obtain compound B-12 (637 mg, overall yield: 16%) as a white solid. LC-MS (APCI): m/z=614.1 $(M+1)^+$. $^1$H NMR (400 MHz, DMSO-$d_6$) δ 8.10 - 7.76 (m, 6H), 7.56 (dt, $J$ = 14.8, 6.8 Hz, 2H), 7.46 - 7.33 (m, 2H), 6.97 - 6.74 (m, 2H), 6.39 (s, 1H), 5.79 - 5.61 (m, 2H), 5.48 (s, 1H), 4.81 - 4.64 (m, 2H), 4.49 (s, 1H), 4.36 (d, $J$ = 29.1 Hz, 2H), 4.00 (s, 1H), 1.16 (d, $J$ = 6.1 Hz, 6H).

**Preparation of Intermediate B-13: (((((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl isopropyl carbonate,**

**B-13a: (((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl isopropyl carbonate and**

**B-13b: (((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl isopropyl carbonate**

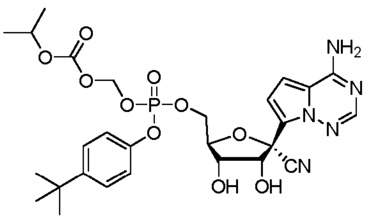

B-13

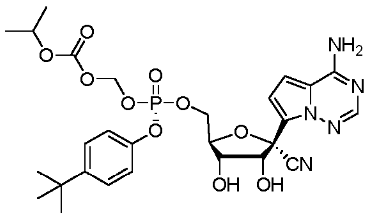

B-13a

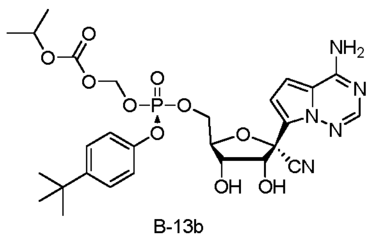

B-13b

Following the synthetic procedure for intermediate B-11, 4-tert-butylphenol (0.75 g, 5 mmol) was used instead of phenol (1.84 g, 19.6 mmol) to obtain compound B-13 (650 mg, overall yield: 21%) as a white solid. LC-MS (APCI): m/z 620.1 $[M+1]^+$. $^1$H NMR (400 MHz, DMSO-$d_6$) δ 7.92 (s, 1H), 7.30 (dd, $J$ = 15.4, 8.0 Hz, 2H), 7.05 (s, 2H), 6.91 (s, 1H), 6.81 (s, 1H), 6.36 (s, 1H), 5.64 (d, $J$ = 13.4 Hz, 2H), 5.45 (s, 1H), 4.80 (d, $J$ = 5.4 Hz, 1H), 4.69 (s, 1H), 4.39 (s, 1H), 4.27 (s, 2H), 3.95 (s, 1H), 1.24 (d, $J$ = 6.3 Hz, 15H).

The racemate compound B-13 was separated with supercritical fluid chromatography to obtain the target product B-13a (retention time: 1.06 min, relative content: 48.1%) and B-13b (retention time: 1.36 min, relative content: 51.9%).

Conditions of chromatographic separation:

Column: CHIRALPAK, IH-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=75:25

**Preparation of Intermediate B-14: (((((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)oxy)methyl isobutyrate**

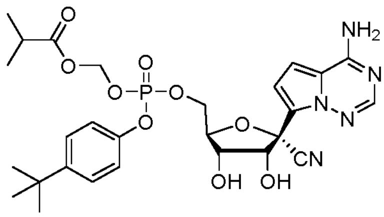

B-14

Following the synthetic procedure for intermediate B-11, 4-tert-butylphenol (0.75 g, 5 mmol) and chloromethyl isobutyrate (1.37 g, 10 mmol) were used instead of phenol (1.84 g, 19.6 mmol) and isopropyl chloromethyl carbonate (5.91 g, 38.7 mmol), respectively, to obtain compound B-14 (452 mg, overall yield: 15%) as a white solid. LC-MS (APCI): m/z=604.2 $(M+1)^+$. $^1H$ NMR (400 MHz, DMSO-$d_6$) δ 7.92 (s, 1H), 7.31 (dd, *J* = 13.7, 8.7 Hz, 2H), 7.11 - 7.01 (m, 2H), 6.90 (t, *J* = 4.6 Hz, 1H), 6.84 - 6.76 (m, 1H), 6.34 (dd, *J* = 6.0, 3.4 Hz, 1H), 5.63 (dd, *J* = 14.1, 5.3 Hz, 2H), 5.44 (t, *J* = 5.3 Hz, 1H), 4.69 (dd, *J* = 8.8, 5.4 Hz, 1H), 4.44 - 4.34 (m, 1H), 4.27 (t, *J* = 6.4 Hz, 2H), 3.96 (dd, *J* = 9.7, 5.0 Hz, 1H), 1.24 (d, *J* = 5.7 Hz, 9H), 1.03 (t, *J* = 7.2 Hz, 6H).

**Preparation of Intermediate B-15: S-(2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)oxy)ethyl) 2,2-dimethylthiopropionate**

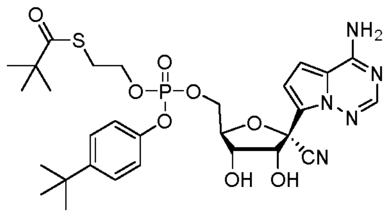

B-15

The following synthetic routes were adopted:

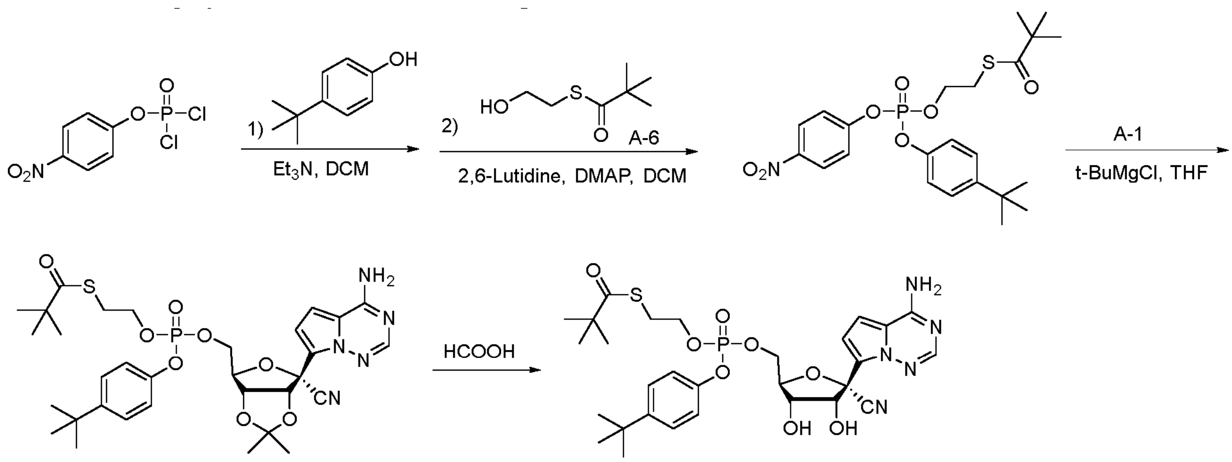


B-15

Step 1 Synthesis of S-(2-(((4-tert-butyl phenoxy)(4-nitrophenoxy)phosphoryl)oxy)ethyl) 2,2-dimethylthiopropionate

4-Nitrophenyl phosphorodichloridate (1.27 g, 5.0 mmol) was dissolved in anhydrous dichloromethane (20 mL). The mixture was cooled to -78°C under nitrogen atmosphere. A solution of triethylamine (759 mg, 7.5 mmol) and 4-tert-butylphenol (750 mg, 5.0 mmol) in dichloromethane (10 mL) was added dropwise. The mixture was stirred at low temperature for 1-2 h. After completion of the reaction as monitored by TLC, intermediate A-6 (972 mg, 6.0 mmol), 2,6-dimethylpyridine (1.1 g, 10 mmol), and DMAP (305 mg, 2.5 mmol) were added. The mixture was warmed to room temperature and stirred overnight. After completion of the reaction as monitored by TLC, excessive water was added for dilution. The mixture was extracted with dichloromethane for 3-4 times. The organic layers were combined, washed with saturated saline solution, concentrated and purified by silica gel column chromatography to obtain 1.16 g of light yellow oily liquid (yield: 47%). LC-MS(APCI): m/z=496.5$(M+1)^+$.

Step 2 Synthesis of S-(2-(((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxole-4-yl)methoxy)(4-tert-butyl phenoxy)phosphoryl)oxy)ethyl) 2,2-dimethylthiopropionate

The intermediate A-1 (0.83 g, 2.5 mmol) and S-(2-(((4-tert-butylphenoxy)(4-nitrophenoxy)phosphoryl)oxy)ethyl) 2,2-dimethylthiopropionate (1.83 g, 3.7 mmol) were added to a reaction flask, followed by the addtion of 30 mL of anhydrous tetrahydrofuran to dissolve. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 1:1) to obtain 1.08 g of light yellow oily liquid (yield: 63%). LC-MS (APCI): m/z=688.3 $(M+1)^+$.

Step 3 Synthesis of Intermediate B-15

S-(2-(((((3aR,4R,6R,6aR)-6-(4-aminopyrrolo[2,1-f][1,2,4]triazin-7-yl)-6-cyano-2,2-dimethyltetrahydrofuro[3,4-d][1,3]dioxole-4-yl)methoxy)(4-tert-butylphenoxy)phosphoryl)oxy)ethyl 2,2-dimethylthiopropionate (300 mg, 0.44 mmol) was added into a reaction flask, followed by the addtion of 10 mL of formic acid. The mixture was stirred at room temperature overnight. After completion of the reaction as monitored by TLC, the solvent was removed by concentration. The residue was purified by silica gel column chromatography to obtain 182 mg light yellow solid (yield: 64%). LC-MS (APCI): m/z=648.5 $(M+1)^+$. $^1$H NMR (400 MHz, DMSO-$d_6$) δ 7.92 (s, 1H), 7.31 (dd, $J$ = 15.5, 8.2 Hz, 2H), 7.07 (s, 2H), 6.91 (s, 1H), 6.81 (s, 1H), 6.34 (s, 1H), 5.43 (s, 1H), 4.69 (s, 1H), 4.35 (s, 1H), 4.26 (s, 2H), 4.10 (s, 2H), 3.97 (s, 1H), 3.08 (d, $J$ = 5.3 Hz, 2H), 1.19 (dd, $J$ = 40.8, 4.7 Hz, 18H).

**Preparation of Intermediate B-16: (((((2R,3S,4R,5R)-5-(4-aminopyrrolo[2,1-f][1,2,4]triazin-7-yl)-5-cyano-3,4-dihydroxytetrahydrofuran-2-yl)methoxy)phosphoryl)bis(oxy))bis(methylene)bis(2,2-dimethylpropionate)**

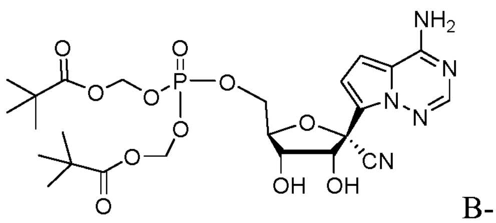

B-16

Following the synthetic procedure for intermediate B-9, chloromethyl pivalate (1.87 g, 12.5 mmol) was used instead of isopropyl chloromethyl carbonate (3.28 g, 21.5 mmol) to obtain compound B-16 (719 mg, overall yield: 24%) as a white solid. LC-MS (APCI): m/z 600.1 $[M+1]^+$. $^1$H NMR (400 MHz, DMSO-$d_6$) δ 8.04 (s, 1H), 7.07 (d, $J$ = 4.5 Hz, 1H), 6.87 (d, $J$ = 4.5 Hz, 1H), 5.57 (d, $J$ = 13.6 Hz, 4H), 4.82 (td, $J$ = 12.2, 6.1 Hz, 2H), 4.65 (d, $J$ = 4.7 Hz, 1H), 4.30 (m, 1H), 4.11 (m, 1H), 1.24 - 0.87 (d, $J$ = 6.4 Hz, 18H).

**Preparation of Intermediate B-17: S-((((((2R,3S,4R,5R)-5-(4-aminopyrrolo[2,1-f][1,2,4]triazin-7-yl)-5-cyano-3,4-dihydroxytetrahydrofuran-2-yl)methoxy)phosphoryl)bis(oxy))bis(ethylidene))bis(2,2-dimethylthiopropionate)**

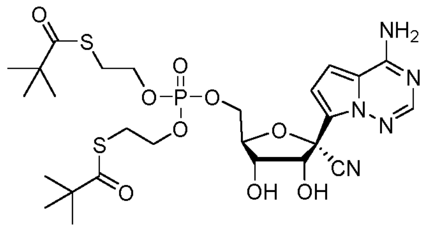

B-17

The following synthetic routes were adopted:

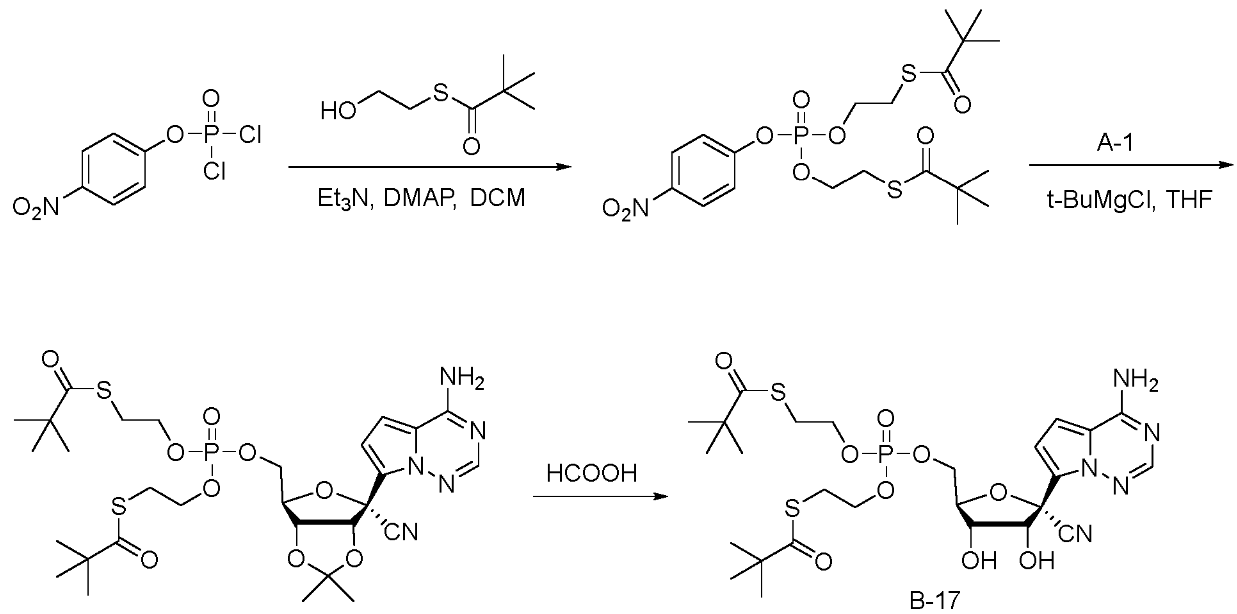


Step 1 Synthesis of S-((((4-nitrophenoxy)phosphoryl)bis(oxy))bis(ethylidene))bis(2,2-dimethylthiopropionate)

4-Nitrophenyl phosphorodichloridate (1.27 g, 5.0 mmol) was dissolved in anhydrous dichloromethane (20 mL) and cooled to -78°C under nitrogen atmosphere. A solution of triethylamine (1.26 g, 12.5 mmol) and S-(2-hydroxyethyl) 2,2-dimethylpropanethioate (1.78 g, 11 mmol) in dichloromethane (10 mL) was added dropwise, followed by the addition of DMAP (305 mg, 2.5 mmol). The mixture was slowly warmed to room temperature and stirred for reaction overnight. After completion of the reaction as monitored by TLC, excessive water was added for dilution. The mixture was extracted with dichloromethane for 3-4 times. The organic layers were combined, washed with saturated saline solution, concentrated and purified by silica gel column chromatography to obtain 1.22 g of light yellow oily liquid (yield: 48%). LC-MS(APCI): m/z=508.5$(M+1)^+$.

Step 2 Synthesis of S-((((((2R,3S,4R,5R)-6-(4-aminopyrrolo[2,1-f][1,2,4]triazin-7-yl)-6-cyano-2,2-dimethyltetrahydrofuro[3,4-d][1,3]dioxole-4-yl)methoxy)phosphoryl)bis(oxy))bis(ethylidene))bis(2,2-dimethylthiopropionate)

The intermediate A-1 (0.83 g, 2.5 mmol) and S-((((4-nitrophenoxy)phosphoryl)bis(oxy))bis(ethylidene))bis(2,2-dimethylthiopropionate) (1.87 g, 3.7 mmol) were added to a reaction flask, followed by the addtion of 30 mL of anhydrous tetrahydrofuran to dissolve. The mixture was cooled to 0°C in an ice bath, and a 1M tetrahydrofuran solution of tert-butylmagnesium chloride (3.7 mL, 3.7 mmol) was added dropwise under nitrogen atmosphere. After the addition, the mixture was slowly warmed to room temperature and stirred for 2-4 hours. After completion of the reaction as monitored by TLC, the mixture was quenched with saturated aqueous ammonium chloride solution and extracted with ethyl acetate 3-4 times. The organic layers were combined, washed with saturated saline solution 3 times, and the organic layers were separated, concentrated, and purified by silica gel column chromatography (petroleum ether/ethyl acetate = 3:1) to obtain 1.1 g of light yellow oily liquid (yield: 63%). LC-MS (APCI): m/z=700.3 $(M+1)^+$.

Step 3 Synthesis of B-17

S-((((((2R,3S,4R,5R)-6-(4-Aminopyrrolo[2,1-f][1,2,4]triazin-7-yl)-6-cyano-2,2-dimethyltetrahydrofuro[3,4-d][1,3]dioxol-4-yl)methoxy)phosphoryl)bis(oxyethylene))bis(2,2-dimethylpropanethioate) (307 mg, 0.44 mmol) was added into a reaction flask, followed by the addtion of 10 mL of formic acid. The mixture was stirred at room temperature overnight. After completion of the reaction as monitored by TLC, the solvent was removed by concentration. The residue was purified by silica gel column chromatography to obtain 148 mg light yellow solid (yield: 51%). LC-MS (APCI): m/z=660.7 $(M+1)^+$. $^1H$ NMR (400 MHz, $CDCl_3$) δ 7.97 (s, 1H), 6.90 (d, *J* = 4.6 Hz, 1H), 6.67 (d, *J* = 4.6 Hz, 1H), 5.88 (s, 2H), 4.64 (dd, *J* = 9.6, 4.3 Hz, 2H), 4.42 (d, *J* = 4.1 Hz, 2H), 4.37 (dd, *J* = 7.7, 3.6 Hz, 1H), 4.30 - 4.20 (m, 4H), 3.17 - 3.02 (m, 4H), 1.19 (d, *J* = 1.1 Hz, 18H).

**Preparation of Intermediate B-18: 2-ethyl butyl (S)-2-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)-1-phenylpropionate**

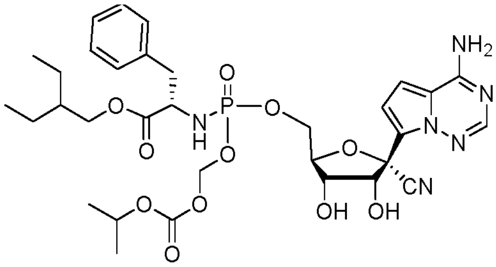

B-18

Following the synthetic procedure for intermediate B-1, 2-ethylbutyl L-phenylalaninate hydrochloride (1.42 g, 5 mmol) was used instead of isopropyl L-alaninate hydrochloride (3.3 g, 19.6 mmol) to obtain compound B-18 (1.08 g, overall yield: 30%) as a white solid. LC-MS (APCI): m/z 719.1 $[M+1]^+$. $^1H$ NMR (400 MHz, $CDCl_3$) δ 7.95 (d, J = 2.9 Hz, 1H), 7.25 - 7.20 (m, 2H), 7.12 - 7.08 (m, 2H), 6.87 (dd, J = 6.3, 4.6 Hz, 1H), 6.62 (dd, J = 10.0, 4.6 Hz, 1H), 5.81 (s, 2H), 5.44 - 5.31 (m, 2H), 4.92 - 4.85 (m, 1H), 4.55 (dd, J = 7.9, 5.7 Hz, 1H), 4.50 (d, J = 2.3 Hz, 1H), 4.33 (ddd, J = 12.0, 5.6, 3.2 Hz, 1H), 4.17 - 4.08 (m, 2H), 3.99 (dd, J = 9.8, 3.6 Hz, 2H), 3.62 (td, J = 10.7, 4.1 Hz, 1H), 3.06 - 2.97 (m, 1H), 2.92 (dd, J = 13.6, 6.7 Hz, 1H), 1.50 - 1.41 (m, 1H), 1.31 - 1.25 (m, 10H), 0.85 (td, J = 7.4, 5.4 Hz, 6H).

**Preparation of Intermediate B-19: 2-ethylbutyl 1-(((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)amino)cyclobutane-1-carboxylate**

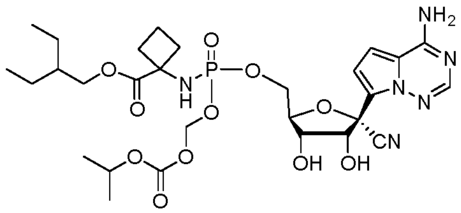

B-19

Following the synthetic procedure for intermediate B-1, 2-ethylbutyl 1-aminocyclobutane-1-carboxylate (1.17 g, 5 mmol) was used instead of isopropyl L-alaninate hydrochloride (3.3 g, 19.6 mmol) to obtain compound B-19 (1.23 g, overall yield: 37%) as a white solid. LC-MS (APCI): m/z 669.4 $[M+1]^+$. $^1H$ NMR (400 MHz, $CDCl_3$) δ 7.92 (s, 1H), 6.87 (s, 1H), 6.65 (d, *J* = 3.2 Hz, 1H), 6.01 (s, 2H), 5.67 - 5.53 (m, 2H), 5.47 (dd, *J* = 12.2, 5.3 Hz, 1H), 4.95 - 4.81 (m, 1H), 4.67 (d, *J* = 5.4 Hz, 1H), 4.56 (s, 1H), 4.40 (s, 1H), 4.28 (d, *J* = 17.5 Hz, 2H), 4.21 - 4.13 (m, 1H), 4.09 (d, *J* = 5.3 Hz, 2H), 3.94 (s, 1H), 2.45 (s, 4H), 2.04 - 1.84 (m, 2H), 1.59 - 1.52 (m, 1H), 1.34 (ddd, *J* = 23.0, 10.0, 5.0 Hz, 10H), 0.90 (t, *J* = 7.4 Hz, 6H).

**Preparation of Intermediate B-20: 1,4-dipentyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)-L-aspartate**

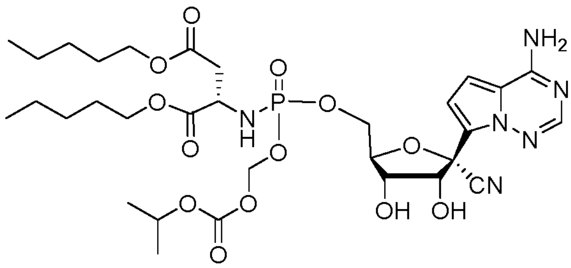

B-20

Following the synthetic procedure for intermediate B-1, 1,4-dipentyl L-aspartate hydrochloride (1.54 g, 5 mmol) was used instead of isopropyl L-alaninate hydrochloride (3.3 g, 19.6 mmol) to obtain compound B-20 (1.22 g, overall yield: 33%) as a white solid. LC-MS (APCI): m/z 743.2 $[M+1]^+$. $^1H$ NMR (400 MHz, DMSO) $\delta$ 7.93 (s, 1H), 6.90 (d, *J* = 4.5 Hz, 1H), 6.80 (d, *J* = 4.5 Hz, 1H), 6.23 (s, 2H), 4.80 (dt, *J* = 12.5, 6.2 Hz, 2H), 4.61 (d, *J* = 3.5 Hz, 2H), 4.25 - 4.10 (m, 6H), 3.12 (dd, *J* = 10.8, 5.9 Hz, 1H), 3.01 (m, 2H), 1.67 (m, 4H), 1.48 (m, 8H), 1.01 - 0.89 (m, 12H).

**Preparation of Intermediate B-21: (((((2R,3S,4R,5R)-5-(4-aminopyrrolo[2,1-f][1,2,4]triazin-7-yl)-5-cyano-3,4-dihydroxytetrahydrofuran-2-yl)methoxy)(3-(hexadecyloxy)propoxy)phosphoryl)oxy)methyl isopropyl carbonate,**

**B-21a: (((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(3-(hexadecyloxy)propoxy)phosphoryl)oxy)methyl isopropyl carbonate and**

**B-21a: (((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(3-(hexadecyloxy)propoxy)phosphoryl)oxy)methyl isopropyl carbonate**

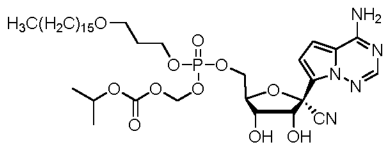

B-21

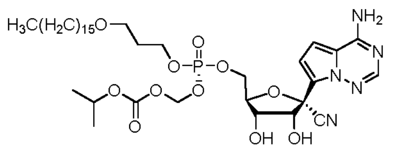

B-21a

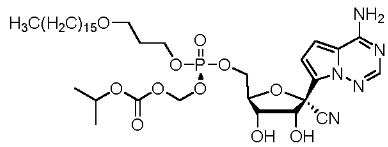

B-21b

Following the synthetic procedure for intermediate B-1, intermediate A-7 (1.5 g, 5 mmol) was used instead of isopropyl L-alaninate hydrochloride (3.3 g, 19.6 mmol) to obtain compound B-21 (0.92 g, overall yield: 24%) as a colorless oily liquid. LC-MS (APCI): m/z 770.2 $[M+1]^+$. $^1H$ NMR (400 MHz, DMSO-$d_6$)) $\delta$ 7.92 (s, 1H), 6.90 (d, *J* = 4.4 Hz, 1H), 6.81 (d, *J* = 4.5 Hz, 1H), 6.35 (dd, *J* = 5.9, 3.3 Hz, 1H), 5.54 (d, *J* = 13.9 Hz, 2H), 5.41 (d, *J* = 5.5 Hz, 1H), 4.87 - 4.76 (m, 1H), 4.67 (t, *J* = 5.4 Hz, 1H), 4.30 - 4.18 (m, 2H), 4.17 - 4.07 (m, 1H), 4.00 (dd, *J* = 13.0, 6.5 Hz, 2H), 3.93 (d, *J* = 4.9 Hz, 1H), 3.31 - 3.25 (m, 2H), 1.77 (dd, *J* = 10.5, 5.9 Hz, 2H), 1.50 - 1.38 (m, 2H), 1.24 (d, *J* = 7.3 Hz, 34H), 0.85 (t, *J* = 6.6 Hz, 3H).

The racemate compound B-21 was separated with supercritical fluid chromatography to obtain the target product B-21a (retention time: 3.87 min, relative content: 51.3%) and B-21b (retention time: 4.06 min, relative content: 48.7%).

Conditions of chromatographic separation:

Column: CHIRALPAK, AD-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=75:25

**Preparation of Intermediate B-22: 1,4-dipentyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate,**

**B-22a: 1,4-dipentyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate and**

**B-22b: 1,4-dipentyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate**

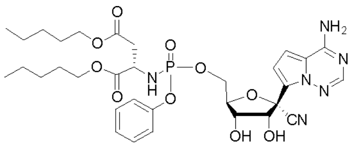
B-22

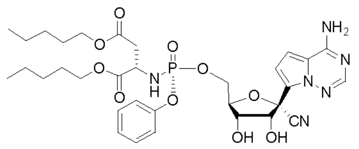
B-22a

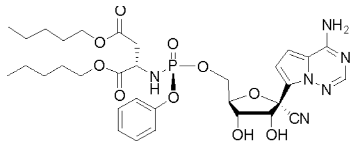
B-22b

Following the synthetic procedure for intermediate B-6, 1,4-dipentyl L-aspartate hydrochloride (3.08 g, 10 mmol) and phenol (1.1 g, 12 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-22 (1.33 g, overall yield: 19%) as a white solid. LC-MS (APCI): m/z 703.2 $[M+1]^+$. $^1H$ NMR (400 MHz, DMSO-$d_6$) δ 7.93 (s, 1H), 7.42 - 7.26 (m, 5H), 6.90 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.25 - 4.10 (m, 5H), 3.12 (dd, J = 10.8, 5.9 Hz, 1H), 3.01 (m, 2H), 1.67 (m, 4H), 1.48 (m, 8H), 1.01 - 0.89 (m, 6H).

The racemate compound B-22 was separated with reverse phase preparative chromatography to obtain the target product B-22a (retention time: 9.47 min, relative content: 52.7%) and B-22b (retention time: 9.97 min, relative content: 47.3%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-23: 1,4-dipentyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)-L-aspartate**

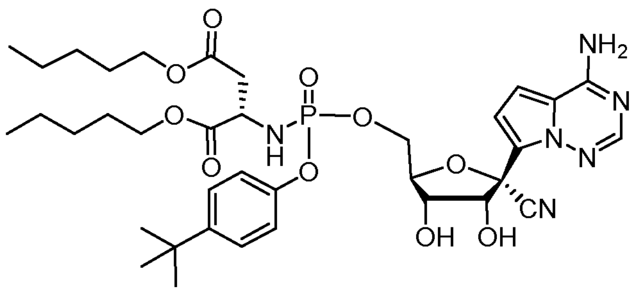
B-23

Following the synthetic procedure for intermediate B-6, 1,4-dipentyl L-aspartate hydrochloride (3.08 g, 10 mmol) and 4-tert-butylphenol (1.8 g, 12 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-23 (495 mg, overall yield: 12%) as a white solid. LC-MS (APCI): m/z 759.2 $[M+1]^+$. $^1H$ NMR (400 MHz, DMSO-$d_6$) δ 7.92 (s, 1H), 7.44 - 7.25 (m, 5H), 6.90 (d, J = 4.5 Hz, 1H), 6.81 (d, J = 4.5 Hz, 1H), 4.78 (dt, J = 12.5, 6.2 Hz, 2H), 4.61 (d, J = 3.5 Hz, 2H), 4.25 - 4.10 (m, 5H), 3.12 (dd, J = 10.8, 5.9 Hz, 1H), 3.01 (m, 2H), 1.66 (m, 4H), 1.45-1.37 (m, 8H), 1.03 - 0.68 (m, 15H).

**Preparation of Intermediate B-24: 2-ethylbutyl 1-(((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)amino)cyclobutane-1-carboxylate,**

**B-24a: 2-ethylbutyl 1-(((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)amino)cyclobutane-1-carboxylate and**

**B-24b: 2-ethylbutyl 1-(((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)amino)cyclobutane-1-carboxylate**

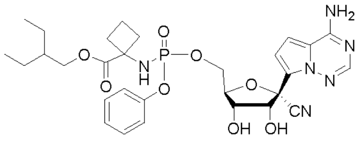
B-24

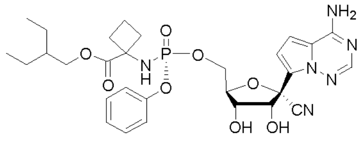
B-24a

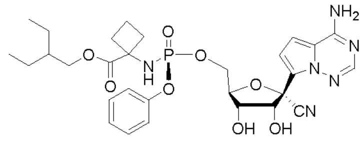
B-24b

Following the synthetic procedure for intermediate B-6, 2-ethylbutyl 1-aminocyclobutane-1-carboxylate (2.35 g, 10 mmol) and phenol (1.1 g, 12 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-24 (1.44 g, overall yield: 23%) as a white solid. LC-MS (APCI): m/z 629.7 $[M+1]^+$. $^1H$ NMR (400 MHz, DMSO-$d_6$) δ 7.93 (s, 1H), 7.42 - 7.26 (m, 5H), 6.90 (d, J = 4.5 Hz, 1H), 6.80 (d, J = 4.5 Hz, 1H), 4.80 (d, J = 6.2 Hz, 2H), 4.63 (m, 2H), 4.22 (m, 3H), 2.45 (m, 2H), 2.25 (m, 2H), 2.07 (m, 1H), 1.48 (m, 4H), 1.01 - 0.89 (m, 6H).

The racemate compound B-24 was separated with reverse phase preparative chromatography to obtain the target product B-24a (retention time: 8.35 min, relative content: 52.1%) and B-24b (retention time: 9.26 min, relative content: 47.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-25: 2-ethyl butyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate,**

**B-25a: 2-ethyl butyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate and**

**B-25b: 2-ethyl butyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate**

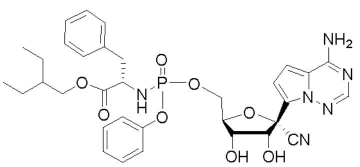
B-25

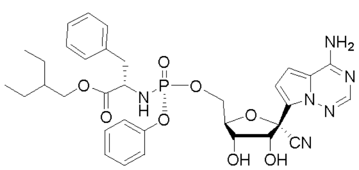
B-25a

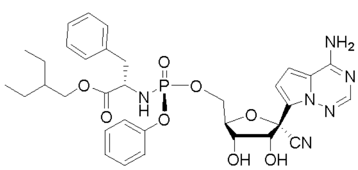
B-25b

Following the synthetic procedure for intermediate B-6, 2-ethylbutyl L-phenylalaninate hydrochloride (2.85 g, 10 mmol) and phenol (1.1 g, 12 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-25 (1.29 g, overall yield: 19%) as a white solid. LC-MS (APCI): m/z 679.6 $[M+1]^+$. $^1H$ NMR (400 MHz, $CDCl_3$) δ 7.91 (s, 1H), 7.21 (dt, J = 17.5, 7.5 Hz, 5H), 7.13 - 7.06 (m, 3H), 7.05 - 6.97 (m, 2H), 6.87 (d, J = 4.6 Hz, 1H), 6.52 (d, J = 4.7 Hz, 1H), 6.22 (d, J = 5.9 Hz, 1H), 5.90 (s, 2H), 5.49 (dd, J = 5.8, 4.0 Hz, 1H), 4.53 (d, J = 3.6 Hz, 1H), 4.36 - 4.28 (m, 1H), 4.24 - 4.12 (m, 2H), 3.96 - 3.77 (m, 3H), 2.91 (d, J = 6.2 Hz, 2H), 1.78 (s, 2H), 1.39 (dt, J = 12.4, 6.3 Hz, 1H), 1.27 - 1.20 (m, 4H), 1.12 - 0.87 (m, 6H).

The racemate compound B-25 was separated with reverse phase preparative chromatography to obtain the target product B-25a (retention time: 10.02 min, relative content: 55.2%) and B-25b (retention time: 10.94 min, relative content: 44.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-26: (((((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl acetate,**

**B-26a: (((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl acetate and**

**B-26b: (((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)oxy)methyl acetate**

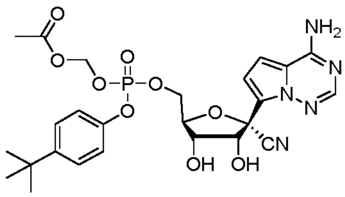
B-26

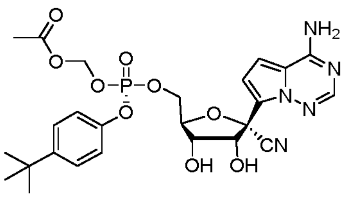
B-26a

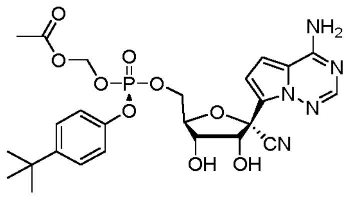
B-26b

Following the synthetic procedure for intermediate B-11, 4-tert-butylphenol (0.75 g, 5 mmol) and chloromethyl acetate (1.09 g, 10 mmol) were used instead of phenol (1.84 g, 19.6 mmol) and isopropyl chloromethyl carbonate (5.91 g, 38.7 mmol), respectively, to obtain compound B-26 (482 mg, overall yield: 17.2%) as a white solid. LC-MS (APCI): m/z 576.1 $[M+1]^+$. $^1$H NMR (400 MHz, DMSO-$d_6$) δ 7.93 (s, 1H), 7.31 (dd, J = 13.7, 8.7 Hz, 2H), 7.11 - 7.01 (m, 2H), 6.90 (t, J = 4.6 Hz, 1H), 6.84 - 6.76 (m, 1H), 6.34 (dd, J = 6.0, 3.4 Hz, 1H), 5.63 (dd, J = 14.1, 5.3 Hz, 2H), 5.44 (t, J = 5.3 Hz, 1H), 4.69 (dd, J = 8.8, 5.4 Hz, 1H), 4.44 - 4.34 (m, 1H), 4.27 (t, J = 6.4 Hz, 2H), 3.96 (dd, J = 9.7, 5.0 Hz, 1H), 2.21 (s, 3H), 1.03 (s, 9H).

The racemate compound B-26 was separated with supercritical fluid chromatography to obtain the target product B-26a (retention time: 1.52 min, relative content: 48.2%) and B-26b (retention time: 1.77 min, relative content: 51.8%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Preparation of Intermediate B-27: (((((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)oxy)methyl pivalate,**

**B-27a: (((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)oxy)methyl pivalate and**

**B-27b: (((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)oxy)methyl pivalate**

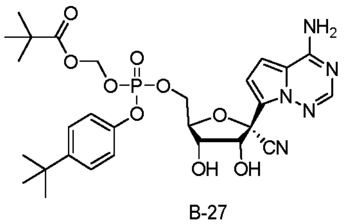

B-27

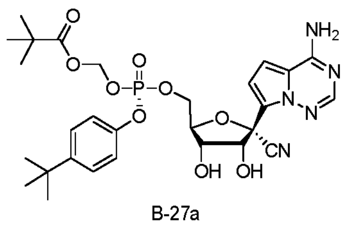

B-27a

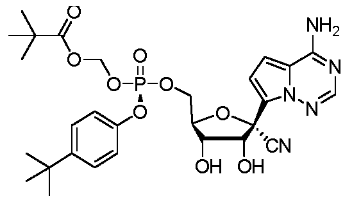

B-27b

Following the synthetic procedure for intermediate B-11, 4-tert-butylphenol (0.75 g, 5 mmol) and chloromethyl pivalate (1.5 g, 10 mmol) were used instead of phenol (1.84 g, 19.6 mmol) and isopropyl chloromethyl carbonate (5.91 g, 38.7 mmol), respectively, to obtain compound B-27 (469 mg, overall yield: 16%) as a white solid. LC-MS (APCI): m/z 618.6 $[M+1]^+$. $^1$H NMR (400 MHz, DMSO-$d_6$) δ 7.92 (s, 1H), 7.31 (dd, J = 13.7, 8.7 Hz, 2H), 7.11 - 7.01 (m, 2H), 6.90 (t, J = 4.6 Hz, 1H), 6.84 - 6.76 (m, 1H), 6.34 (dd, J = 6.0, 3.4 Hz, 1H), 5.63 (dd, J = 14.1, 5.3 Hz, 2H), 5.44 (t, J = 5.3 Hz, 1H), 4.69 (dd, J = 8.8, 5.4 Hz, 1H), 4.44 - 4.34 (m, 1H), 4.27 (t, J = 6.4 Hz, 2H), 3.96 (dd, J = 9.7, 5.0 Hz, 1H), 1.24 - 1.03 (m, 18H).

The racemate compound B-27 was separated with supercritical fluid chromatography to obtain the target product B-27a (retention time: 1.69 min, relative content: 52.3%) and B-27b (retention time: 1.87 min, relative content: 47.7%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Preparation of Intermediate B-28: (((((2R,3S,4R,5R)-5-(4-aminopyrrolo[2,1-f][1,2,4]triazin-7-yl)-5-cyano-3,4-dihydroxytetrahydrofuran-2-yl)methoxy)phosphoryl)bis(oxy))bis(methylene)bis(isobutyrate)**

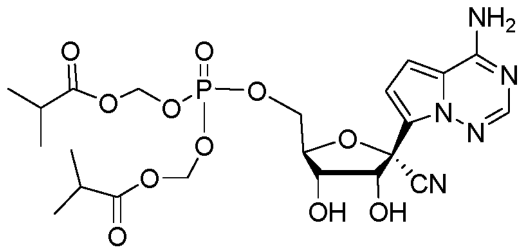

B-28

Following the synthetic procedure for intermediate B-9, chloromethyl isobutyrate (1.85 g, 12.5 mmol) was used instead of isopropyl chloromethyl carbonate (3.28 g, 21.5 mmol) to obtain compound B-28 (715 mg, overall yield: 23%) as a white solid. LC-MS (APCI): m/z 572.3 $[M+1]^+$. $^1$H NMR (400 MHz, DMSO-$d_6$) δ 8.04 (s, 1H), 7.07 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 1.21 - 0.89 (m, 12H).

**Preparation of Intermediate B-29: dodecyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-alaninate**

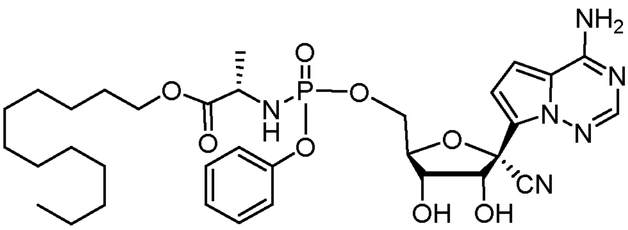

B-29

Following the synthetic procedure for intermediate B-6, intermediate A-8 (1.47 g, 5 mmol) and phenol (0.57 g, 6 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-29 (686 mg, overall yield: 20%) as a colorless oily liquid. LC-MS (APCI): m/z 687.5 $[M+1]^+$.

**Preparation of Intermediate B-30: (9Z,12Z)-octadeca-9,12-dien-1-yl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-alaninate**

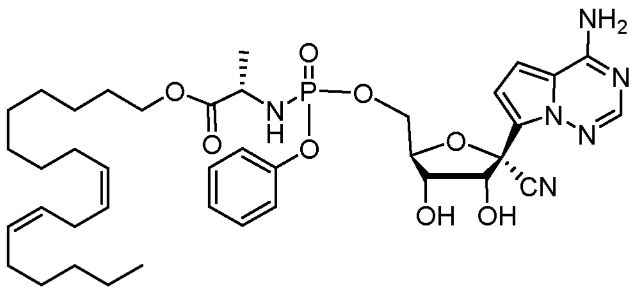

B-30

Following the synthetic procedure for intermediate B-6, intermediate A-9 (1.87 g, 5 mmol) and phenol (0.57 g, 6 mmol) were used instead of 2-ethylbutyl L-alaninate hydrochloride (1.05 g, 5.0 mmol) and ethyl 2-(hydroxymethyl) benzoate (1.08 g, 6.0 mmol), respectively. After TLC confirmed reaction completion, the mixture was concentrated and carried forward directly to the next step.

**Preparation of Intermediate B-31: (9Z,12Z)-octadeca-9,12-dien-1-yl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)-L-alaninate,**

**B-31a: (9Z,12Z)-octadeca-9,12-dien-1-yl N-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)-L-alaninate and**

**B-31b: (9Z,12Z)-octadeca-9,12-dien-1-yl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-(tert-butyl)phenoxy)phosphoryl)-L-alaninate**

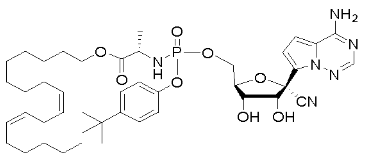

B-31

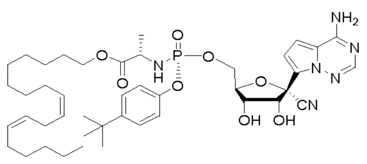

B-31a

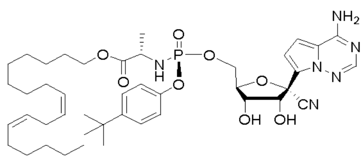

B-31b

Following the synthetic procedure for intermediate B-6, intermediate A-9 (1.87 g, 5 mmol) and 4-tert-butylphenol (0.75 g, 5 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-31 (1.11 g, overall yield: 27%) as a colorless oily liquid. LC-MS (APCI): m/z 823.5 $[M+1]^+$. $^1H$ NMR (400 MHz, DMSO-$d_6$) δ 8.03 (s, 1H), 7.05 (d, J = 4.5 Hz, 1H), 6.87 (d, J = 4.5 Hz, 1H), 5.57 (d, J = 13.6 Hz, 4H), 4.81 (td, J = 12.2, 6.1 Hz, 2H), 4.65 (d, J = 4.7 Hz, 1H), 4.30 (m, 1H), 4.11 (m, 1H), 2.05 (m, 2H), 1.21 - 0.89 (d, J = 6.4 Hz, 20H), 0.81 (s, 9H). The racemate compound B-31 was separated with reverse phase preparative chromatography to obtain the target product B-31a (retention time: 9.48 min, relative content: 53.2%) and B-31b (retention time: 11.1 min, relative content: 46.8%).

Conditions of chromatographic separation:

Column: Waters, Prep C18 OBD™, 150×19 mm, 5 μm
Column temperature: 30°C
Flow rate: 1.0 mL/min

Wavelength of UV detection: 220 nm
Mobile phase: pure water:acetonitrile=40:60

**Preparation of Intermediate B-32: ((2R,3S,4R,5R)-5-(4-aminopyrrolo[2,1-f][1,2,4]triazin-7-yl)-5-cyano-3,4-dihydroxytetrahydrofuran-2-yl)methyl ((9Z,12Z)-octadeca-9,12-dien-1-yl) phenyl phosphate**

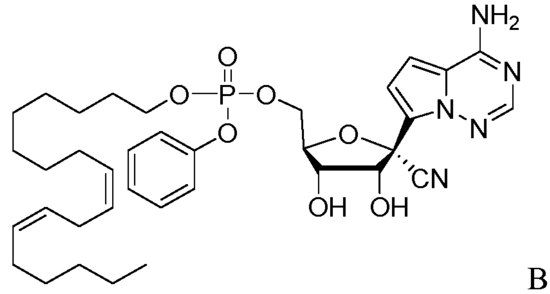

B-32

The following synthetic routes were adopted:

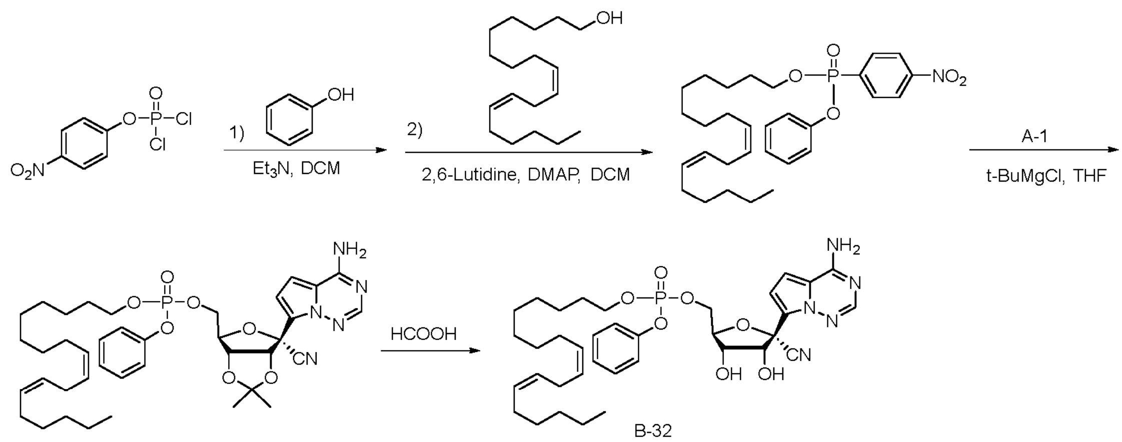


Following the synthetic procedure for intermediate B-6, phenol (0.94 g, 10 mmol) and (9Z,12Z)-octadeca-9,12-dien-1-ol (1.33 g, 5 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-32 (486 mg, overall yield: 14%) as a colorless oily liquid. LC-MS (APCI): m/z 696.7 $[M+1]^+$. $^1H$ NMR (400 MHz, DMSO-$d_6$) $\delta$ 7.93 (d, J = 1.6 Hz, 1H), 7.35 (dd, J = 16.6, 8.6 Hz, 2H), 7.25 - 7.14 (m, 3H), 6.90 (t, J = 4.8 Hz, 1H), 6.80 (dd, J = 4.4, 3.0 Hz, 1H), 6.36 (t, J = 5.9 Hz, 1H), 5.42 (t, J = 7.3 Hz, 1H), 4.68 (t, J = 5.5 Hz, 1H), 4.37 (dd, J = 14.3, 8.0 Hz, 1H), 4.24 (dd, J = 19.6, 4.5 Hz, 2H), 4.10 (dt, J = 7.9, 4.0 Hz, 2H), 3.97 (dd, J = 11.1, 5.7 Hz, 1H), 3.25 (dd, J = 15.9, 9.2 Hz, 2H), 1.79 (dd, J = 10.7, 5.8 Hz, 2H), 1.40 (dd, J = 19.8, 13.7 Hz, 2H), 1.24 - 1.08 (m, 20H), 0.85 (t, J = 6.7 Hz, 3H).

**Preparation of Intermediate B-33: ((2R,3S,4R,5R)-5-(4-aminopyrrolo[2,1-f][1,2,4]triazin-7-yl)-5-cyano-3,4-dihydroxytetrahydrofuran-2-yl)methyl (4-(tert-butyl)phenyl) ((9Z,12Z)-octadeca-9,12-dien-1-yl) phosphate**

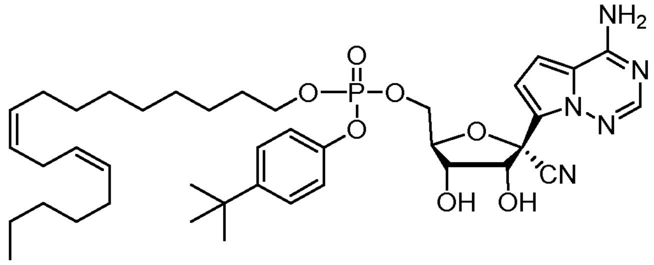

B-33

Following the synthetic procedure for intermediate B-6, 4-tert-butylphenol (1.5 g, 10 mmol) and (9Z,12Z)-octadeca-9,12-dien-1-ol (1.33 g, 5 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-33 (523 mg, overall yield: 22%) as a colorless oily liquid. LC-MS (APCI): m/z 752.3 $[M+1]^+$. $^1H$ NMR (400 MHz, DMSO-$d_6$) δ 7.93 (d, J = 1.6 Hz, 1H), 7.36 (dd, J = 16.6, 8.6 Hz, 2H), 7.25 - 7.14 (m, 3H), 6.90 (t, J = 4.8 Hz, 1H), 6.80 (dd, J = 4.4, 3.0 Hz, 1H), 6.36 (t, J = 5.9 Hz, 1H), 5.42 (t, J = 7.3 Hz, 1H), 4.68 (t, J = 5.5 Hz, 1H), 4.37 (dd, J = 14.3, 8.0 Hz, 1H), 4.24 (dd, J = 19.6, 4.5 Hz, 2H), 4.10 (dt, J = 7.9, 4.0 Hz, 2H), 3.97 (dd, J = 11.1, 5.7 Hz, 1H), 3.25 (dd, J = 15.9, 9.2 Hz, 2H), 1.79 (dd, J = 10.7, 5.8 Hz, 2H), 1.40 (dd, J = 19.8, 13.7 Hz, 2H), 1.22 - 1.14 (t, J = 11.4 Hz, 29H), 0.86 (s, 9H).

**Preparation of Intermediate B-34: (9Z,12Z)-octadeca-9,12-dien-1-yl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)-L-alaninate,**

**B-34a: (9Z,12Z)-octadeca-9,12-dien-1-yl N-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)-L-alaninate, and**

**B-34b: (9Z,12Z)-octadeca-9,12-dien-1-yl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(((isopropoxycarbonyl)oxy)methoxy)phosphoryl)-L-alaninate**

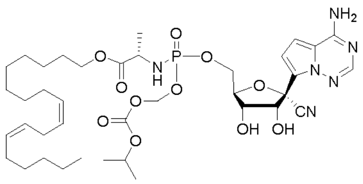

B-34

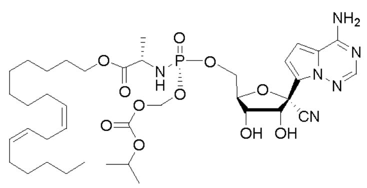

B-34a

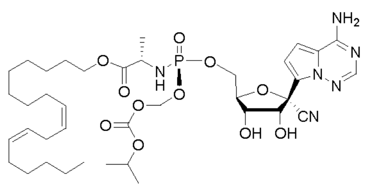

B-34b

Following the synthetic procedure for intermediate B-1, intermediate A-9 (1.87 g, 5 mmol) was used instead of isopropyl L-alaninate hydrochloride (3.3 g, 19.6 mmol) to obtain compound B-34 (846 mg, overall yield: 21%) as a white solid. LC-MS (APCI): m/z 807.1 $[M+1]^+$. $^1H$ NMR (400 MHz, $CDCl_3$) δ 7.95 (d, J = 2.9 Hz, 1H), 7.25 - 7.20 (m, 2H), 7.12 - 7.08 (m, 2H), 6.87 (dd, J = 6.3, 4.6 Hz, 1H), 6.62 (dd, J = 10.0, 4.6 Hz, 1H), 5.81 (s, 2H), 5.44 - 5.31 (m, 2H), 4.92 - 4.85 (m, 1H), 4.55 (dd, J = 7.9, 5.7 Hz, 1H), 4.50 (d, J = 2.3 Hz, 1H), 4.33 (ddd, J = 12.0, 5.6, 3.2 Hz, 1H), 4.17 - 4.08 (m, 2H), 3.99 (dd, J = 9.8, 3.6 Hz, 2H), 3.62 (td, J = 10.7, 4.1 Hz, 1H), 3.06 - 2.97 (m, 1H), 2.92 (dd, J = 13.6, 6.7 Hz, 1H), 1.50 - 1.41 (m, 1H), 1.31 - 1.25 (m, 10H), 0.85 (td, J = 7.4, 5.4 Hz, 6H).

The racemate compound B-34 was separated with reverse phase preparative chromatography to obtain the target product B-34a (retention time: 13.22 min, relative content: 51.1%) and B-34b (retention time: 15.01 min, relative content: 48.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-35: (9Z,12Z)-octadeca-9,12-dien-1-yl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)((pivaloyloxy)methoxy)phosphoryl)-L-alaninate**

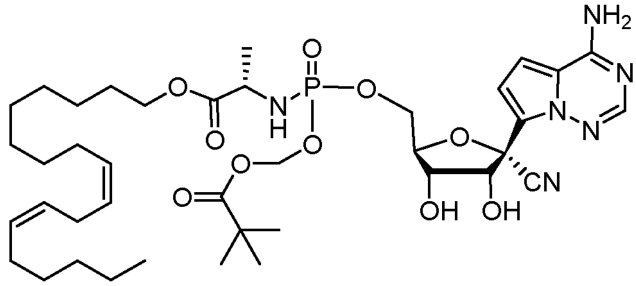

B-35

Following the synthetic procedure for Intermediate B-1, intermediate A-9 (1.87 g, 5 mmol) and chloromethyl pivalate (1.5 g, 10 mmol) were used instead of isopropyl L-alaninate hydrochloride (3.3 g, 19.6 mmol) and isopropyl chloromethyl carbonate (5.91 g, 38.7 mmol), respectively, to obtain compound B-35 (1.08 g, overall yield: 23%) as a colorless oily liquid. LC-MS (APCI): m/z 805.5 $[M+1]^+$. $^1H$ NMR (400 MHz, $CDCl_3$) δ 7.95 (d, J = 2.9 Hz, 1H), 7.25 - 7.20 (m, 2H), 7.12 - 7.08 (m, 2H), 6.87 (dd, J = 6.3, 4.6 Hz, 1H), 6.62 (dd, J = 10.0, 4.6 Hz, 1H), 5.81 (s, 2H), 5.44 - 5.31 (m, 2H), 4.92 - 4.85 (m, 1H), 4.55 (dd, J = 7.9, 5.7 Hz, 1H), 4.50 (d, J = 2.3 Hz, 1H), 4.33 (ddd, J = 12.0, 5.6, 3.2 Hz, 1H), 4.17 - 4.08 (m, 2H), 3.99 (dd, J = 9.8, 3.6 Hz, 2H), 3.62 (td, J = 10.7, 4.1 Hz, 1H), 3.06 - 2.97 (m, 1H), 2.92 (dd, J = 13.6, 6.7 Hz, 1H), 1.50 - 1.41 (m, 1H), 1.31 - 1.25 (m, 10H), 0.85 (s, 9H).

**Preparation of Intermediate B-36: 2-ethyl butyl N-((((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)-L-phenylalaninate,**

**B-36a: 2-ethyl butyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)-L-phenylalaninate and**

**B-36b: 2-ethyl butyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(4-tert-butyl phenoxy)phosphoryl)-L-phenylalaninate**

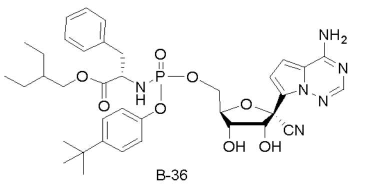

B-36

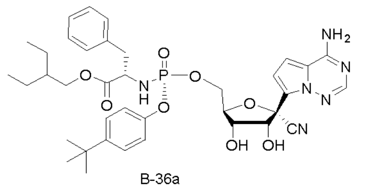

B-36a

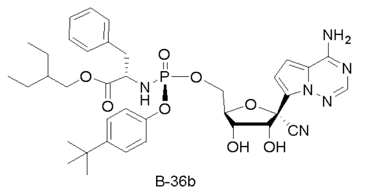

B-36b

Following the synthetic procedure for intermediate B-6, 2-ethylbutyl L-phenylalaninate hydrochloride (1.42 g, 5.0 mmol) and 4-tert-butylphenol (0.9 g, 6.0 mmol) were used instead of 2-ethylbutyl L-alaninate hydrochloride (1.05 g, 5.0 mmol) and ethyl 2-(hydroxymethyl)benzoate (1.08 g, 6.0 mmol), respectively, to obtain compound B-36 (605 mg, overall yield: 25%) as a white solid. LC-MS (APCI): m/z 735.4 $[M+1]^+$. $^1H$ NMR (400 MHz, $CDCl_3$) δ 7.93 (s, 1H), 7.22 (dt, J = 17.5, 7.5 Hz, 5H), 7.13 - 7.06 (m, 3H), 7.05 - 6.97 (m, 2H), 6.87 (d, J = 4.6 Hz, 1H), 6.52 (d, J = 4.7 Hz, 1H), 6.22 (d, J = 5.9 Hz, 1H), 5.90 (s, 2H), 5.49 (dd, J = 5.8, 4.0 Hz, 1H), 4.53 (d, J = 3.6 Hz, 1H), 4.36 - 4.28 (m, 1H), 4.24 - 4.12 (m, 2H), 3.96 - 3.77 (m, 3H), 2.91 (d, J = 6.2 Hz, 2H), 1.78 (s, 2H), 1.39 (dt, J = 12.4, 6.3 Hz, 1H), 1.27 - 1.20 (m, 6H), 0.97 (s, 9H).

The racemate compound B-36 was separated with reverse phase preparative chromatography to obtain the target product B-36a (retention time: 10.11 min, relative content: 53.2%) and B-36b (retention time: 10.99 min, relative content: 46.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-37: 1,4-dicyclobutyl N-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate,**

**B-37a: 1,4-dicyclobutyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate and**

**B-37b: 1,4-dicyclobutyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate**

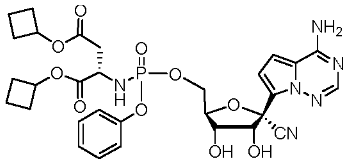

B-37

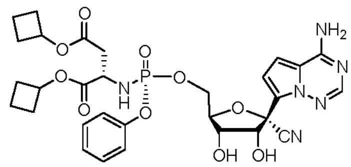

B-37a

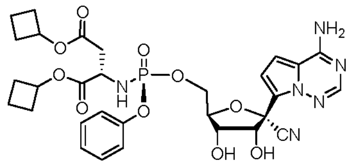

B-37b

Following the synthetic procedure for intermediate B-6, intermediate A-10 (2.78 g, 10 mmol) and phenol (1.1 g, 12 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-37 (526 mg, overall yield: 30%) as a white solid. LC-MS (APCI): m/z 671.2 $[M+1]^+$. $^1H$ NMR (400 MHz, DMSO-$d_6$) δ 7.93 (s, 1H), 7.44 - 7.22 (m, 5H), 6.91 (d, J = 4.5 Hz, 1H), 6.82 (d, J = 4.5 Hz, 1H), 4.80 (dt, J = 12.5, 6.2 Hz, 2H), 4.61 (d, J = 3.5 Hz, 2H), 4.25 - 4.10 (m, 5H), 3.15 (dd, J = 10.8, 5.9 Hz, 1H), 3.01 (m, 2H), 1.63 (m, 4H).

The racemate compound B-37 was separated with reverse phase preparative chromatography to obtain the target product B-37a (retention time: 9.44 min, relative content: 50.2%) and B-37b (retention time: 9.57 min, relative content: 49.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-38: 1,4-dicyclopentyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate,**

**B-38a: 1,4-dicyclopentyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate and**

**B-38b: 1,4-dicyclopentyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate**

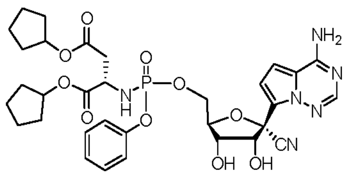

B-38

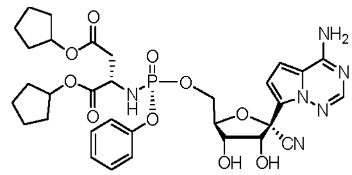

B-38a

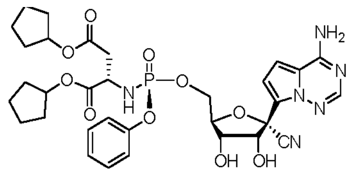

B-38b

Following the synthetic procedure for intermediate B-6, intermediate A-11 (3.06 g, 10 mmol) and phenol (1.1 g, 12 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-38 (729 mg, overall yield: 35%) as a white solid. LC-MS (APCI): m/z 699.8 $[M+1]^+$. $^1H$ NMR (400 MHz, DMSO-$d_6$) δ 7.92 (s, 1H), 7.42 - 7.26 (m, 5H), 6.92 (d, J = 4.5 Hz, 1H), 6.80 (d, J = 4.5 Hz, 1H), 4.81 (dt, J = 12.5, 6.2 Hz, 2H), 4.61 (d, J = 3.5 Hz, 2H), 4.25 - 4.10 (m, 5H), 3.12 (dd, J = 10.8, 5.9 Hz, 1H), 3.01 (m, 2H), 1.69 (m, 4H), 1.48-1.38 (m, 8H).

The racemate compound B-38 was separated with reverse phase preparative chromatography to obtain the

target product B-38a (retention time: 9.43 min, relative content: 50.2%) and B-38b (retention time: 9.52 min, relative content: 49.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-39: 1,4-bis(2-ethyl butyl) N-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate,**

**B-39a: 1,4-bis(2-ethyl butyl) *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate and**

**B-39b: 1,4-bis(2-ethyl butyl) *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-aspartate**

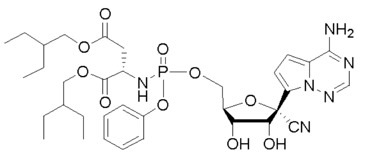
B-39

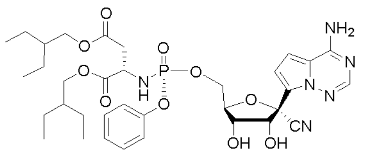
B-39a

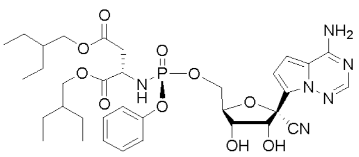
B-39b

Following the synthetic procedure for intermediate B-6, intermediate A-12 (3.38 g, 10 mmol) and phenol (1.1 g, 12 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-39 (508 mg, overall yield: 22%) as a white solid. LC-MS (APCI): m/z 731.2 $[M+1]^+$. $^1H$ NMR (400 MHz, DMSO-$d_6$) δ 7.93 (s, 1H), 7.42 - 7.26 (m, 5H), 6.90 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.25 - 4.10 (m, 5H), 3.12 (dd, J = 10.8, 5.9 Hz, 1H), 3.01 (m, 2H), 1.67 (m, 4H), 1.48 (m, 8H), 1.01 - 0.89 (m, 6H).

The racemate compound B-39 was separated with reverse phase preparative chromatography to obtain the target product B-39a (retention time: 9.42 min, relative content: 50.2%) and B-39b (retention time: 9.54 min, relative content: 49.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-40: cyclobutyl *N*-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate,**

**B-40a: cyclobutyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate and**

**B-40b: cyclobutyl *N*-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate**

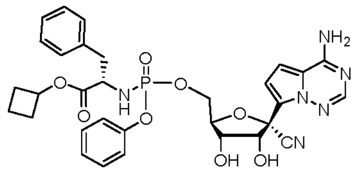

B-40

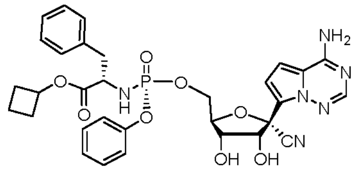

B-40a

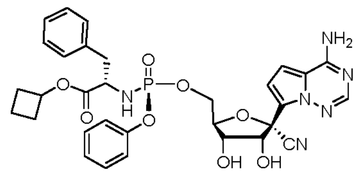

B-40b

Following the synthetic procedure for intermediate B-6, intermediate A-13 (2.56 g, 10 mmol) and phenol (1.1 g, 12 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-40 (693 mg, overall yield: 17%) as a white solid. LC-MS (APCI): m/z 649.5 $[M+1]^+$. $^1H$ NMR (400 MHz, $CDCl_3$) δ 7.91 (s, 1H), 7.21 (dt, J = 17.3, 7.5 Hz, 5H), 7.13 - 7.06 (m, 3H), 7.05 - 6.97 (m, 2H), 6.87 (d, J = 4.6 Hz, 1H), 6.52 (d, J = 4.7 Hz, 1H), 6.21 (d, J = 5.9 Hz, 1H), 5.90 (s, 2H), 5.49 (dd, J = 5.8, 4.0 Hz, 1H), 4.55 (d, J = 3.6 Hz, 1H), 4.36 - 4.28 (m, 1H), 4.24 - 4.12 (m, 2H), 3.96 - 3.77 (m, 3H), 2.90 (d, J = 6.2 Hz, 2H), 1.78 (s, 2H), 1.39 (dt, J = 12.4, 6.3 Hz, 1H), 1.27 - 1.20 (m, 4H), 1.12 - 0.85 (m, 6H).

The racemate compound B-40 was separated with reverse phase preparative chromatography to obtain the target product B-40a (retention time: 10.15 min, relative content: 52.4%) and B-40b (retention time: 11.91 min, relative content: 47.6%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Preparation of Intermediate B-41: 2-methoxy-2-methylpropyl N-((((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate,**

**B-41a: 2-methoxy-2-methylpropyl *N*-((R)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate and**

**B-41b: 2-methoxy-2-methylpropyl N-((S)-(((2R,3S,4R,5R)-5-(4-aminopyrrolo[2,1-f][1,2,4]triazin-7-yl)-5-cyano-3,4-dihydroxytetrahydrofuran-2-yl)methoxy)(phenoxy)phosphoryl)-L-phenylalaninate**

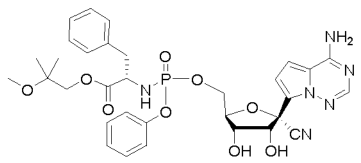

B-41

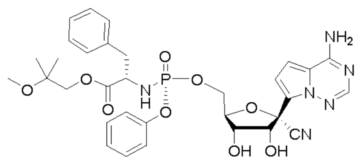

B-41a

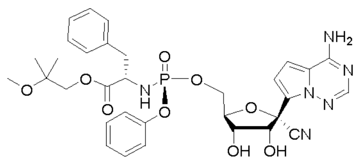

B-41b

Following the synthetic procedure for intermediate B-6, intermediate A-14 (2.87 g, 10 mmol) and phenol (1.1 g, 12 mmol) were used instead of 2-ethylbutyl L-alaninate hydrochloride (1.05 g, 5.0 mmol) and ethyl 2-(hydroxymethyl) benzoate (1.08 g, 6.0 mmol), respectively, to obtain compound B-41 (347 mg, overall yield: 9%) as a white solid. LC-MS (APCI): m/z 681.2 $[M+1]^+$. $^1H$ NMR (400 MHz, $CDCl_3$) δ 7.91 (s, 1H), 7.21 (dt, J = 17.5, 7.5 Hz, 5H), 7.13 - 7.06 (m, 3H), 7.05 - 6.97 (m, 2H), 6.87 (d, J = 4.6 Hz, 1H), 6.52 (d, J = 4.7 Hz, 1H), 6.22 (d, J = 5.9 Hz, 1H), 5.90 (s, 2H), 5.49 (dd, J = 5.8, 4.0 Hz, 1H), 4.53 (d, J = 3.6 Hz, 1H), 4.36 - 4.28 (m, 1H), 4.24 - 4.12 (m, 2H), 3.96 - 3.77 (m, 3H), 2.91 (d, J = 6.2 Hz, 2H), 1.78 (s, 2H), 1.39 (dt, J = 12.4, 6.3 Hz, 1H), 1.27 - 1.20 (m, 4H), 1.12 - 0.87 (m, 6H).

The racemate compound B-41 was separated with reverse phase preparative chromatography to obtain the target product B-41a (retention time: 10.12 min, relative content: 51.4%) and B-41b (retention time: 11.85 min, relative content: 48.6%).

Conditions of chromatographic separation:

Column: Waters, Prep C18 OBD™, 150×19 mm, 5 μm
Column temperature: 30°C

Flow rate: 1.0 mL/min
Wavelength of UV detection: 220 nm
Mobile phase: pure water:acetonitrile=40:60

**Preparation of Intermediate B-42: (((((2R,3S,4R,5R)-5-(4-amino-5-iodopyrrolo[2,1-f][1,2,4]triazin-7-yl)-5-cyano-3,4-dihydroxytetrahydrofuran-2-yl)methoxy)phosphoryl)bis(oxy))bis(methylene)diisopropyl bis(carbonate)**

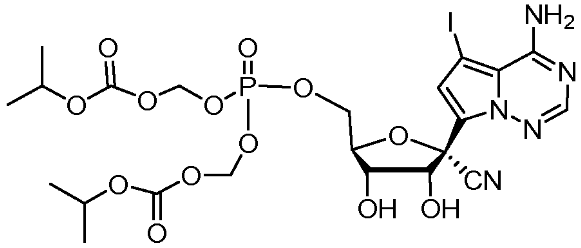

B-42

Following the synthetic procedure for intermediate B-9, intermediate A-2 (1.14 g, 2.5 mmol) was used instead of intermediate A-1 (0.82 g, 2.5 mmol) to obtain 275 mg of compound B-42 (yield: 58%) as an off-white solid. LC-MS (APCI): m/z=730.3 $(M+1)^+$. $^1H$ NMR (400 MHz, DMSO-$d_6$) δ 8.11 (s, 1H), 6.93 (s, 1H), 5.59 (d, J = 12.4 Hz, 4H), 4.77 (dd, J = 11.4, 5.6 Hz, 2H), 4.63 (d, J = 4.4 Hz, 1H), 4.31 (m, 1H), 4.12 (m, 1H), 3.94 - 3.81 (m, 2H), 1.25 (d, J = 6.4 Hz, 12H).

**Preparation of Intermediate B-43: (((((2R,3S,4R,5R)-5-(4-amino-6-bromopyrrolo[2,1-f][1,2,4]triazin-7-yl)-5-cyano-3,4-dihydroxytetrahydrofuran-2-yl)methoxy)phosphoryl)bis(oxy))bis(methylene)diisopropyl bis(carbonate)**

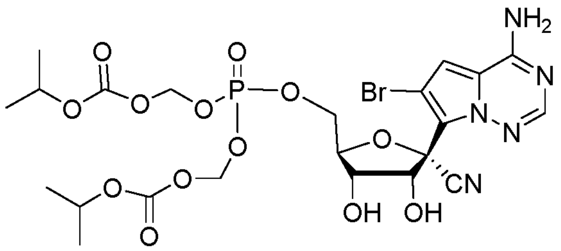

B-43

The following synthetic routes were adopted:

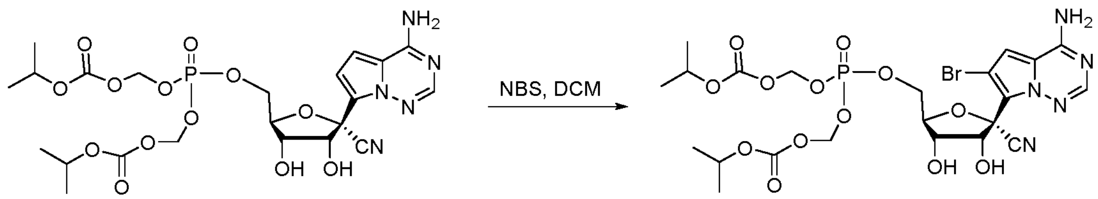


B-9 B-43

The intermediate B-9 (100 mg, 0.16 mmol) was added to a reaction flask, followed by the addtion of 5 mL of anhydrous dichloromethane to dissolve. N-Bromosuccinimide (35.4 mg, 0.20 mmol) was added in portions at room temperature under nitrogen atmosphere. The mixture was stirred at room temperature overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 31 mg of the target product (yield: 28%). LC-MS (APCI): m/z=682.2 $(M+1)^+$. $^1H$ NMR (400 MHz, DMSO-$d_6$) δ 8.38 (s, 1H), 7.49 (s, 1H), 5.59 (d, J = 13.2 Hz, 4H), 4.80 (td, J = 12.4, 6.4 Hz, 2H), 4.62 (d, J = 4.7 Hz, 1H), 4.30 (m, 1H), 4.12 (m, 1H), 3.93 - 3.86 (m, 2H), 1.24 (d, J = 6.4 Hz, 12H).

**Example 1: Preparation of (2R,3R,4R,5R)-2-(4-aminopyrrolo[2,1-f][1,2,4]triazin-7-yl)-2-cyano-5-((((((S)-1-isopropoxy-1-oxopropan-2-yl)amino)(((isopropoxycarbonyl)oxy)methoxy)phosphoryl)oxy)methyl)tetrahydrofuran-3,4-diyl diacetate (Compound 1),**

**(2R,3R,4R,5R)-2-(4-aminopyrrolo[2,1-f][1,2,4]triazin-7-yl)-2-cyano-5-((((R)-(((S)-1-isopropoxy-1-oxopropan-2-yl)amino)(((isopropoxycarbonyl)oxy)methoxy)phosphoryl)oxy)methyl)tetrahydrofuran-3,4-diyl diacetate (Compound 1a) and**

**(2R,3R,4R,5R)-2-(4-aminopyrrolo[2,1-f][1,2,4]triazin-7-yl)-2-cyano-5-((((S)-(((S)-1-isopropoxy-1-oxopropan-2-yl)amino)(((isopropoxycarbonyl)oxy)methoxy)phosphoryl)oxy)methyl)tetrahydrofuran-3,4-diyl diacetate (Compound 1b)**

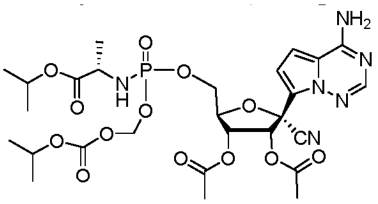
1

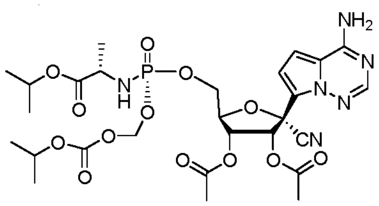
1a

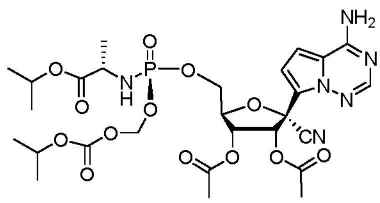
1b

The following synthetic routes were adopted:

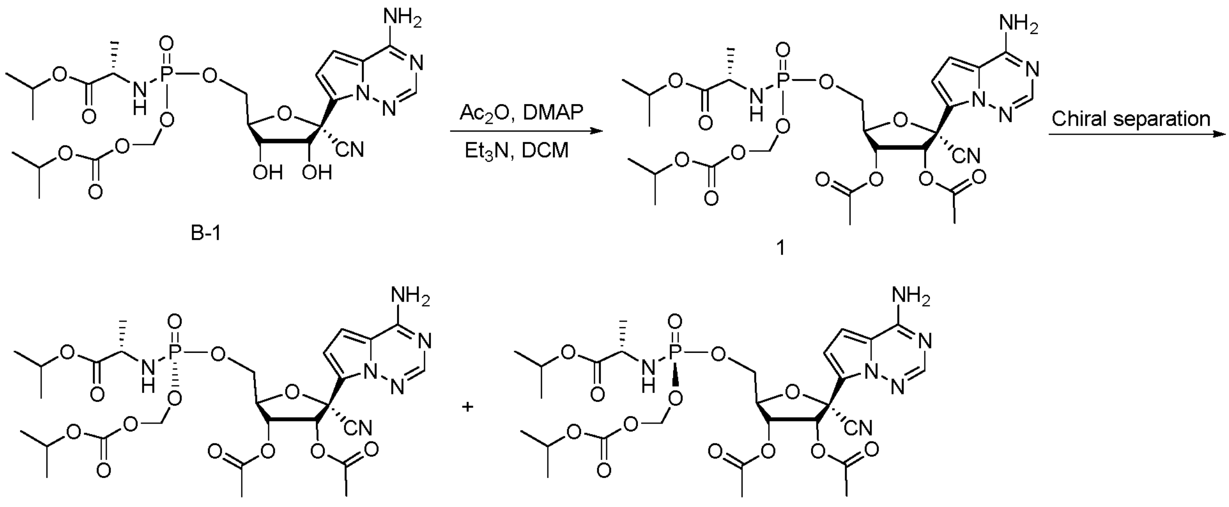


Step 1 Synthesis of Compound 1

The compound B-1 (186 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 99 mg of the target product (yield: 47%). LC-MS (APCI): m/z=685.1 $(M+1)^+$. $^1H$ NMR (400 MHz, DMSO-$d_6$) δ 7.94 (s, 1H), 6.93 (d, $J$ = 3.6 Hz, 1H), 6.81 (d, $J$ = 4.5 Hz, 1H), 6.04 (d, $J$ = 5.9 Hz, 1H), 5.84 (dd, $J$ = 22.5, 12.4 Hz, 1H), 5.55 - 5.32 (m, 3H), 4.90 - 4.72 (m, 2H), 4.58 (dd, $J$ = 15.2, 3.8 Hz, 1H), 4.26 - 4.04 (m, 2H), 3.59 (ddd, $J$ = 26.9, 16.9, 9.7 Hz, 1H), 2.11 (d, $J$ = 3.4 Hz, 6H), 1.23 (d, $J$ = 6.2 Hz, 6H), 1.20 - 1.09 (m, 9H).

Step 2 Synthesis of Compound 1a and Compound 1b

The racemate compound 1 was separated with reverse phase preparative chromatography to obtain the target product 1a (retention time: 9.16 min, relative content: 52.5%) and 1b (retention time: 10.20 min, relative content: 47.5%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 2: Preparation of (2R,3R,4R,5R)-2-(4-aminopyrrolo[2,1-f][1,2,4]triazin-7-yl)-2-cyano-5-((((((S)-1-isopropoxy-1-oxopropan-2-yl)amino)(((isopropoxycarbonyl)oxy)methoxy)phosphoryl)oxy)methyl)tetrahydrofuran-3,4-diyl dipropionate (Compound 2),**

**(2R,3R,4R,5R)-2-(4-aminopyrrolo[2,1-f][1,2,4]triazin-7-yl)-2-cyano-5-((((R)-(((S)-1-isopropoxy-1-oxopropan-2-yl)amino)(((isopropoxycarbonyl)oxy)methoxy)phosphoryl)oxy)methyl)tetrahydrofuran-3,4-diyl dipropionate (Compound 2a) and**

**(2R,3R,4R,5R)-2-(4-aminopyrrolo[2,1-f][1,2,4]triazin-7-yl)-2-cyano-5-((((S)-(((S)-1-isopropoxy-1-oxopropan-2-yl)amino)(((isopropoxycarbonyl)oxy)methoxy)phosphoryl)oxy)methyl)tetrahydrofuran-3,4-diyl dipropionate (Compound 2b)**

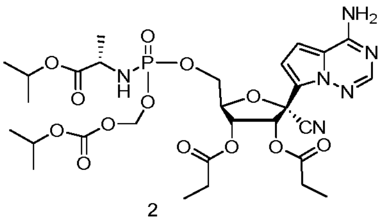

2

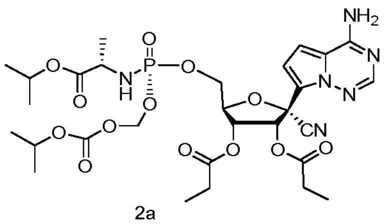

2a

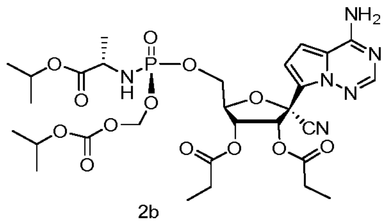

2b

The following synthetic routes were adopted:

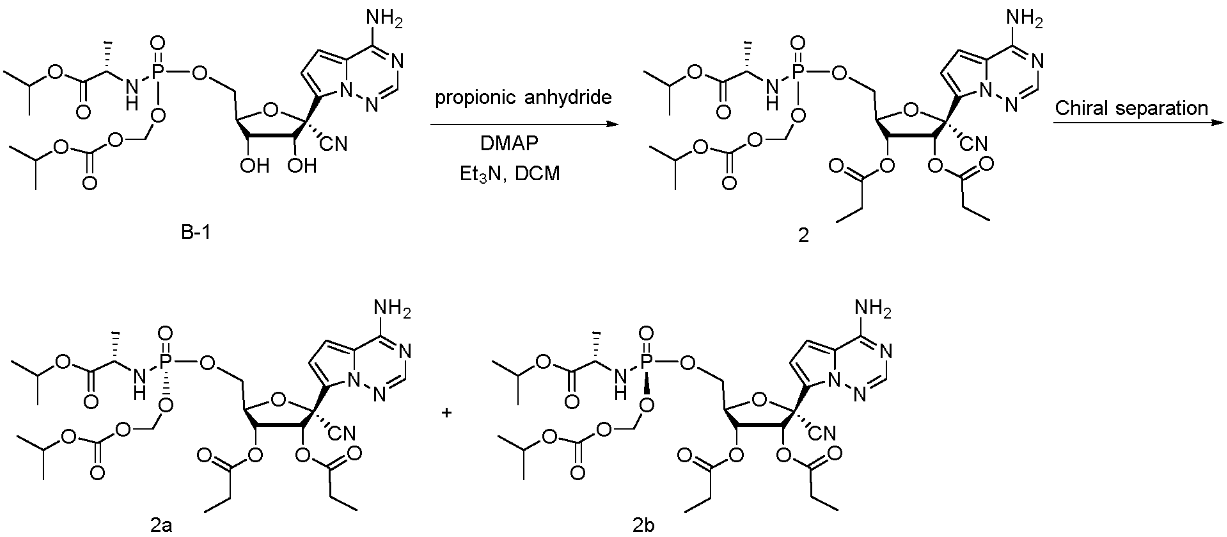


B-1 2

+

2a 2b

Step 1 Synthesis of Compound 2

The compound B-1 (186 mg, 0.31 mmol) was added to a reaction flask, followed by the addtion of 10 mL of

anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 128 mg of the target product (yield: 58%). LC-MS (APCI): m/z=713.3 $(M+1)^+$. $^1H$ NMR (400 MHz, DMSO-$d_6$) δ 7.94 (s, 1H), 6.93 (d, *J* = 4.4 Hz, 1H), 6.80 (d, *J= 4.5* Hz, 1H), 6.06 (d, *J* = 5.8 Hz, 1H), 5.84 (dd, *J* = 23.2, 13.2 Hz, 1H), 5.52 - 5.38 (m, 3H), 4.91 - 4.74 (m, 2H), 4.58 (dd, *J* = 14.9, 3.5 Hz, 1H), 4.26 - 4.07 (m, 2H), 3.67 - 3.49 (m, 1H), 2.47 - 2.31 (m, 4H), 1.29 - 0.97 (m, 21H).

Step 2 Synthesis of Compound 2a and Compound 2b

The racemate compound 2 was separated with reverse phase preparative chromatography to obtain the target product 2a (retention time: 8.47 min, relative content: 54.5%) and 2b (retention time: 9.18 min, relative content: 45.5%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 3: Preparation of (2R,3R,4R,5R)-2-(4-aminopyrrolo[2,1-f][1,2,4]triazin-7-yl)-2-cyano-5-((((((S)-1-isopropoxy-1-oxopropan-2-yl)amino)(((isopropoxycarbonyl)oxy)methoxy)phosphoryl)oxy)methyl)tetrahydrofuran-3,4-diyl bis(2-methylpropionate) (Compound 3),**

**(2R,3R,4R,5R)-2-(4-aminopyrrolo[2,1-f][1,2,4]triazin-7-yl)-2-cyano-5-((((R)-(((S)-1-isopropoxy-1-oxopropan-2-yl)amino)(((isopropoxycarbonyl)oxy)methoxy)phosphoryl)oxy)methyl)tetrahydrofuran-3,4-diyl bis(2-methylpropionate) (Compound 3a) and**

**(2R,3R,4R,5R)-2-(4-aminopyrrolo[2,1-f][1,2,4]triazin-7-yl)-2-cyano-5-((((S)-(((S)-1-isopropoxy-1-oxopropan-2-yl)amino)(((isopropoxycarbonyl)oxy)methoxy)phosphoryl)oxy)methyl)tetrahydrofuran-3,4-diyl bis(2-methylpropionate) (Compound 3b)**

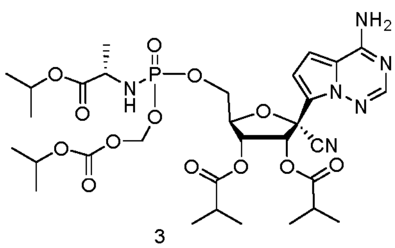
3

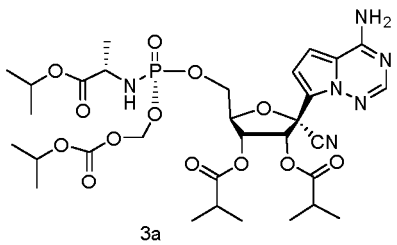
3a

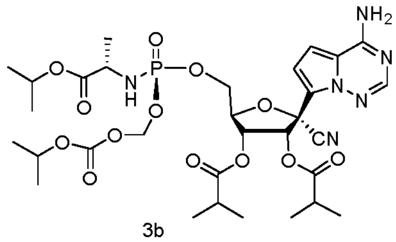
3b

The following synthetic routes were adopted:

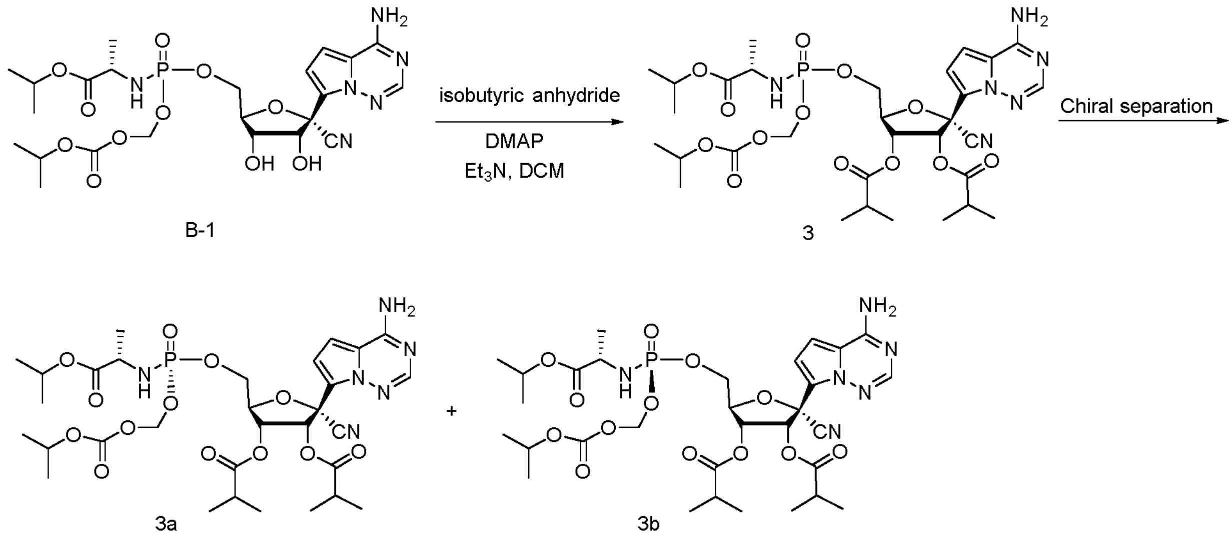


B-1

3

3a

+

3b

Step 1 Synthesis of Compound 3

The intermediate B-1 (186 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 89 mg of the target product (yield: 39%). LC-MS (APCI): m/z=741.3 $(M+1)^+$. $^1H$ NMR (400 MHz, DMSO-$d_6$) $\delta$ 7.93 (s, 1H), 6.92 (s, 1H), 6.79 (s, 1H), 6.05 (s, 1H), 5.84 (d, $J$ = 11.0 Hz, 1H), 5.47 (d, $J$ = 12.8 Hz, 3H), 4.82 (d, $J$ = 5.0 Hz, 2H), 4.58 (d, $J$ = 14.2 Hz, 1H), 4.18 (s, 2H), 3.58 (d, $J$ = 18.6 Hz, 1H), 2.70 - 2.54 (m, 2H), 1.44 - 0.91 (m, 27H).

Step 2 Synthesis of Compound 3a and Compound 3b

The racemate compound 3 was separated with reverse phase preparative chromatography to obtain the target product 3a (retention time: 9.15 min, relative content: 54.1%) and 3b (retention time: 10.04 min, relative content: 45.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 4: Preparation of (2R,3R,4R,5R)-2-(4-aminopyrrolo[2,1-f][1,2,4]triazin-7-yl)-2-cyano-5-((((((S)-1-isopropoxy-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 4),**

**(2R,3R,4R,5R)-2-(4-aminopyrrolo[2,1-f][1,2,4]triazin-7-yl)-2-cyano-5-((((R)-(((S)-1-isopropoxy-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 4a) and**

**(2R,3R,4R,5R)-2-(4-aminopyrrolo[2,1-f][1,2,4]triazin-7-yl)-2-cyano-5-((((S)-(((S)-1-isopropoxy-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 4b)**

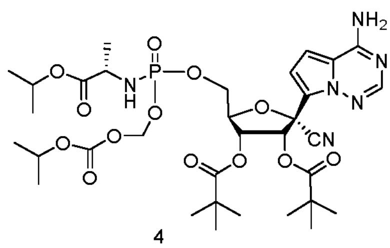
4

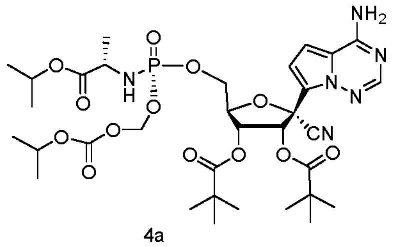
4a

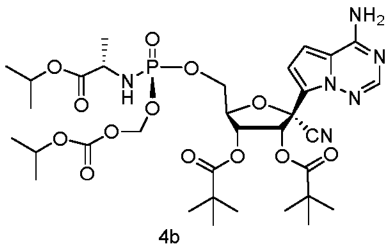
4b

The following synthetic routes were adopted:

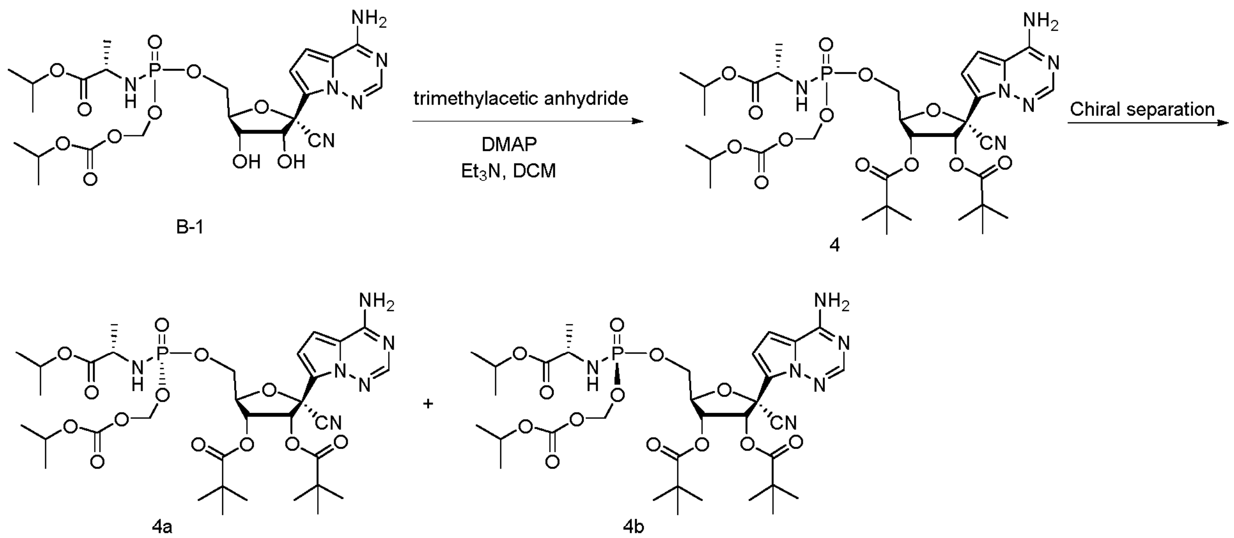

B-1
4
4a
+
4b

Step 1 Synthesis of Compound 4

The compound B-1 (186 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively.

The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 114 mg of the target product (yield: 48%). LC-MS (APCI): m/z=769.4 $(M+1)^+$. $^1H$ NMR (400 MHz, DMSO-$d_6$) δ 7.94 (s, 1H), 6.92 (s, 1H), 6.77 (s, 1H), 6.04 (d, *J* = 5.2 Hz, 1H), 5.83 (d, *J* = 12.0 Hz, 1H), 5.56 - 5.37 (m, 3H), 4.83 (s, 2H), 4.55 (d, *J* = 17.8 Hz, 1H), 4.17 (s, 2H), 1.18 (d, *J* = 27.9 Hz, 33H).

Step 2 Synthesis of Compound 4a and Compound 4b

The racemate compound 4 was separated with reverse phase preparative chromatography to obtain the target product 4a (retention time: 7.38 min, relative content: 53.4%) and 4b (retention time: 8.14 min, relative content: 46.6%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 5: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl diacetate (Compound 5),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl diacetate (Compound 5a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl diacetate (Compound 5b)**

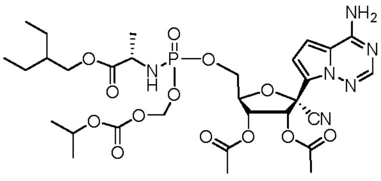

5

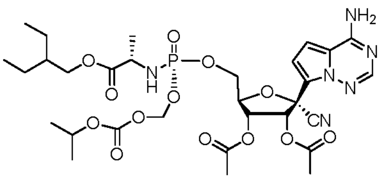

5a

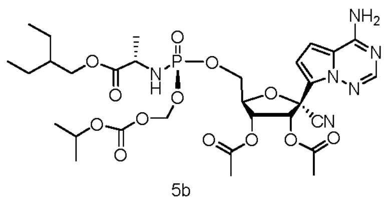

5b

The following synthetic routes were adopted:

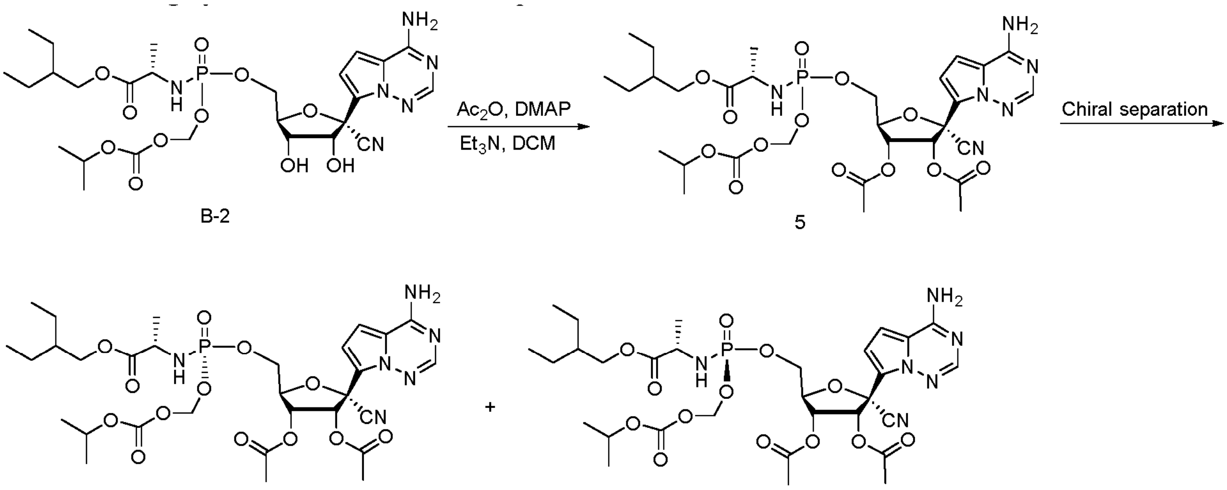


B-2

5

5a

+

5b

Step 1 Synthesis of Compound 5

The compound B-2 (199 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 151 mg of the target product (yield: 67%). LC-MS (APCI): m/z=727.4 $(M+1)^+$. $^1H$ NMR (400 MHz, DMSO-$d_6$) δ 7.92 (s, 1H), 6.91 (d, $J$ = 4.6 Hz, 1H), 6.81 (d, $J$ = 4.5 Hz, 1H), 6.03 (d, $J$ = 5.8 Hz, 1H), 5.86 (dd, $J$ = 12.7, 10.1 Hz, 1H), 5.51 - 5.39 (m, 3H), 4.80 (dt, $J$ = 12.5, 6.2 Hz, 1H), 4.60 (d, $J$ = 3.5 Hz, 1H), 4.25 - 4.10 (m, 2H), 3.99 (dd, $J$ = 10.8, 5.9 Hz, 1H), 3.90 (dd, $J$ = 10.8, 5.5 Hz, 1H), 3.65 (dd, $J$ = 16.8, 9.6 Hz, 1H), 2.46 - 2.30 (m, 4H), 1.46 (dt, $J$ = 12.3, 6.1 Hz, 1H), 1.32 - 1.00 (m, 13H), 0.82 (t, $J$ = 7.4 Hz, 6H).

Step 2 Synthesis of Compound 5a and Compound 5b

The racemate compound 5 was separated with reverse phase preparative chromatography to obtain the target product 5a (retention time: 8.69 min, relative content: 51.7%) and 5b (retention time: 9.75 min, relative content: 48.3%).

Conditions of chromatographic separation:

Column: Waters, Prep C18 OBD™, 150×19 mm, 5 μm

Column temperature: 30°C
Flow rate: 1.0 mL/min
Wavelength of UV detection: 220 nm
Mobile phase: pure water:acetonitrile=40:60

**Example 6: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl dipropionate (Compound 6),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl dipropionate (Compound 6a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl dipropionate (Compound 6b)**

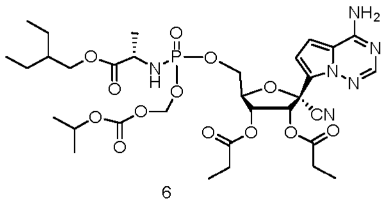
6

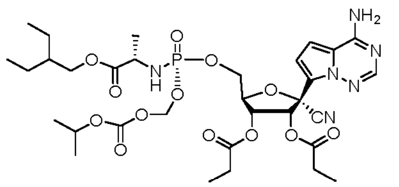
6a

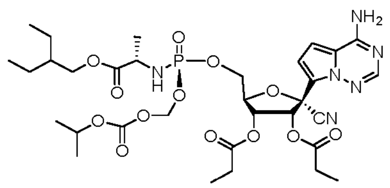
6b

The following synthetic routes were adopted:

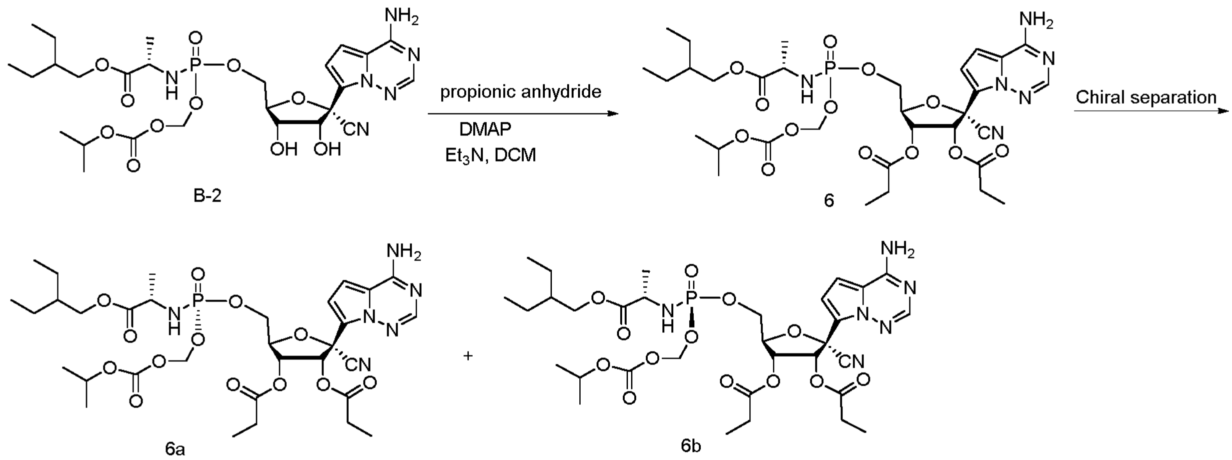

B-2
6
6a
+
6b

Step 1 Synthesis of Compound 6

The compound B-2 (199 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 86 mg of the target product (yield: 37%). LC-MS (APCI): m/z=755.5 $(M+1)^+$. $^1$H NMR (400 MHz, DMSO-$d_6$) δ 7.94 (s, 1H), 6.92 (d, *J* = 4.5 Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 2H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 2.46 - 2.32 (m, 4H), 1.46 (dt, *J* = 12.3, 6.1 Hz, 1H), 1.33 - 1.00 (m, 19H), 0.82 (t, *J* = 7.4 Hz, 6H).

Step 2 Synthesis of Compound 6a and Compound 6b

The racemate compound 6 was separated with reverse phase preparative chromatography to obtain the target product 6a (retention time: 8.37 min, relative content: 53.6%) and 6b (retention time: 9.25 min, relative content: 46.4%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 7: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2-methylpropionate) (Compound 7),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2-methylpropionate) (Compound 7a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2-methylpropionate) (Compound 7b)**

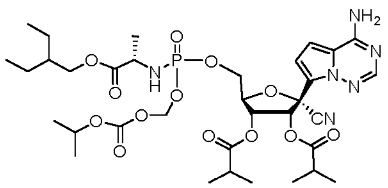
7

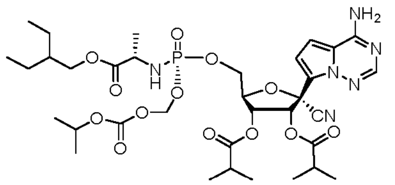
7a

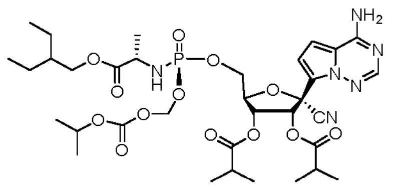
7b

The following synthetic routes were adopted:

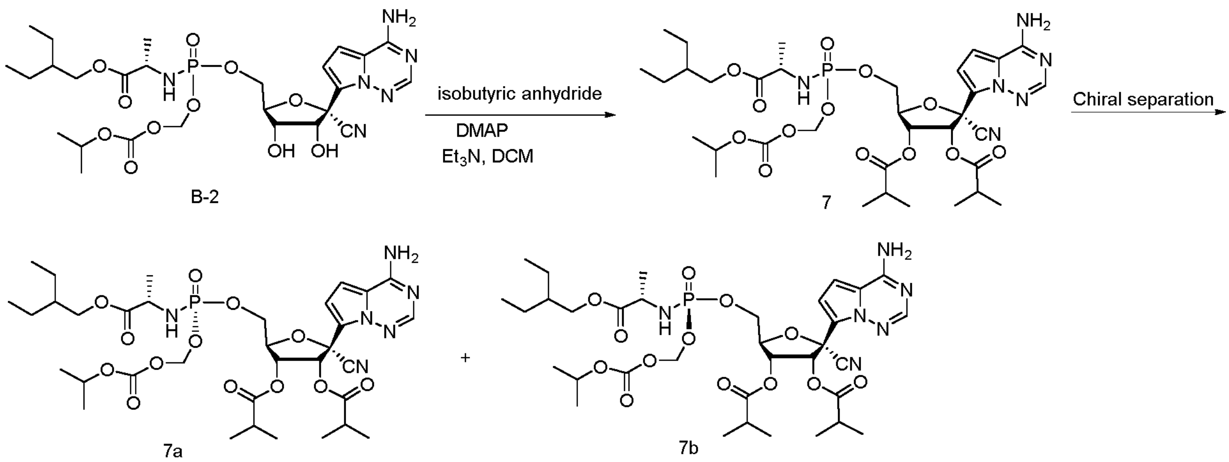

B-2
7
7a
+
7b

Step 1 Synthesis of Compound 7

The intermediate B-2 (199 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small

amount of water and extracted with dichloromethane 3-4 times. The organic layers were combined, washed with saturated saline solution, concentrated, and purified by silica gel column chromatography to obtain 136 mg of the target product (yield: 56%). LC-MS (APCI): m/z=783.7 $(M+1)^+$. $^1H$ NMR (400 MHz, DMSO-$d_6$) δ 7.91 (s, 1H), 6.90 (d, $J$ = 4.6 Hz, 1H), 6.80 (d, $J$ = 4.6 Hz, 1H), 6.06 (d, $J$ = 5.4 Hz, 1H), 5.86 (dd, $J$ = 12.6, 10.1 Hz, 1H), 5.51 - 5.39 (m, 3H), 4.80 (dt, $J$ = 12.5, 6.2 Hz, 1H), 4.60 (d, $J$ = 3.5 Hz, 1H), 4.25 - 4.10 (m, 2H), 3.99 (dd, $J$ = 10.8, 5.9 Hz, 1H), 3.90 (dd, $J$ = 10.8, 5.7 Hz, 1H), 3.65 (dd, $J$ = 16.8, 9.6 Hz, 1H), 2.46 - 2.32 (m, 2H), 1.46 (dt, $J$ = 12.3, 6.1 Hz, 1H), 1.33 - 1.00 (m, 19H), 0.81 (m, 12H).

Step 2 Synthesis of Compound 7a and Compound 7b

The racemate compound 7 was separated with reverse phase preparative chromatography to obtain the target product 7a (retention time: 8.39 min, relative content: 51.6%) and 7b (retention time: 9.27 min, relative content: 48.4%).
Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 8: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 8),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 8a) and**

**(2R,3R,4R,5R)-2-(4-aminopyrrolo [2,1-f][1,2,4]triazin-7-yl)-2-cyano-5-((((S)-(((S)-1-(2-ethylbutoxy)-1-oxopropan-2-yl)amino)(((isopropoxycarbonyl)oxy)methoxy)phosphoryl)oxy)methyl)tetrahydrofuran-3,4-diyl bis(2,2-dimethylpropionate) (Compound 8b)**

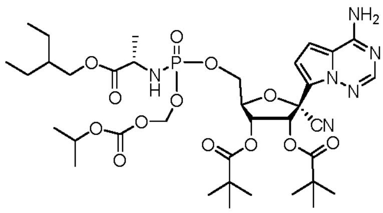
8

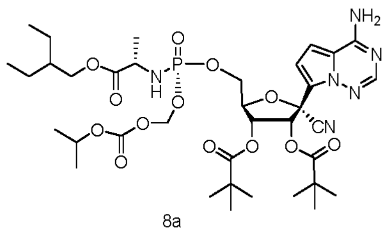
8a

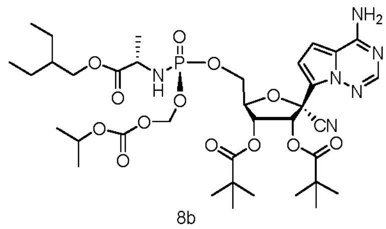
8b

The following synthetic routes were adopted:

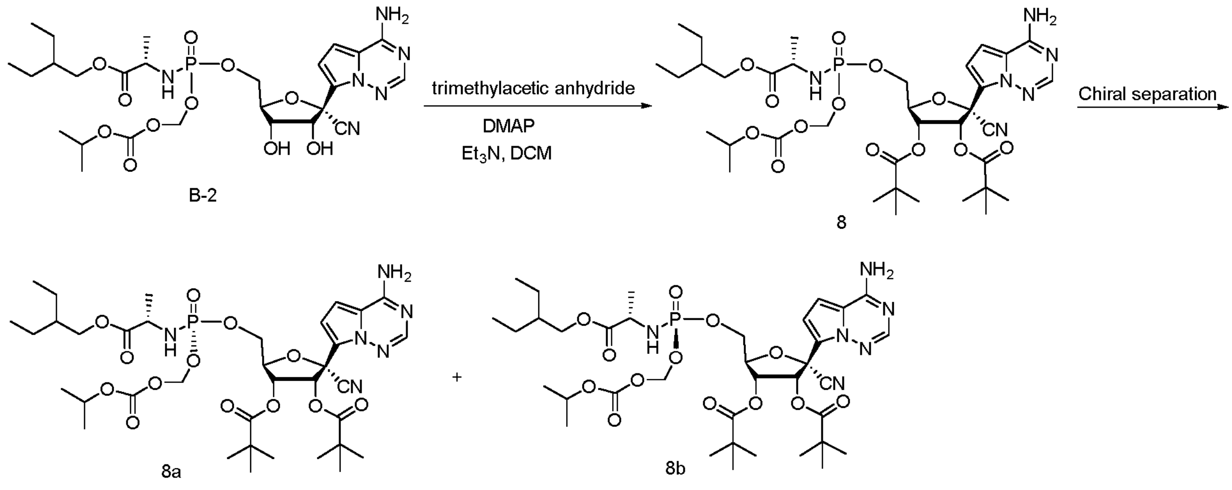


B-2 8 8a + 8b

Step 1 Synthesis of Compound 8

The intermediate B-2 (199 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 100 mg of the target product (yield: 40%). LC-MS (APCI): m/z=811.2 $(M+1)^+$. $^1H$ NMR (400 MHz, DMSO-$d_6$) δ 7.94 (s, 1H), 6.92 (d, *J* = 4.5 Hz, 1H), 6.80 (d, *J= 4.5* Hz, 1H), 6.06 (d, *J* = 5.4 Hz, 1H), 5.86 (dd, *J* = 12.6, 10.2 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 2H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 1.46 (dt, *J* = 12.3, 6.1 Hz, 1H), 1.33 - 0.86 (m, 37H).

Step 2 Synthesis of Compound 8a and Compound 8b

The racemate compound 8 was separated with reverse phase preparative chromatography to obtain the target product 8a (retention time: 7.38 min, relative content: 54.1%) and 8b (retention time: 8.24 min, relative content: 45.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 9: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)((pivaloyloxy)methoxy)phosphoryl)oxy)methyl)tetrahydrofuran-3,4-diyl diacetate (Compound 9),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)((pivaloyloxy)methoxy)phosphoryl)oxy)methyl)tetrahydrofuran-3,4-diyl diacetate (Compound 9a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)((pivaloyloxy)methoxy)phosphoryl)oxy)methyl)tetrahydrofuran-3,4-diyl diacetate (Compound 9b)**

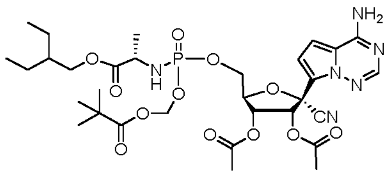

9

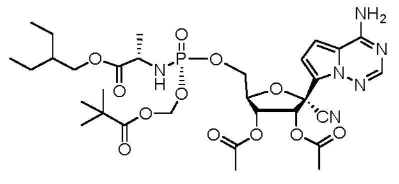

9a

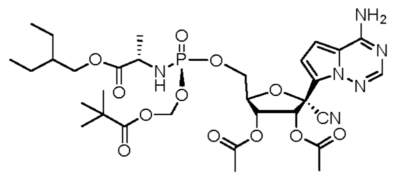

9b

The following synthetic routes were adopted:

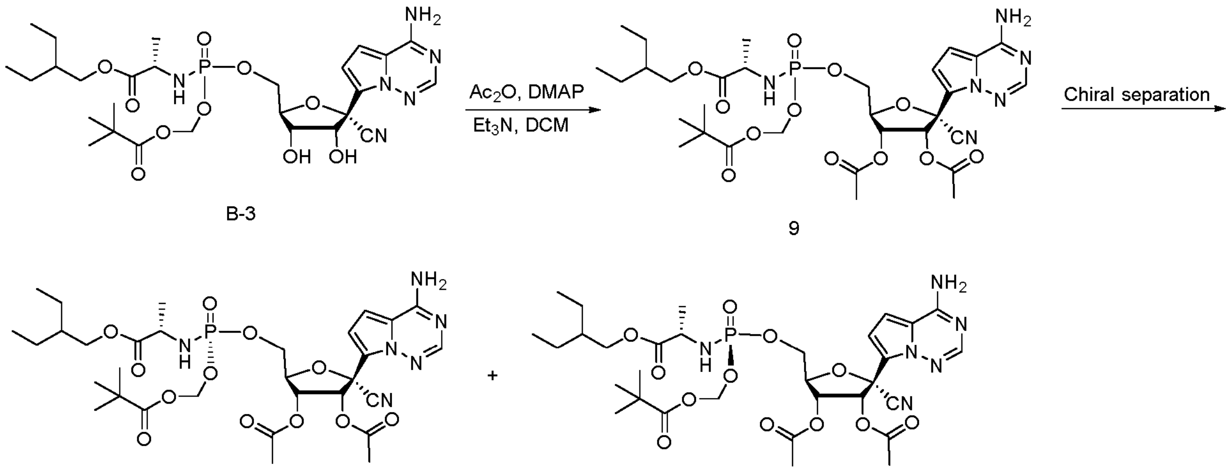


B-3

9

+

9a

9b

Step 1 Synthesis of Compound 9

The intermediate B-3 (198 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 164 mg of the target product (yield: 73%). LC-MS (APCI): m/z=725.6 $(M+1)^+$. $^1$H NMR (400 MHz, DMSO-$d_6$) δ 7.94 (s, 1H), 6.92 (d, *J= 4.5* Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 1H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 2.03 (s, 6H), 1.46 (dt, *J* = 12.3, 6.1 Hz, 1H), 1.33 - 0.85 (m, 22H).

Step 2 Synthesis of Compound 9a and Compound 9b

The racemate compound 9 was separated with reverse phase preparative chromatography to obtain the target product 9a (retention time: 8.23 min, relative content: 56.3%) and 9b (retention time: 9.21 min, relative content: 43.7%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 10: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)((pivaloyloxy)methoxy)phosphoryl)oxy)methyl)tetrahydrofuran-3,4-diyl dipropionate (Compound 10),**

<u>**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)((pivaloyloxy)methoxy)phosphoryl)oxy)methyl)tetrahydrofuran-3,4-diyl dipropionate (Compound 10a) and**</u>

<u>**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)((pivaloyloxy)methoxy)phosphoryl)oxy)methyl)tetrahydrofuran-3,4-diyl dipropionate (Compound 10b)**</u>

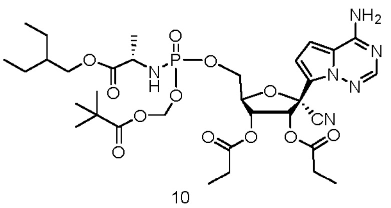
10

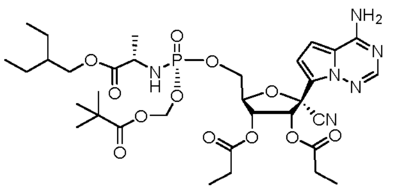
10a

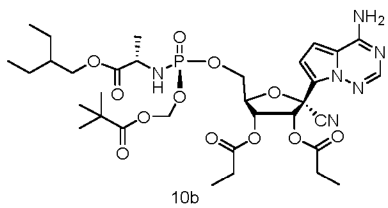
10b

The following synthetic routes were adopted:

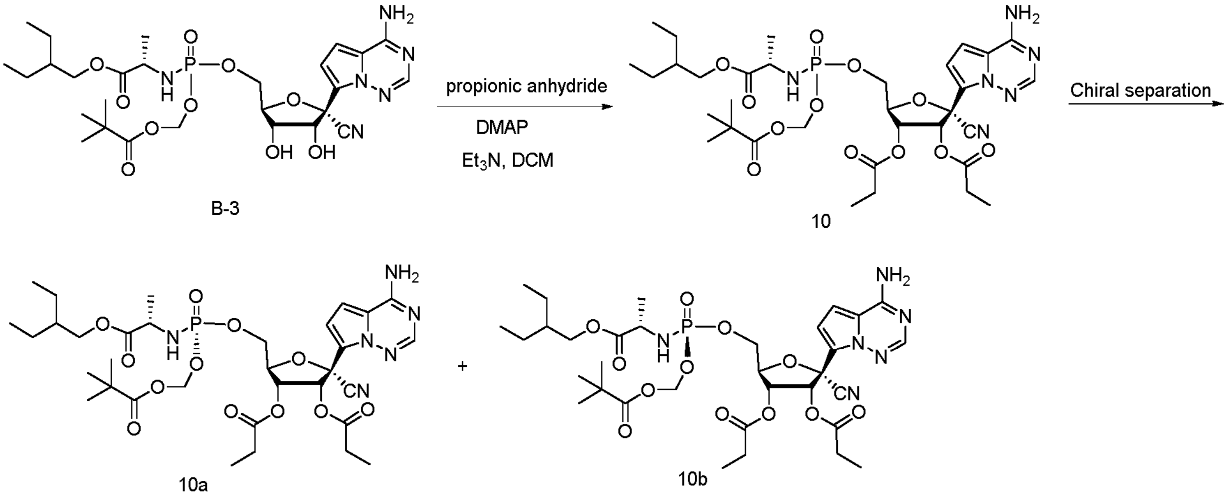

B-3
10
10a
+
10b

Step 1 Synthesis of Compound 10

The intermediate B-3 (198 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 128 mg of the target product (yield: 55%). LC-MS (APCI): m/z=753.2 $(M+1)^+$. $^1H$ NMR (400 MHz, DMSO-$d_6$) δ 7.94 (s, 1H), 6.92 (d, $J$ = 4.5 Hz, 1H), 6.80 (d, $J$ = 4.5 Hz, 1H), 6.06 (d, $J$ = 5.8 Hz, 1H), 5.86 (dd, $J$ = 12.7, 10.1 Hz, 1H), 5.51 - 5.39 (m, 3H), 4.80 (dt, $J$ = 12.5, 6.2 Hz, 1H), 4.60 (d, $J$ = 3.5 Hz, 1H), 4.25 - 4.10 (m, 1H), 3.99 (dd, $J$ = 10.8, 5.9 Hz, 1H), 3.90 (dd, $J$ = 10.8, 5.7 Hz, 1H), 3.65 (dd, $J$ = 16.8, 9.6 Hz, 1H), 2.03 (m, 4H), 1.46 (dt, $J$ = 12.3, 6.1 Hz, 1H), 1.33 - 0.85 (m, 28H).

Step 2 Synthesis of Compound 10a and Compound 10b

The racemate compound 10 was separated with reverse phase preparative chromatography to obtain the target product 10a (retention time: 8.15 min, relative content: 52.4%) and 10b (retention time: 9.01 min, relative content: 47.6%).

Conditions of chromatographic separation:

Column: Waters, Prep C18 OBD™, 150×19 mm, 5 μm
Column temperature: 30°C

Flow rate: 1.0 mL/min
Wavelength of UV detection: 220 nm
Mobile phase: pure water:acetonitrile=40:60

**Example 11: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)((pivaloyloxy)methoxy)phosphoryl)oxy)methyl)tetrahydrofuran-3,4-diyl bisdiyl bis(2-methylpropionate) (Compound 11),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)((pivaloyloxy)methoxy)phosphoryl)oxy)methyl)tetrahydrofuran-3,4-diyl bis(2-methylpropionate) (Compound 11a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)((pivaloyloxy)methoxy)phosphoryl)oxy)methyl)tetrahydrofuran-3,4-diyl bis(2-methylpropionate) (Compound 11b)**

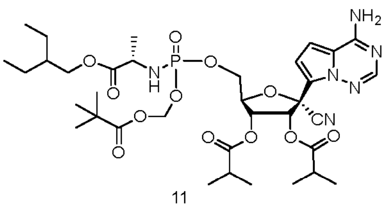
11

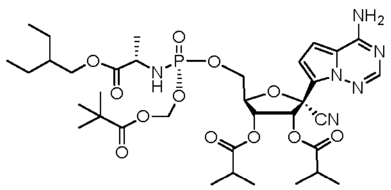
11a

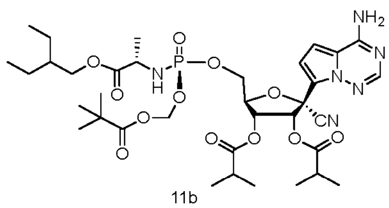
11b

The following synthetic routes were adopted:

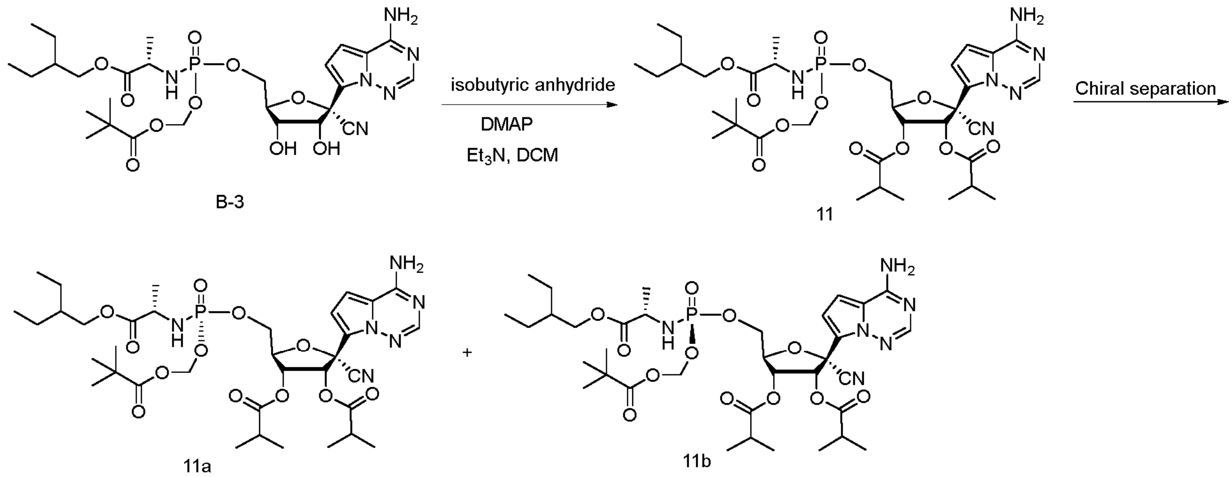

B-3

11

11a + 11b

Step 1 Synthesis of Compound 11

The intermediate B-3 (198 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 128 mg of the target product (yield: 53%). LC-MS (APCI): m/z=781.1 $(M+1)^+$. $^1H$ NMR (400 MHz, DMSO-$d_6$) δ 7.94 (s, 1H), 6.92 (d, *J*= *4.5* Hz, 1H), 6.80 (d, *J*= *4.5* Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 1H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 2.04 (m, 2H), 1.46 (dt, *J* = 12.3, 6.1 Hz, 1H), 1.33 - 0.84 (m, 34H).

Step 2 Synthesis of Compound 11a and Compound 11b

The racemate compound 11 was separated with reverse phase preparative chromatography to obtain the target product 11a (retention time: 8.67 min, relative content: 54.7%) and 11b (retention time: 9.93 min, relative content: 45.3%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 12: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)((pivaloyloxy)methoxy)phosphoryl)oxy)methyl)tetrahydrofuran-3,4-diyl bis(2,2-dimethylpropionate) (Compound 12),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)((pivaloyloxy)methoxy)phosphoryl)oxy)methyl)tetrahydrofuran-3,4-diyl bis(2,2-dimethylpropionate) (Compound 12a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)((pivaloyloxy)methoxy)phosphoryl)oxy)methyl)tetrahydrofuran-3,4-diyl bis(2,2-dimethylpropionate) (Compound 12b)**

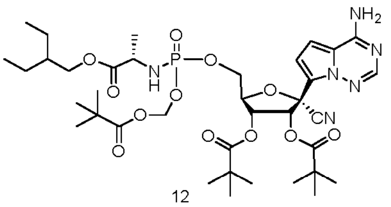
12

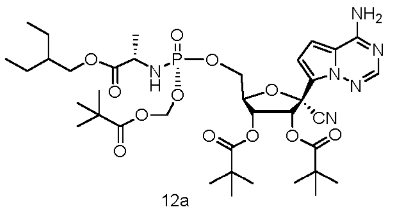
12a

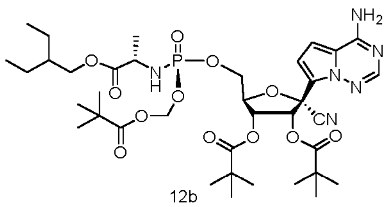
12b

The following synthetic routes were adopted:

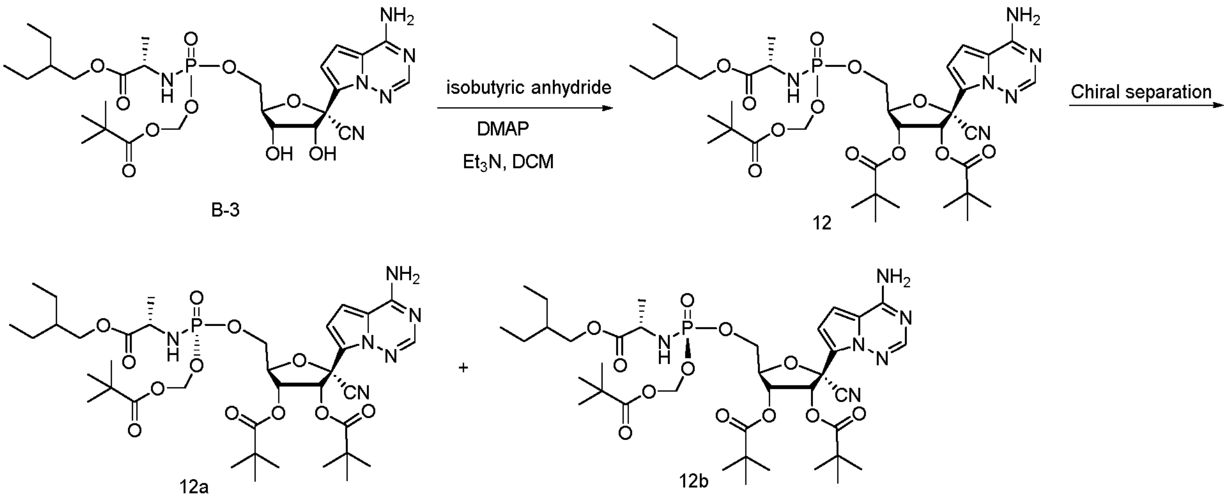

B-3
12
12a
+
12b

Step 1 Synthesis of Compound 12

The intermediate B-3 (198 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen

atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 93 mg of the target product (yield: 37%). LC-MS (APCI): m/z=809.4 $(M+1)^+$. $^1H$ NMR (400 MHz, DMSO-$d_6$) δ 7.94 (s, 1H), 6.92 (d, *J*= *4.5* Hz, 1H), 6.80 (d, *J*= *4.5* Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.60 (d, *J* = 3.5 Hz, 1H), 4.25 - 4.10 (m, 1H), 3.99 (dd, *J* = 10.8, 5.9 Hz, 1H), 3.90 (dd, *J* = 10.8, 5.7 Hz, 1H), 3.65 (dd, *J* = 16.8, 9.6 Hz, 1H), 1.46 (dt, *J* = 12.3, 6.1 Hz, 1H), 1.33 - 0.85 (m, 40H).

Step 2 Synthesis of Compound 12a and Compound 12b

The racemate compound 12 was separated with reverse phase preparative chromatography to obtain the target product 12a (retention time: 8.01 min, relative content: 52.4%) and 12b (retention time: 8.92 min, relative content: 47.6%).
Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 13: Preparation of (2R,3R,4R,5R)-2-(4-aminopyrrolo[2,1-f][1,2,4]triazin-7-yl)-2-cyano-5-((((((S)-1-cyclobutoxy-1-oxopropan-2-yl)amino)(((isopropoxycarbonyl)oxy)methoxy)phosphoryl)oxy)methyl)tetrahydrofuran-3,4-diyl bis(2-methylpropionate) (Compound 13),**

**(2R,3R,4R,5R)-2-(4-aminopyrrolo[2,1-f][1,2,4]triazin-7-yl)-2-cyano-5-((((R)-(((S)-1-cyclobutoxy-1-oxopropan-2-yl)amino)(((isopropoxycarbonyl)oxy)methoxy)phosphoryl)oxy)methyl)tetrahydrofuran-3,4-diyl bis(2-methylpropionate) (Compound 13a) and**

**(2R,3R,4R,5R)-2-(4-aminopyrrolo[2,1-f][1,2,4]triazin-7-yl)-2-cyano-5-((((S)-(((S)-1-cyclobutoxy-1-oxopropan-2-yl)amino)(((isopropoxycarbonyl)oxy)methoxy)phosphoryl)oxy)methyl)tetrahydrofuran-**3,4-diyl **bis(2-methylpropionate) (Compound 13b)**

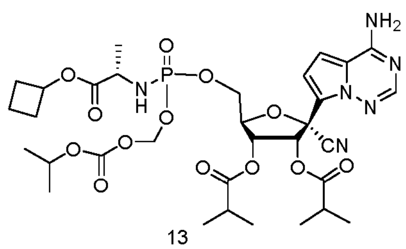
13

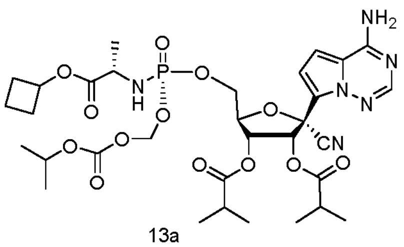
13a

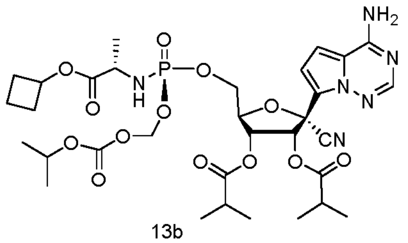
13b

The following synthetic routes were adopted:

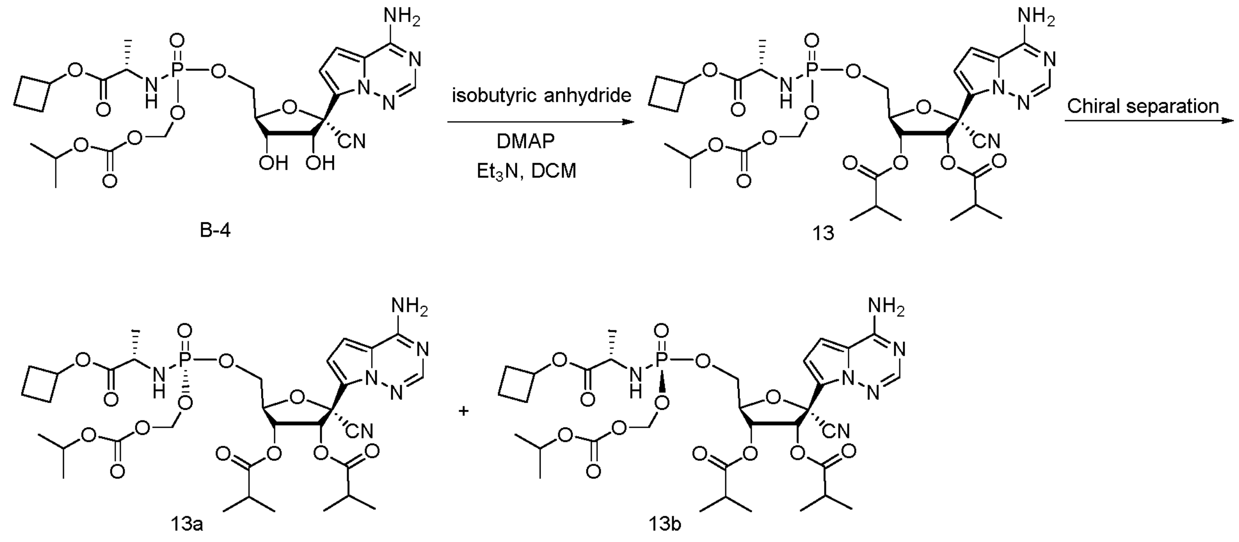


B-4 13 13a + 13b

Step 1 Synthesis of Compound 13

The intermediate B-4 (190 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 121 mg of the target product (yield: 52%). LC-MS (APCI): m/z=753.3 $(M+1)^+$. $^1H$ NMR (400 MHz, DMSO-$d_6$) δ 7.93 (s, 1H), 6.93 (d, *J* = 4.7 Hz, 1H), 6.79 (d, *J*= 4.6 Hz, 1H), 6.06 (d, *J* = 5.8 Hz, 1H), 5.83 (dd, *J* = 13.0, 10.0 Hz, 1H), 5.45 (dq, *J* = 7.2, 5.6 Hz, 3H), 4.88 - 4.76 (m, 2H), 4.60 (d, *J* = 3.2 Hz, 1H), 4.19 (d, *J* = 3.8 Hz, 2H), 3.57 (d, *J* = 7.2 Hz, 1H), 2.62 (dd, *J* = 12.0, 5.0 Hz, 3H), 2.24 (dd, *J* = 16.3, 8.4 Hz, 2H), 2.06 - 1.89 (m, 4H), 1.72 (d, *J* = 10.0 Hz, 1H), 1.57 (dd, *J* = 18.5, 8.0 Hz, 1H), 1.23 (dd, *J* = 6.0, 1.5 Hz, 9H), 1.19 - 1.06 (m, 12H).

Step 2 Synthesis of Compound 13a and Compound 13b

The racemate compound 13 was separated with reverse phase preparative chromatography to obtain the target product 13a (retention time: 7.34 min, relative content: 55.1%) and 13b (retention time: 8.37 min, relative content: 44.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

## Example 14: Preparation of (2R,3R,4R,5R)-2-(4-aminopyrrolo[2,1-f][1,2,4]triazin-7-yl)-2-cyano-5-((((((S)-1-(2,2-dimethylbutoxy)-1-oxopropan-2-yl)amino)(((isopropoxycarbonyl)oxy)methoxy)phosphoryl)oxy)methyl)tetrahydrofuran-3,4-diyl bis(2-methylpropionate) (Compound 14),

**(2R,3R,4R,5R)-2-(4-aminopyrrolo[2,1-f][1,2,4]triazin-7-yl)-2-cyano-5-((((R)-(((S)-1-(2,2-dimethylbutoxy)-1-oxopropan-2-yl)amino)(((isopropoxycarbonyl)oxy)methoxy)phosphoryl)oxy)methyl)tetrahydrofuran-3,4-diyl bis(dimethyl 2-methylpropanoate) (Compound 14a) and**

**(2R,3R,4R,5R)-2-(4-aminopyrrolo[2,1-f][1,2,4]triazin-7-yl)-2-cyano-5-((((S)-(((S)-1-(2,2-dimethylbutoxy)-1-oxopropan-2-yl)amino)(((isopropoxycarbonyl)oxy)methoxy)phosphoryl)oxy)methyl)tetrahydrofuran-3,4-diyl bis(2-methylpropionate) (Compound 14b)**

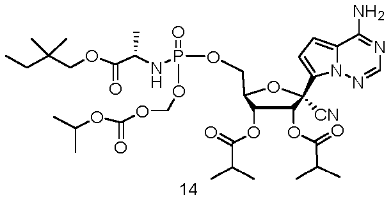

14

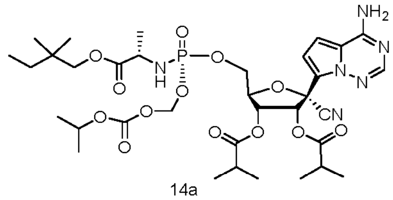

14a

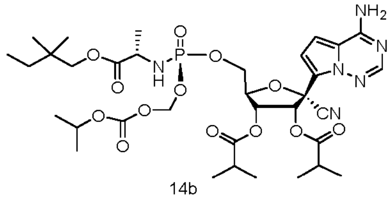

14b

The following synthetic routes were adopted:

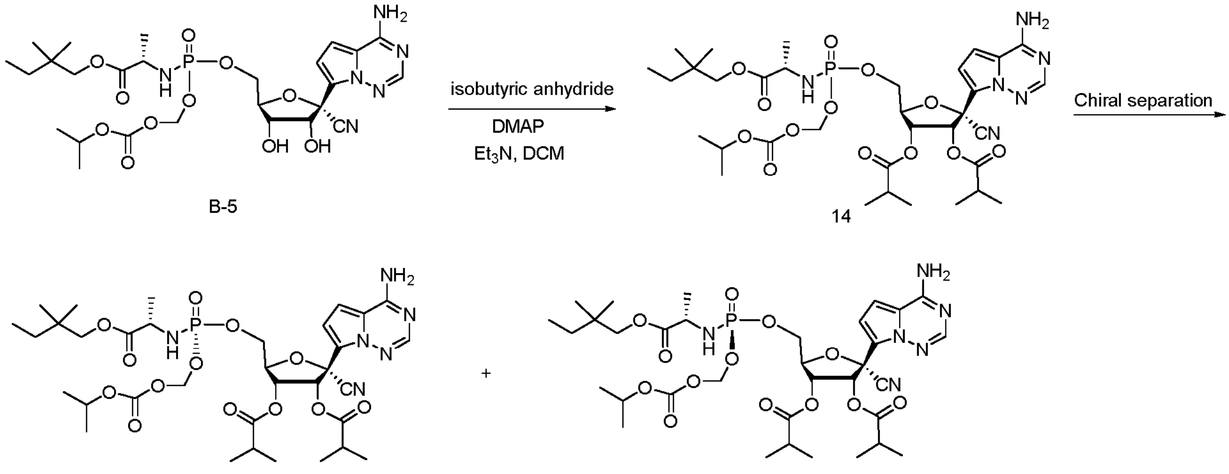


B-5

14

14a

+

14b

Step 1 Synthesis of Compound 14

The intermediate B-4 (199 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-5 times. The organic layers were combined, washed with saturated saline solution, concentrated, and purified by silica gel column chromatography to obtain 153 mg of the target product (yield: 63%). LC-MS (APCI): m/z=783.4 $(M+1)^+$. $^1$H NMR (400 MHz, DMSO-$d_6$) δ 7.93 (s, 1H), 6.90 (d, *J*= 4.5 Hz, 1H), 6.80 (d, *J*= *4.5* Hz, 1H), 6.06 (d, *J* = 5.8 Hz, 1H), 5.86 (dd, *J* = 12.7, 10.1 Hz, 1H), 5.51 - 5.39 (m, 3H), 4.80 (dt, *J* = 12.5, 6.2 Hz, 1H), 4.61 (d, *J*= *3.5* Hz, 1H), 4.25 - 4.10 (m, 2H), 3.99 (dd, *J* = 10.8, 5.9 Hz, 1H), 3.90 (dd, *J* = 10.8, 5.7 Hz, 1H), 3.63 (dd, *J* = 16.8, 9.6 Hz, 1H), 1.46 (dt, *J* = 12.3, 6.1 Hz, 1H), 1.32 - 0.85 (m, 21H).

Step 2 Synthesis of Compound 14a and Compound 14b

The racemate compound 14 was separated with reverse phase preparative chromatography to obtain the target products 14a and 14b.

**Example 15: Preparation of (2R,3R,4R,5R)-2-(4-aminopyrrolo[2,1-f][1,2,4]triazin-7-yl)-2-cyano-5-(((((2-(ethoxycarbonyl)benzyl)oxy)(((S)-1-(2-ethylbutoxy)-1-oxopropan-2-yl)amino)phosphoryl)oxy)methyl)tetrahydrofuran-3,4-diyl bis(2-methylpropionate) (Compound 15),**

**(2R,3R,4R,5R)-2-(4-aminopyrrolo[2,1-f][1,2,4]triazin-7-yl)-2-cyano-5-((((S)-((2-(ethoxycarbonyl)benzyl)oxy)(((S)-1-(2-ethylbutoxy)-1-oxopropan-2-yl)amino)phosphoryl)oxy)methyl)tetrahydrofuran-3,4-diyl bis(2-methylpropionate) (Compound 15a) and**

**(2R,3R,4R,5R)-2-(4-aminopyrrolo[2,1-f][1,2,4]triazin-7-yl)-2-cyano-5-((((R)-((2-(ethoxycarbonyl)benzyl)oxy)(((S)-1-(2-ethylbutoxy)-1-oxopropan-2-yl)amino)phosphoryl)oxy)methyl)tetrahydrofuran-3,4-diyl bis(2-methylpropionate) (Compound 15b)**

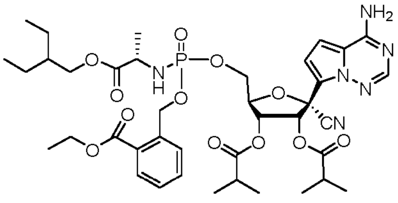

15

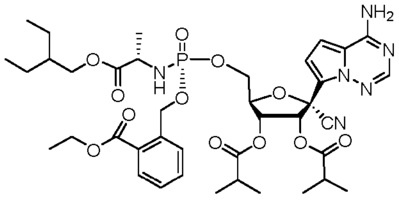

15a

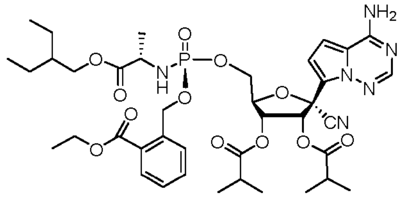

15b

The following synthetic routes were adopted:

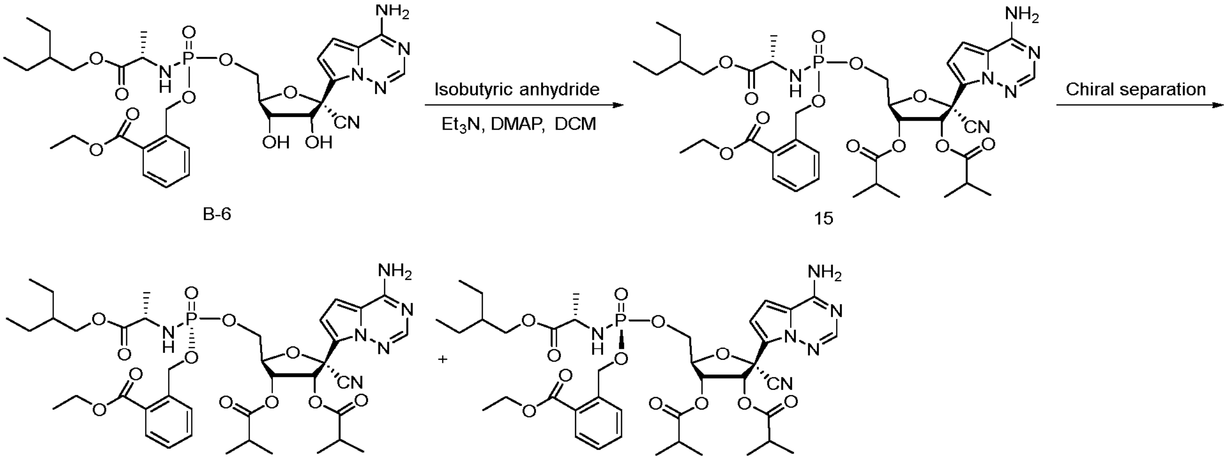


B-6

15

15a

+

15b

Step 1 Synthesis of Compound 15

The intermediate B-4 (213 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-6 times. The organic layers were combined, washed with saturated saline solution, concentrated, and purified by silica gel column chromatography to obtain 136 mg of the target product (yield: 53%). LC-MS (APCI): m/z=829.3 $(M+1)^+$. $^1H$ NMR (400 MHz, DMSO-$d_6$) $\delta$ 7.93 (s, 1H), 7.44 (d, *J= 3.5* Hz,1H), 7.42 (t, *J= 3.5* Hz, 1H), 7.22 (m, 2H), 6.90 (d, *J* = 4.5 Hz, 1H), 6.80 (d, *J* = 4.5 Hz, 1H), 6.06 (d, *J* = 5.8 Hz, 1H), 5.86 (dd, *J* = 1g2.7, 10.1 Hz, 1H), 5.51 - 5.39 (m, 2H), 4.80 (dt, *J* = 12.5, 6.2 Hz, 2H), 4.61 (d, *J*= 3.5 Hz, 2H), 4.25 - 4.10 (m, 6H), 3.99 (dd, *J* = 10.8, 5.9 Hz, 1H), 2.1 (dd, *J* = 16.8, 9.6 Hz, 2H), 1.35 - 0.81 (m, 28H).

Step 2 Synthesis of Compound 15a and Compound 15b

The racemate compound 15 was separated with reverse phase preparative chromatography to obtain the target product 15a (retention time: 7.64 min, relative content: 53.7%) and 15b (retention time: 8.47 min, relative content: 46.3%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 16: Preparation of (2R,3R,4R,5R)-2-(4-aminopyrrolo[2,1-f][1,2,4]triazin-7-yl)-2-cyano-5-((((((S)-1-(2-ethylbutoxy)-1-oxopropan-2-yl)amino)((2-methyl benzyl)oxy)phosphoryl)oxy)methyl)tetrahydrofuran-3,4-diyl bis(2-methylpropionate) (Compound 16),**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxopropan-2-yl)amino)((2-methyl benzyl)oxy)phosphoryl)oxy)methyl)tetrahydrofuran-3,4-diyl bis(2-methylpropionate) (Compound 16a) and**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxopropan-2-yl)amino)((2-methyl benzyl)oxy)phosphoryl)oxy)methyl)tetrahydrofuran-3,4-diyl bis(2-methylpropionate) (Compound 16b)**

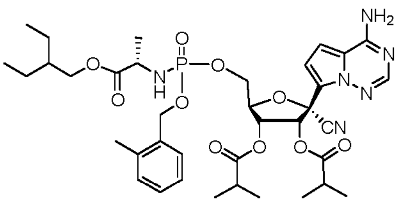
16

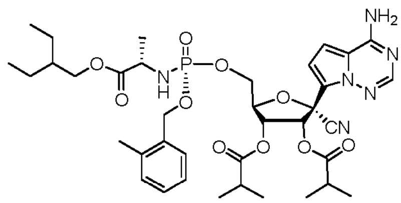
16a

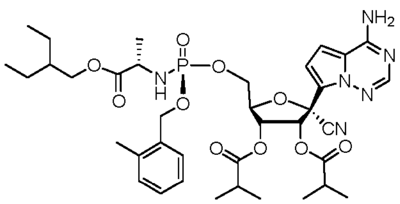
16b

The following synthetic routes were adopted:

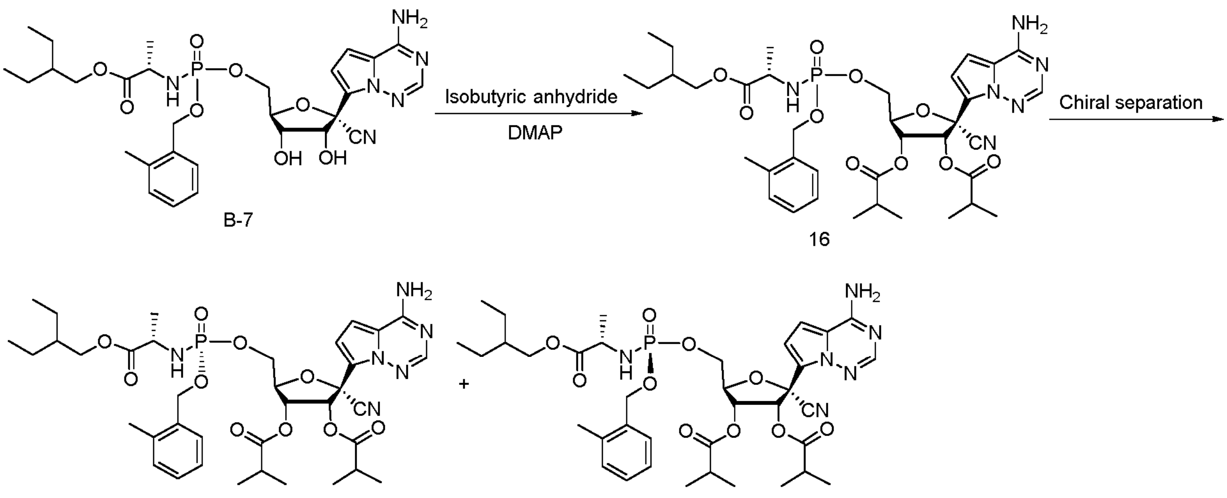

B-7 16 16a + 16b

Step 1 Synthesis of Compound 16

The intermediate B-4 (195 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-7 times. The organic layers were combined, washed with saturated saline solution, concentrated, and purified by silica gel column chromatography to obtain 81 mg of the target product (yield: 34%). LC-MS (APCI): m/z=771.2 $(M+1)^+$. $^1H$ NMR (400 MHz, DMSO-$d_6$) δ 7.94 (s, 1H), 7.42 (d, *J*= 3.5 Hz,1H), 7.45 (t, *J*= *3.5* Hz, 1H), 7.22 (m, 2H), 6.90 (d, *J* = 4.5 Hz, 1H), 6.80 (d, *J* = 4.5 Hz, 1H), 6.06 (d, *J* = 5.8 Hz, 1H), 5.86 (dd, *J* = 12.7, 10.1 Hz, 1H), 5.51 - 5.39 (m, 2H), 4.80 (dt, *J* = 12.5, 6.2 Hz, 2H), 4.61 (d, *J* = 3.5 Hz, 2H), 4.25 - 4.10 (m, 4H), 3.99 (dd, *J* = 10.8, 5.9 Hz, 1H), 2.2 (s, 3H), 2.1 (dd, *J* = 16.8, 9.6 Hz, 2H), 1.32 - 0.85 (m, 25H).

Step 2 Synthesis of Compound 16a and Compound 16b

The racemate compound 16 was separated with reverse phase preparative chromatography to obtain the target product 16a (retention time: 8.13 min, relative content: 50.7%) and 16b (retention time: 8.98 min, relative content: 49.3%).
Conditions of chromatographic separation:

Column: Waters, Prep C18 OBD™, 150×19 mm, 5 μm

Column temperature: 30°C
Flow rate: 1.0 mL/min
Wavelength of UV detection: 220 nm
Mobile phase: pure water:acetonitrile=40:60

**Example 17: Preparation of (2R,3R,4R,5R)-2-(4-aminopyrrolo[2,1-f][1,2,4]triazin-7-yl)-5-((((bis(((isopropoxycarbonyl)oxy)methoxy)phosphoryl))oxy)methyl)-2-cyanotetrahydrofuran-3,4-diyl diacetate (Compound 17)**

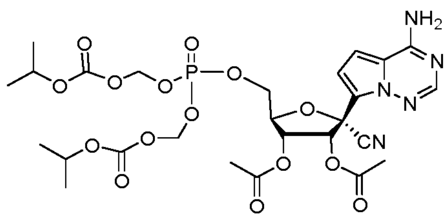

17

The following synthetic routes were adopted:

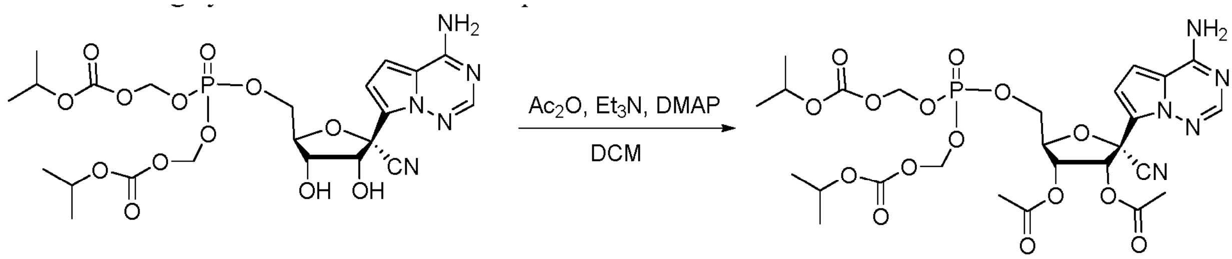


B-9

17

The intermediate B-9 (200 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 132 mg of the target product (yield: 62%). LC-MS (APCI): m/z=688.4 $(M+1)^+$. $^1H$ NMR (400 MHz, DMSO-$d_6$) δ 7.98 (s, 1H), 6.99 (d, *J*= *4.5* Hz, 1H), 6.84 (d, *J*= *4.5* Hz, 1H), 6.04 (d, *J* = 5.9 Hz, 1H), 5.54 (d, *J* = 13.1 Hz, 4H), 5.41 - 5.34 (m, 1H), 4.81 (dt, *J*= 12.3, 6.1 Hz, 2H), 4.60 (s, 1H), 4.33 (dd, *J* = 13.9, 7.8 Hz, 2H), 2.11 (s, 6H), 1.23 (d, *J* = 5.8 Hz, 12H).

**Example 18: Preparation of (2R,3R,4R,5R)-2-(4-aminopyrrolo[2,1-f][1,2,4]triazin-7-yl)-5-((((bis(((isopropoxycarbonyl)oxy)methoxy))phosphoryl)oxy)methyl)-2-cyanotetrahydrofuran-3,4-diyl dipropionate (Compound 18)**

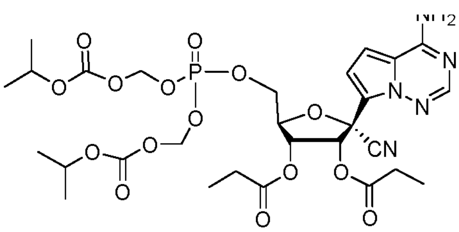

18

The following route was adopted for synthesis:

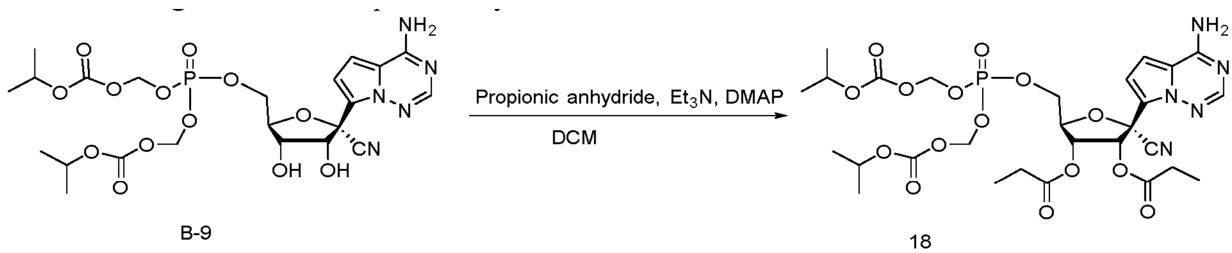


B-9

18

The intermediate B-9 (200 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 122 mg of the target product (yield: 55%). LC-MS (APCI): m/z=716.8 $(M+1)^+$. $^1H$ NMR (400 MHz, DMSO-$d_6$) δ 7.96 (s, 1H), 7.02 (d, *J*= 4.5 Hz, 1H), 6.81 (d, *J*= *4.5* Hz, 1H), 6.06 (d, *J* = 5.9 Hz, 1H), 5.54 (d, *J* = 13.1 Hz, 4H), 5.40 - 5.31 (m, 1H), 4.82 (dt, *J* = 12.3, 6.1 Hz, 2H), 4.60 (s, 1H), 4.33 (dd, *J* = 13.9, 7.8 Hz, 2H), 2.07 (m, 4H), 1.26 (d, *J* = 5.8 Hz, 12H), 1.22 (t, *J* = 4.6 Hz, 6H).

**Example 19: Preparation of (2R,3R,4R,5R)-2-(4-aminopyrrolo[2,1-f][1,2,4]triazin-7-yl)-5-((((bis(((isopropoxycarbonyl)oxy)methoxy))phosphoryl)oxy)methyl)-2-cyanotetrahydrofuran-3,4-diyl bis(2-methylpropionate) (Compound 19)**

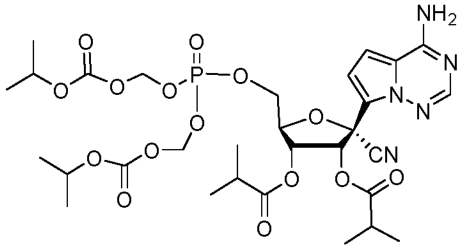

19

The following route was adopted for synthesis:

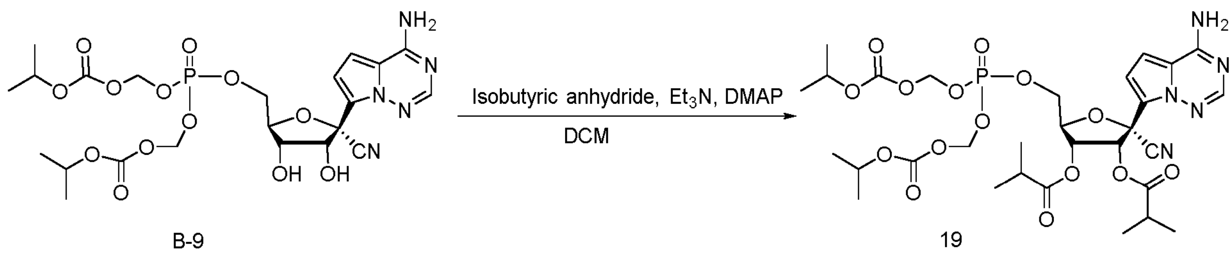


B-9

19

The intermediate B-4 (200 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-9 times. The organic layers were combined, washed with saturated saline solution, concentrated, and purified by silica gel column chromatography to obtain 117 mg of the target product (yield: 51%). LC-MS (APCI): m/z=744.4 $(M+1)^+$. $^1H$ NMR (400 MHz, DMSO-$d_6$) δ 7.97 (s, 1H), 6.99 (d, *J*= 4.6 Hz, 1H), 6.82 (d, *J* = 4.6 Hz, 1H), 6.05 (d, *J* = 5.8 Hz, 1H), 5.58 - 5.49 (m, 4H), 5.42 (dd, *J* = 5.7, 4.1 Hz, 1H), 4.81 (dt, *J* = 12.5, 6.2 Hz, 2H), 4.60 (d, *J* = 3.7 Hz, 1H), 4.34 (t, *J* = 5.9 Hz, 2H), 2.60 (dt, *J* = 14.0, 7.0 Hz, 2H), 1.23 (d, *J* = 6.1 Hz, 12H), 1.15 (dd, *J* = 8.3, 7.1 Hz, 6H), 1.09 (d, *J* = 7.0 Hz, 6H).

**Example 20: Preparation of (2R,3R,4R,5R)-2-(4-aminopyrrolo[2,1-f][1,2,4]triazin-7-yl)-5-((((bis(((isopropoxycarbonyl)oxy)methoxy))phosphoryl)oxy)methyl)-2-cyanotetrahydrofuran-3,4-diyl bis(2,2-dimethylpropionate) (Compound 20)**

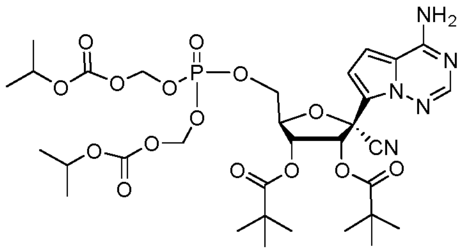

20

The following route was adopted for synthesis:

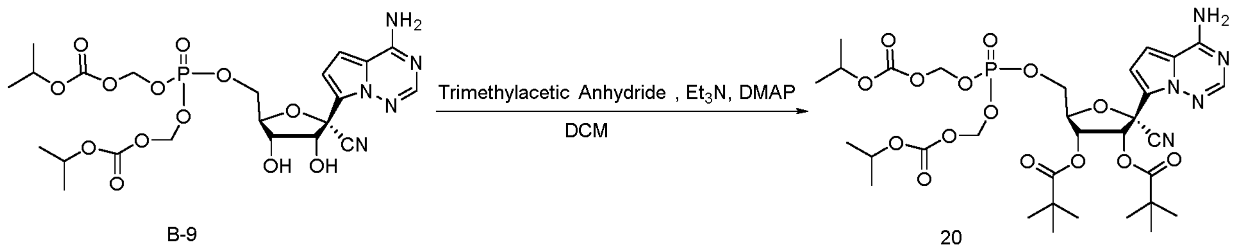


B-9

20

The intermediate B-9 (200 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 160 mg of the target product (yield: 67%). LC-MS (APCI): m/z=772.6 $(M+1)^+$. $^1H$ NMR (400 MHz, DMSO-$d_6$) δ 7.93 (s, 1H), 6.92 (d, *J*= *4.5* Hz, 1H), 6.79 (d, *J*= 4.6 Hz, 1H), 6.08 (d, *J* = 5.7 Hz, 1H), 5.53 (ddd, *J* = 11.1, 6.9, 4.4 Hz, 4H), 5.41 (dd, *J* = 5.5, 3.4 Hz, 1H), 4.81 (dt, *J* = 12.3, 6.1 Hz, 2H), 4.56 (d, *J* = 2.7 Hz, 1H), 4.39 - 4.25 (m, 2H), 1.23 (dd, *J* = 7.4, 2.9 Hz, 21H), 1.14 (s, 9H).

**Example 21: Preparation of (2R,3R,4R,5R)-2-(4-amino-5-deuteriopyrrolo[2,1-f][1,2,4]triazin-7-yl)-5-((((bis(((isopropoxycarbonyl)oxy)methoxy))phosphoryl)oxy)methyl)-2-cyanotetrahydrofuran-3,4-diyl bis(2-methylpropionate) (Compound 21)**

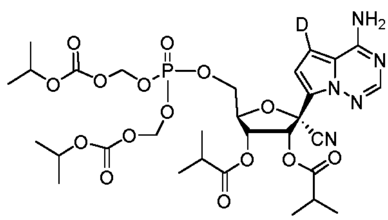

21

The following route was adopted for synthesis:

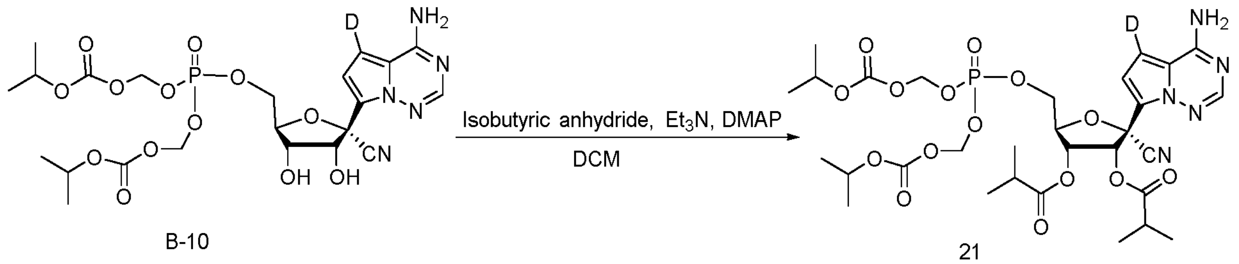


B-10 21

The intermediate B-10 (200 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 151 mg of the target product (yield: 66%). LC-MS (APCI): m/z=745.2 $(M+1)^+$. $^1H$ NMR (400 MHz, DMSO-$d_6$) δ 7.95 (s, 1H), 6.80 (s, 1H), 6.05 (d, *J* = 5.6 Hz, 1H), 5.59 - 5.49 (m, 4H), 5.41 (dd, *J*= 5.7, 4.1 Hz, 1H), 4.80 (dt, *J*= 12.5, 6.2 Hz, 2H), 4.61 (d, *J*= 3.7 Hz, 1H), 4.34 (t, *J* = 5.9 Hz, 2H), 2.60 (dt, *J* = 14.0, 7.0 Hz, 2H), 1.23 (d, *J* = 6.1 Hz, 12H), 1.15 (dd, *J* = 8.3, 7.1 Hz, 6H), 1.09 (d, *J* = 7.0 Hz, 6H).

**Example 22: Preparation of (2R,3R,4R,5R)-2-(4-aminopyrrolo[2,1-f][1,2,4]triazin-7-yl)-5-((((4-tert-butyl phenoxy)(((isopropoxycarbonyl)oxy)methoxy)phosphoryl)oxy)methyl)-2-cyanotetrahydrofuran-3,4-diyl diacetate (Compound 22),**

**(2R,3R,4R,5R)-2-(4-aminopyrrolo[2,1-f][1,2,4]triazin-7-yl)-5-((((S)-(4-tert-butyl phenoxy)(((isopropoxycarbonyl)oxy)methoxy)phosphoryl)oxy)methyl)-2-cyanotetrahydrofuran-3,4-diyl diacetate (Compound 22a) and**

**((2R,3R,4R,5R)-2-(4-aminopyrrolo[2,1-f][1,2,4]triazin-7-yl)-5-((((R)-(4-tert-butyl phenoxy)(((isopropoxycarbonyl)oxy)methoxy)phosphoryl)oxy)methyl)-2-cyanotetrahydrofuran-3,4-diyl diacetate (Compound 22b)**

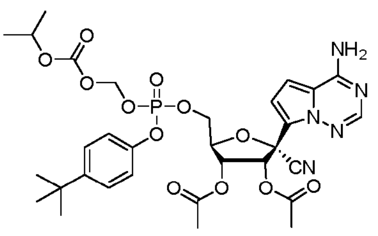

22

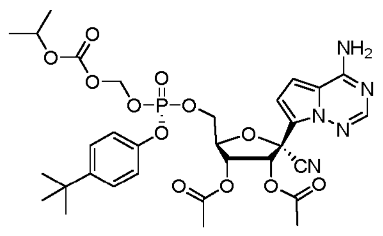

22a

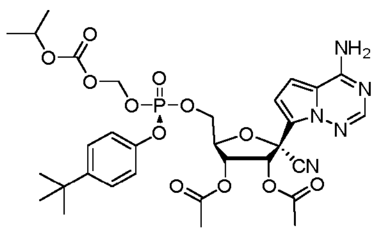

22b

The following synthetic routes were adopted:

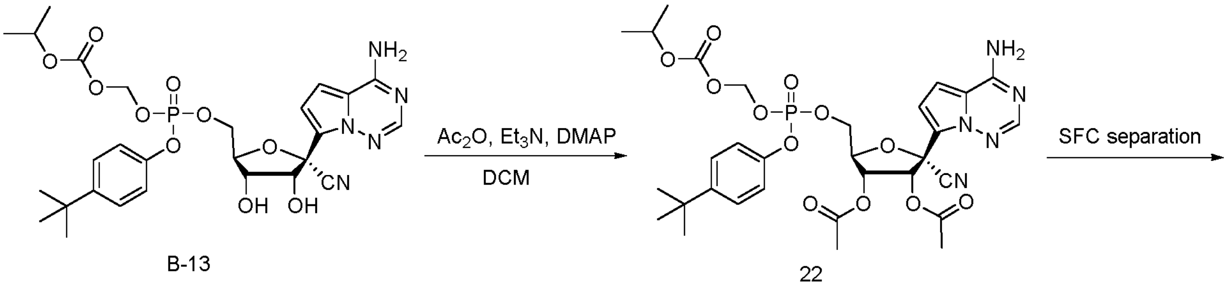


B-13

22

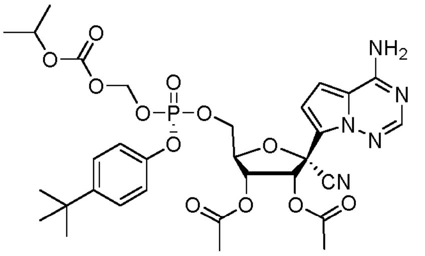

22a

+

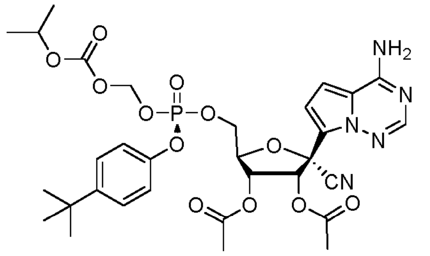

22b

Step 1 Synthesis of Compound 22

The intermediate B-13 (192 mg, 0.31 mmol) was added to a reaction flask, followed by the addtion of 10 mL of anhydrous dichloromethane to dissolve. Acetic anhydride (80 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 157 mg of the target product (yield: 72%). LC-MS (APCI): m/z=704.3 $(M+1)^+$. $^1$H NMR (400 MHz, DMSO-$d_6$) δ 7.93 (d, $J$ = 2.9 Hz, 1H), 7.24 (t, $J$ = 8.2 Hz, 2H), 6.99 (dd, $J$ = 7.9, 4.9 Hz, 2H), 6.93 (dd, $J$ = 7.1, 4.6 Hz, 1H), 6.79 (d, $J$ = 4.6 Hz, 1H), 6.05 (dd, $J$ = 6.1, 2.2 Hz, 1H), 5.68 - 5.56 (m, 2H), 5.41 (ddd, $J$ = 6.1, 4.4, 3.2 Hz, 1H), 4.85 - 4.74 (m, 1H), 4.62 (s, 1H), 4.49 - 4.37 (m, 2H), 2.11 (d, $J$ = 3.6 Hz, 6H), 1.22 (dd, $J$ = 8.1, 4.5 Hz, 15H).

Step 2 Synthesis of Compound 22a and Compound 22b

The racemate compound 22 was separated with supercritical fluid chromatography to obtain the target product 22a (retention time: 1.80 min, relative content: 50.7%) and 22b (retention time: 1.94 min, relative content: 49.3%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 23: Preparation of (2R,3R,4R,5R)-2-(4-aminopyrrolo[2,1-f][1,2,4]triazin-7-yl)-5-((((4-tert-butyl phenoxy)(((isopropoxycarbonyl)oxy)methoxy)phosphoryl)oxy)methyl)-2-cyanotetrahydrofuran-3,4-diyl dipropionate (Compound 23),**

**(2R,3R,4R,5R)-2-(4-aminopyrrolo[2,1-f][1,2,4]triazin-7-yl)-5-((((S)-(4-tert-butyl phenoxy)(((isopropoxycarbonyl)oxy)methoxy)phosphoryl)oxy)methyl)-2-cyanotetrahydrofuran-3,4-diyl dipropionate (Compound 23a) and**

**((2R,3R,4R,5R)-2-(4-aminopyrrolo[2,1-f][1,2,4]triazin-7-yl)-5-((((R)-(4-tert-butyl phenoxy)(((isopropoxycarbonyl)oxy)methoxy)phosphoryl)oxy)methyl)-2-cyanotetrahydrofuran-3,4-diyl dipropionate (Compound 23b)**

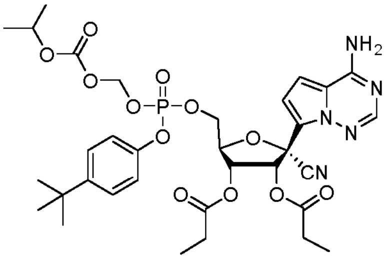

23

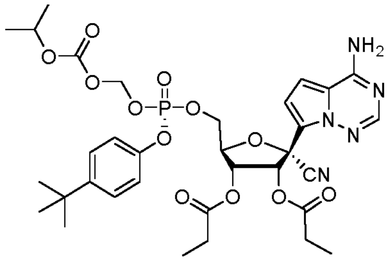

23a

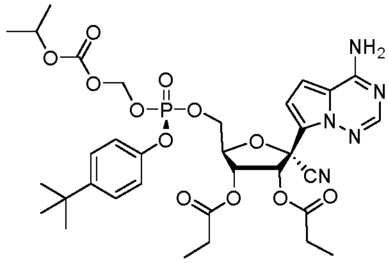

23b

The following synthetic routes were adopted:

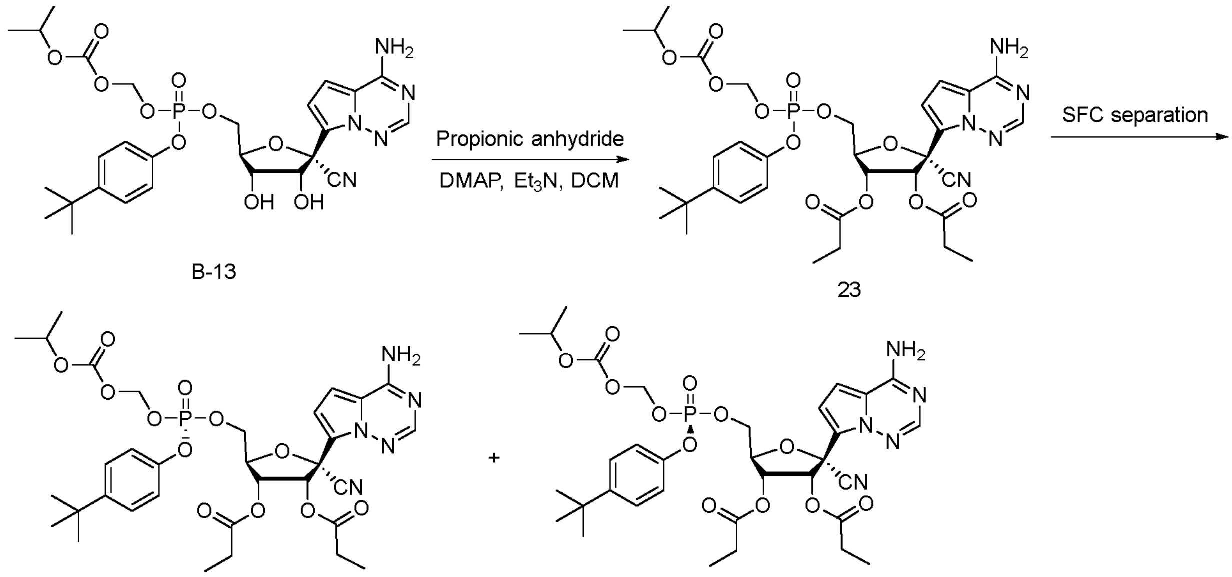


B-13

23

23a

23b

Step 1 Synthesis of Compound 23

The intermediate B-13 (192 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 129 mg of the target product (yield: 57%). LC-MS (APCI): m/z=732.9 $(M+1)^+$. $^1H$ NMR (400 MHz, DMSO-$d_6$) δ 7.92 (d, *J* = 2.9 Hz, 1H), 7.29 - 7.20 (m, 2H), 6.99 (dd, *J* = 8.0, 5.9 Hz, 2H), 6.93 (dd, *J* = 7.4, 4.7 Hz, 1H), 6.78 (d, *J* = 4.6 Hz, 1H), 6.07 (dd, *J* = 5.9, 3.5 Hz, 1H), 5.69 - 5.57 (m, 2H), 5.44 (dt, *J* = 6.0, 4.1 Hz, 1H), 4.86 - 4.74 (m, 1H), 4.62 (s, 1H), 4.44 (dd, *J* = 10.0, 4.2 Hz, 2H), 2.46 - 2.34 (m, 4H), 1.22 (dd, *J* = 8.4, 4.4 Hz, 15H), 1.06 (dt, *J* = 17.2, 7.5 Hz, 6H).

Step 2 Synthesis of Compound 23a and Compound 23b

The racemate compound 23 was separated with supercritical fluid chromatography to obtain the target product 23a (retention time: 1.78 min, relative content: 51.8%) and 23b (retention time: 1.89 min, relative content: 48.2%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 24: Preparation of (2R,3R,4R,5R)-2-(4-aminopyrrolo[2,1-f][1,2,4]triazin-7-yl)-5-((((4-tert-butyl phenoxy)(((isopropoxycarbonyl)oxy)methoxy)phosphoryl)oxy)methyl)-2-cyanotetrahydrofuran-3,4-diyl bis(2-methylpropionate) (Compound 24),**

**(2R,3R,4R,5R)-2-(4-aminopyrrolo[2,1-f][1,2,4]triazin-7-yl)-5-((((S)-(4-tert-butyl phenoxy)(((isopropoxycarbonyl)oxy)methoxy)phosphoryl)oxy)methyl)-2-cyanotetrahydrofuran-3,4-diyl bis(2-methylpropionate) (Compound 24a) and**

**((2R,3R,4R,5R)-2-(4-aminopyrrolo[2,1-f][1,2,4]triazin-7-yl)-5-((((R)-(4-tert-butyl phenoxy)(((isopropoxycarbonyl)oxy)methoxy)phosphoryl)oxy)methyl)-2-cyanotetrahydrofuran-3,4-diyl bis(2-methylpropionate) (Compound 24b)**

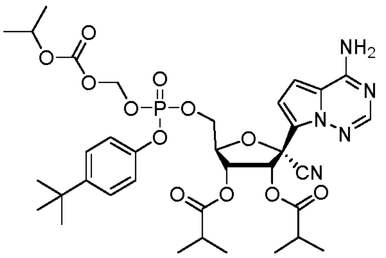

24

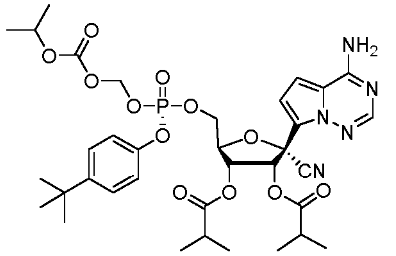

24a

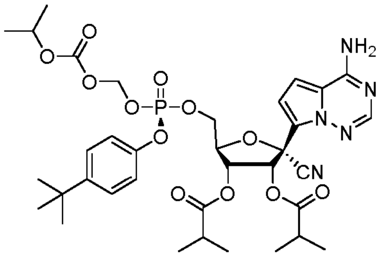

24b

The following synthetic routes were adopted:

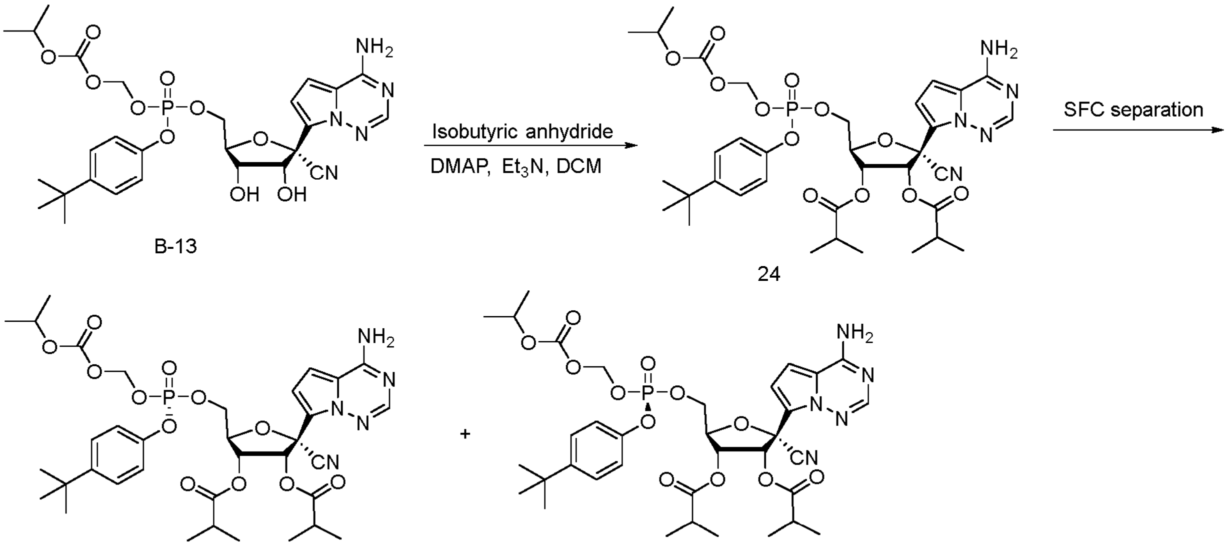


B-13

24

+

24a

24b

Step 1 Synthesis of Compound 24

The intermediate B-4 (192 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-13 times. The organic layers were combined, washed with saturated saline solution, concentrated, and purified by silica gel column chromatography to obtain 129 mg of the target product (yield: 55%). LC-MS (APCI): m/z=760.2 $(M+1)^+$. $^1H$ NMR (400 MHz, DMSO-$d_6$) δ 7.91 (d, *J* = 3.3 Hz, 1H), 7.24 (t, *J* = 8.5 Hz, 2H), 6.99 (dd, *J* = 8.1, 6.4 Hz, 2H), 6.92 (dd, *J* = 8.0, 4.6 Hz, 1H), 6.76 (dd, *J* = 4.6, 1.9 Hz, 1H), 6.05 (t, *J* = 6.1 Hz, 1H), 5.63 (ddd, *J* = 10.9, 5.5, 3.0 Hz, 2H), 5.44 (td, *J* = 5.7, 3.8 Hz, 1H), 4.79 (dt, *J* = 12.5, 6.3 Hz, 1H), 4.62 (s, 1H), 4.52 - 4.36 (m, 2H), 2.60 (tt, *J* = 14.3, 7.1 Hz, 2H), 1.22 (dd, *J* = 8.4, 4.4 Hz, 15H), 1.19 - 1.07 (m, 12H).

Step 2 Synthesis of Compound 24a and Compound 24b

The racemate compound 24 was separated with supercritical fluid chromatography to obtain the target product 24a (retention time: 1.76 min, relative content: 52.8%) and 24b (retention time: 1.89 min, relative content: 47.2%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 25: Preparation of (2R,3R,4R,5R)-2-(4-aminopyrrolo[2,1-f][1,2,4]triazin-7-yl)-5-((((4-tert-butyl phenoxy)(((isopropoxycarbonyl)oxy)methoxy)phosphoryl)oxy)methyl)-2-cyanotetrahydrofuran-3,4-diyl bis(2,2-dimethylpropionate) (Compound 25),**

**(2R,3R,4R,5R)-2-(4-aminopyrrolo[2,1-f][1,2,4]triazin-7-yl)-5-((((S)-(4-tert-butyl phenoxy)(((isopropoxycarbonyl)oxy)methoxy)phosphoryl)oxy)methyl)-2-cyanotetrahydrofuran-3,4-diyl bis(2,2-dimethylpropionate) (Compound 25a) and**

**((2R,3R,4R,5R)-2-(4-aminopyrrolo[2,1-f][1,2,4]triazin-7-yl)-5-((((R)-(4-tert-butyl phenoxy)(((isopropoxycarbonyl)oxy)methoxy)phosphoryl)oxy)methyl)-2-cyanotetrahydrofuran-3,4-diyl bis(2,2-dimethylpropionate) (Compound 25b)**

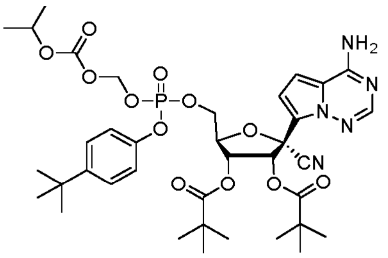

25

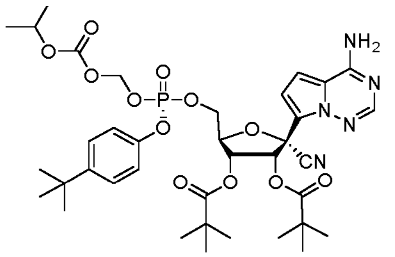

25a

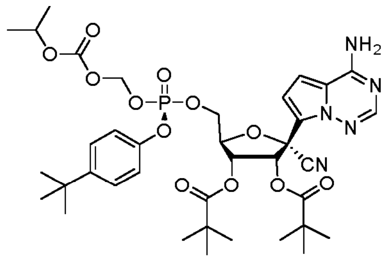

25b

The following synthetic routes were adopted:

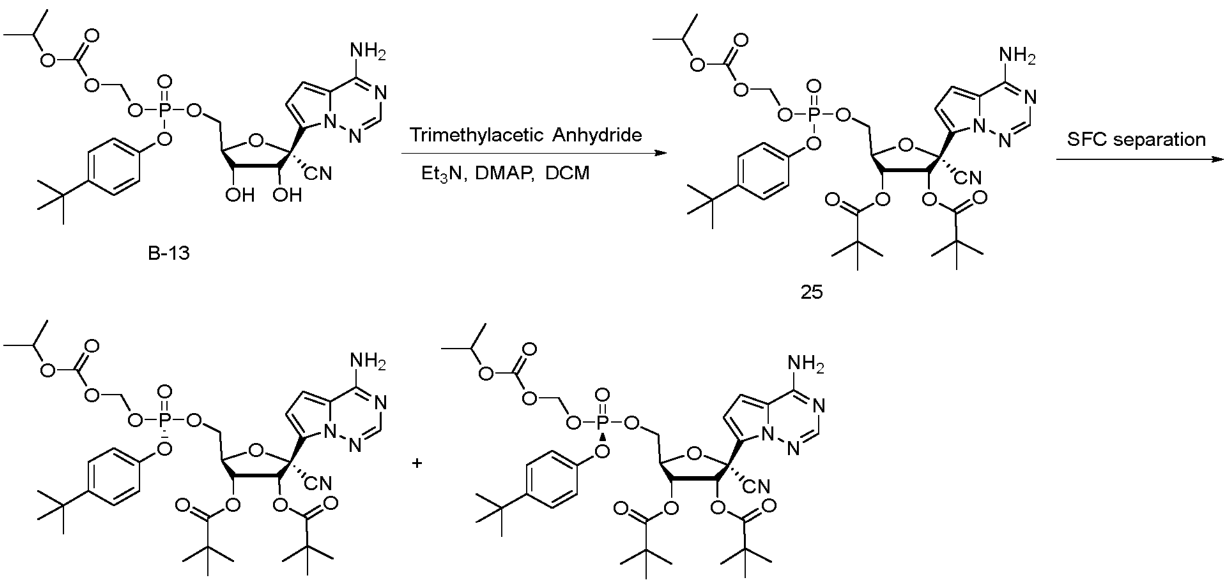


B-13

25

+

25a

25b

Step 1 Synthesis of Compound 25

The intermediate B-13 (192 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 93 mg of the target product (yield: 38%). LC-MS (APCI): m/z=788.1 $(M+1)^+$. $^1H$ NMR (400 MHz, DMSO-$d_6$) δ 7.91 (d, *J* = 4.3 Hz, 1H), 7.24 (t, *J* = 8.9 Hz, 2H), 6.98 (t, *J* = 7.6 Hz, 2H), 6.92 (dd, *J* = 9.2, 4.7 Hz, 1H), 6.74 (t, *J* = 4.7 Hz, 1H), 6.06 (dd, *J* = 11.5, 5.8 Hz, 1H), 5.68 - 5.57 (m, 2H), 5.41 (ddd, *J* = 8.7, 5.8, 3.2 Hz, 1H), 4.84 - 4.74 (m, 1H), 4.58 (s, 1H), 4.52 - 4.35 (m, 2H), 1.26 - 1.19 (m, 24H), 1.15 (d, *J* = 0.7 Hz, 9H).

Step 2 Synthesis of Compound 25a and Compound 25b

The racemate compound 25 was separated with supercritical fluid chromatography to obtain the target product 25a (retention time: 1.69 min, relative content: 54.0%) and 25b (retention time: 1.85 min, relative content: 46.0%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 26: Preparation of (2R,3R,4R,5R)-2-(4-aminopyrrolo[2,1-f][1,2,4]triazin-7-yl)-5-((((4-tert-butyl phenoxy)((isobutyryloxy)methoxy)phosphoryl)oxy)methyl)-2-cyanotetrahydrofuran-3,4-diyl bis(2-methylpropionate) (Compound 26),**

**(2R,3R,4R,5R)-2-(4-aminopyrrolo[2,1-f][1,2,4]triazin-7-yl)-5-((((S)-(4-tert-butyl phenoxy)((isobutyryloxy)methoxy)phosphoryl)oxy)methyl)-2-cyanotetrahydrofuran-3,4-diyl bis(2-methylpropionate) (Compound 26a) and**

**(2R,3R,4R,5R)-2-(4-aminopyrrolo[2,1-f][1,2,4]triazin-7-yl)-5-((((R)-(4-tert-butyl phenoxy)((isobutyryloxy)methoxy)phosphoryl)oxy)methyl)-2-cyanotetrahydrofuran-3,4-diyl bis(2-methylpropionate) (Compound 26b)**

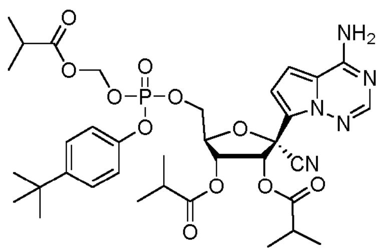

26

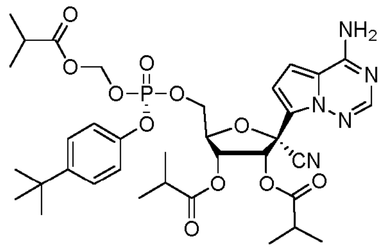

26a

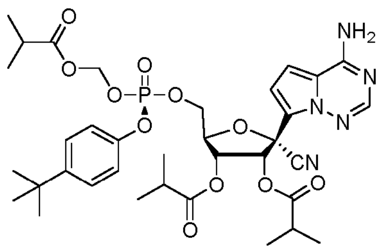

26b

The following synthetic routes were adopted:

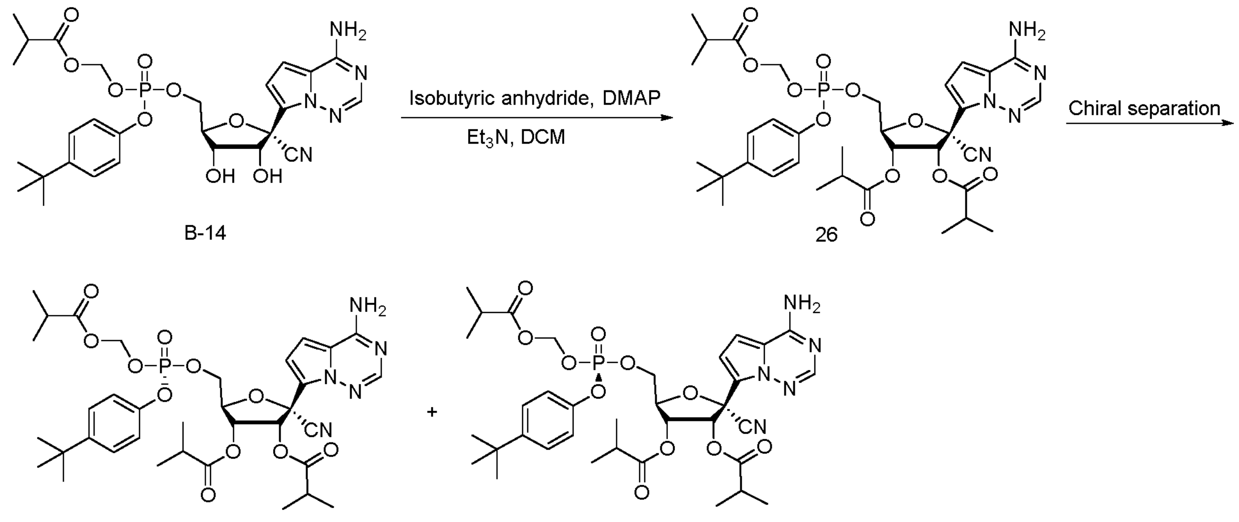


B-14 26

26a + 26b

Step 1 Synthesis of Compound 26

The intermediate B-14 (187 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and 4-dimethylaminopyridine (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 113 mg of the target product (yield: 49%). LC-MS (APCI): m/z=744.4 $(M+1)^+$. $^1$H NMR (400 MHz, DMSO-$d_6$) $\delta$ 7.90 (s, 1H), 7.33 (dd, $J$ = 13.4, 8.6 Hz, 2H), 7.11 - 7.05 (m, 2H), 6.92 (t, $J$ = 4.6 Hz, 1H), 6.84 - 6.76 (m, 1H), 6.34 (dd, $J$ = 6.0, 3.4 Hz, 1H), 5.62 (dd, $J$ = 14.1, 5.3 Hz, 2H), 5.44 (t, $J$ = 5.3 Hz, 1H), 4.69 (dd, $J$ = 8.8, 5.4 Hz, 1H), 4.44 - 4.34 (m, 1H), 4.27 (t, $J$ = 6.4 Hz, 2H), 3.96 (dd, $J$ = 9.7, 5.0 Hz, 1H), 2.01 (m, 2H), 1.22 (m, 21H), 1.01 (t, $J$ = 7.4 Hz, 6H).

Step 2 Synthesis of Compound 26a and Compound 26b

The racemate compound 26 was separated with supercritical fluid chromatography to obtain the target products 26a and 26b.

**Example 27: Preparation of (2R,3R,4R,5R)-2-(4-aminopyrrolo[2,1-f][1,2,4]triazin-7-yl)-5-((((4-tert-butyl phenoxy)(2-(pivaloylthio)ethoxy)phosphoryl)oxy)methyl)-2-cyanotetrahydrofuran-3,4-diyl bis(2-methylpropionate) (Compound 27),**

**(2R,3R,4R,5R)-2-(4-aminopyrrolo[2,1-f][1,2,4]triazin-7-yl)-5-((((R)-(4-tert-butyl phenoxy)(2-(pivaloylthio)ethoxy) phosphoryl)oxy)methyl)-2-cyanotetrahydrofuran-3,4-diyl bis(2-methylpropionate) (Compound 27a) and**

**(2R,3R,4R,5R)-2-(4-aminopyrrolo[2,1-f][1,2,4]triazin-7-yl)-5-((((S)-(4-tert-butyl phenoxy)(2-(pivaloylthio)ethoxy) phosphoryl)oxy)methyl)-2-cyanotetrahydrofuran-3,4-diyl bis(2-methylpropionate) (Compound 27b)**

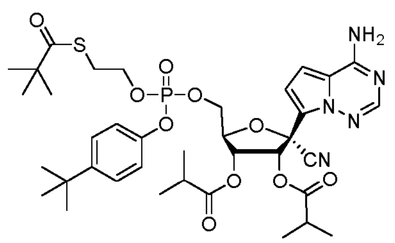
27

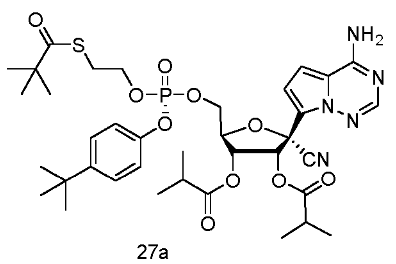
27a

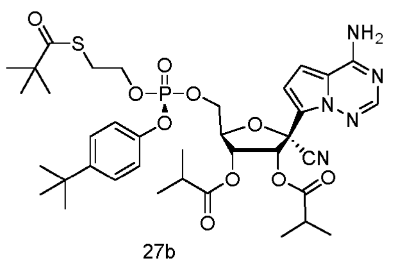
27b

The following synthetic routes were adopted:

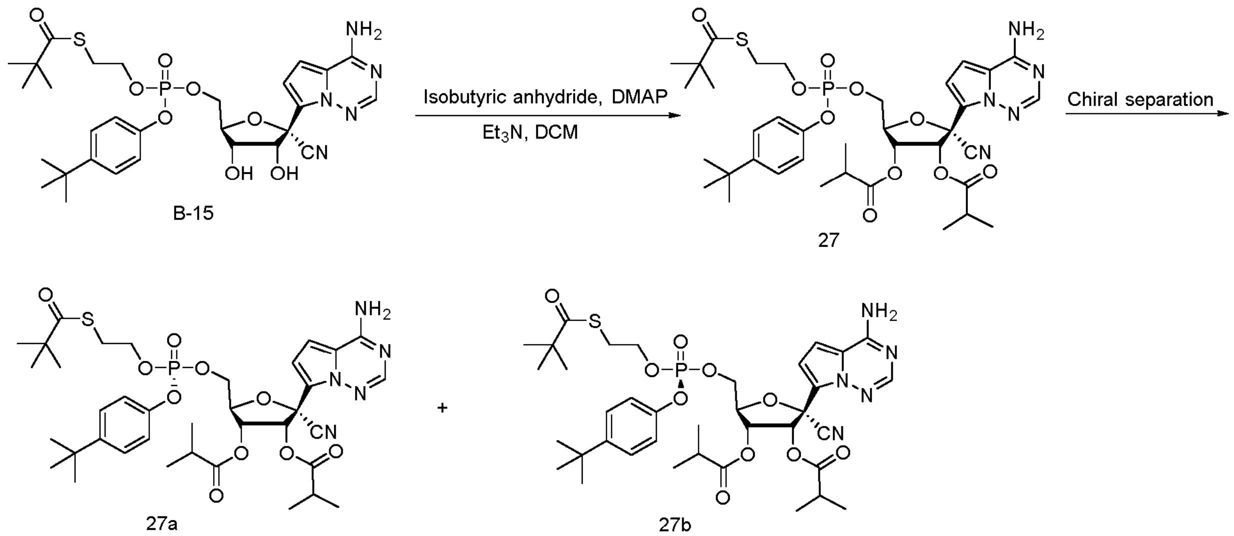


B-15 27 27a + 27b

Step 1 Synthesis of Compound 27

The intermediate B-4 (201 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-15 times. The organic layers were combined, washed with saturated saline solution, concentrated, and purified by silica gel column chromatography to obtain 178 mg of the target product (yield: 73%). LC-MS (APCI): m/z=788.4 $(M+1)^+$. $^1H$ NMR (400 MHz, DMSO-$d_6$) $\delta$ 7.92 (d, $J$ = 3.2 Hz, 1H), 7.26 (dd, $J$ = 8.1, 5.9 Hz, 2H), 7.02 (t, $J$ = 8.2 Hz, 2H), 6.94 (dd, $J$ = 7.1, 4.6 Hz, 1H), 6.76 (d, $J$ = 4.2 Hz, 1H), 6.06 (d, $J$ = 5.6 Hz, 1H), 5.46 (dd, $J$ = 9.6, 6.5 Hz, 1H), 4.63 (s, 1H), 4.42 (s, 2H), 4.07 (dd, $J$ = 12.8, 6.2 Hz, 2H), 3.05 (d, $J$ = 5.1 Hz, 2H), 2.60 (tt, $J$ = 14.0, 6.9 Hz, 2H), 1.33 - 1.04 (m, 30H).

Step 2 Synthesis of Compound 27a and Compound 27b

The racemate compound 27 was separated with supercritical fluid chromatography to obtain the target products 27a and 27b.

**Example 28: Preparation of (2R,3R,4R,5R)-2-(4-aminopyrrolo[2,1-f][1,2,4]triazin-7-yl)-5-((((bis(((isopropoxycarbonyl)oxy)methoxy))phosphoryl)oxy)methyl)-2-cyanotetrahydrofuran-3,4-diyldiisopropyl bis(carbonate) (Compound 28)**

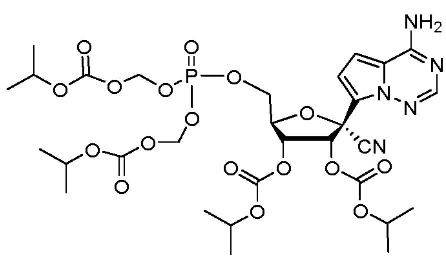

28

The following route was adopted for synthesis:

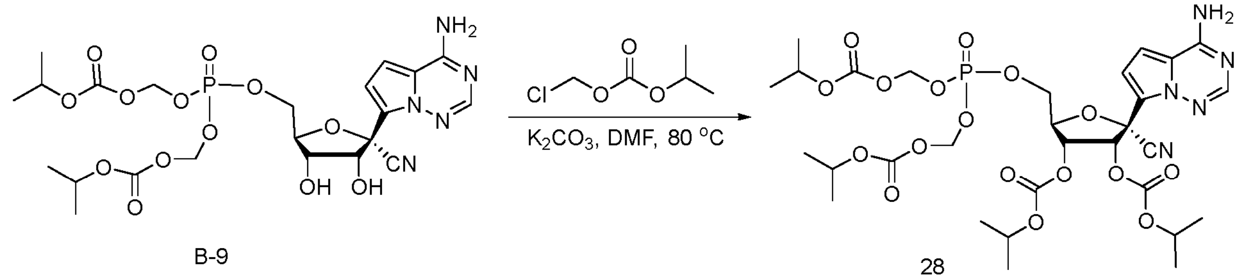


B-9

28

The intermediate B-9 (500 mg, 0.83 mmol) was added to a reaction flask, followed by the addition of 8 mL of anhydrous DMF to dissolve. Isopropyl chloromethyl carbonate (441 mg, 2.9 mmol) and potassium carbonate (459 mg, 3.32 mmol) were added successively. After the addition, the mixture was heated to 80°C under nitrogen atmosphere and stirred overnight. After completion of the reaction as monitored by TLC, the mixture was quenched with excess water and extracted with ethyl acetate 3-4 times. The organic layers were combined, washed with saturated saline solution, concentrated, and purified by silica gel column chromatography to obtain 90 mg of white solid (yield: 14%). LC-MS (APCI): m/z=776.7 $(M+1)^+$. $^1H$ NMR (400 MHz, DMSO-*d*$_6$) δ 7.97 (s, 1H), 6.99 (d, *J* = 4.6 Hz, 1H), 6.82 (d, *J* = 4.6 Hz, 1H), 6.05 (d, *J* = 5.8 Hz, 1H), 5.58 - 5.49 (m, 4H), 5.42 (dd, *J* = 5.7, 4.1 Hz, 1H), 4.81 (dt, *J* = 12.5, 6.2 Hz, 2H), 4.60 (d, *J* = 3.7 Hz, 1H), 4.34 (t, *J* = 5.9 Hz, 2H), 4.21 (m, 2H), 1.23 (d, *J* = 6.1 Hz, 12H), 1.15 (dd, *J* = 8.3, 7.1 Hz, 6H), 1.09 (d, *J* = 7.0 Hz, 6H).

**Example 29: Preparation of (2R,3R,4R,5R)-2-(4-aminopyrrolo[2,1-f][1,2,4]triazin-7-yl)-5-((((bis(((tert-butylcarbonyl)oxy)methoxy))phosphoryl)oxy)methyl)-2-cyanotetrahydrofuran-3,4-diyl bis(2-methylpropionate) (Compound 29)**

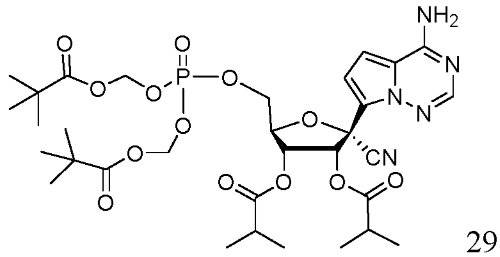

29

The following synthetic routes were adopted:

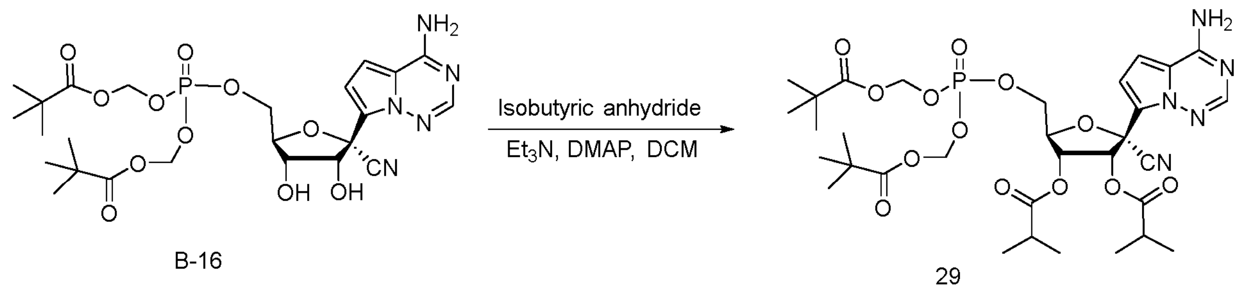


B-16

29

The intermediate B-4 (186 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-16 times. The organic layers were combined, washed with saturated saline solution, concentrated, and purified by silica gel column chromatography to obtain 147 mg of the target product (yield: 64%). LC-MS (APCI): m/z=740.7 $(M+1)^+$. $^1H$ NMR (400 MHz, DMSO-*d*$_6$) δ 8.04 (s, 1H), 7.07 (d, *J*= *4.5* Hz, 1H), 6.87 (d, *J*= *4.5* Hz, 1H), 5.57 (d, *J* = 13.6 Hz, 4H), 4.82 (td, *J* = 12.2, 6.1 Hz, 2H), 4.65 (d, *J* = 4.7 Hz, 1H), 4.30 (m, 1H), 4.11 (m, 1H), 2.05 (m, 2H), 1.24 - 0.87 (d, *J* = 6.4 Hz, 30H).

**Example 30: Preparation of (2R,3R,4R,5R)-2-(4-aminopyrrolo[2,1-f][1,2,4]triazin-7-yl)-5-((((bis(2-(pivaloylthio)ethoxy))phosphoryl)oxy)methyl)-2-cyanotetrahydrofuran-3,4-diyl bis(2-methylpropionate) (Compound 30)**

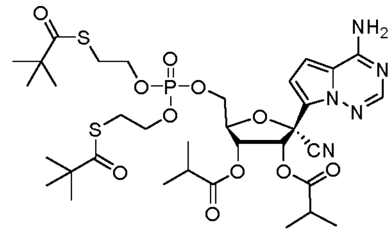

30

The following synthetic routes were adopted:

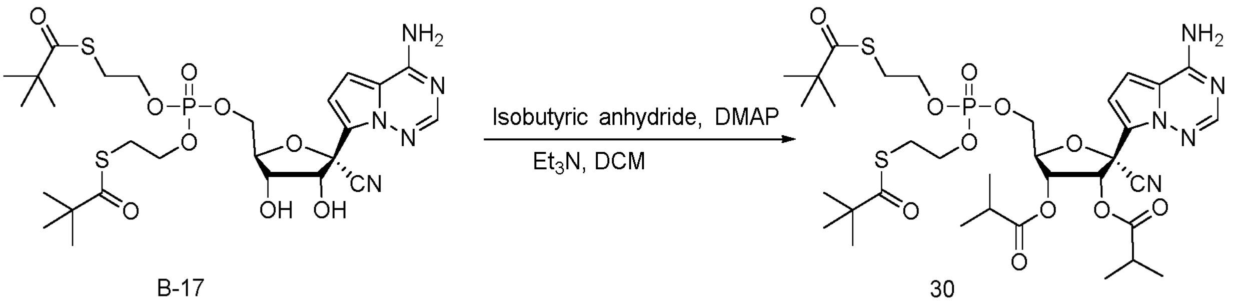


B-17 30

The intermediate B-4 (204 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-17 times. The organic layers were combined, washed with saturated saline solution, concentrated, and purified by silica gel column chromatography to obtain 168 mg of the target product (yield: 68%). LC-MS (APCI): m/z=800.7 $(M+1)^+$. $^1H$ NMR (400 MHz, $CDCl_3$) δ 7.97 (s, 1H), 6.90 (d, *J* = 4.6 Hz, 1H), 6.67 (d, *J* = 4.6 Hz, 1H), 5.88 (s, 2H), 4.64 (dd, *J* = 9.6, 4.3 Hz, 2H), 4.42 (d, *J* = 4.1 Hz, 2H), 4.37 (dd, *J* = 7.7, 3.6 Hz, 1H), 4.30 - 4.20 (m, 4H), 3.17 - 3.02 (m, 4H), 2.03 (m, 2H), 1.19 -0.89 (m, 30H).

**Example 31: Preparation of (2R,3R,4R,5R)-2-(4-aminopyrrolo[2,1-f][1,2,4]triazin-7-yl)-2-cyano-5-((((((S)-1-(2-ethylbutoxy)-1-oxo-3-phenylpropan-2-yl)amino)(((isopropoxycarbonyl)oxy)methoxy)phosphoryl)oxy)methyl)tetrahydrofuran-3,4-diyl bis(2-methylpropionate) (Compound 31),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxo-3-phenylpropan-2-yl)amino)(((isopropoxycarbonyl)oxy)methoxy)phosphoryl)oxy)methyl)tetrahydrofuran-3,4-diyl bis(2-methylpropionate) (Compound 31a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxo-3-phenylpropan-2-yl)amino)(((isopropoxycarbonyl)oxy)methoxy)phosphoryl)oxy)methyl)tetrahydrofuran-3,4-diyl bis(2-methylpropionate) (Compound 31b)**

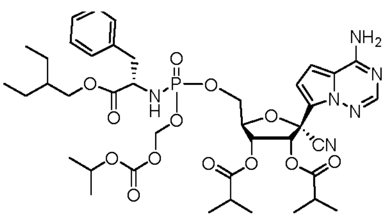

31

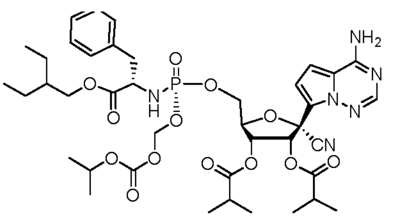

31a

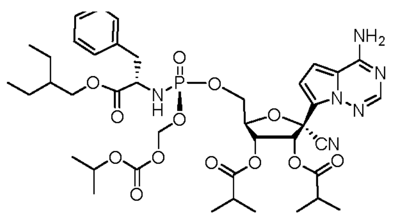

31b

The following synthetic routes were adopted:

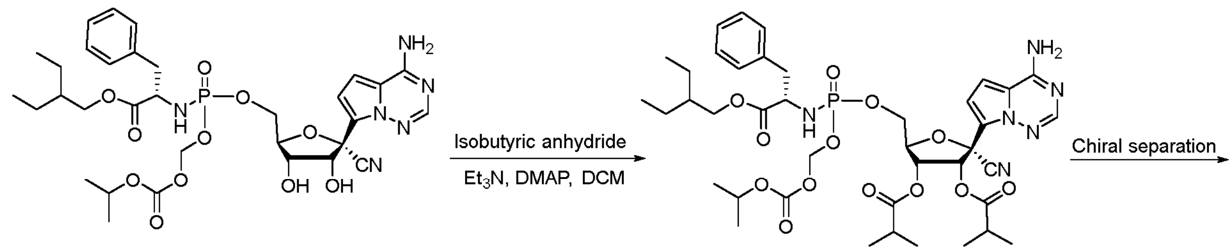


B-18

31

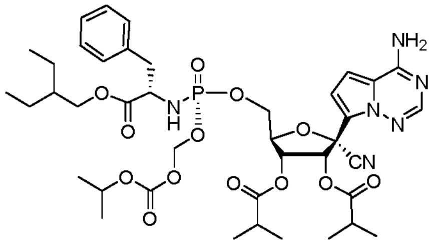

\+

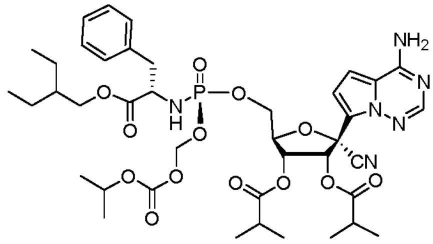

31a

31b

Step 1 Synthesis of Compound 31

The intermediate B-4 (222 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-18 times. The organic layers were combined, washed with saturated saline solution, concentrated, and purified by silica gel column chromatography to obtain 85 mg of the target product (yield: 32%). LC-MS (APCI): m/z=859.5 $(M+1)^+$. $^1H$ NMR (400 MHz, $CDCl_3$) δ 8.00 (s, 1H), 7.25 - 7.20 (m, 2H), 7.09 (d, *J* = 6.9 Hz, 2H), 6.93 (d, *J* = 4.6 Hz, 1H), 6.56 (d, *J* = 4.6 Hz, 1H), 6.22 (d, *J* = 5.8 Hz, 1H), 5.69 (s, 2H), 5.55 - 5.49 (m, 1H), 5.48 - 5.37 (m, 2H), 4.89 (dt, *J* = 12.4, 6.2 Hz, 1H), 4.53 (d, *J* = 3.7 Hz, 1H), 4.21 (ddt, *J* = 19.3, 15.1, 7.9 Hz, 3H), 3.97 (d, *J* = 5.5 Hz, 2H), 3.54 (t, *J* = 10.7 Hz, 1H), 2.94 (qd, *J* = 13.7, 6.2 Hz, 2H), 2.63 (ddt, *J* = 20.9, 14.0, 7.0 Hz, 2H), 1.44 (dt, *J* = 12.1, 6.1 Hz, 1H), 1.23 (ddd, *J* = 24.4, 21.1, 6.6 Hz, 22H), 0.84 (t, *J* = 7.4 Hz, 6H).

Step 2 Synthesis of Compound 31a and Compound 31b

The racemate compound 31 was separated with reverse phase preparative chromatography to obtain the target product 31a (retention time: 11.21 min, relative content: 50.4%) and 31b (retention time: 12.31 min, relative content: 49.6%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 32: Preparation of (2R,3R,4R,5R)-2-(4-aminopyrrolo[2,1-f][1,2,4]triazin-7-yl)-2-cyano-5-1-((2-ethylbutoxy)carbonyl)cyclobutyl)amino)(((isopropoxycarbonyl)oxy)methoxy)phosphoryl) oxy)methyl)tetrahydrofuran-3,4-diyl bis(2-methylpropionate) (Compound 32),**

**(2R,3R,4R,5R)-2-(4-aminopyrrolo[2,1-f][1,2,4]triazin-7-yl)-2-cyano-5-((((R)-((1-((2-ethylbutoxy)carbonyl)cyclobutyl)amino)(((isopropoxycarbonyl)oxy)methoxy)phosphoryl) oxy)methyl)tetrahydrofuran-3,4-diyl bis(2-methylpropionate) (Compound 32a) and**

**(2R,3R,4R,5R)-2-(4-aminopyrrolo[2,1-f][1,2,4]triazin-7-yl)-2-cyano-5-((((S)-((1-((2-ethylbutoxy)carbonyl)cyclobutyl)amino)(((isopropoxycarbonyl)oxy)methoxy)phosphoryl) oxy)methyl)tetrahydrofuran-3,4-diyl bis(2-methylpropionate) (Compound 32b)**

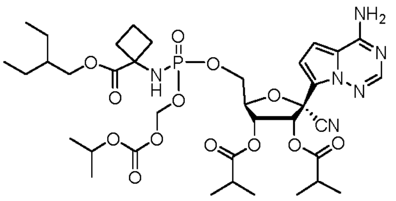

32

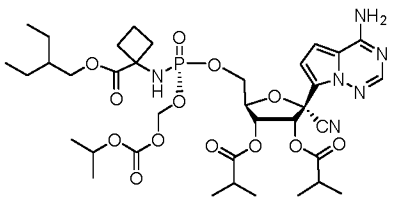

32a

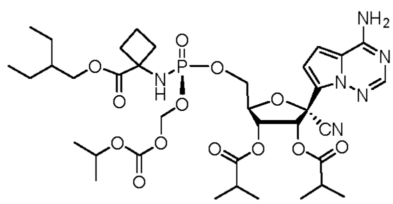

32b

The following synthetic routes were adopted:

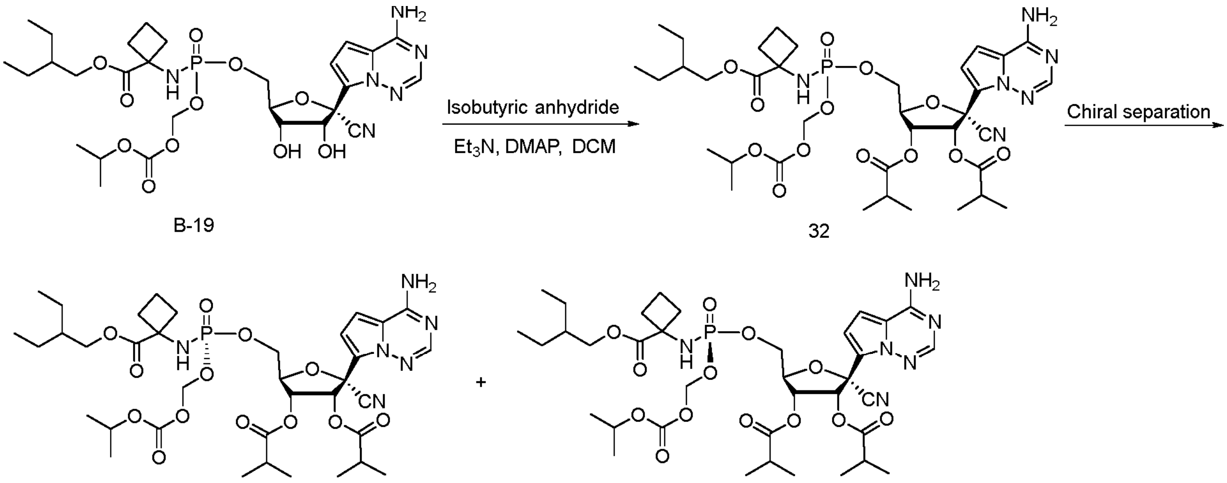


B-19

32

+

32a

32b

Step 1 Synthesis of Compound 32

The intermediate B-19 (207 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 105 mg of the target product (yield: 42%). LC-MS (APCI): m/z=809.7 $(M+1)^+$. $^1H$ NMR (400 MHz, $CDCl_3$) δ 7.92 (s, 1H), 6.87 (s, 1H), 6.65 (d, *J* = 3.2 Hz, 1H), 6.01 (s, 2H), 5.67 - 5.53 (m, 2H), 5.47 (dd, *J* = 12.2, 5.3 Hz, 1H), 4.95 - 4.81 (m, 1H), 4.67 (d, *J* = 5.4 Hz, 1H), 4.56 (s, 1H), 4.40 (s, 1H), 4.28 (d, *J* = 17.5 Hz, 2H), 4.21 - 4.13 (m, 1H), 4.09 (d, *J= 5.3* Hz, 2H), 3.94 (s, 1H), 2.45 (s, 4H), 2.04 - 1.84 (m, 4H), 1.59 - 1.52 (m, 1H), 1.34 (ddd, *J* = 23.0, 10.0, 5.0 Hz, 22H), 0.90 (t, *J* = 7.4 Hz, 6H).

Step 2 Synthesis of Compound 32a and Compound 32b

The racemate compound 32 was separated with reverse phase preparative chromatography to obtain the target product 32a (retention time: 11.33 min, relative content: 51.4%) and 32b (retention time: 12.01 min, relative content: 48.6%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm

Mobile phase: pure water:acetonitrile=40:60

**Example 33: Preparation of (2R,3R,4R,5R)-2-(4-aminopyrrolo[2,1-f][1,2,4]triazin-7-yl)-2-cyano-5-((((((S)-1,4-dipentyloxy-1,4-dioxobutan-2-yl)amino)(((isopropoxycarbonyl)oxy)methoxy)phosphoryl)oxy)methyl)tetrahydrofuran-3,4-diyl bis(2-methylpropionate) (Compound 33),**

**(2R,3R,4R,5R)-2-(4-aminopyrrolo[2,1-f][1,2,4]triazin-7-yl)-2-cyano-5-((((R)-(((S)-1,4-dipentyloxy-1,4-dioxobutan-2-yl)amino)(((isopropoxycarbonyl)oxy)methoxy)phosphoryl)oxy)methyl)tetrahydrofuran-3,4-diyl bis(2-methylpropionate) (Compound 33a) and**

**(2R,3R,4R,5R)-2-(4-aminopyrrolo[2,1-f][1,2,4]triazin-7-yl)-2-cyano-5-((((S)-(((S)-1,4-dipentyloxy-1,4-dioxobutan-2-yl)amino)(((isopropoxycarbonyl)oxy)methoxy)phosphoryl)oxy)methyl)tetrahydrofuran-3,4-diyl bis(2-methylpropionate) (Compound 33b)**

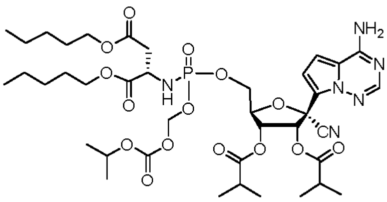

33

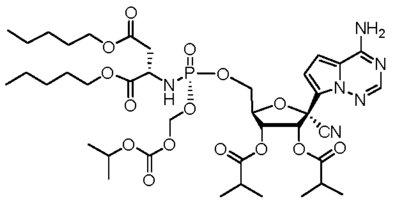

33a

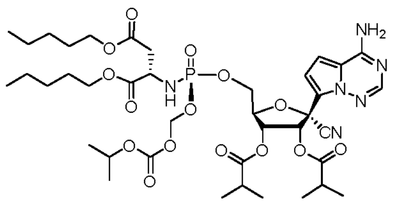

33b

The following synthetic routes were adopted:

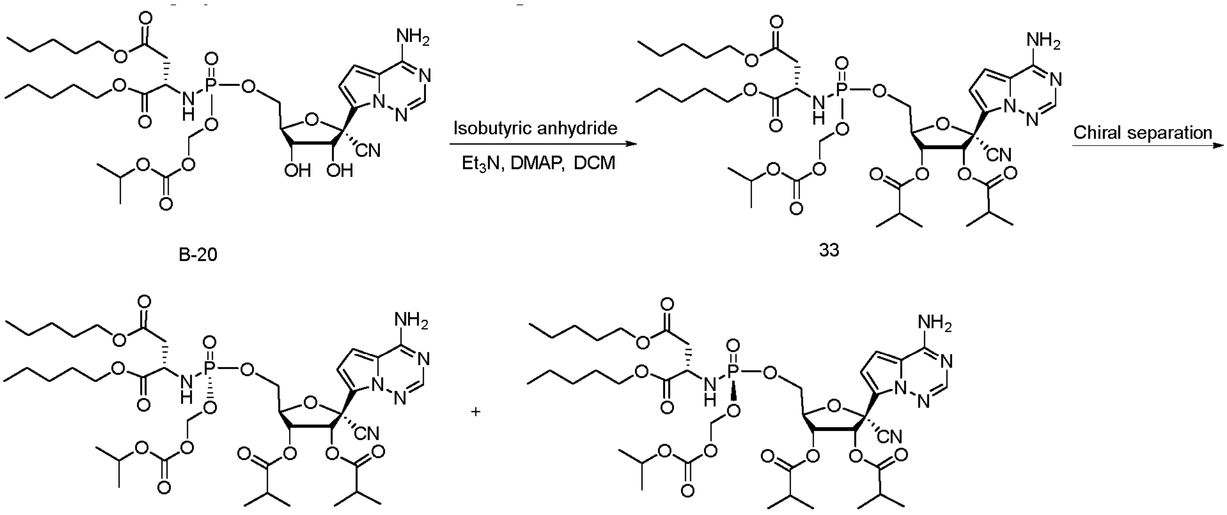


B-20

33

+

33a

33b

Step 1 Synthesis of Compound 33

The intermediate B-4 (230 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-20 times. The organic layers were combined, washed with saturated saline solution, concentrated, and purified by silica gel column chromatography to obtain 98 mg of the target product (yield: 36%). LC-MS (APCI): m/z=883.4 $(M+1)^+$. $^1H$ NMR (400 MHz, DMSO-$d_6$) δ 7.93 (s, 1H), 6.90 (d, J = 4.5 Hz, 1H), 6.80 (d, J = 4.5 Hz, 1H), 6.23 (s, 2H), 4.80 (dt, J = 12.5, 6.2 Hz, 2H), 4.61 (d, J = 3.5 Hz, 2H), 4.25 - 4.10 (m, 6H), 3.12 (dd, J = 10.8, 5.9 Hz, 1H), 3.01 (m, 2H), 2.05 (m, 2H), 1.67 (m, 4H), 1.48 (m, 8H), 1.01 - 0.89 (m, 24H).

Step 2 Synthesis of Compound 33a and 33b

The racemate compound 33 was separated with reverse phase preparative chromatography to obtain the target products 33a and 33b.

**Example 34: Preparation of (2R,3R,4R,5R)-2-(4-aminopyrrolo[2,1-f][1,2,4]triazin-7-yl)-2-cyano-5-((((3-(hexadecyloxy)propoxy)(((isopropoxycarbonyl)oxy)methoxy)phosphoryl)oxy)methyl)tetr ahydrofuran-3,4-diyl diacetate (Compound 34),**

**(2R,3R,4R,5R)-2-(4-aminopyrrolo[2,1-f][1,2,4]triazin-7-yl)-2-cyano-5-((((S)-(3-(hexadecyloxy)propoxy)(((isopropoxycarbonyl)oxy)methoxy)phosphoryl)oxy)methyl)tetr ahydrofuran-3,4-diyl diacetate (Compound 34a) and**

**(2R,3R,4R,5R)-2-(4-aminopyrrolo[2,1-f][1,2,4]triazin-7-yl)-2-cyano-5-((((R)-(3-(hexadecyloxy)propoxy)(((isopropoxycarbonyl)oxy)methoxy)phosphoryl)oxy)methyl)tetr ahydrofuran-3,4-diyl diacetate (Compound 34b)**

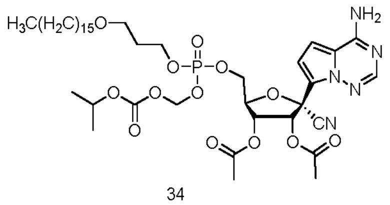

34

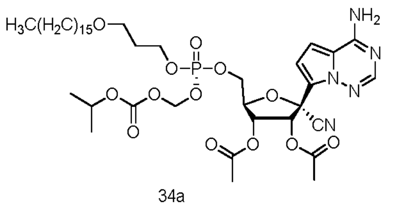

34a

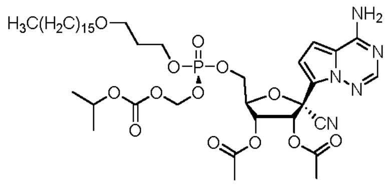

34b

The following synthetic routes were adopted:

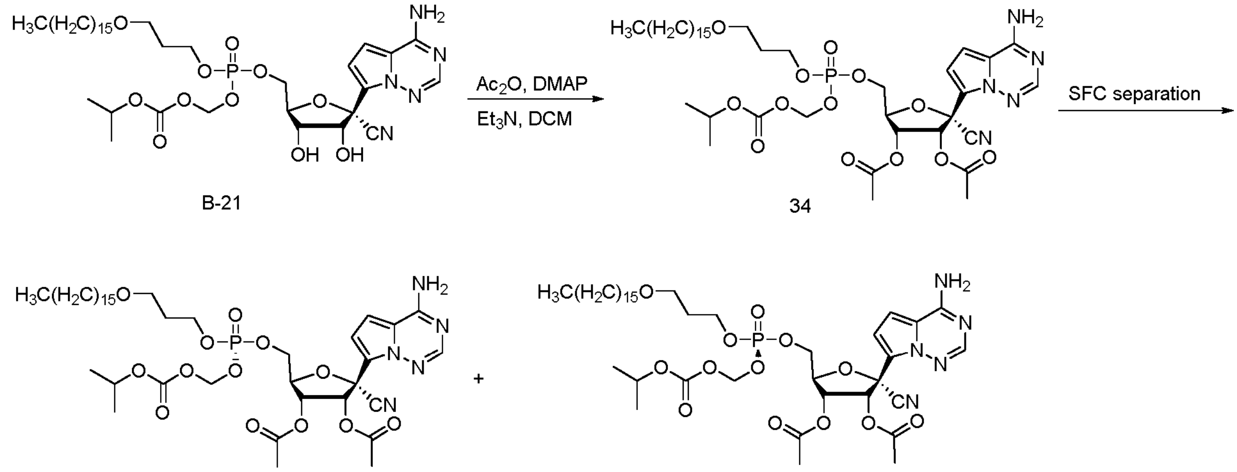


B-21 34 34a 34b

Step 1 Synthesis of Compound 34

The intermediate B-21 (238 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight.

After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 108 mg of the target product (yield: 41%). LC-MS (APCI): m/z=854.4 (M+1)$^+$. $^1$H NMR (400 MHz, DMSO-*d$_6$*) δ 7.92 (s, 1H), 6.90 (d, *J*= 4.4 Hz, 1H), 6.81 (d, *J*= *4.5* Hz, 1H), 6.35 (dd, *J* = 5.9, 3.3 Hz, 1H), 5.54 (d, *J*= 13.9 Hz, 2H), 5.41 (d, *J*= 5.5 Hz, 1H), 4.87 - 4.76 (m, 1H), 4.67 (t, *J* = 5.4 Hz, 1H), 4.30 - 4.18 (m, 2H), 4.17 - 4.07 (m, 1H), 4.00 (dd, *J* = 13.0, 6.5 Hz, 2H), 3.93 (d, *J* = 4.9 Hz, 1H), 3.31 - 3.25 (m, 2H), 2.07 (s, 6H), 1.77 (dd, *J* = 10.5, 5.9 Hz, 2H), 1.50 - 1.38 (m, 2H), 1.24 (d, *J* = 7.3 Hz, 34H), 0.85 (t, *J* = 6.6 Hz, 3H).

Step 2 Synthesis of Compound 34a and Compound 34b

The racemate compound 34 was separated with supercritical fluid chromatography to obtain the target product 34a (retention time: 1.85 min, relative content: 51.1%) and 34b (retention time: 1.96 min, relative content: 48.9%).

Conditions of chromatographic separation:

Column: CHIRALPAK, IH-3, 50×4.6 mm I.D., 3 μm
Column temperature: 35°C
Flow rate: 3.0 mL/min
Wavelength of UV detection: 220 nm
Mobile phase: carbon dioxide:ethanol (0.05% diethylamine)=80:20

**Example 35: Preparation of (2R,3R,4R,5R)-2-(4-aminopyrrolo[2,1-f][1,2,4]triazin-7-yl)-2-cyano-5-((((3-(hexadecyloxy)propoxy)(((isopropoxycarbonyl)oxy)methoxy)phosphoryl)oxy)methyl)tetr ahydrofuran-3,4-diyl dipropionate (Compound 35),**

**(2R,3R,4R,5R)-2-(4-aminopyrrolo[2,1-f][1,2,4]triazin-7-yl)-2-cyano-5-((((S)-(3-(hexadecyloxy)propoxy)(((isopropoxycarbonyl)oxy)methoxy)phosphoryl)oxy)methyl)tetr ahydrofuran-3,4-diyl dipropionate (Compound 35a) and**

**(2R,3R,4R,5R)-2-(4-aminopyrrolo[2,1-f][1,2,4]triazin-7-yl)-2-cyano-5-((((R)-(3-(hexadecyloxy)propoxy)(((isopropoxycarbonyl)oxy)methoxy)phosphoryl)oxy)methyl)tetr ahydrofuran-3,4-diyl dipropionate (Compound 35b)**

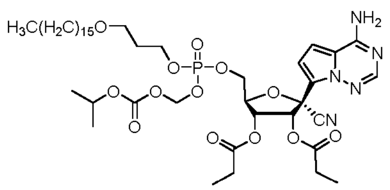

35

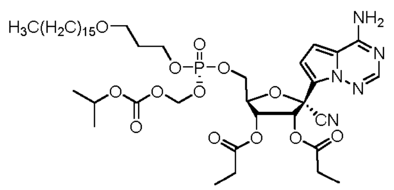

35a

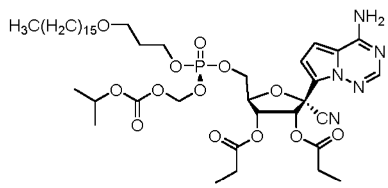

35b

The following synthetic routes were adopted:

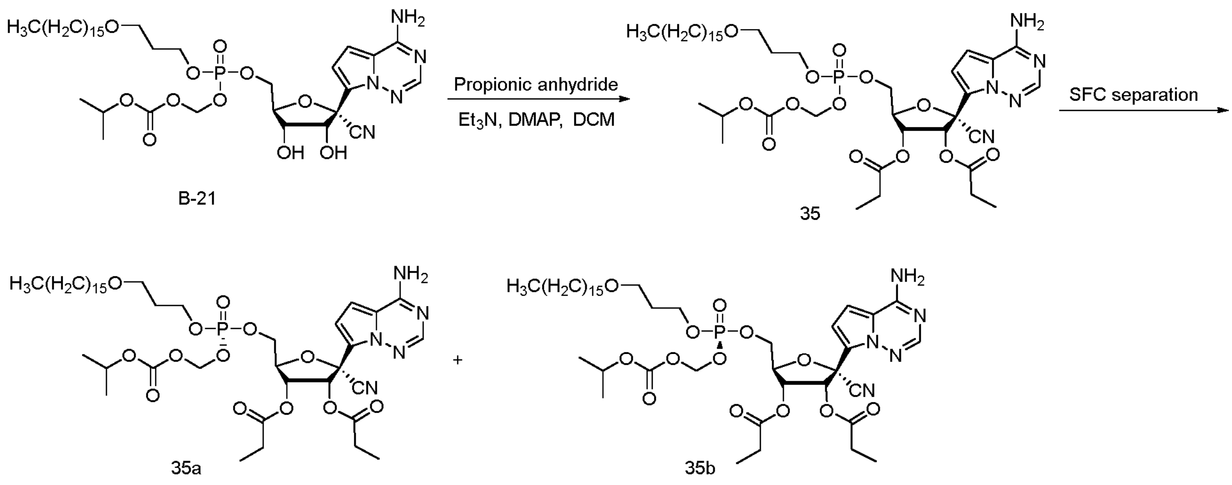


Step 1 Synthesis of Compound 35

The intermediate B-21 (238 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and 4-dimethylaminopyridine (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed

with saturated saline solution, concentrated, and purified by silica gel column chromatography to obtain 93 mg of the target product (yield: 34%). LC-MS (APCI): m/z=882.6 $(M+1)^+$. $^1H$ NMR (400 MHz, DMSO-$d_6$) δ 7.92 (s, 1H), 6.90 (d, $J$ = 4.4 Hz, 1H), 6.81 (d, $J$ = 4.5 Hz, 1H), 6.35 (dd, $J$ = 5.9, 3.3 Hz, 1H), 5.54 (d, $J$ = 13.9 Hz, 2H), 5.41 (d, $J$ = 5.5 Hz, 1H), 4.87 - 4.76 (m, 1H), 4.67 (t, $J$ = 5.4 Hz, 1H), 4.30 - 4.18 (m, 2H), 4.17 - 4.07 (m, 1H), 4.00 (dd, $J$ = 13.0, 6.5 Hz, 2H), 3.93 (d, $J$ = 4.9 Hz, 1H), 3.31 - 3.25 (m, 2H), 2.06 (m, 4H), 1.77 (dd, $J$ = 10.5, 5.9 Hz, 2H), 1.50 - 1.38 (m, 2H), 1.24 (m, 40H), 0.85 (t, $J$ = 6.6 Hz, 3H).

Step 2 Synthesis of Compound 35a and Compound 35b

The racemate compound 35 was separated with supercritical fluid chromatography to obtain the target product 35a (retention time: 1.88 min, relative content: 49.8%) and 35b (retention time: 2.01 min, relative content: 50.2%).
Conditions of chromatographic separation:

Column: CHIRALPAK, IH-3, 50×4.6 mm I.D., 3 μm
Column temperature: 35°C
Flow rate: 3.0 mL/min
Wavelength of UV detection: 220 nm
Mobile phase: carbon dioxide:ethanol (0.05% diethylamine)=80:20

**Example 36: Preparation of (2R,3R,4R,5R)-2-(4-aminopyrrolo[2,1-f][1,2,4]triazin-7-yl)-2-cyano-5-((((3-(hexadecyloxy)propoxy)(((isopropoxycarbonyl)oxy)methoxy)phosphoryl)oxy)methyl)tetr ahydrofuran-3,4-diyl bis(2-methylpropionate) (Compound 36),**

**(2R,3R,4R,5R)-2-(4-aminopyrrolo[2,1-f][1,2,4]triazin-7-yl)-2-cyano-5-((((S)-(3-(hexadecyloxy)propoxy)(((isopropoxycarbonyl)oxy)methoxy)phosphoryl)oxy)methyl)tetr ahydrofuran-3,4-diyl bis(2-methylpropionate) (Compound 36a) and**

**(2R,3R,4R,5R)-2-(4-aminopyrrolo[2,1-f][1,2,4]triazin-7-yl)-2-cyano-5-((((R)-(3-(hexadecyloxy)propoxy)(((isopropoxycarbonyl)oxy)methoxy)phosphoryl)oxy)methyl)tetr ahydrofuran-3,4-diyl bis(2-methylpropionate) (Compound 36b)**

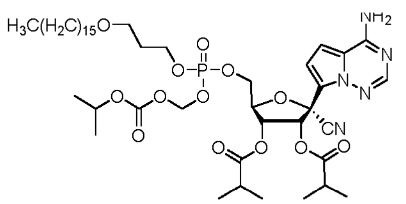
36

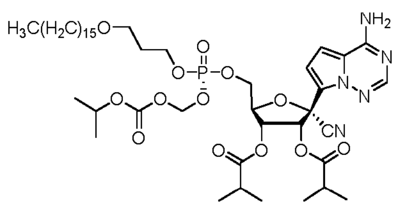
36a

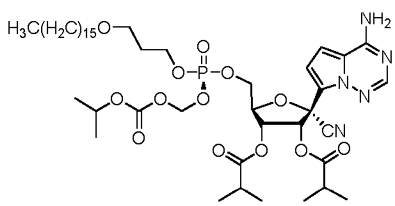
36b

The following synthetic routes were adopted:

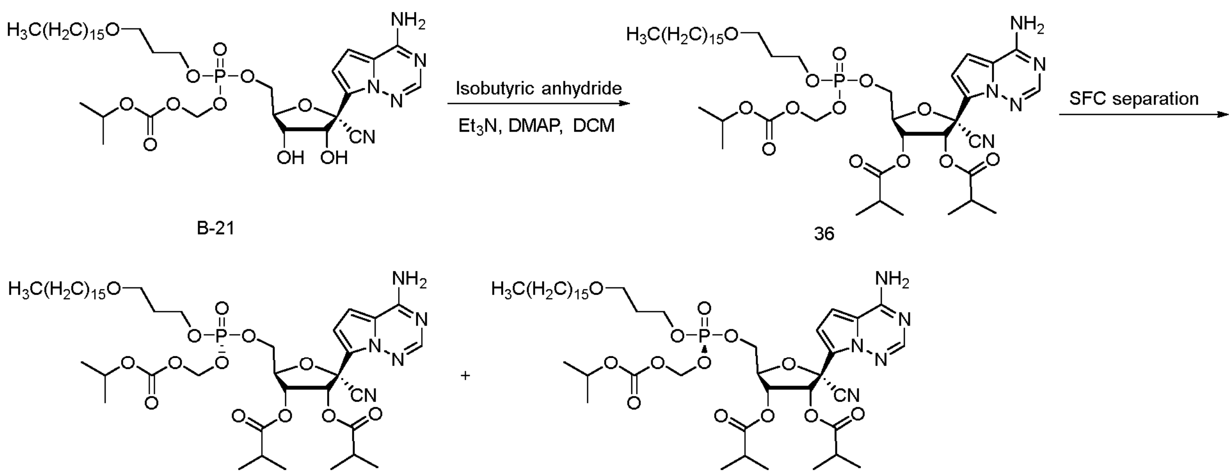

B-21 36 36a + 36b

Step 1 Synthesis of Compound 36

The intermediate B-4 (238 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-21 times. The organic layers were combined, washed with saturated saline solution, concentrated, and purified by silica gel column chromatography to obtain 82 mg of the target product (yield: 29%). LC-MS (APCI): m/z=910.1 $(M+1)^+$. $^1H$ NMR (400 MHz, DMSO-$d_6$) δ 7.92 (s, 1H), 6.90 (d, *J*= 4.4 Hz, 1H), 6.81 (d, *J*= *4.5* Hz, 1H), 6.35 (dd, *J* = 5.9, 3.3 Hz, 1H), 5.54 (d, *J*= 13.9 Hz, 2H), 5.41 (d, *J*= 5.5 Hz, 1H), 4.87 - 4.76 (m, 1H), 4.67 (t, *J* = 5.4 Hz, 1H), 4.30 - 4.18 (m, 2H), 4.17 - 4.07 (m, 1H), 4.00 (dd, *J* = 13.0, 6.5 Hz, 2H), 3.93 (d, *J* = 4.9 Hz, 1H), 3.31 - 3.25 (m, 2H), 2.03 (m, 2H), 1.77 (dd, *J* = 10.5, 5.9 Hz, 2H), 1.50 - 1.38 (m, 2H), 1.24 (m, 46H), 0.85 (t, *J* = 6.6 Hz, 3H).

Step 2 Synthesis of Compound 36a and Compound 36b

The racemate compound 36 was separated with supercritical fluid chromatography to obtain the target product 36a (retention time: 1.80 min, relative content: 52.2%) and 36b (retention time: 1.93 min, relative content: 47.8%).

Conditions of chromatographic separation:

Column: CHIRALPAK, IH-3, 50×4.6 mm I.D., 3 μm
Column temperature: 35°C
Flow rate: 3.0 mL/min
Wavelength of UV detection: 220 nm
Mobile phase: carbon dioxide:ethanol (0.05% diethylamine)=80:20

**Example 37: Preparation of (2R,3R,4R,5R)-2-(4-aminopyrrolo[2,1-f][1,2,4]triazin-7-yl)-2-cyano-5-((((3-(hexadecyloxy)propoxy)(((isopropoxycarbonyl)oxy)methoxy)phosphoryl)oxy)methyl)tetr ahydrofuran-3,4-diyl bis(2,2-dimethylpropionate) (Compound 37),**

**(2R,3R,4R,5R)-2-(4-aminopyrrolo[2,1-f][1,2,4]triazin-7-yl)-2-cyano-5-((((S)-(3-(hexadecyloxy)propoxy)(((isopropoxycarbonyl)oxy)methoxy)phosphoryl)oxy)methyl)tetr ahydrofuran-3,4-diyl bis(2,2-dimethylpropionate) (Compound 37a) and**

**(2R,3R,4R,5R)-2-(4-aminopyrrolo[2,1-f][1,2,4]triazin-7-yl)-2-cyano-5-((((R)-(3-(hexadecyloxy)propoxy)(((isopropoxycarbonyl)oxy)methoxy)phosphoryl)oxy)methyl)tetr ahydrofuran-3,4-diyl bis(2,2-dimethylpropionate) (Compound 37b)**

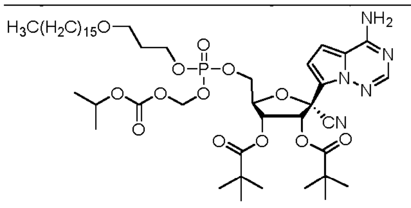
37

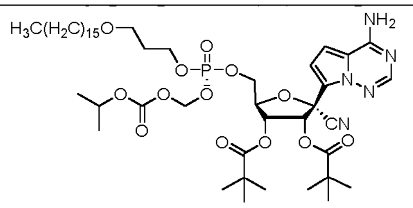
37a

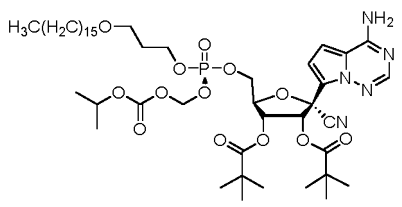
37b

The following synthetic routes were adopted:

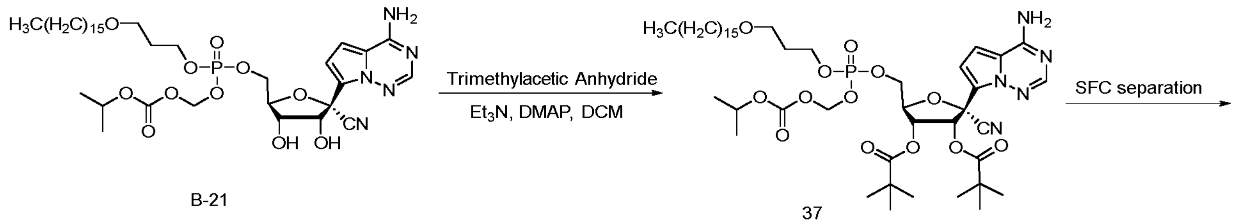


B-21

37

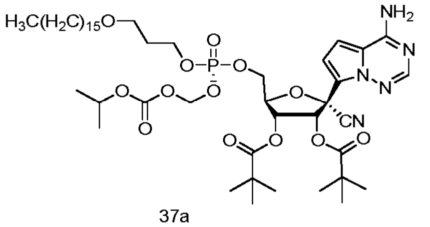

37a

+

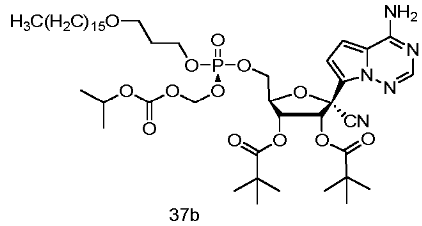

37b

Step 1 Synthesis of Compound 37

The intermediate B-21 (238 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 67 mg of the target product (yield: 23%). LC-MS (APCI): m/z=938.4 $(M+1)^+$. $^1H$ NMR (400 MHz, DMSO-$d_6$) δ 7.92 (s, 1H), 6.90 (d, *J*= 4.4 Hz, 1H), 6.81 (d, *J*= *4.5* Hz, 1H), 6.35 (dd, *J* = 5.9, 3.3 Hz, 1H), 5.54 (d, *J*= 13.9 Hz, 2H), 5.41 (d, *J*= 5.5 Hz, 1H), 4.87 - 4.76 (m, 1H), 4.67 (t, *J* = 5.4 Hz, 1H), 4.30 - 4.18 (m, 2H), 4.17 - 4.07 (m, 1H), 4.00 (dd, *J* = 13.0, 6.5 Hz, 2H), 3.93 (d, *J* = 4.9 Hz, 1H), 3.31 - 3.25 (m, 2H), 1.77 (dd, *J* = 10.5, 5.9 Hz, 2H), 1.50 - 1.38 (m, 2H), 1.24 - 1.12 (m, 52H), 0.85 (t, *J* = 6.6 Hz, 3H).

Step 2 Synthesis of Compound 37a and Compound 37b

The racemate compound 37 was separated with supercritical fluid chromatography to obtain the target product 37a (retention time: 1.63 min, relative content: 51.1%) and 37b (retention time: 1.89 min, relative content: 48.9%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:ethanol (0.05% diethylamine)=80:20

**<u>Example 38: Preparation of (2R,3R,4R,5R)-2-(4-aminopyrrolo[2,1-f][1,2,4]triazin-7-yl)-2-cyano-5-((((((S)-1,4-dipentyloxy-1,4-dioxobutan-2-yl)amino)(phenoxy)phosphoryl)oxy)methyl)tetrahydrofuran-3,4-diyl bis(2-methylpropionate) (Compound 38),</u>**

**<u>(2R,3R,4R,5R)-2-(4-aminopyrrolo[2,1-f][1,2,4]triazin-7-yl)-2-cyano-5-((((R)-(((S)-1,4-dipentyloxy-1,4-dioxobutan-2-yl)amino)(phenoxy)phosphoryl)oxy)methyl)tetrahydrofuran-3,4-diyl bis(2-methylpropionate) (Compound 38a) and</u>**

**<u>(2R,3R,4R,5R)-2-(4-aminopyrrolo[2,1-f][1,2,4]triazin-7-yl)-2-cyano-5-((((S)-(((S)-1,4-dipentyloxy-1,4-dioxobutan-2-yl)amino)(phenoxy)phosphoryl)oxy)methyl)tetrahydrofuran-3,4-diyl bis(2-methylpropionate) (Compound 38b)</u>**

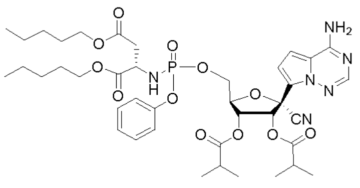

38

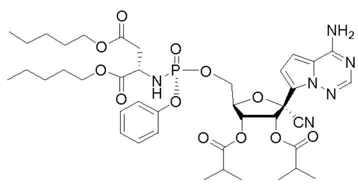

38a

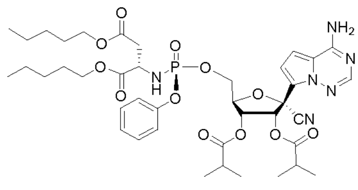

38b

The following synthetic routes were adopted:

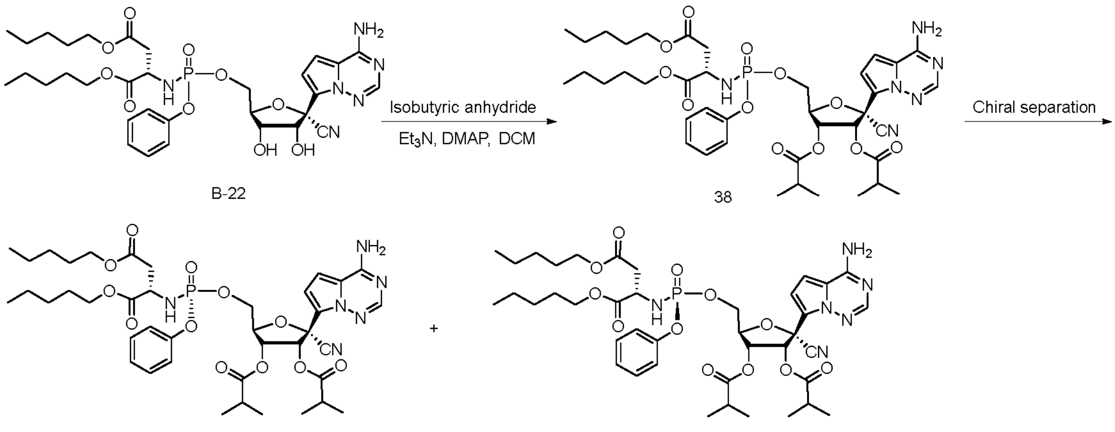


B-22

38

38a + 38b

Step 1 Synthesis of Compound 38

The intermediate B-22 (218 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 115 mg of the target product (yield: 44%). LC-MS (APCI): m/z=843.3 $(M+1)^+$. $^1H$ NMR (400 MHz, DMSO-$d_6$) δ 7.93 (s, 1H), 7.42-7.26 (m, 5H), 6.90 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.25-4.10 (m, 5H), 3.12 (dd, J = 10.8, 5.9 Hz, 1H), 3.01 (m, 2H), 2.03 (m, 2H), 1.67 (m, 4H), 1.48 (m, 8H), 1.23-0.86 (m, 18H).

Step 2 Synthesis of Compound 38a and Compound 38b

The racemate compound 38 was separated with reverse phase preparative chromatography to obtain the target product 38a (retention time: 9.63 min, relative content: 50.9%) and 38b (retention time: 10.23 min, relative content: 49.1%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**<u>Example 39: Preparation of (2R,3R,4R,5R)-2-(4-aminopyrrolo[2,1-f][1,2,4]triazin-7-yl)-2-cyano-5-((((((S)-1,4-dipentyloxy-1,4-dioxobutan-2-yl)amino)(4-tert-butyl phenoxy)phosphoryl)oxy)methyl)tetrahydrofuran-3,4-diyl bis(2-methylpropionate) (Compound 39),</u>**

**<u>(2R,3R,4R,5R)-2-(4-aminopyrrolo[2,1-f][1,2,4]triazin-7-yl)-2-cyano-5-((((R)-(((S)-1,4-dipentyloxy-1,4-dioxobutan-2-yl)amino)(4-tert-butyl phenoxy)phosphoryl)oxy)methyl)tetrahydrofuran-3,4-diyl bis(2-methylpropionate) (Compound 39a) and</u>**

<u>**(2R,3R,4R,5R)-2-(4-aminopyrrolo[2,1-f][1,2,4]triazin-7-yl)-2-cyano-5-((((S)-(((S)-1,4-dipentyloxy-1,4-dioxobutan-2-yl)amino)(4-tert-butyl phenoxy)phosphoryl)oxy)methyl)tetrahydrofuran-3,4-diyl bis(2-methylpropionate)** (Compound 39b)</u>

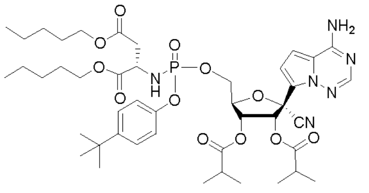

39

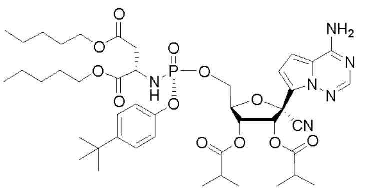

39a

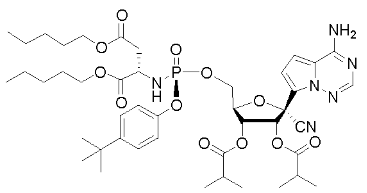

39b

The following synthetic routes were adopted:

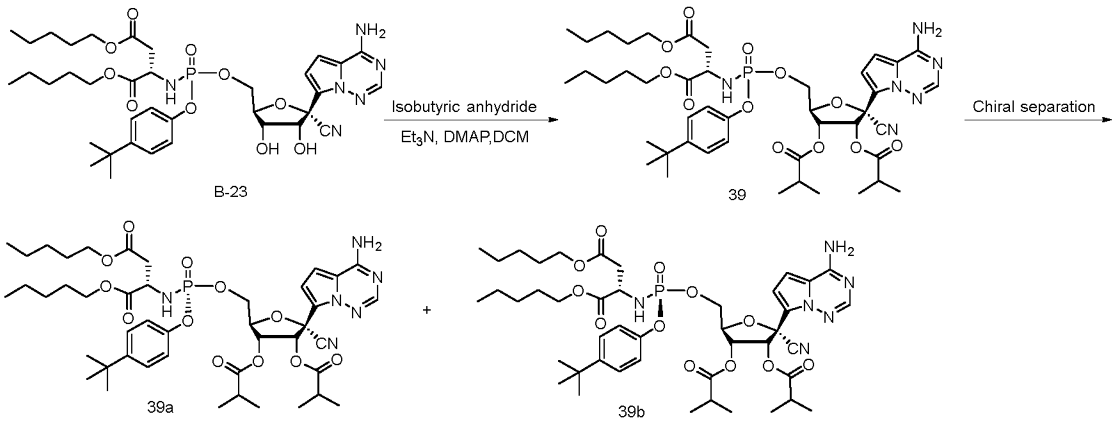


B-23 39 39a + 39b

Step 1 Synthesis of Compound 39

The intermediate B-23 (235 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 164 mg of the target product (yield: 59%). LC-MS (APCI): m/z=899.6 $(M+1)^+$. $^1H$ NMR (400 MHz, DMSO-$d_6$) δ 7.92 (s, 1H), 7.42-7.26 (m, 5H), 6.91 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.25-4.10 (m, 5H), 3.12 (dd, J = 10.8, 5.9 Hz, 1H), 3.01 (m, 2H), 2.06 (m, 2H), 1.64 (m, 4H), 1.44-1.31 (m, 8H), 1.26-0.74 (m, 27H).

Step 2 Synthesis of Compound 39a and Compound 39b

The racemate compound 39 was separated with reverse phase preparative chromatography to obtain the target product 39a (retention time: 12.53 min, relative content: 50.5%) and 39b (retention time: 14.36 min, relative content: 49.5%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 40: Preparation of (2R,3R,4R,5R)-2-(4-aminopyrrolo[2,1-f][1,2,4]triazin-7-yl)-2-cyano-5-(((((1-((2-ethylbutoxy)carbonyl)cyclobutyl)amino)(phenoxy)phosphoryl)oxy)methyl)tetrahydrofura n-3,4-diyl bis(2-methylpropionate) (Compound 40),**

**(2R,3R,4R,5R)-2-(4-aminopyrrolo[2,1-f][1,2,4]triazin-7-yl)-2-cyano-5-((((R)-((1-((2-ethylbutoxy)carbonyl)cyclobutyl)amino)(phenoxy)phosphoryl)oxy)methyl)tetrahydrofura n-3,4-diyl bis(2-methylpropionate) (Compound 40a) and**

**(2R,3R,4R,5R)-2-(4-aminopyrrolo[2,1-f][1,2,4]triazin-7-yl)-2-cyano-5-((((S)-((1-((2-ethylbutoxy)carbonyl)cyclobutyl)amino)(phenoxy)phosphoryl)oxy)methyl)tetrahydrofura n-3,4-diyl bis(2-methylpropionate) (Compound 40b)**

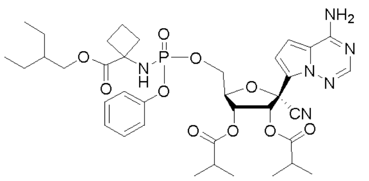

40

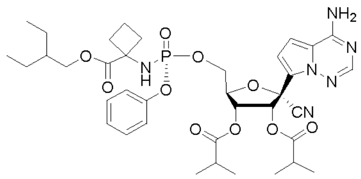

40a

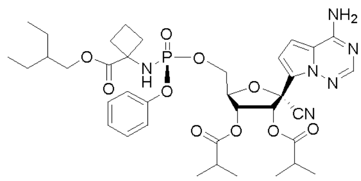

40b

The following synthetic routes were adopted:

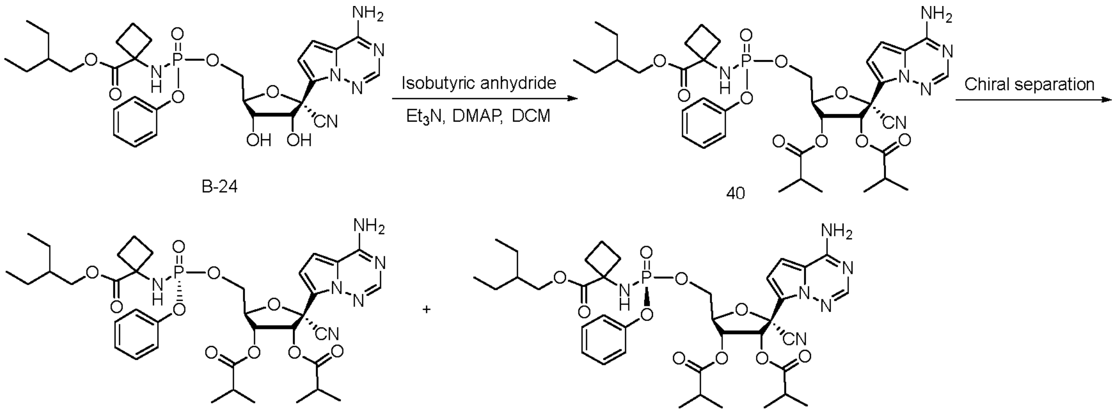


B-24

40

+

40a

40b

Step 1 Synthesis of Compound 40

The intermediate B-24 (195 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 124 mg of the target product (yield: 52%). LC-MS (APCI): m/z=769.3 $(M+1)^+$. $^1$H NMR (400 MHz, DMSO-$d_6$) δ 7.93 (s, 1H), 7.42-7.26 (m, 5H), 6.90 (d, J = 4.5 Hz, 1H), 6.80 (d, J = 4.5 Hz, 1H), 4.80 (d, J = 6.2 Hz, 2H), 4.63 (m, 2H), 4.22 (m, 3H), 2.45 (m, 2H), 2.25 (m, 2H), 2.07 (m, 3H), 1.48 (m, 4H), 1.01-0.89 (m, 18H).

Step 2 Synthesis of Compound 40a and Compound 40b

The racemate compound 40 was separated with reverse phase preparative chromatography to obtain the target product 40a (retention time: 8.37 min, relative content: 53.3%) and 40b (retention time: 9.24 min, relative content: 46.7%).

Conditions of chromatographic separation:

Column: Waters, Prep C18 OBD™, 150×19 mm, 5 μm

Column temperature: 30°C
Flow rate: 1.0 mL/min
Wavelength of UV detection: 220 nm
Mobile phase: pure water:acetonitrile=40:60

**Example 41: Preparation of (2R,3R,4R,5R)-2-(4-aminopyrrolo[2,1-f][1,2,4]triazin-7-yl)-2-cyano-5-((((((S)-1-(2-ethylbutoxy)-1-oxo-3-phenylpropan-2-yl)amino)(phenoxy)phosphoryl)oxy)methyl)tetrahydrofuran-3,4-diyl bis(2-methylpropionate) (Compound 41),**

**(2R,3R,4R,5R)-2-(4-aminopyrrolo[2,1-f][1,2,4]triazin-7-yl)-2-cyano-5-((((R)-(((S)-1-(2-ethylbutoxy)-1-oxo-3-phenylpropan-2-yl)amino)(phenoxy)phosphoryl)oxy)methyl)tetrahydrofuran-3,4-diyl bis(2-methylpropionate) (Compound 41a) and**

**(2R,3R,4R,5R)-2-(4-aminopyrrolo[2,1-f][1,2,4]triazin-7-yl)-2-cyano-5-((((S)-(((S)-1-(2-ethylbutoxy)-1-oxo-3-phenylpropan-2-yl)amino)(phenoxy)phosphoryl)oxy)methyl)tetrahydrofuran-3,4-diyl bis(2-methylpropionate) (Compound 41b)**

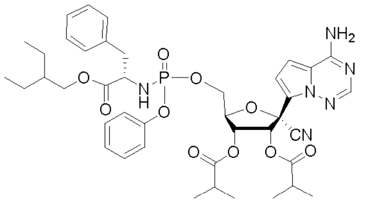
41

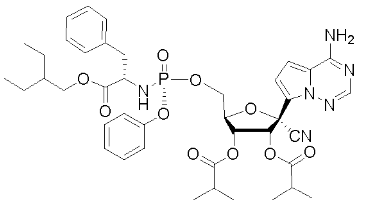
41a

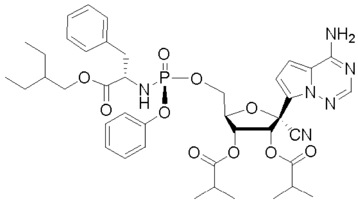
41b

The following synthetic routes were adopted:

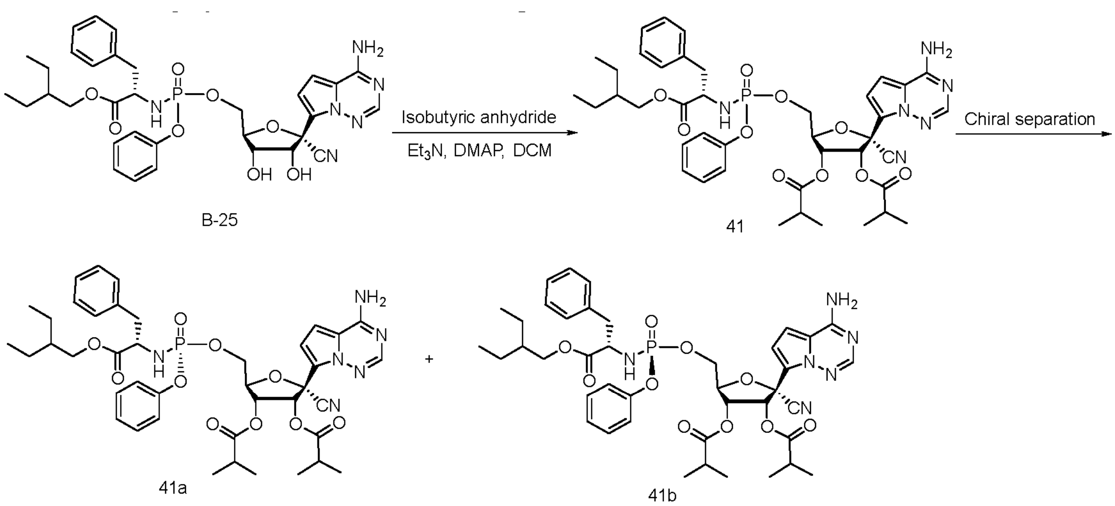

B-25
41
41a
41b

Step 1 Synthesis of Compound 41

The intermediate B-25 (210 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 122 mg of the target product (yield: 48%). LC-MS (APCI): m/z=819.5 $(M+1)^+$. $^1H$ NMR (400 MHz, $CDCl_3$) $\delta$ 7.91 (s, 1H), 7.21 (dt, J = 17.5, 7.5 Hz, 5H), 7.13-7.06 (m, 3H), 7.05-6.97 (m, 2H), 6.87 (d, J = 4.6 Hz, 1H), 6.52 (d, J = 4.7 Hz, 1H), 6.22 (d, J = 5.9 Hz, 1H), 5.90 (s, 2H), 5.49 (dd, J = 5.8, 4.0 Hz, 1H), 4.53 (d, J = 3.6 Hz, 1H), 4.36-4.28 (m, 1H), 4.24-4.12 (m, 2H), 3.96-3.77 (m, 3H), 2.91 (d, J =

6.2 Hz, 2H), 2.69-2.55 (m, 2H), 1.78 (s, 2H), 1.39 (dt, J = 12.4, 6.3 Hz, 1H), 1.27- 1.20 (m, 10H), 1.17 (dd, J = 7.0, 1.2 Hz, 6H), 0.80 (t, J = 7.4 Hz, 6H).

Step 2 Synthesis of Compound 41a and Compound 41b

The racemate compound 41 was separated with reverse phase preparative chromatography to obtain the target product 41a (retention time: 9.64 min, relative content: 54.9%) and 41b (retention time: 10.24 min, relative content: 45.1%).
Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 42: Preparation of (2R,3R,4R,5R)-2-(4-amino-5-iodopyrrolo[2,1-f][1,2,4]triazin-7-yl)-5-(((bis(((isopropoxycarbonyl)oxy)methoxy)phosphoryl)oxy)methyl)-2-cyanotetrahydrofuran-3,4-diyl bis(2-methylpropionate) (Compound 42)**

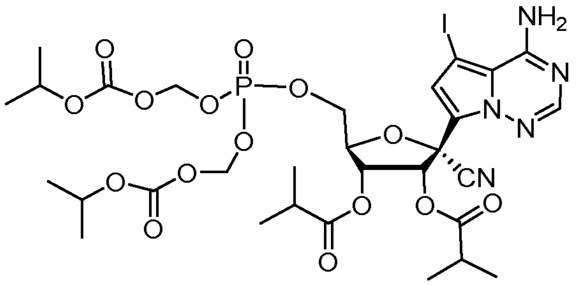

The following synthetic routes were adopted:

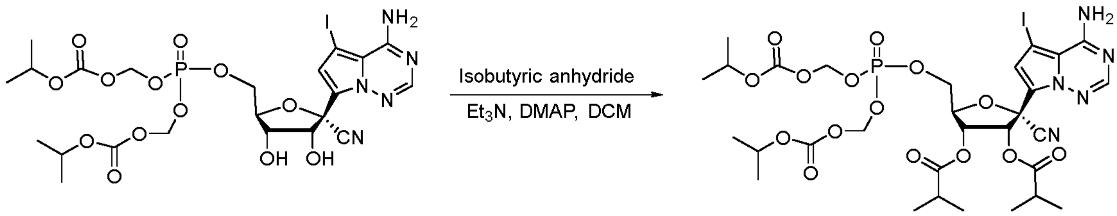


B-42 42

B-42 (226 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 127 mg of the target product (yield: 47%). LC-MS (APCI): m/z=870.6 $(M+1)^+$. $^1H$ NMR (400 MHz, DMSO-$d_6$) δ 7.99 (s, 1H), 6.75 (s, 1H), 6.11 (d, J = 5.5 Hz, 1H), 5.58 - 5.45 (m, 4H), 5.44 (dd, J = 5.5, 4.3 Hz, 1H), 4.83 (dt, J = 12.5, 6.4 Hz, 2H), 4.60 (d, J = 3.7 Hz, 1H), 4.34 (t, J = 5.9 Hz, 2H), 2.61 (dt, J = 14.0, 7.0 Hz, 2H), 1.26 (d, J = 6.1 Hz, 12H), 1.17 (dd, J = 8.3, 7.1 Hz, 6H), 1.07 (d, J = 7.0 Hz, 6H).

**Example 43: Preparation of (2R,3R,4R,5R)-2-(4-aminopyrrolo[2,1-f][1,2,4]triazin-7-yl)-5-((((acetoxylmethoxy)(4-tert-butyl phenoxy)phosphoryl)oxy)methyl)-2-cyanotetrahydrofuran-3,4-diyl bis(2-methylpropionate) (Compound 43),**

**(2R,3R,4R,5R)-2-(4-aminopyrrolo[2,1-f][1,2,4]triazin-7-yl)-5-(((((S)-acetoxylmethoxy)(4-tert-butyl phenoxy) phosphoryl)oxy)methyl)-2-cyanotetrahydrofuran-3,4-diyl bis(2-methylpropionate) (Compound 43a) and**

**(2R,3R,4R,5R)-2-(4-aminopyrrolo[2,1-f][1,2,4]triazin-7-yl)-5-(((((R)-acetoxylmethoxy)(4-tert-butyl phenoxy) phosphoryl)oxy)methyl)-2-cyanotetrahydrofuran-3,4-diyl bis(2-methylpropionate) (Compound 43b)**

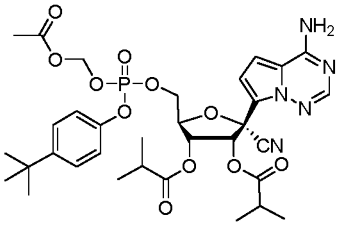

43

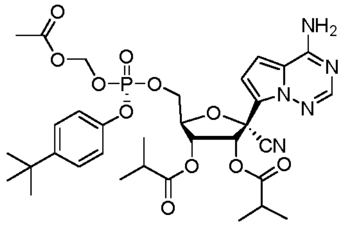

43a

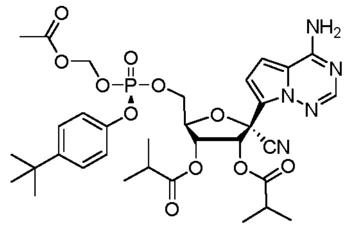

43b

The following synthetic routes were adopted:

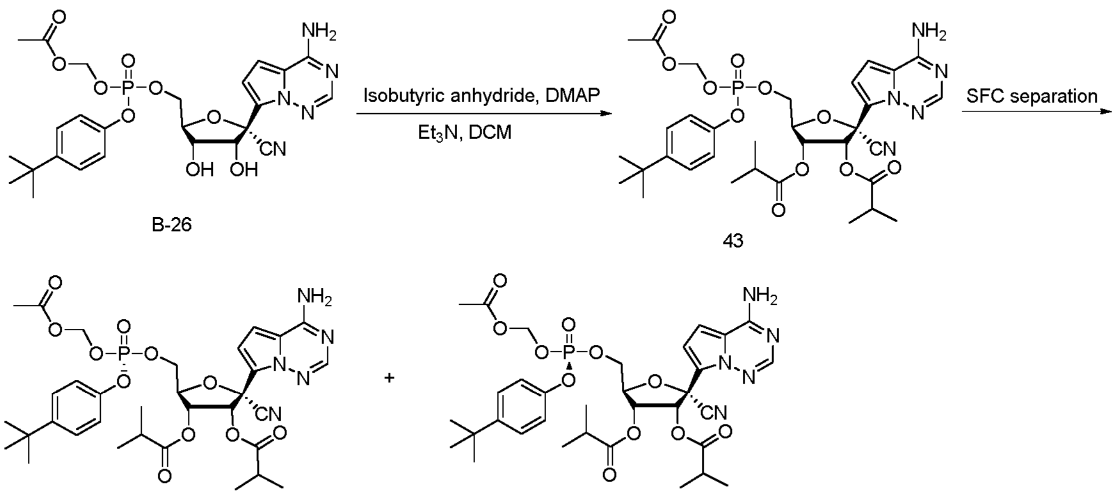


B-26

43

+

43a

43b

Step 1 Synthesis of Compound 43

The intermediate B-26 (178 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 93 mg of the target product (yield: 42%). LC-MS (APCI): m/z=716.4 $(M+1)^+$. $^1$H NMR (400 MHz, DMSO-$d_6$) δ 7.91 (s, 1H), 7.31 (dd, J = 13.4, 8.6 Hz, 2H), 7.11 - 7.05 (m, 2H), 6.92 (t, J = 4.6 Hz, 1H), 6.84 - 6.76 (m, 1H), 6.34 (dd, J = 6.0, 3.4 Hz, 1H), 5.62 (dd, J = 14.1, 5.3 Hz, 2H), 5.44 (t, J = 5.3 Hz, 1H), 4.69 (dd, J = 8.8, 5.4 Hz, 1H), 4.44 - 4.34 (m, 1H), 4.27 (t, J = 6.4 Hz, 2H), 3.96 (dd, J = 9.7, 5.0 Hz, 1H), 2.01 (m, 2H), 1.22 - 1.01 (m, 21H).

Step 2 Synthesis of Compound 43a and Compound 43b

The racemate compound 43 was separated with supercritical fluid chromatography to obtain 43a (retention time: 1.51 min, relative content: 49.2%) and 43b (retention time: 1.62 min, relative content: 50.8%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 44: Preparation of (2R,3R,4R,5R)-2-(4-aminopyrrolo[2,1-f][1,2,4]triazin-7-yl)-5-((((4-tert-butyl phenoxy)(((tert-butylcarbonyl)oxy)methoxy)phosphoryl)oxy)methyl)-2-cyanotetrahydrofuran-3,4-diyl bis(2-methylpropionate) (Compound 44),**

**(2R,3R,4R,5R)-2-(4-aminopyrrolo[2,1-f][1,2,4]triazin-7-yl)-5-(((((S)-4-tert-butyl phenoxy)(((tert-butylcarbonyl)oxy)methoxy)phosphoryl)oxy)methyl)-2-cyanotetrahydrofuran-3,4-diyl bis(2-methylpropionate) (Compound 44a) and**

**(2R,3R,4R,5R)-2-(4-aminopyrrolo[2,1-f][1,2,4]triazin-7-yl)-5-(((((R)-4-tert-butyl phenoxy)(((tert-butylcarbonyl)oxy)methoxy)phosphoryl)oxy)methyl)-2-cyanotetrahydrofuran-3,4-diyl bis(2-methylpropionate) (Compound 44b)**

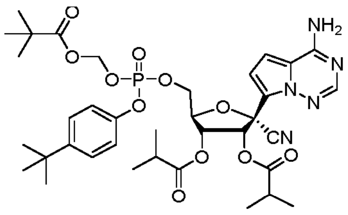
44

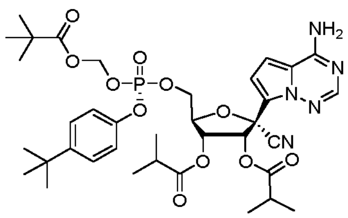
44a

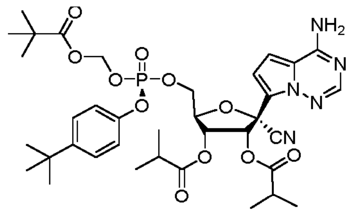
44b

The following synthetic routes were adopted:

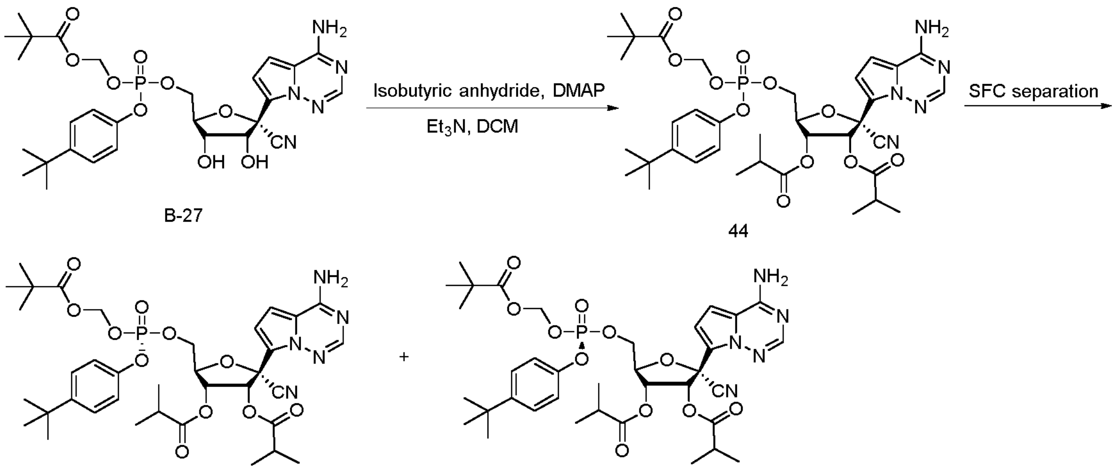

B-27
44
+
44a
44b

Step 1 Synthesis of Compound 44

The intermediate B-27 (188 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 99 mg of the target product (yield: 42%). LC-MS (APCI): m/z=758.3 $(M+1)^+$. $^1H$ NMR (400 MHz, DMSO-$d_6$) δ 7.90 (s, 1H), 7.33 (dd, J = 13.4, 8.6 Hz, 2H), 7.11 - 7.05 (m, 2H), 6.92 (t, J = 4.6 Hz, 1H), 6.84 - 6.76 (m, 1H), 6.34 (dd, J = 6.0, 3.4 Hz, 1H), 5.62 (dd, J = 14.1, 5.3 Hz, 2H), 5.44 (t, J = 5.3 Hz, 1H), 4.69 (dd, J = 8.8, 5.4 Hz, 1H), 4.44 - 4.34 (m, 1H), 4.27 (t, J = 6.4 Hz, 2H), 3.96 (dd, J = 9.7, 5.0 Hz, 1H), 2.01 (m, 2H), 1.22 - 1.01 (m, 30H).

Step 2 Synthesis of Compound 44a and Compound 44b

The racemate compound 44 was separated with supercritical fluid chromatography to obtain the target product

44a (retention time: 1.52 min, relative content: 49.0%) and 44b (retention time: 1.61 min, relative content: 51.0%).

Conditions of chromatographic separation:

Column: CHIRALPAK, IG-3, 50×4.6 mm I.D., 3 μm
Column temperature: 35°C
Flow rate: 3.0 mL/min
Wavelength of UV detection: 220 nm
Mobile phase: carbon dioxide:isopropanol (0.05% diethylamine)=60:40

**Example 45: Preparation of (2R,3R,4R,5R)-2-(4-aminopyrrolo[2,1-f][1,2,4ltriazin-7-yl)-5-((((bis(((isopropylcarbonyl)oxy)methoxy))phosphoryl)oxy)methyl)-2-cyanotetrahydrofuran-3,4-diyl bis(2-methylpropionate) (Compound 45)**

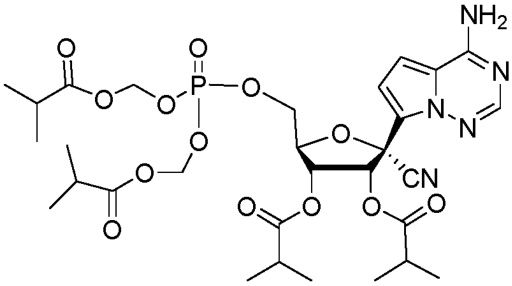

45

The following route was adopted for synthesis:

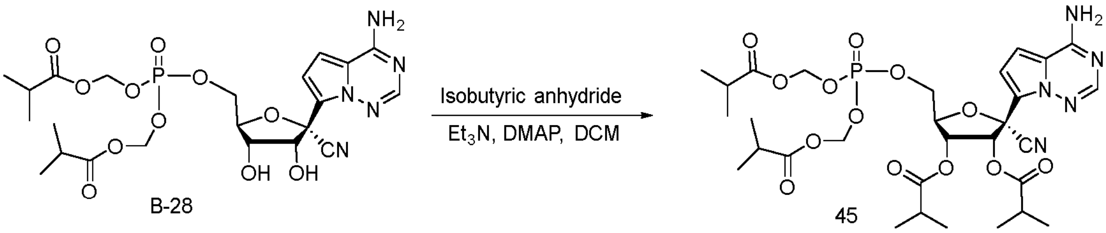


B-28

45

The intermediate B-28 (177 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 105 mg of the product (yield: 48%). LC-MS (APCI): m/z=712.3 $(M+1)^+$. $^1H$ NMR (400 MHz, DMSO-$d_6$) δ 8.00 (s, 1H), 7.04 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 2.05 (m, 2H), 1.24 - 0.87 (m, 24H).

**Example 46: Preparation of (2R,3R,4R,5R)-2-(4-aminopyrrolo[2,1-f][1,2,4]triazin-7-yl)-2-cyano-5-((((((S)-1-(dodecyloxy)-1-oxopropan-2-yl)amino)(phenoxy)phosphoryl)oxy)methyl)tetrahydrofuran-3,4-diyl bis(2-methylpropionate) (Compound 46),**

**(2R,3R,4R,5R)-2-(4-aminopyrrolo[2,1-f][1,2,41triazin-7-yl)-2-cyano-5-((((R)-(((S)-1-(dodecyloxy)-1-oxopropan-2-yl)amino)(phenoxy)phosphoryl)oxy)methyl)tetrahydrofuran-3,4-diyl bis(2-methylpropionate) (Compound 46a) and**

**(2R,3R,4R,5R)-2-(4-aminopyrrolo[2,1-f][1,2,4]triazin-7-yl)-2-cyano-5-((((S)-(((S)-1-(dodecyloxy)-1-oxopropan-2-yl)amino)(phenoxy)phosphoryl)oxy)methyl)tetrahydrofuran-3,4-diyl bis(2-methylpropionate) (Compound 46b)**

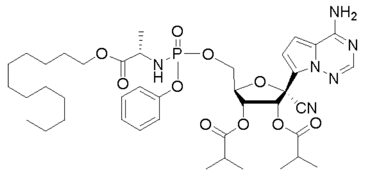

46

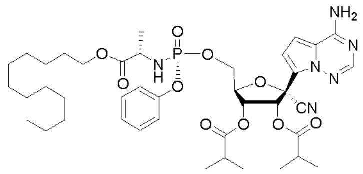

46a

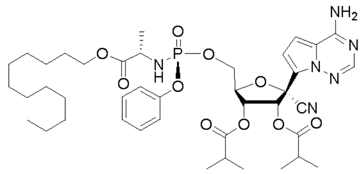

46b

The following route was adopted for synthesis:

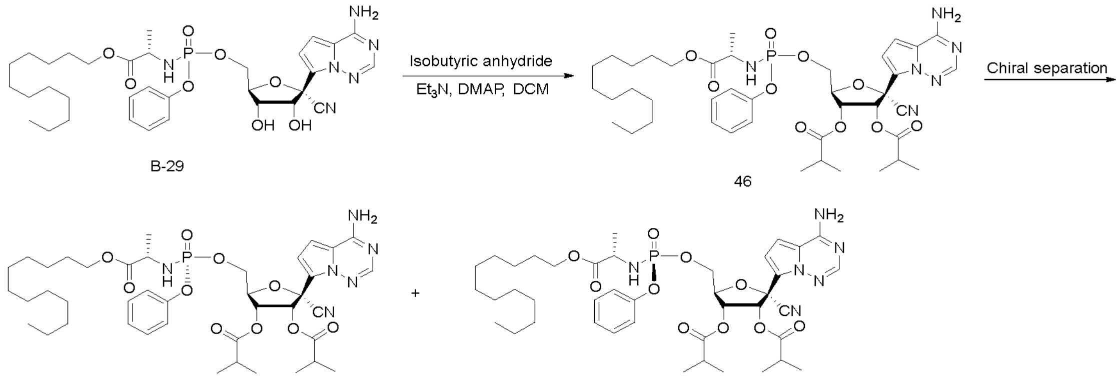


B-29

46

46a

46b

Step 1 Synthesis of Compound 46

The intermediate B-29 (213 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 159 mg of the target product (yield: 62%). LC-MS (APCI): m/z=827.6 $(M+1)^+$. $^1H$ NMR (400 MHz, DMSO-$d_6$) δ 7.99 (s, 1H), 7.04 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.62 (d, J = 4.7 Hz, 1H), 4.30 (m, 1H), 4.11 (m, 1H), 2.05 (m, 2H), 1.27-0.82 (m, 24H).

Step 2 Synthesis of Compound 46a and Compound 46b

The racemate compound 46 was separated with reverse phase preparative chromatography to obtain the target product 46a (retention time: 11.6 min, relative content: 51.6%) and 46b (retention time: 13.2 min, relative content: 48.4%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 47: Preparation of (2R,3R,4R,5R)-2-(4-aminopyrrolo[2,1-f][1,2,4]triazin-7-yl)-2-cyano-5-((((((S)-1-(((9Z,12Z)-octadeca-9,12-dien-1-yl)oxy)-1-oxopropan-2-yl)amino)(phenoxy)phosphoryl)oxy)methyl)tetrahydrofuran-3,4-diyl bis(2-methylpropionate) (Compound 47),**

**(2R,3R,4R,5R)-2-(4-aminopyrrolo[2,1-f][1,2,41triazin-7-yl)-2-cyano-5-((((R)-(((S)-1-(((9Z,12Z)-octadeca-9,12-dien-1-yl)oxy)-1-oxopropan-2-yl)amino)(phenoxy)phosphoryl)oxy)methyl)tetrahydrofuran-3,4-diyl bis(2-methylpropionate) (Compound 47a) and**

**(2R,3R,4R,5R)-2-(4-aminopyrrolo[2,1-f][1,2,4]triazin-7-yl)-2-cyano-5-((((S)-(((S)-1-(((9Z,12Z)-octadeca-9,12-dien-1-yl)oxy)-1-oxopropan-2-yl)amino)(phenoxy)phosphoryl)oxy)methyl)tetrahydrofuran-3,4-diyl bis(2-methylpropionate) (Compound 47b)**

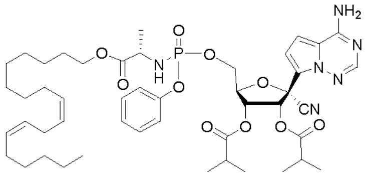

47

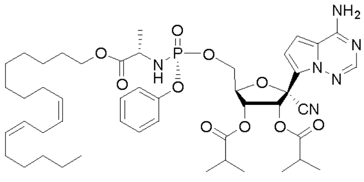

47a

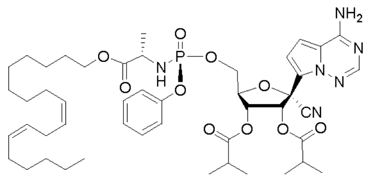

47b

The following route was adopted for synthesis:

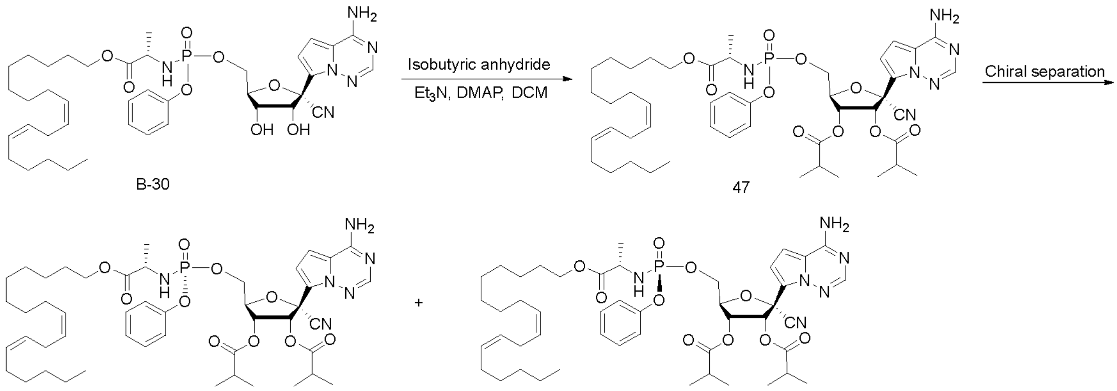


B-30

47

47a

+

47b

Step 1 Synthesis of Compound 47

Following the synthetic procedure for compound 46, intermediate B-30 (239 mg, 0.31 mmol) was substituted with Intermediate B-29 (213 mg, 0.31 mmol) to obtain compound 47 (544 mg) as a colorless oily liquid. LC-MS (APCI): m/z = 907.7 $[M+1]^+$. $^1H$ NMR (400 MHz, DMSO-$d_6$) δ 8.04 (s, 1H), 7.07 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 2.05 (m, 2H), 1.24 - 0.87 (m, 30H).

Step 2 Synthesis of Compound 47a and Compound 47b

The racemate compound 47 was separated with reverse phase preparative chromatography to obtain the target product 47a (retention time: 11.3 min, relative content: 54.7%) and 47b (retention time: 13.5 min, relative content: 45.3%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 48: Preparation of (2R,3R,4R,5R)-2-(4-aminopyrrolo[2,1-f][1,2,4]triazin-7-yl)-2-cyano-5-((((4-tert-butyl phenoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 48),**

**(2R,3R,4R,5R)-2-(4-aminopyrrolo[2,1-f][1,2,4]triazin-7-yl)-2-cyano-5-((((R)-(4-tert-butyl phenoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 48a) and**

**(2R,3R,4R,5R)-2-(4-aminopyrrolo[2,1-f][1,2,4]triazin-7-yl)-2-cyano-5-((((S)-(4-tert-butyl phenoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 48b)**

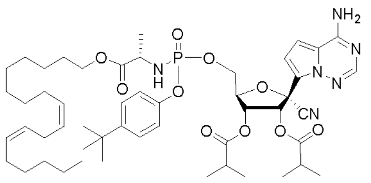

48

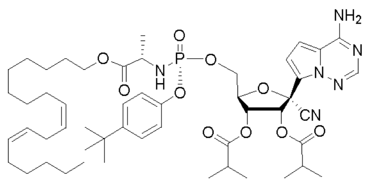

48a

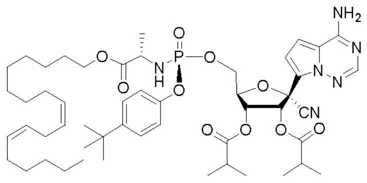

48b

The following route was adopted for synthesis:

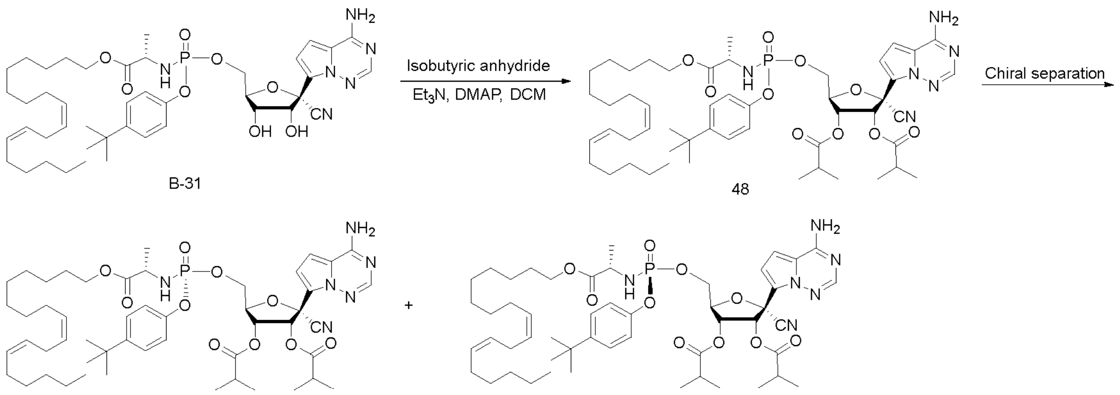


48a

48b

Step 1 Synthesis of Compound 48

The intermediate B-31 (255 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 152 mg of the target product (yield: 51%). LC-MS (APCI): m/z=963.4 $(M+1)^+$. $^1H$ NMR (400 MHz, DMSO-$d_6$) $\delta$ 8.02 (s, 1H), 7.03 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 2.05 (m, 2H), 1.25 - 0.87 (d, J = 6.4 Hz, 21H), 0.82 (s, 9H).

Step 2 Synthesis of Compound 48a and Compound 48b

The racemate compound 48 was separated with reverse phase preparative chromatography to obtain the target product 48a (retention time: 13.4 min, relative content: 50.3%) and 48b (retention time: 15.4 min, relative content: 49.7%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 49: Preparation of (2R,3R,4R,5R)-2-(4-aminopyrrolo[2,1-f][1,2,4]triazin-7-yl)-2-cyano-5-((((((9Z,12Z)-octadeca-9,12-dien-1-yl)oxy)(phenoxy)phosphoryl)oxy)methyl)tetrahydrofuran-3,4-diyl bis(2-methylpropionate) (Compound 49)**

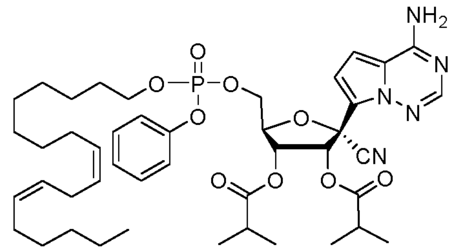

49

The following route was adopted for synthesis:

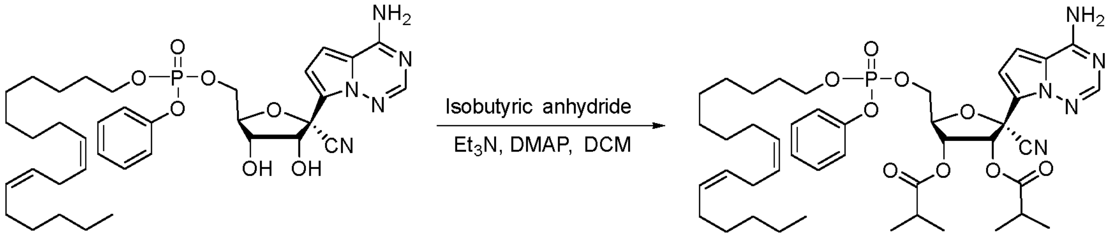


B-32

49

The intermediate B-32 (215 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 114 mg of the target product (yield: 44%). LC-MS (APCI): m/z=836.2 $(M+1)^+$. $^1H$ NMR (400 MHz, DMSO-$d_6$) δ 8.01 (s, 1H), 7.07 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 2.05 (m, 2H), 1.26 - 0.84 (m, 30H).

**Example 50: Preparation of (2R,3R,4R,5R)-2-(4-aminopyrrolo[2,1-f][1,2,4]triazin-7-yl)-2-cyano-5-((((((9Z,12Z)-octadeca-9,12-dien-1-yl)oxy)(4-tert-butyl phenoxy)phosphoryl)oxy)methyl)tetrahydrofuran-3,4-diyl bis(2-methylpropionate)** (Compound 50)

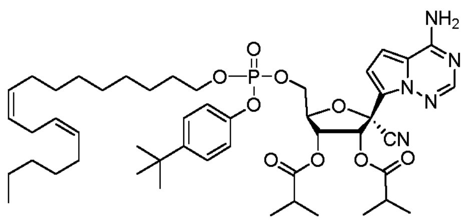

50

The following route was adopted for synthesis:

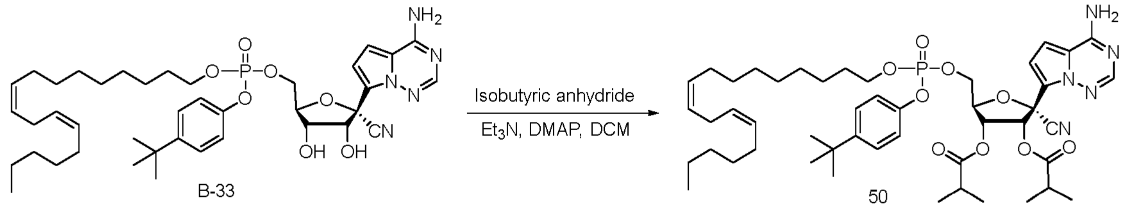


The intermediate B-33 (233 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 121 mg of the target product (yield: 44%). LC-MS (APCI): m/z=892.4 $(M+1)^+$. $^1H$ NMR (400 MHz, DMSO-$d_6$) δ 8.03 (s, 1H), 7.11 (d, J = 4.5 Hz, 1H), 6.87 (d, J = 4.5 Hz, 1H), 5.57 (d, J = 13.6 Hz, 4H), 4.82 (td, J = 12.2, 6.1 Hz, 2H), 4.65 (d, J = 4.7 Hz, 1H), 4.30 (m, 1H), 4.11 (m, 1H), 2.05 (m, 2H), 1.24 - 0.87 (d, J = 6.4 Hz, 30H).

Example 51: **<u>Preparation of (2R,3R,4R,5R)-2-(4-aminopyrrolo[2,1-f][1,2,4]triazin-7-yl)-2-cyano-5-((((((S)-1-(((9Z,12Z)-octadeca-9,12-dien-1-yl)oxy)-1-oxopropan-2-yl)amino)((pivaloyloxy)methoxy)phosphoryl)oxy)methyl)tetrahydrofuran-3,4-diyl bis(2-methylpropionate) (Compound 51)</u>**

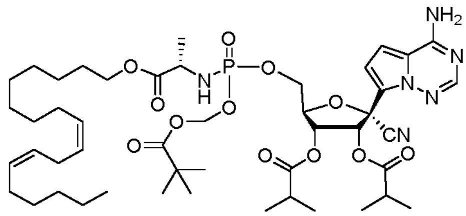

51

The following synthetic routes were adopted:

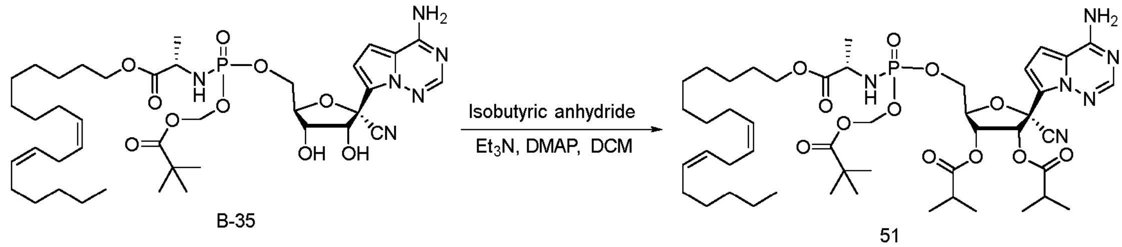


The intermediate B-35 (249 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 170 mg of the target product (yield: 58%). LC-MS (APCI): m/z=945.7 $(M+1)^+$. $^1H$ NMR (400 MHz, $CDCl_3$) δ 8.00 (s, 1H), 7.25 - 7.20 (m, 2H), 7.09 (d, J = 6.9 Hz, 2H), 6.93 (d, J = 4.6 Hz, 1H), 6.56 (d, J = 4.6 Hz, 1H), 6.22 (d, J = 5.8 Hz, 1H), 5.69 (s, 2H), 5.55 - 5.49 (m, 1H), 5.48 - 5.37 (m, 2H), 4.89 (dt, J = 12.4, 6.2 Hz, 1H), 4.53 (d, J = 3.7 Hz, 1H), 4.21 (ddt, J = 19.3, 15.1, 7.9 Hz, 3H), 3.97 (d, J = 5.5 Hz, 2H), 3.54 (t, J = 10.7 Hz, 1H), 2.94 (qd, J = 13.7, 6.2 Hz, 2H), 2.63 (ddt, J = 20.9, 14.0, 7.0 Hz, 2H), 1.44 (dt, J = 12.1, 6.1 Hz, 1H), 1.23 (ddd, J = 24.4, 21.1, 6.6 Hz, 22H), 0.84 (s, 9H).

**<u>Example 52: Preparation of (2R,3R,4R,5R)-2-(4-aminopyrrolo[2,1-f][1,2,4]triazin-7-yl)-2-cyano-5-((((((isopropoxycarbonyl)oxy)methoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 52),</u>**

**(2R,3R,4R,5R)-2-(4-aminopyrrolo[2,1-f][1,2,4]triazin-7-yl)-2-cyano-5-((((R)-(((isopropoxycarbonyl)oxy)methoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 52a) and**

**(2R,3R,4R,5R)-2-(4-aminopyrrolo[2,1-f][1,2,4]triazin-7-yl)-2-cyano-5-((((S)-(((isopropoxycarbonyl)oxy)methoxy)(((S)-1-(((9Z,12Z)-octadeca-9,12-dien-1-yl)oxy)-1-oxopropan-2-yl)amino)phosphoryl)oxy)methyl)tetrahydrofuran-3,4-diyl bis(2-methylpropionate) (Compound 52b)**

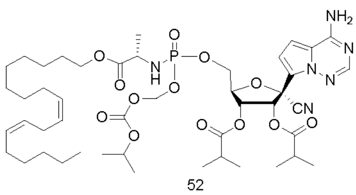

52

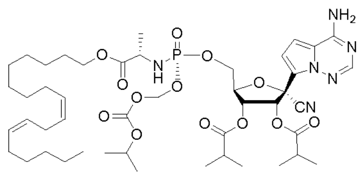

52a

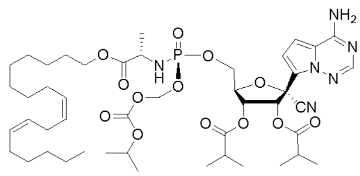

52b

The following synthetic routes were adopted:

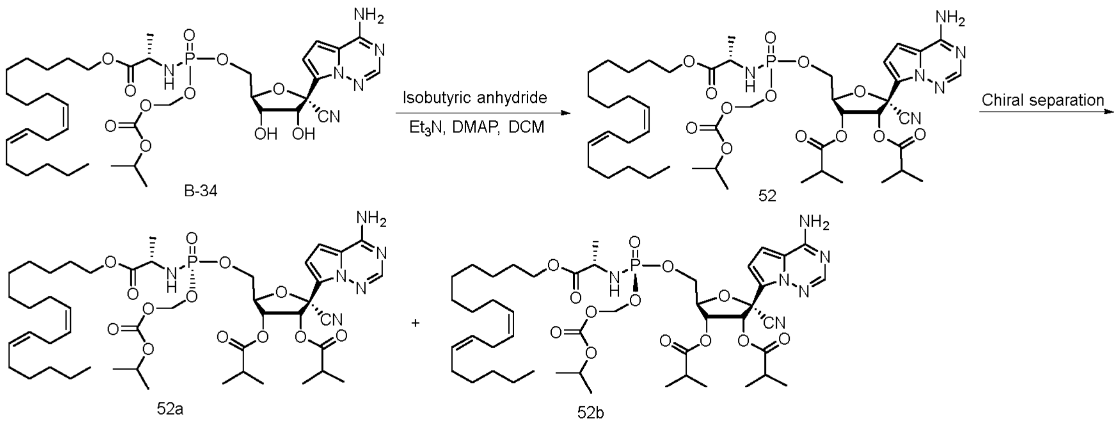


B-34

52

52a

+

52b

Step 1 Synthesis of Compound 52

The intermediate B-34 (250 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 132 mg of the target product (yield: 45%). LC-MS (APCI): m/z=947.5 $(M+1)^+$. $^1H$ NMR (400 MHz, $CDCl_3$) $\delta$ 8.01 (s, 1H), 7.26 - 7.20 (m, 2H), 7.11 (d, J = 6.9 Hz, 2H), 6.92 (d, J = 4.6 Hz, 1H), 6.56 (d, J = 4.6 Hz, 1H), 6.24 (d, J = 5.8 Hz, 1H), 5.66 (s, 2H), 5.55 - 5.49 (m, 1H), 5.48 - 5.37 (m, 2H), 4.89 (dt, J = 12.4, 6.2 Hz, 1H), 4.53 (d, J = 3.7 Hz, 1H), 4.21 (ddt, J = 19.3, 15.1, 7.9 Hz, 3H), 3.97 (d, J = 5.5 Hz, 2H), 3.54 (t, J = 10.7 Hz, 1H), 2.94 (qd, J = 13.7, 6.2 Hz, 2H), 2.63 (ddt, J = 20.9, 14.0, 7.0 Hz, 2H), 1.44 (dt, J = 12.1, 6.1 Hz, 1H), 1.23 (ddd, J = 24.4, 21.1, 6.6 Hz, 22H), 0.84 (t, J = 7.4 Hz, 6H).

Step 2 Synthesis of Compound 52a and Compound 52b

The racemate compound 52 was separated with reverse phase preparative chromatography to obtain the target product 52a (retention time: 11.25 min, relative content: 50.4%) and 52b (retention time: 12.39 min, relative content: 49.6%).

Conditions of chromatographic separation:

Column: Waters, Prep C18 OBD™, 150×19 mm, 5 μm
Column temperature: 30°C
Flow rate: 1.0 mL/min

Wavelength of UV detection: 220 nm
Mobile phase: pure water:acetonitrile=40:60

**Example 53: Preparation of (2R,3R,4R,5R)-2-(4-aminopyrrolo[2,1-f][1,2,4]triazin-7-yl)-2-cyano-5-((((((S)-1-(2-ethylbutoxy)-1-oxo-3-phenylpropan-2-yl)amino)(4-tert-butyl phenoxy)phosphoryl)oxy)methyl)tetrahydrofuran-3,4-diyl bis(2-methylpropionate) (Compound 53),**

**(2R,3R,4R,5R)-2-(4-aminopyrrolo[2,1-f][1,2,4]triazin-7-yl)-2-cyano-5-((((((S)-1-(2-ethylbutoxy)-1-oxo-3-phenylpropan-2-yl)amino)((R)-4-tert-butyl phenoxy)phosphoryl)oxy)methyl)tetrahydrofuran-3,4-diyl bis(2-methylpropionate) (Compound 53a) and**

**(2R,3R,4R,5R)-2-(4-aminopyrrolo[2,1-f][1,2,4]triazin-7-yl)-2-cyano-5-((((((S)-1-(2-ethylbutoxy)-1-oxo-3-phenylpropan-2-yl)amino)((S)-4-tert-butyl phenoxy)phosphoryl)oxy)methyl)tetrahydrofuran-3,4-diyl bis(2-methylpropionate) (Compound 53b)**

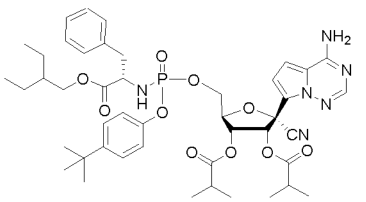
53

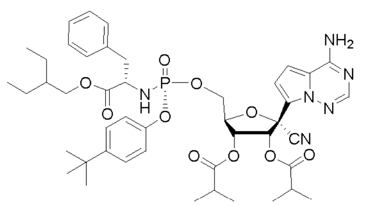
53a

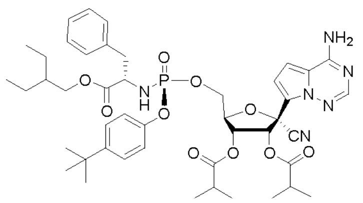
53b

The following synthetic routes were adopted:

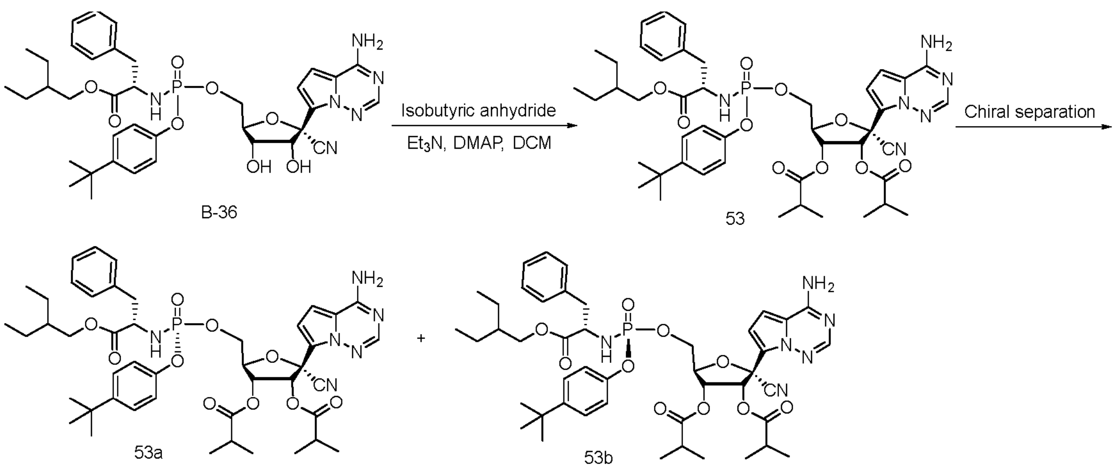

B-36 53 53a + 53b

Step 1 Synthesis of Compound 53

The intermediate B-36 (227 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 179 mg of the target product (yield: 66%). LC-MS (APCI): m/z=875.2 $(M+1)^+$. $^1H$ NMR (400 MHz, $CDCl_3$) $\delta$ 7.91 (s, 1H), 7.21 (dt, J = 17.5, 7.5 Hz, 5H), 7.13 - 7.06 (m, 3H), 7.05 - 6.97 (m, 2H), 6.87 (d, J = 4.6 Hz, 1H), 6.52 (d, J = 4.7 Hz, 1H), 6.22 (d, J = 5.9 Hz, 1H), 5.90 (s, 2H), 5.49 (dd, J = 5.8, 4.0 Hz, 1H), 4.53 (d, J = 3.6 Hz, 1H), 4.36 - 4.28 (m, 1H), 4.24 - 4.12 (m, 2H), 3.96 - 3.77 (m, 3H), 2.91 (d, J = 6.2 Hz, 2H), 2.69 - 2.55 (m, 2H), 1.78 (s, 2H), 1.39 (dt, J = 12.4, 6.3 Hz, 1H), 1.27 - 1.20 (m, 10H), 1.17 (dd, J = 7.0, 1.2 Hz, 6H), 0.81 (t, J = 7.4 Hz, 9H).

Step 2 Synthesis of Compound 53a and Compound 53b

The racemate compound 53 was separated with reverse phase preparative chromatography to obtain the target product 53a (retention time: 11.26 min, relative content: 53.4%) and 53b (retention time: 13.24 min, relative content: 46.6%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 54: Preparation of (2R,3R,4R,5R)-2-(4-aminopyrrolo[2,1-f][1,2,4]triazin-7-yl)-2-cyano-5-((((((S)-1,4-dicyclobutoxy-1,4-dioxobutan-2-yl)amino)(phenoxy)phosphoryl)oxy)methyl)tetrahydrofuran-3,4-diyl bis(2-methylpropionate) (Compound 54),**

**(2R,3R,4R,5R)-2-(4-aminopyrrolo[2,1-f][1,2,4]triazin-7-yl)-2-cyano-5-((((R)-(((S)-1,4-dicyclobutoxy-1,4-dioxobutan-2-yl)amino)(phenoxy)phosphoryl)oxy)methyl)tetrahydrofuran-3,4-diyl bis(2-methylpropionate) (Compound 54a) and**

**(2R,3R,4R,5R)-2-(4-aminopyrrolo[2,1-f][1,2,4]triazin-7-yl)-2-cyano-5-((((S)-(((S)-1,4-dicyclobutoxy-1,4-dioxobutan-2-yl)amino)(phenoxy)phosphoryl)oxy)methyl)tetrahydrofuran-3,4-diyl bis(2-methylpropionate) (Compound 54b)**

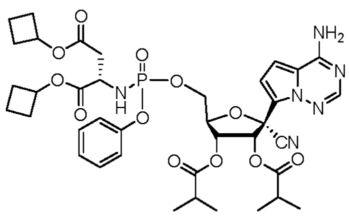

54

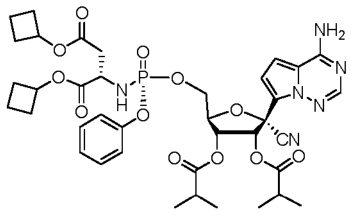

54a

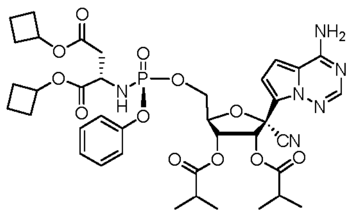

54b

The following synthetic routes were adopted:

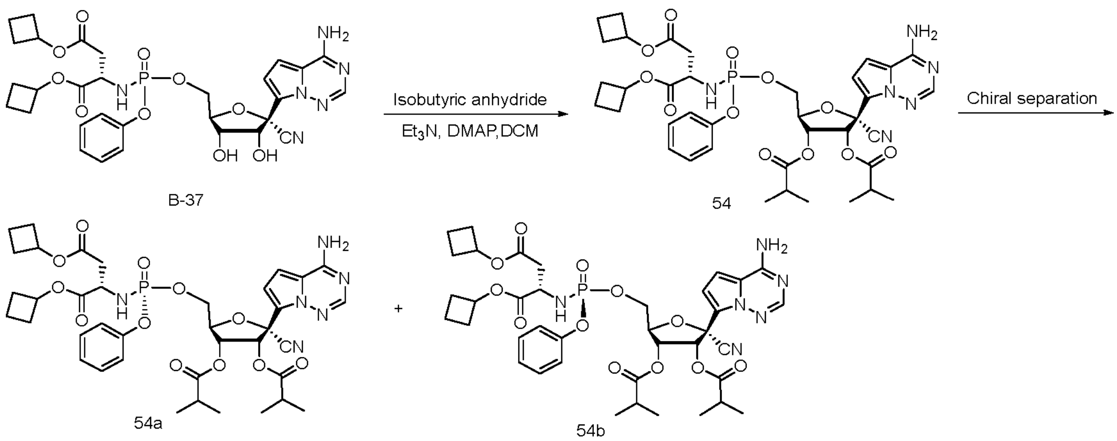


B-37

54

54a

+

54b

Step 1 Synthesis of Compound 54

The intermediate B-37 (208 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen

atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 85 mg of the target product (yield: 34%). LC-MS (APCI): m/z=811.1 $(M+1)^+$. $^1$H NMR (400 MHz, DMSO-$d_6$) δ 7.91 (s, 1H), 7.40 - 7.22 (m, 5H), 6.91 (d, J = 4.5 Hz, 1H), 6.81 (d, J = 4.5 Hz, 1H), 4.81 (dt, J = 12.5, 6.2 Hz, 2H), 4.61 (d, J = 3.5 Hz, 2H), 4.25 - 4.10 (m, 5H), 3.12 (dd, J = 10.8, 5.9 Hz, 1H), 3.05 (m, 2H), 2.06 (m, 2H), 1.64 (m, 4H), 1.43(m, 8H), 1.22 - 0.94 (m, 12H).

Step 2 Synthesis of Compound 54a and Compound 54b

The racemate compound 54 was separated with reverse phase preparative chromatography to obtain the target product 54a (retention time: 9.62 min, relative content: 50.4%) and 54b (retention time: 10.21 min, relative content: 49.6%).
Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 55: Preparation of (2R,3R,4R,5R)-2-(4-aminopyrrolo[2,1-f][1,2,4]triazin-7-yl)-2-cyano-5-((((((S)-1,4-dicyclobutoxy-1,4-dioxobutan-2-yl)amino)(phenoxy)phosphoryl)oxy)methyl)tetrahydrofuran-3,4-diyl diacetate (Compound 55),**

**(2R,3R,4R,5R)-2-(4-aminopyrrolo[2,1-f][1,2,4]triazin-7-yl)-2-cyano-5-((((R)-(((S)-1,4-dicyclobutoxy-1,4-dioxobutan-2-yl)amino)(phenoxy)phosphoryl)oxy)methyl)tetrahydrofuran-3,4-diyl diacetate (Compound 55a) and**

**(2R,3R,4R,5R)-2-(4-aminopyrrolo[2,1-f][1,2,4]triazin-7-yl)-2-cyano-5-((((S)-(((S)-1,4-dicyclobutoxy-1,4-dioxobutan-2-yl)amino)(phenoxy)phosphoryl)oxy)methyl)tetrahydrofuran-3,4-diyl diacetate (Compound 55b)**

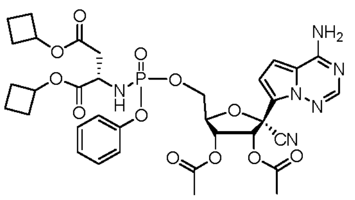
55

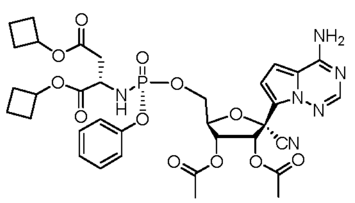
55a

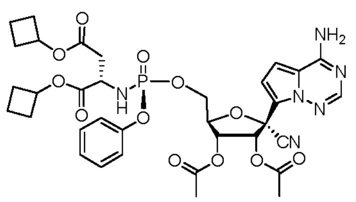
55b

The following synthetic routes were adopted:

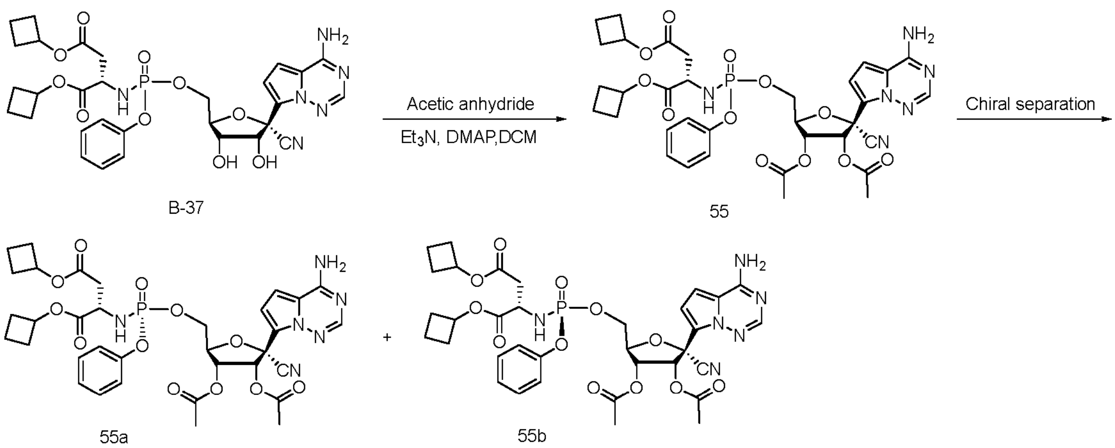

B-37
55
55a
+
55b

Step 1 Synthesis of Compound 55

The intermediate B-37 (208 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 147 mg of the product (yield: 63%). LC-MS (APCI): m/z=755.3 $(M+1)^+$. $^1H$ NMR (400 MHz, DMSO-$d_6$) δ 7.89 (s, 1H), 7.37 - 7.26 (m, 5H), 6.88 (d, J = 4.5 Hz, 1H), 6.81 (d, J = 4.5 Hz, 1H), 4.80 (dt, J = 12.5, 6.2 Hz, 2H), 4.59 (d, J = 3.6 Hz, 2H), 4.25 - 4.10 (m, 5H), 3.12 (dd, J = 10.8, 5.9 Hz, 1H), 3.01 (m, 2H), 2.03 (m, 2H), 1.67 (m, 4H), 1.41-1.26 (m, 8H), 1.21 (m, 6H).

Step 2 Synthesis of Compound 55a and Compound 55b

The racemate compound 55 was separated with reverse phase preparative chromatography to obtain the target product 55a (retention time: 8.36 min, relative content: 49.8%) and 55b (retention time: 9.28 min, relative content: 50.2%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 56: Preparation of (2R,3R,4R,5R)-2-(4-aminopyrrolo[2,1-f][1,2,4]triazin-7-yl)-2-cyano-5-((((((S)-1,4-dicyclobutoxy-1,4-dioxobutan-2-yl)amino)(phenoxy)phosphoryl)oxy)methyl)tetrahydrofuran-3,4-diyl dipropionate (Compound 56),**

**(2R,3R,4R,5R)-2-(4-aminopyrrolo[2,1-f][1,2,4]triazin-7-yl)-2-cyano-5-((((R)-(((S)-1,4-dicyclobutoxy-1,4-dioxobutan-2-yl)amino)(phenoxy)phosphoryl)oxy)methyl)tetrahydrofuran-3,4-diyl dipropionate (Compound 56a) and**

**(2R,3R,4R,5R)-2-(4-aminopyrrolo[2,1-f][1,2,4]triazin-7-yl)-2-cyano-5-((((S)-(((S)-1,4-dicyclobutoxy-1,4-dioxobutan-2-yl)amino)(phenoxy)phosphoryl)oxy)methyl)tetrahydrofuran-3,4-diyl dipropionate (Compound 56b)**

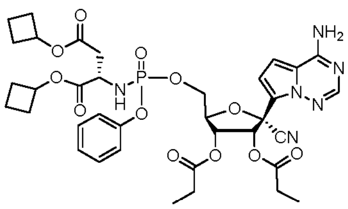
56

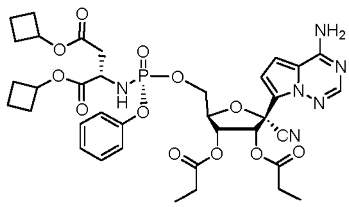
56a

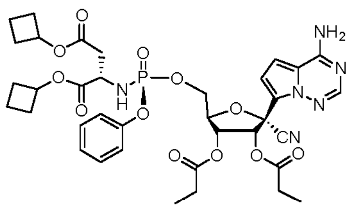
56b

The following synthetic routes were adopted:

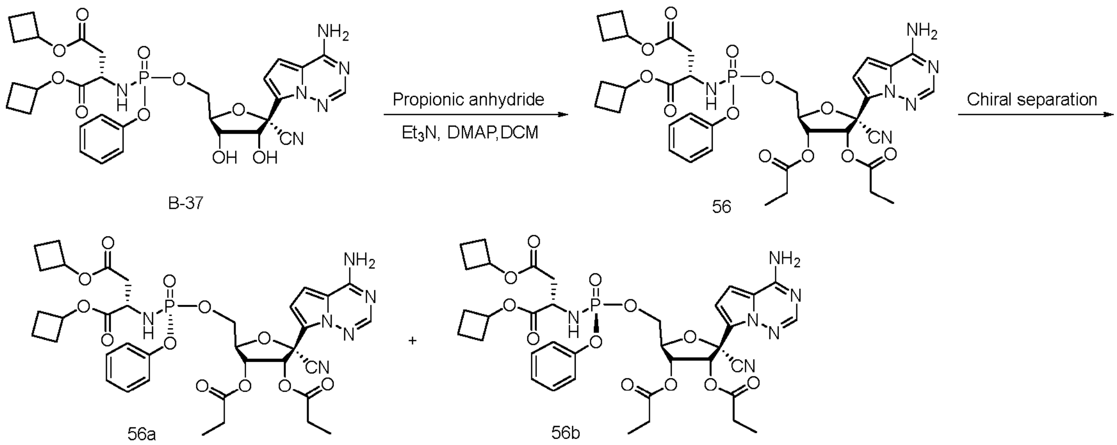


B-37 56 56a + 56b

Step 1 Synthesis of Compound 56

The intermediate B-37 (208 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 133 mg of the product (yield: 55%). LC-MS (APCI): m/z=783.2 $(M+1)^+$. $^1H$ NMR (400 MHz, DMSO-$d_6$) δ 7.93 (s, 1H), 7.40 - 7.26 (m, 5H), 6.90 (d, J = 4.5 Hz, 1H), 6.85 (d, J = 4.5 Hz, 1H), 4.80 (dt, J = 12.5, 6.2 Hz, 2H), 4.61 (d, J = 3.5 Hz, 2H), 4.23 - 4.10 (m, 5H), 3.12 (dd, J = 10.8, 5.9 Hz, 1H), 3.05 (m, 2H), 2.03 (m, 2H), 1.63 (m, 4H), 1.44-1.37 (m, 8H), 1.18 (m, 6H).

Step 2 Synthesis of Compound 56a and Compound 56b

The racemate compound 56 was separated with reverse phase preparative chromatography to obtain the target product 56a (retention time: 11.22 min, relative content: 51.2%) and 56b (retention time: 12.36 min, relative content: 48.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 57: Preparation of (2R,3R,4R,5R)-2-(4-aminopyrrolo[2,1-f][1,2,4]triazin-7-yl)-2-cyano-5-((((((S)-1,4-dicyclobutoxy-1,4-dioxobutan-2-yl)amino)(phenoxy)phosphoryl)oxy)methyl)tetrahydrofuran-3,4-diyl bis(2,2-dimethylpropionate) (Compound 57),**

**(2R,3R,4R,5R)-2-(4-aminopyrrolo[2,1-f][1,2,4]triazin-7-yl)-2-cyano-5-((((R)-(((S)-1,4-dicyclobutoxy-1,4-dioxobutan-2-yl)amino)(phenoxy)phosphoryl)oxy)methyl)tetrahydrofuran-3,4-diyl bis(2,2-dimethylpropionate) (Compound 57a) and**

**(2R,3R,4R,5R)-2-(4-aminopyrrolo[2,1-f][1,2,4]triazin-7-yl)-2-cyano-5-((((S)-(((S)-1,4-dicyclobutoxy-1,4-dioxobutan-2-yl)amino)(phenoxy)phosphoryl)oxy)methyl)tetrahydrofuran-3,4-diyl bis(2,2-dimethylpropionate) (Compound 57b)**

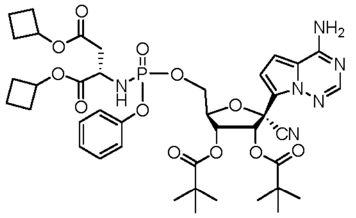

57

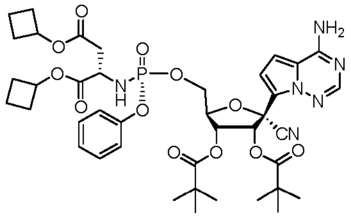

57a

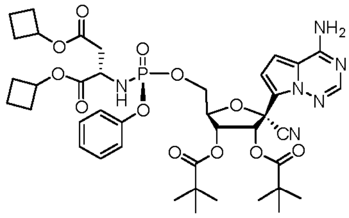

57b

The following synthetic routes were adopted:

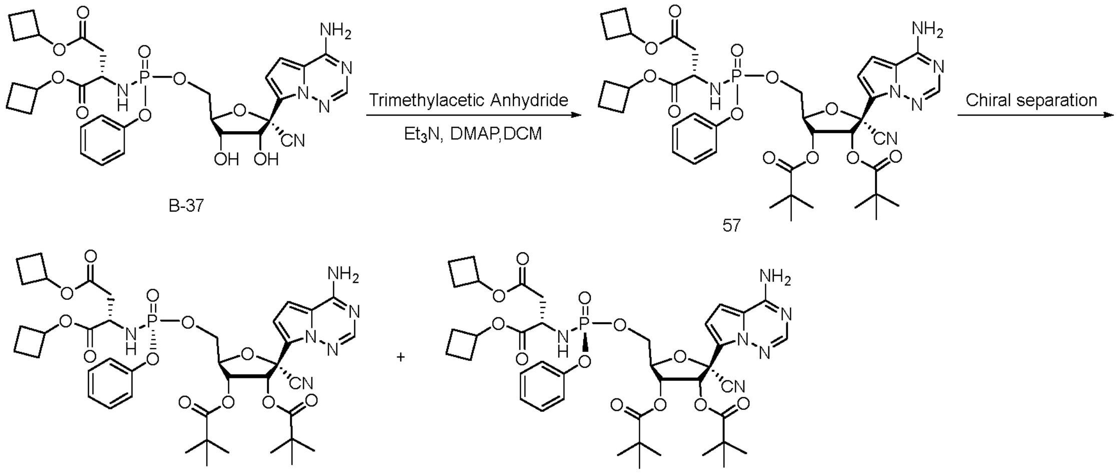


B-37

57

57a

57b

Step 1 Synthesis of Compound 57

The intermediate B-37 (208 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 86 mg of the target product (yield: 33%). LC-MS (APCI): m/z=839.4 $(M+1)^+$. $^1H$ NMR (400 MHz, DMSO-$d_6$) $\delta$ 7.91 (s, 1H), 7.41-7.26 (m, 5H), 6.90 (d, J = 4.5 Hz, 1H), 6.80 (d, J = 4.5 Hz, 1H), 4.80 (dt, J = 12.5, 6.2 Hz, 2H), 4.64 (d, J = 3.5 Hz, 2H), 4.25-4.10 (m, 5H), 3.12 (dd, J = 10.8, 5.9 Hz, 1H), 3.06 (m, 2H), 2.01 (m, 2H), 1.67 (m, 4H), 1.45-1.27 (m, 8H), 1.23-0.91 (m, 18H).

Step 2 Synthesis of Compound 57a and Compound 57b

The racemate compound 57 was separated with reverse phase preparative chromatography to obtain the target product 57a (retention time: 12.35 min, relative content: 48.7%) and 57b (retention time: 13.57 min, relative content: 51.3%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 58: Preparation of (2R,3R,4R,5R)-2-(4-aminopyrrolo[2,1-f][1,2,4]triazin-7-yl)-2-cyano-5-(((((S)-1,4-bicyclopentyloxy-1,4-dioxobutan-2-yl)amino)(phenoxy)phosphoryl)oxy)methyl)tetrahydrofuran-3,4-diyl diacetate (Compound 58),**

**(2R,3R,4R,5R)-2-(4-aminopyrrolo[2,1-f][1,2,4]triazin-7-yl)-2-cyano-5-((((R)-(((S)-1,4-bicyclopentyloxy-1,4-dioxobutan-2-yl)amino)(phenoxy)phosphoryl)oxy)methyl)tetrahydrofuran-3,4-diyl diacetate (Compound 58a) and**

**(2R,3R,4R,5R)-2-(4-aminopyrrolo[2,1-f][1,2,4]triazin-7-yl)-2-cyano-5-((((S)-(((S)-1,4-bicyclopentyloxy-1,4-dioxobutan-2-yl)amino)(phenoxy)phosphoryl)oxy)methyl)tetrahydrofuran-3,4-diyl diacetate (Compound 58b)**

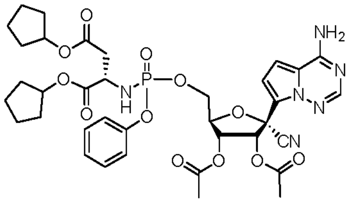

58

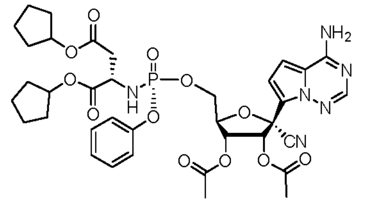

58a

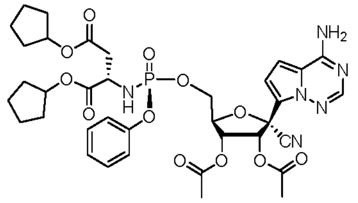

58b

The following synthetic routes were adopted:

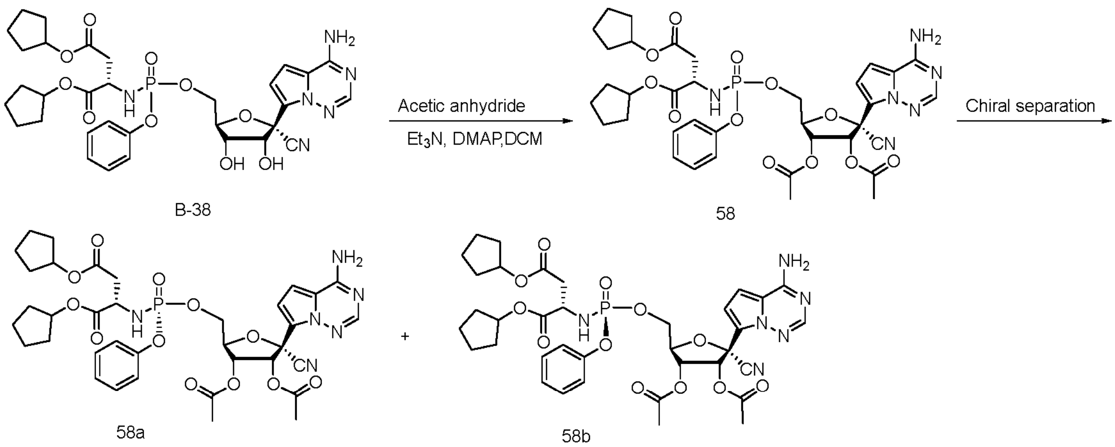


Step 1 Synthesis of Compound 58

The intermediate B-38 (216 mg, 0.31 mmol) was added to a reaction flask, followed by the addtion of 10 mL of anhydrous dichloromethane to dissolve. Acetic anhydride (79 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 179 mg of the target product (yield: 74%). LC-MS (APCI): m/z=783.3 $(M+1)^+$. $^1H$ NMR (400 MHz, DMSO-$d_6$) δ 7.92 (s, 1H), 7.42-7.26 (m, 5H), 6.88 (d, J = 4.5 Hz, 1H), 6.80 (d, J = 4.5 Hz, 1H), 4.80 (dt, J = 12.5, 6.2 Hz, 2H), 4.62 (d, J = 3.5 Hz, 2H), 4.25-4.10 (m, 5H), 3.12 (dd, J = 10.8, 5.9 Hz, 1H), 3.01 (m, 2H), 2.07 (m, 2H), 1.67 (m, 4H), 1.48-1.29 (m, 8H).

Step 2 Synthesis of Compound 58a and Compound 58b

The racemate compound 58 was separated with reverse phase preparative chromatography to obtain the target product 58a (retention time: 8.56 min, relative content: 51.2%) and 58b (retention time: 9.34 min, relative content: 48.8%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 59: Preparation of (2R,3R,4R,5R)-2-(4-aminopyrrolo[2,1-f][1,2,4]triazin-7-yl)-2-cyano-5-((((((S)-1,4-bicyclopentyloxy-1,4-dioxobutan-2-yl)amino)(phenoxy)phosphoryl)oxy)methyl)tetrahydrofuran-3,4-diyl dipropionate (Compound 59),**

**(2R,3R,4R,5R)-2-(4-aminopyrrolo[2,1-f][1,2,4]triazin-7-yl)-2-cyano-5-((((R)-(((S)-1,4-bicyclopentyloxy-1,4-dioxobutan-2-yl)amino)(phenoxy)phosphoryl)oxy)methyl)tetrahydrofuran-3,4-diyl dipropionate (Compound 59a) and**

**(2R,3R,4R,5R)-2-(4-aminopyrrolo[2,1-f][1,2,4]triazin-7-yl)-2-cyano-5-((((S)-(((S)-1,4-bicyclopentyloxy-1,4-dioxobutan-2-yl)amino)(phenoxy)phosphoryl)oxy)methyl)tetrahydrofuran-3,4-diyl dipropionate (Compound 59b)**

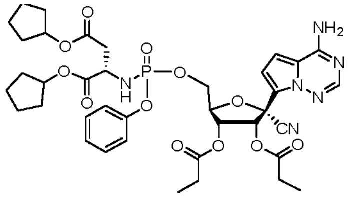
59

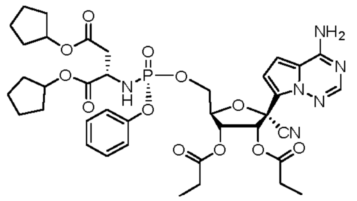
59a

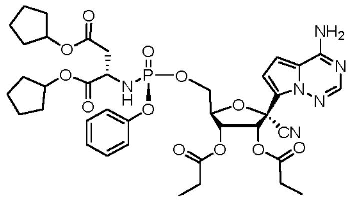
59b

The following synthetic routes were adopted:

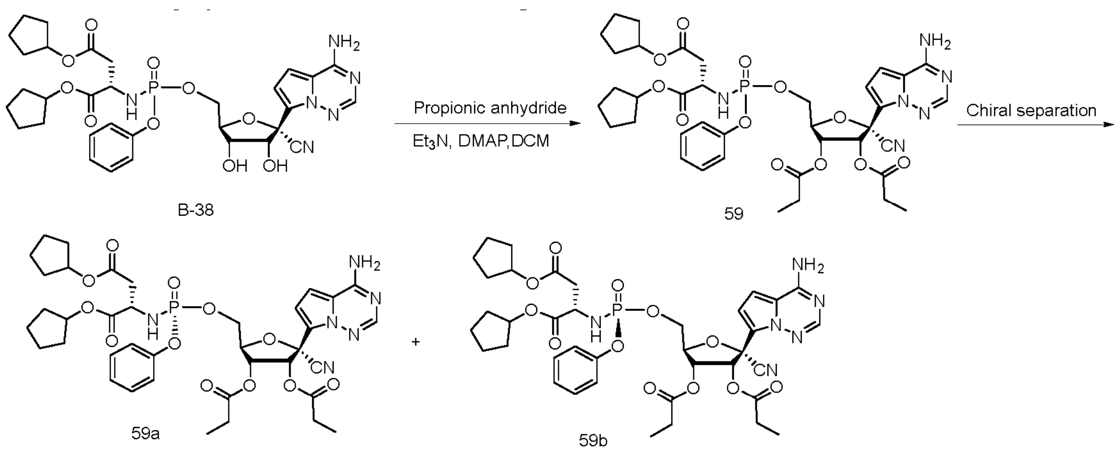

B-38 59 59a + 59b

Step 1 Synthesis of Compound 59

The intermediate B-38 (216 mg, 0.31 mmol) was added to a reaction flask, followed by the addtion of 10 mL of anhydrous dichloromethane to dissolve. Propionic anhydride (101 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 118 mg of the target product (yield: 47%). LC-MS (APCI): m/z=811.4 $(M+1)^+$. $^1$H NMR (400 MHz, DMSO-$d_6$) δ 7.91 (s, 1H), 7.39-7.26 (m, 5H), 6.91 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.27-4.10 (m, 5H), 3.12 (dd, J = 10.8, 5.9 Hz, 1H), 3.01 (m, 2H), 2.03 (m, 2H), 1.67 (m, 4H), 1.48 (m, 8H), 1.22-1.10 (m, 6H).

Step 2 Synthesis of Compound 59a and Compound 59b

The racemate compound 59 was separated with reverse phase preparative chromatography to obtain the target product 59a (retention time: 9.37 min, relative content: 51.0%) and 59b (retention time: 10.42 min, relative content: 49.0%).

Conditions of chromatographic separation:

Column: Waters, Prep C18 OBD™, 150×19 mm, 5 μm

Column temperature: 30°C
Flow rate: 1.0 mL/min
Wavelength of UV detection: 220 nm
Mobile phase: pure water:acetonitrile=40:60

**Example 60: Preparation of (2R,3R,4R,5R)-2-(4-aminopyrrolo[2,1-f][1,2,4]triazin-7-yl)-2-cyano-5-((((((S)-1,4-bicyclopentyloxy-1,4-dioxobutan-2-yl)amino)(phenoxy)phosphoryl)oxy)methyl)tetrahydrofuran-3,4-diyl bis(2-methylpropionate) (Compound 60),**

**(2R,3R,4R,5R)-2-(4-aminopyrrolo[2,1-f][1,2,4]triazin-7-yl)-2-cyano-5-((((R)-(((S)-1,4-bicyclopentyloxy-1,4-dioxobutan-2-yl)amino)(phenoxy)phosphoryl)oxy)methyl)tetrahydrofuran-3,4-diyl bis(2-methylpropionate) (Compound 60a) and**

**(2R,3R,4R,5R)-2-(4-aminopyrrolo[2,1-f][1,2,4]triazin-7-yl)-2-cyano-5-((((S)-(((S)-1,4-bicyclopentyloxy-1,4-dioxobutan-2-yl)amino)(phenoxy)phosphoryl)oxy)methyl)tetrahydrofuran-3,4-diyl bis(2-methylpropionate) (Compound 60b)**

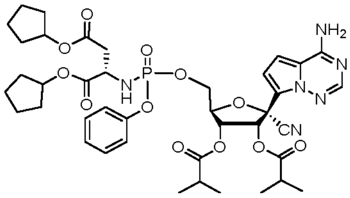
60

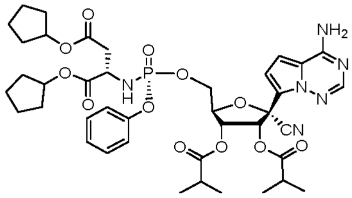
60a

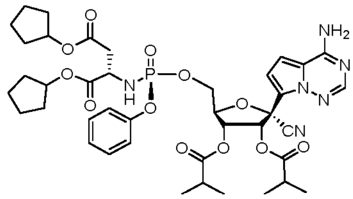
60b

The following synthetic routes were adopted:

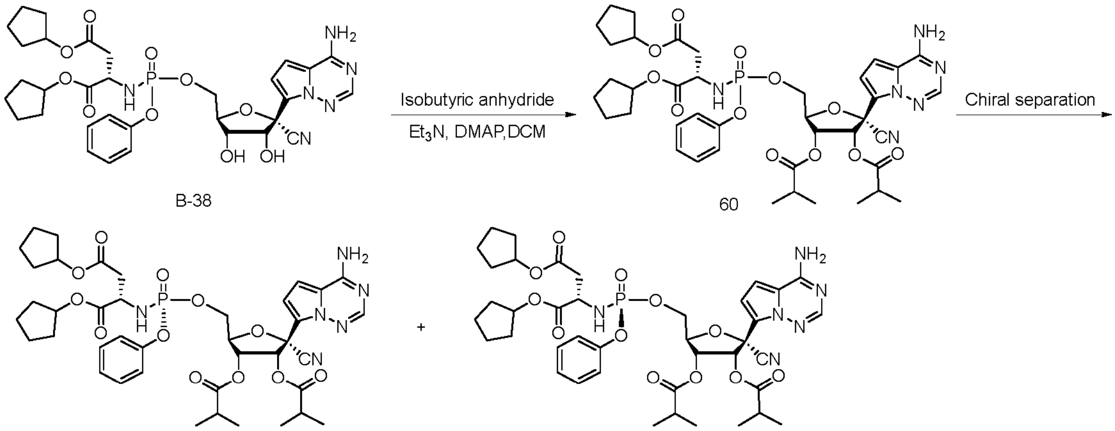

B-38
60
60a
+
60b

Step 1 Synthesis of Compound 60

The intermediate B-38 (216 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 174 mg of the product (yield: 67%). LC-MS (APCI): m/z=839.7 $(M+1)^+$. $^1H$ NMR (400 MHz, DMSO-$d_6$) δ 7.92 (s, 1H), 7.38-7.22 (m, 5H), 6.90 (d, J = 4.5 Hz, 1H), 6.81 (d, J = 4.5 Hz, 1H), 4.80 (dt, J = 12.5, 6.2 Hz, 2H), 4.62 (d, J = 3.5 Hz, 2H), 4.25-4.10 (m, 5H), 3.12 (dd, J = 10.8, 5.9 Hz, 1H), 2.99 (m, 2H), 2.04 (m, 2H), 1.65 (m, 4H), 1.44 (m, 8H), 1.22-0.97 (m, 12H).

Step 2 Synthesis of Compound 60a and Compound 60b

The racemate compound 60 was separated with reverse phase preparative chromatography to obtain the target product 60a (retention time: 9.78 min, relative content: 51.1%) and 60b (retention time: 10.93 min, relative content: 48.9%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 61: Preparation of (2R,3R,4R,5R)-2-(4-aminopyrrolo[2,1-f][1,2,4]triazin-7-yl)-2-cyano-5-((((((S)-1,4-bicyclopentyloxy-1,4-dioxobutan-2-yl)amino)(phenoxy)phosphoryl)oxy)methyl)tetrahydrofuran-3,4-diyl bis(2,2-dimethylpropionate) (Compound 61),**

**(2R,3R,4R,5R)-2-(4-aminopyrrolo[2,1-f][1,2,4]triazin-7-yl)-2-cyano-5-((((R)-(((S)-1,4-bicyclopentyloxy-1,4-dioxobutan-2-yl)amino)(phenoxy)phosphoryl)oxy)methyl)tetrahydrofuran-3,4-diyl bis(2,2-dimethylpropionate) (Compound 61a) and**

**(2R,3R,4R,5R)-2-(4-aminopyrrolo[2,1-f][1,2,4]triazin-7-yl)-2-cyano-5-((((S)-(((S)-1,4-bicyclopentyloxy-1,4-dioxobutan-2-yl)amino)(phenoxy)phosphoryl)oxy)methyl)tetrahydrofuran-3,4-diyl bis(2,2-dimethylpropionate) (Compound 61b)**

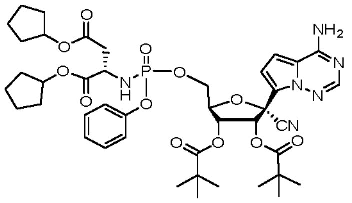
61

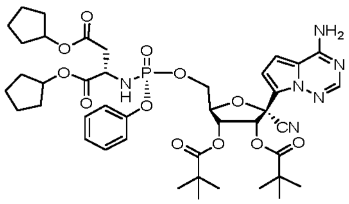
61a

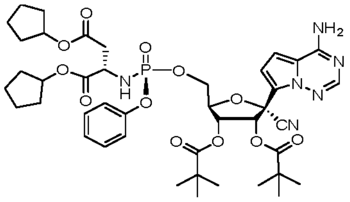
61b

The following synthetic routes were adopted:

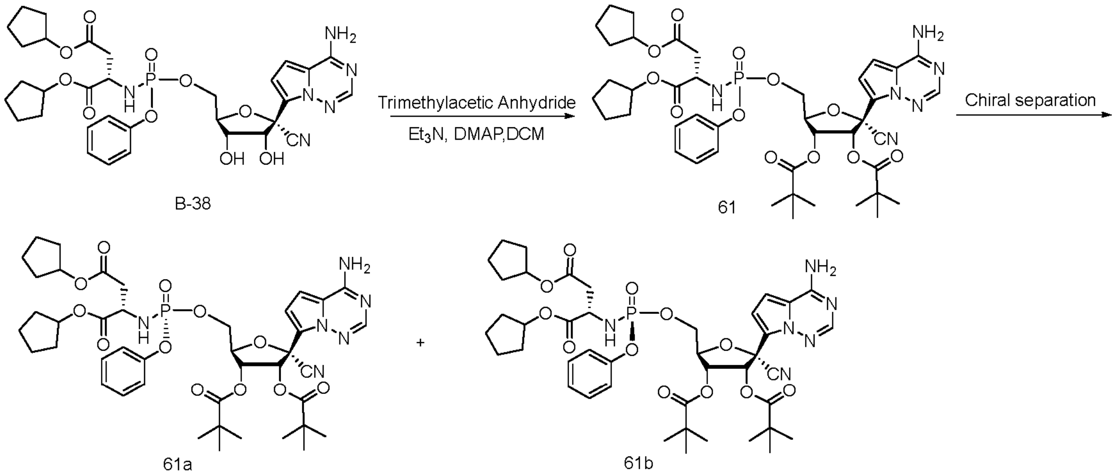

B-38
61
61a
+
61b

Step 1 Synthesis of Compound 61

The intermediate B-38 (216 mg, 0.31 mmol) was added to a reaction flask, followed by the addtion of 10 mL of anhydrous dichloromethane to dissolve. Pivalic anhydride (145 mg, 0.78 mmol), triethylamine (111 mg, 1.1 mmol) and DMAP (19.6 mg, 0.16 mmol) were added successively. The mixture was stirred at room temperature under nitrogen

atmosphere overnight.

After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 78 mg of the target product (yield: 29%). LC-MS (APCI): m/z=867.1 $(M+1)^+$. $^1H$ NMR (400 MHz, DMSO-$d_6$) δ 7.93 (s, 1H), 7.42-7.26 (m, 5H), 6.91 (d, J = 4.5 Hz, 1H), 6.82 (d, J = 4.5 Hz, 1H), 4.80 (dt, J = 12.5, 6.2 Hz, 2H), 4.61 (d, J = 3.5 Hz, 2H), 4.25-4.10 (m, 5H), 3.12 (dd, J = 10.8, 5.9 Hz, 1H), 3.01 (m, 2H), 2.03 (m, 2H), 1.69 (m, 4H), 1.45 (m, 6H), 1.31-0.91 (m, 18H).

Step 2 Synthesis of Compound 61a and Compound 61b

The racemate compound 61 was separated with reverse phase preparative chromatography to obtain the target product 61a (retention time: 9.58 min, relative content: 49.9%) and 61b (retention time: 10.64 min, relative content: 50.1%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 62: Preparation of (2R,3R,4R,5R)-2-(4-aminopyrrolo[2,1-f][1,2,4]triazin-7-yl)-2-cyano-5-((((((S)-1,4-bis(2-ethylbutoxy)-1,4-dioxobutan-2-yl)amino)(phenoxy)phosphoryl)oxy)methyl)tetrahydrofuran-3,4-diyl bis(2-methylpropionate) (Compound 62),**

**(2R,3R,4R,5R)-2-(4-aminopyrrolo[2,1-f][1,2,4]triazin-7-yl)-2-cyano-5-((((R)-(((S)-1,4-bis(2-ethylbutoxy)-1,4-dioxobutan-2-yl)amino)(phenoxy)phosphoryl)oxy)methyl)tetrahydrofuran-3,4-diyl bis(2-methylpropionate) (Compound 62a) and**

**(2R,3R,4R,5R)-2-(4-aminopyrrolo[2,1-f][1,2,4]triazin-7-yl)-2-cyano-5-((((S)-(((S)-1,4-bis(2-ethylbutoxy)-1,4-dioxobutan-2-yl)amino)(phenoxy)phosphoryl)oxy)methyl)tetrahydrofuran-3,4-diyl bis(2-methylpropionate) (Compound 62b)**

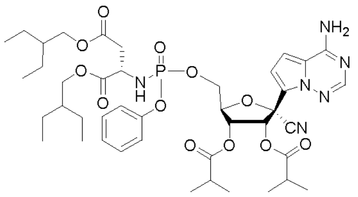
62

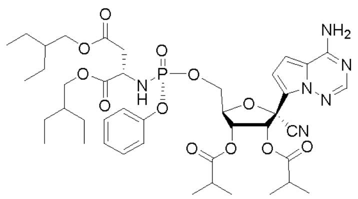
62a

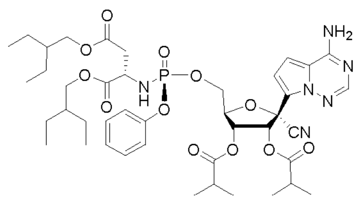
62b

The following synthetic routes were adopted:

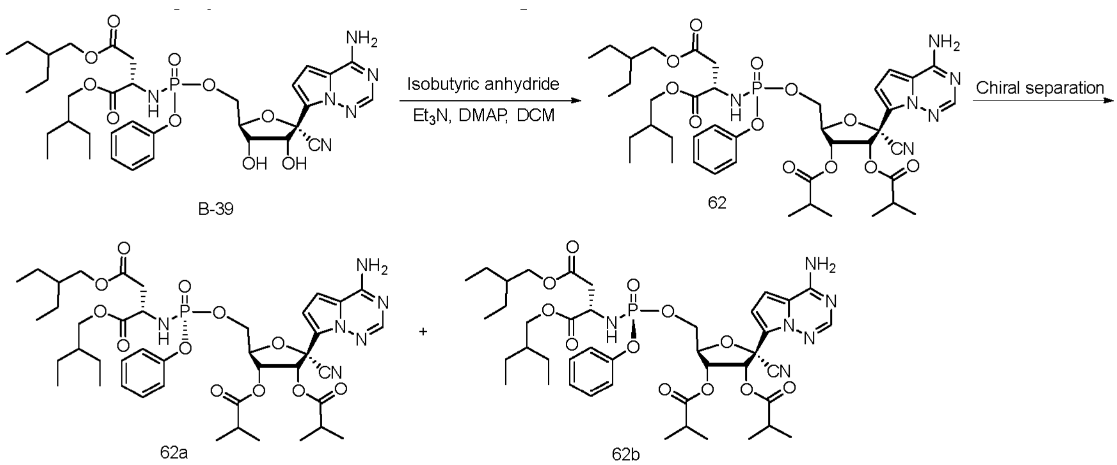


B-39 62

62a + 62b

Step 1 Synthesis of Compound 62

The intermediate B-39 (226 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 135 mg of the product (yield: 50%). LC-MS (APCI): m/z=871.2 $(M+1)^+$. $^1H$ NMR (400 MHz, DMSO-$d_6$) $\delta$ 7.93 (s, 1H), 7.42-7.26 (m, 5H), 6.90 (d, J = 4.5 Hz, 1H), 6.80 (d, J = 4.5 Hz, 1H), 4.80 (dt, J = 12.5, 6.2 Hz, 2H), 4.61 (d, J = 3.5 Hz, 2H), 4.25-4.10 (m, 5H), 3.12 (dd, J = 10.8, 5.9 Hz, 1H), 3.01 (m, 2H), 2.03 (m, 2H), 1.67 (m, 4H), 1.48 (m, 8H), 1.23-0.86 (m, 18H).

Step 2 Synthesis of Compound 62a and Compound 62b

The racemate compound 62 was separated with reverse phase preparative chromatography to obtain the target product 62a (retention time: 9.07 min, relative content: 48.6%) and 62b (retention time: 10.11 min, relative content: 51.4%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 63: Preparation of (2R,3R,4R,5R)-2-(4-aminopyrrolo[2,1-f][1,2,4]triazin-7-yl)-2-cyano-5-((((((S)-1-cyclobutoxy-1-oxo-3-phenylpropan-2-yl)amino)(phenoxy)phosphoryl)oxy)methyl)tetrahydrofuran-3,4-diyl bis(2-methylpropionate) (Compound 63),**

**(2R,3R,4R,5R)-2-(4-aminopyrrolo[2,1-f][1,2,41triazin-7-yl)-2-cyano-5-((((R)-(((S)-1-cyclobutoxy-1-oxo-3-phenylpropan-2-yl)amino)(phenoxy)phosphoryl)oxy)methyl)tetrahydrofuran-3,4-diyl bis(2-methylpropionate) (Compound 63a) and**

**(2R,3R,4R,5R)-2-(4-aminopyrrolo[2,1-f][1,2,4]triazin-7-yl)-2-cyano-5-((((S)-(((S)-1-cyclobutoxy-1-oxo-3-phenylpropan-2-yl)amino)(phenoxy)phosphoryl)oxy)methyl)tetrahydrofuran-3,4-diyl bis(2-methylpropionate) (Compound 63b)**

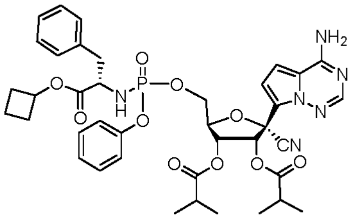

63

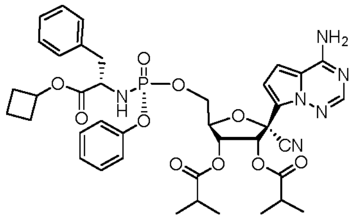

63a

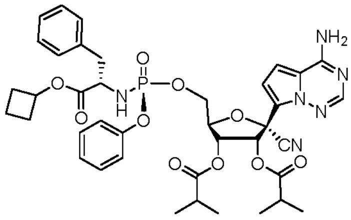

63b

The following synthetic routes were adopted:

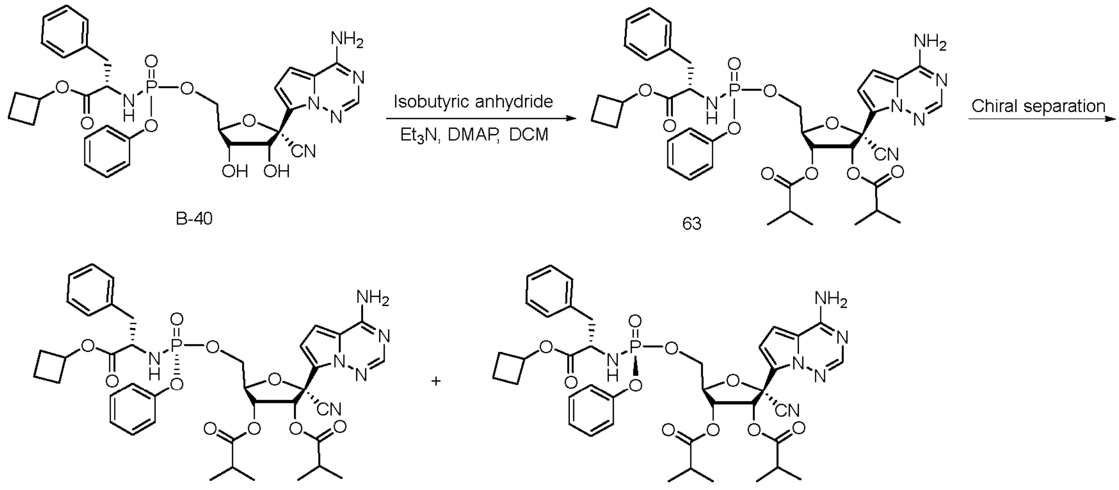


B-40

63

63a

\+

63b

Step 1 Synthesis of Compound 63

The intermediate B-40 (201 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 134 mg of the product (yield: 55%). LC-MS (APCI): m/z=789.3 $(M+1)^+$. $^1H$ NMR (400 MHz, $CDCl_3$) $\delta$ 7.92 (s, 1H), 7.21 (dt, J = 16.8, 7.5 Hz, 5H), 7.16-7.05 (m, 3H), 7.05-6.98 (m, 2H), 6.87 (d, J = 4.6 Hz, 1H), 6.51 (d, J = 4.7 Hz, 1H), 6.22 (d, J = 5.9 Hz, 1H), 5.90 (s, 2H), 5.52 (dd, J = 5.8, 4.0 Hz, 1H), 4.50 (d, J = 3.6 Hz, 1H), 4.36-4.28 (m, 1H), 4.25-4.11 (m, 2H), 3.96-3.77 (m, 3H), 2.92 (d, J = 6.2 Hz, 2H), 2.70-2.53 (m, 2H), 1.78 (s, 2H), 1.41 (dt, J = 12.4, 6.3 Hz, 1H), 1.27-1.20 (m, 10H), 1.19 (dd, J = 7.0, 1.2 Hz, 6H), 0.82 (t, J = 7.4 Hz, 6H).

Step 2 Synthesis of Compound 63a and Compound 63b

The racemate compound 63 was separated with reverse phase preparative chromatography to obtain the target product 63a (retention time: 9.62 min, relative content: 54.9%) and 63b (retention time: 10.20 min, relative content: 45.1%).

Conditions of chromatographic separation:

Column: Waters, Prep C18 OBD™, 150×19 mm, 5 μm
Column temperature: 30°C
Flow rate: 1.0 mL/min
Wavelength of UV detection: 220 nm
Mobile phase: pure water:acetonitrile=40:60

**Example 64: Preparation of (2R,3R,4R,5R)-2-(4-aminopyrrolo[2,1-f][1,2,4]triazin-7-yl)-2-cyano-5-(((((S)-1-(2-methoxy-2-methylpropoxy)-1-oxo-3-phenylpropan-2-yl)amino)(phenoxy)phosphoryl)oxy)methyl)tetrahydrofuran-3,4-diyl bis(2-methylpropionate) (Compound 64),**

**(2R,3R,4R,5R)-2-(4-aminopyrrolo[2,1-f][1,2,4]triazin-7-yl)-2-cyano-5-((((R)-(((S)-1-(2-methoxy-2-methylpropoxy)-1-oxo-3-phenylpropan-2-yl)amino)(phenoxy)phosphoryl)oxy)methyl)tetrahydrofuran-3,4-diyl bis(2-methylpropionate) (Compound 64a) and**

**(2R,3R,4R,5R)-2-(4-aminopyrrolo[2,1-f][1,2,4]triazin-7-yl)-2-cyano-5-((((S)-(((S)-1-(2-methoxy-2-methylpropoxy)-1-oxo-3-phenylpropan-2-yl)amino)(phenoxy)phosphoryl)oxy)methyl)tetrahydrofuran-3,4-diyl bis(2-methylpropionate) (Compound 64b)**

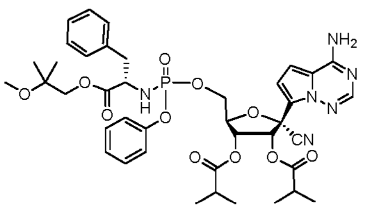

64

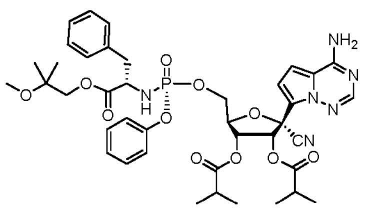

64a

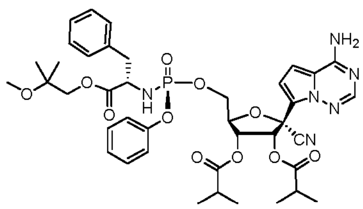

64b

The following synthetic routes were adopted:

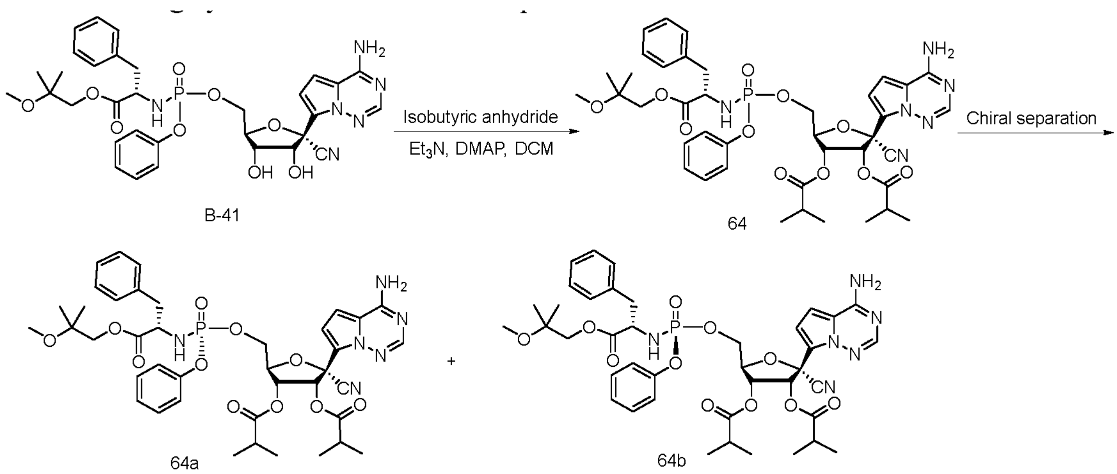


B-41

64

64a

+

64b

Step 1 Synthesis of Compound 64

The intermediate B-41 (211 mg, 0.31 mmol) was added to a reaction flask, followed by the addtion of 10 mL of anhydrous dichloromethane to dissolve. Isobutyric anhydride (123 mg, 0.78 mmol), triethylamine (111 mg, 1.1 mmol) and DMAP (19.6 mg, 0.16 mmol) were added successively. The mixture was stirred at room temperature under nitrogen atmosphere overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 84 mg of the target product (yield: 33%). LC-MS (APCI): m/z=821.2 $(M+1)^+$. $^1H$ NMR (400 MHz, $CDCl_3$) $\delta$ 7.91 (s, 1H), 7.19 (dt, J = 17.5, 7.5 Hz, 5H), 7.16-7.03 (m, 3H), 7.05-6.98 (m, 2H), 6.87 (d, J = 4.6 Hz, 1H), 6.51 (d, J = 4.7 Hz, 1H), 6.22 (d, J = 5.9 Hz, 1H), 5.90 (s, 2H), 5.52 (dd, J = 5.8, 4.0 Hz, 1H), 4.50 (d, J = 3.6 Hz, 1H), 4.36-4.28 (m, 1H), 4.25-4.11 (m, 2H), 3.96-3.77 (m, 3H), 2.92 (d, J = 6.2 Hz, 2H), 2.70-2.53 (m, 2H), 1.78 (s, 2H), 1.40 (dt, J = 12.4, 6.3 Hz, 1H), 1.27-1.20 (m, 10H), 1.19 (dd, J = 7.0, 1.2 Hz, 6H), 0.82 (t, J = 7.4 Hz, 6H).

Step 2 Synthesis of Compound 64a and Compound 64b

The racemate compound 64 was separated with reverse phase preparative chromatography to obtain the target product 64a (retention time: 9.61 min, relative content: 50.7%) and 64b (retention time: 10.14 min, relative content: 49.3%).

Conditions of chromatographic separation:

Column: Waters, Prep C18 OBD™, 150×19 mm, 5 μm

Column temperature: 30°C
Flow rate: 1.0 mL/min
Wavelength of UV detection: 220 nm
Mobile phase: pure water:acetonitrile=40:60

**Example 65: Preparation of (2R,3R,4R,5R)-2-(4-amino-6-bromopyrrolo[2,1-f][1,2,4]triazin-7-yl)-5-(((bis(((isopropoxycarbonyl)oxy)methoxy)phosphoryl)oxy)methyl)-2-cyanotetrahydrofuran-3,4-diyl bis(2-methylpropionate) (Compound 65)**

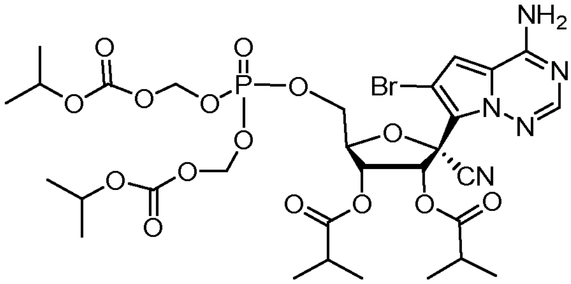

The following synthetic routes were adopted:

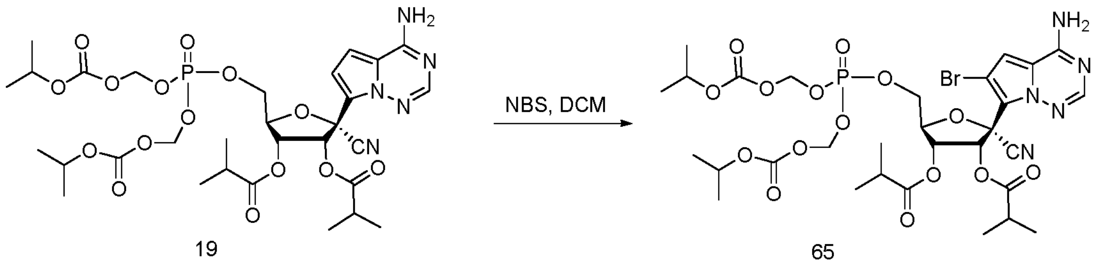


19 65

The compound 19 (100 mg, 0.13 mmol) was added to a reaction flask, followed by the addtion of 5 mL of anhydrous dichloromethane to dissolve. N-Bromosuccinimide (28.7 mg, 0.16 mmol) was added in portions at room temperature under nitrogen atmosphere. The mixture was stirred at room temperature overnight. After completion of the reaction as monitored by TLC, the mixture was quenched with a small amount of water and extracted with dichloromethane 3-4 times. The organic layers were combined, washed with saturated saline solution, concentrated, and purified by silica gel column chromatography to obtain 39.5 mg of the target product (yield: 37%). LC-MS (APCI): m/z=822.5 $(M+1)^+$. $^1H$ NMR (400 MHz, DMSO-$d_6$) δ 8.36 (s, 1H), 7.21 (s, 1H), 6.14 (d, J = 6.0 Hz, 1H), 5.58 - 5.45 (m, 4H), 5.42 (dd, J = 5.7, 4.1 Hz, 1H), 4.82 (dt, J = 12.5, 6.2 Hz, 2H), 4.60 (d, J = 3.7 Hz, 1H), 4.36 (t, J = 5.2 Hz, 2H), 2.64 (dt, J = 14.0, 7.0 Hz, 2H), 1.22 (d, J = 6.2 Hz, 12H), 1.17 (dd, J = 8.2, 7.2 Hz, 6H), 1.11 (d, J = 7.2 Hz, 6H).

**Biological Activity Testing**

(1) SARS-CoV-2 replicon reporter assay

The anti-SARS-CoV-2 (WT or Omicron mutant) activity of the compounds of the present invention was evaluated using an in vitro SARS-CoV-2 replicon reporter assay, based on changes in reporter gene expression levels of the pseudovirus (replication-competent only) and cell viability. Remdesivir was selected as the positive control compound.

Method: After electroporation of SARS-CoV-2 replicon RNA into Huh7 (human hepatoma) cells, the cells were seeded at an appropriate density into microplates containing serially diluted test compounds (8 concentration points, duplicate wells). An HPE control (cells electroporated with SARS-CoV-2 replicon but without compound treatment) was set up. Cells were cultured for 1 day in a 5% $CO_2$ incubator at 37°C. GFP-expressing cells in each well were quantified. For cytotoxicity assessment (performed under identical conditions as the antiviral assay), cell viability was measured using the CellTiter-Glo kit (Promega). Compound antiviral activity and cytotoxicity were calculated based on dose-dependent effects on pseudovirus reporter expression and cell viability, respectively. Using GraphPad Prism, nonlinear regression analysis was performed on the inhibition rates and cell viability rates to calculate the half-maximal effective concentration

($EC_{50}$) and half-maximal cytotoxic concentration ($CC_{50}$) values of the compounds, with activity grades defined as: A represents $EC_{50} \leq 5nM$, B represents $EC_{50}$ of 5-10 nM, C represents $EC_{50}$ of 10-50 nM, D represents $EC_{50}$ of 50-500 nM, and E represents $EC_{50} \geq 500$ nM. Results demonstrated that the compounds of the present invention exhibit potent anti-SARS-CoV-2 (WT or Omicron mutant) activity. Representative compounds' inhibitory effects on Huh7 cell proliferation are shown in the following table:

| Compound number | SARS-CoV-2 Replicon (Huh-7cell) $EC_{50}$ |
|---|---|
| 1a | C |
| 1b | B |
| 2a | B |
| 2b | A |
| 3a | C |
| 3b | A |
| 4a | D |
| 4b | D |
| 5a | D |
| 5b | B |
| 6a | B |
| 6b | A |
| 7a | C |
| 7b | C |
| 9a | B |
| 9b | A |
| 10a | C |
| 10b | A |
| 11a | C |
| 11b | A |
| 12a | D |
| 12b | D |
| 13a | A |
| 13b | A |
| 14 | C |
| 16a | D |
| 16b | D |
| 17 | A |
| 18 | A |
| 19 | A |
| 20 | C |
| 21 | A |
| 22a | A |
| 22b | B |
| 23a | A |
| 23b | B |

(continued)

| Compound number | SARS-CoV-2 Replicon (Huh-7cell) $EC_{50}$ |
|---|---|
| 24a | A |
| 24b | C |
| 26a | B |
| 27 | C |
| 28 | C |
| 29 | C |
| 30 | A |
| 31a | C |
| 31b | C |
| 32a | C |
| 32b | C |
| 36 | D |
| 38a | D |
| 38b | C |
| 39a | D |
| 39b | C |
| 40a | C |
| 40b | D |
| 41a | D |
| 41b | B |
| 43a | B |
| 43b | B |
| 44a | B |
| 44b | C |
| 45 | A |
| 46a | D |
| 46b | D |
| 51 | D |
| 52a | D |
| 52b | D |
| 53a | D |
| 53b | C |
| 54a | C |
| 54b | A |
| 60a | D |
| 60b | A |
| 62a | D |
| 62b | C |
| 63a | C |

(continued)

| Compound number | SARS-CoV-2 Replicon (Huh-7cell) $EC_{50}$ |
|---|---|
| 63b | A |
| 64a | A |
| 64b | A |
| 65 | D |

(2) Vero E6 cytopathic effect (CPE) assay

The anti-SARS-CoV-2 (WT or Omicron mutant) activity and cytotoxicity of the compounds were evaluated using an in vitro Vero E6 (African green monkey kidney cells) CPE assay, based on the inhibition rate (%) of virus-induced cytopathic effects and cell viability (%). Remdesivir was selected as the positive control compound.

Method: Cells were seeded in microplates at an appropriate density and cultured overnight in a 5% $CO_2$ incubator at 37°C. The following day, two-fold serially diluted compounds (6 concentration points, duplicate wells) and virus were added. Cell control (cells, without compound treatment or viral infection), virus control (cells with viral infection, without compound treatment), and culture medium control (only with culture medium) were set up. The final concentration of DMSO in the culture medium was 0.5%. Cells were cultivated in an incubator for 3 days. Cytotoxicity testing followed identical conditions without viral infection. The cell viability was measured using the CellTiter-Glo kit (Promega). The antiviral activity and cytotoxicity of the compounds were determined by the inhibition rate (%) of virus-induced cytopathic effects and cell viability (%) at different compound concentrations, respectively. Using GraphPad Prism, nonlinear regression analysis was performed on the inhibition rates and cell viability rates to calculate the half-maximal effective concentration ($EC_{50}$) and half-maximal cytotoxic concentration ($CC_{50}$) values of the compounds.

Results demonstrated that the compounds of the present invention exhibit potent anti-SARS-CoV-2 (WT or Omicron mutant) activity and selectivity.

(3) Calu-3 cytopathic effect assay

The anti-SARS-CoV-2 (WT or Omicron mutant) activity and cytotoxicity of the compounds of the present invention were evaluated using an in vitro Calu-3 (human lung adenocarcinoma cells) cytopathic effect (CPE) assay, based on the inhibition rate (%) of virus-induced cytopathic effects and cell viability (%). Remdesivir was selected as the positive control compound. Method: Cells were seeded in microplates at an appropriate density and cultured overnight in a 5% $CO_2$ incubator at 37°C. The following day, two-fold serially diluted compounds (6 concentration points, duplicate wells) and virus were added. Cell control (cells, without compound treatment or viral infection), virus control (cells with viral infection, without compound treatment), and culture medium control (only with culture medium) were set up. The final concentration of DMSO in the culture medium was 0.5%. Cells were cultivated in an incubator for 3 days. Cytotoxicity testing followed identical conditions without viral infection. The cell viability was measured using the CellTiter-Glo kit (Promega). The antiviral activity and cytotoxicity of the compounds were determined by the inhibition rate (%) of virus-induced cytopathic effects and cell viability (%) at different compound concentrations, respectively. Using GraphPad Prism, nonlinear regression analysis was performed on the inhibition rates and cell viability rates to calculate the half-maximal effective concentration ($EC_{50}$) and half-maximal cytotoxic concentration ($CC_{50}$) values of the compounds.

Results demonstrated that the compounds of the present invention exhibit potent anti-SARS-CoV-2 (WT or Omicron mutant) activity.

(4) ARS-CoV-2 immunofluorescence assay

The anti-SARS-CoV-2 (WT or Omicron mutant) activity and cytotoxicity of the compounds of the present invention were evaluated using an in vitro Vero cell immunofluorescence assay by detecting viral nucleocapsid (N) protein expression and calculating the inhibition rate (%) of viral replication. Remdesivir was selected as the positive control compound.

Method: Vero cells were seeded in microplates at an appropriate density and cultured overnight in a 5% $CO_2$ incubator at 37°C. The following day, two-fold serially diluted compounds (10 concentration points, duplicate wells) and virus were added. After 24 hours of infection, immunofluorescence assay was used to detect the expression of virus N protein. The number of cells in the cell test wells was detected by Hoechst staining method. The cytotoxicity was calculated based on the number of cells in different wells. The antiviral activity of the compound was represented by the inhibition rate (%) of viral replication at various concentrations. The $EC_{50}$ and $CC_{50}$ values of the compound were calculated using

GraphPad Prism or XLfit software. Results demonstrated that the compounds of the present invention exhibit potent anti-SARS-CoV-2 (WT or Omicron mutant) activity and selectivity.

(5) RSV CPE assay

HEp-2 (Human laryngeal epidermoid cancer cells) cells were seeded in microplates at an appropriate density and cultured overnight in a 5% $CO_2$ incubator at 37°C. On the following day, gradient-diluted test compounds (8 concentration gradients, in duplicate wells) and RSV A2 virus were added. ALS-8112 or Ziresovir was selected as the positive control compound. Cell control (cells, without compound treatment or viral infection) and virus control (cells with viral infection, without compound treatment) were set up. The final concentrations of DMSO in the culture medium were both 0.5%. Cells were cultivated in an incubator at 37°C, 5% $CO_2$ for 5 days. Cytotoxicity testing followed identical conditions without viral infection. The cell viability was measured using the kit CCK8. The antiviral activity and cytotoxicity of the compounds were determined by the inhibition rate (%) of virus-induced cytopathic effects and HEp-2 cell viability (%) at different compound concentrations, respectively. The inhibition rates and cell viability rates of the compounds were subjected to nonlinear regression analysis using GraphPad Prism to calculate the EC50 and CC50 values, with activity grades defined as: A represents $EC_{50} \leq 100$ nM, B represents $EC_{50}$ of 100-200 nM, C represents $EC_{50}$ of 200-500 nM, D represents $EC_{50}$ of 500-1000 nM, and E represents $EC_{50} \geq 1000$ nM.

Results demonstrated that the compounds of the present invention exhibit potent antiviral activity and selectivity against RSV A2 virus. Representative compounds' inhibitory effects on HEp-2 cell proliferation are shown in the following table:

| Compound number | RSV A2 (Hep-2 cell) $EC_{50}$ |
|---|---|
| 1a | B |
| 1b | B |
| 3a | C |
| 3b | B |
| 5a | C |
| 5b | B |
| 7a | C |
| 7b | B |
| 11b | C |
| 13b | C |
| 17 | B |
| 19 | C |
| 20 | E |
| 24a | E |
| 26 | E |
| 27 | C |
| 28 | E |
| 29 | D |
| 30 | C |
| 31b | C |
| 32b | D |
| 38b | A |
| 39a | A |
| 39b | A |
| 41b | A |

(continued)

| Compound number | RSV A2 (Hep-2 cell) $EC_{50}$ |
|---|---|
| 53a | A |
| 53b | A |
| 54a | B |
| 54b | A |
| 55a | B |
| 55b | B |
| 56a | C |
| 56b | B |
| 57a | D |
| 57b | C |
| 58a | C |
| 58b | A |
| 59a | C |
| 59b | A |
| 60a | C |
| 60b | A |
| 61a | E |
| 61b | D |
| 62a | C |
| 62b | A |
| 63a | C |
| 63b | A |
| 64a | B |
| 64b | A |

(6) RSV ELISA assay

HEp-2 cells were seeded in microplates at an appropriate density and cultured overnight in a 5% $CO_2$ incubator at 37°C. On the following day, gradient-diluted test compounds (8 concentration gradients, in duplicate wells) and RSV virus were added. ALS-8112 or Ziresovir was selected as the positive control compound. Cell control (cells, without compound treatment or viral infection) and virus control (cells with viral infection, without compound treatment) were set up. The final concentrations of DMSO in the culture medium were both 0.5%. Cells were cultivated in an incubator at 37°C, 5% $CO_2$ for 5 days. After the cells were fixed, ELISA was used to detect the protein expression of RSV in the cells. Cytotoxicity testing followed identical conditions without viral infection. The cell viability was measured using the kit CCK8. The antiviral activity and cytotoxicity of the compounds were determined by the inhibition rate (%) of viral replication and HEp-2 cell viability (%) at different compound concentrations, respectively. Using GraphPad Prism, nonlinear regression analysis was performed on the inhibition rates and cell viability rates to calculate $EC_{50}$ and $CC_{50}$ values of the compounds.

Results demonstrated that the compounds of the present invention exhibit potent antiviral activity and selectivity against RSV virus.

(7) RSV plaque reduction assay

HEp-2 cells were seeded in microplates at an appropriate density and cultured overnight in a 5% $CO_2$ incubator at 37°C. On the following day, gradient-diluted test compounds (5 concentration gradients, in duplicate wells) and RSV virus were added. ALS-8112 or Ziresovir was selected as the positive control compound. Virus control (cells viral infection,

without compound treatment) was set up. The final concentrations of DMSO in the culture medium were both 0.5%. The infection supernatant was aspirated 1-2 hours post-infection and replaced with maintenance medium containing the corresponding concentrations of compounds. Cells were cultivated in an incubator at 37°C, 5% $CO_2$ for 3-4 days. The cells were fixed and stained, followed by quantification of viral plaques per well. The antiviral activity of the compound was represented by the inhibition rate (%) of viral plaque formation at various concentrations. Nonlinear fitting analysis was performed on the inhibition rate of the compound using GraphPad Prism, and the $EC_{50}$ value of the compound was calculated.

Results demonstrated that the compounds of the present invention exhibit potent antiviral activity against RSV virus.

(8) Dengue virus plaque reduction assay

Vero cells were seeded in 6-well plates at an appropriate density and cultured overnight at 37°C with 5% $CO_2$. The following day, two-fold serially diluted compounds (5 concentration points, single well) and virus (50 PFU/mL) were added. After 2-4 hours of infection, the supernatant was aspirated and replaced with maintenance medium containing corresponding concentrations of compounds. The final concentration of DMSO in the cell culture was 0.5%. After 7 days of incubation at 37°C with 5% $CO_2$, cells were fixed and stained with crystal violet, followed by plaque counting in each well. Cytotoxicity testing was performed in parallel using 96-well plates. Cell viability was measured using cell viability detection reagents, with raw data used for calculating both the inhibitory activity of compounds against viral plaque formation and cytotoxicity. Dose-response curves were analyzed and $EC_{50}$ and $CC_{50}$ values were calculated using GraphPad Prism software.

Results demonstrated that the compounds of the present invention exhibit potent antiviral activity and selectivity against Dengue virus.

(9) Zika virus cytopathic effect (CPE) assay

Huh7 cells were seeded in 96-well plates at an appropriate density and cultured overnight at 37°C with 5% $CO_2$. The following day, two-fold serially diluted compounds (8 concentrations, duplicate wells) and virus were added. Cell control (uninfected) and virus control were set up. The final concentration of DMSO in the cell culture was 0.5%. Cells were cultured at 37°C with 5% $CO_2$ for 3 days until distinct cytopathic effects (CPE) became evident in the virus control wells. Cytotoxicity testing followed identical conditions without viral infection. Cell viability was measured using cell viability detection reagents, with raw data used for calculating antiviral activity and cytotoxicity. Dose-response curves were analyzed and $EC_{50}$ and $CC_{50}$ values were calculated using GraphPad Prism software.

Results demonstrated that the compounds of the present invention exhibit potent antiviral activity and selectivity against Zika virus.

(10) Evaluation of in vivo pharmacological efficacy of the compound of the present invention using ACE2 transgenic mouse infection model

Model building

Animal species and strains: C57BL/6-Tgtn(CAG-humanACE2-IRES-Luciferase-WPRE-polyA)Smoc (NM-TG-200002) ACE2 transgenic humanized mice, male, 21-week-old. Facilities: SPF Animal house (AAALAC), BSL-3 Laboratory (Animal Biosafety Level III Laboratory of the Second Military Medical University of the People's Liberation Army, No.: Jun ABSL3-003).

Strain: SARS-CoV-2 virus (Pubmed No: MT627325).

Infection method: Intranasal inoculation with $1\times10^4$ PFU SARS-CoV-2 virus suspended in serum-free DMEM medium.

Viral load: Mean greater than 1e7 copies/g.

Experimental Protocol:

Group design: Model control group, positive control group, test compound group (low/medium/high dose), 5 animals/group;
Administration time: 0-3 days after viral infection;
Administration mode: Gavage;
Dosing frequency/duration: Twice daily for 3 consecutive days;

Sample collection: On day 4, the mice were treated and lung tissues were collected;
Sample testing: the virus titers in the above lung tissue samples were detected.

| | | |
|---|---|---|
| Day 0 | P3 Laboratory | The mice were placed into the laboratory environment for adaptation, and the body weights were measured. |
| Day 1 | P3 Laboratory | The body weights were measured, and infected with euvirus, and administered once immediately 1 hour after infection, and administered again after 6 hours. |
| Day 2-3 | P3 Laboratory | The body weights were measured, the status of mice was observed twice, and administered twice a day. |
| Day 4-6 | P3 Laboratory | 72 hours after infection, lung tissue samples were collected; Half of the pulmonary lobes were fixed for HE pathology; mRNA was extracted from the other half of the pulmonary lobes and the lung load was measured. |

Clinical observation and detection indicators:

clinical signs were observed, the body weights were measured, and the survival rate was recorded;
72 hours after infection, samples were taken to detect the viral load in lung tissues;
The lung tissues were fixed for the pulmonary HE pathology.

The experimental results demonstrated that the compound of the present invention exhibits excellent in vivo potency and low toxicity.

(11) Metabolic stability evaluation

Metabolic stability is generally used to describe the rate and extent of compound metabolism, and is one of the main factors affecting its pharmacokinetic properties. Many compounds are substrates for CYP450 enzymes and other drug metabolizing enzymes, and liver microsomes are CYP450-rich systems. The purpose of this experiment is to study the in vitro metabolic stability of the compound by incubating the compound of the present invention with human, rat, and mouse liver microsomes, respectively and detect the remaining proportion of the compound using LC-MS/MS.

①Formulation of solution

Phosphate buffered saline (PBS): 150 mL of the pre-formulated $KH_2PO_4$(0.5M) solution and 700 mL of $K_2HPO_4$ (0.5M) solution were taken and mixed, the pH value of the mixed solution was adjusted to 7.4 with $K_2HPO_4$ (0.5M) solution, and stored at 4°C as a 5-fold concentration PBS for later use. It was diluted 5 times with ultrapure water before use, and 3.3 mM of magnesium chloride was added to obtain phosphate buffered saline PBS (100 mM).
NADPH regeneration system solution: NADPH solution containing 6.5 mM of NADP, 16.5 mM of G-6-P, 3 U/mL of G-6-P D was formulated with 5mL of PBS.
Internal standard termination solution: 50 ng/mL of propranolol hydrochloride and 200 ng/mL of Tolbutamide were formulated with acetonitrile as the internal standard working solutions.
Human liver microsomal solution: 0.31 mL of human liver microsomes (25 mg/mL) was taken and added to 0.961 mL of PBS (pH 7.4) to mix well, so as to obtain a human liver microsome diluent with a protein concentration of 0.625 mg/mL.
Rat liver microsomal solution: 0.31 mL of rat liver microsomes (25 mg/mL) was taken and added to 0.961 mL of PBS (pH 7.4) to mix well, so as to obtain a rat liver microsome diluent with a protein concentration of 0.625 mg/mL.
Mouse liver microsomal solution: 0.31 mL of mouse liver microsomes (25 mg/mL) was taken and added to 0.961 mL of PBS (pH 7.4) to mix well, so as to obtain a mouse liver microsome diluent with a protein concentration of 0.625 mg/mL.
Sample working solution: The compound of the present invention, non-deuterated compound powder, and positive controls (dextromethorphan powder and omeprazole powder) were prepared as 10 mM stock solutions in DMSO. These were then diluted with 70% acetonitrile-water to obtain 0.25 mM sample working solutions.

②Sample incubation

398 μL of human liver microsomal diluent was taken and added into a 96-well incubation plate (N=2), followed by 2 μL of 0.25 mM test compound or dextromethorphan, and mixed thoroughly. 398 μL of SD rat liver microsomal diluent was

taken and added into a 96-well incubation plate (N=2), followed by 2 μL of 0.25 mM test compound or omeprazole, and mixed thoroughly.

300 μL of pre-chilled stop solution was added to each well of a 96-well deep-well plate placed on ice (termination plate).

The 96-well incubation plate and NADPH regeneration system were placed in a 37°C water bath with 100 rpm shaking for 5 min. 80 μL of incubation mixture was transferred from each well of the incubation plate to the termination plate, mixed, and supplemented with 20 μL of NADPH regeneration system solution (0 min sample). 80 μL of NADPH regeneration system solution was then added to each well of the incubation plate to initiate the reaction (timing started). The reaction concentration of the compound to be tested was 1 μM, and the protein concentration was 0.5 mg/mL.

At 10, 30, and 90 minutes into the reaction, 100 μL of the reaction solution was collected and transferred to the termination plate, followed by vortexing for 3 minutes to stop the reaction.

The termination plate was centrifuged at 5000 rpm and 4°C for 15 minutes. 200 μL of supernatant was taken and added to a 96-well plate prefilled with 200 μL of ultrapure water, mixed well and analyzed by LC-MS/MS (injection volume: 10 μL).

③Analysis method of sample

The LC-MS/MS system was employed to detect peak areas of the test compound, dextromethorphan, omeprazole, and internal standard, followed by calculation of the compound-to-internal standard peak area ratio.

④Data processing

Peak areas of samples and internal standards were acquired using the mass spectrometer and Analyst software. The remaining compound percentage (R%) versus time was plotted using GraphPad Prism 7.0 software with a mono-exponential decay model to determine the substrate elimination rate constant (K):

$$Ct/C0 = \exp(-K*t)$$

the half-life period $T_{1/2}$ and intrinsic clearance rate $CL_{int}$ were calculated according to the following formula, wherein V/M was equal to 1/C (protein).

$$T_{1/2} = -\frac{0.693}{Slope}, \quad CL_{int} = \frac{0.693}{t_{1/2}} \bullet \frac{V}{M},$$

$t_{1/2}$(min); $CL_{int}$(μL/min/mg).

Experimental results demonstrated that the compound of the present invention exhibits excellent metabolic stability.

(12) Rat pharmacokinetic study

Male rats (n=3 per group) were fasted for 12 hours prior to dosing with free access to water, and received standardized feeding 4 hours post-dose. The vehicle consisted of 5% DMSO + 5% EtOH + 40% PEG300 + 50% saline. The intravenous injection group received 2 mg/kg of the test compound, with blood collected from the jugular vein at 0.083 h, 0.25 h, 0.5 h, 1 h, 2 h, 4 h, 6 h, 8 h, and 24 h after administration. The oral gavage group received 10 mg/kg of the test compound, with blood collected from the jugular vein at 0.25 h, 0.5 h, 1 h, 2 h, 4 h, 6 h, 8 h, and 24 h after administration. Approximately 0.2 mL of each sample was collected, anticoagulated with heparin sodium, and placed on wet ice after collection. The plasma was separated by centrifugation within 1 hour (centrifugation conditions: 6000 g, 3 minutes, 2-8°C). For PK blood sample analysis, the hydrolyzed parent drug GS-441524 was measured. Plasma samples were stored in a freezer at -80°C before analysis. Pharmacokinetic parameters were calculated using Phoenix WinNonlin 8.2.0 based on plasma drug concentration data at different time points.

Experimental results demonstrated that the compound of the present invention exhibits superior pharmacokinetic properties in animals. Following a single intravenous bolus administration of 2 mg/kg of the representative compound of the present invention to male rats, the peak plasma concentration ($C_{max}$) of the representative compound ranged from 45-1000 ng/mL, with an area under the plasma concentration-time curve from time zero to the last quantifiable time point ($AUC_{0-last}$) of 150-850 ng·h/mL. After a single oral administration of 10 mg/kg of the representative compound of the present invention to male rats, the peak plasma concentration ($C_{max}$) of the representative compound ranged from 20-390

ng/mL, with an area under the plasma concentration-time curve from time zero to the last quantifiable time point ($AUC_{0\text{-last}}$) of 155-1646 ng·h/mL.

The above content provides further detailed explanation of the present invention in conjunction with specific preferred embodiments. It should not be construed that the practical implementation of the present invention is limited only to these descriptions. For those skilled in the art to which this invention pertains, any simple deductions or substitutions made without departing from the inventive concept shall all be considered as falling within the protection scope of the present invention.

## Claims

1. A compound of formula (I):

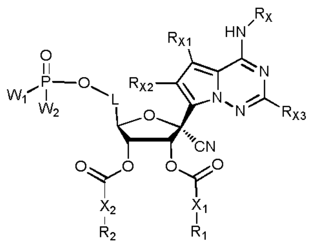

(I)

wherein,

$R_X$ is H or OH;
$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$;
wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;
$W_1$ is formula (A):

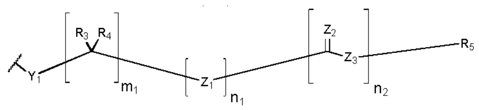

formula (A)

wherein,
$Y_1$ is O or S;
$Z_1$ is O, NH or S;
$Z_2$ is O, NH or S;
$Z_3$ is a bond, O, NH or S;
$m_1$ is 1, 2, 3, 4, 5 or 6;
$n_1$ is 0 or 1;
$n_2$ is 0 or 1;
each of $R_3$ and $R_4$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

$W_2$ is formula (B):

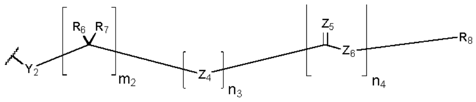

formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, any one of $W_1$ and $W_2$ together with the 3'-position C atom of glycosyl form $-Y_3-$, wherein $Y_3$ is O, NH or S; or, $W_1$ and $W_2$ together with the P atom to which they are attached, form $-Y_4(C(R_3R_4)_p)Y_4-$, wherein $Y_4$ is O, NH or S, and p is 2, 3, 4 or 5;

each R is independently D, halogen, $-R_a$, $-C(O)R_d$, $-C(O)OR_d$, $-C(O)NR_eR_f$, $-NR_eR_f$,$-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$,$-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$,$-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$,$-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$,$-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$,$-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;

or a tautomer, stereoisomer, prodrug, crystalline form, pharmaceutically acceptable salt, hydrate or solvate thereof.

**2.** The compound of claim 1, which is of formula (Ia) or formula (Ib):

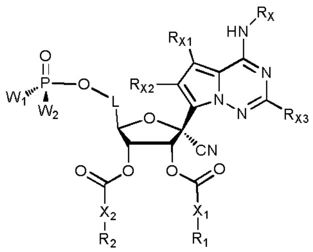

formula (Ia), or

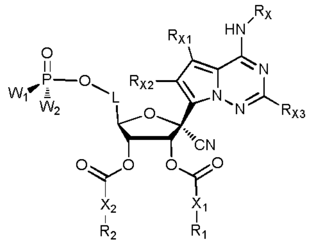

formula (Ib)

or a tautomer, stereoisomer, prodrug, crystalline form, pharmaceutically acceptable salt, hydrate or solvate thereof.

**3.** The compound of claim 1 or 2, or a tautomer, stereoisomer, prodrug, crystalline form, pharmaceutically acceptable salt, hydrate or solvate thereof, wherein $W_1$ is:

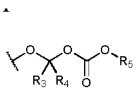
,
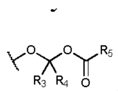
,
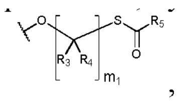
,
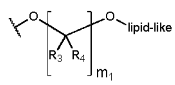
,
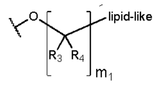
or
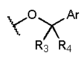
;

wherein, lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl and $C_{8-28}$ alkynyl are optionally substituted with one or more R;
Ar is $C_{6-10}$ aryl or 5-10 membered heteroaryl, wherein said $C_{6-10}$ aryl and 5-10 membered heteroaryl are optionally substituted with one or more R;
$R_3$, $R_4$, $R_5$, $m_1$ and R are as described in claim 1.

**4.** The compound of any one of claims 1 to 3, which is a compound of formula (II), (III) or (IV):

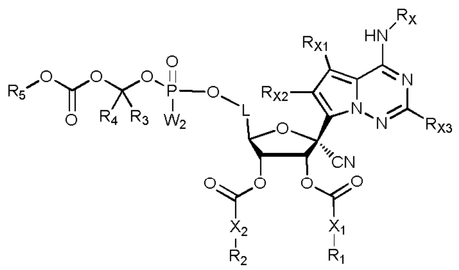

formula (II),

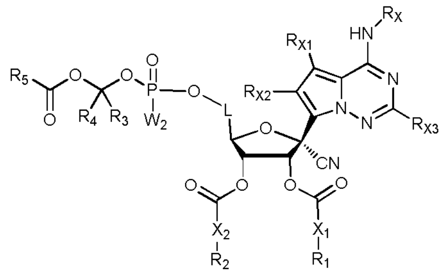

formula (III), or

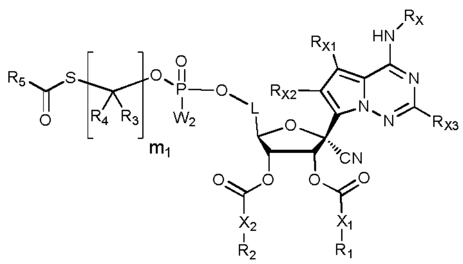

formula (IV)

wherein,

$R_X$ is H or OH;
$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_b$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or

$-S(O)_2R_a$;

wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration; $X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$m_1$ is 1, 2, 3, 4, 5 or 6;

$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_5$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

$W_2$ is formula (B):

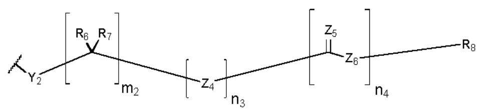

formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, $W_2$ together with the 3'-position C atom of glycosyl form $-Y_3-$, wherein $Y_3$ is O, NH or S;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered

heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$,$-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$,$-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;

or a tautomer, stereoisomer, prodrug, crystalline form, pharmaceutically acceptable salt, hydrate or solvate thereof.

**5.** The compound of claim 4, which is a compound of formula (IIa), formula (IIb), formula (IIIa), formula (IIIb), formula (IVa) or formula (IVb):

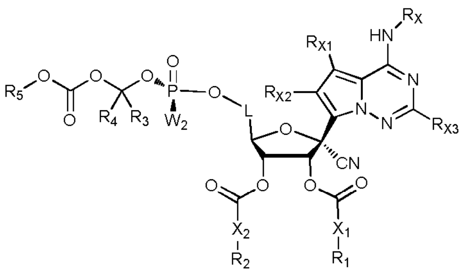

formula (IIa),

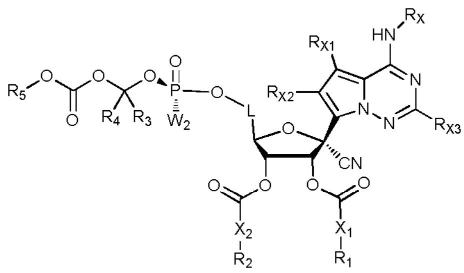

formula (IIb),

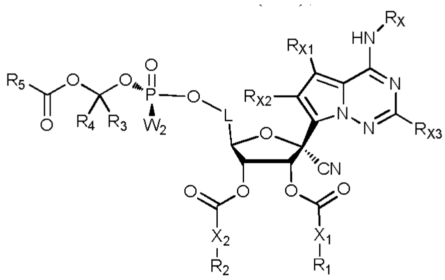

formula (IIIa),

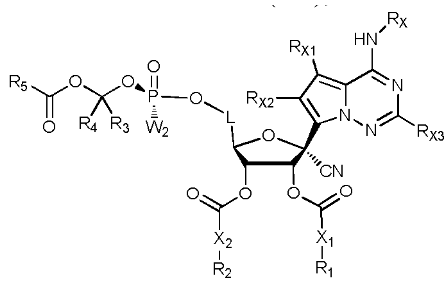

formula (IIIb)

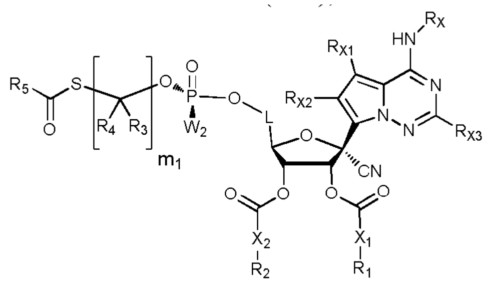

formula (IVa) or 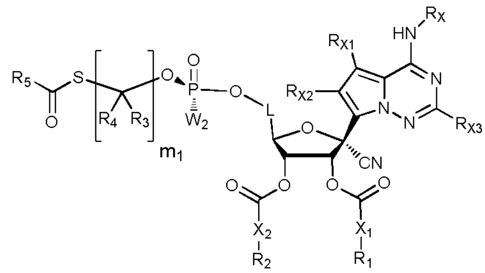 formula (IVb)

or a tautomer, stereoisomer, prodrug, crystalline form, pharmaceutically acceptable salt, hydrate or solvate thereof.

**6.** The compound of claim 4 or 5, or a tautomer, stereoisomer, prodrug, crystalline form, pharmaceutically acceptable salt, hydrate or solvate thereof, wherein:

$R_X$ is H or OH;
$R_{X1}$, $R_{X2}$ and $R_{X3}$ are independently H, D, halogen or $C_{1-6}$ alkyl, wherein said $C_{1-6}$ alkyl are optionally substituted with one or more deuterium atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
L is $CH_2$, $CD_2$ or CHD;
$m_1$ is 1, 2 or 3;
$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein said $C_{1-6}$ alkyl, $C_{3-6}$ cycloalkyl and 3-7 membered heterocyclyl are optionally substituted with one or more R';
$R_5$ is $C_{1-28}$ alkyl, $C_{1-28}$ haloalkyl, $C_{3-6}$ cycloalkyl, 3-7 membered heterocyclyl or $C_{2-28}$ alkenyl, wherein said $C_{1-28}$ alkyl, $C_{1-28}$ haloalkyl, $C_{3-6}$ cycloalkyl, 3-7 membered heterocyclyl and $C_{2-28}$ alkenyl are optionally substituted with one or more R;
$W_2$ is:

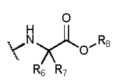 , 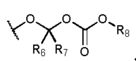 , 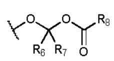 , 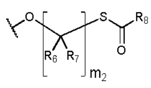 , 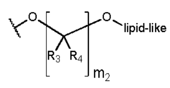 , 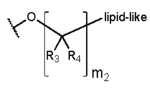 , 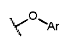 or 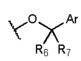 ;

wherein,
$m_2$ is 1, 2, 3, 4, 5 or 6;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl and $C_{8-28}$ alkynyl are optionally substituted with one or more R;
Ar is $C_{6-10}$ aryl or 5-10 membered heteroaryl, wherein said $C_{6-10}$ aryl and 5-10 membered heteroaryl are optionally substituted with one or more R;
each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full

deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration.

**7.** The compound of any one of claims 4 to 6, or a tautomer, stereoisomer, prodrug, crystalline form, pharmaceutically acceptable salt, hydrate or solvate thereof, wherein:

$W_2$ is

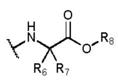 , 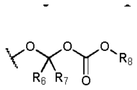 , 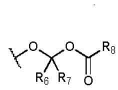 , 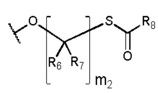 , 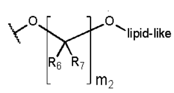 , 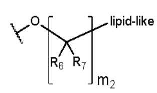 , 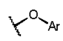

or

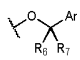 ;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ alkoxy, $C_{3-6}$ cycloalkyl and 3-7 membered heterocyclyl are optionally substituted with one or more R';

each $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein said $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl and $C_{8-28}$ alkynyl are optionally substituted with one or more R; Ar is $C_{6-10}$ aryl or 5-10 membered heteroaryl, wherein said $C_{6-10}$ aryl and 5-10 membered heteroaryl are optionally substituted with one or more R.

**8.** The compound of any one of claims 4 to 7, or a tautomer, stereoisomer, prodrug, crystalline form, pharmaceutically acceptable salt, hydrate or solvate thereof, wherein,

$W_2$ is

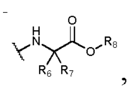 ,

wherein, $R_6$ is $C_{1-6}$ alkyl, and $R_7$ is H, or $R_6$ and $R_7$ together with the C atom to which they are attached, form a $C_{3-6}$ cycloalkyl, wherein said $C_{1-6}$ alkyl and $C_{3-6}$ cycloalkyl are optionally substituted with one or more groups selected from phenyl or -C(O)O$C_{1-6}$ alkyl;

$R_8$ is independently $C_{1-28}$ alkyl, $C_{3-6}$ cycloalkyl, 3-7 membered heterocyclyl or $C_{2-28}$ alkenyl, wherein said $C_{1-28}$ alkyl, $C_{3-6}$ cycloalkyl, 3-7 membered heterocyclyl or $C_{2-28}$ alkenyl is optionally substituted with one or more $C_{1-6}$ alkoxy;

preferably, $W_2$ is

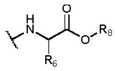;

preferably, $W_2$ is

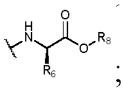;

preferably, $W_2$ is

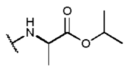, 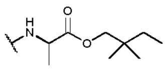, 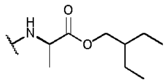, 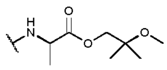, 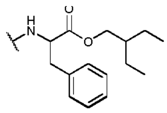,

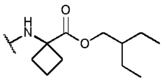, 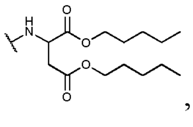, 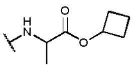, 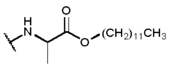 or 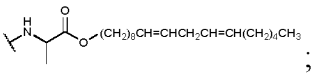;

preferably, $W_2$ is

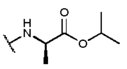, 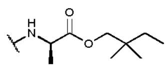, 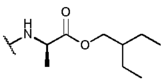, 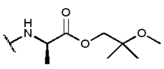, 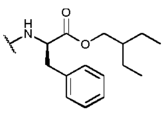,

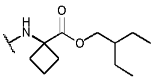, 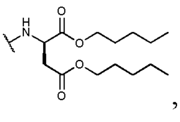, 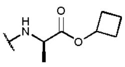, 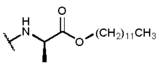 or 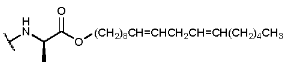;

preferably, $W_2$ is

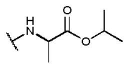, 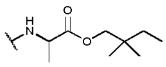, 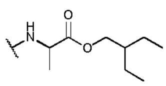, 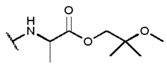, 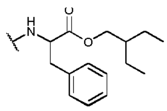,

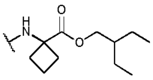, 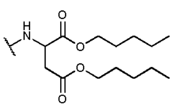, 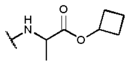, 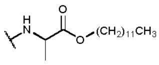, 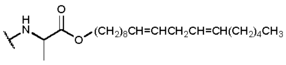, 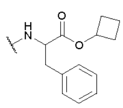,

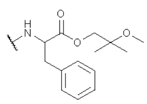 or 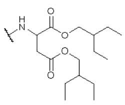;

preferably, $W_2$ is

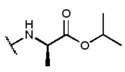, 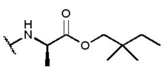, 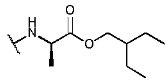, 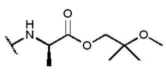, 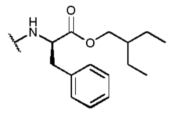,

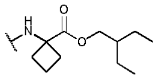, 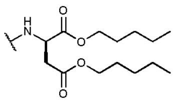, 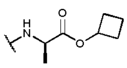, 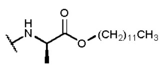, 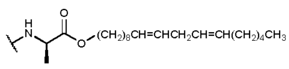, 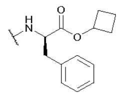,

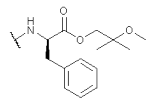 or 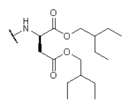.

**9.** The compound of any one of claims 4 to 7, or a tautomer, stereoisomer, prodrug, crystalline form, pharmaceutically acceptable salt, hydrate or solvate thereof, wherein,

$W_2$ is

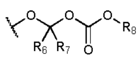,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

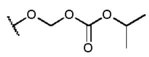.

**10.** The compound of any one of claims 4 to 7, or a tautomer, stereoisomer, prodrug, crystalline form, pharmaceutically acceptable salt, hydrate or solvate thereof, wherein,

$W_2$ is

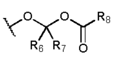,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

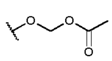, 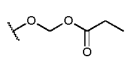 or 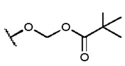;

preferably, $W_2$ is

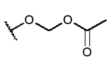, 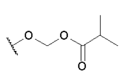 or 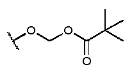.

**11.** The compound of any one of claims 4 to 7, or a tautomer, stereoisomer, prodrug, crystalline form, pharmaceutically acceptable salt, hydrate or solvate thereof, wherein,

$W_2$ is

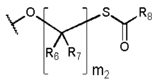,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

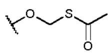, 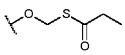, 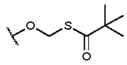, 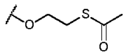, 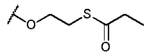 or 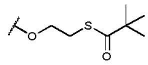.

**12.** The compound of any one of claims 4 to 7, or a tautomer, stereoisomer, prodrug, crystalline form, pharmaceutically acceptable salt, hydrate or solvate thereof, wherein,

$W_2$ is

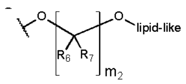,

wherein, $m_2$ is 2, 3 or 4;
$R_6$ and $R_7$ are independently H or $C_{1-6}$ alkoxy, wherein said $C_{1-6}$ alkoxy is optionally substituted with one or more groups selected from $C_{6-10}$ aryl or 5-10 membered heteroaryl, wherein said $C_{6-10}$ aryl or 5-10 membered heteroaryl is optionally substituted with one or more groups selected from halogen or $C_{1-6}$ alkoxy;
lipid-like is $C_{8-28}$ alkyl or $C_{8-28}$ alkenyl;
preferably, $W_2$ is

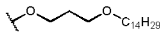, 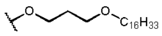, 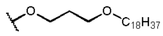, 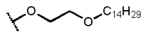, 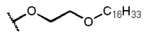,

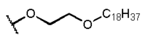, 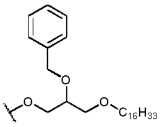, 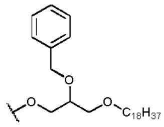, 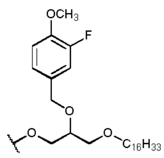 or 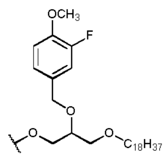;

preferably, $W_2$ is

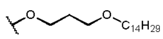, 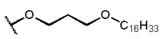, 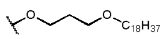, 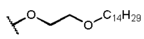, 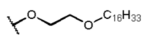,

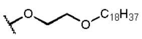, 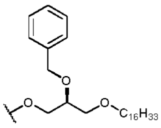, 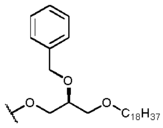, 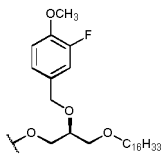 or 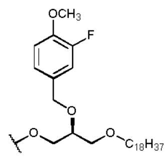.

**13.** The compound of any one of claims 4 to 7, or a tautomer, stereoisomer, prodrug, crystalline form, pharmaceutically acceptable salt, hydrate or solvate thereof, wherein,

$W_2$ is

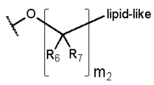,

wherein, $m_2$ is 2, 3 or 4;
$R_6$ and $R_7$ are H;
lipid-like is $C_{8-28}$ alkenyl;
preferably, $W_2$ is

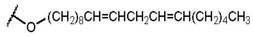.

**14.** The compound of any one of claims 4 to 7, or a tautomer, stereoisomer, prodrug, crystalline form, pharmaceutically acceptable salt, hydrate or solvate thereof, wherein,

$W_2$ is

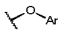,

wherein, Ar is $C_{6-10}$ aryl or 5-10 membered heteroaryl, wherein said $C_{6-10}$ aryl or 5-10 membered heteroaryl is optionally substituted with one or more $C_{1-6}$ alkyl;
preferably, $W_2$ is

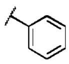, 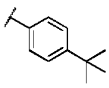 or 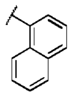.

**15.** The compound of any one of claims 4 to 7, or a tautomer, stereoisomer, prodrug, crystalline form, pharmaceutically acceptable salt, hydrate or solvate thereof, wherein,

$W_2$ is

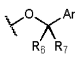,

wherein, $R_6$ and $R_7$ are H;
Ar is $C_{6-10}$ aryl or 5-10 membered heteroaryl, wherein said $C_{6-10}$ aryl or 5-10 membered heteroaryl is optionally substituted with one or more groups selected from $C_{1-6}$ alkyl or - $C(O)OC_{1-6}$ alkyl;
preferably, $W_2$ is

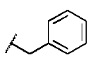, 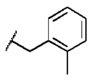, 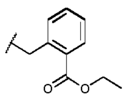 or 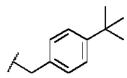.

**16.** The compound of any one of claims 4 to 15, or a tautomer, stereoisomer, prodrug, crystalline form, pharmaceutically acceptable salt, hydrate or solvate thereof, wherein, $W_2$ is

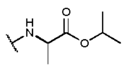,

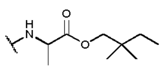, 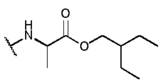, 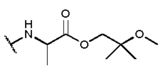, 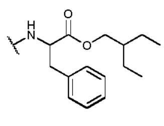, 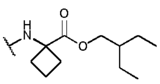, 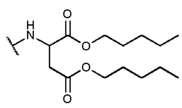,

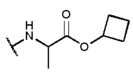, 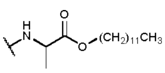, 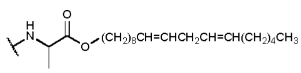, 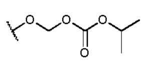, 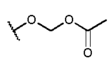, 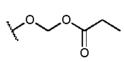,

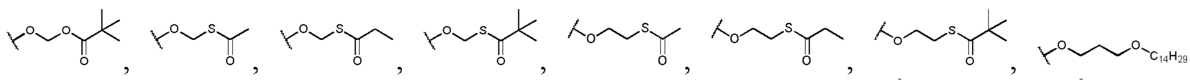

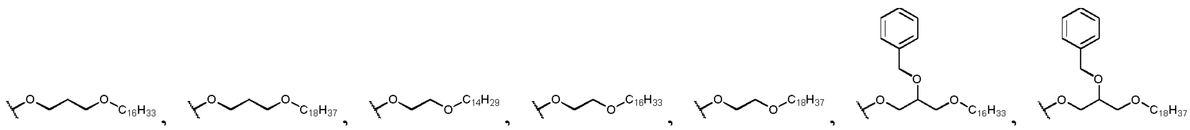

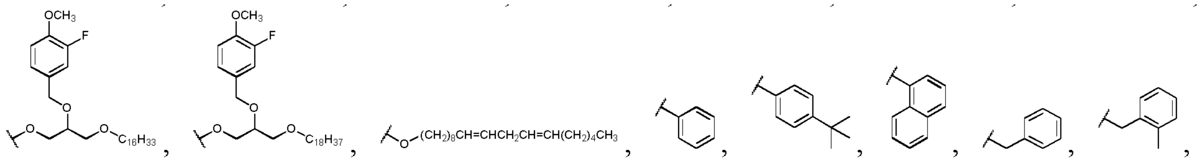

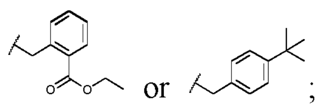

or ;

preferably, $W_2$ is

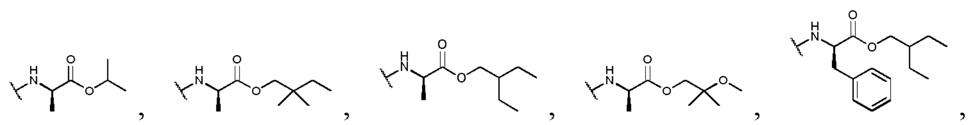

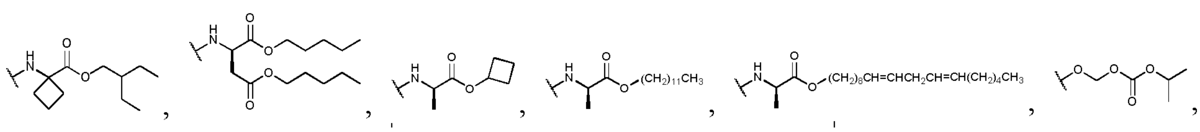

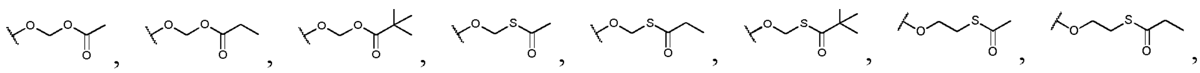

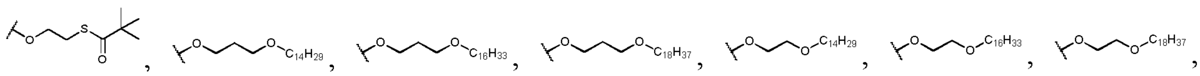

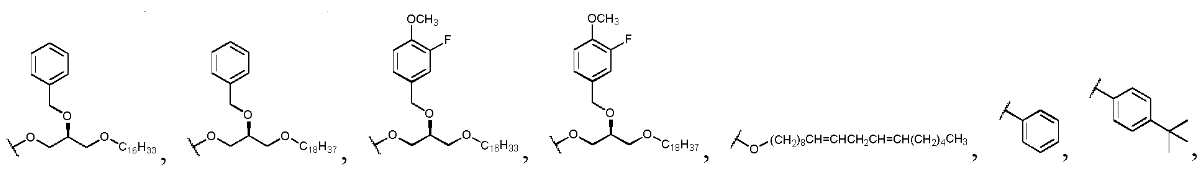

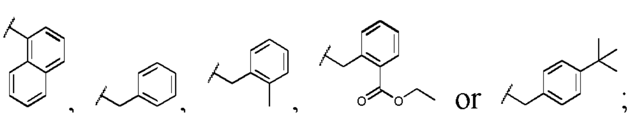

or ;

preferably, $W_2$ is

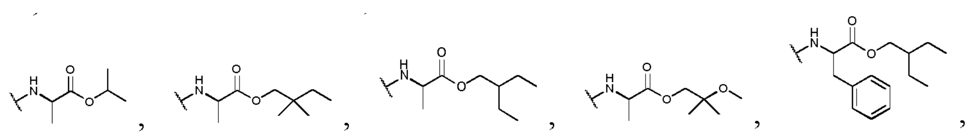

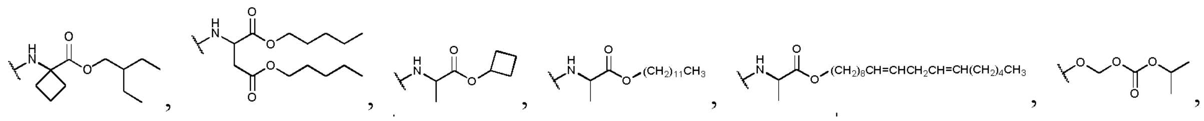

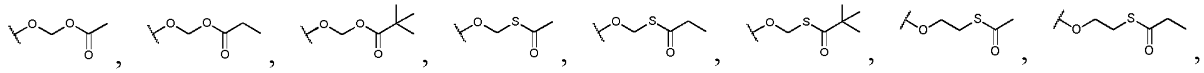

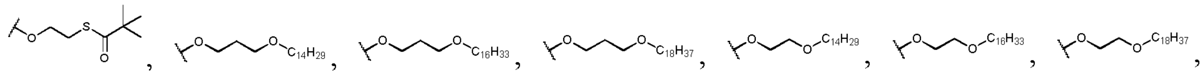

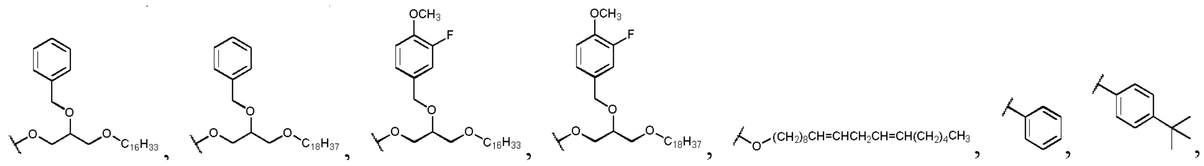

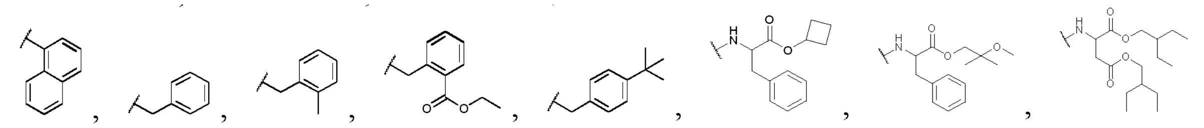

or

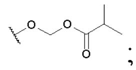

preferably, $W_2$ is

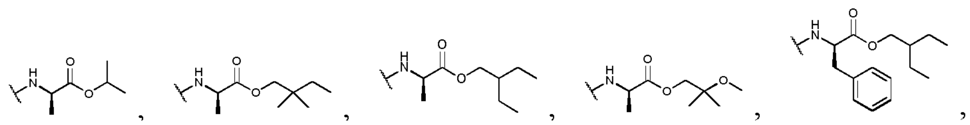

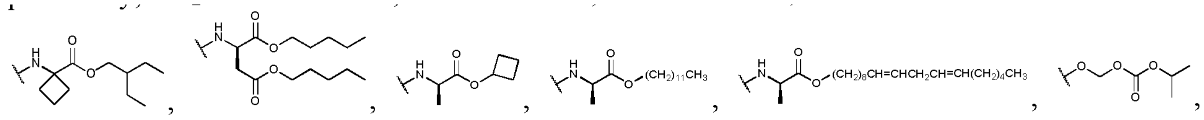

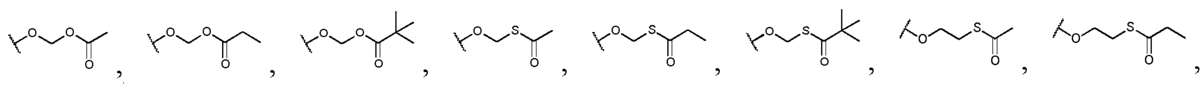

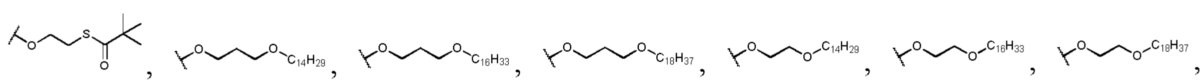

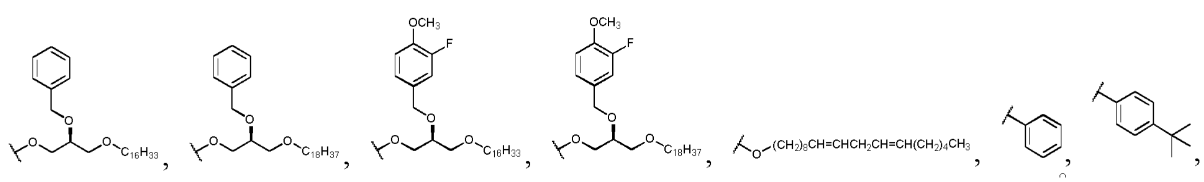

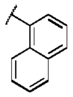, 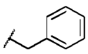, 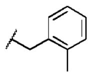, 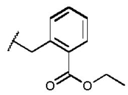, 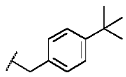, 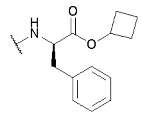, 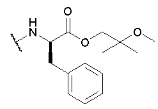, 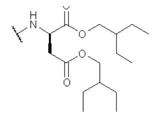,

or

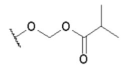.

**17.** The compound of any one of claims 4 to 16, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$m_1$ is 1, 2 or 3;
$R_3$ and $R_4$ are independently H, D or $C_{1-6}$ alkyl;
$R_5$ is $C_{1-28}$ alkyl, $C_{3-6}$ cycloalkyl, 3-7 membered heterocyclyl or $C_{2-28}$ alkenyl.

**18.** The compound of any one of claims 4 to 17, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$m_1$ is 1, 2 or 3;
$R_3$ and $R_4$ are independently H or D;
$R_5$ is $C_{1-6}$ alkyl.

**19.** The compound of claim 5, which is a compound of formula (IIa) or formula (IIb):

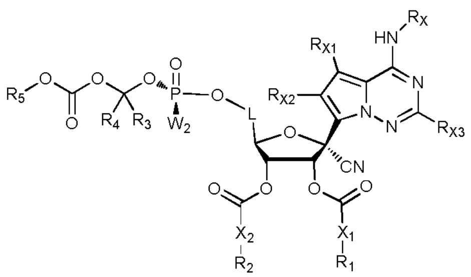

formula (IIa),

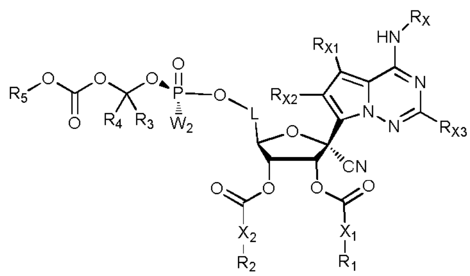

formula (IIb),

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond, O, S or NH, preferably a bond or O, and more preferably a bond;
$R_1$ and $R_2$ are independently $C_{1-6}$ alkyl (preferably $C_{1-3}$ alkyl), which is optionally substituted with one or more deuterium atoms, up to full deuteration;
L is $CH_2$, $CD_2$ or CHD;
$R_3$ and $R_4$ are independently H, D, halogen, $C_{1-6}$ alkyl or $C_{1-6}$ haloalkyl, preferably H or D;

$R_5$ is $C_{1-6}$ alkyl (preferably isopropyl), which is optionally substituted with one or more deuterium atoms, up to full deuteration;

$W_2$ is

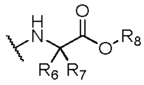, 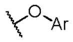, 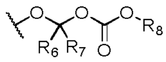 or 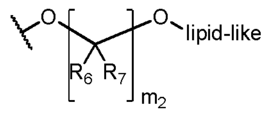;

each of $R_6$ and $R_7$ is independently H, D, $C_{1-6}$ alkyl (preferably $C_{1-3}$ alkyl) or $-OR_a$, or $R_6$ and $R_7$ together with the C atom to which they are attached, form a $C_{3-6}$ cycloalkyl or 3-7 membered heterocyclyl, which is optionally substituted with one or more R', which is optionally substituted with one or more deuterium atoms, up to full deuteration;

each R' is independently $-C(O)OC_{1-6}$ alkyl, $C_{6-10}$ aryl or 5-10 membered heteroaryl, preferably - $C(O)OC_{1-6}$ alkyl or phenyl;

$R_8$ is $C_{1-6}$ alkyl, $C_{3-6}$ cycloalkyl or 3-7 membered heterocyclyl, preferably $C_{1-6}$ alkyl or $C_{3-6}$ cycloalkyl, which is optionally substituted with one or more R, which is optionally substituted with one or more deuterium atoms, up to full deuteration;

Ar is phenyl, which is optionally substituted with one or more R;

R is independently D, $C_{1-6}$ alkyl or $-OC_{1-6}$ alkyl, which is optionally substituted with one or more deuterium atoms, up to full deuteration;

$m_2$ is 1, 2, 3, 4, 5 or 6, preferably 2, 3 or 4;

lipid-like is $C_{8-28}$ alkyl or $C_{8-28}$ alkenyl, preferably $C_{12-20}$ alkyl, which is optionally substituted with one or more deuterium atoms, up to full deuteration;

$R_d$ is independently $(CH_2)_{1-3}$-$C_{6-10}$ aryl or $(CH_2)_{1-3}$-5-10 membered heteroaryl, preferably $CH_2$-phenyl, which is optionally substituted with one or more R";

R" is independently D, halogen or $-OC_{1-6}$ alkyl, which is optionally substituted with one or more deuterium atoms, up to full deuteration;

or a tautomer, stereoisomer, prodrug, crystalline form, pharmaceutically acceptable salt, hydrate or solvate thereof.

**20.** The compound of claim 19, wherein,

$R_X$ is H or OH, preferably H;

$R_{X1}$, $R_{X2}$ and $R_{X3}$ are independently H, D or halogen, preferably H or D;

$X_1$ and $X_2$ are independently a bond or O, preferably a bond;

$R_1$ and $R_2$ are independently $C_{1-6}$ alkyl (preferably $C_{1-3}$ alkyl), which is optionally substituted with one or more deuterium atoms, up to full deuteration;

L is $CH_2$, $CD_2$ or CHD;

$R_3$ and $R_4$ are independently H, D, halogen, $C_{1-6}$ alkyl or $C_{1-6}$ haloalkyl, preferably H or D;

$R_5$ is $C_{1-6}$ alkyl (preferably isopropyl), which is optionally substituted with one or more deuterium atoms, up to full deuteration;

$W_2$ is

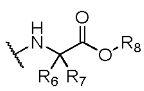 or 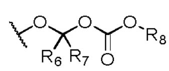;

each of $R_6$ and $R_7$ is independently H, D or $C_{1-3}$ alkyl, or $R_6$ and $R_7$ together with the C atom to which they are attached, form a $C_{3-6}$ cycloalkyl or 3-7 membered heterocyclyl, which is optionally substituted with one or more R', which is optionally substituted with one or more deuterium atoms, up to full deuteration;

each R' is independently $-C(O)OC_{1-6}$ alkyl or $C_{6-10}$ aryl, preferably $-C(O)OC_{1-6}$ alkyl or phenyl; $R_8$ is $C_{1-6}$ alkyl or $C_{3-6}$ cycloalkyl, which is optionally substituted with one or more R, which is optionally substituted with one or more deuterium atoms, up to full deuteration;

R is independently $-OC_{1-6}$ alkyl;

or a tautomer, stereoisomer, prodrug, crystalline form, pharmaceutically acceptable salt, hydrate or solvate thereof.

**21.** The compound of claim 20, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more deuterium atoms, up to full deuteration;
L is $CH_2$, $CD_2$ or CHD;
$R_3$ and $R_4$ are independently H, D, halogen, $C_{1-6}$ alkyl or $C_{1-6}$ haloalkyl, preferably H or D;
$R_5$ is $C_{1-6}$ alkyl (preferably isopropyl), which is optionally substituted with one or more deuterium atoms, up to full deuteration;
$W_2$ is

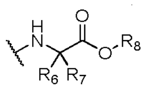 or 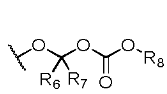 ;

each of $R_6$ and $R_7$ is independently H, D or $C_{1-3}$ alkyl, which is optionally substituted with one or more deuterium atoms, up to full deuteration;
$R_8$ is $C_{1-6}$ alkyl or $C_{3-6}$ cycloalkyl, which is optionally substituted with one or more deuterium atoms, up to full deuteration;
or a tautomer, stereoisomer, prodrug, crystalline form, pharmaceutically acceptable salt, hydrate or solvate thereof.

**22.** The compound of claim 19, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more deuterium atoms, up to full deuteration;
L is $CH_2$, $CD_2$ or CHD;
$R_3$ and $R_4$ are independently H, D, halogen, $C_{1-6}$ alkyl or $C_{1-6}$ haloalkyl, preferably H or D;
$R_5$ is $C_{1-6}$ alkyl (preferably isopropyl), which is optionally substituted with one or more deuterium atoms, up to full deuteration;
$W_2$ is

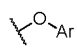 or 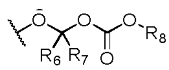 ;

Ar is phenyl, which is optionally substituted with one or more R;
R is independently $C_{1-6}$ alkyl, which is optionally substituted with one or more deuterium atoms, up to full deuteration;
each of $R_6$ and $R_7$ is independently H, D or $C_{1-3}$ alkyl, which is optionally substituted with one or more deuterium atoms, up to full deuteration;
$R_8$ is $C_{1-6}$ alkyl or $C_{3-6}$ cycloalkyl, which is optionally substituted with one or more deuterium atoms, up to full deuteration;
or a tautomer, stereoisomer, prodrug, crystalline form, pharmaceutically acceptable salt, hydrate or solvate thereof.

**23.** The compound of claim 19, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more deuterium atoms, up to full deuteration;

L is $CH_2$, $CD_2$ or CHD;
$R_3$ and $R_4$ are independently H or D;
$R_5$ is isopropyl, which is optionally substituted with one or more deuterium atoms, up to full deuteration;
$W_2$ is

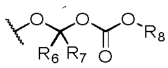;

each of $R_6$ and $R_7$ is independently H or D;
$R_8$ is isopropyl, which is optionally substituted with one or more deuterium atoms, up to full deuteration;
or a tautomer, stereoisomer, prodrug, crystalline form, pharmaceutically acceptable salt, hydrate or solvate thereof.

**24.** The compound of claim 5, which is a compound of formula (IIIa) or formula (IIIb):

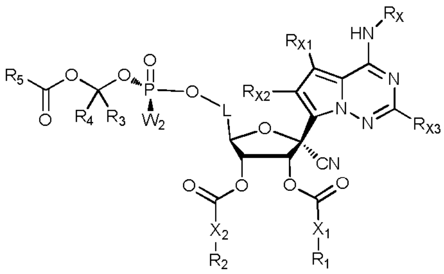

formula (IIIa),

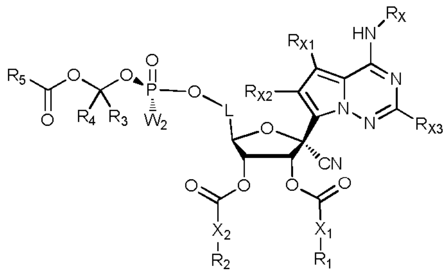

formula (IIIb)

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$R_3$ and $R_4$ are independently H or D;
$R_5$ is $C_{1-6}$ alkyl, which is optionally substituted with one or more D, until they are completely deuterated;
$W_2$ is

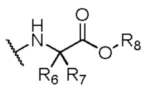, 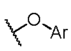 or 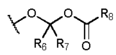;

each of $R_6$ and $R_7$ is independently H, D or $C_{1-3}$ alkyl, which is optionally substituted with one or more D, up to full deuteration;
$R_8$ is $C_{1-6}$ alkyl, which is optionally substituted with one or more D, up to full deuteration;
Ar is phenyl, which is optionally substituted with one or more R;
R is independently $C_{1-6}$ alkyl, which is optionally substituted with one or more D, up to full deuteration;
or a tautomer, stereoisomer, prodrug, crystalline form, pharmaceutically acceptable salt, hydrate or solvate thereof.

**25.** The compound of claim 24, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D,

up to full deuteration;
L is $CH_2$, $CD_2$ or CHD;
$R_3$ and $R_4$ are independently H or D;
$R_5$ is $C_{1-6}$ alkyl (preferably tert-butyl), which is optionally substituted with one or more D, up to full deuteration;
$W_2$ is

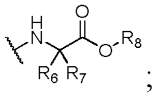

each of $R_6$ and $R_7$ is independently H, D or $C_{1-3}$ alkyl, which is optionally substituted with one or more D, up to full deuteration;
$R_8$ is $C_{1-6}$ alkyl, which is optionally substituted with one or more D, up to full deuteration;
or a tautomer, stereoisomer, prodrug, crystalline form, pharmaceutically acceptable salt, hydrate or solvate thereof.

**26.** The compound of claim 24, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$R_3$ and $R_4$ are independently H or D;
$R_5$ is $C_{1-6}$ alkyl (preferably $C_{1-3}$ alkyl, preferably isopropyl), which is optionally substituted with one or more D, up to full deuteration;
$W_2$ is

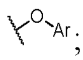

Ar is phenyl, which is optionally substituted with one or more R;
R is independently $C_{1-6}$ alkyl, which is optionally substituted with one or more D, up to full deuteration;
or a tautomer, stereoisomer, prodrug, crystalline form, pharmaceutically acceptable salt, hydrate or solvate thereof.

**27.** The compound of claim 24, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$R_3$ and $R_4$ are independently H or D;
$R_5$ is $C_{1-6}$ alkyl (preferably $C_{1-3}$ alkyl, preferably tert-butyl, more preferably isopropyl), which is optionally substituted with one or more D, up to full deuteration;
$W_2$ is

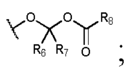

each of $R_6$ and $R_7$ is independently H or D;
$R_8$ is $C_{1-6}$ alkyl (preferably $C_{1-3}$ alkyl, preferably tert-butyl, more preferably isopropyl), which is optionally substituted with one or more D, up to full deuteration;
or a tautomer, stereoisomer, prodrug, crystalline form, pharmaceutically acceptable salt, hydrate or solvate thereof.

**28.** The compound of claim 5, which is a compound of formula (IVa) or formula (IVb):

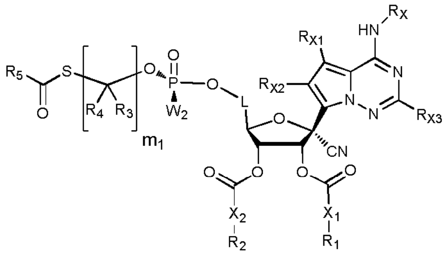

formula (IVa), or

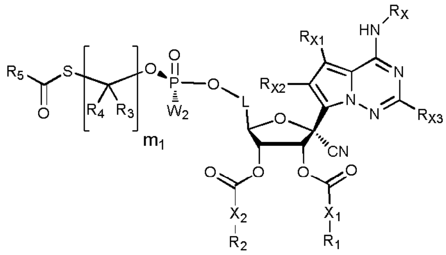

formula (IVb)

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl), which is optionally substituted with one or more D, up to full deuteration;
L is $CH_2$, $CD_2$ or CHD;
$m_1$ is 1, 2 or 3;
$R_3$ and $R_4$ are independently H or D;
$R_5$ is $C_{1-6}$ alkyl (preferably tert-butyl), which is optionally substituted with one or more D, up to full deuteration;
$W_2$ is

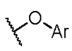 or 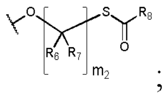 ;

$m_2$ is 1, 2 or 3;
each of $R_6$ and $R_7$ is independently H or D, which is optionally substituted with one or more D, up to full deuteration;
$R_8$ is $C_{1-6}$ alkyl (preferably tert-butyl), which is optionally substituted with one or more D, up to full deuteration;
Ar is phenyl, which is optionally substituted with one or more R;
R is independently $C_{1-6}$ alkyl, which is optionally substituted with one or more D, up to full deuteration;
or a tautomer, stereoisomer, prodrug, crystalline form, pharmaceutically acceptable salt, hydrate or a solvate thereof.

**29.** The compound of any one of claims 1 to 3, which is a compound of formula (V) or (VI):

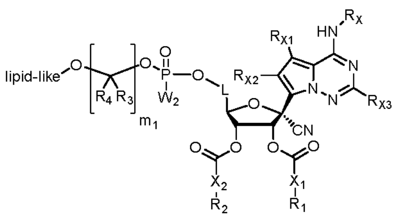

formula (V), or

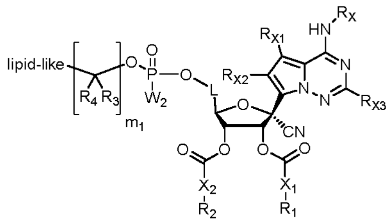

formula (VI)

wherein,

$R_X$ is H or OH;
$R_{X1}$, $R_{X2}$ and $R_{X3}$ are independently H, D, halogen, $-R_d$, $-C(O)R_d$, $-C(O)OR_d$, $-C(O)NR_bR_c$, $-NR_bR_c$, $-NR_aC(O)R_b$, $-NR_aC(O)OR_d$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$;
wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N

atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;

$m_1$ is 1, 2, 3, 4, 5 or 6;

$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl or $C_{8-28}$ alkynyl is optionally substituted with one or more R;

$W_2$ is formula (B):

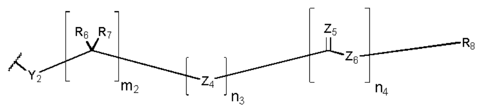

formula (B)

wherein,

$Y_2$ is O, NH or S;

$Z_4$ is O, NH or S;

$Z_5$ is O, NH or S;

$Z_6$ is a bond, O, NH or S;

$m_2$ is 0, 1, 2, 3, 4, 5 or 6;

$n_3$ is 0 or 1;

$n_4$ is 0 or 1;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

or, $W_2$ together with the 3'-position carbon C of glycosyl form $-Y_3-$, wherein $Y_3$ is O, NH or S;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)$

$OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;
wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;
or a tautomer, stereoisomer, prodrug, crystalline form, pharmaceutically acceptable salt, hydrate or a solvate thereof.

**30.** The compound of claim 29, which is a compound of formula (Va), formula (Vb), formula (VIa) or formula (VIb):

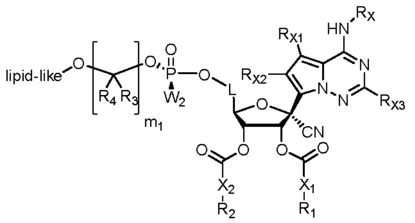 formula (Va),

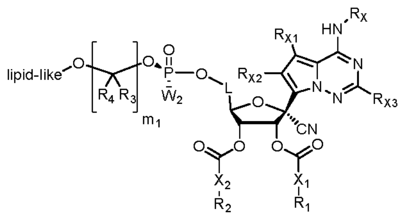 formula (Vb),

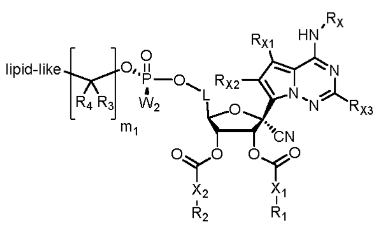 formula (VIa), or

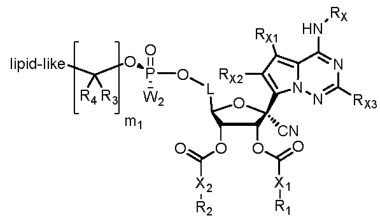 formula (VIb)

or a tautomer, stereoisomer, prodrug, crystalline form, pharmaceutically acceptable salt, hydrate or solvate thereof.

**31.** The compound of claim 29 or 30, or a tautomer, stereoisomer, prodrug, crystalline form, pharmaceutically acceptable salt, hydrate or solvate thereof, wherein:

$R_X$ is H or OH;
$R_{X1}$, $R_{X2}$ and $R_{X3}$ are independently H, D, halogen or $C_{1-6}$ alkyl, wherein said $C_{1-6}$ alkyl are optionally substituted with one or more deuterium atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R';
L is $CH_2$, $CD_2$ or CHD;
$m_1$ is 2, 3 or 4;
$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein said $C_{1-6}$ alkyl, $C_{3-6}$ cycloalkyl and 3-7 membered heterocyclyl are optionally substituted with one or more R';
lipid-like is $C_{1-28}$ alkyl, $C_{1-28}$ haloalkyl, $C_{2-28}$ alkenyl or $C_{2-28}$ alkynyl, wherein said $C_{1-28}$ alkyl, $C_{1-28}$ haloalkyl, $C_{2-28}$ alkenyl and $C_{2-28}$ alkynyl are optionally substituted with one or more R;
$W_2$ is:

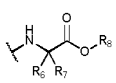, 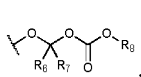, 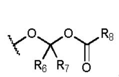, 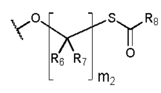, 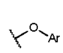 or 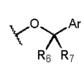;

wherein,

$m_2$ is 1, 2, 3, 4, 5 or 6;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

Ar is $C_{6-10}$ aryl or 5-10 membered heteroaryl, wherein said $C_{6-10}$ aryl and 5-10 membered heteroaryl are optionally substituted with one or more R;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_a$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration.

**32.** The compound of any one of claims 29 to 31, or a tautomer, stereoisomer, prodrug, crystalline form, pharmaceutically acceptable salt, hydrate or solvate thereof, wherein:

$W_2$ is

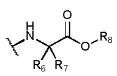 , 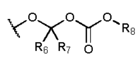 , 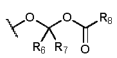 , 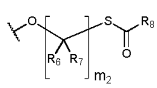 , 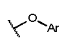 or 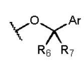 ;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ alkoxy, $C_{3-6}$ cycloalkyl and 3-7 membered heterocyclyl are optionally substituted with one or more R';

each $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein said $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

Ar is $C_{6-10}$ aryl or 5-10 membered heteroaryl, wherein said $C_{6-10}$ aryl and 5-10 membered heteroaryl are optionally substituted with one or more R.

**33.** The compound of any one of claims 29 to 32, or a tautomer, stereoisomer, prodrug, crystalline form, pharmaceutically acceptable salt, hydrate or solvate thereof, wherein,

$W_2$ is

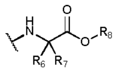,

wherein, $R_6$ is $C_{1-6}$ alkyl, and $R_7$ is H, or $R_6$ and $R_7$ together with the C atom to which they are attached, form a $C_{3-6}$ cycloalkyl, wherein said $C_{1-6}$ alkyl and $C_{3-6}$ cycloalkyl are optionally substituted with one or more groups selected from phenyl or -C(O)O$C_{1-6}$ alkyl;
$R_8$ is independently $C_{1-28}$ alkyl, $C_{3-6}$ cycloalkyl, 3-7 membered heterocyclyl or $C_{2-28}$ alkenyl, wherein said $C_{1-28}$ alkyl, $C_{3-6}$ cycloalkyl, 3-7 membered heterocyclyl or $C_{2-28}$ alkenyl is optionally substituted with one or more $C_{1-6}$ alkoxy;
preferably, $W_2$ is

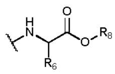;

preferably, $W_2$ is

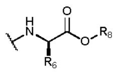;

preferably, $W_2$ is

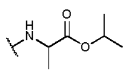, 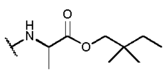, 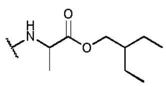, 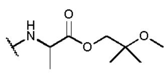, 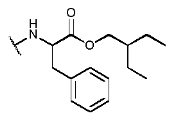,

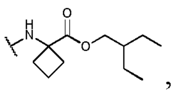, 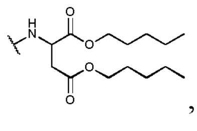, 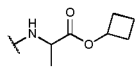, 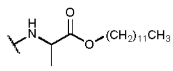 or 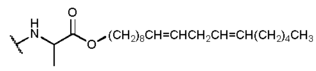;

preferably, $W_2$ is

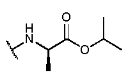, 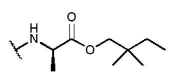, 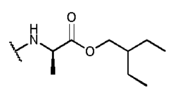, 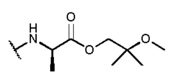, 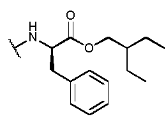,

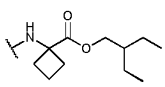, 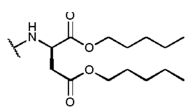, 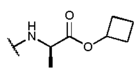, 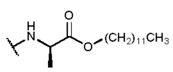 or 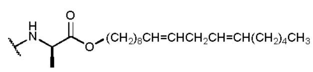.

**34.** The compound of any one of claims 29 to 32, or a tautomer, stereoisomer, prodrug, crystalline form, pharmaceutically acceptable salt, hydrate or solvate thereof, wherein,

$W_2$ is

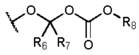,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

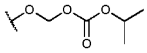.

**35.** The compound of any one of claims 29 to 32, or a tautomer, stereoisomer, prodrug, crystalline form, pharmaceutically acceptable salt, hydrate or solvate thereof, wherein,

$W_2$ is

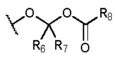,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

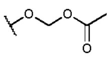, 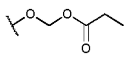 or 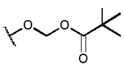.

**36.** The compound of any one of claims 29 to 32, or a tautomer, stereoisomer, prodrug, crystalline form, pharmaceutically acceptable salt, hydrate or solvate thereof, wherein,

$W_2$ is

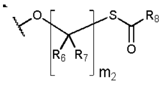,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

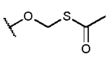, 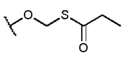, 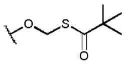, 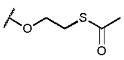, 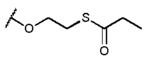 or 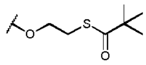.

**37.** The compound of any one of claims 29 to 32, or a tautomer, stereoisomer, prodrug, crystalline form, pharmaceutically acceptable salt, hydrate or solvate thereof, wherein,

$W_2$ is

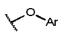,

wherein, Ar is $C_{6-10}$ aryl or 5-10 membered heteroaryl, wherein said $C_{6-10}$ aryl or 5-10 membered heteroaryl is optionally substituted with one or more $C_{1-6}$ alkyl;
preferably, $W_2$ is

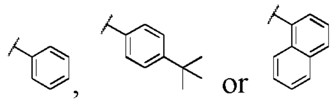, or .

**38.** The compound of any one of claims 29 to 32, or a tautomer, stereoisomer, prodrug, crystalline form, pharmaceutically acceptable salt, hydrate or solvate thereof, wherein,

$W_2$ is

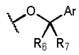,

wherein, $R_6$ and $R_7$ are H;
Ar is $C_{6-10}$ aryl or 5-10 membered heteroaryl, wherein said $C_{6-10}$ aryl or 5-10 membered heteroaryl are optionally substituted with one or more groups selected from $C_{1-6}$ alkyl or - $C(O)OC_{1-6}$ alkyl;
preferably, $W_2$ is

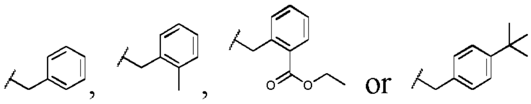, , or .

**39.** The compound of any one of claims 29 to 38, or a tautomer, stereoisomer, prodrug, crystalline form, pharmaceutically acceptable salt, hydrate or solvate thereof, wherein, $W_2$ is

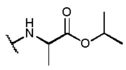,

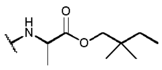, 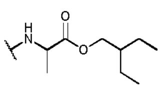, 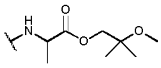, 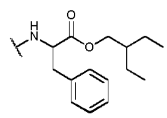, 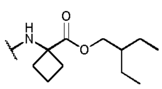, 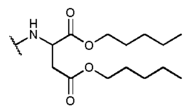,

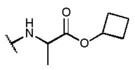, 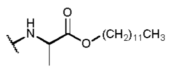, 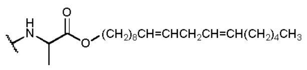, 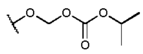, 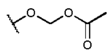, 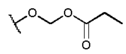,

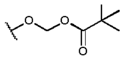, 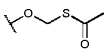, 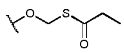, 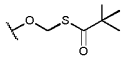, 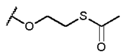, 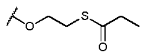, 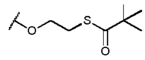, 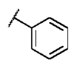,

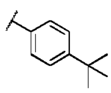, 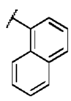, 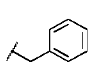, 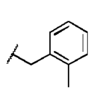, 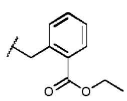 or 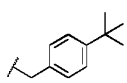;

preferably, $W_2$ is

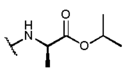, 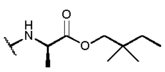, 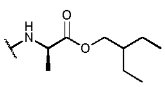, 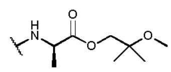, 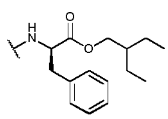,

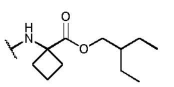, 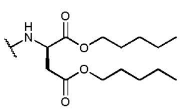, 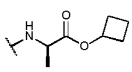, 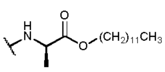, 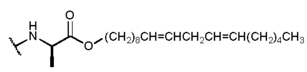,

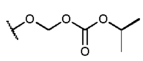, 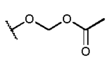, 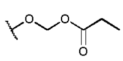, 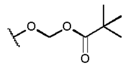, 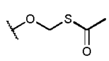, 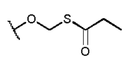, 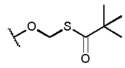, 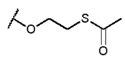,

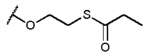, 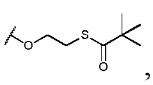, 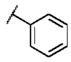, 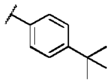, 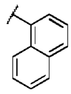, 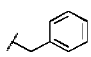, 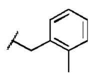, 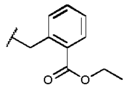 or 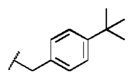.

**40.** The compound of any one of claims 29 to 39, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$m_1$ is 2, 3 or 4;
$R_3$ and $R_4$ are independently H, D or $C_{1-6}$ alkoxy, wherein said $C_{1-6}$ alkoxy is optionally substituted with one or more groups selected from $C_{6-10}$ aryl or 5-10 membered heteroaryl, wherein said $C_{6-10}$ aryl or 5-10 membered heteroaryl is optionally substituted with one or more groups selected from halogen or $C_{1-6}$ alkoxy;
lipid-like is $C_{1-28}$ alkyl or $C_{2-28}$ alkenyl.

**41.** The compound of any one of claims 29 to 40, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$m_1$ is 2, 3 or 4;
$R_3$ and $R_4$ are independently H, D or $C_{1-6}$ alkoxy, wherein said $C_{1-6}$ alkoxy is optionally substituted with one or more groups selected from $C_{6-10}$ aryl or 5-10 membered heteroaryl, wherein said $C_{6-10}$ aryl or 5-10 membered heteroaryl is optionally substituted with one or more groups selected from halogen or $C_{1-6}$ alkoxy;
lipid-like is $C_{1-28}$ alkyl or $C_{2-28}$ alkenyl.

**42.** The compound of any one of claims 1 to 3, which is a compound of formula (VII):

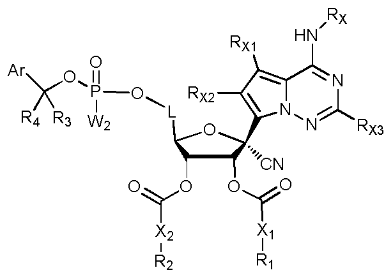

formula (VII)

wherein,

$R_X$ is H or OH;
$R_{X1}$, $R_{X2}$ and $R_{X3}$ are independently H, D, halogen, $-R_a$, $-C(O)R_a$, $-C(O)OR_a$, $-C(O)NR_bR_c$, $-NR_bR_c$, $-NR_aC(O)R_b$, $-NR_aC(O)OR_d$, $-NR_aC(O)NR_bR_c$, $-OR_a$, $-OC(O)R_a$, $-OC(O)NR_bR_c$, $-NR_aS(O)_2R_b$, $-S(O)_2NR_a$, $-S(O)R_a$ or $-S(O)_2R_a$;
wherein each of $R_a$, $R_b$ and $R_c$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_b$ and $R_c$ together with the N atom to which they are attached, form a 3-7 membered heterocyclyl or 5-10 membered heteroaryl; wherein said

$C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;
$X_1$ and $X_2$ are independently a bond, O, S or NH;
$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R; L is $CH_2$, $CD_2$ or CHD;
$R_3$ and $R_4$ are independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_3$ and $R_4$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
Ar is $C_{6-10}$ aryl or 5-10 membered heteroaryl, wherein said $C_{6-10}$ aryl and 5-10 membered heteroaryl are optionally substituted with one or more R;
$W_2$ is formula (B):

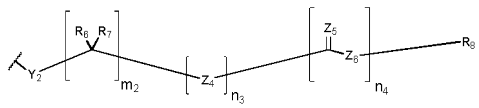
formula (B)

wherein,
$Y_2$ is O, NH or S;
$Z_4$ is O, NH or S;
$Z_5$ is O, NH or S;
$Z_6$ is a bond, O, NH or S;
$m_2$ is 0, 1, 2, 3, 4, 5 or 6;
$n_3$ is 0 or 1;
$n_4$ is 0 or 1;
each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';
$R_8$ is H, D, $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;
or, $W_2$ together with the 3'-position C atom of glycosyl form $-Y_3-$, wherein $Y_3$ is O, NH or S;
each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;
each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";
wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";
each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$

cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration;

or a tautomer, stereoisomer, prodrug, crystalline form, pharmaceutically acceptable salt, hydrate or solvate thereof.

**43.** The compound of claim 42, which is a compound of formula (VIIa) or formula (VIIb):

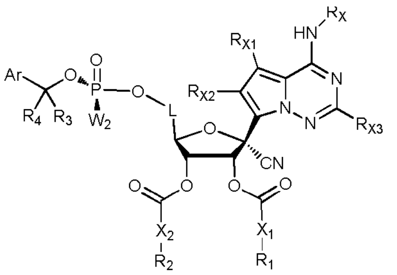

formula (VIIa), or

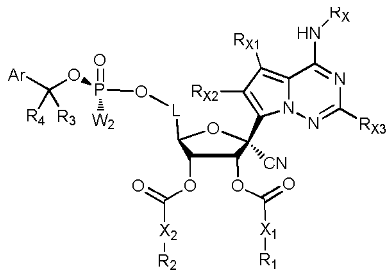

formula (VIIb)

or a tautomer, stereoisomer, prodrug, crystalline form, pharmaceutically acceptable salt, hydrate or solvate thereof.

**44.** The compound of claim 42 or 43, or a tautomer, stereoisomer, prodrug, crystalline form, pharmaceutically acceptable salt, hydrate or solvate thereof, wherein:

$R_X$ is H or OH;

$R_{X1}$, $R_{X2}$ and $R_{X3}$ are independently H, D, halogen or $C_{1-6}$ alkyl, wherein said $C_{1-6}$ alkyl is optionally substituted with one or more deuterium atoms, up to full deuteration;

$X_1$ and $X_2$ are independently a bond, O, S or NH;

$R_1$ and $R_2$ are independently $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R';

L is $CH_2$, $CD_2$ or CHD;

$R_3$ and $R_4$ are independently H, D, $C_{1-6}$ alkyl, $C_{3-6}$ cycloalkyl or 3-7 membered heterocyclyl, or $R_3$ and $R_4$ together with the C atom to which they are attached, form a $C_{3-6}$ cycloalkyl or 3-7 membered heterocyclyl, wherein said $C_{1-6}$ alkyl, $C_{3-6}$ cycloalkyl and 3-7 membered heterocyclyl are optionally substituted with one or more R';

Ar is $C_{6-10}$ aryl or 5-10 membered heteroaryl, wherein said $C_{6-10}$ aryl and 5-10 membered heteroaryl are optionally substituted with one or more R;

$W_2$ is:

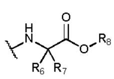 , 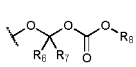 , 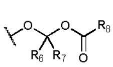 , 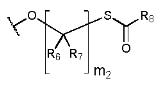 , 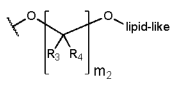 or 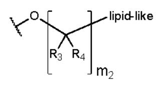 ;

wherein,

$m_2$ is 1, 2, 3, 4, 5 or 6;

each of $R_6$ and $R_7$ is independently H, D, halogen, $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ haloalkyl, $C_{1-6}$ alkoxy, $C_{1-6}$ haloalkoxy, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R';

$R_8$ is $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$

aryl or 5-10 membered heteroaryl, wherein said $C_{1-28}$ alkyl, $C_{1-28}$ haloalkyl, $C_{3-6}$ cycloalkyl, 3-7 membered heterocyclyl, $C_{2-28}$ alkenyl, $C_{2-28}$ alkynyl, $C_{6-10}$ aryl and 5-10 membered heteroaryl are optionally substituted with one or more R;

lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein said $C_{6-10}$ aryl and 5-10 membered heteroaryl are optionally substituted with one or more R;

each R is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R", up to full deuteration;

each R' is independently D, halogen, $-R_d$, $-C(O)R_d$, $-C(O)OR_d$, $-C(O)NR_eR_f$, $-NR_eR_f$, $-NR_dC(O)R_e$, $-NR_dC(O)OR_e$, $-NR_dC(O)NR_eR_f$, $-OR_d$, $-OC(O)R_d$, $-OC(O)NR_eR_f$, $-NR_dS(O)_2R_e$, $-S(O)_2NR_d$, $-S(O)R_d$ or $-S(O)_2R_d$, or two R' on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more R";

wherein each of $R_d$, $R_e$ and $R_f$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_e$ and $R_f$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more R";

each R" is independently D, halogen, $-R_g$, $-C(O)R_g$, $-C(O)OR_g$, $-C(O)NR_hR_j$, $-NR_hR_j$, $-NR_gC(O)R_h$, $-NR_gC(O)OR_h$, $-NR_gC(O)NR_hR_j$, $-OR_g$, $-OC(O)R_g$, $-OC(O)NR_hR_j$, $-NR_gS(O)_2R_h$, $-S(O)_2NR_g$, $-S(O)R_g$ or $-S(O)_2R_g$, or two R" on the same atom or two adjacent atoms, together with the atoms to which they are attached, form a $C_{3-6}$ cycloalkyl, 3-7 membered heterocyclyl, $C_{6-10}$ aryl or 5-10 membered heteroaryl, wherein said $C_{3-6}$ cycloalkyl, 3-7 membered heterocyclyl, $C_{6-10}$ aryl and 5-10 membered heteroaryl are optionally substituted with one or more deuterium atoms, up to full deuteration;

wherein each of $R_g$, $R_h$ and $R_j$ is independently H, D, $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl or 5-10 membered heteroaryl, or $R_h$ and $R_j$ together with the N atom to which they are attached, form a 3-7 membered heterocyclyl or 5-10 membered heteroaryl; wherein said $C_{1-6}$ alkyl, $C_{1-6}$ haloalkyl, $C_{3-6}$ cycloalkyl, 3-7 membered heterocyclyl, $C_{2-6}$ alkenyl, $C_{2-6}$ alkynyl, $C_{6-10}$ aryl and 5-10 membered heteroaryl are optionally substituted with one or more deuterium atoms, up to full deuteration.

**45.** The compound of any one of claims 42 to 44, or a tautomer, stereoisomer, prodrug, crystalline form pharmaceutically acceptable salt hydrate or solvate thereof, wherein:

$W_2$ is

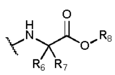, 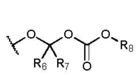, 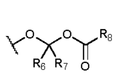, 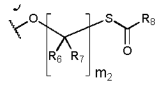, 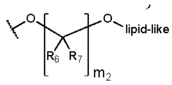 or 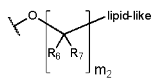;

wherein, $m_2$ is 1, 2, 3 or 4;

each of $R_6$ and $R_7$ is independently H, D, $C_{1-6}$ alkyl, $C_{1-6}$ alkoxy, $C_{3-6}$ cycloalkyl or 3-7 membered heterocyclyl, or $R_6$ and $R_7$ together with the C atom to which they are attached, form a $C_{3-6}$ cycloalkyl or 3-7 membered heterocyclyl, wherein said $C_{1-6}$ alkyl, $C_{1-6}$ alkoxy, $C_{3-6}$ cycloalkyl and 3-7 membered heterocyclyl are optionally substituted with one or more R';

each $R_8$ is independently $C_{1-28}$ alkyl, $C_{1-28}$ haloalkyl, $C_{3-6}$ cycloalkyl, 3-7 membered heterocyclyl, $C_{2-28}$ alkenyl or $C_{2-28}$ alkynyl, wherein said $C_{1-28}$ alkyl, $C_{1-28}$ haloalkyl, $C_{3-6}$ cycloalkyl, 3-7 membered heterocyclyl, $C_{2-28}$ alkenyl and $C_{2-28}$ alkynyl are optionally substituted with one or more R;

lipid-like is $C_{8-28}$ alkyl, $C_{8-28}$ haloalkyl, $C_{8-28}$ alkenyl or $C_{8-28}$ alkynyl, wherein said $C_{8-28}$ alkyl, $C_{8-28}$ haloalkyl, $C_{8-28}$ alkenyl and $C_{8-28}$ alkynyl are optionally substituted with one or more R;

Ar is $C_{6-10}$ aryl or 5-10 membered heteroaryl, wherein said $C_{6-10}$ aryl and 5-10 membered heteroaryl are optionally substituted with one or more R.

**46.** The compound of any one of claims 42 to 45, or a tautomer, stereoisomer, prodrug, crystalline form, pharmaceutically

acceptable salt, hydrate or solvate thereof, wherein,

$W_2$ is

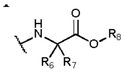,

wherein, $R_6$ is $C_{1-6}$ alkyl, and $R_7$ is H, or $R_6$ and $R_7$ together with the C atom to which they are attached, form a $C_{3-6}$ cycloalkyl, wherein said $C_{1-6}$ alkyl and $C_{3-6}$ cycloalkyl are optionally substituted with one or more groups selected from phenyl or -C(O)O$C_{1-6}$ alkyl;

$R_8$ is independently $C_{1-28}$ alkyl, $C_{3-6}$ cycloalkyl, 3-7 membered heterocyclyl or $C_{2-28}$ alkenyl, wherein said $C_{1-28}$ alkyl, $C_{3-6}$ cycloalkyl, 3-7 membered heterocyclyl or $C_{2-28}$ alkenyl are optionally substituted with one or more $C_{1-6}$ alkoxy:

preferably, $W_2$ is

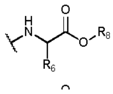;

preferably, $W_2$ is

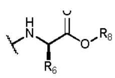;

preferably, $W_2$ is

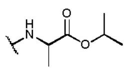, 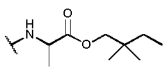, 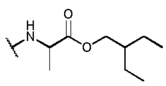, 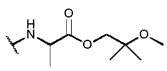, 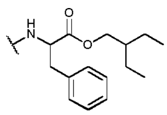,

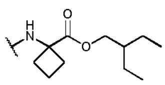, 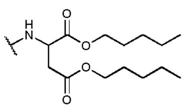, 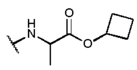, 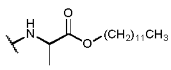 or 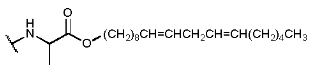;

preferably, $W_2$ is

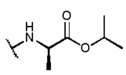, 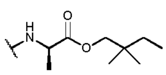, 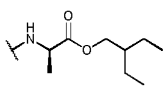, 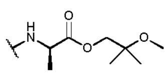, 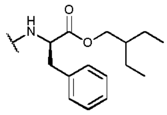,

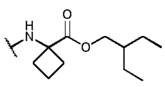, 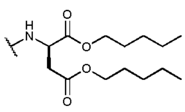, 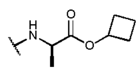, 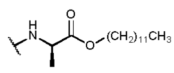 or 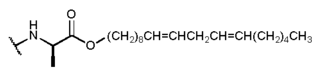.

**47.** The compound of any one of claims 42 to 45, or a tautomer, stereoisomer, prodrug, crystalline form, pharmaceutically acceptable salt, hydrate or solvate thereof, wherein,

$W_2$ is

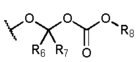,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

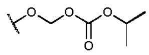.

**48.** The compound of any one of claims 42 to 45, or a tautomer, stereoisomer, prodrug, crystalline form, pharmaceutically acceptable salt, hydrate or solvate thereof, wherein,

$W_2$ is

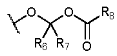,

wherein, $R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

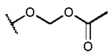, 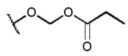 or 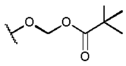.

**49.** The compound of any one of claims 42 to 45, or a tautomer, stereoisomer, prodrug, crystalline form, pharmaceutically acceptable salt, hydrate or solvate thereof, wherein,

$W_2$ is

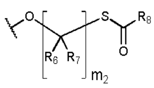,

wherein, $m_2$ is 1 or 2;
$R_6$ and $R_7$ are H;
$R_8$ is independently $C_{1-28}$ alkyl, $C_{3-6}$ cycloalkyl, 3-7 membered heterocyclyl or $C_{2-28}$ alkenyl;
preferably, $W_2$ is

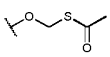, 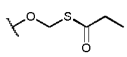, 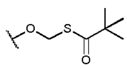, 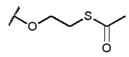, 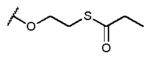 or 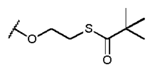.

**50.** The compound of any one of claims 42 to 45, or a tautomer, stereoisomer, prodrug, crystalline form, pharmaceutically acceptable salt, hydrate or solvate thereof, wherein,

$W_2$ is

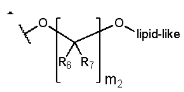,

wherein, $m_2$ is 2, 3 or 4;
$R_6$ and $R_7$ are independently H or $C_{1-6}$ alkoxy, wherein said $C_{1-6}$ alkoxy are optionally substituted with one or more groups selected from $C_{6-10}$ aryl or 5-10 membered heteroaryl, wherein said $C_{6-10}$ aryl or 5-10 membered heteroaryl are optionally substituted with one or more groups selected from halogen or $C_{1-6}$ alkoxy;
lipid-like is $C_{8-28}$ alkyl or $C_{8-28}$ alkenyl;
preferably, $W_2$ is

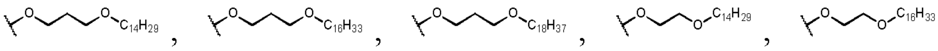 , , , , ,

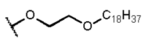, 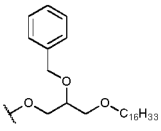, 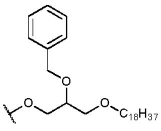, 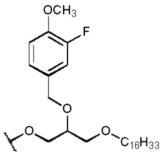 or 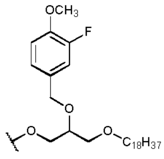;

preferably, $W_2$ is

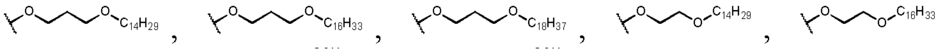 , , , , ,

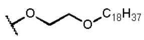, 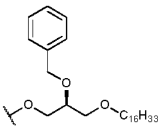, 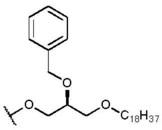, 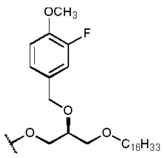 or 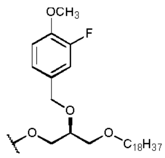.

**51.** The compound of any one of claims 42 to 45, or a tautomer, stereoisomer, prodrug, crystalline form, pharmaceutically acceptable salt, hydrate or solvate thereof, wherein,

$W_2$ is

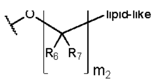,

wherein, $m_2$ is 2, 3 or 4;
$R_6$ and $R_7$ are H;
lipid-like is $C_{8-28}$ alkenyl;
preferably, $W_2$ is

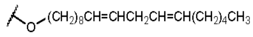.

**52.** The compound of any one of claims 42 to 51, or a tautomer, stereoisomer, prodrug, crystalline form, pharmaceutically acceptable salt, hydrate or solvate thereof, wherein, $W_2$ is

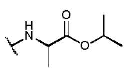,

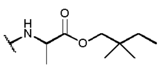, 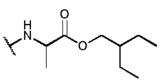, 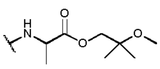, 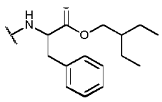, 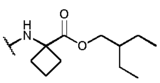, 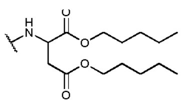,

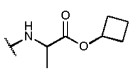, 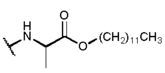, 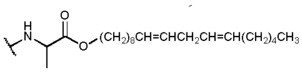, 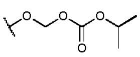, 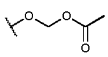, 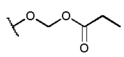,

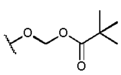, 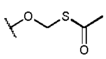, 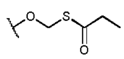, 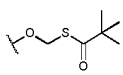, 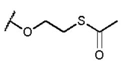, 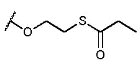, 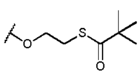, 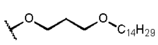,

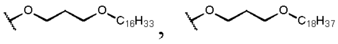, 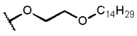, 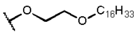, 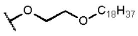, 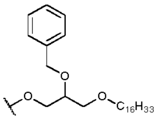, 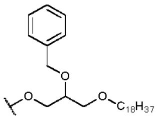,

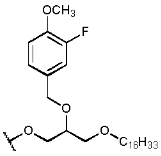, 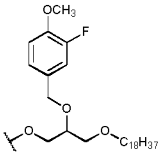 or 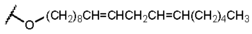;

preferably, $W_2$ is

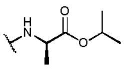, 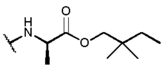, 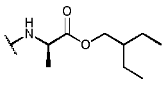, 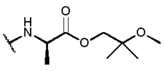, 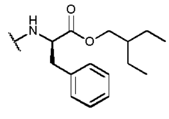,

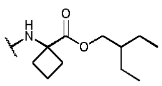, 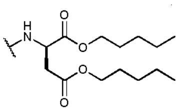, 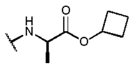, 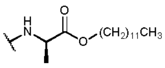, 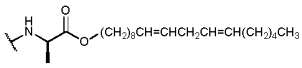,

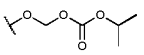, 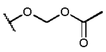, 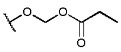, 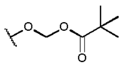, 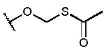, 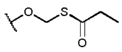, 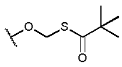, 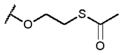,

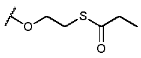, 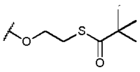, 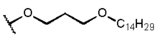, 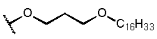, 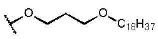, 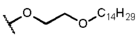, 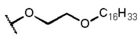,

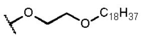, 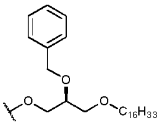, 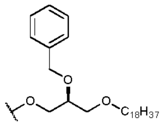, 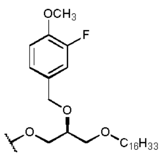, 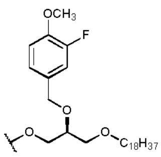 or 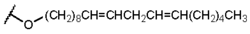.

**53.** The compound of any one of claims 42 to 52, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H, D or halogen;
$X_1$ and $X_2$ are independently a bond or O;
$R_1$ and $R_2$ are independently $C_{1-6}$ alkyl, $C_{3-6}$ cycloalkyl or $C_{6-10}$ aryl;
L is $CH_2$, $CD_2$ or CHD;
$R_3$ and $R_4$ are independently H, D or $C_{1-6}$ alkyl;
Ar is $C_{6-10}$ aryl or 5-10 membered heteroaryl.

**54.** The compound of any one of claims 42 to 53, or a tautomer, stereoisomer, prodrug, crystalline form, pharmaceutically acceptable salt, hydrate or solvate thereof, wherein,

$R_X$ is H;
$R_{X1}$, $R_{X2}$ and $R_{X3}$ are independently H or D;
$X_1$ and $X_2$ are independently a bond or O;
$R_1$ and $R_2$ are independently $C_{1-6}$ alkyl;
L is $CH_2$, $CD_2$ or CHD;
$R_3$ and $R_4$ are independently H or D;

Ar is $C_{6-10}$ aryl.

**55.** A compound of formula (VIIa), formula (VIIa) or formula (VIIb):

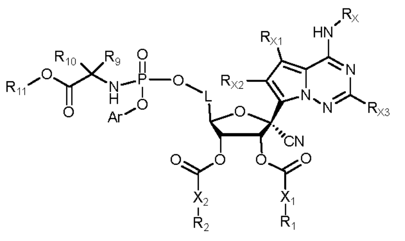

formula (VII),

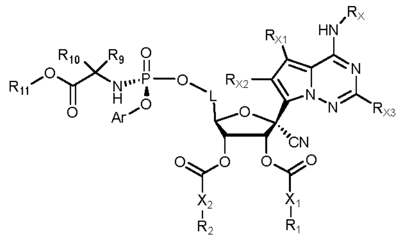

formula (VIIa)

or

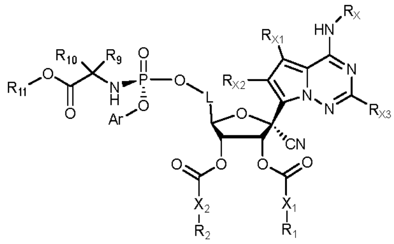

formula (VII)

wherein,

$R_X$ is H or OH, preferably H;
$R_{X1}$, $R_{X2}$ and $R_{X3}$ are independently H, D or halogen, preferably H or D;
$X_1$ and $X_2$ are independently a bond, O, S or NH, preferably a bond or O, and more preferably a bond;
$R_1$ and $R_2$ are independently $C_{1-6}$ alkyl (preferably $C_{1-3}$ alkyl), which is optionally substituted with one or more deuterium atoms, up to full deuteration;
L is $CH_2$, $CD_2$ or CHD;
$R_9$ is $CH_2$-$C_{6-10}$ aryl, $CH_2$-5-10 membered heteroaryl, $CH_2C(O)O$-$C_{1-6}$ alkyl, $CH_2C(O)O$-$C_{3-6}$ cycloalkyl or $CH_2C(O)O$-3-7 membered heteroaryl, preferably $CH_2$-phenyl, $CH_2C(O)O$-$C_{1-6}$ alkyl or $CH_2C(O)O$-$C_{3-6}$ cycloalkyl, which is optionally substituted with one or more deuterium atoms, up to full deuteration; and $R_{10}$ is H or D;
or, $R_9$ and $R_{10}$ together with the C atom to which they are attached, form a $C_{3-6}$ cycloalkyl or 3-7 membered heterocyclyl, which is optionally substituted with one or more deuterium atoms, up to full deuteration;
Ar is phenyl, which is optionally substituted with one or more R;
R is independently D, $C_{1-6}$ alkyl or -O$C_{1-6}$ alkyl, which is optionally substituted with one or more deuterium atoms, up to full deuteration;
or a tautomer, a stereoisomer, prodrug, crystalline form, pharmaceutically acceptable salt, hydrate or solvate thereof.

**56.** The compound of claim 55, wherein,

$R_X$ is H or OH, preferably H;
$R_{X1}$, $R_{X2}$ and $R_{X3}$ are independently H, D or halogen, preferably H or D;
$X_1$ and $X_2$ are independently a bond or O, preferably a bond;
$R_1$ and $R_2$ are independently $C_{1-6}$ alkyl (preferably $C_{1-3}$ alkyl, more preferably isopropyl), which is optionally substituted with one or more deuterium atoms, up to full deuteration;
L is $CH_2$, $CD_2$ or CHD;
$R_9$ is $CH_2$-phenyl, $CH_2C(O)O$-$C_{1-6}$ alkyl or $CH_2C(O)O$-$C_{3-6}$ cycloalkyl, which is optionally substituted with one or more deuterium atoms, up to full deuteration; and $R_{10}$ is H or D;
Ar is phenyl, which is optionally substituted with one or more R;
R is independently $C_{1-6}$ alkyl (preferably tert-butyl), which is optionally substituted with one or more deuterium atoms, up to full deuteration;
or a tautomer, stereoisomer, prodrug, crystalline form, pharmaceutically acceptable salt, hydrate or solvate

thereof.

**57.** A compound, or a tautomer, stereoisomer, prodrug, crystalline form, pharmaceutically acceptable salt, hydrate or solvate thereof, wherein, the compound is selected from the group consisting of:

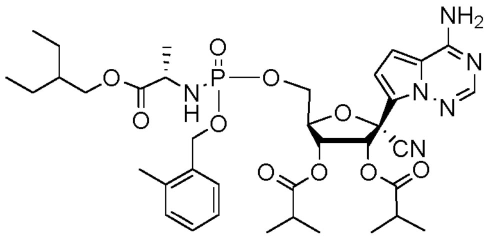
,
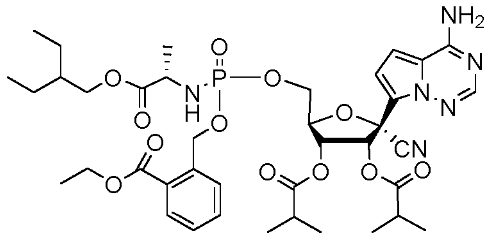
,

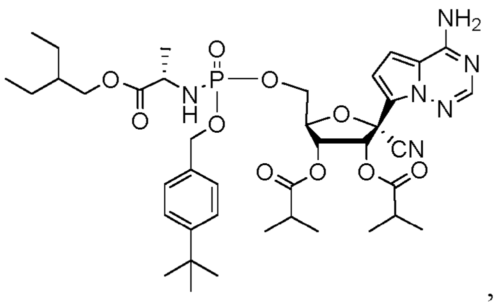
,
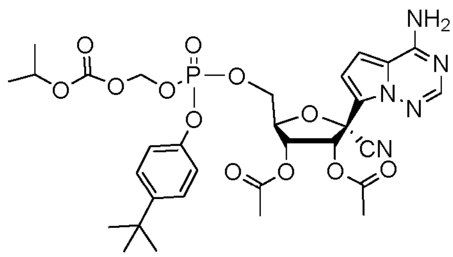
,

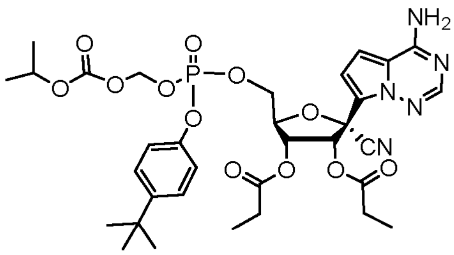
,
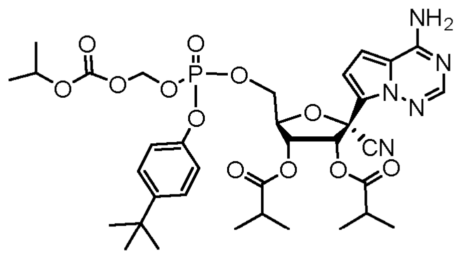
,

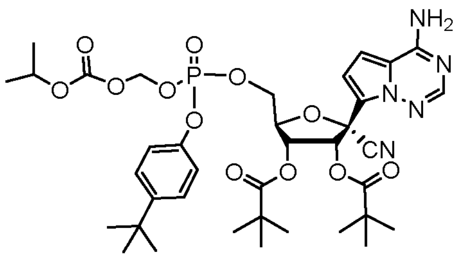
,
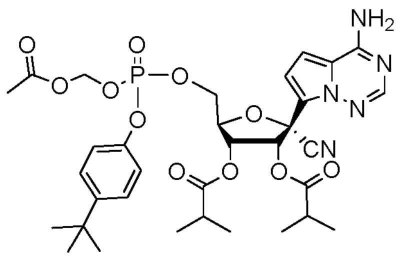
,

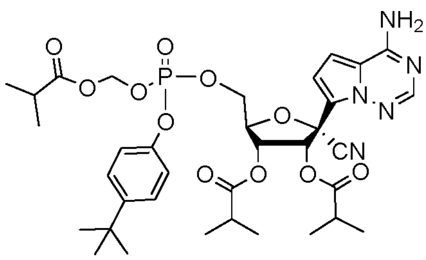
,
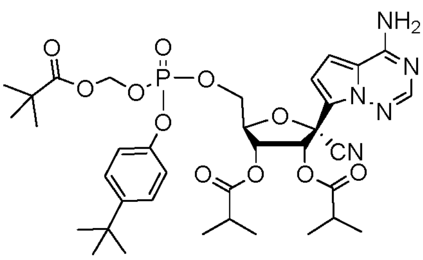
,

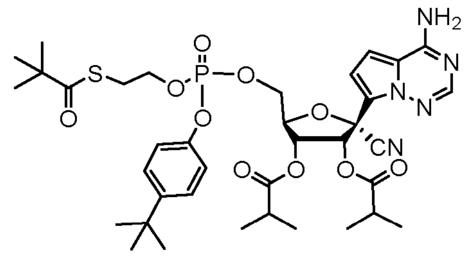

,

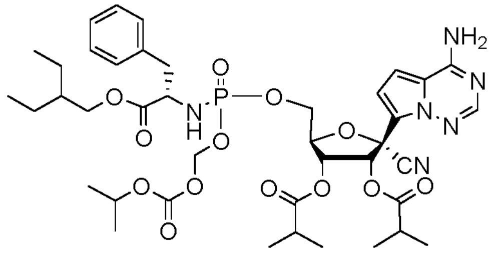

,

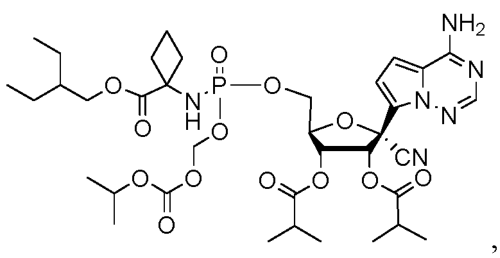

,

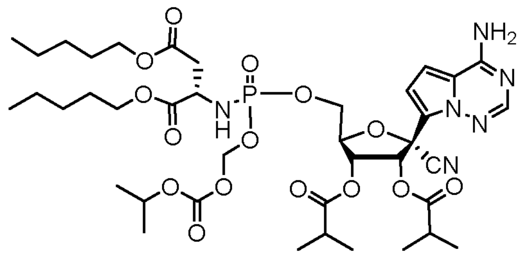

,

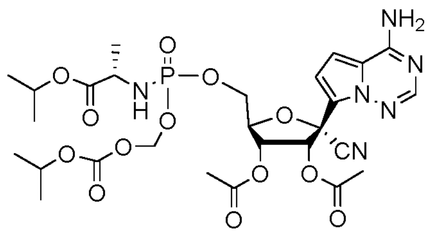

,

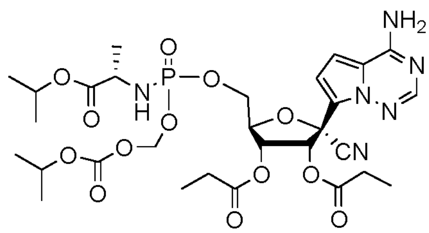

,

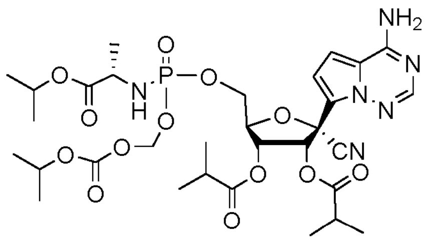

,

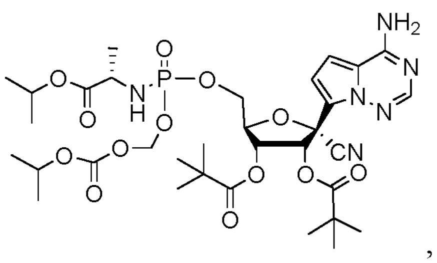

,

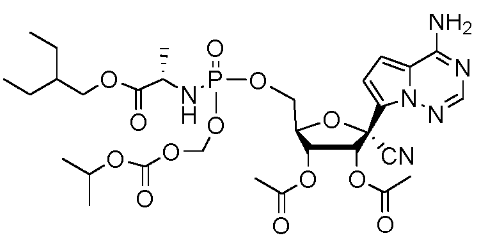

,

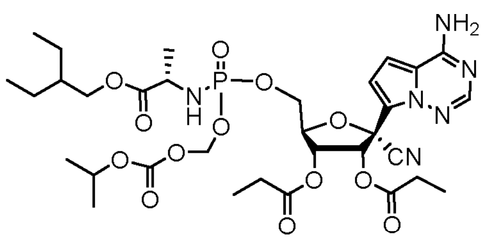

,

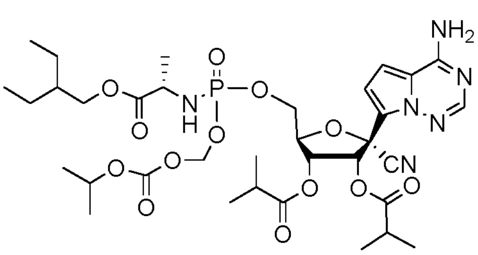

,

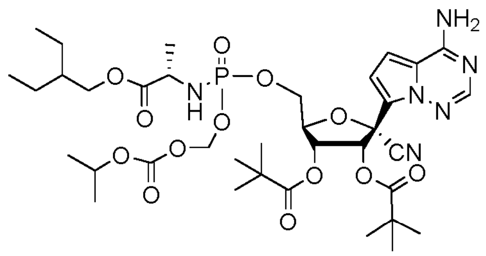

,

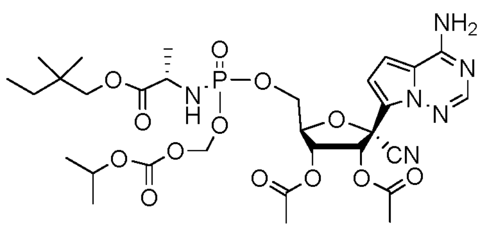,
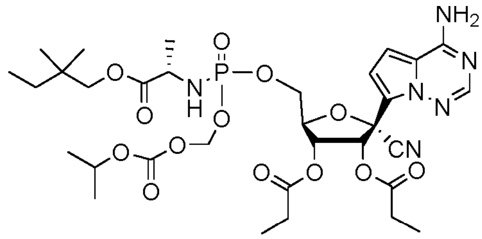,

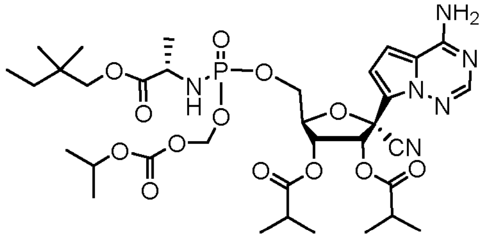,
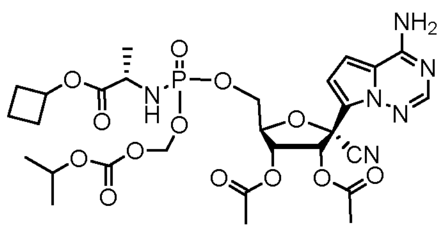,

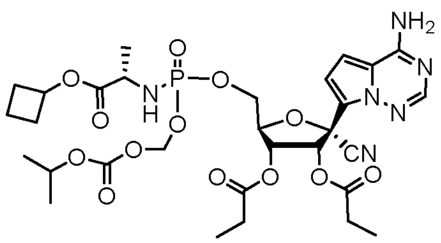,
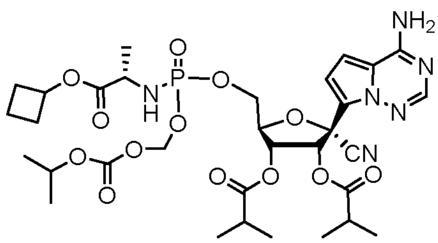,

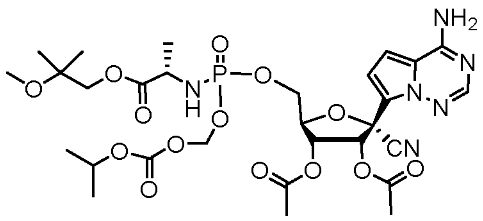,
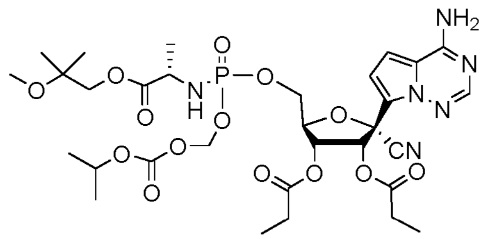,

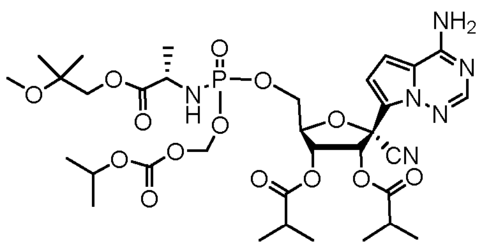,
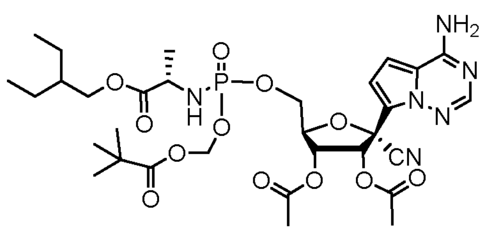,

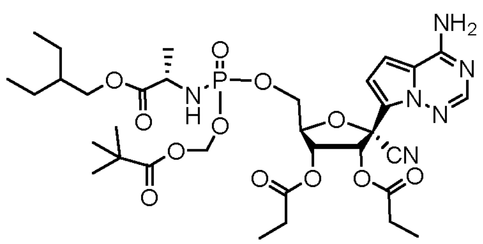,
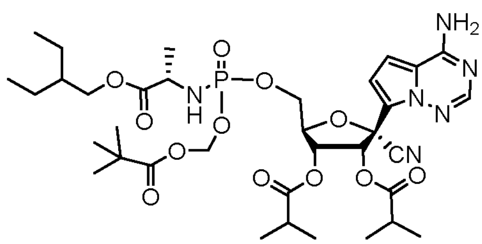,

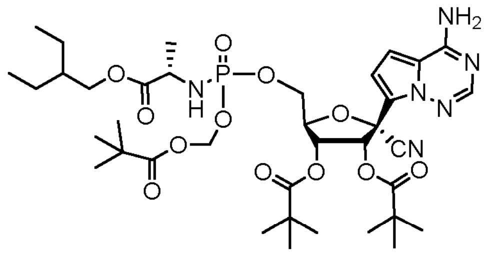,
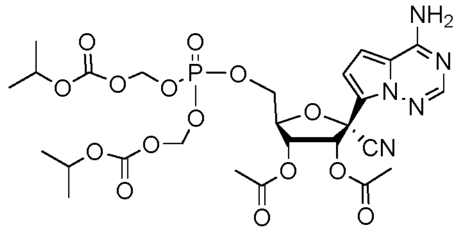,

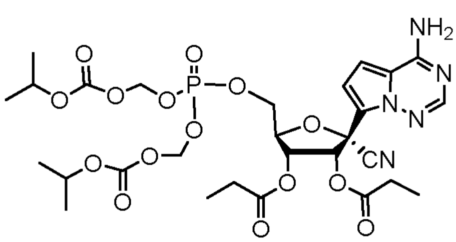,
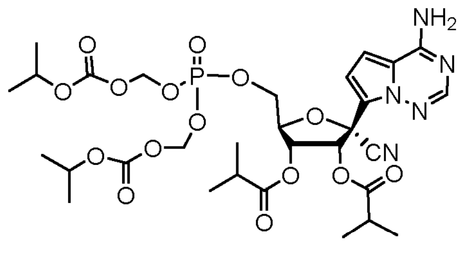,

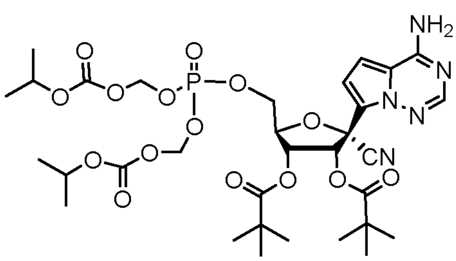,
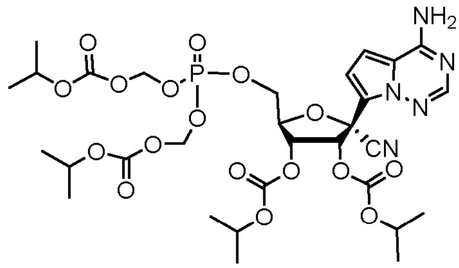,

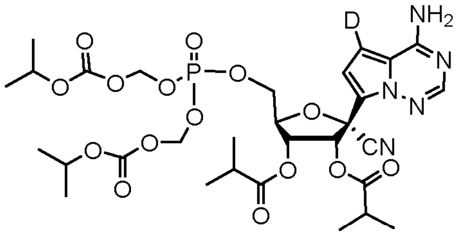,
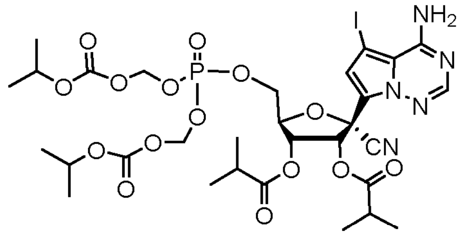,

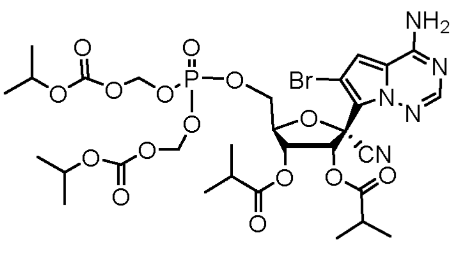,
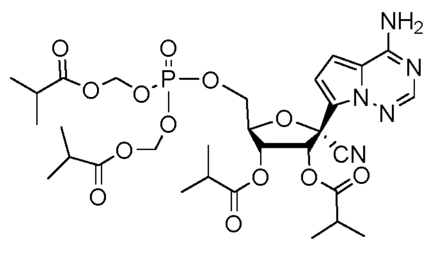,

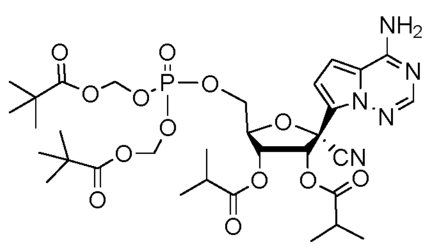,
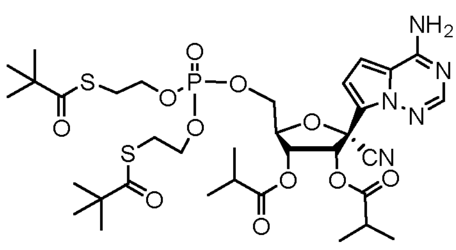,

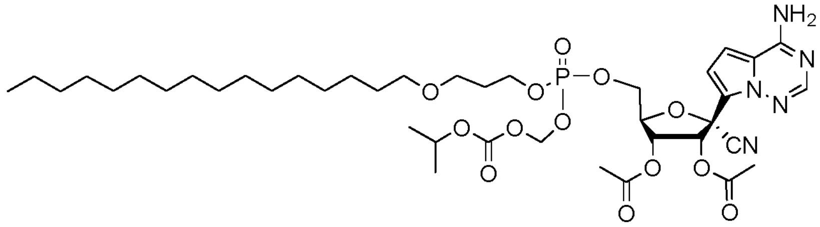

,

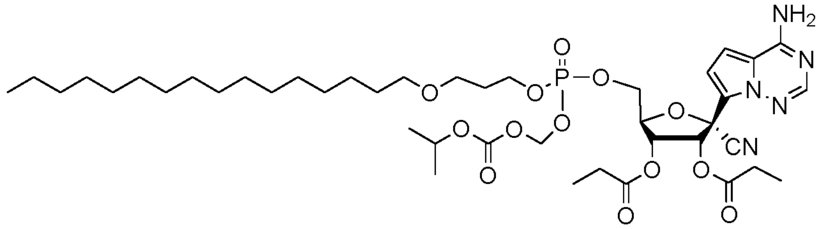

,

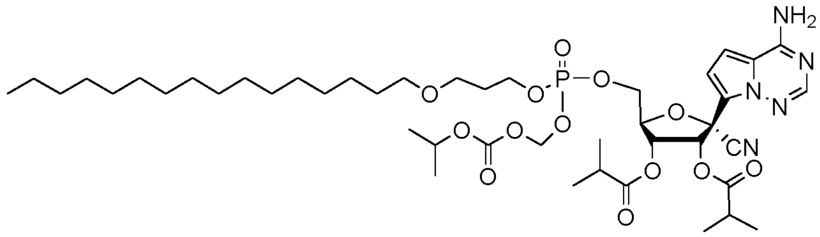

,

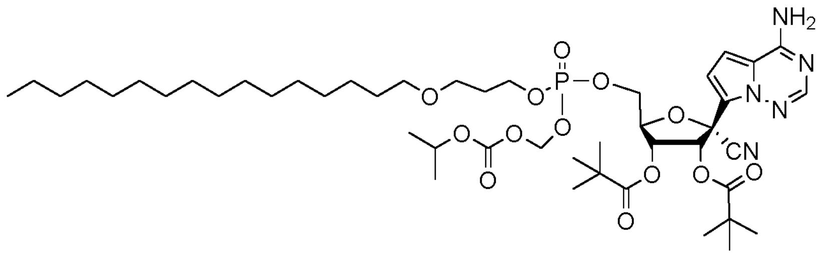

,

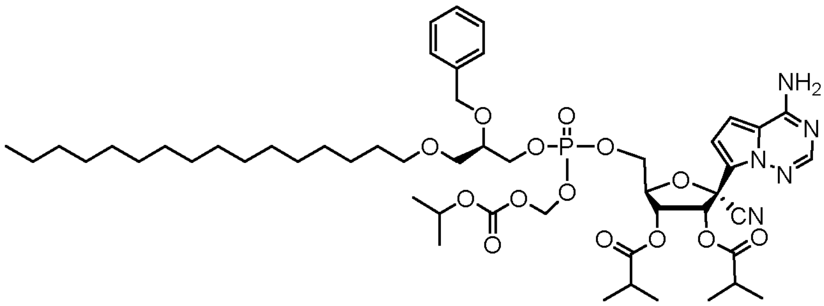

,

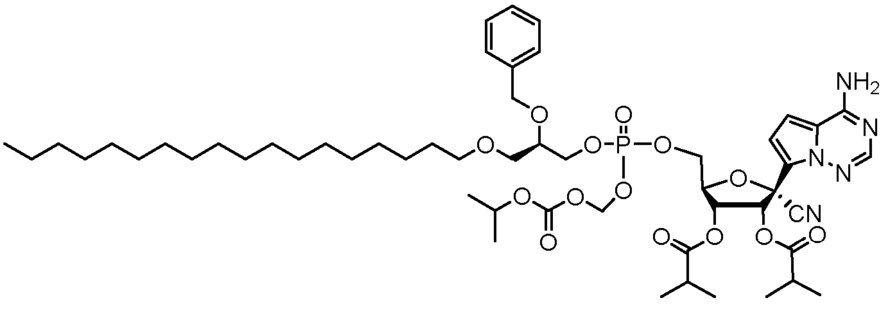

,

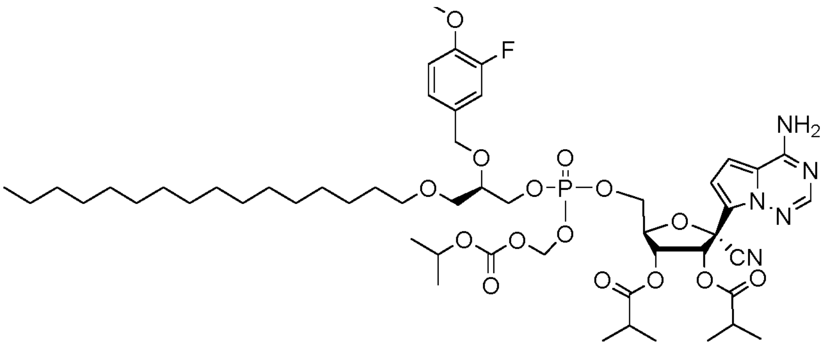

,

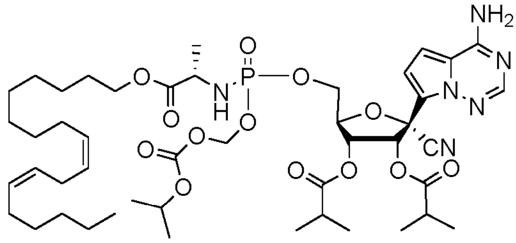

,

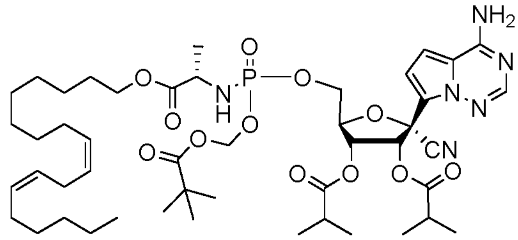

,

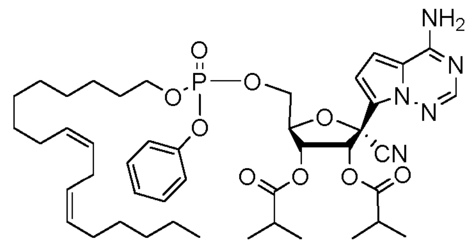

,

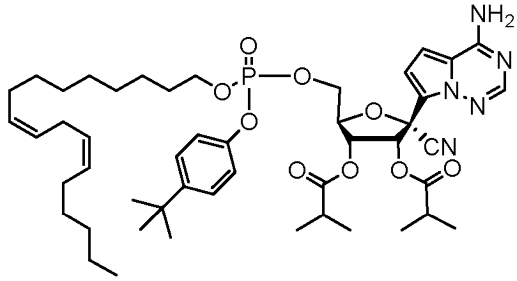

,

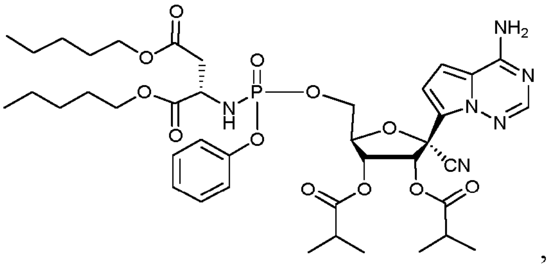

,

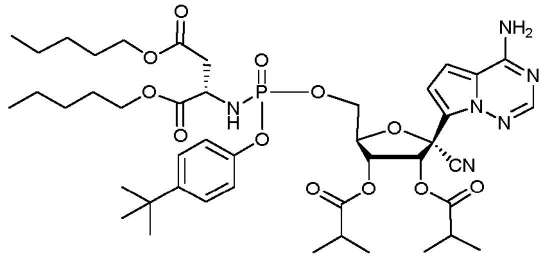

,

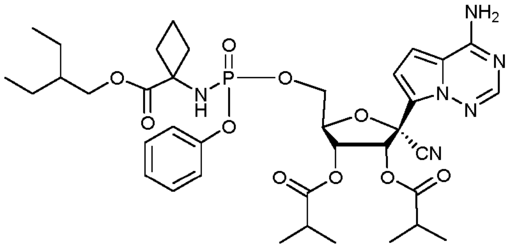,
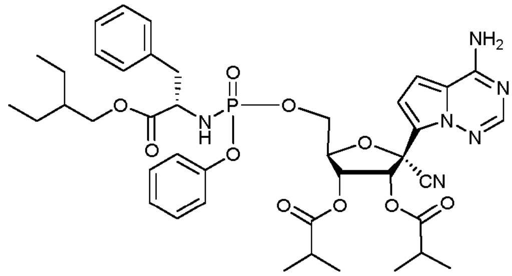,

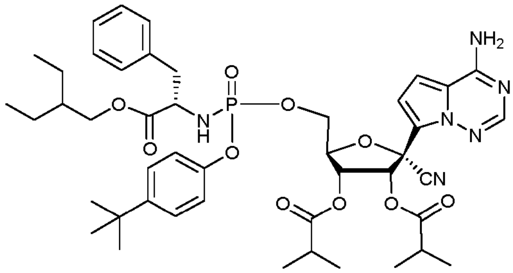,
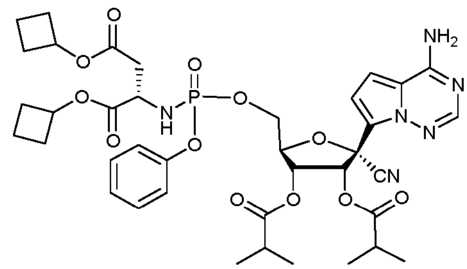,

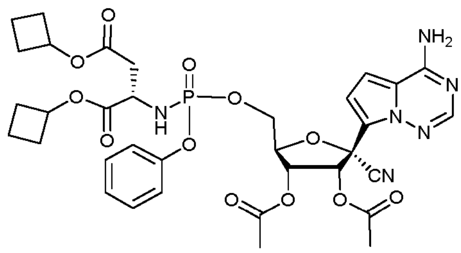,
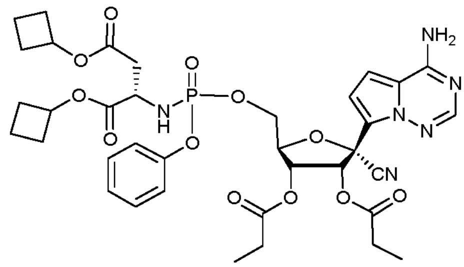,

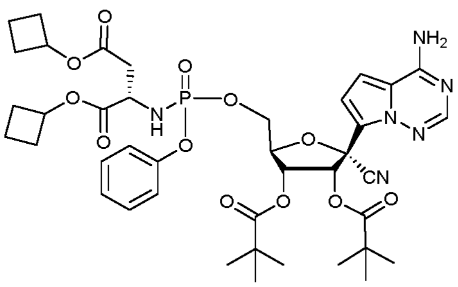,
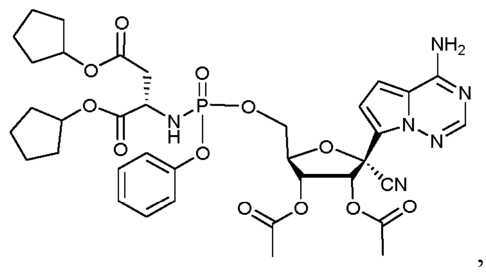,

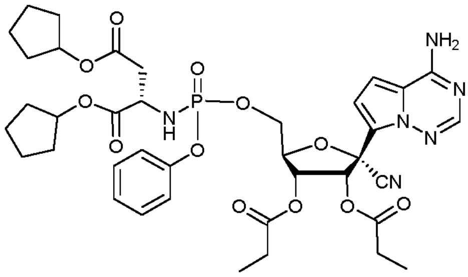,
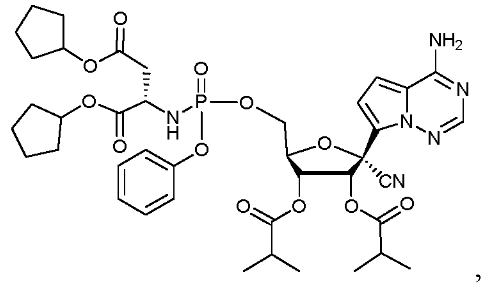,

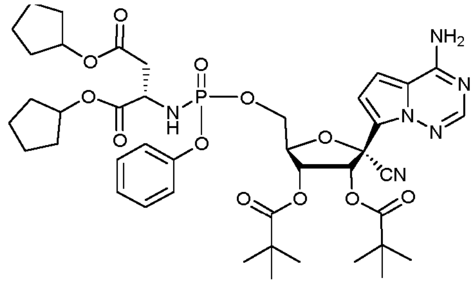

,

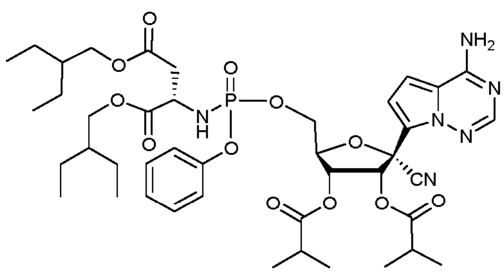

,

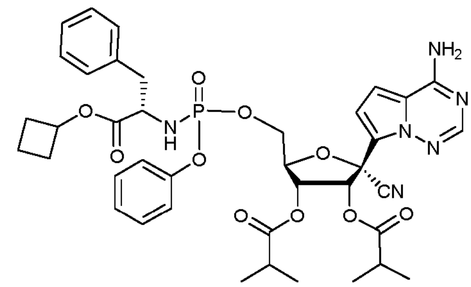

or

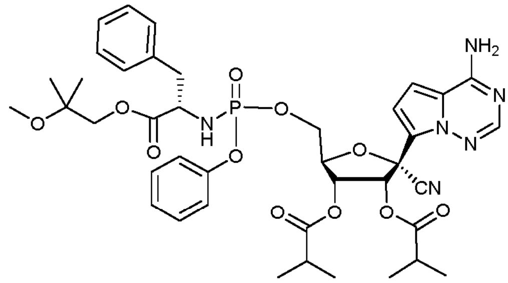

;

preferably, the compound is selected from the group consisting of:

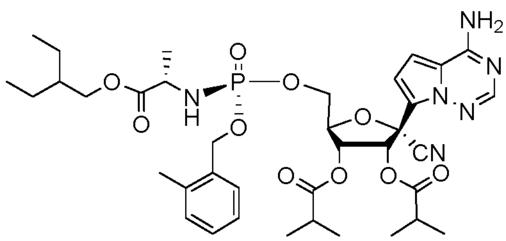

,

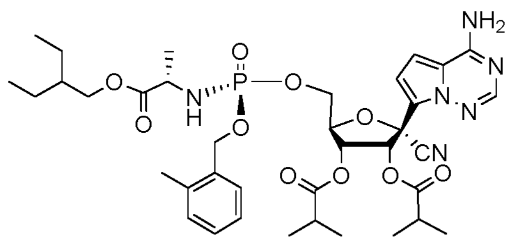

,

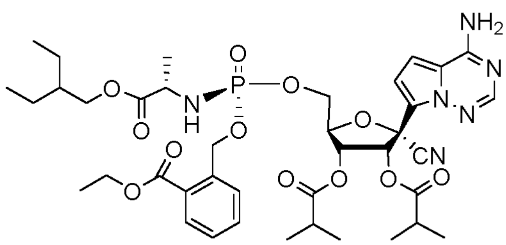

,

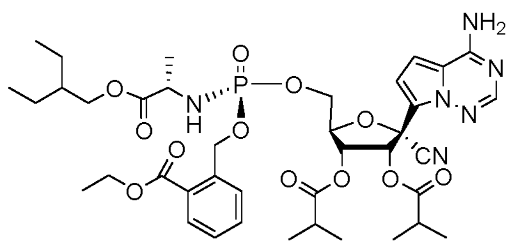

,

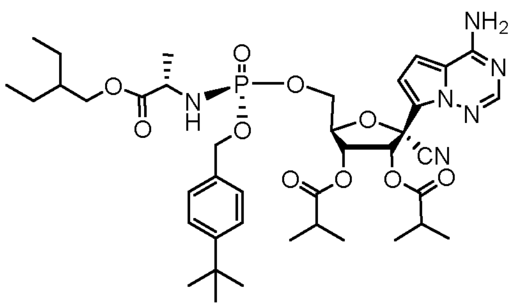

,

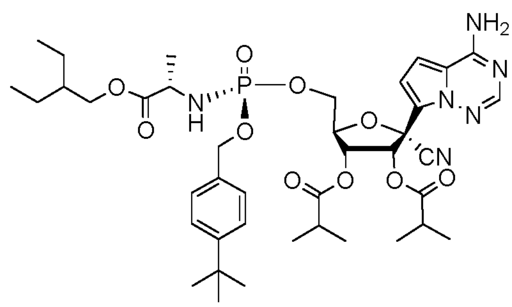

,

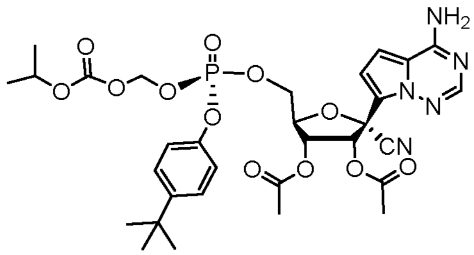
,
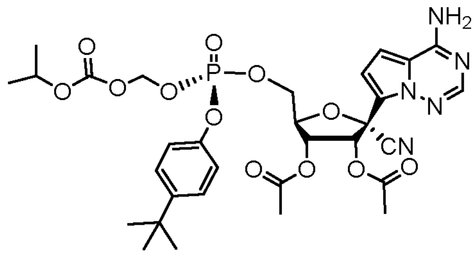
,

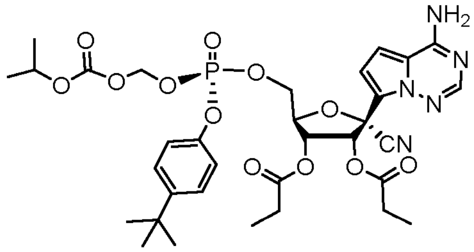
,
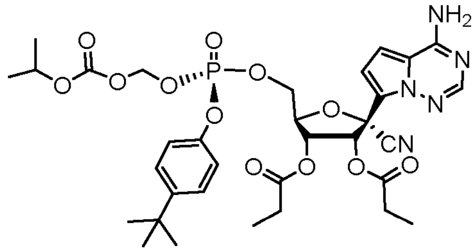
,

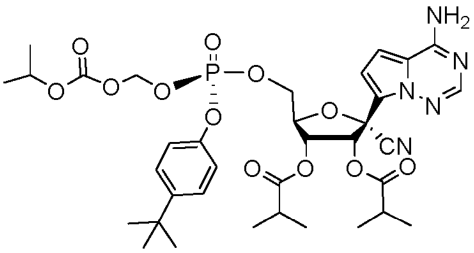
,
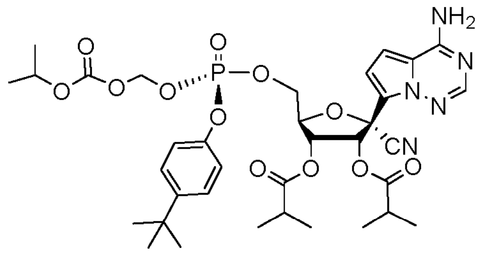
,

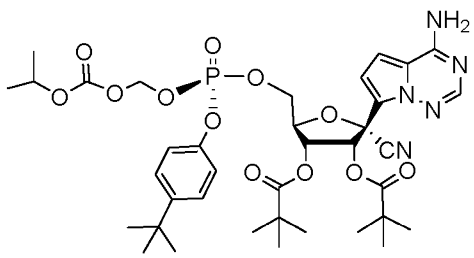
,
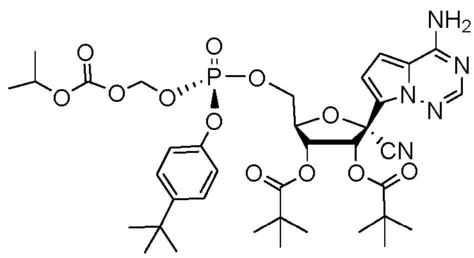
,

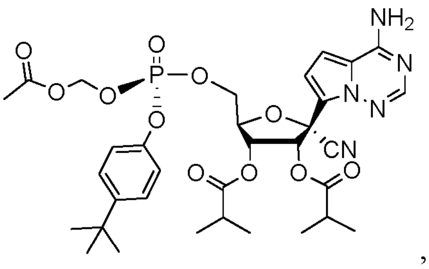
,
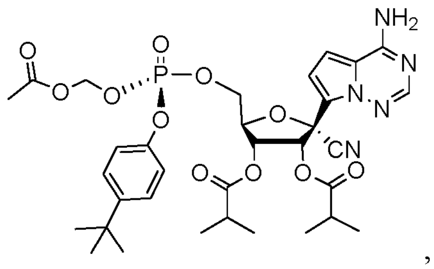
,

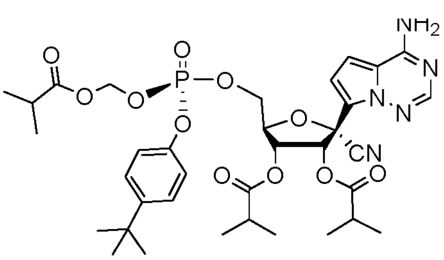
,
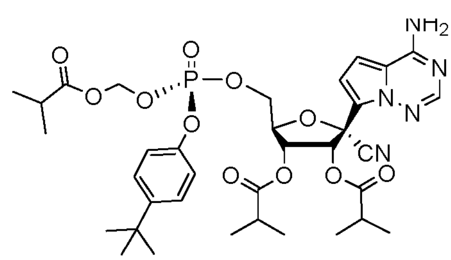
,

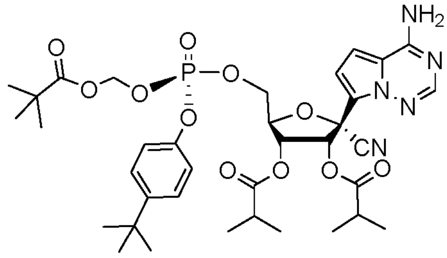,
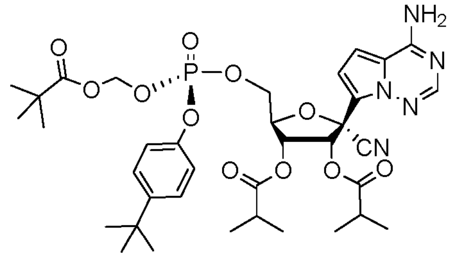,

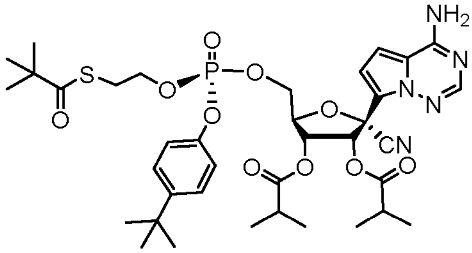,
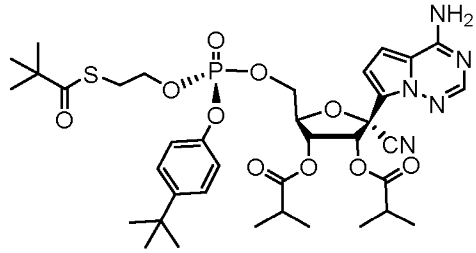,

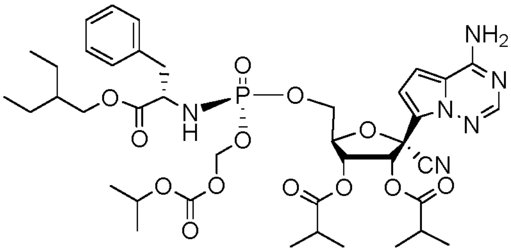,
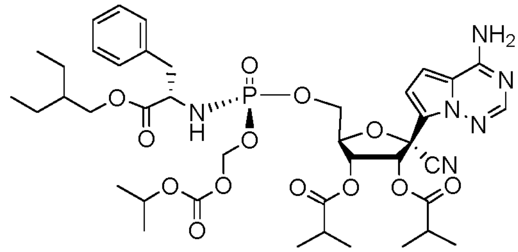,

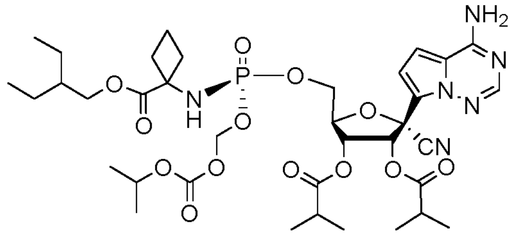,
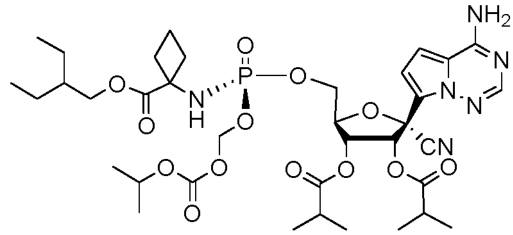,

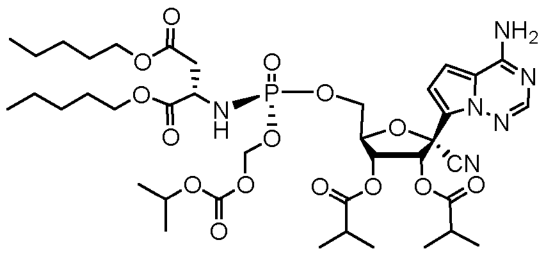,
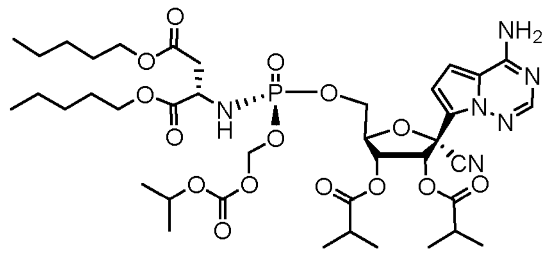,

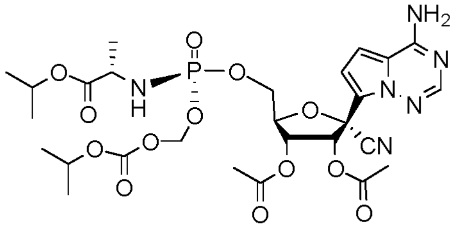,
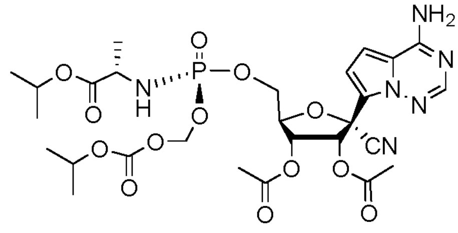,

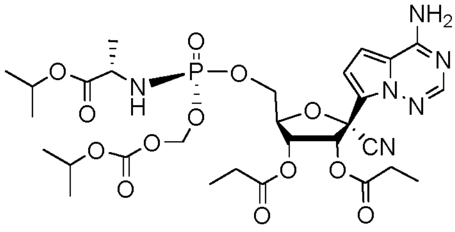,
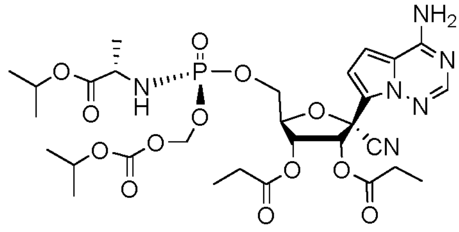,

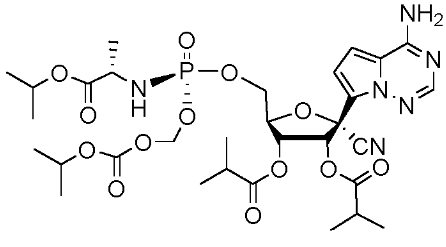,
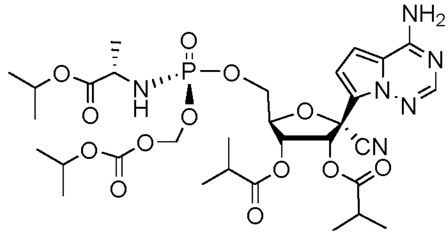,

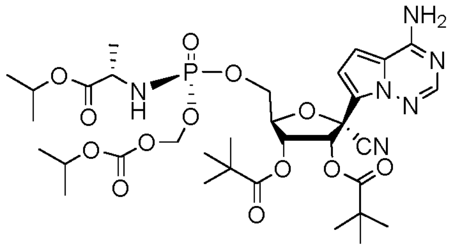,
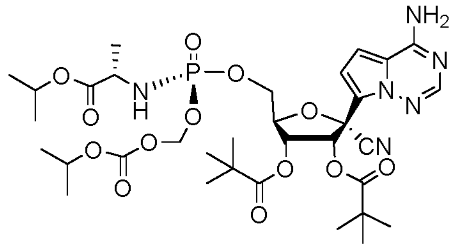,

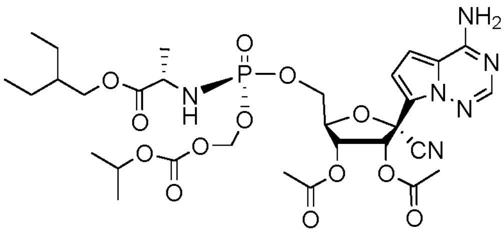,
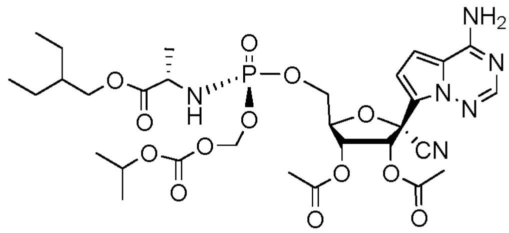,

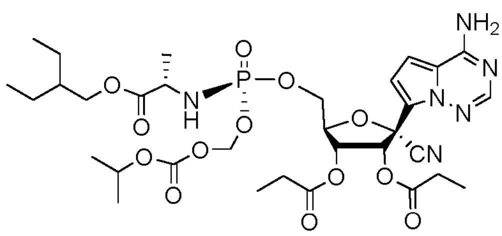,
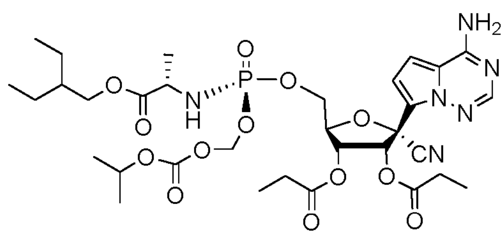,

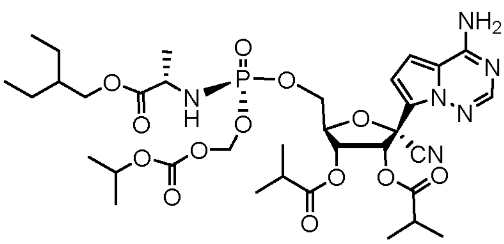,
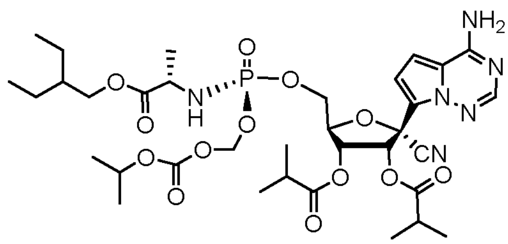,

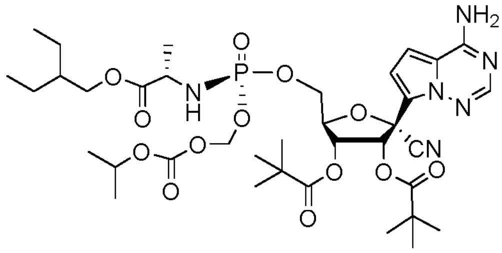,
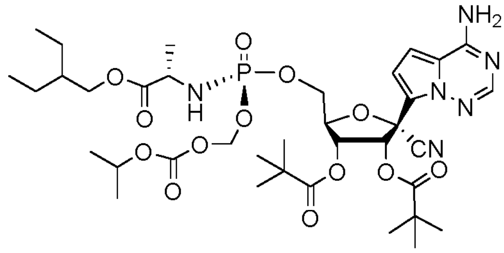,
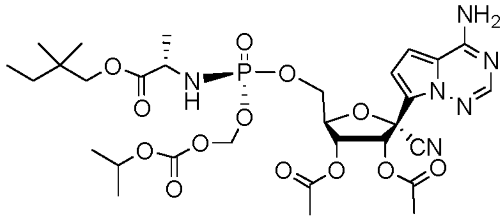,
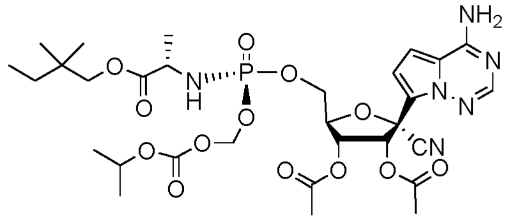,
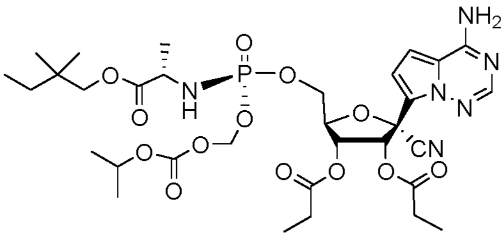,
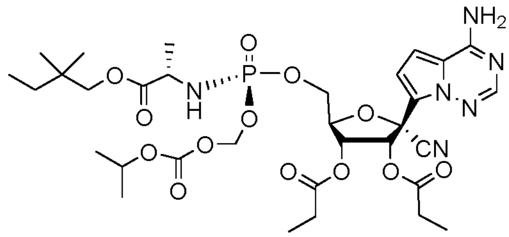,
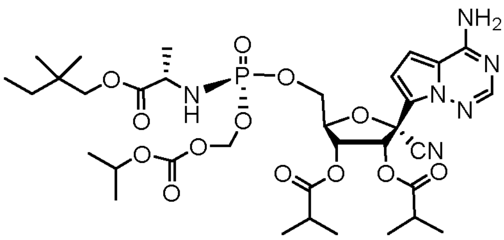,
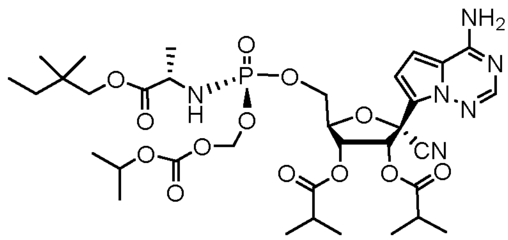,
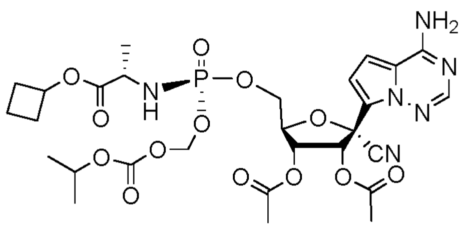,
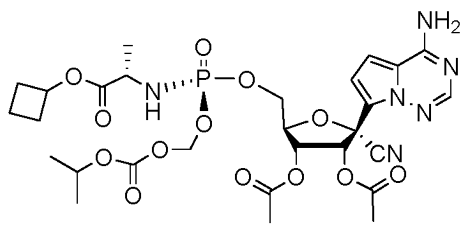,
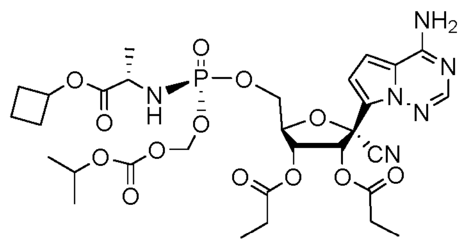,
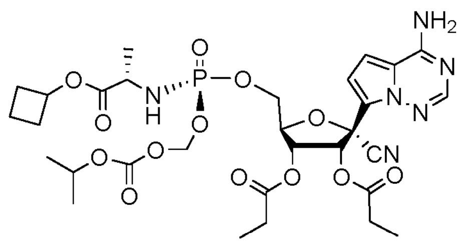,

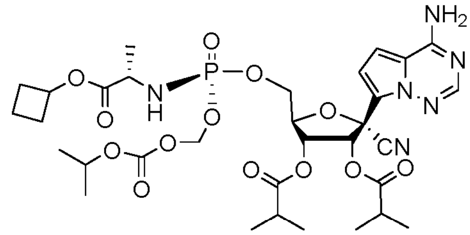
,
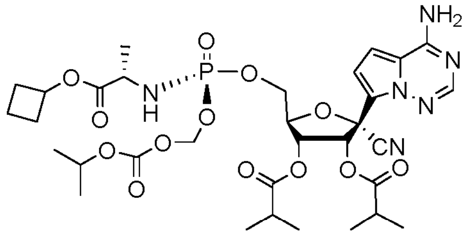
,
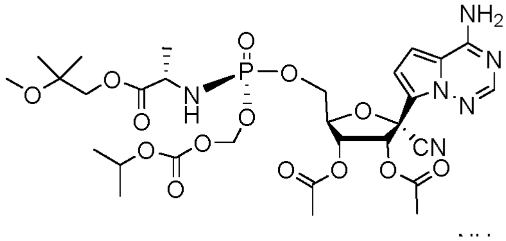
,
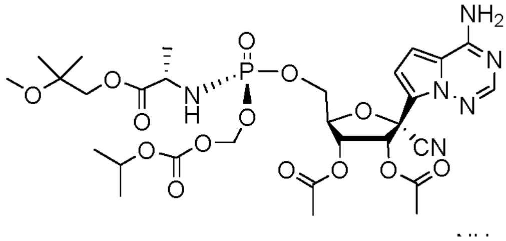
,
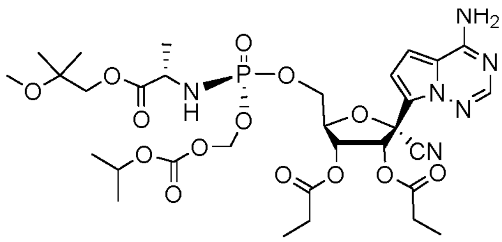
,
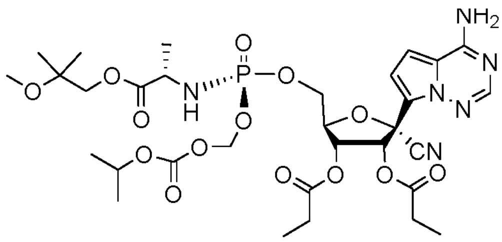
,
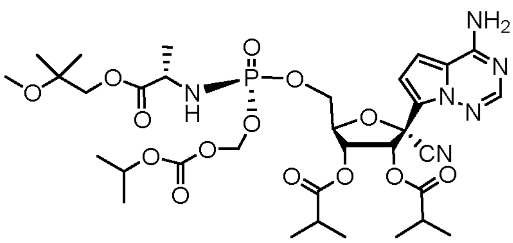
,
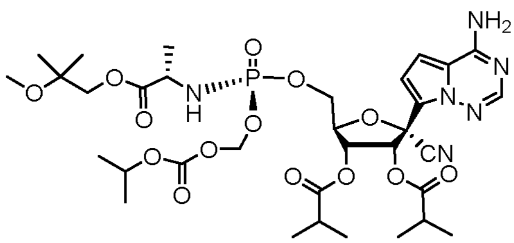
,
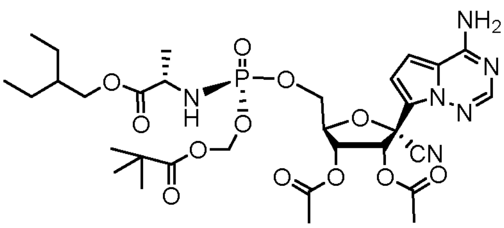
,
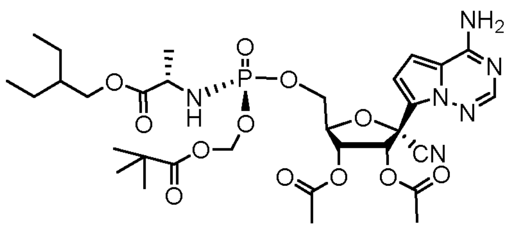
,
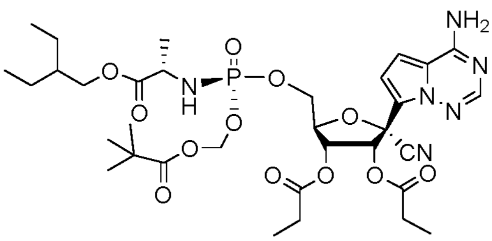
,
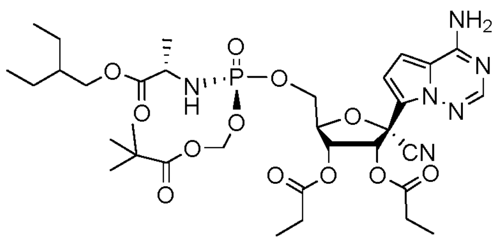
,

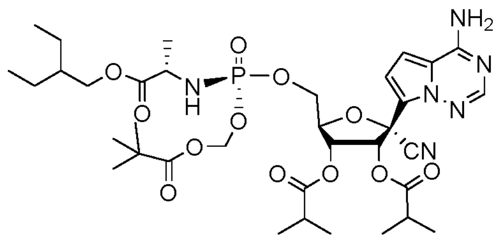 , 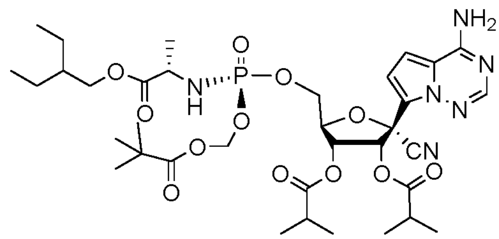 ,

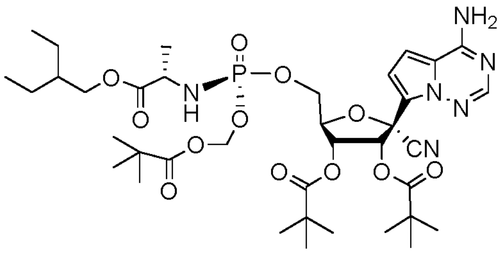 , 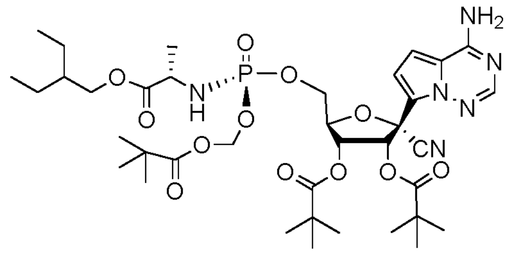 ,

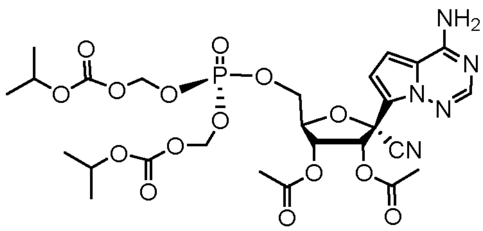 , 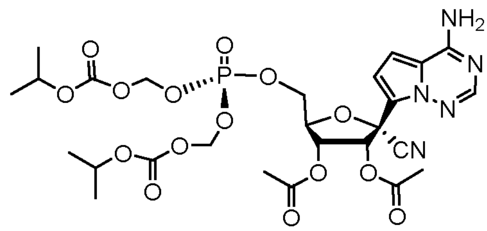 ,

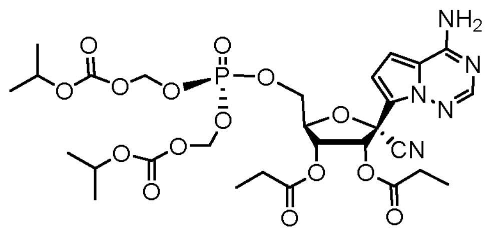 , 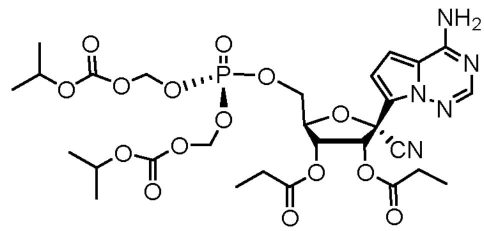 ,

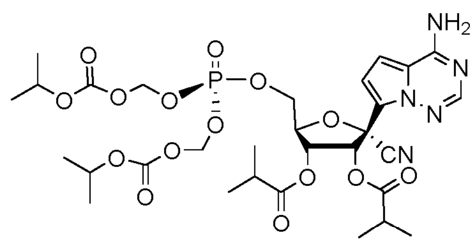 , 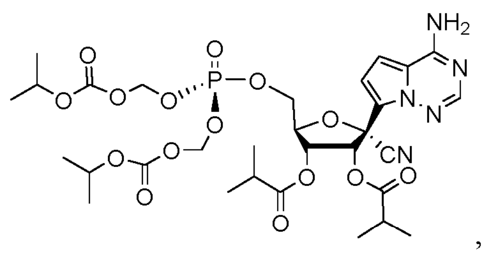 ,

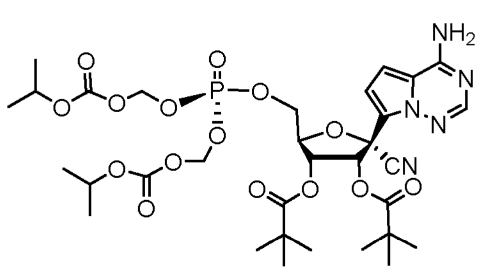 , 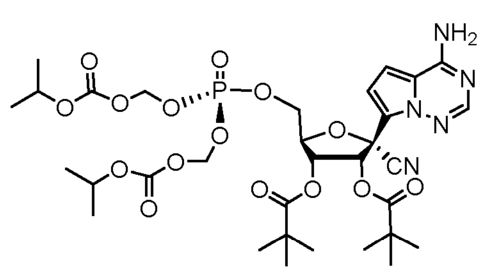 ,

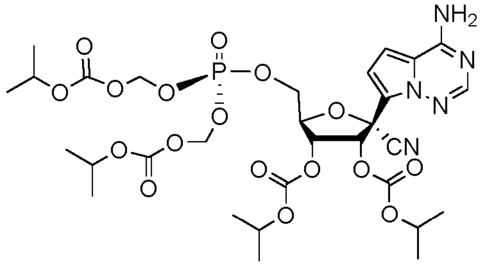,
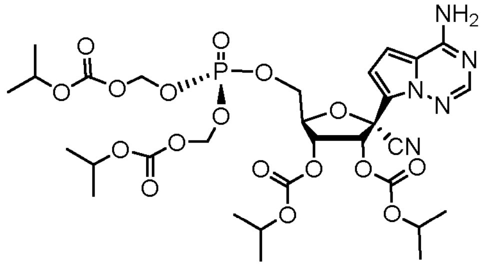,

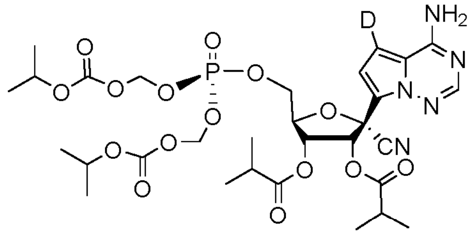,
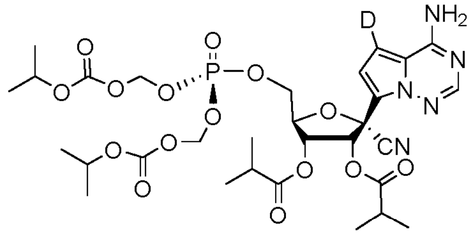,

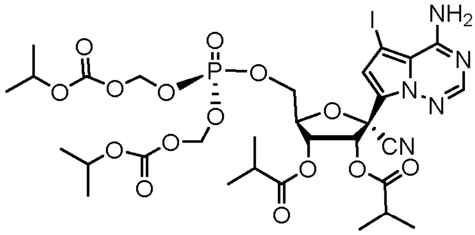,
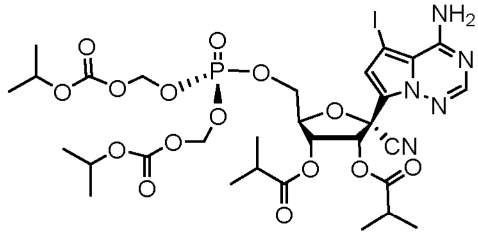,

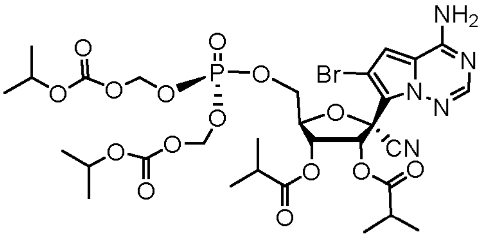,
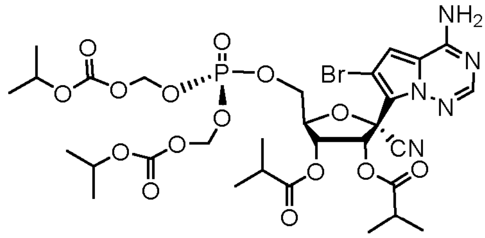,

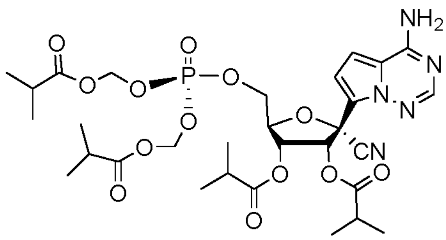,
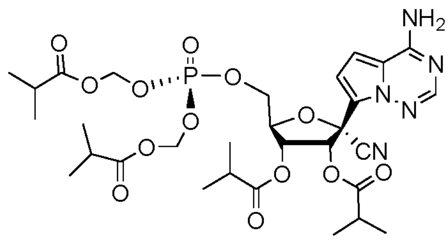,

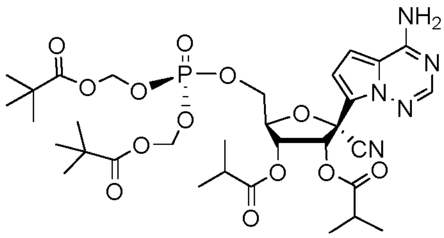,
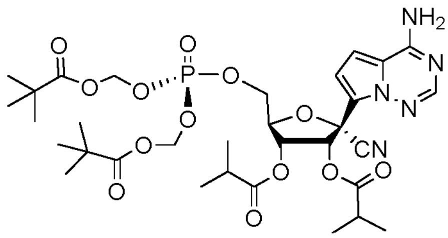,

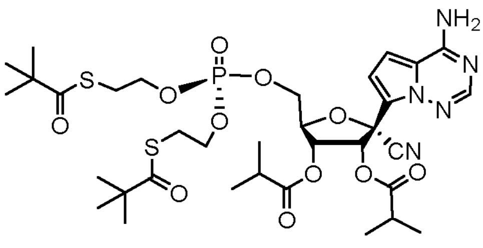
,
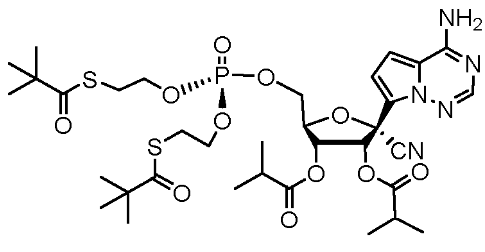
,

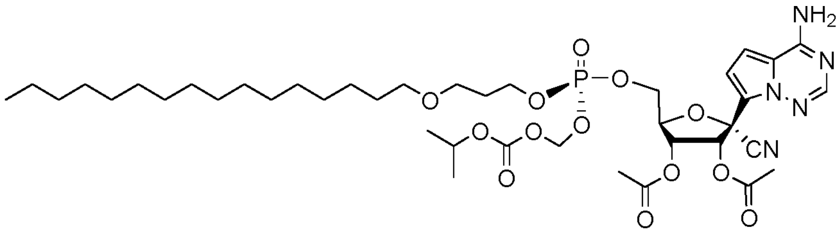
,

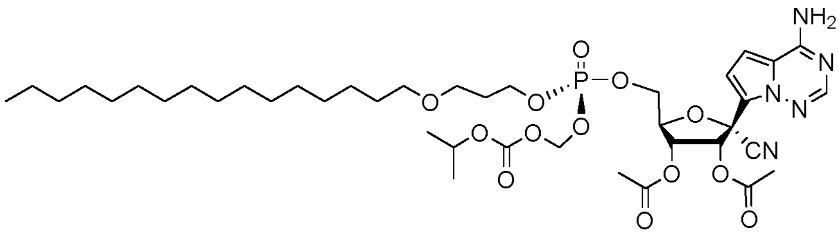
,

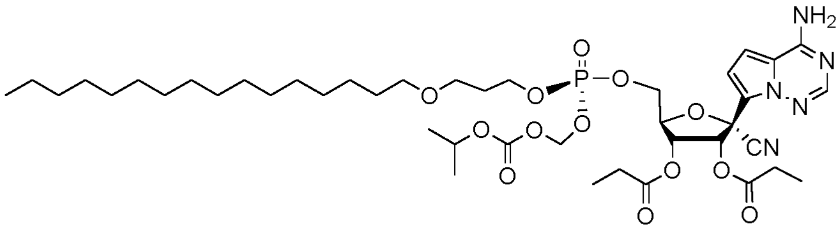
,

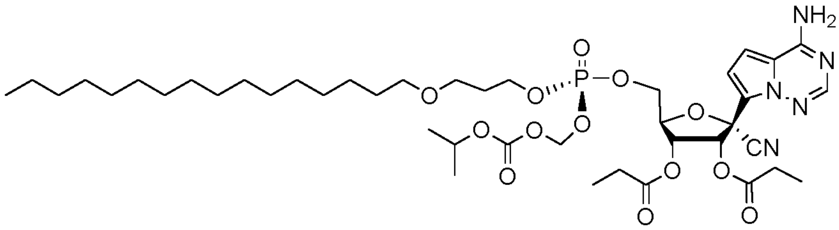
,

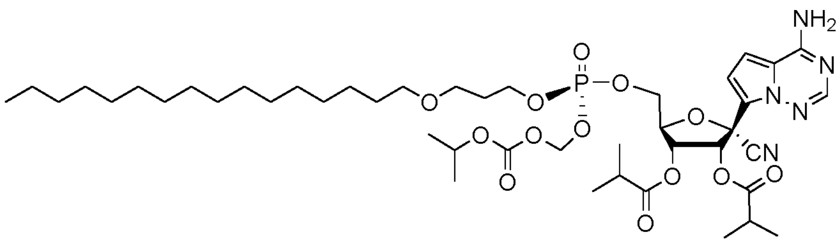
,

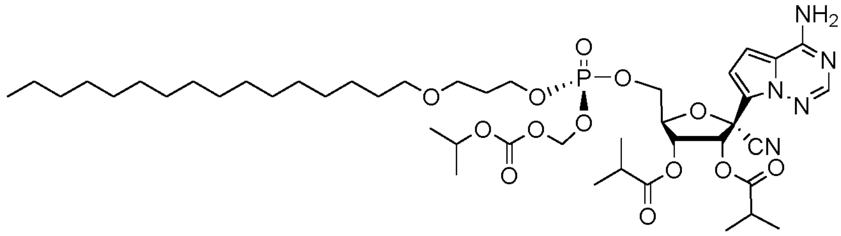
,

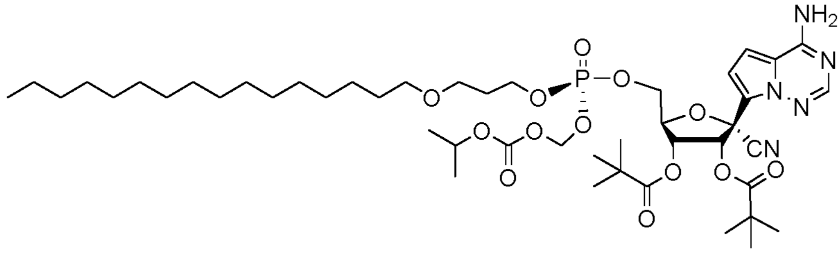
,

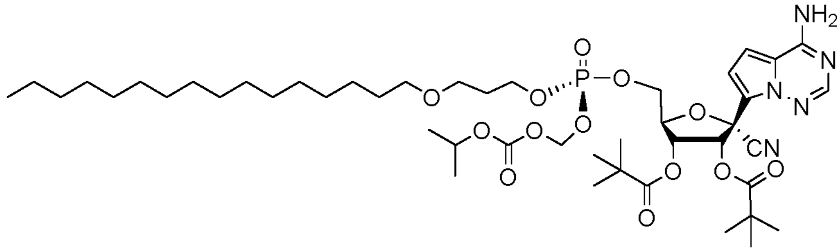
,

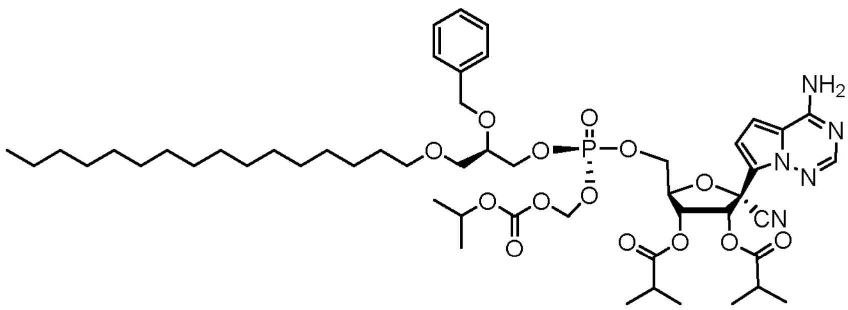
,

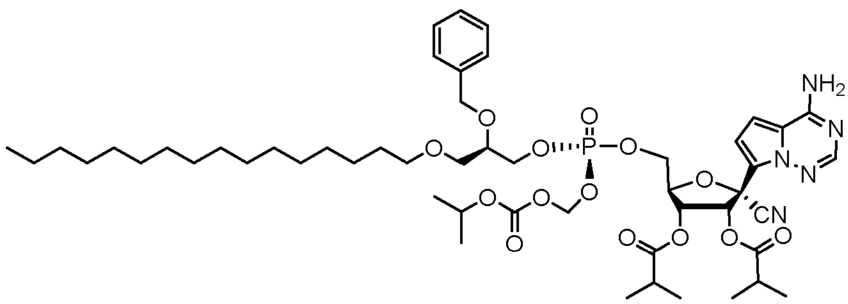
,

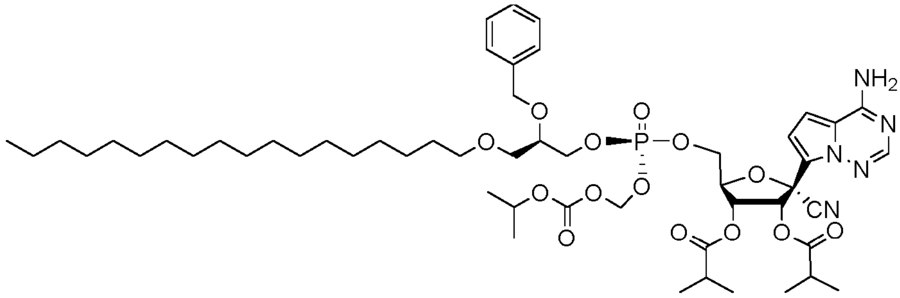

,

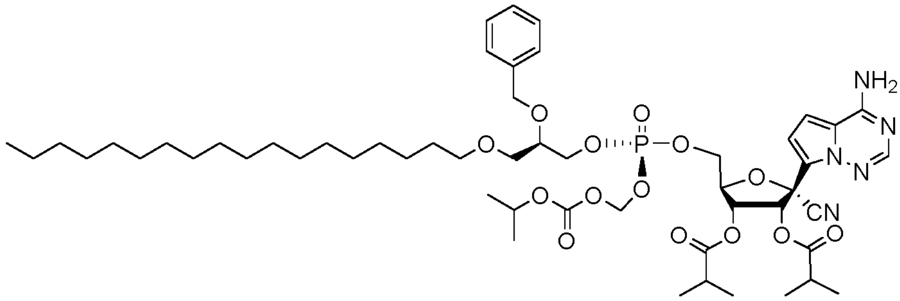

,

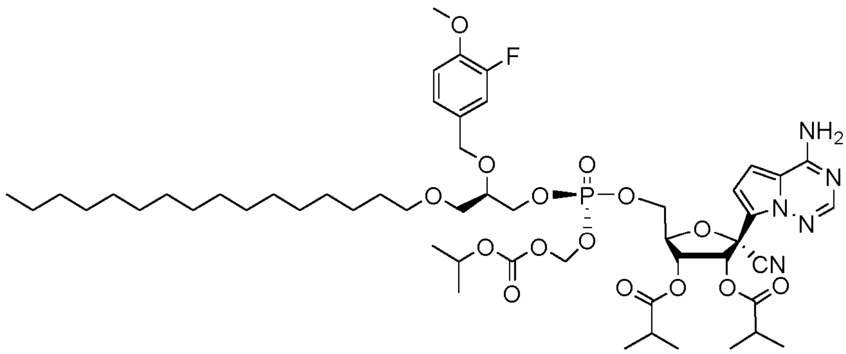

,

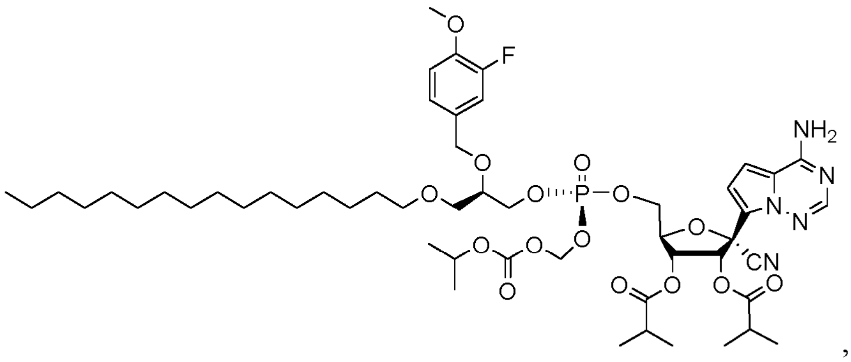

,

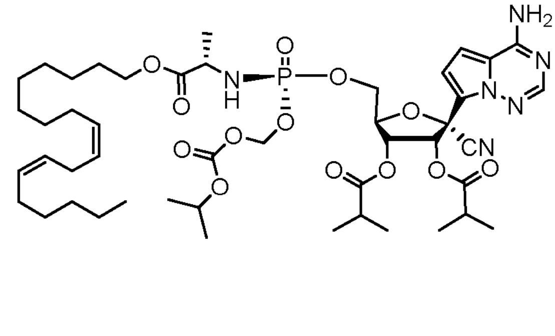

,

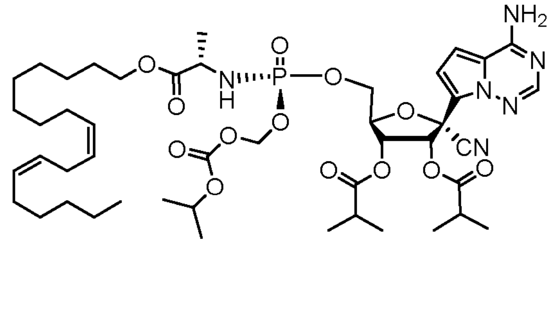

,

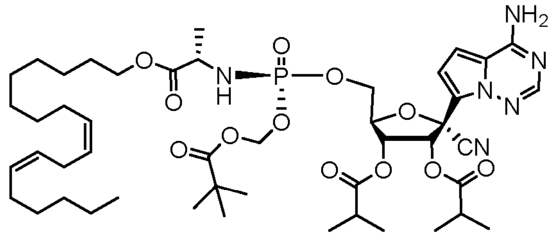 , 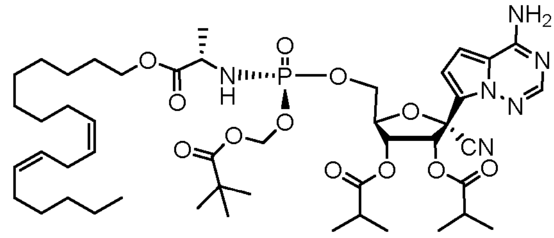 ,

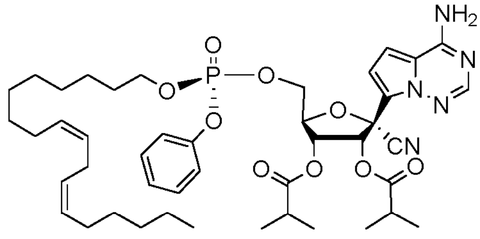 , 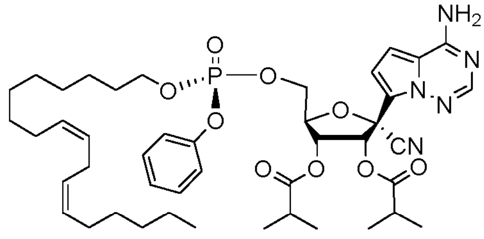 ,

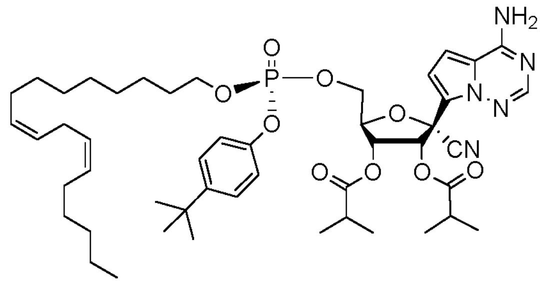 , 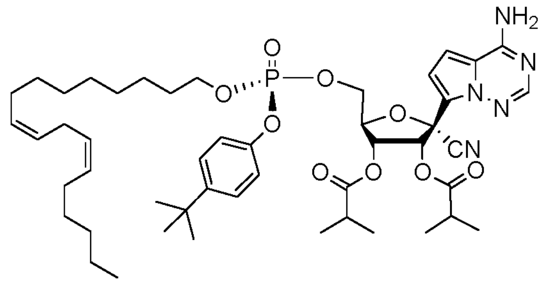 ,

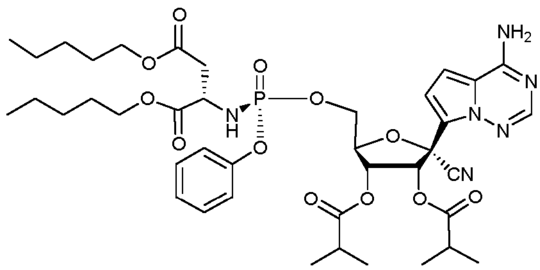 , 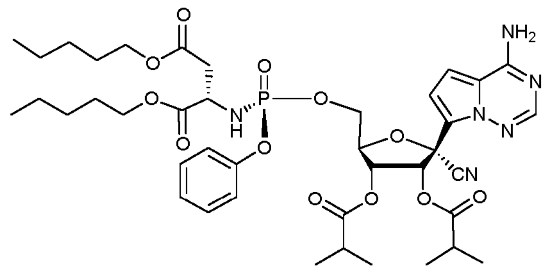 ,

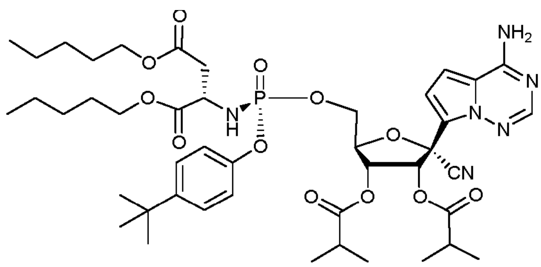 , 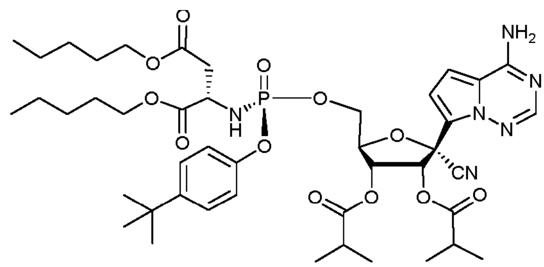 ,

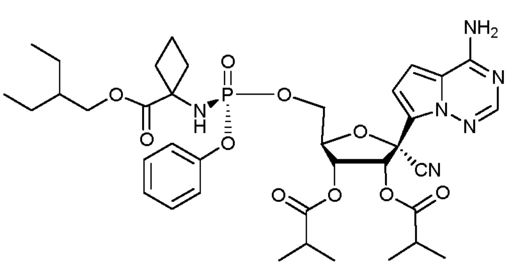 , 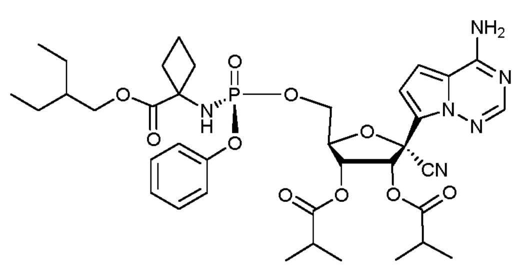 ,

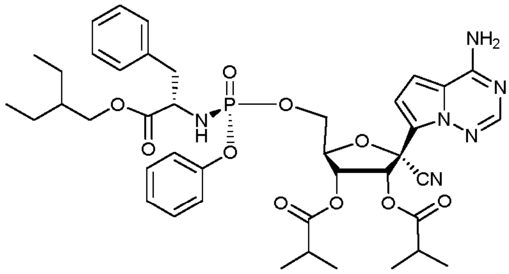
,
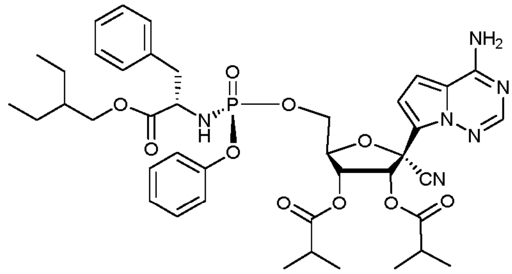
,
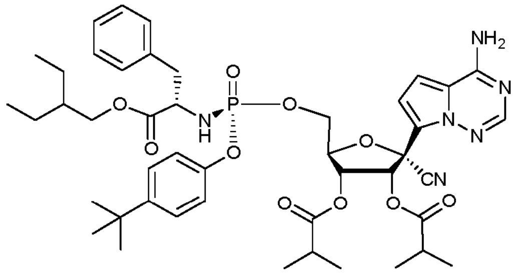
,
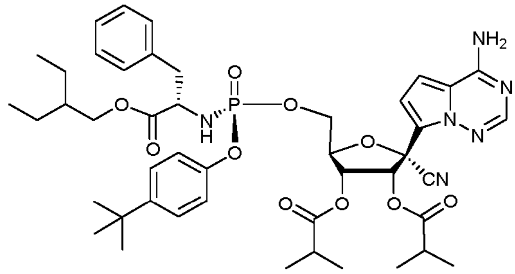
,
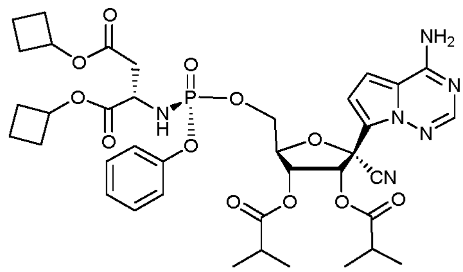
,
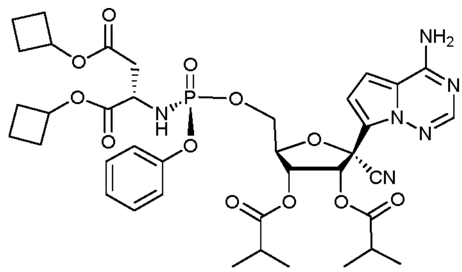
,
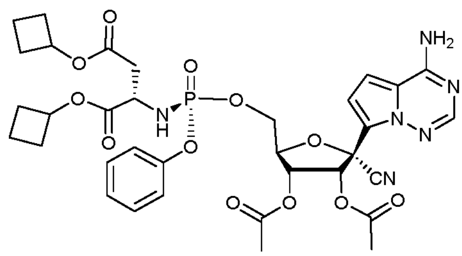
,
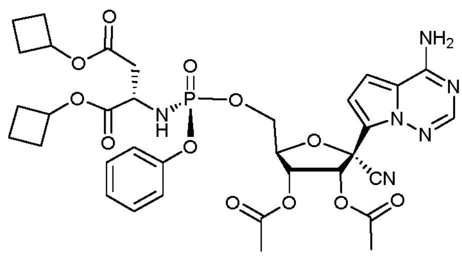
,
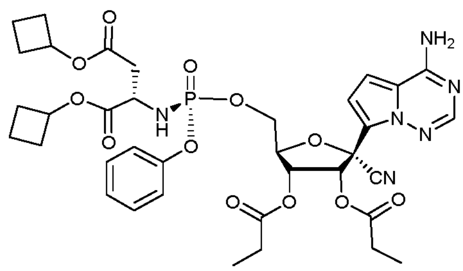
,
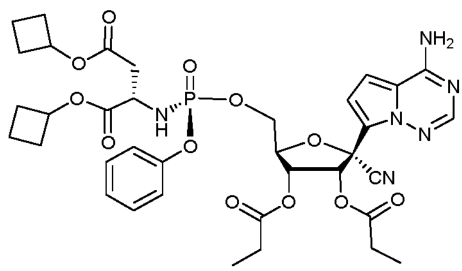
,

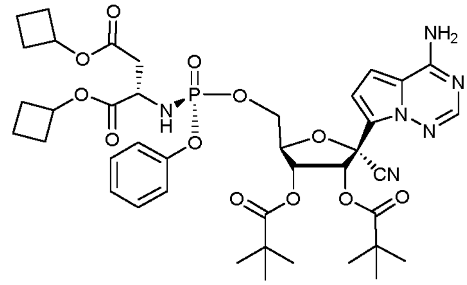
,
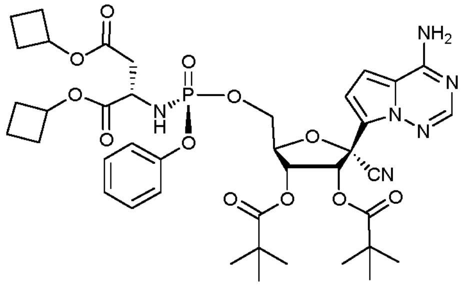
,

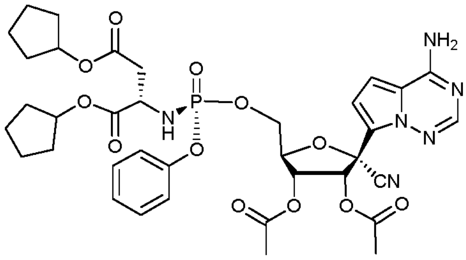
,
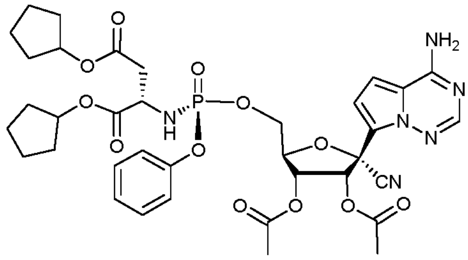
,

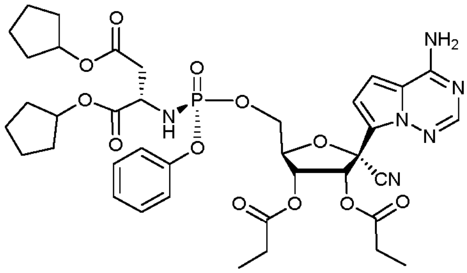
,
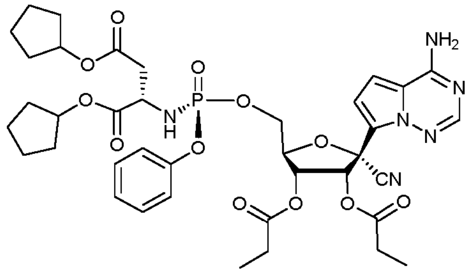
,

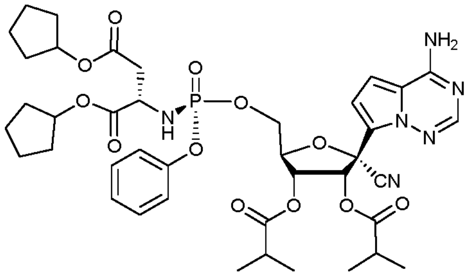
,
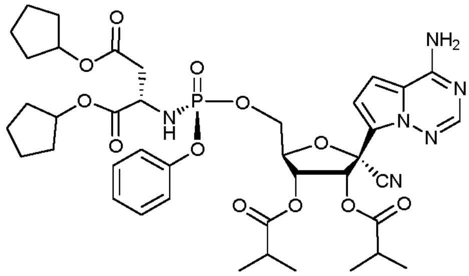
,

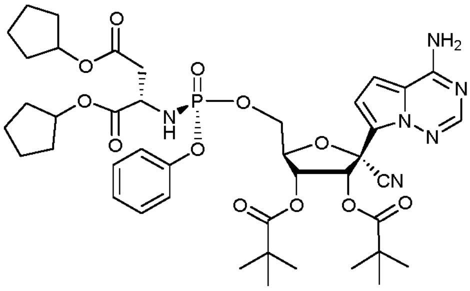
,
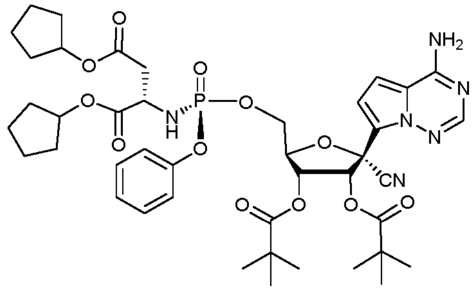
,

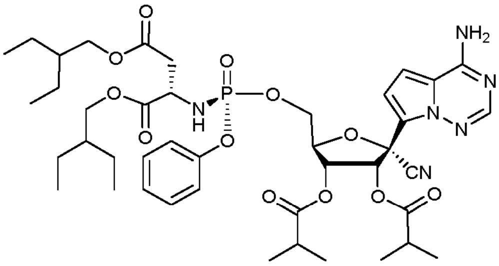,

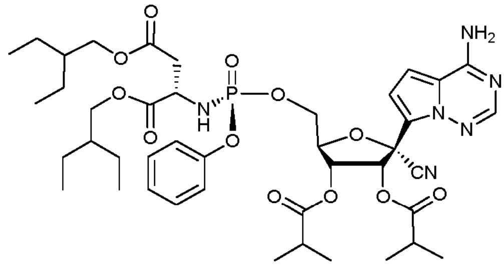,

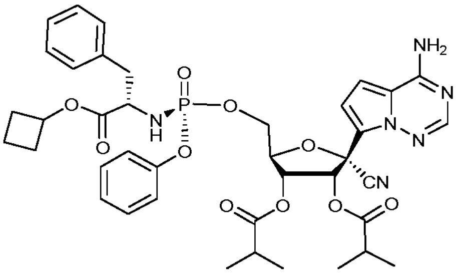,

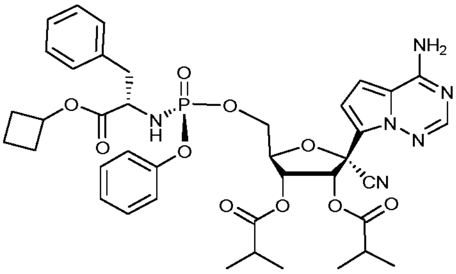,

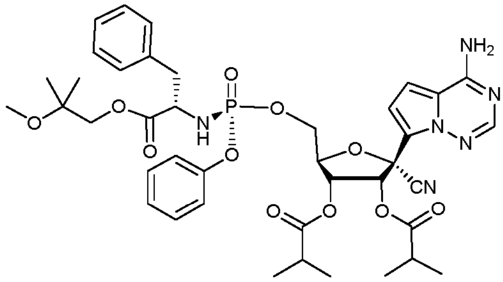

or

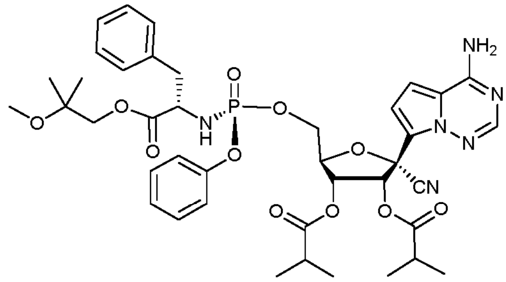.

**58.** An intermediate compound, or tautomer, stereoisomer, prodrug, crystalline form, pharmaceutically acceptable salt, hydrate or solvate thereof, wherein, the intermediate compound is selected from the group consisting of:

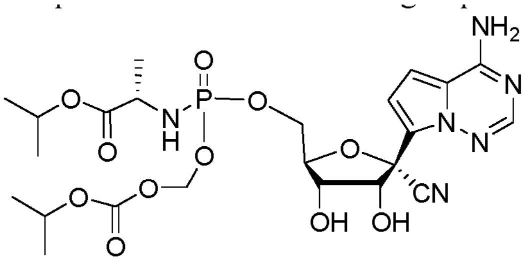,

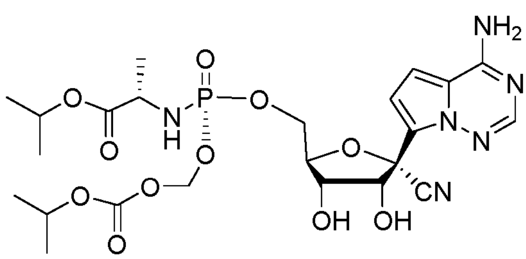,

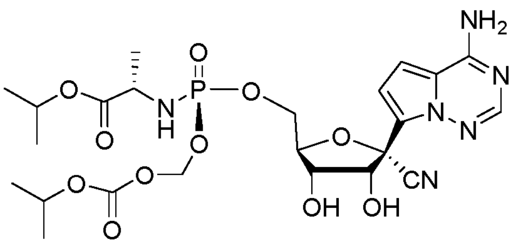,

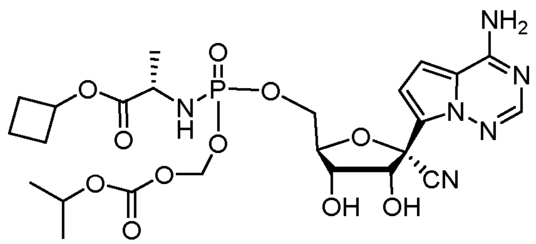,

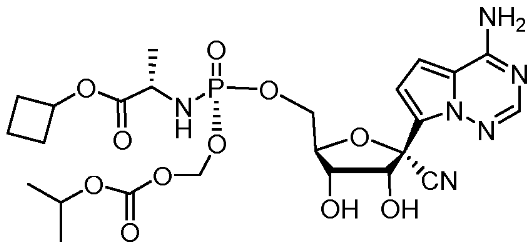
,
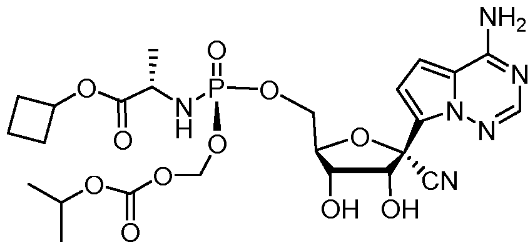
,
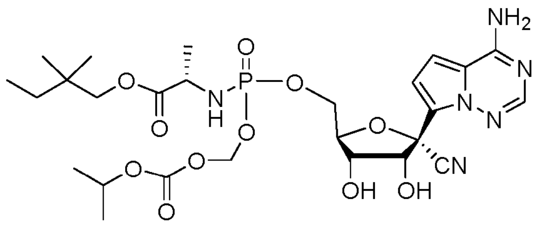
,
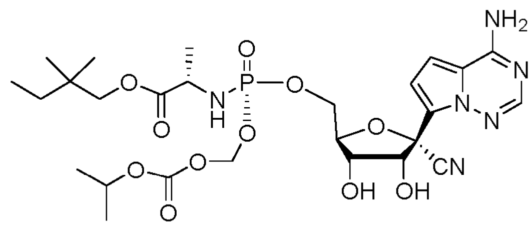
,
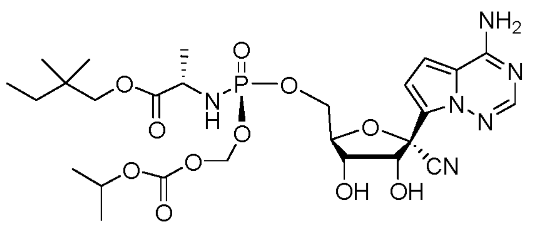
,
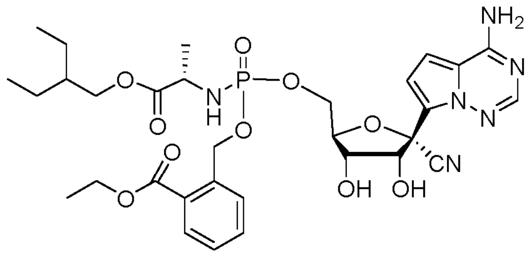
,
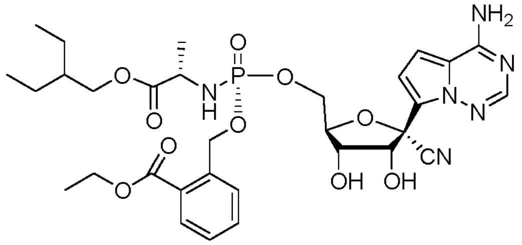
,
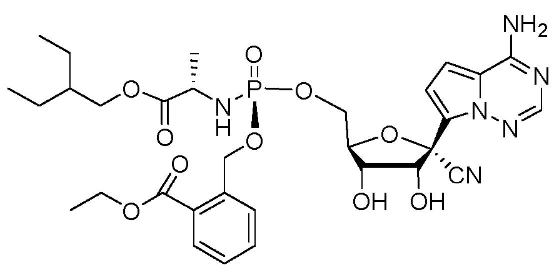
,
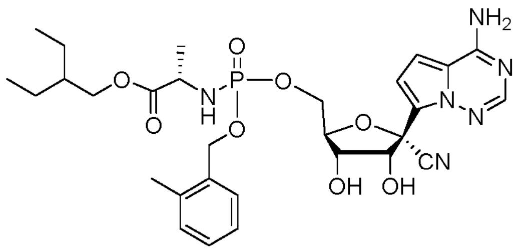
,
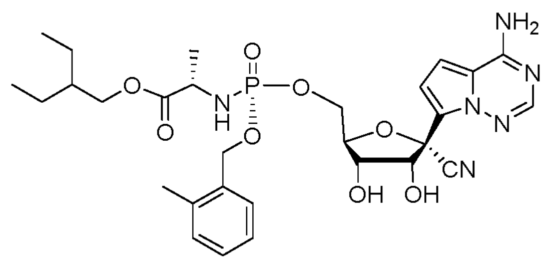
,

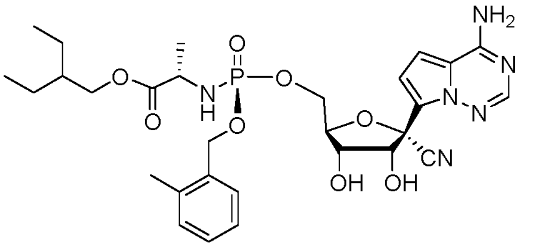,

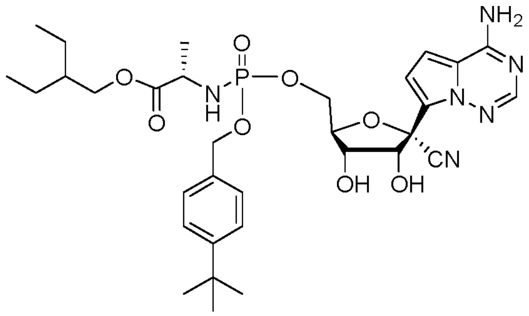,

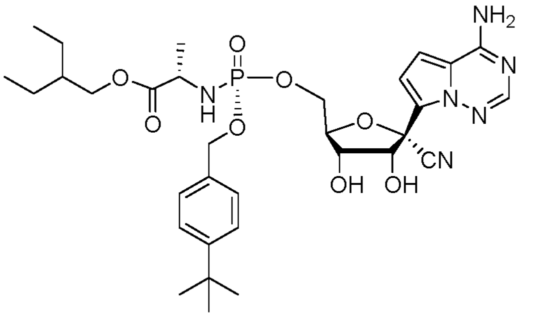,

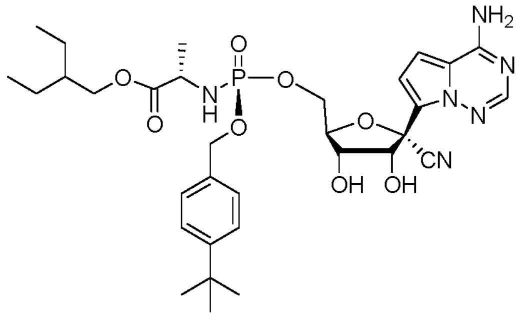,

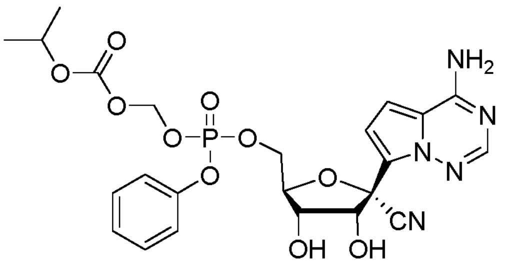,

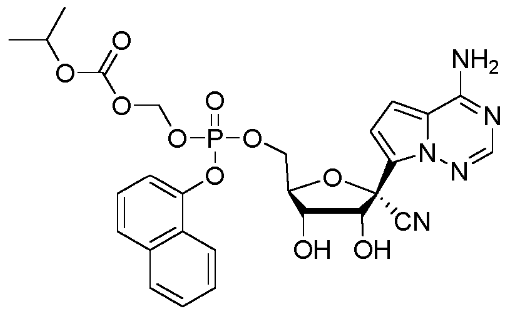,

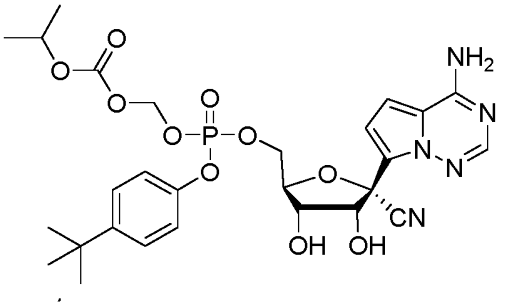,

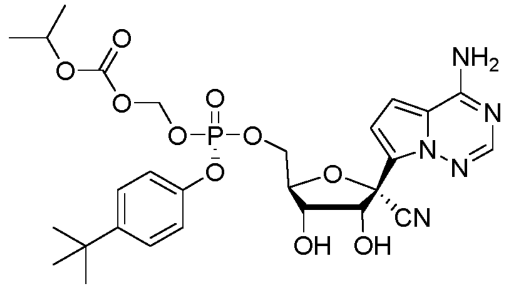,

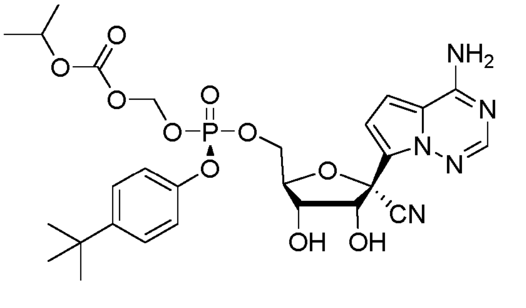,

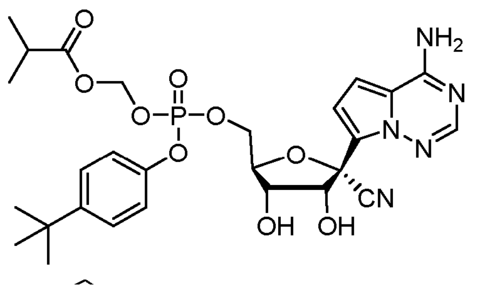,

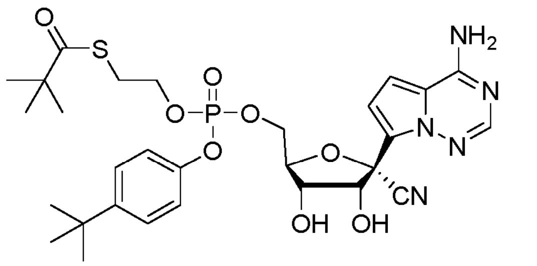
,
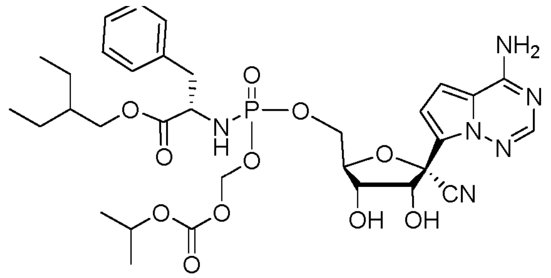
,
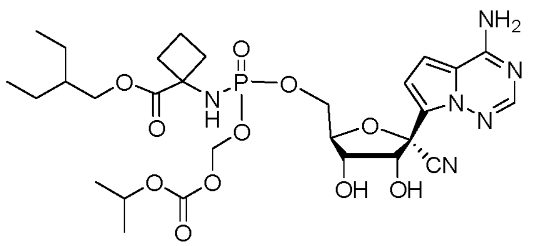
,
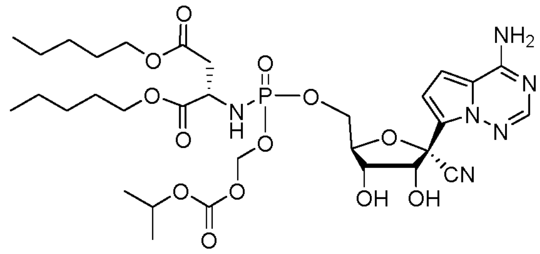
,
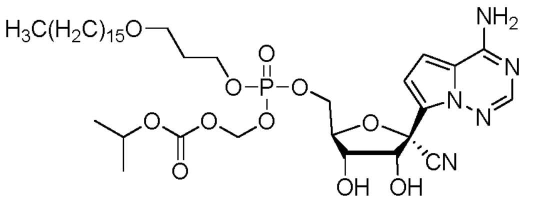
,
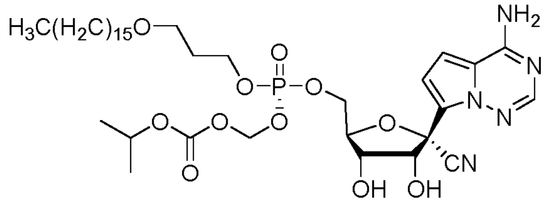
,
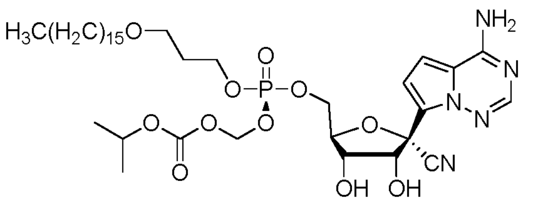
,
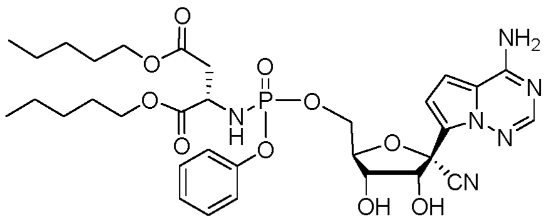
,
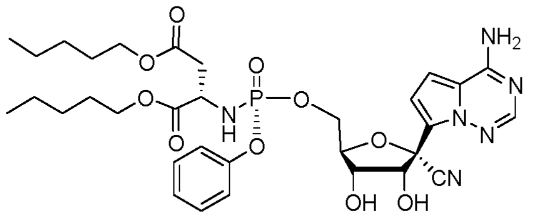
,
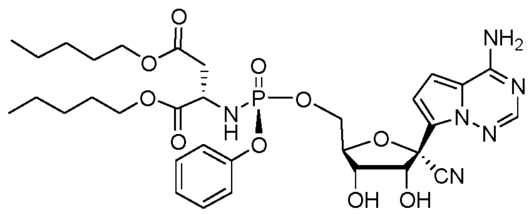
,
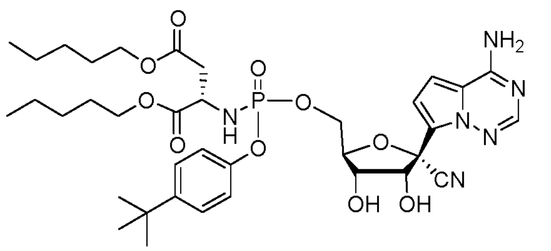
,
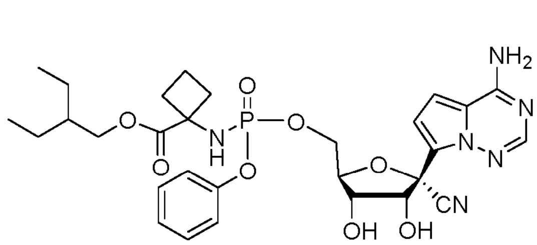
,

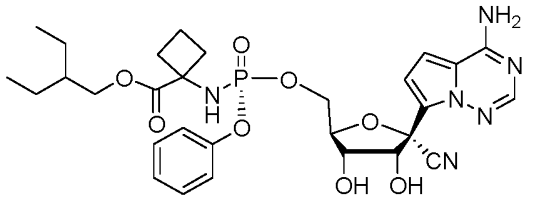,

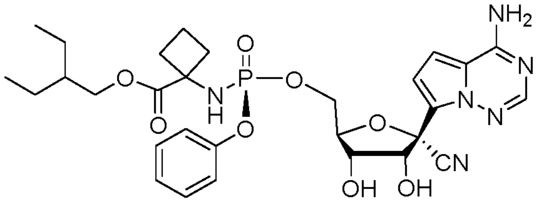,

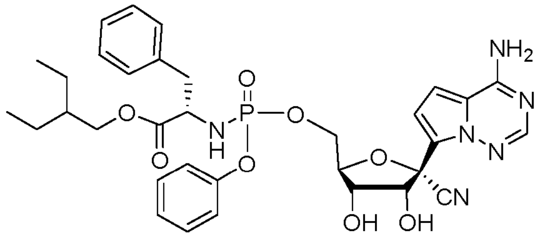,

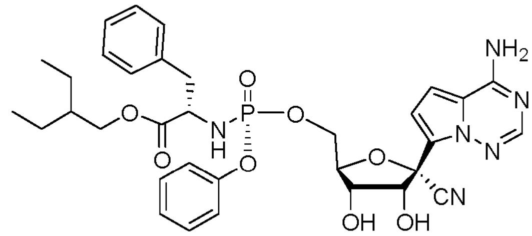,

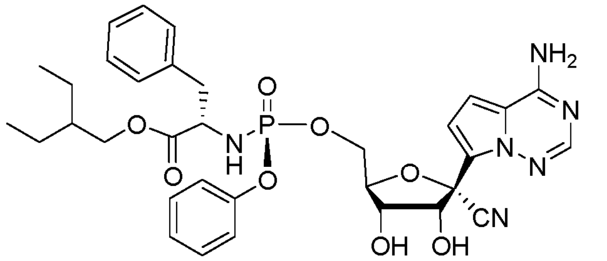,

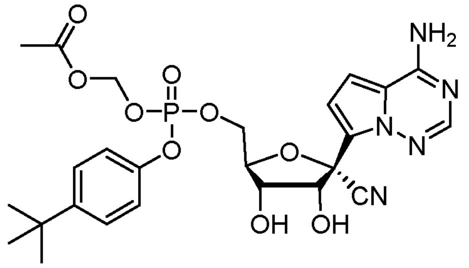,

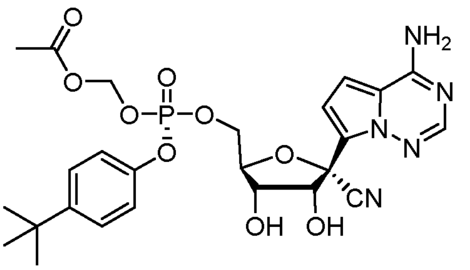,

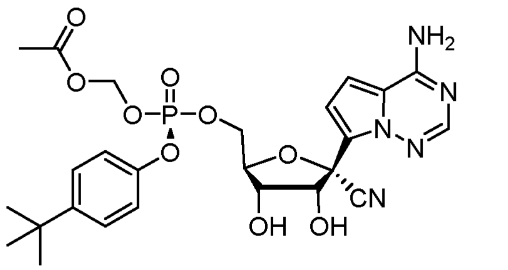,

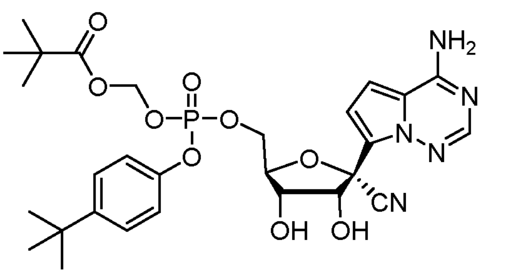,

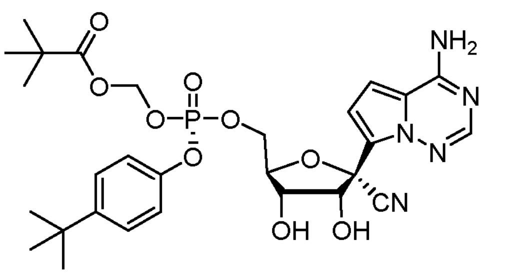,

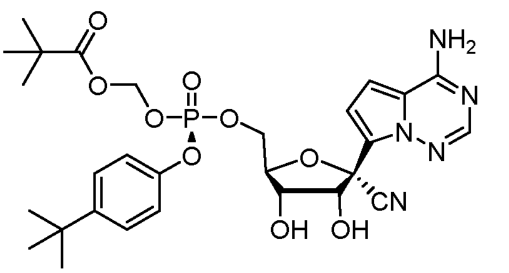,

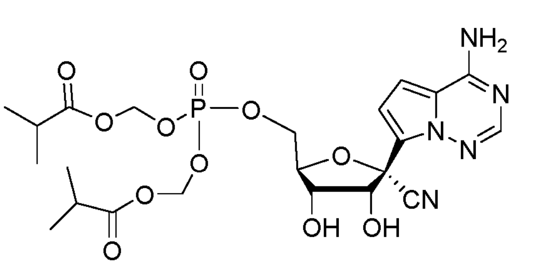,

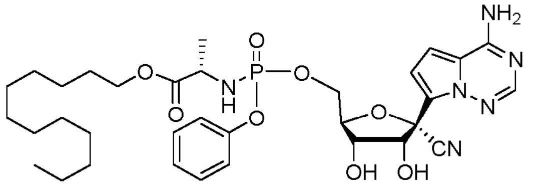,

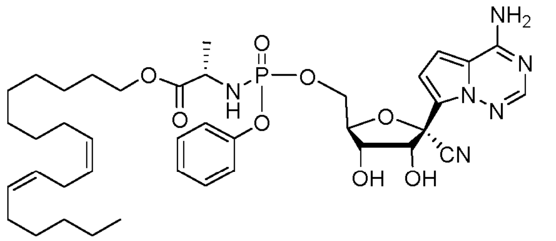,

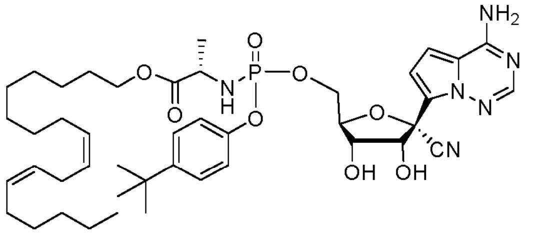,

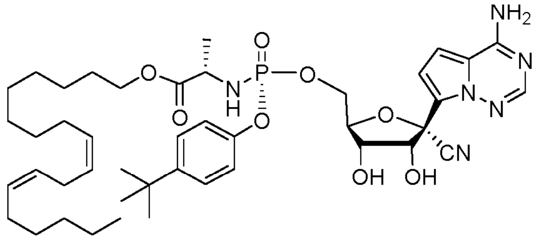,

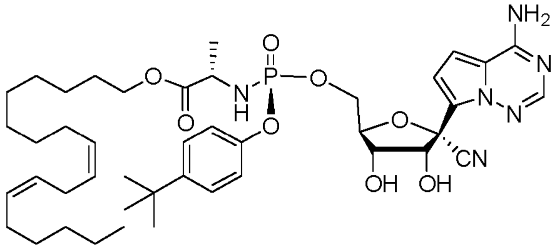,

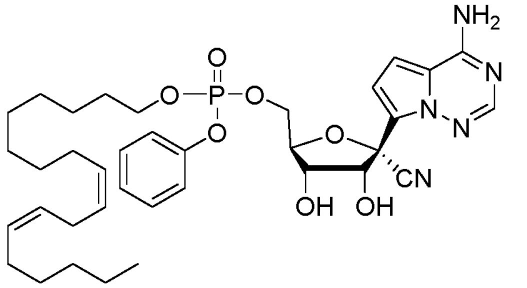,

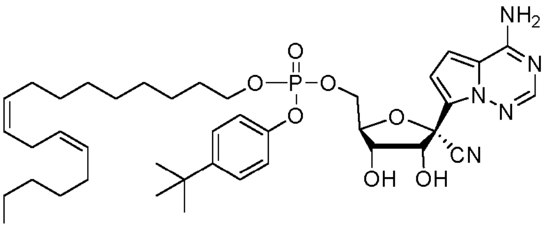

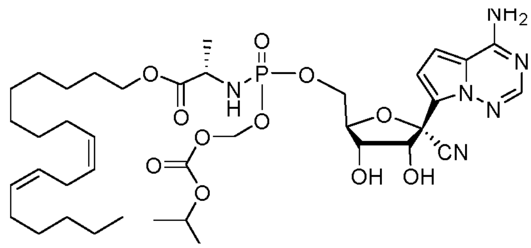

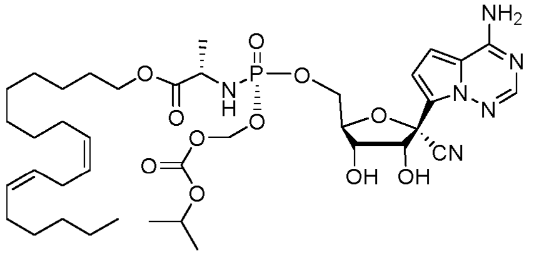,

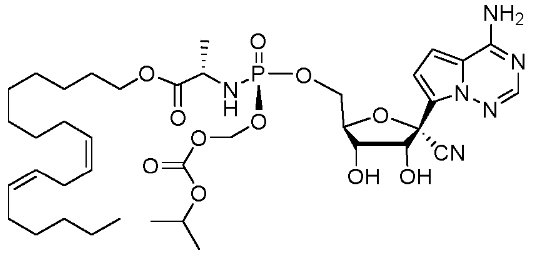,

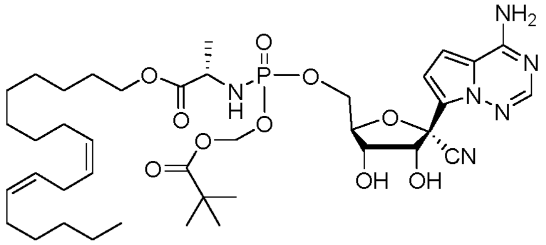,

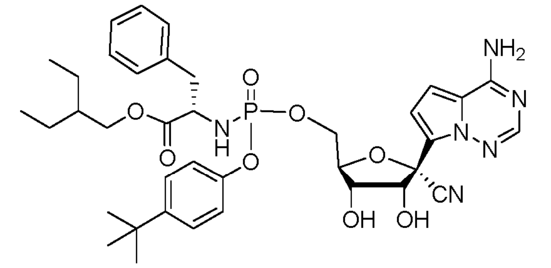,

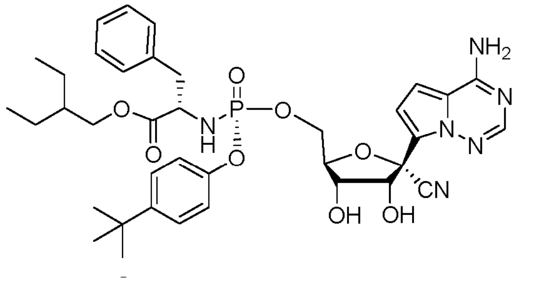

,

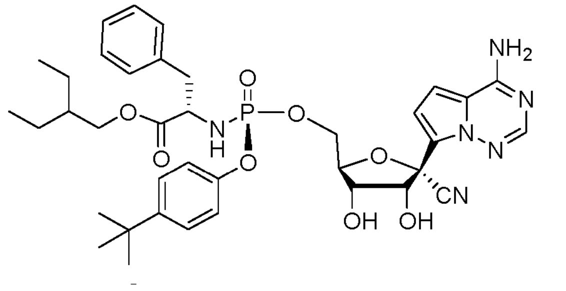

,

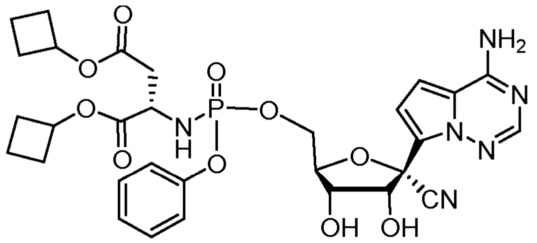

,

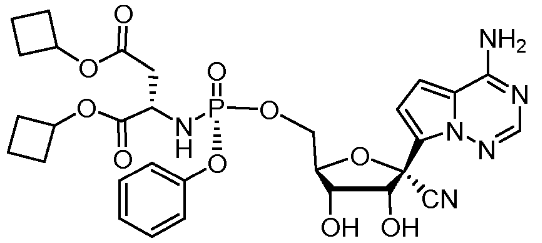

,

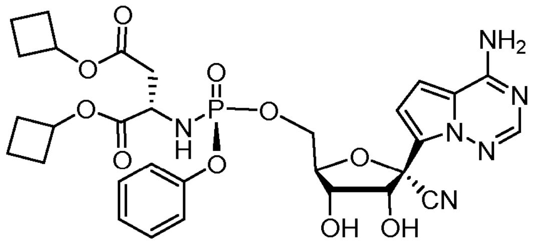

,

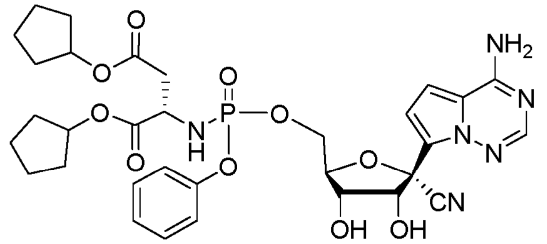

,

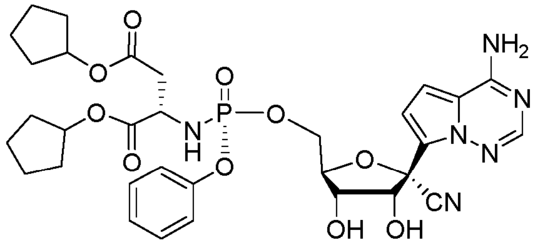

,

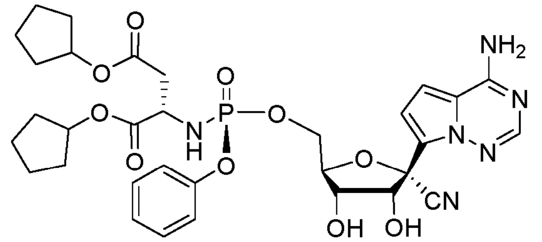

,

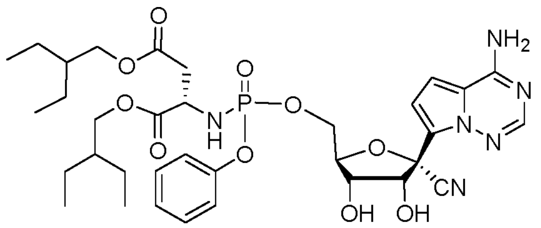

,

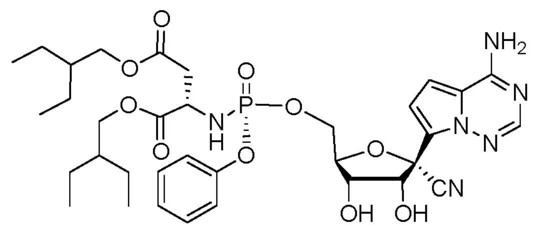

,

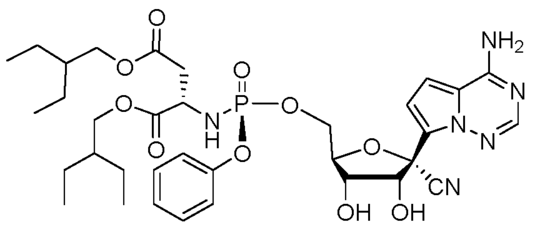

,

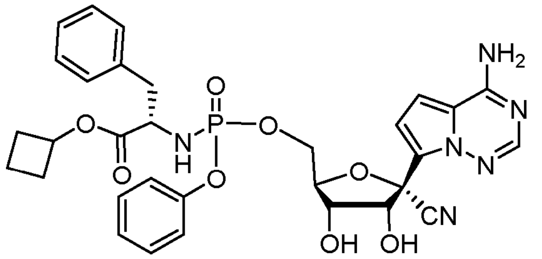

,

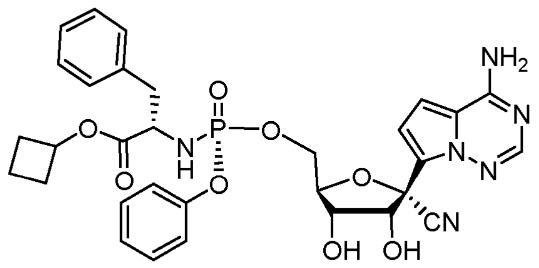
,
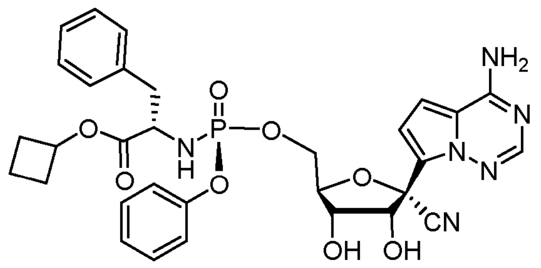
,
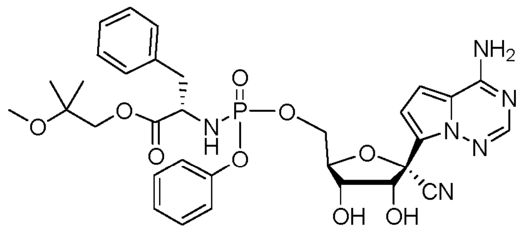
,
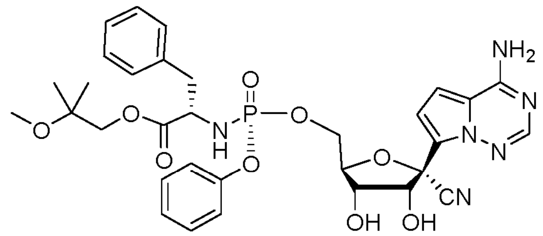
,
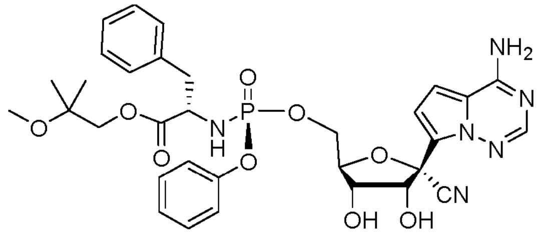
,
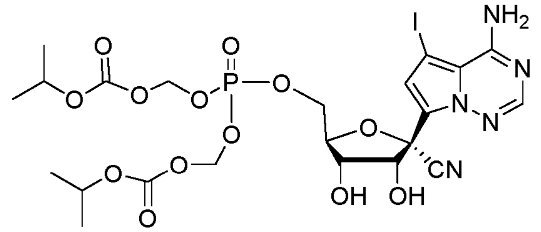
,
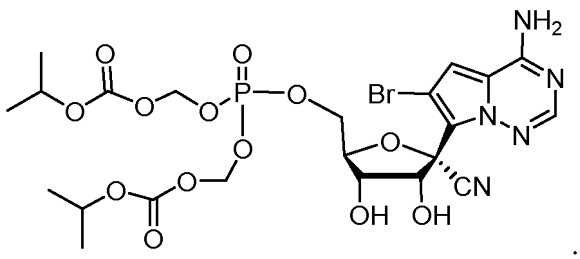
.

**59.** A pharmaceutical composition comprising the compound of any one of claims 1 to 58, or a tautomer, stereoisomer, prodrug, crystalline form, pharmaceutically acceptable salt, hydrate or solvate thereof, and a pharmaceutically acceptable excipient, as well as optionally, other therapeutic agents.

**60.** Use of the compound of any one of claims 1 to 58, or a tautomer, stereoisomer, prodrug, crystalline form, pharmaceutically acceptable salt, hydrate or solvate thereof, or the pharmaceutical composition of claim 59 in the preparation of a medicament for the treatment and/or prevention of viral infections.

**61.** A method for treating and/or preventing viral infections in a subject, the method comprising administering the compound of any one of claims 1 to 58, or a pharmaceutically acceptable salt, stereoisomer, solvate, hydrate, polymorph, prodrug or isotopic variant thereof, or the pharmaceutical composition of claim 59 to said subject.

**62.** A compound of any one of claims 1 to 58, or a tautomer, stereoisomer, prodrug, crystalline form, pharmaceutically acceptable salt, hydrate or solvate thereof, or the pharmaceutical composition of claim 59, for use in treatment and/or prevention of viral infections.

**63.** The use of claim 60, the method of claim 61, or the use of compound or composition of claim 62, wherein said viral infections are *Coronaviridae* infections, *Paramyxoviridae* infections, *Pneumoviridae* infections, *Picornaviridae* infections, *Flaviviridae* infections, *Filoviridae* infections, *Arenaviridae* infections, *Orthomyxoviridae* infections, or monkeypox;

preferably, said coronavirus is SARS-CoV virus and variants thereof, MERS-CoV virus and variants thereof and SARS-CoV-2 virus and variants thereof, other human coronaviruses (229E, NL63, OC43, HKU1 or WIV1), zoonotic coronaviruses (PEDV or HKU CoV isolates, such as HKU3, HKU5 or HKU9), feline coronaviruses (Feline Enteric Coronavirus or Feline infectious peritonitis virus) or porcine epidemic diarrhea virus; preferably, said coronavirus is SARS-CoV virus and variants thereof, MERS-CoV virus and variants thereof and SARS-CoV-2 virus and variants thereof; preferably, said coronavirus is SARS-CoV-2 virus and variants thereof; preferably, said SARS-CoV-2 virus variants are Alpha variant, Beta variant, Delta variant, Gamma variant or Omicron variant;
preferably, said *Paramyxoviridae* is parainfluenza virus, measles or mumps virus;
preferably, said *Pneumoviridae* is RSV or Human metapneumovirus; preferably, said *Pneumoviridae* is RSV;
preferably, said *Picornavirridae* is Enterovirus or Rhinovirus;
preferably, said *Flaviviridae* are Dengue virus, Yellow Fever virus, West Nile virus, Zika virus, Japanese Encephalitis virus and Hepatitis C;
preferably, said *Filoviridae* is Ebola virus, Zaire ebolavirus, Bundibugio ebolavirus, Sudan ebolavirus, Tai Forest ebolavirus, Reston ebolavirus or Marburg virus;
preferably, said *Arenaviridae* is Lassa virus or Junin virus.

**INTERNATIONAL SEARCH REPORT**

International application No.
**PCT/CN2023/136943**

**A. CLASSIFICATION OF SUBJECT MATTER**

C07F9/6561(2006.01)i; A61K31/53(2006.01)i; A61P31/12(2006.01)i; A61P31/14(2006.01)i; A61P31/16(2006.01)i

According to International Patent Classification (IPC) or to both national classification and IPC

**B. FIELDS SEARCHED**

Minimum documentation searched (classification system followed by classification symbols)

C07F, A61K, A61P

Documentation searched other than minimum documentation to the extent that such documents are included in the fields searched

Electronic data base consulted during the international search (name of data base and, where practicable, search terms used)

CNTXT, ENTXT, VEN, CNKI, STN(MARPAT, REGISTRY, CAPLUS): 核苷酸, 磷脂, 病毒, nucleoside, phospholipid, virus, 检索结构式, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022046631 A1 (GILEAD SCIENCES INC.) 03 March 2022 (2022-03-03) abstract, claims 1-119, and embodiment 13 | 1-54, 57-63 |
| X | CN 112778310 A (SHANGHAI INSTITUTE OF MATERIA MEDICA, CHINESE ACADEMY OF SCIENCES et al.) 11 May 2021 (2021-05-11) abstract, and claims 1-10 | 1-54, 57-63 |
| X | CN 108348526 A (GILEAD SCIENCES, INC.) 31 July 2018 (2018-07-31) abstract, and claims 39-74 | 1-54, 57-63 |
| A | WO 2021262826 A2 (GILEAD SCIENCES INC.) 30 December 2021 (2021-12-30) abstract, and claims 1-137 | 1-54, 57-63 |
| PX | CN 116987118 A (BEIJING MUHUA BIOTECHNOLOGY CO., LTD.) 03 November 2023 (2023-11-03) abstract, claims 1-10, and description, paragraphs [0171]-[0181] | 1-54, 57-63 |
| PX | CN 116217621 A (BEIJING MUHUA BIOTECHNOLOGY CO., LTD.) 06 June 2023 (2023-06-06) abstract, and claims 1-10 | 1-54, 57-63 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **26 February 2024** | **29 February 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/136943**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **61-63**
because they relate to subject matter not required to be searched by this Authority, namely:

Claims 61-63 relate to a method for treatment of a disease, and the present search report is provided on the basis of a corresponding pharmaceutical use thereof.

2. ☑ Claims Nos.: **55-56**
because they relate to parts of the international application that do not comply with the prescribed requirements to such an extent that no meaningful international search can be carried out, specifically:

The structural formula in claims 55-56 does not defined $R_{11}$, so that the scopes of protection of said claims are unclear, no meaningful search and examination can be made, and said claims do not comply with PCT Article 6.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/136943**

| Patent document cited in search report | Publication date (day/month/year) | Patent family member(s) | Publication date (day/month/year) |
|---|---|---|---|
| WO 2022046631 A1 | 03 March 2022 | IL 300412 A | 01 April 2023 |
| | | US 2023250117 A1 | 10 August 2023 |
| | | AU 2021330835 A1 | 30 March 2023 |
| | | AU 2021330835 B2 | 14 December 2023 |
| | | CR 20230100 A | 28 April 2023 |
| | | CO 2023001953 A2 | 17 March 2023 |
| | | BR 112023002951 A2 | 21 March 2023 |
| | | DOP 2023000037 A | 09 July 2023 |
| | | KR 20230057419 A | 28 April 2023 |
| | | EP 4200301 A1 | 28 June 2023 |
| | | TW 202222323 A | 16 June 2022 |
| | | TWI 807399 B | 01 July 2023 |
| | | CA 3188373 A1 | 03 March 2022 |
| | | US 2024025932 A1 | 25 January 2024 |
| | | US 2022081455 A1 | 17 March 2022 |
| | | US 11773122 B2 | 03 October 2023 |
| | | CL 2023000524 A1 | 29 September 2023 |
| | | JP 2023538442 A | 07 September 2023 |
| CN 112778310 A | 11 May 2021 | None | |
| CN 108348526 A | 31 July 2018 | MX 2018003161 A | 17 May 2018 |
| | | PL 3785717 T3 | 02 May 2022 |
| | | MA 42819 A | 25 July 2018 |
| | | KR 20180050742 A | 15 May 2018 |
| | | EP 3785717 A1 | 03 March 2021 |
| | | EP 3785717 B1 | 05 January 2022 |
| | | DK 3785717 T3 | 21 March 2022 |
| | | SI 3785717 T1 | 29 April 2022 |
| | | LT 3349758 T | 11 July 2022 |
| | | HRP 20220740 T1 | 11 November 2022 |
| | | PT 3349758 T | 13 July 2022 |
| | | EP 4088718 A1 | 16 November 2022 |
| | | HK 1256872 A1 | 04 October 2019 |
| | | CY 1125089 T1 | 09 June 2023 |
| | | ES 2909419 T3 | 06 May 2022 |
| | | TW 201733595 A | 01 October 2017 |
| | | JP 2020111587 A | 27 July 2020 |
| | | JP 7254738 B2 | 10 April 2023 |
| | | CA 2998189 A1 | 23 March 2017 |
| | | CA 2998189 C | 03 August 2021 |
| | | US 2019255085 A1 | 22 August 2019 |
| | | US 10695361 B2 | 30 June 2020 |
| | | MX 2021005087 A | 18 August 2022 |
| | | EP 3349758 A1 | 25 July 2018 |
| | | EP 3349758 B1 | 06 April 2022 |
| | | HK 1257628 A1 | 25 October 2019 |
| | | HK 1255732 A1 | 23 August 2019 |
| | | SG 10202001878 WA | 29 April 2020 |
| | | US 2020352967 A1 | 12 November 2020 |
| | | US 11007208 B2 | 18 May 2021 |
| | | LT 3785717 T | 11 April 2022 |

Form PCT/ISA/210 (patent family annex) (July 2022)

**INTERNATIONAL SEARCH REPORT**
**Information on patent family members**

International application No.
**PCT/CN2023/136943**

| Patent document cited in search report | Publication date (day/month/year) | Patent family member(s) | Publication date (day/month/year) |
|---|---|---|---|
| | | SG 10202109869 XA | 28 October 2021 |
| | | MA 52371 A | 22 September 2021 |
| | | DK 3349758 T3 | 13 June 2022 |
| | | AU 2023200990 A1 | 23 March 2023 |
| | | JP 2018531227 A | 25 October 2018 |
| | | JP 6742403 B2 | 19 August 2020 |
| | | PL 3349758 T3 | 12 September 2022 |
| | | AU 2020233714 A1 | 08 October 2020 |
| | | AU 2020233714 B2 | 24 November 2022 |
| | | PT 3785717 T | 14 April 2022 |
| | | BR 112018005048 B1 | 23 February 2021 |
| | | BR 112018005048 B8 | 23 March 2021 |
| | | BR 122020020217 B1 | 17 August 2021 |
| | | JP 2021107431 A | 29 July 2021 |
| | | HRP 20220355 T1 | 13 May 2022 |
| | | WO 2017049060 A1 | 23 March 2017 |
| | | JP 2023085459 A | 20 June 2023 |
| | | ES 2918585 T3 | 19 July 2022 |
| | | HUE 057928 T2 | 28 June 2022 |
| | | SI 3349758 T1 | 31 August 2022 |
| | | US 2021393659 A1 | 23 December 2021 |
| | | US 11382926 B2 | 12 July 2022 |
| | | US 2017071964 A1 | 16 March 2017 |
| | | US 10251904 B2 | 09 April 2019 |
| | | EA 201890494 A1 | 31 October 2018 |
| | | EA 038141 B1 | 13 July 2021 |
| | | HUE 058737 T2 | 28 September 2022 |
| | | AU 2016323604 A1 | 29 March 2018 |
| | | AU 2016323604 B2 | 09 July 2020 |
| | | US 2023027727 A1 | 26 January 2023 |
| WO 2021262826 A2 | 30 December 2021 | None | |
| CN 116987118 A | 03 November 2023 | None | |
| CN 116217621 A | 06 June 2023 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5376645 A


### Non-patent literature cited in the description


- **BERGE et al.** *J. Pharmaceutical Sciences*, 1977, vol. 66, 1-19
- Prodrugs as Novel Delivery Systems, A.C.S. Symposium Series. **T. HIGUCHI** ; **V. STELLA**. Bioreversible Carriers in Drug Design. Pergamon Press, 1987, vol. 14
- **D. FLEISHER** ; **S. RAMON** ; **H. BARBRA**. Improved oral drug delivery: solubility limitations overcome by the use of prodrugs. *Advanced Drug Delivery Reviews*, 1996, vol. 19 (2), 115-130
- Remington's Pharmaceutical Sciences. Mack Publishing Company, 1985